# EUROPÄISCHE PATENTSCHRIFT

(11) **EP 3 169 665 B1**
(45) Veröffentlichungstag und Bekanntmachung des Hinweises auf die Patenterteilung: **09.05.2018**
(21) Anmeldenummer: 15737988.4
(22) Anmeldetag: 14.07.2015
(51) Int. Cl.: C07D 209/96, A61K 31/403, A61P 29/00

(54) **SUBSTITUIERTE AZA-SPIRO(4.5)DECAN-DERIVATE**
SUBSTITUTED AZA-SPIRO(4.5)DECANE DERIVATIVES
DÉRIVÉS D'AZA-SPIRO(4.5)DÉCANE SUBSTITUÉS

(30) Priorität: 15.07.2014 EP 14002438
(43) Veröffentlichungstag der Anmeldung: 24.05.2017
(73) Patentinhaber: Grünenthal GmbH, 52078 Aachen (DE)
(72) Erfinder: WEGERT, Anita, 52457 Aldenhoven (DE); NOLTE, Bert, 53902 Bad Münstereifel (DE); LINZ, Klaus, 53359 Rheinbach (DE); HARLFINGER, Stephanie, 4055 Basel (CH); KÖGEL, Babette-Yvonne, 52379 Langerwehe-Hamich (DE); RATCLIFFE, Paul, 52076 Aachen (DE); THEIL, Fritz, 13125 Berlin (DE); GRÖGER, Olga, 12587 Berlin (DE); BRAUN, Birgit, 12101 Berlin (DE)
(86) Internationale Anmeldenummer: PCT/EP2015/001446
(87) Internationale Veröffentlichungsnummer: WO 2016/008583

(56) Entgegenhaltungen:
- WO-A1-2007/019987
- US-A1- 2010 048 554
- HALFPENNY P R ET AL: "HIGHLY SELECTIVE KAPPA-OPIOID ANALGESICS. 3. SYNTHESIS AND STRUCTURE-ACVITIY REALTIONSHIPS OF NOVEL N-Ä2-(1-PYRROLIDINYL)-4- OR 5-SUBSTITUTED-CYCLOHEXYLÜARYLACETAMIDE DERIVATIVES", JOURNAL OF MEDICINAL CHEMISTRY, AMERICAN CHEMICAL SOCIETY, US, Bd. 33, Nr. 1, 1. Januar 1990 (1990-01-01) , Seiten 286-291, XP002952674, ISSN: 0022-2623, DOI: 10.1021/JM00163A047

## Beschreibung

Die vorliegende Erfindung betrifft substituierte spirocyclische Cyclohexan-Derivate, welche eine Affinität an den µ-Opioid-Rezeptor und den ORL1-Rezeptor aufweisen, Verfahren zu deren Herstellung, Arzneimittel enthaltend diese Verbindungen und die Verwendung dieser Verbindungen zur Herstellung von Arzneimitteln.

Spirocyclische Cyclohexan-Derivate, welche eine Affinität an den µ-Opioid-Rezeptor und den ORL1-Rezeptor aufweisen, sind im Stand der Technik bekannt. In diesem Zusammenhang kann beispielsweise vollumfänglich verwiesen werden auf WO2004/043967, WO2005/ 063769, WO2005/066183, WO2006/018184, WO2006/108565, WO2007/124903, WO2008/ 009416, WO2008/101659, WO2009/118169 und WO2009/118173.

Die bekannten Verbindungen sind jedoch nicht in jeglicher Hinsicht zufrieden stellend und es besteht ein Bedarf an weiteren Verbindungen mit vergleichbaren oder besseren Eigenschaften.

So zeigen die bekannten Verbindungen in geeigneten Bindungsassays mitunter eine gewisse Affinität zum hERG-Ionenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner zeigen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen , die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner zeigen die bekannten Verbindungen mitunter eine nur geringe Selektivität gegenüber dem kappa-Opioid-Rezeptor auf, welcher für Nebenwirkungen, insbesondere Dysphorie, Sedation, Diurese verantwortlich ist. Darüber hinaus zeigen die bekannten Verbindungen mitunter eine sehr hohe Affinität an den µ-Opioid-Rezeptor, welcher im Zusammenhang mit anderen Nebenwirkungen, insbesondere Atemdepression, Obstipation und Suchtabhängigkeit zu stehen scheint.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst.

Es wurde überraschend gefunden, dass substituierte spirocyclische Cyclohexanderivate hergestellt werden können, welche eine Affinität an den µ-Opioid-Rezeptor und den ORL1-Rezeptor aufweisen.

Die Erfindung betrifft Verbindungen der allgemeinen Formel (1), 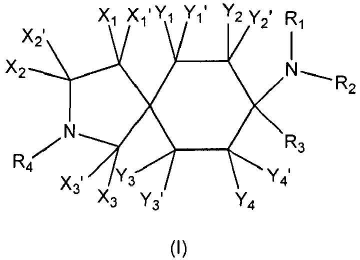 worin
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂ -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N+(R₀)₂O⁻, -NHC(=O)R₀, -NHC(=O)OR₀, -NHC(=O)NH₂, -NHC(=O)NHR₀ und -NHC(=O)N(R₀)₂; vorzugsweise jeweils unabhängig voneinander ausgewählt sind aus der Gruppe bestehend aus -H, -F, -Cl, -CN, -C₁₋₈-Aliphat, -C₁₋₈-Aliphat-NHC₁₋₈-Aliphat, -C₁₋₈-Aliphat-N(C₁₋₈-Aliphat)₂, -S-C₁₋₈-Aliphat, -S-Aryl, -Aryl, -C₁₋₈-Aliphat-Aryl; oder Y₁ und Y₁', oder Y₂ und Y₂', oder Y₃ und Y₃', oder Y₄ und Y₄' gemeinsam für =O stehen;
X₁, X₁', X₂, X₂', X₃ und X₃' jeweils unabhängig voneinander für -H, -F, -Cl, -Br, -I, -NO₂, -CF₃, -OR₅, -SR₅, -SO₂R₅, -S(=O)₂OR₅, -CN, -COOR₅, -CONR₅, -NR₆R₇, oder -R₀ stehen; oder X₁ und X₁', oder X₂ und X₂', oder X₃ und X₃' gemeinsam für =O stehen; oder X₁ und X₂, oder X₂ und X₃ gemeinsam für -(CH₂)₂₋₆- stehen, wobei einzelne Wasserstoffatome auch durch -F, -Cl, -Br, -I, -NO₂, -CF₃, -OR₅, -CN oder -C₁₋₆-Aiiphat ersetzt sein können; oder X₁ und X₁' oder X₂ und X₂' oder X₃ und X₃' gemeinsam für ein C₃₋₆-Cycloaliphat stehen, wobei einzelne Wasserstoffatome auch durch -F, -Cl, -Br, -I, -NO₂, -CF₃, -OR₅, -CN oder -C₁₋₆-Aliphat ersetzt sein können;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₈CH₂CH₂- oder -(CH₂)₃₋₆- stehen;
R₃ für -R₀ steht;
R₄ für H oder -Z-R₁₁ steht,
   wobei
Z abwesend oder -C(=O)-, -S(=O)- oder -S(=O)₂- sein kann, und
R₁₁ für -C₁₋₆-Alkyl, -C₃₋₆-Cycloalkyl, oder -C₁₋₃-Alkyl-C₃₋₆-Cycloalkyl steht, wobei in der C₃₋₆-Cycloalkylgruppe ein Ringkohlenstoffatom durch ein Sauerstoffatom ersetzt sein kann und -C₁₋₆-Alkyl, -C₃₋₆-Cycloalkyl oder -C₁₋₃-Alkyl-C₃₋₆-Cycloalkyl unsubstituiert, einfach oder mehrfach substituiert sein kann, mit Substituenten unabhängig voneinander ausgesucht aus der Gruppe bestehend aus -F, -Cl, -Br, -I, -CN, -OH,-SH, -O-C₁₋₃-Alkyl, und -S-C₁₋₃-Alkyl, wobei -C₁₋₃-Alkyl substituiert sein kann mit einem oder mehreren Substituenten aus der Gruppe bestehend aus -F, -Cl, -Br, -I, -CN,-OH, -OCH₃, -SH und -SCH₃;
R₅ jeweils unabhängig für -H oder -R₀ steht;
R₆ und R₇ unabhängig voneinander für -H oder -R₀ stehen; oder R₆ und R₇ zusammen für -CH₂CH₂OCH₂CH₂-, -CH₂CH₂NR₁₀CH₂CH₂- oder -(CH₂)₃₋₆- stehen;
R₈ für -H, -R₀ oder -C(=O)R₀ steht;
R₁₀ für -H oder -C₁₋₆-Aliphat steht;
   wobei
"Aliphat" jeweils ein verzweigter oder unverzweigter, gesättigter oder ein ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf);
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O -R₀, -C(=O)R₀, -C(=O)H, -C(=O)-OH, -C(=O)OR₀, -C(=O)NH₂ -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O -R₀, -C(=O)R₀, -C(=O)H, -C(=O)OH, -C(=O)OR₀, -C(=O)NH₂ -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂ -NHC(=O)NHR₀, -NHC(=O)N(R₀)₂, -Si(R₀)₃, -PO(OR₀)₂ verstanden wird; wobei ggf. vorhandene N-Ringatome jeweils oxidiert sein können (N-Oxid);
in Form eines einzelnen Stereoisomers oder deren Mischung, der freien Verbindungen und/oder ihrer physiologisch verträglichen Salze und/oder Solvate.

Bei der Zusammenfassung verschiedener Reste, beispielsweise Y₁, Y₁', Y₂, Y₂', Y₃, Y₃', Y₄ und Y₄' sowie der Zusammenfassung von Resten an deren Substituenten, wie z. B. -OR₀,-OC(=O)R₀, -OC(=O)NHR₀, kann ein Substituent, z.B. R₀, für zwei oder mehrere Reste, beispielsweise -OR₀, -OC(=O)R₀, -OC(=O)NHR₀, innerhalb einer Substanz unterschiedliche Bedeutungen annehmen.

Die erfindungsgemäßen Verbindungen zeigen gute Bindung an den ORL1-Rezeptor und/oder den µ-Opioid-Rezeptor, vorzugsweise an den ORL1-Rezeptor und den µ-Opioid-Rezeptor.

Die erfindungsgemäßen Verbindungen weisen bevorzugt einen Kᵢ-Wert am µ-Opioid-Rezeptor von höchstens 500 nM, bevorzugter höchstens 100 nM oder höchstens 50 nM, noch bevorzugter höchstens 10 nM, am bevorzugtesten höchstens 1,0 nM und insbesondere höchstens 0,5 nM auf.

Methoden zur Bestimmung des Kᵢ-Werts am µ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen Kᵢ-Wert am ORL1-Rezeptor von höchstens 500 nM, bevorzugter höchstens 100 nM oder höchstens 50 nM, noch bevorzugter höchstens 10 nM, am bevorzugtesten höchstens 1,0 nM und insbesondere höchstens 0,75 nM auf.

Methoden zur Bestimmung des Kᵢ-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass die erfindungsgemäßen Verbindungen mit Affinität zum ORL1- und µ-Opioid-Rezeptor ein pharmakologisches Profil aufweisen, das verglichen mit dem anderer Opioidrezeptorliganden deutliche Vorteile aufweist:
1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen µ-Opioiden deutlich bessere Verträglichkeit aus.
2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und µ-Opioid Komponente zurückzuführen ist.
3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.
4. Im Gegensatz zu gebräuchlichen Stufe-3 Opioiden zeigen die erfindungsgemäßen Verbindungen in Tiermodellen für chronischen Entzündungsschmerz (u.a. Carrageenan- oder CFA-induzierte Hyperalgesie, viszeraler Entzündungsschmerz) eine deutliche Wirkverstärkung gegenüber dem Akutschmerz.
5. Im Gegensatz zu gebräuchlichen Stufe-3 Opioiden sind µ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise nicht zu beobachten.

Aufgrund der reduzierten µ-opioiden Nebenwirkungen einerseits und der erhöhten Wirksamkeit bei chronischem, bevorzugt neuropathischem Schmerz andererseits zeichnen sich die gemischten ORL1/µ-Agonisten somit durch deutlich vergrößerte Sicherheitsabstände gegenüber reinen µ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

Eine bevorzugte Ausführungsform der Erfindung betrifft Verbindungen der allgemeinen Formel (2), d. h. Y₁', Y₂', Y₃', und Y₄' sind jeweils gleich -H:

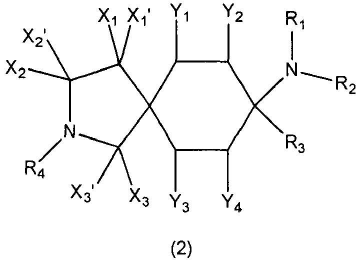

In einer bevorzugten Ausführungsform der erfindungsgemäßen Verbindung (2) sind Y₁, Y₂, Y₃ und Y₄ ungleich -H. In einer anderen bevorzugten Ausführungsform der erfindungsgemäßen Verbindung (2) sind drei der Reste Y₁, Y₂, Y₃ und Y₄ ungleich -H, und der übrige Rest ist gleich -H. In einer anderen bevorzugten Ausführungsform sind zwei der Reste Y₁, Y₂, Y₃ und Y₄ ungleich -H, und die übrigen zwei Reste sind gleich -H. In einer weiteren bevorzugten Ausführungsform der erfindungsgemäßen Verbindung (2) ist einer der Reste Y₁, Y₂, Y₃ und Y₄ ungleich -H, und die übrigen Reste gleich -H.

In einer besonders bevorzugten Ausführungsform der erfindungsgemäßen Verbindung (2) stehen Y₁, Y₂, Y₃, und Y₄ jeweils für -H.

Besonders bevorzugt sind Verbindungen der allgemeinen Formel (1) oder (2), worin
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂.

Eine bevorzugte Ausführungsform der erfindungsgemäßen Verbindung (2) betrifft Verbindungen der allgemeinen Formel (2.1):

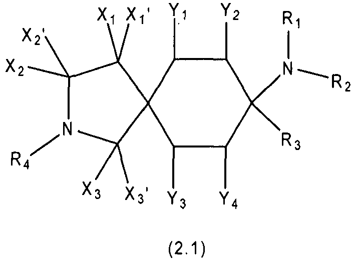

Besonders bevorzugt sind Verbindungen der allgemeinen Formel (2), worin
R₃ für -C₁₋₈-Aliphat, -Aryl, -Heteroaryl, -C₁₋₃-Aliphat-Aryl, -C₁₋₃-Aliphat-Heteroaryl oder -C₁₋₃-Aliphat-C₅₋₆-Cycloaliphat steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, -OH, -OCH₃, -OC₂H₅ und -N(CH₃)₂;
und X₁, X₁', X₂, X₂', X₃, X₃' jeweils unabhängig voneinander für -H, -F, -Cl, -Br, -I, -NO₂, -CF₃, -OR₅, -SR₅, -SO₂R₅, -S(=O)₂OR₅, -CN, -COOR₅, -CONR₅, -NR₆R₇, oder -R₀ stehen; oder X₁ und X₁', oder X₂ und X₂', oder X₃ und X₃' gemeinsam für =O stehen.

Besonders bevorzugt sind Verbindungen der allgemeinen Formel (3), d. h. Y₁, Y₁', Y₂, Y₂', Y₃, Y₃', Y₄ und Y₄' sind jeweils gleich -H:

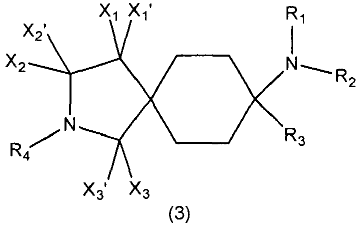

In weiteren Ausführungsformen der Verbindungen der allgemeinen Formel (3) steht einer der Reste X₁ und X₁' für H und der andere für -C₁₋₈-Aliphat, -C₃₋₁₂-Cycloaliphat, -Aryl, -Heteroaryl, -C₁₋₈-Aliphat-C₃₋₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂;

Bevorzugte Ausführungsformen der Verbindungen der allgemeinen Formel (3) haben die allgemeine Formel (3.1): 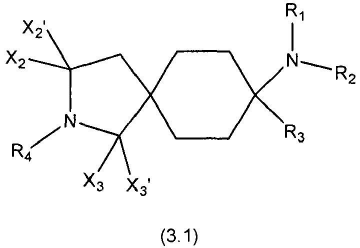 Diese Ausführungsformen betreffen Verbindungen der allgemeinen Formel (3), in denen X₁ und X₁' gleich -H sind.

Besonders bevorzugt sind Verbindungen der allgemeinen Formeln (3.1), worin
X₂, X₂', X₃ und X₃' für H stehen; oder X₂ und X₂', oder X₃ und X₃' gemeinsam für =O stehen;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂;
R₁ für -CH₃ steht;
R₂ für -H oder -CH₃ steht; oder
R₁ und R₂ gemeinsam einen Ring bilden und für -(CH₂)₃₋₄- stehen; und
R₃ für -C₁₋₈-Aliphat, -Aryl, -Heteroaryl, -C₁₋₃-Aliphat-Aryl, -C₁₋₃-Aliphat-Heteroaryl oder -C₁₋₃-Aliphat-C₅₋₆-Cycloaliphat steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, -OH, -OCH₃, -OC₂H₅ und -N(CH₃)₂; und;
R₄ für H oder -Z-R₁₁ steht,
wobei
Z abwesend oder -C(=O)-, -S(=O)- oder -S(=O)₂- sein kann, und
R₁₁ für -C₁₋₆-Alkyl, -C₃₋₆-Cycloalkyl, oder -C₁₋₃-Alkyl-C₃₋₆-Cycloalkyl steht, wobei in der C₃₋₆-Cycloalkylgruppe ein Ringkohlenstoffatom durch ein Sauerstoffatom ersetzt sein kann und -C₁₋₆-Alkyl, -C₃₋₆-Cycloalkyl oder -C₁₋₃-Alkyl-C₃₋₆-Cycloalkyl unsubstituiert, einfach oder mehrfach substituiert sein kann, mit Substituenten unabhängig voneinander ausgesucht aus der Gruppe bestehend aus -F, -Cl, -Br, -I, -CN, -OH,-SH, -O-C₁₋₃-Alkyl, und -S-C₁₋₃-Alkyl, wobei -C₁₋₃-Alkyl substituiert sein kann mit einem oder mehreren Substituenten aus der Gruppe bestehend aus -F, -Cl, -Br, -I, -CN,-OH, -OCH₃, -SH und -SCH₃;

Bevorzugte Ausführungsformen der Verbindungen der allgemeinen Formeln (3.1) haben die allgemeine Formel (3.1.1), (3.1.2), (3.1.3), (3.1.4), (3.1.5) oder (3.1.6):

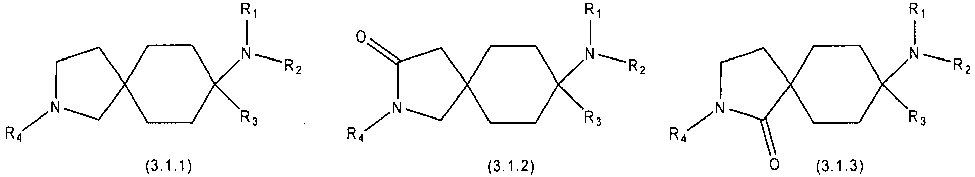

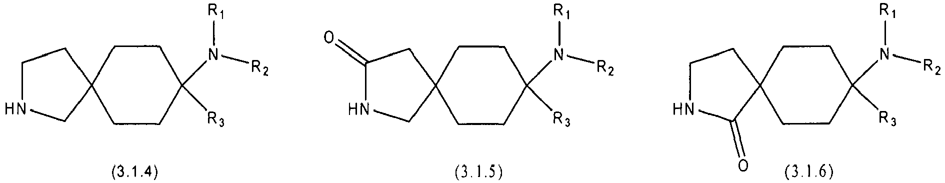

Eine weitere bevorzugte Ausführungsform betrifft Verbindungen der allgemeinen Formeln (4.1), d. h. R₁ und R₂ sind jeweils gleich -CH₃.

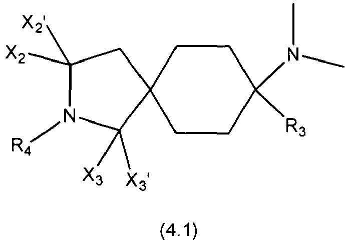

Bevorzugte Ausführungsformen der Verbindungen der allgemeinen Formeln (4.1) haben die allgemeine Formel (4.1.1), (4.1.2), (4.1.3), (4.1.4), (4.1.5) oder (4.1.6):

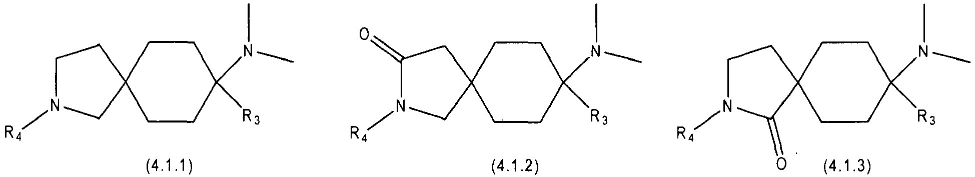

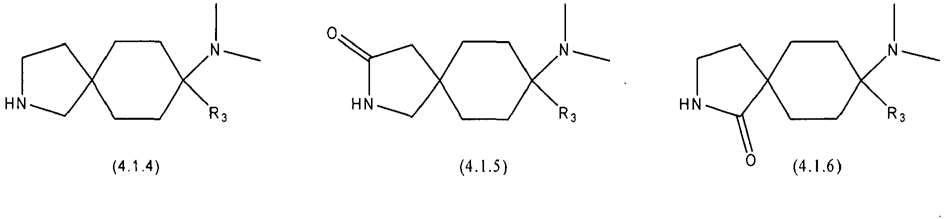

Bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -NH₂, -NH-C₁₋₆-Aliphat, -NH-C₃₋₈-Cycloaliphat, -NH-C₁₋₆-Aliphat-OH, -N(C₁₋₆-Aliphat)₂, -N(C₃₋₈-Cycloaliphat)₂, -N(C₁₋₆-Aliphat-OH)₂, -NO₂, -NH-C₁₋₆-Aliphat-C₃₋₈-Cycloaliphat, -NH-C₁₋₆-Aliphat-Aryl, -NH-C₁₋₆-Aliphat-Heteroaryl, -NH-Aryl, -NH-Heteroaryl, -SH, -S-C₁₋₆-Aliphat, -S-C₃₋₈-Cycloaliphat, -S-C₁₋₆-Aliphat-C₃₋₈-Cycloaliphat, -S-C₁₋₆-Aliphat-Aryl, -S-C₁₋₆-Aliphat-Heteroaryl, -S-Aryl, -S-Heteroaryl, -OH, -O-C₁₋₆-Aliphat, -O-C₃₋₈-Cycloaliphat, -O-C₁₋₆-Aliphat-OH, -O-C₁₋₆-Aliphat-C₃₋₈-Cycloaliphat, -O-C₁₋₆-Aliphat-Aryl, -O-C₁₋₆-Aliphat-Heretoaryl, -O-Aryl, -O-Heteroaryl, -O-C(=O)C₁₋₆-Aliphat, -O-C(=O)C₃₋₈-Cycloaliphat, -O-C(=O)C₁₋₆-Aliphat-OH, -O-C(=O)C₁₋₆-Aliphat-C₃₋₈-Cycloaliphat, -O-C(=O)C₁₋₆-Aliphat-Aryl, -O-C(=0)Ci.6-Aliphat-Heretoaryl, -O-C(=O)Aryl, -O-C(=O)Heteroaryl, -C₁₋₆-Aliphat, -C₃₋₈-Cycloaliphat, -C₁₋₆-Aliphat-C₃₋₈-Cycloaliphat, -C₁₋₆-Aliphat-Aryl, -C₁₋₆-Aliphat-Heteroaryl, -Aryl, -Heteroaryl, -C(=O)C₁₋₆-Aliphat, -C(=O)C₃₋₈-Cycloaliphat, -C(=O)C₁₋₆-Aliphat-C₃₋₈-Cycloaliphat, -C(=O)C₁₋₆-Aliphat-Aryl, -C(=O)C₁₋₆-Aliphat-Heteroaryl, -C(=O)Aryl, -C(=O)Heteroaryl, -CO₂H -CO₂-C₁₋₆-Aliphat, -CO₂-C₃₋₈-Cycloaliphat, -CO₂-C₁₋₆-Aliphat-C₃₋₈-Cycloaliphat, -CO₂-C₁₋₆-Aliphat-Aryl, -CO₂-C₁₋₆-Aliphat-Heteroaryl, -CO₂-Aryl, -CO₂-Heteroaryl; oder Y₁ und Y₁', oder Y₂ und Y₂', oder Y₃ und Y₃', oder Y₄ und Y₄' stehen gemeinsam für =O.

Bevorzugter sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -C₁₋₆-Aliphat, -C₁₋₆-Aliphat-NHC₁₋₆-Aliphat, -C₁₋₆-Aliphat-N(C₁₋₈-Aliphat)₂, -C₃₋₈-Cycloaliphat, -C₁₋₆-Aliphat-C₃₋₈-Cycloaliphat, -C₁₋₆-Aliphat-Aryl, -C₁₋₆-Aliphat-Heteroaryl, -S-C₁₋₈-Aliphat, -S-Aryl, -Aryl oder -Heteroaryl.

Besonders bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -C₁₋₆-Alkyl, -C₂₋₆-Alkenyl, -C₁₋₆-Alkyl-NH-C₁₋₆-Alkyl, -C₁₋₆-Alkyl-N(C₁₋₆-Alkyl)₂, -Aryl, -C₁₋₆-Alkyl-Aryl, -S-C₁₋₆-Alkyl und -S-Aryl.

In einer bevorzugten Ausführungsform ist mindestens einer der Reste Y₁, Y₁', Y₂, Y₂', Y₃, Y₃', Y₄ und Y₄' ungleich -H und die übrigen Reste stehen für -H.

Besonders bevorzugt stehen Y₁, Y₁', Y₂, Y₂', Y₃, Y₃', Y₄ und Y₄' jeweils für -H.

Bevorzugt stehen X₁, X₁', X₂, X₂', X₃ und X₃' jeweils unabhängig voneinander für -H, -F, -Cl, -Br, -I, -NO₂, -NR₆R₇, -C₁₋₆-Aliphat, -C₃₋₈-Cycloaliphat, -Aryl, -Heteroaryl, -C₁₋₆-Aliphat-Aryl, -C₁₋₆-Aliphat-Heteroaryl, oder -C₁₋₆-Aliphat-C₃₋₈-Cycloaliphat, oder X₁ und X₁', oder X₂ und X₂', oder X₃ und X₃' stehen gemeinsam für =O; oder X₁ und X₂, oder X₂ und X₃ stehen gemeinsam für -(CH₂)₂₋₆; oder X₁ und X₁' stehen gemeinsam für ein C₃-C₆-Cycloaliphat, vorzugsweise ein C₃₋₆-Cycloalkyl.

Bevorzugt sind insbesondere auch Verbindungen, bei denen X₁ X₁', X₂, X₂', X₃ und X₃ jeweils unabhängig voneinander für -H, -C₁₋₅-Aliphat, -Aryl oder über eine -C₁₋₃-Aliphat-Gruppe (Brücke) verknüpftes -Aryl stehen; oder X₁ und X₁', oder X₂ und X₂', oder X₃ und X₃' gemeinsam für =O stehen.

Besonders bevorzugt stehen X₁, X₁', X₂, X₂', X₃ und X₃' jeweils unabhängig voneinander für -H, -CH₃, -Phenyl oder -Benzyl, insbesondere für -H, oder X₁ und X₁', oder X₂ und X₂', oder X₃ und X₃' stehen gemeinsam für =O.

Ganz besonders bevorzugt stehen X₁ X₁', X₂, X₂', X₃ und X₃' für H; oder X₂ und X₂', oder X₃ und X₃' stehen gemeinsam für =O.

In einer bevorzugten Ausführungsform stehen X₂ und X₂' gemeinsam für =O und X₁, X₁', X₃ und X₃' stehen für -H.

In einer anderen bevorzugten Ausführungsform stehen X₃ und X₃' gemeinsam für =O und X₁, X₁', X₂ und X₂' stehen für -H.

In einer weiteren bevorzugten Ausführungsform stehen X₁, X₁', X₂, X₂', X₃ und X₃' für H.

R₀ steht bevorzugt jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₈-Aliphat-Heteroaryl. Dabei bedeuten -C₁₋₈-Aliphat-C₃₋₁₂-Cycloaliphat, -C₁₋₈-Aliphat-Aryl oder -C₁₋₈-Aliphat-Heteroaryl, dass die Reste -C₃₋₁₂-Cycloaliphat, -Aryl oder -Heteroaryl jeweils über eine zweibindige Brücke -C₁₋₈-Aliphat- gebunden sind. Bevorzugte Beispiele für -C₁₋₈-Aliphat-Aryl sind -CH₂-C₆H₅, -CH=CH-C₆H₅ und -CH₂CH₂-C₆H₅. Ein bevorzugtes Beispiel für -C₁₋₈-Aliphat-Heteroaryl ist -CH₂-Pyridyl. Ein bevorzugtes Beispiel für -C₁₋₈-Aliphat-C₃₋₁₂Cycloaliphat ist -CH₂-Cyclopentyl.

Bevorzugt stehen R₁ und R₂, unabhängig voneinander für -H; -C₁₋₆-Aliphat; -C₃₋₈-Cycloaliphat, -C₁₋₆-Aliphat-Aryl, -C₁₋₆-Aliphat-C₃₋₈-Cycloaliphat oder -C₁₋₆-Aliphat-Heteroaryl; oder die Reste R₁ und R₂ bilden zusammen einen Ring und bedeuten -CH₂CH₂OCH₂CH₂-, -CH₂CH₂NR₈CH₂CH₂- oder -(CH₂)₃₋₆-.

Bevorzugter stehen R₁ und R₂ unabhängig voneinander für -H; -C₁₋₅-Aliphat; oder die Reste R₁ und R₂ bilden zusammen einen Ring und bedeuten -CH₂CH₂OCH₂CH₂-, -CH₂CH₂NR₈-CH₂CH₂- oder -(CH₂)₃₋₆-, wobei R₈ bevorzugt -H oder -C₁₋₅-Aliphat bedeutet.

Besonders bevorzugt sind Verbindungen, worin R₁ und R₂ unabhängig voneinander für -CH₃ oder -H stehen, wobei R₁ und R₂ nicht gleichzeitig -H bedeuten; oder R₁ und R₂ bilden einen Ring und bedeuten -(CH₂)₃₋₄-.

Ganz besonders bevorzugt sind Verbindungen, worin R₁ und R₂ für -CH₃ stehen.

Bevorzugt steht R₃ für -C₁₋₈-Aliphat, -C₃₋₈-Cycloaliphat, -Aryl, -Heteroaryl; oder für jeweils über eine -C₁₋₃-Aliphat-Gruppe gebundenes -Aryl, -Heteroaryl oder -C₃₋₈-Cycloaliphat.

Bevorzugt steht R₃ für -C₁₋₅-Aliphat; jeweils gesättigt oder ungesättigt, unsubstituiert oder ein- oder mehrfach substituiert mit -OH, -OCH₃ oder -OC₂H₅; -Aryl, -Heteroaryl; jeweils unsubstituiert oder ein- oder mehrfach substituiert mit -F, -Cl, -Br, -CN, -CH₃, -C₂H₅, -NH₂,-NO₂, -SH, -CF₃, -OH, -OCH₃, -OC₂H₅ oder -N(CH₃)₂; oder für ein über eine -C₁₋₃-Aliphat-Gruppe gebundenes -C₅₋₆-Cycloaliphat.

Am bevorzugtesten steht R₃ für -Aryl, -Heteroaryl; jeweils unsubstituiert oder ein- oder mehrfach substituiert mit -F, -Cl, -Br, -CN, -CH₃, -C₂H₅, -NH₂, -NO₂, -SH, -CF₃, -OH, -OCH₃, -OC₂H₅ oder -N(CH₃)₂; oder für ein über eine -C₁₋₃-Aliphat-Gruppe gebundenes -C₅₋₆-Cycloaliphat.

Besonders bevorzugt steht R₃ für -Vinyl, -Ethyl, -Allyl, -Propyl, -Butyl, -Pentyl, -Hexyl, -Heptyl, -Cyclopentyl, -Cyclohexyl, -Phenyl, -Benzyl, -Naphthyl, -Anthracenyl, -Thiophenyl (-Thienyl), -Benzothiophenyl, -Furyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -C₁₋₃-Aliphat-Gruppe gebundenes -C₅₋₆-Cycloaliphat, -Phenyl, -Naphthyl, -Anthracenyl, -Thiophenyl, -Benzothiophenyl, -Pyridyl, -Furyl, -Benzofuranyl, -Benzodioxolanyl, -Indolyl, -Indanyl, -Benzodioxanyl, -Pyrrolyl, -Pyrimidyl, -Triazolyl oder -Pyrazinyl, jeweils unsubstituiert oder ein- oder mehrfach substituiert.

Noch bevorzugter steht R₃ für -Propyl, -Butyl, -Pentyl, -Hexyl, -Phenyl, -Phenethyl, -Thiophenyl (-Thienyl), -Pyridyl, -Triazolyl, -Benzothiophenyl oder -Benzyl, jeweils substituiert oder unsubstituiert, besonders bevorzugt für -Propyl, -3-Methoxypropyl, -Butyl, -Pentyl, -Hexyl, -Phenyl, -3-Methylphenyl, -3-Fluorphenyl, -Benzo[1,3]-dioxolyl, -Thienyl, -5-Methyl-thiophen-2-yl, -Benzothiophenyl, -4-Chlorbenzyl, -Benzyl, -3-Chlorbenzyl, -4-Methylbenzyl, -2-Chlorbenzyl, -4-Fluorbenzyl, -3-Methylbenzyl, -2-Methylbenzyl, -3-Fluorbenzyl, -2-Fluorbenzyl, -1-Methyl-1,2,4-triazolyl oder-Phenethyl.

Am bevorzugtesten steht R₃ für -Phenyl, -Benzyl, -Phenethyl, jeweils unsubstituiert oder am Ring ein- oder mehrfach substituiert; -C₁₋₅-Aliphat, -C₄₋₆-Cycloaliphat, -Pyridyl, -Thienyl,-Thiazolyl, -Imidazolyl, -1,2,4 Triazolyl oder -Benzimidazolyl, unsubstituiert oder ein- oder mehrfach substituiert.

Insbesondere bevorzugt steht R₃ für -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂, -NO₂, -SH, -CF₃, -OH, -OCH₃, -OC₂H₅ oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl, jeweils unsubstituiert oder ein- oder mehrfach substituiert mit -OH, -OCH₃ oder -OC₂H₅.

Insbesondere bevorzugt steht R₃ für -Phenyl oder -Thienyl, jeweils unsubstituiert oder einfach substituiert mit -F, -Cl, -CH₃; -Ethyl, -n-Propyl, n-Butyl, -Vinyl, oder -Allyl, unsubstituiert oder ein- oder mehrfach substituiert mit -OCH₃, -OH oder -OC₂H₅, insbesondere mit -OCH₃ oder -OC₂H₅.

In weiteren bevorzugten Ausführungsformen steht R3 für einen Rest ausgesucht aus der Gruppe bestehend aus Phenyl, Benzyl und 2-Thienyl, jeweils unsubstituiert oder ein oder mehrfach substituiert mit Substituenten unabhängig voneinander ausgesucht aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂.

Bevorzugt steht R₄ für H oder -Z-R₁₁ steht,
wobei
Z abwesend oder -C(=O)-, sein kann, und
R₁₁ für -C₁₋₆-Alkyl, -C₃₋₆-Cycloalkyl, oder -C₁₋₃-Alkyl-C₃₋₆-Cycloalkyl steht, wobei in der C₃₋₆-Cycyloalkylgruppe ein Ringkohlenstoffatom durch ein Sauerstoffatom ersetzt sein kann und -C₁₋₆-Alkyl, -C₃₋₆-Cycloalkyl oder -C₁₋₃-Alkyl-C₃₋₆-Cycloalkyl unsubstituiert, einfach oder mehrfach substituiert sein können, mit Substituenten unabhängig voneinander ausgesucht aus der Gruppe bestehend aus -F, -Cl, -Br, -I, -CN, -OH,-SH, -O-C₁₋₃-Alkyl, und -S-C₁₋₃-Alkyl, wobei -C₁₋₃-Alkyl substituiert sein kann mit einem oder mehreren Substituenten unabhängig voneinander ausgesucht aus der Gruppe bestehend aus -F, -Cl, -Br, -I, -CN, -OH, -OCH₃, -SH und -SCH₃.

Vorzugsweise sind in dem Rest R₁₁ die -C₃₋₆-Cycloalkylgruppen sowie deren sauerstoffhaltigen Derivate ausgesucht aus der Gruppe bestehend aus Cyclopropyl, Cyclobutyl, Cyclopentyl, Cyclohexyl, Oxetanyl, Oxolanyl (Tetrahydrofuranyl) und Oxanyl (Tetrahydropyranyl).

In weiteren bevorzugten Ausführungsformen steht R₄ für H, CH₃, Ethyl, n-Propyl, i-Propyl, n-Butyl, s-Butyl, iso-Butyl, t-Butyl, n-Pentyl, s-Pentyl, iso-Pentyl, 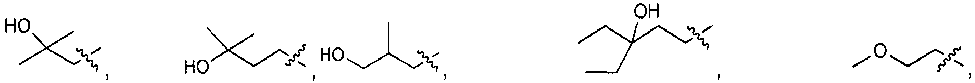 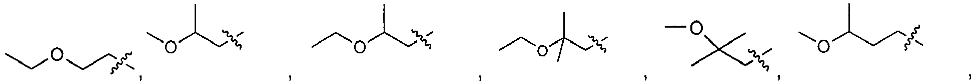 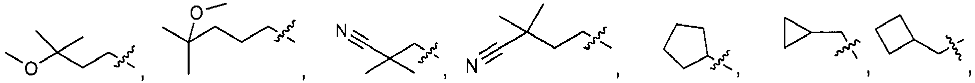 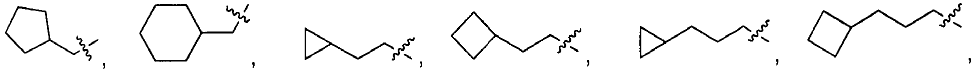 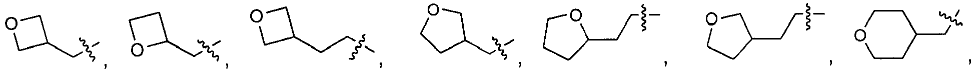 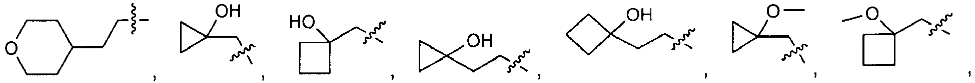 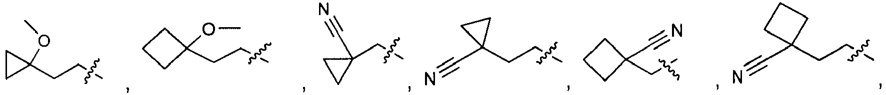 Acetyl,

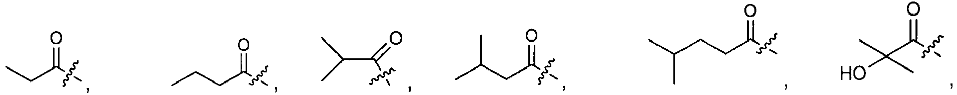

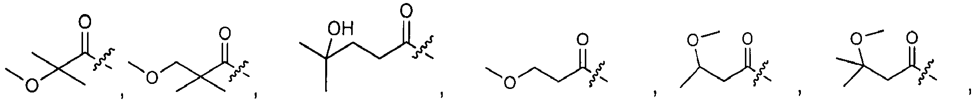

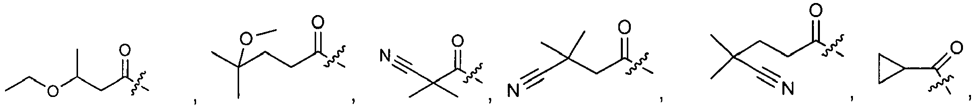

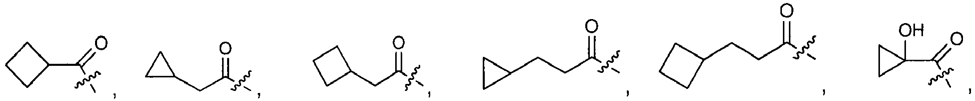

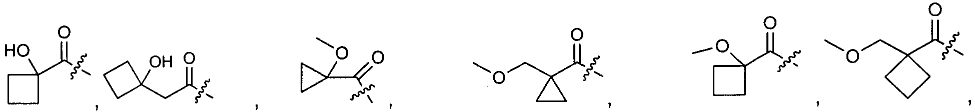

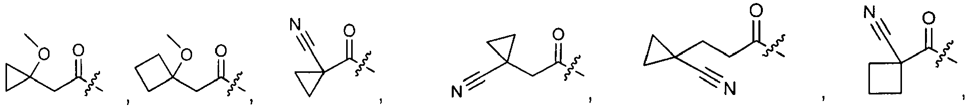

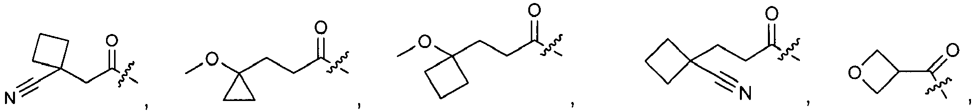

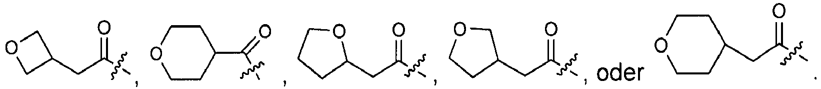

Bevorzugt steht R₅ für -H, -C₁₋₅-Aliphat, -C₃₋₈-Cycloaliphat, -Aryl, oder -Heteroaryl; oder für ein jeweils über eine -C₁₋₃-Aliphat-Gruppe gebundenes -Aryl, -C₃₋₈-Cycloaliphat oder -Heteroaryl.

Bevorzugt stehen R₆ und R₇ unabhängig voneinander für -H, -C₁₋₅-Aliphat, -C₃₋₈-Cycloaliphat, -Aryl, oder -Heteroaryl, oder für ein jeweils über eine -C₁₋₃-Aliphat-Gruppe gebundenes -Aryl, -C₃₋₈-Cycloaliphat oder -Heteroaryl; oder R₆ und R₇ bilden zusammen -CH₂CH₂OCH₂CH₂-, -CH₂CH₂N-R₁₀CH₂CH₂- oder -(CH₂)₃₋₆-. Besonders bevorzugt stehen R₆ und R₇ unabhängig voneinander für -H, -C₁₋₅-Aliphat; oder R₆ und R₇ bilden zusammen -CH₂CH₂OCH₂CH₂-, -CH₂CH₂N-R₁₀CH₂CH₂- oder -(CH₂)₃₋₆-.

Bevorzugt steht R₈ für -H, -C₁₋₅-Aliphat, -C₃₋₈-Ccycloaliphat, -Aryl, oder -Heteroaryl, -C₁₋₆-Aliphat-Aryl, -C₁₋₆-Aliphat-C₃₋₈-Cycloaliphat, -C₁₋₆-Aliphat-Heteroaryl, -C(=O)Aryl, -C(=O)Heteroaryl, oder -C(=O)-C₁₋₆-Aliphat.

Besonders bevorzugt steht R₁₀ für -H oder -C₁₋₅-Aliphat.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl). Anders ausgedrückt wird der Begriff "Cycloaliphat" hier so verstanden, dass darunter sowohl Cycloalkyle und Heterocycloalkyle sowie ungesättigte - aber nicht aromatische - Derivate davon darunterfallen. Der Begriff "C₃₋₈-Cycloaliphat" umfasst somit u.a. sowohl 3- bis 8-gliedrige Cycloalkyle, wie z.B. Cyclopropyl, Cyclobutyl, Cyclopentyl und Cyclohexyl als auch 3- bis 8-gliedrige nicht aromatische Heterocyclen in denen eine oder mehrere Kohlenstoffatome bzw. -(CH₂)- Gruppen durch ein Heteroatom ausgetauscht sind, (z.B. Tetrahydrofuranyl, Tetrahydropyranyl, Piperidyl oder Piperazyl etc.)

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen-.

Bevorzugt ist Aliphat jeweils ein verzweigter oder unverzweigter, gesättigter oder ein ein- oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -NO₂, -CHO, =O, -R₀, -C(=O)R₀, -C(=O)-OH, -C(=O)OR₀, -C(=O)NH₂ -C(=O)NHR₀, -C(=O)N(R₀)₂, -OH, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -NHS(=O)₁₋₂R₀, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C≡C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂-CH₂CH₃ und -CH₂CH₂-CH₂CH₂CH₂CH₃; aber auch -CH=CH₂, -C=CH, -CH₂CH=CH₂, -CH=CHCH₃, -CH₂C≡CH, -C≡CCH₃ und -CH=CHCH=CH₂. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-, -CH₂CH(CH₃)-CH₂-, -CH₂CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡CCH₂-. Bevorzugte substituierte einbindige Aliphaten umfassen -CH₂F, -CHF₂, -CF₃, -CH₂CF₃, -CF₂CF₃, -CH₂OH, -CH₂CH₂OH, -CH₂CHOHCH₃, -CH₂OCH₃ und CH₂CH₂OCH₃. Bevorzugte substituierte zweibindige Aliphaten umfassen -CF₂-, -CF₂CF₂-, -CH₂CHOH-, -CHOHCH₂- und -CH₂CHOHCH₂-.

Besonders bevorzugte Aliphaten sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -NHS(=O)₁₋₂R₀, -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl. Besonders bevorzugte C₃₋₈-Cycloaliphaten sind Cyclopentyl und Cyclohexyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, -OC₁₋₆-Alkyl, -OC(=O)C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH verstanden. Bevorzugt sind Verbindungen, wobei "Aliphat substituiert" oder "Cycloaliphat substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂-OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder -OC₂H₅.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)-NHR₀, -OC(=O)N(R₀)₂, -SH, -SR₀, -SO₃H -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -Si(R₀)₃, -PO(OR₀)₂. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, -NO₂, -CHO, =O -R₀, -C(=O)R₀, -C(=O)OH, -C(=O)OR₀, -C(=O)-NH₂, -C(=O)NHR₀, -C(=O)N(R₀)₂, -OH, -O(CH₂₎₁₋₂O-, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NH-C(=O)R₀, -NHC(=O)OR₀, -NHC(=O)NH₂, -NHC(=O)NHR₀, -NHC(=O)N(R₀)₂, -Si(R₀)₃, -PO(OR₀)₂; wobei ggf. vorhandene N-Ringatome jeweils oxidiert sein können (N-Oxid).

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die Substituenten von Aryl und Heteroaryl jeweils unabhängig voneinander ausgewählt aus -F, -Cl, -Br, -I, -CN, -CHO, -CO₂H, -NH₂, -NO₂, -NHR₀, -N(R₀)₂, -N⁺(R₀)₃, -N⁺(R₀)₂O⁻, -SH, -SR₀, -OH, -OR₀, -C(=O)R₀, -CO₂R₀, -C(=O)NH₂, -C(=O)NHR₀, -C(=O)N(R₀)₂, -S(=O)₁₋₂R₀, -S(=O)₂NH₂, -SO₃H, =O oder -R₀. Bevorzugte Substituenten sind -F, -Cl, -Br, -I, -OH, -OC₁₋₆-Alkyl, -O-C(=O)-C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH. Bevorzugt sind Verbindungen, wobei "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -CH₃, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Bei den erfindungsgemäßen Verbindungen handelt es sich in Bezug auf das spiro-Ringsystem um Isomere, bei denen das Substitutionsmuster am spiro-Cyclohexanringsystem auch mit cis/trans, Z/E oder syn/anti bezeichnet werden kann. "Cis-trans-Isomere" sind eine Untergruppe der Stereoisomere (Konfigurationsisomere).

Die Cis-trans-Isomere der erfindungsgemäßen Verbindung der allgemeinen Formel (1) haben die allgemeinen Formel (1a) bzw. (1b): 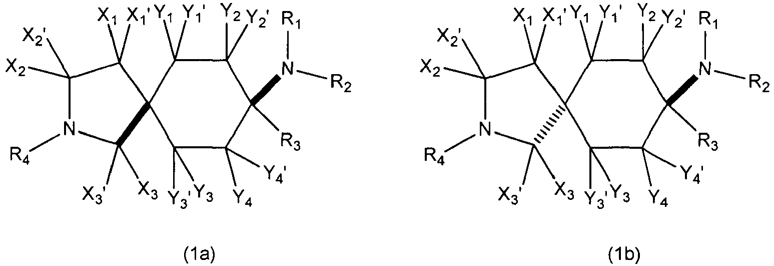 Die Zuordnung der beiden Stereoisomere (1a) und (1b) in Abhängigkeit vom Substitutionsmuster als cis- bzw. trans-Isomer ist dem Fachmann bekannt.

In einer bevorzugten Ausführungsform beträgt der Diastereomerenüberschuss des cis-Isomers wenigstens 50 %de, bevorzugter wenigstens 75 %de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des trans-Isomers wenigstens 50 %de, bevorzugter wenigstens 75 %de, noch bevorzugter wenigstens 90%de, am bevorzugtesten wenigstens 95%de und insbesondere wenigstens 99%de.

Geeignete Methoden zur Trennung der Isomere (Diastereomere) sind dem Fachmann bekannt. Als Beispiele können Säulenchromatographie, präparative HPLC und Kristallisationsverfahren genannt werden.

Ein Fachmann erkennt außerdem, dass die erfindungsgemäßen Verbindungen je nach Substitutionsmuster chiral oder achiral sein können.

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Die erfindungsgemäßen Verbindungen sind durch Substituenten definiert, beispielsweise durch R₁, R₂ und R₃ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise Y₁ = -R₀ wobei R₀ = -C₁₋₈-Aliphat (Substituent der 1. Generation), so kann -C₁₋₈-Aliphat seinerseits substituiert sein, z.B. mit -OR₀ wobei R₀ = -Aryl (Substituent der 2. Generation). Es ergibt sich daraus die funktionelle Gruppe -C₁₋₈-Aliphat-OAryl. -Aryl kann dann seinerseits erneut substituiert sein, z.B. mit -Cl (Substituent der 3. Generation). Es ergibt sich daraus dann insgesamt die funktionelle Gruppe -C₁₋₈-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für R₀ bis R₁₀ jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für R₀ bis R₁₀ jeweils nicht substituiert sein.

Bevorzugt sind Verbindungen, wobei "Aliphat substituiert" oder "Cycloaliphat substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet; und "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet, in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Ganz besonders bevorzugt sind Verbindungen gemäß der allgemeinen Formel (3),

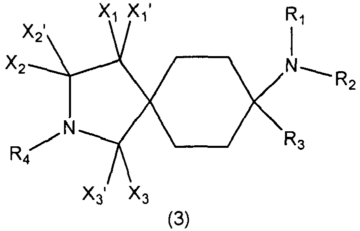

| **Beispiel** | **R₁** | **R₂** | **R₃** | **X₂/X₂'** | **X₃/X₃'** | **R₄** |
|---|---|---|---|---|---|---|
| **1; 2** | CH₃ | CH₃ | Benzyl | H/H | =O | H |
| **3; 4** | CH₃ | CH₃ | Benzyl | H/H | H/H | H |
| **5; 6** | CH₃ | CH₃ | Benzyl | H/H | H/H | CH₃ |
| **7; 8** | CH₃ | CH₃ | Benzyl | H/H | H/H | Acetyl |
| **9** | CH₃ | CH₃ | Benzyl | H/H | H/H | n-Butyl |
| **10; 11** | CH₃ | CH₃ | Benzyl | H/H | H/H | 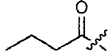 |
| **12** | CH₃ | CH₃ | Benzyl | H/H | H/H | 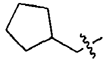 |
| **13** | CH₃ | CH₃ | Phenyl | H/H | =O | H |
| **14; 15** | CH₃ | CH₃ | Phenyl | =O | H/H | H |
| **16** | CH₃ | CH₃ | n-Butyl | H/H | =O | H |
| **17** | CH₃ | CH₃ | 2-Thienyl | H/H | =O | H |
| **18; 25** | CH₃ | CH₃ | 2-Thienyl | H/H | H/H | H |
| **19** | CH₃ | CH₃ | 2-Thienyl | H/H | H/H | CH₃ |
| **20a/b** | CH₃ | CH₃ | 2-Thienyl | H/H | H/H | 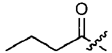 |
| **21** | CH₃ | CH₃ | 2-Thienyl | H/H | H/H | n-Butyl |
| **22** | CH₃ | CH₃ | 2-Thienyl | H/H | H/H | 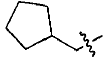 |
| **24a/b** | CH₃ | CH₃ | 2-Thienyl | =O | H/H | H |
| **27** | CH₃ | CH₃ | Benzyl | H/H | H/H | n-Butyl |
| **28; 29** | CH₃ | CH₃ | Phenyl | H/H | H/H | 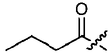 |
| **30** | CH₃ | CH₃ | 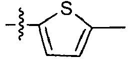 | H/H | =O | H |
| **31** | CH₃ | CH₃ | 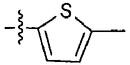 | H/H | H/H | H |
| **32** | CH₃ | CH₃ | 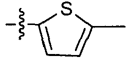 | H/H | H/H | 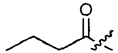 |
| **33** | CH₃ | CH₃ | 2-Thienyl | =O | H/H | n-Butyl |
| **34** | CH₃ | CH₃ | 2-Thienyl | =O | H/H | CH₃ |
| **35** | CH₃ | CH₃ | 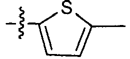 | H/H | H/H | n-Butyl |
| **36** | CH₃ | CH₃ | 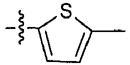 | H/H | H/H | 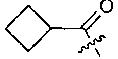 |
| **37** | CH₃ | CH₃ | 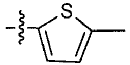 | H/H | H/H | 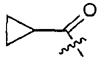 |
| **38** | CH₃ | CH₃ | 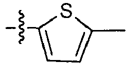 | H/H | H/H | 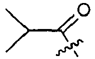 |
| **39** | CH₃ | CH₃ | 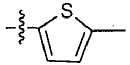 | H/H | H/H | 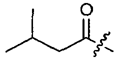 |
| **40** | CH₃ | CH₃ | 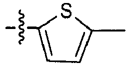 | H/H | H/H | 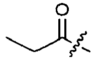 |
| **41** | CH₃ | CH₃ | 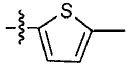 | H/H | H/H | 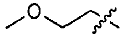 |
| **42** | CH₃ | CH₃ | 2-Thienyl | H/H | H/H | 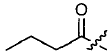 |
| **43** | CH₃ | CH₃ | 2-Thienyl | H/H | H/H | 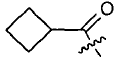 |
| **44** | CH₃ | CH₃ | 2-Thienyl | H/H | H/H | 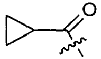 |
| **45** | CH₃ | CH₃ | 2-Thienyl | H/H | H/H | 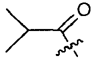 |
| **46** | CH₃ | CH₃ | 2-Thienyl | H/H | H/H | 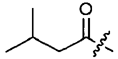 |
| **47** | CH₃ | CH₃ | 2-Thienyl | H/H | H/H | 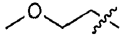 |
| **48** | CH₃ | CH₃ | 2-Thienyl | H/H | H/H | 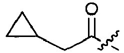 |
| **49** | CH₃ | CH₃ | 2-Thienyl | H/H | H/H | 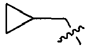 |
| **50** | CH₃ | CH₃ | 2-Thienyl | H/H | H/H | 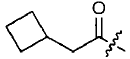 |
| **51** | CH₃ | CH₃ | 2-Thienyl | H/H | H/H | 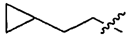 |
| **52** | CH₃ | CH₃ | 2-Thienyl | H/H | H/H | 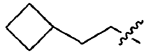 |
| **53** | CH₃ | CH₃ | 2-Thienyl | H/H | H/H | 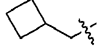 |
| **54** | CH₃ | CH₃ | 2-Thienyl | H/H | H/H | 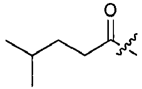 |
| **55** | CH₃ | CH₃ | 2-Thienyl | H/H | H/H | 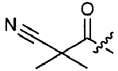 |
| **56** | CH₃ | CH₃ | 2-Thienyl | H/H | H/H | 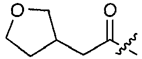 |
| **57** | CH₃ | CH₃ | 2-Thienyl | H/H | H/H | 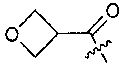 |
| **58** | CH₃ | CH₃ | 2-Thienyl | H/H | H/H | 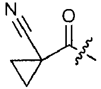 |
| **59** | CH₃ | CH₃ | 2-Thienyl | H/H | H/H | 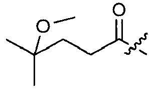 |
| **60** | CH₃ | CH₃ | 2-Thienyl | H/H | H/H | 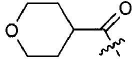 |
| **61** | CH₃ | CH₃ | 2-Thienyl | H/H | H/H | 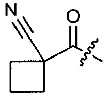 |
| **62** | CH₃ | CH₃ | 2-Thienyl | H/H | H/H | 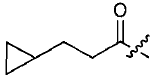 |
| **63** | CH₃ | CH₃ | 2-Thienyl | H/H | H/H | 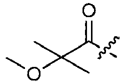 |
| **64** | CH₃ | CH₃ | 2-Thienyl | H/H | H/H | 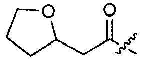 |
| **65** | CH₃ | CH₃ | 2-Thienyl | H/H | H/H | 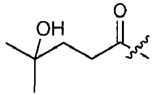 |
| **66** | CH₃ | CH₃ | 2-Thienyl | H/H | H/H | 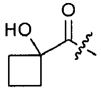 |
| **67** | CH₃ | CH₃ | 2-Thienyl | H/H | H/H | 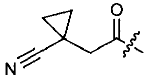 |
| **68** | CH₃ | CH₃ | 2-Thienyl | H/H | H/H | 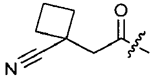 |
| **69** | CH₃ | CH₃ | 2-Thienyl | H/H | H/H | 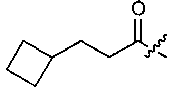 |
| **70** | CH₃ | CH₃ | 2-Thienyl | H/H | H/H | 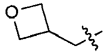 |
| **71** | CH₃ | CH₃ | Phenyl | H/H | H/H | H |
| **72** | CH₃ | CH₃ | Phenyl | H/H | H/H | 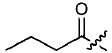 |
| **73** | CH₃ | CH₃ | Phenyl | H/H | H/H | Acetyl |
| **74** | CH₃ | CH₃ | Phenyl | H/H | H/H | n-Butly |
| **75** | CH₃ | CH₃ | Phenyl | H/H | H/H | 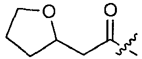 |
| **76** | CH₃ | CH₃ | Phenyl | H/H | H/H | 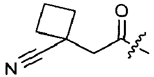 |
| **77** | CH₃ | CH₃ | Phenyl | H/H | H/H | 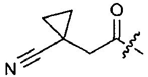 |
| **78** | CH₃ | CH₃ | Phenyl | H/H | H/H | 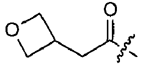 |
| **79** | CH₃ | CH₃ | Phenyl | H/H | H/H | 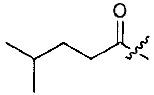 |
| **80** | CH₃ | CH₃ | Phenyl | H/H | H/H | 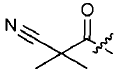 |
| **81** | CH₃ | CH₃ | Phenyl | H/H | H/H | 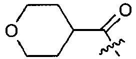 |
| **82** | CH₃ | CH₃ | Phenyl | H/H | H/H | 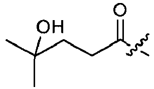 |
| **83** | CH₃ | CH₃ | Phenyl | H/H | H/H | 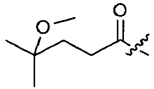 |
| **84** | CH₃ | CH₃ | Phenyl | H/H | H/H | 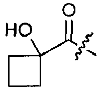 |
| **85** | CH₃ | CH₃ | Phenyl | H/H | H/H | 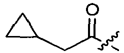 |
| **86** | CH₃ | CH₃ | Phenyl | H/H | H/H | 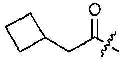 |
| **87** | CH₃ | CH₃ | Phenyl | H/H | H/H | 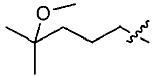 |
| **88** | CH₃ | CH₃ | 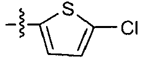 | H/H | H/H | 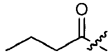 |
| **89** | CH₃ | CH₃ | 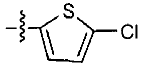 | H/H | H/H | n-Butyl |
| **90** | CH₃ | CH₃ | 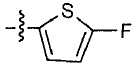 | H/H | H/H | 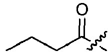 |
| **91** | CH₃ | CH₃ | 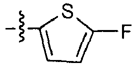 | H/H | H/H | n-Butyl |
| **92** | CH₃ | CH₃ | 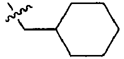 | H/H | H/H | H |
| **93** | CH₃ | CH₃ | 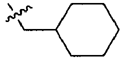 | H/H | H/H | 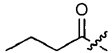 |
| **94** | CH₃ | CH₃ | 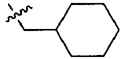 | H/H | H/H | n-Butyl |
| **95** | CH₃ | CH₃ | 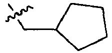 | H/H | H/H | 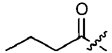 |
| **96** | CH₃ | CH₃ | 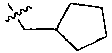 | H/H | H/H | n-Butyl |
| **97** | CH₃ | CH₃ | 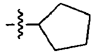 | H/H | H/H | 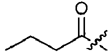 |
| **98** | CH₃ | CH₃ | 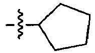 | H/H | H/H | n-Butyl |
| **99** | CH₃ | CH₃ | 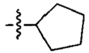 | H/H | H/H | 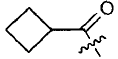 |
| **100** | CH₃ | CH₃ | 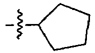 | H/H | H/H | 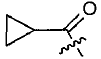 |
| **101** | CH₃ | CH₃ | 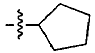 | H/H | H/H | 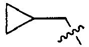 |
| **102** | CH₃ | CH₃ | 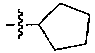 | H/H | H/H | 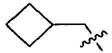 |
| **103** | CH₃ | CH₃ | 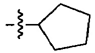 | H/H | H/H | 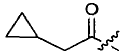 |
| **104** | CH₃ | CH₃ | 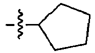 | H/H | H/H | 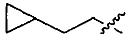 |
| **105** | CH₃ | CH₃ | 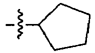 | H/H | H/H | 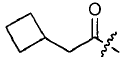 |
| **106; 107** | CH₃ | CH₃ | 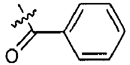 | H/H | H/H | n-Butyl |
| **108; 109** | -CH₂CH₂CH₂- | | 2-Thienyl | H/H | H/H | 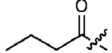 |
| **110; 111** | -CH₂CH₂CH₂- | | Phenyl | H/H | H/H | 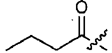 |
| **112; 113** | -CH₂CH₂CH₂- | | Phenyl | H/H | H/H | n-Butyl |
| **114** | CH₃ | CH₃ | 2-Thienyl | H/H | =O | CH₃ |
| **115** | CH₃ | CH₃ | 2-Thienyl | H/H | =O | n-Butyl |
| **116** | CH₃ | CH₃ | 2-Thienyl | H/H | =O | 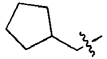 |
| **117** | CH₃ | CH₃ | 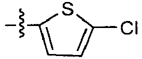 | H/H | =O | H |
| **118; 119** | CH₃ | CH₃ | Benzyl | H/H | =O | CH₃ |
| **120; 121** | CH₃ | CH₃ | Benzyl | H/H | =O | n-Butyl |
| **122; 123** | CH₃ | CH₃ | Benzyl | H/H | =O | 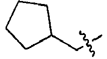 |
| **124** | CH₃ | CH₃ | 2-Thienyl | =O | H/H | CH₃ |
| **125** | CH₃ | CH₃ | 2-Thienyl | =O | H/H | n-Butyl |
| **126; 127** | CH₃ | CH₃ | 2-Thienyl | =O | H/H | 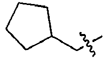 |
| **128; 129** | CH₃ | CH₃ | 2-Thienyl | =O | H/H | 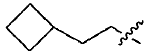 |
| **130; 131** | CH₃ | CH₃ | 2-Thienyl | =O | H/H | 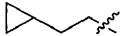 |
| **132; 133** | CH₃ | CH₃ | 2-Thienyl | =O | H/H | 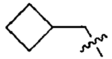 |
| **134; 135** | CH₃ | CH₃ | 2-Thienyl | =O | H/H | 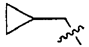 |
| **136; 137** | CH₃ | CH₃ | 2-Thienyl | =O | H/H | 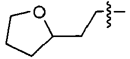 |
| **138; 139** | CH₃ | CH₃ | 2-Thienyl | =O | H/H | 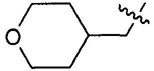 |
| **140; 141** | CH₃ | CH₃ | 2-Thienyl | =O | H/H | 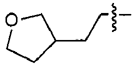 |
| **142; 143** | CH₃ | CH₃ | 2-Thienyl | =O | H/H | 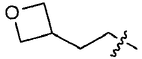 |
| **144; 145** | CH₃ | CH₃ | 2-Thienyl | =O | H/H | 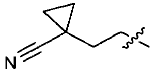 |
| **146; 147** | CH₃ | CH₃ | 2-Thienyl | =O | H/H | 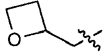 |
| **148; 149** | CH₃ | CH₃ | 2-Thienyl | =O | H/H | 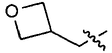 |
| **150; 151** | CH₃ | CH₃ | 2-Thienyl | =O | H/H | 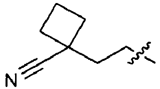 |
| **152; 153** | CH₃ | CH₃ | 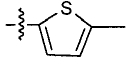 | =O | H/H | H |
| **154; 155** | CH₃ | CH₃ | 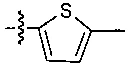 | =O | H/H | CH₃ |
| **156; 157** | CH₃ | CH₃ | 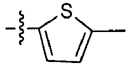 | =O | H/H | n-Butyl |
| **158; 159** | CH₃ | CH₃ | 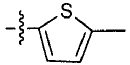 | =O | H/H | 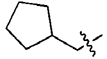 |
| **160** | CH₃ | CH₃ | 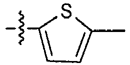 | =O | H/H | 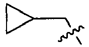 |
| **161** | CH₃ | CH₃ | 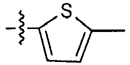 | =O | H/H | 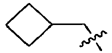 |
| **162** | CH₃ | CH₃ | Phenyl | =O | H/H | CH₃ |
| **163; 164** | CH₃ | CH₃ | Phenyl | =O | H/H | n-Butyl |
| **165; 166** | CH₃ | CH₃ | Phenyl | =O | H/H | 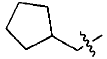 |
| **167; 168** | CH₃ | CH₃ | Phenyl | =O | H/H | 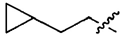 |
| **169; 170** | CH₃ | CH₃ | Phenyl | =O | H/H | 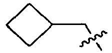 |
| **171; 172** | CH₃ | CH₃ | Phenyl | =O | H/H | 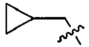 |
| **173; 174** | CH₃ | CH₃ | Phenyl | =O | H/H | 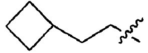 |
| **175; 176** | CH₃ | CH₃ | Phenyl | =O | H/H | 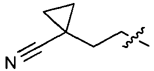 |
| **177; 178** | CH₃ | CH₃ | Phenyl | =O | H/H | 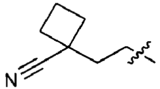 |
| **179; 180** | CH₃ | CH₃ | Phenyl | =O | H/H | 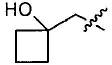 |
| **181** | CH₃ | CH₃ | Phenyl | =O | H/H | 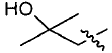 |
| **182; 183** | CH₃ | CH₃ | Phenyl | =O | H/H | 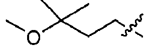 |
| **184; 185** | CH₃ | CH₃ | Phenyl | =O | H/H | 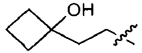 |
| **186; 187** | CH₃ | CH₃ | Phenyl | =O | H/H | 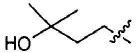 |
| **188** | CH₃ | CH₃ | Phenyl | H/H | H/H | 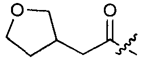 |
| **189** | CH₃ | CH₃ | Phenyl | H/H | H/H | 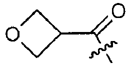 |
| **190** | CH₃ | CH₃ | Phenyl | H/H | H/H | 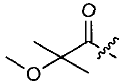 |
| **191** | CH₃ | CH₃ | Phenyl | H/H | H/H | 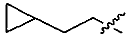 |
| **192** | CH₃ | CH₃ | Phenyl | H/H | H/H | 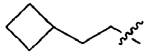 |
| **193** | CH₃ | CH₃ | Phenyl | H/H | H/H | 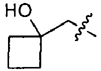 |
| **194** | CH₃ | CH₃ | Phenyl | H/H | H/H | 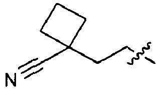 |
| **195** | CH₃ | CH₃ | Phenyl | H/H | H/H | 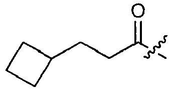 |
| **196** | CH₃ | CH₃ | Phenyl | H/H | H/H | 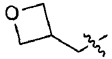 |
| **197** | CH₃ | CH₃ | Phenyl | H/H | H/H | 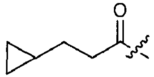 |
| **198** | CH₃ | CH₃ | Phenyl | H/H | H/H | 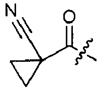 |
| **199** | CH₃ | CH₃ | Phenyl | H/H | H/H | 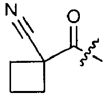 |
| **200** | CH₃ | CH₃ | Phenyl | H/H | H/H | 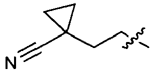 |
| **201** | CH₃ | CH₃ | Phenyl | H/H | H/H | 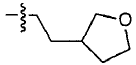 |
| **202** | CH₃ | CH₃ | Phenyl | H/H | H/H | 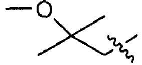 |
| **203** | CH₃ | CH₃ | Phenyl | H/H | H/H | 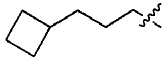 |
| **204** | CH₃ | CH₃ | Phenyl | H/H | H/H | 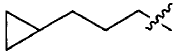 |
| **205** | CH₃ | CH₃ | Phenyl | H/H | H/H | 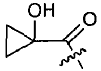 |
| **206** | CH₃ | CH₃ | Phenyl | H/H | H/H | 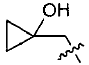 |
| **207** | CH₃ | CH₃ | 2-Thienyl | H/H | H/H | 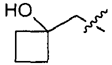 |
| **208** | CH₃ | CH₃ | 2-Thienyl | H/H | H/H | 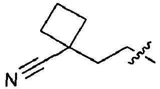 |
| **209** | CH₃ | CH₃ | 2-Thienyl | H/H | H/H | 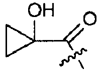 |
| **210** | CH₃ | CH₃ | 2-Thienyl | H/H | H/H | 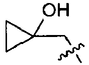 |
| **211** | CH₃ | CH₃ | 2-Thienyl | H/H | H/H | 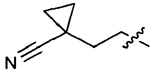 |
| **212** | CH₃ | CH₃ | 2-Thienyl | H/H | H/H | 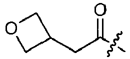 |
| **213** | CH₃ | CH₃ | 2-Thienyl | H/H | H/H | 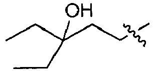 |
| **214** | CH₃ | CH₃ | 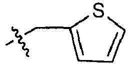 | H/H | H/H | 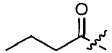 |
| **215; 216** | CH₃ | CH₃ | 2-Thienyl | =O | H/H | 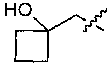 |
| **217; 218** | CH₃ | CH₃ | 2-Thienyl | =O | H/H | 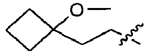 |
| **219; 220** | CH₃ | CH₃ | 2-Thienyl | =O | H/H | 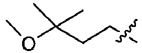 |
| **221; 222** | CH₃ | CH₃ | 2-Thienyl | =O | H/H | 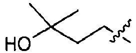 |
| **223** | CH₃ | CH₃ | 2-Thienyl | H/H | H/H | 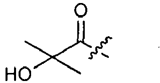 |
| **224; 225** | CH₃ | CH₃ | 2-Thienyl | =O | H/H | 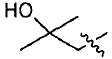 |
| **226** | CH₃ | CH₃ | 2-Thienyl | H/H | H/H | 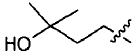 |
| **227** | CH₃ | CH₃ | 2-Thienyl | H/H | H/H | 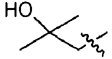 |
| **228** | CH₃ | CH₃ | Phenyl | H/H | H/H | 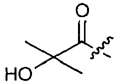 |
| **229** | CH₃ | CH₃ | Phenyl | H/H | H/H | 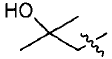 |
| **230; 231** | CH₃ | CH₃ | Phenyl | =O | H/H | 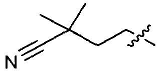 |
| **232** | CH₃ | CH₃ | Phenyl | H/H | H/H | 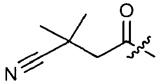 |
| **233** | CH₃ | CH₃ | 2-Thienyl | H/H | H/H | 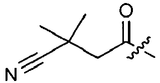 |
| **234** | CH₃ | CH₃ | 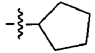 | =O | H/H | 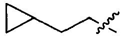 |
| **235** | CH₃ | CH₃ | 2-Thienyl | H/H | H/H | 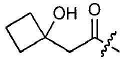 |
| **236** | CH₃ | CH₃ | 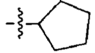 | =O | H/H | 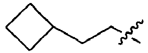 |
| **237** | CH₃ | CH₃ | Phenyl | H/H | H/H | 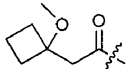 |
| **238** | CH₃ | CH₃ | 2-Thienyl | H/H | H/H | 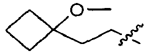 |
| **239** | CH₃ | CH₃ | 2-Thienyl | H/H | H/H | 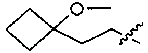 |
| **240** | CH₃ | CH₃ | 2-Thienyl | =O | H/H | 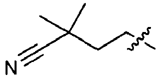 |
| **241; 242** | CH₃ | CH₃ | 2-Thienyl | =O | H/H | 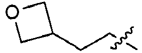 |
| **243** | CH₃ | CH₃ | 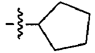 | =O | H/H | 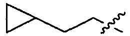 |
| **244** | CH₃ | CH₃ | 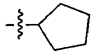 | =O | H/H | 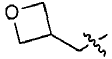 |
| **245** | CH₃ | CH₃ | 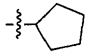 | =O | H/H | 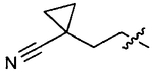 |
| **246** | CH₃ | CH₃ | 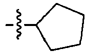 | =O | H/H | 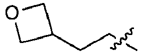 |
| **247** | CH₃ | CH₃ | 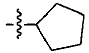 | H/H | H/H | 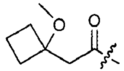 |
| **248** | CH₃ | CH₃ | 2-Thienyl | H/H | H/H | 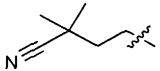 |
| **249** | CH₃ | CH₃ | Phenyl | H/H | H/H | 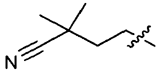 |
| **250** | CH₃ | CH₃ | 2-Thienyl | =O | H/H | 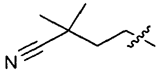 |
| **251; 252** | CH₃ | CH₃ | Phenyl | =O | H/H | 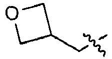 |
| **253** | CH₃ | CH₃ | 2-Thienyl | H/H | H/H | 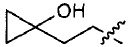 |
| **254; 255** | CH₃ | CH₃ | Phenyl | =O | H/H | 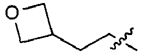 |
| **256** | CH₃ | CH₃ | 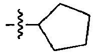 | =O | H/H | 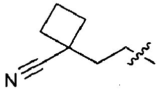 |
| **257** | CH₃ | CH₃ | 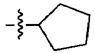 | H/H | H/H | 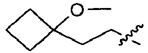 |
| **258** | CH₃ | CH₃ | 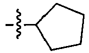 | H/H | H/H | 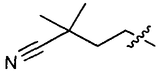 |
| **259** | CH₃ | CH₃ | 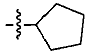 | H/H | H/H | 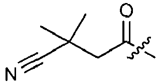 |
| **260; 261** | CH₃ | CH₃ | Phenyl | =O | H/H | 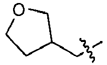 |
| **262; 263** | CH₃ | CH₃ | Phenyl | =O | H/H | 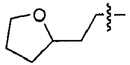 |
| **264** | CH₃ | CH₃ | Phenyl | =O | H/H | 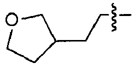 |
| **265** | CH₃ | CH₃ | 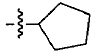 | H/H | H/H | 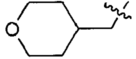 |
| **266** | CH₃ | CH₃ | 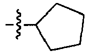 | H/H | H/H | 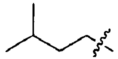 |
| **267** | CH₃ | CH₃ | 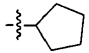 | H/H | H/H | 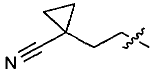 |
| **268** | CH₃ | CH₃ | 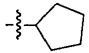 | H/H | H/H | 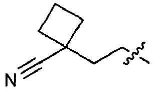 |
| **269; 270** | CH₃ | CH₃ | Phenyl | =O | H/H | 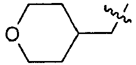 |
| **271** | CH₃ | CH₃ | Phenyl | =O | H/H | 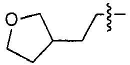 |
| **272** | CH₃ | CH₃ | Phenyl | H/H | H/H | 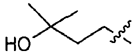 |
| **273** | CH₃ | CH₃ | 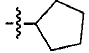 | H/H | H/H | 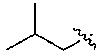 |
| **274** | CH₃ | CH₃ | 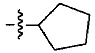 | =O | H/H | 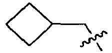 |
| **275** | CH₃ | H | Phenyl | =O | H/H | 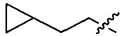 |
| **276** | CH₃ | CH₃ | 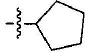 | =O | H/H | 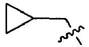 |
| **277** | CH₃ | CH₃ | 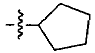 | =O | H/H | 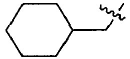 |
| **278** | CH₃ | CH₃ | 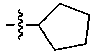 | =O | H/H | 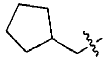 |
| **279; 280** | CH₃ | CH₃ | 2-Thienyl | =O | H/H | 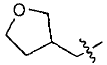 |
| **281** | CH₃ | CH₃ | 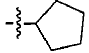 | =O | H/H | n-Butyl |
| **282** | CH₃ | CH₃ | 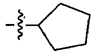 | =O | H/H | CH₃ |
| **283** | CH₃ | CH₃ | Phenyl | H/H | H/H | 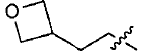 |
| **284** | CH₃ | CH₃ | Phenyl | H/H | H/H | 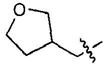 |
| **285** | CH₃ | CH₃ | Phenyl | H/H | H/H | 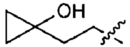 |
| **286** | CH₃ | CH₃ | 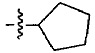 | =O | H/H | 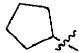 |
| **287** | CH₃ | CH₃ | 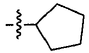 | =O | H/H | 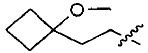 |
| **288; 289** | CH₃ | CH₃ | 2-Thienyl | =O | H/H | 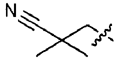 |
| **290** | CH₃ | H | 2-Thienyl | =O | H/H | 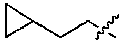 |
| **291** | CH₃ | CH₃ | 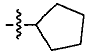 | H/H | H/H | 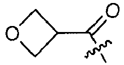 |
| **292** | CH₃ | CH₃ | 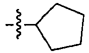 | H/H | H/H | 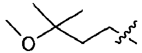 |
| **293; 294** | CH₃ | CH₃ | 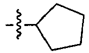 | =O | H/H | 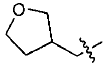 |
| **295** | CH₃ | CH₃ | 2-Thienyl | H/H | H/H | 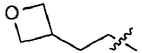 |
| **296** | CH₃ | CH₃ | 2-Thienyl | H/H | H/H | 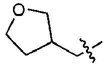 |
| **297** | CH₃ | CH₃ | 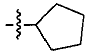 | H/H | H/H | 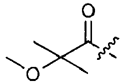 |
| **298** | CH₃ | CH₃ | 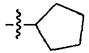 | H/H | H/H | 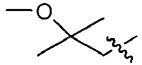 |
| **299** | CH₃ | CH₃ | 2-Thienyl | H/H | H/H | 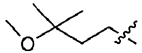 |
| **300** | CH₃ | CH₃ | Phenyl | H/H | H/H | 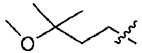 |
| **301** | CH₃ | CH₃ | 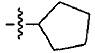 | H/H | H/H | 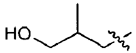 |
| **302** | CH₃ | CH₃ | 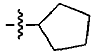 | H/H | H/H | 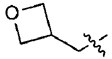 |
| **303** | CH₃ | CH₃ | Phenyl | H/H | H/H | 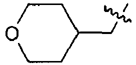 |
| **304** | CH₃ | CH₃ | 2-Thienyl | H/H | H/H | 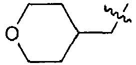 |
| **305** | CH₃ | CH₃ | Phenyl | H/H | H/H | 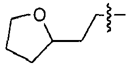 |
| **306** | CH₃ | CH₃ | 2-Thienyl | H/H | H/H | 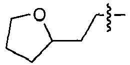 |
| **307; 308** | CH₃ | H | 2-Thienyl | =O | H/H | 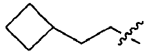 |
| **309** | CH₃ | CH₃ | 2-Thienyl | H/H | H/H | 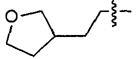 |
| **310** | CH₃ | CH₃ | Phenyl | =O | H/H | 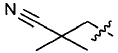 |
| **311** | CH₃ | CH₃ | 2-Thienyl | =O | H/H | 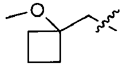 |
| **312** | CH₃ | CH₃ | Phenyl | =O | H/H | 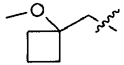 |
| **313** | CH₃ | CH₃ | 2-Thienyl | =O | H/H | 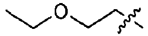 |
| **314** | CH₃ | CH₃ | 2-Thienyl | H/H | H/H | 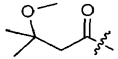 |
| **315** | CH₃ | CH₃ | 2-Thienyl | H/H | H/H | 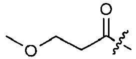 |
| **316** | CH₃ | H | 2-Thienyl | =O | H/H | 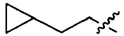 |
| **317** | CH₃ | CH₃ | 2-Thienyl | =O | H/H | 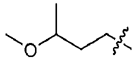 |
| **318** | CH₃ | CH₃ | 2-Thienyl | =O | H/H | 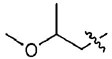 |
| **319** | CH₃ | CH₃ | Phenyl | =O | H/H | 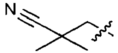 |
| **320** | CH₃ | CH₃ | Phenyl | H/H | H/H | 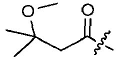 |
| **321** | CH₃ | CH₃ | 2-Thienyl | =O | H/H | 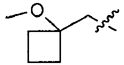 |
| **322** | CH₃ | CH₃ | Phenyl | =O | H/H | 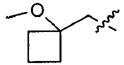 |
| **323** | CH₃ | CH₃ | Phenyl | =O | H/H | 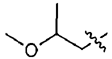 |
| **324** | CH₃ | CH₃ | Phenyl | =O | H/H | 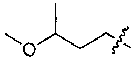 |
| **325** | CH₃ | CH₃ | Phenyl | =O | H/H | 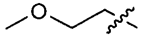 |
| **326** | CH₃ | CH₃ | Phenyl | =O | H/H | 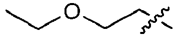 |
| **327** | CH₃ | CH₃ | 2-Thienyl | H/H | H/H | 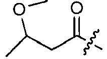 |
| **328** | CH₃ | CH₃ | Phenyl | H/H | H/H | 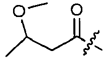 |
| **329** | CH₃ | CH₃ | Phenyl | H/H | H/H | 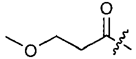 |
| **330** | CH₃ | H | Phenyl | =O | H/H | 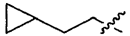 |
| **331** | CH₃ | H | Phenyl | =O | H/H | 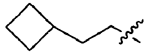 |
| **332** | CH₃ | CH₃ | 2-Thienyl | =O | H/H | 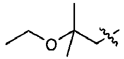 |
| **333** | CH₃ | CH₃ | 2-Thienyl | H/H | H/H | 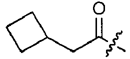 |
| **334** | CH₃ | CH₃ | 2-Thienyl | =O | H/H | 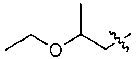 |
| **335** | CH₃ | CH₃ | Phenyl | =O | H/H | 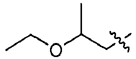 |
| **336** | CH₃ | CH₃ | 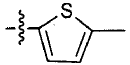 | =O | H/H | 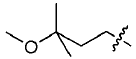 |
| **337** | CH₃ | CH₃ | 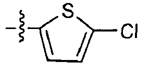 | =O | H/H | 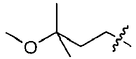 |
| **338** | CH₃ | CH₃ | Phenyl | H/H | H/H | 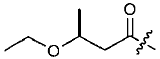 |
| **339** | CH₃ | CH₃ | 2-Thienyl | H/H | H/H | 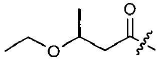 |
| **340** | CH₃ | CH₃ | Phenyl | =O | H/H | 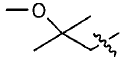 |
| **341** | CH₃ | H | 2-Thienyl | =O | H/H | 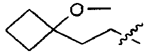 |
| **342** | CH₃ | CH₃ | Phenyl | H/H | H/H | 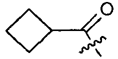 |
| **343** | CH₃ | CH₃ | Phenyl | H/H | H/H | 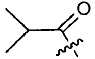 |
| **344** | CH₃ | CH₃ | 2-Thienyl | =O | H/H | 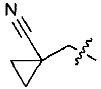 |
| **345** | CH₃ | CH₃ | Phenyl | H/H | H/H | 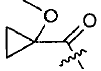 |
| **346** | CH₃ | H | 2-Thienyl | H/H | H/H | 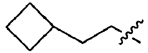 |
| **347** | CH₃ | CH₃ | 2-Thienyl | =O | H/H | 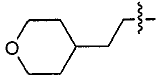 |
| **348** | CH₃ | CH₃ | Phenyl | =O | H/H | 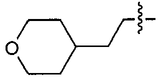 |
| **349** | CH₃ | CH₃ | Phenyl | =O | H/H | 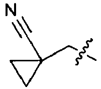 |
| **350** | CH₃ | CH₃ | Phenyl | H/H | H/H | 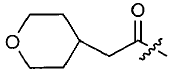 |
| **351** | CH₃ | CH₃ | 2-Thienyl | H/H | H/H | 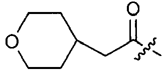 |
| **352** | CH₃ | CH₃ | Phenyl | H/H | H/H | 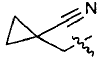 |
| **353** | CH₃ | CH₃ | 2-Thienyl | =O | H/H | 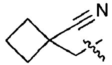 |
| **354** | CH₃ | CH₃ | Phenyl | H/H | H/H | 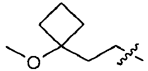 |
| **355** | CH₃ | CH₃ | 2-Thienyl | H/H | H/H | 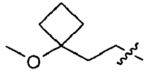 |
| **356** | CH₃ | CH₃ | 2-Thienyl | H/H | H/H | 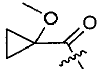 |
| **357** | CH₃ | CH₃ | Phenyl | =O | H/H | 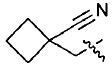 |
| **358** | CH₃ | CH₃ | 2-Thienyl | H/H | H/H | 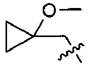 |
| **359** | CH₃ | CH₃ | Phenyl | H/H | H/H | 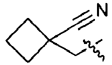 |
| **360** | CH₃ | CH₃ | 2-Thienyl | H/H | H/H | 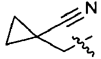 |
| **361** | CH₃ | CH₃ | 2-Thienyl | H/H | H/H | 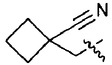 |
| **362** | CH₃ | CH₃ | Phenyl | H/H | H/H | 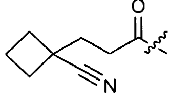 |
| **363** | CH₃ | CH₃ | 2-Thienyl | H/H | H/H | 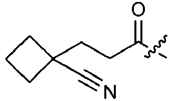 |
| **364** | CH₃ | CH₃ | n-Butyl | H/H | H/H | 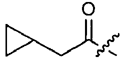 |
| **365** | CH₃ | CH₃ | Phenyl | H/H | H/H | 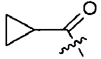 |
| **366** | CH₃ | CH₃ | Phenyl | H/H | H/H | 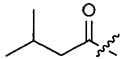 |
| **367** | CH₃ | CH₃ | n-Butyl | H/H | H/H | 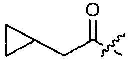 |
| **368** | CH₃ | CH₃ | n-Butyl | H/H | H/H | 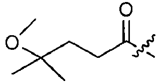 |
| **369** | CH₃ | CH₃ | Phenyl | H/H | H/H | 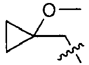 |
| **370** | CH₃ | CH₃ | 2-Thienyl | =O | H/H | 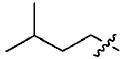 |
| **371** | CH₃ | CH₃ | Phenyl | H/H | H/H | 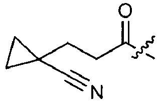 |
| **372** | CH₃ | CH₃ | 2-Thienyl | H/H | H/H | 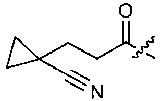 |
| **373** | CH₃ | CH₃ | 2-Thienyl | H/H | H/H | 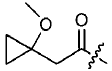 |
| **374** | CH₃ | CH₃ | Phenyl | H/H | H/H | 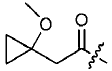 |
| **375** | CH₃ | CH₃ | n-Butyl | H/H | H/H | 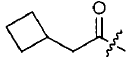 |
| **376** | CH₃ | CH₃ | Phenyl | =O | H/H | 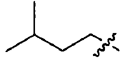 |
| **377** | CH₃ | CH₃ | 2-Thienyl | =O | H/H | 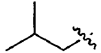 |
| **378** | CH₃ | CH₃ | Phenyl | =O | H/H | 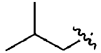 |
| **379** | CH₃ | CH₃ | Phenyl | H/H | H/H | 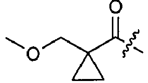 |
| **380** | CH₃ | CH₃ | Phenyl | H/H | H/H | 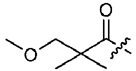 |
| **381** | CH₃ | CH₃ | Phenyl | H/H | H/H | 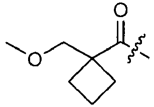 |
| **382** | CH₃ | CH₃ | 2-Thienyl | H/H | H/H | 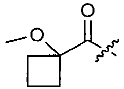 |
| **383** | CH₃ | CH₃ | 2-Thienyl | H/H | H/H | 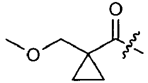 |
| **384** | CH₃ | CH₃ | 2-Thienyl | H/H | H/H | 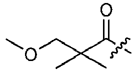 |
| **385** | CH₃ | CH₃ | 2-Thienyl | H/H | H/H | 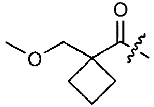 |
| **386** | CH₃ | CH₃ | 2-Thienyl | =O | H/H | 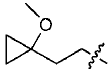 |
| **387** | CH₃ | CH₃ | Phenyl | =O | H/H | 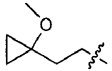 |
| **388** | CH₃ | CH₃ | n-Butyl | H/H | H/H | 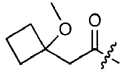 |
| **389** | CH₃ | CH₃ | n-Butyl | H/H | H/H | 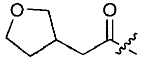 |
| **390** | CH₃ | CH₃ | Phenyl | H/H | H/H | 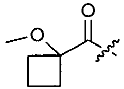 |
| **391** | CH₃ | CH₃ | 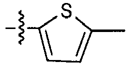 | H/H | H/H | 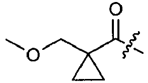 |
| **392** | CH₃ | CH₃ | 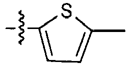 | H/H | H/H | 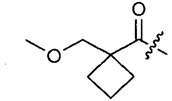 |
| **393** | CH₃ | CH₃ | 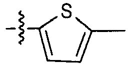 | H/H | H/H | 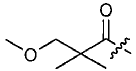 |
| **394** | CH₃ | CH₃ | Phenyl | H/H | H/H | 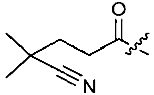 |
| **395** | CH₃ | CH₃ | 2-Thienyl | H/H | H/H | 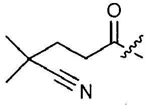 |
| **396** | CH₃ | CH₃ | 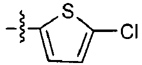 | H/H | H/H | 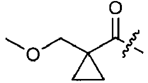 |
| **397** | CH₃ | CH₃ | 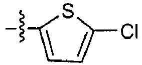 | H/H | H/H | 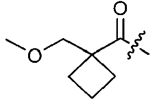 |
| **398** | CH₃ | CH₃ | 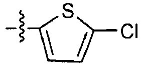 | H/H | H/H | 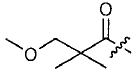 |
| **399; 400** | CH₃ | CH₃ | n-Butyl | =O | H/H | 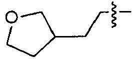 |
| **401; 402** | CH₃ | CH₃ | n-Butyl | =O | H/H | 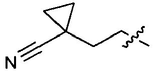 |
| **403; 404** | CH₃ | CH₃ | n-Butyl | =O | H/H | 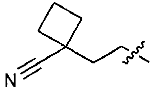 |
| **405** | CH₃ | CH₃ | Phenyl | H/H | H/H | 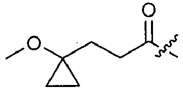 |
| **406** | CH₃ | CH₃ | n-Butyl | =O | H/H | 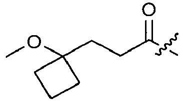 |
| **407** | CH₃ | CH₃ | 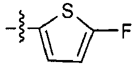 | H/H | H/H | 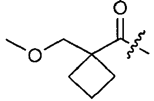 |
| **408** | CH₃ | CH₃ | Phenyl | H/H | H/H | 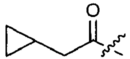 |
| **409; 410** | CH₃ | CH₃ | n-Butyl | =O | H/H | 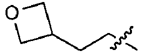 |
| **411** | CH₃ | CH₃ | 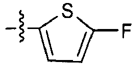 | H/H | H/H | 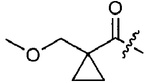 |
| **412** | CH₃ | CH₃ | 2-Thienyl | H/H | H/H | 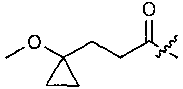 |
| **413** | CH₃ | CH₃ | n-Butyl | =O | H/H | 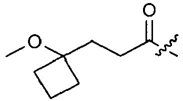 |
| **414** | CH₃ | CH₃ | Phenyl | H/H | H/H | 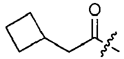 |
| **415** | CH₃ | CH₃ | 2-Thienyl | H/H | H/H | 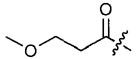 |
| **416** | CH₃ | CH₃ | 2-Thienyl | H/H | H/H | 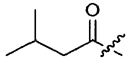 |
| **417** | CH₃ | CH₃ | 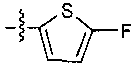 | =O | H/H | 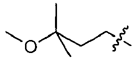 |
| **418** | CH₃ | CH₃ | 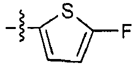 | H/H | H/H | 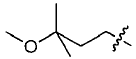 |
| **419** | CH₃ | H | 2-Thienyl | H/H | H/H | 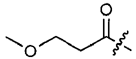 |
| **420** | CH₃ | H | Phenyl | H/H | H/H | 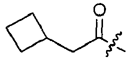 |
| **421** | CH₃ | CH₃ | 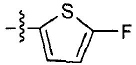 | =O | H/H | 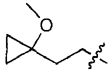 |
| **422** | CH₃ | H | Phenyl | H/H | H/H | 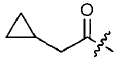 |
| **423** | CH₃ | H | 2-Thienyl | H/H | H/H | 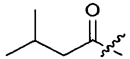 |
| **424** | CH₃ | CH₃ | 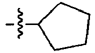 | H/H | H/H | H |
| **425** | CH₃ | CH₃ | 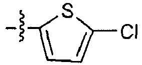 | H/H | H/H | H |
| **426** | CH₃ | CH₃ | 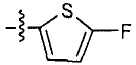 | H/H | H/H | H |
| **427; 428** | -CH₂CH₂CH₂- | | 2-Thienyl | H/H | H/H | H |
| **429; 430** | -CH₂CH₂CH₂- | | Phenyl | H/H | H/H | H |
| **431** | CH₃ | CH₃ | Phenyl | =O | H/H | H |
| **432; 433** | CH₃ | CH₃ | n-Butyl | =O | H/H | H |

wobei diese Verbindungen vorliegen können in Form eines einzelnen Stereoisomers oder deren Mischung, der freien Verbindungen und/oder ihrer physiologisch verträglichen Salze und/oder Solvate.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, visceralem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nichtäquimolare oder äquimolare Mischung der Diastereomere und/oder Enantiomere vorliegt.

Ein weiterer Gegenstand der Erfindung betrifft ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels.

Ein weiterer Gegenstand der Erfindung betrifft ein Verfahren zur Herstellung der erfindungsgemäßen Verbindungen wie in der folgenden Beschreibung und Beispielen ausgeführt.

### Allgemeine Syntheseschemata:

In einer bevorzugten Ausführungsform folgt die Synthese der erfindungsgemäßen Verbindungen dem folgenden allgemeinen Syntheseschema:

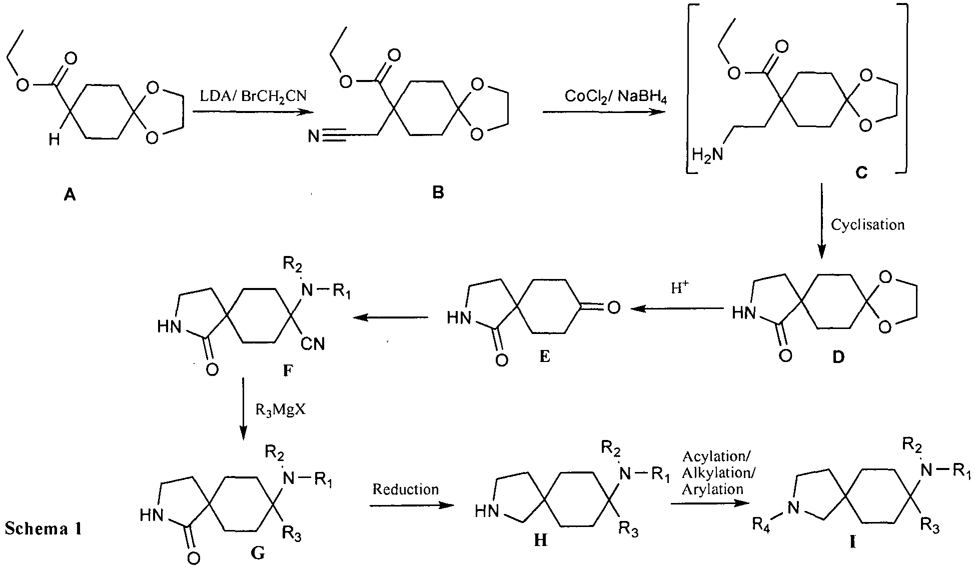

In Stufe 1 wird Verbindung A (WO2007079930) unter basischen Bedingungen in das Nitril B überführt (WO2007127763; Reimann, Eberhard et al., Archiv der Pharmazie (Weinheim, Germany) (1988), 321(12), 935-41). Die Reduktion des Nitrils erfolgt, z.B. mit Cobaltborid (WO2007127763), wobei das Intermediat C spontan zum Lactam D cyclisiert. Das Lactam D wird unter sauren Bedingungen entschützt (Cerammoniumnitrat/ Acetonitril/Wasser (I. Márko et al., Angew. Chem. 1999, 111, 3411-3413; Tetrahedron 2003, 59, 8989-8999), Palladiumchlorid-bis-Acetonitril-Komplex/Aceton (B. H. Lipshutz et al., Tetrahedron Lett. 1985, 26, 705-708), Natriumiodid/Cer(III)-chlorid/Acetonitril (E. Marcantoni et al., J. Org. Chem. 1997, 62, 4183-4184) und Thioharnstoff/Ethanol/Wasser (S. Majumdar, A. Bhattacharjya, J. Org. Chem. 1999, 64, 5682-5685) und anschliessend wird es in einer Streckerreaktion in das Nitril F überführt (WO2008101660, WO2008009415). Das Nitril F reagiert in einer Bruylants-Reaktion (D.Alberti et al., Bioorg. Med. Chem. Lett. 2006, 16, 4321-4325) mit einem Grignardreagenz zu der Verbindung der allgemeinen Formel G. Die Verbindungen der allgemeinen Formel G werden nach literaturbekannten Methoden reduziert, z.B. mit Lithiumaluminiumhydrid (Wang, Jun et al., J. Am. Chem. Soc., 131(23), 8066-8076; 2009; Bhandari, Kalpana et al., Chemistry & Industry (London, United Kingdom), (17), 547-8; 1990). Die Verbindungen der allgemeinen Formel H werden nach literaturbekannten Methoden am Stickstoff alkyliert (Hutchins, Robert O., Markowitz, Morris J. Org. Chem. 46(17), 3571-4; 1981; Setaki, Despina et al., Bioorg. Chem., 34(5), 248-273; 2006; Stamatiou, G. et al.;Bioorg. & Med. Chem. Lett. 11(16), 2137-2142; 2001), aryliert (WO2007070826, US7157456, WO2002085838) und acyliert (WO2008034731, WO2008036755, US20070117824, WO2007030061). Alternativ kann auch zuerst die Verbindung G alkyliert oder aryliert werden und danach reduziert werden. Nach dieser Syntheseroute entstehen ein polares und ein unpolares Diastereomer der allgemeinen Formel G, bevorzugt aber das polare Diastereomer G.

In einer anderen bevorzugten Ausführungsform folgt die Synthese der erfindungsgemäßen Verbindungen dem folgenden allgemeinen Syntheseschema:

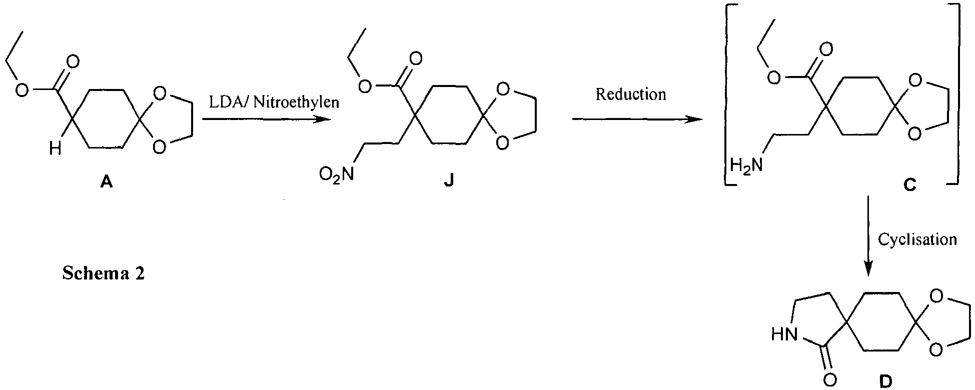

In Stufe 1 wird Verbindung A (WO2007079930) unter basischen Bedingungen in die Nitroverbindung J überführt und anschliessend reduziert (G. H. Posner, D. R. Crouch, Tetrahedron 1990, 46, 7509-7530; R.J. Flintoft et al., Tetrahedron Lett. 1999, 44, 4485-4488; E.A. Krafft et al., Synthesis 2005, 3245-3252). Die weitere Umsetzung der Verbindung D erfolgt wie in Schema 1 beschrieben.

In einer anderen bevorzugten Ausführungsform folgt die Synthese der erfindungsgemäßen Verbindungen dem folgenden allgemeinen Syntheseschema:

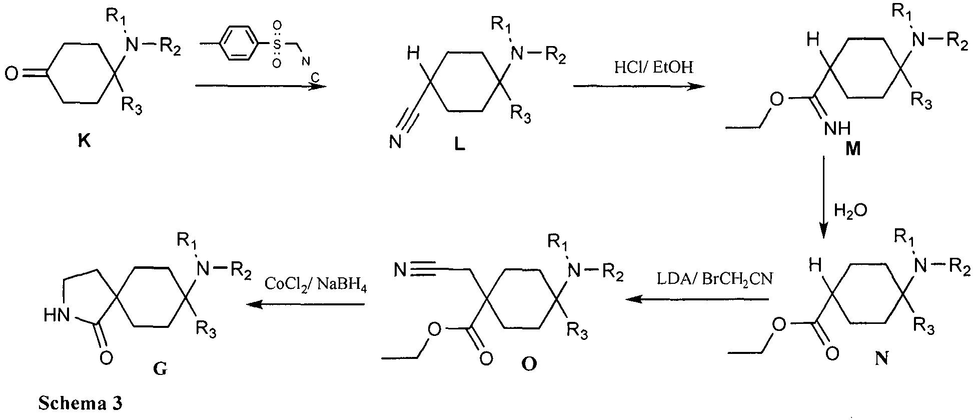

In Stufe 1 werden Ketone der allgemeinen Formel K (synthetisiert in Analogie zu WO2006/031610 und US6573386) mit TosMIC in Nitrile der allgemeinen Formel L überführt (Van Leusen, Daan et al., Organic Reactions (Hoboken, NJ, United States), 57, 2001). Das Nitril L wird in einer Pinner-Reaktion in den Imidoester M (Whitlock, Gavin A. et al., Bioorg. & Med. Chem. Lett. 18(9), 2930-2934, 2008; Geffken, Detlef et al., Archiv der Pharmazie (Weinheim, Germany), 321(1), 45-9; 1988) überführt und anschliessend hydrolysiert (US2002/58687). Der Ester N wird unter basischen Bedingungen wie der Ester A in Schema 1 in das Nitril O überführt. Das Nitril O wird unter literaturbekannten Bedingungen reduziert und zu dem Lactam G cyclisiert (WO2007127763). Nach dieser Syntheseroute entstehen ein polares und ein unpolares Diastereomer der allgemeinen Formel G. Die weitere Umsetzung der Verbindung G erfolgt wie in Schema 1 beschrieben.

In einer anderen bevorzugten Ausführungsform folgt die Synthese der erfindungsgemäßen Verbindungen dem folgenden allgemeinen Syntheseschema:

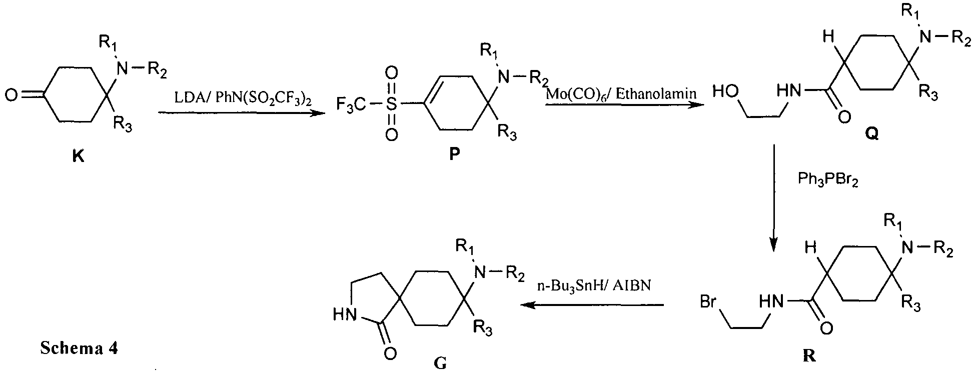

In Stufe 1 werden Ketone der allgemeinen Formel K (synthetisiert in Analogie zu WO2006/031610 und US6573386) in Enoltriflate (P) überführt (WO2009111056). Die Aminocarbonylierung mit Ethanolamin verläuft unter extrem milden Bedingungen (O. Lagerlund et al., Tetrahedron 2009, 65, 7646-7652; A.I. Meyers et al., Tetrahedron Lett. 1991, 33, 1181-1184). Der Alkohol Q wird unter literaturbekannten Bedingungen in ein Bromderivat der allgemeinen Formel R überführt (Van der Mey, Margaretha et al., J. Med. Chem. 45(12), 2520-2525; 2002). Anschliessend erfolgt eine exo-trig-Cyclisierung zwischen einem primären Radikal und einem α,β-ungesättigten Carbonsäurederivat zu der Verbindung G (T.J. Murray et al. Tetrahedron 1995, 51, 635-640). Nach dieser Syntheseroute entstehen ein polares und ein unpolares Diastereomer der allgemeinen Formel G. Die weitere Umsetzung der Verbindung G erfolgt wie in Schema 1 beschrieben.

In einer anderen bevorzugten Ausführungsform folgt die Synthese der erfindungsgemäßen Verbindungen dem folgenden allgemeinen Syntheseschema:

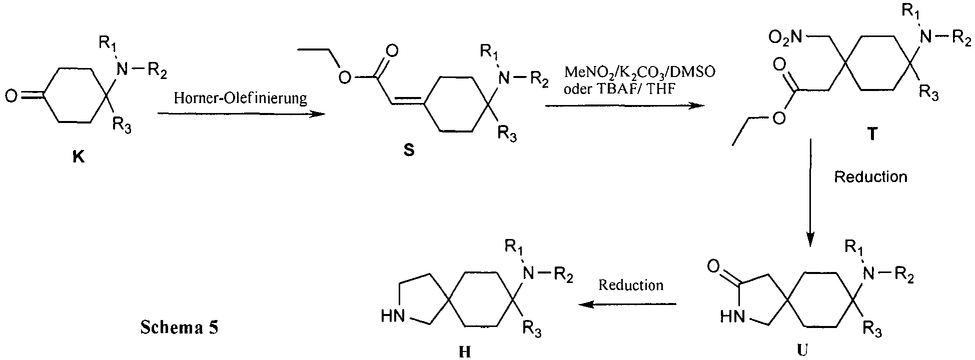

In Stufe 1 werden Ketone der allgemeinen Formel K (synthetisiert in Analogie zu WO2006/031610 und US6573386) in einer literaturbekannten Horner-Olefinierung in die Verbindungen S überführt (Wadsworth, W. S., Jr. Et al., Organic Syntheses, 45, 1965). Die Verbindungen der allgemeinen Formel S werden in einer Michael-Addition mit Nitromethan zur Verbindung T umgesetzt (US5091567; WO2008/129007; J.S. Bryans et al., J. Med. Chem. 1998, 41, 1838-1845). Die Nitroverbindung T wird unter literaturbekannten Bedingungen reduziert und in situ zu dem Lactam U cyclisiert (G.H. Posner, D.R. Crouch, Tetrahedron 1990, 46, 7509-7530; R.J. Flintoft et al., Tetrahedron Lett. 1999, 44, 4485-4488; E.A. Krafft et al., Synthesis 2005, 3245-3252). Durch Reduktion von U erhält man die Zielverbindungen der allgemeinen Formel H (Wang, Jun et al., J. Am. Chem. Soc., 131(23), 8066-8076; 2009; Bhandari, Kalpana et al., Chemistry & Industry (London, United Kingdom), (17), 547-8; 1990). Nach dieser Syntheseroute entstehen ein polares und ein unpolares Diastereomer der allgemeinen Formel U. Die Verbindungen der allgemeinen Formel H werden nach literaturbekannten Methoden am Stickstoff alkyliert (Hutchins, Robert O., Markowitz, Morris J. Org. Chem. 46(17), 3571-4; 1981; Setaki, Despina et al., Bioorg. Chem., 34(5), 248-273; 2006; Stamatiou, G. et al.; Bioorg. & Med. Chem. Lett. 11(16), 2137-2142; 2001), aryliert (WO2007070826, US7157456, WO2002085838) und acyliert (WO2008034731, WO2008036755, US20070117824, WO2007030061). Alternativ kann auch zuerst die Verbindung U alkyliert oder aryliert werden und danach reduziert werden. Bezüglich weiterer Einzelheiten zur Synthese der erfindungsgemäßen Verbindungen, insbesondere im Hinblick auf die Synthese geeigneter Eduktbausteine, wird ferner vollumfänglich verwiesen auf WO2004/043967, WO2005/063769, WO2005/066183, WO2006/018184, WO2006/108565, WO2007/124903, WO2008/004915 und WO2008/009416. Ein Fachmann erkennt, dass geeignete Eduktbausteine für die Synthese der erfindungsgemäßen Verbindungen in Analogie zu den in diesen Druckschriften offenbarten Syntheseschemata und Ausführungsbeispielen hergestellt werden können.

### Beispiele

Die folgenden Beispiele dienen zur näheren Erläuterung der Erfindung, sind jedoch nicht einschränkend auszulegen.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

### Synthesevorschriften

### Beispiel Nr. 1

### Stufe 1:8-Cyanomethyl-1,4-dioxaspiro[4.5]decan-8-carbonsäureethylester

Zu einer Lösung von Diisopropylamin (5.56 g, 55 mmol) in wasserfreiem Tetrahydrofuran (80 mL) wurde unter Argon bei -78 °C eine 2.5 *M* Lösung von *n*-Butyllithium in *n*-Hexan (22 mL, 55 mmol) getropft und anschließend 15 min bei 0 °C gerührt. Zu dieser zitronengelben Lösung wurde bei -78 °C innerhalb von 20 min eine Lösung von Ethyl-1,4-dioxaspiro[4.5]decan-8-carboxylat (10.7 g, 50 mmol) in Tetrahydrofuran (15 mL) getropft. Das dunkelgelbe Gemisch wurde 1.5 h bei -78 °C gerührt und dann tropfenweise mit einer Lösung von Bromacetonitril (7.16 g, 3.98 mL, 60 mmol) und 1,3-Dimethyl-3,4,5,6-tetrahydro-2-(1*H*)pyrimidon (DMPU, 3.20 g, 3.0 mL, 25 mmol) in Tetrahydrofuran (15 mL) versetzt. Danach wurde die orangefarbene Lösung langsam auf Raumtemperatur erwärmt und über Nacht gerührt. Die nun rotbraune Lösung wurde mit 0.5 *N* Salzsäure (38 mL) versetzt und die Phasen wurden getrennt. Die wässrige Phase wurde mit Diethylether (3 × 50 mL) extrahiert. Die vereinigten organischen Phasen wurden mit gesättigter Natriumhydrogencarbonat-Lösung (2 × 100 mL) und mit gesättigter Natriumchloridlösung (4 × 100 mL) gewaschen, mit Natriumsulfat getrocknet und i. Vak. eingeengt. Das Rohprodukt (12.1 g) wurde durch Flashchromatographie (400 g, 20 × 7.5 cm) mit Ethylacetat / Cyclohexan (1:2) gereinigt.
Ausbeute: 6.50 g (51 %), gelbliches Öl
¹H-NMR (CDCl₃): 1.29 (t, 3H, J = 7.1 Hz); 1.62-1.76 (m, 6H); 2.17-2.29 (m, 2H); 2.57 (s, 2H); 3.93 (t, 4H, J = 2.2 Hz); 4.23 (q, 2H, J = 7.1 Hz).

### Stufe 2: 1,4-Dioxa-10-aza-dispiro[4.2.4.2]tetradecan-9-on

Zu einer himbeerfarbenen Mischung aus 8-Cyanomethyl-1,4-dioxaspiro[4.5]decan-8-carbonsäureethylester (6.50 g, 25.6 mmol) und wasserfreiem Cobalt(II)-chlorid (1.66 g, 12.8 mmol) in Tetrahydrofuran (100 mL) und Wasser (50 mL) wurde unter Argon bei 0 °C portionsweise Natriumborhydrid (4.84 g, 128 mmol) gegeben und dann über Nacht bei Raumtemperatur gerührt. Dabei färbte sich die Lösung schwarz. Da die Umsetzung noch nicht vollständig war, wurden nochmals Cobalt(II)-chlorid (830 mg, 6.4 mmol) und Natriumborhydrid (2.42 g, 64 mmol) zugegeben und weitere 24 h gerührt. Das Reaktionsgemisch wurde mit 25 % wässriger Ammoniaklösung (5 mL) versetzt und filtriert. Der Filterrückstand wurde mit Tetrahydrofuran / Wasser (2:1) gewaschen. Das Filtrat wurde i. Vak. eingeengt und die wässrige Lösung mit Dichlormethan (3 × 50 mL) extrahiert. Die vereinigten organischen Extrakte wurden mit Natriumsulfat getrocknet und i. Vak. eingeengt. Ausbeute: 4.64 g (86 %), weißer Feststoff, der noch ca. 30 % Edukt enthält.

### Stufe 3: 2-Azaspiro[4.5]decan-1,8-dion

Eine Lösung von 1,4-Dioxa-10-aza-dispiro[4.2.4.2]tetradecan-9-on (4.64 g, 21.9 mmol) in Methanol (75 mL) und Wasser (25 mL) wurde mit p-Toluolsulfonsäure (5.00 g, 26.3 mmol) versetzt und 24 h bei Raumtemperatur sowie 24 h bei 50 °C gerührt. Das Reaktionsgemisch wurde anschließend mit 5 *N* Natronlauge alkalisch gestellt und eingeengt. Der Rückstand wurde mit Wasser (50 mL) verdünnt und mit Dichlormethan (6 × 30 mL) extrahiert. Die organische Phase wurde mit Natriumsulfat getrocknet und i. Vak. eingeengt. Das Rohprodukt (2.09 g) wurde durch Flashchromatographie (200 g, 20 × 5.7 cm) mit Ethylacetat / Dichlormethan (4:1) und Ethylacetat / Dichlormethan / Methanol (3:1:1) gereinigt. Die Mischfraktionen (850 mg) wurden erneut durch Flashchromatographie (100 g, 20 × 4.0 cm) mit *tert*-Butylmethylether / Methanol (14:1) gereinigt.
Ausbeute: 1.20 g (33 %), weißer Feststoff
Schmelzpunkt: 128-130 °C
¹H-NMR (CDCl₃): 1.73-1.89 (m, 2H); 2.08-2.21 (m, 4H); 2.33 (ddd, 2H, J = 5.8, 10.2 und 15.0 Hz); 2.70 (td, 2H, J = 6.3 und 14.8 Hz); 3.41 (dt, 2H, J = 0.8 und 7.1 Hz); 3.72 (s, 1H).

### Stufe 4: Dimethylamino-1-oxo-2-azaspiro[4.5]decan-8-carbonitril

Zu einer auf 0 °C gekühlten 40 % wässrigen Dimethylaminlösung (3.6 mL, 28.7 mmol) und Methanol (1.6 mL) wurde 4 *N* Salzsäure (2.15 mL, 8.56 mmol) und 2-Azaspiro[4.5]decan-1,8-dion (1.20 g, 7.17 mmol) in Methanol (16 mL) gegeben. Dieses Gemisch wurde mit Kaliumcyanid (931 mg, 14.3 mmol) versetzt und über das Wochenende bei Raumtemperatur gerührt. Nach Zugabe von Wasser (30 mL) wurde die Lösung mit Diethylether und Dichlormethan (je 3 × 30 mL) extrahiert. Die vereinigten organischen Phasen wurden mit Natriumsulfat getrocknet und i. Vak. eingeengt.
Ausbeute: 1.40 g (88 %), weißer Feststoff
¹H-NMR (CDCl₃): 1.35-1.67 (m, 3H); 1.76-2.09 (m, 5H); 2.18-2.31 (m, 2H); 2.33 und 2.35 (2s, 6H); 3.28-3.35 (m, 2H); 6.50 und 6.60 (2s, 1H). Es handelt sich um ein Diastereoisomerengemisch im Verhältnis von ca. 2:1.

### Stufe 5: 8-Benzyl-8-(dimethyl-amino)-3-azaspiro[4.5]decan-4-on (Beispiel Nr. 1, polares Diastereomer, Beispiel Nr. 2, unpolares Diastereomer)

Zu einer 2 M Lösung von Benzylmagnesiumchlorid in Tetrahydrofuran (9.5 mL, 19 mmol) wurde bei 0 °C unter Argon eine Lösung von Dimethylamino-1-oxo-2-azaspiro[4.5]decan-8-carbonitril (1.40 g, 6.3 mmol) in wasserfreiem Tetrahydrofuran (60 mL) getropft und danach bei Raumtemperatur über Nacht gerührt. Anschließend wurde die Reaktionslösung mit 20 % Ammoniumchloridlösung (25 mL) versetzt. Die Phasen wurden getrennt und die wässrige Phase mit Ethylacetat (3 × 30 mL) extrahiert. Die vereinigten organischen Phasen wurden mit Natriumsulfat getrocknet und i. Vak. eingeengt. Das Rohprodukt (2.00 g) wurde durch Flashchromatographie (100 g, 20 × 4.0 cm) mit Dichlormethan / Methanol (48:1) und 0.25 % Ammoniak (25 % in Wasser) gereinigt. Die Mischfraktionen (560 mg) wurden nochmals durch Flashchromatographie (38 g, 20 × 2.5 cm) mit Dichlormethan / Isopropanol (95:5) und 1 % Ammoniak (25 % in Wasser) gereinigt.

### Beispiel Nr. 1 (polares Diastereoisomer)

Ausbeute: 511 mg (28 %), farbloses Öl, das noch ca. 20 % des unpolaren Diastereoisomeren enthält.*
¹H-NMR (CDCl₃): 1.53-1.63 (m, 4H); 1.67-1.75 (m, 2H); 1.85-1.92 (m, 2H); 1.95 (t, 2H, J = 6.8 Hz); 2.28 (s, 6H); 2.77 (s, 2H); 3.21-3.26 (m, 2H); 5.71 (br s, 1H); 7.13-7.26 (m, 5H).
¹³C-NMR (CDCl₃): 28.2; 29.0; 35.3; 36.7; 37.4; 38.6; 41.5; 57.6; 125.7; 127.7; 130.8; 139.2; 182.6.
LC-MS: m/z: [M+H]⁺ = 287.3, Rₜ = 1.0 min.

### Beispiel Nr. 2 (unpolares Diastereoisomer)

Ausbeute: 970 mg (54 %), weißer Feststoff
Schmelzpunkt: 202-204 °C
¹H-NMR (CDCl₃): 1.05-1.19 (m, 4H); 1.67-1.80 (m, 4H); 2.00-2.14 (m, 2H); 2.30 (s, 6H); 2.62 (s, 2H); 3.15 (t, 2H, J = 7.2 Hz); 5.90 (br s, 1H); 7.00-7.13 (m, 2H); 7.15-7.28 (m, 3H).
¹³C-NMR (CDCl₃): 26.9; 28.6; 31.6; 37.0; 38.8; 43.6; 57.1; 125.6; 127.7; 130.6; 139.3; 183.3.
LC-MS:: m/z: [M+H]⁺ = 287.3, Rₜ = 2.3 min.

### Beispiel Nr. 3

### (8-Benzyl-3-azaspiro[4.5]decan-8-yl)-dimethyl-amin (Beispiel Nr. 3, polares Diastereomer)

Eine Aufschlämmung von Lithiumaluminiumhydrid (436 mg, 11.5 mmol) in wasserfreiem Tetrahydrofuran (20 mL) wurde unter Eiskühlung mit einer Lösung von 8-Benzyl-8-(dimethyl-amino)-3-azaspiro[4.5]decan-4-on (polares Diastereomer) (663 mg, 2.3 mmol) in wasserfreiem Tetrahydrofuran (35 mL) versetzt und anschließend über Nacht bei 60 °C gerührt. Das Gemisch wurde unter Eiskühlung mit Wasser (300 µL), 1 *N* Natronlauge (1 mL) und erneut mit Wasser (1 mL) versetzt und 1 h bei Raumtemperatur gerührt. Die Suspension wurde durch Seesand filtriert und der Rückstand mit Tetrahydrofuran gewaschen. Das Filtrat wurde mit Natriumsulfat getrocknet und i. Vak. eingeengt.

### Beispiel Nr. 3 (polares Diastereoisomer)

Ausbeute: 588 mg (94 %), farbloses Öl)

Ein Teil des Rohproduktes (165 mg) wurde durch Flashchromatographie (5 g, 15 × 0.9 cm) mit Dichlormethan / Methanol (9:1) und 1 % Ammoniak (25 % in Wasser) gereinigt, wodurch die Testsubstanz (130 mg) erhalten wurde.
¹H-NMR (CDCl₃): 1.07-1.22 (m, 4H); 1.48 (t, 2H, J = 7.2 Hz); 1.62-1.75 (m, 4H); 2.30 (s, 6H); 2.41 (s, 2H); 2..60 (s, 2H); 2.80 (br s, 1H); 2.85 (t, 2H, J = 7.2 Hz); 7.08-7.11 (m, 2H); 7.14-7.26 (m, 3H).
¹³C-NMR (CDCl₃): 30.0; 31.3; 36.7; 37.1; 41.1; 42.2; 45.5; 55.9; 57.6; 125.6; 127.7; 130.6; 139.3.
LC-MS: m/z: [M+H]⁺ = 273.4, Rₜ = 0.2 min.

### Beispiel Nr. 4

### (8-Benzyl-3-azaspiro[4.5]decan-8-yl)-dimethyl-amin (Beispiel Nr. 4, unpolares Diastereomer)

Eine Aufschlämmung von Lithiumaluminiumhydrid (463 mg, 12.2 mmol) in wasserfreiem Tetrahydrofuran (20 mL) wurde unter Eiskühlung mit einer Lösung von 8-Benzyl-8-(dimethyl-amino)-3-azaspiro[4.5]decan-4-on (unpolares Diastereomer) (700 mg, 2.44 mmol) in wasserfreiem Tetrahydrofuran (30 mL) versetzt und anschließend über Nacht bei 60 °C gerührt. Das Gemisch wurde unter Eiskühlung mit Wasser (300 µL), 1 *N* Natronlauge (1 mL) und erneut mit Wasser (1 mL) versetzt und 1 h bei Raumtemperatur gerührt. Die Suspension wurde durch Seesand filtriert und der Rückstand mit Tetrahydrofuran gewaschen. Das Filtrat wurde mit Natriumsulfat getrocknet und i. Vak. eingeengt.

### Beispiel Nr. 4 (unpolares Diastereoisomer)

Ausbeute: 640 mg (96 %), farbloses Öl

Ein Teil des Rohproduktes (154 mg) wurde durch Flashchromatographie (5 g; 15 × 0.9 cm) mit Dichlormethan / Methanol (9:1) und 1 % Ammoniak (25 % in Wasser) gereinigt, wodurch die Testsubstanz (117 mg) erhalten wurde.
¹H-NMR (CDCl₃): 1.09-1.27 (m, 6H); 1.62-1.71 (m, 4H); 2.23 (br s, 1H); 2.29 (s, 6H); 2.58 (s, 2H); 2.61 (s, 2H); 2.82 (t, 2H, J = 7.1 Hz); 7.09-7.26 (m, 5H).
¹³C-NMR (CDCl₃): 29.8; 31.3; 36.0; 36.7; 37.1; 42.4; 46.6; 57.6; 61.3; 125.5; 127.6; 130.6; 139.4.
LC-MS: m/z: [M+H]⁺ = 273.4, Rₜ = 0.3 min.

### Beispiel Nr. 5

### (8-Benzyl-3-methyl-3-azaspiro[4.5]decan-8-yl)-dimethyl-amin (Beispiel Nr. 5, polares Diastereomer)

Eine Lösung von (8-Benzyl-3-azaspiro[4.5]decan-8-yl)-dimethyl-amin (polares Diastereomer) (207 mg, 0.76 mmol) in Methanol (6.5 mL) wurde mit 37 % Formalinlösung (1.30 mL), Eisessig (650 µL) und Natriumcyanoborhydrid (205 mg, 3.2 mmol) versetzt und 16 h bei Raumtemperatur gerührt. Nach Zugabe von gesättigter Natriumhydrogencarbonat-Lösung (20 mL) wurde das Gemisch mit Dichlormethan (3 × 20 mL) extrahiert. Die vereinigten organischen Phasen wurden mit Natriumsulfat getrocknet und i. Vak. eingeengt. Das Rohprodukt (220 mg) wurde durch Flashchromatographie (10 g, 20 × 1.5 cm) mit Dichlormethan / Methanol (9:1) und 1 % Ammoniak (25 % in Wasser) gereinigt.

### Beispiel Nr. 5 (polares Diastereoisomer)

Ausbeute: 160 mg (73 %), weißer Feststoff
Schmelzpunkt: 89-91 °C
¹H-NMR (CDCl₃): 1.07-1.17 (m, 2H); 1.26-1.33 (m, 2H); 1.56 (t, 2H, J = 6.9 Hz); 1.62-1.72 (m, 4H); 2.07 (s, 2H); 2.21 (s, 3H); 2.29 (s, 6H); 2.43 (t, 2H, J = 6.9 Hz); 2.61 (s, 2H); 7.09-7.13 (m, 2H); 7.15-7.27 (m, 3H).
¹³C-NMR (CDCl₃): 30.1; 33.3; 36.8; 37.2; 40.8; 41.7; 42.7; 55.6; 57.5; 66.7; 125.6; 127.7; 130.7; 139.5.
LC-MS: m/z: [M+H]⁺ = 287.3, Rₜ = 0.2 min.

### Beispiel Nr. 6

### (8-Benzyl-3-methyl-3-azaspiro[4.5]decan-8-yl)-dimethyl-amin (Beispiel Nr. 6, unpolares Diastereomer)

Eine Lösung von (8-Benzyl-3-azaspiro[4.5]decan-8-yl)-dimethyl-amin (unpolares Diastereomer) (150 mg, 0.55 mmol) in Methanol (5 mL) wurde mit 37 % Formalinlösung (1 mL), Eisessig (500 µL) und Natriumcyanoborhydrid (138 mg, 2.2 mmol) versetzt und 16 h bei Raumtemperatur gerührt. Nach Zugabe von gesättigter Natriumhydrogencarbonat-Lösung (20 mL) wurde das Gemisch mit Dichlormethan (3 × 20 mL) extrahiert. Die vereinigten organischen Phasen wurden mit Natriumsulfat getrocknet und i. Vak. eingeengt. Das Rohprodukt (199 mg) wurde durch Flashchromatographie (10 g, 20 × 1.5 cm) mit Dichlormethan / Methanol (9:1) und 1 % Ammoniak (25 % in Wasser) gereinigt.

### Beispiel Nr. 6 (unpolares Diastereoisomer)

Ausbeute: 110 mg (70 %), weißer Feststoff
Schmelzpunkt: 57-58 °C
¹H-NMR (CDCl₃): 1.18-1.30 (m, 4H); 1.37 (t, 2H, J = 6.9 Hz); 1.64-1.77 (m, 4H); 2.28 (s, 3H); 2.31 (s, 2H); 2.33 (s, 6H); 2.45 (t, 2H, J = 6.9 Hz); 2.65 (s, 2H); 7.14-7.31 (m, 5H). Das Signal bei 4.76 ppm (s) gehört zu einer unbekannten Verunreinigung.
¹³C-NMR (CDCl₃): 29.4: 32.8; 36.0; 36.8; 37.1; 41.5; 42.7; 56.4; 57.5; 71.2; 74.8; 125.5; 127.7, 130.7; 139.4. Eines der Signale zwischen 56.0 und 75.0 gehört zu einer unbekannten Verunreinigung.
LC-MS: m/z: [M+H]⁺ = 287.4, Rₜ = 0.2 min.

### Beispiel Nr. 7

### 1-[8-Benzyl-8-(dimethyl-amino)-3-azaspiro[4.5]decan-3-yl]-ethanon (Beispiel Nr. 7, polares Diastereomer)

Eine Lösung von (8-Benzyl-3-azaspiro[4.5]decan-8-yl)-dimethyl-amin (polares Diastereomer) (183 mg, 0.67 mmol) in wasserfreiem Dichlormethan (5 mL) wurde mit Triethylamin (203 mg, 279 µL, 2.01 mmol) und Essigsäureanhydrid (137 mg, 126 µL, 1.34 mmol) versetzt und 3 h bei Raumtemperatur gerührt. Nach Zugabe von Dichlormethan (30 mL) wurde die Lösung mit 25 % gesättigter Kaliumcarbonat-Lösung (10 mL) gewaschen. Die organische Phase wurde mit Natriumsulfat getrocknet und i. Vak. eingeengt. Das Rohprodukt (220 mg) wurde durch Flashchromatographie (10 g, 20 × 1.5 cm) mit Dichlormethan / Methanol (95:5) und 1 % Ammoniak gereinigt.

### Beispiel Nr. 7 (polares Diastereoisomer)

Ausbeute: 138 mg (65 %), farbloses Öl
¹H-NMR (CDCl₃): 1.05-1.25 (m, 4H); 1.59-1.77 (m, 6H); 1.88 und 1.98 (2 s, 3H); 2.28 und 2.30 (2 s, 6H); 2.60 und 2.63 (2 s, 2H); 2.92 und 3.05 (2 s, 2H); 3.37-3.45 (m, 2H); 7.04-7.29 (m, 5H).
¹³C-NMR (CDCl₃): 22.1; 22.4; 29.7; 29.74; 29.9; 36.8; 36.9; 37.0; 37.1; 38.2; 39.8; 40.2; 42.1; 43.7; 45.7; 54.0; 55.8; 57.6; 57.62; 125.8; 126.0; 127.8; 128.0; 130.5; 130.6; 138.8; 139.1; 169.2; 169.4.
Die teilweise doppelten Signalsätze beruhen auf gehinderter Rotation.
LC-MS: m/z: [M+H]⁺ = 315.3, Rₜ = 2.3 min.

### Beispiel Nr. 8

### 1-[8-Benzyl-8-(dimethyl-amino)-3-azaspiro[4.5]decan-3-yl]-ethanon (Beispiel Nr. 8, unpolares Diastereomer)

Eine Lösung von (8-Benzyl-3-azaspiro[4.5]decan-8-yl)-dimethyl-amin (unpolares Diastereomer) (169 mg, 0.62 mmol) in wasserfreiem Dichlormethan (5 mL) wurde mit Triethylamin (188 mg, 258 µL, 1.86 mmol) und Essigsäureanhydrid (126 mg, 116 µL, 1.24 mmol) versetzt und 3 h bei Raumtemperatur gerührt. Nach Zugabe von Dichlormethan (40 mL) wurde die Lösung mit 25 % gesättigter Kaliumcarbonat-Lösung (10 mL) gewaschen. Die organische Phase wurde mit Natriumsulfat getrocknet und i. Vak. eingeengt. Das Rohprodukt (203 mg) wurde durch Flashchromatographie (10 g, 20 × 1.5 cm) mit Dichlormethan / Methanol (95:5) und 1 % Ammoniak gereinigt.

### Beispiel Nr. 8 (unpolares Diastereoisomer)

Ausbeute: 146 mg (75 %), farbloses Öl
¹H-NMR (CDCl₃): 1.11-1.26 (m, 4H); 1.54 und 1.52 (2 t, 2H, J = 7.2 Hz); 1.63-1.77 (m, 4H); 1.97 und 1.98 (2 s, 3H); 2.28 und 2.95 (2 s, 6H); 2.63 (s, 2H); 3.14 und 3.19 (2 s, 2H); 3.34 und 3.38 (2 t, 2H, J = 7.2 Hz); 7.09-7.29 (m, 5H).
¹³C-NMR (CDCl₃): 22.0; 22.4; 29.0; 29.1; 29.54; 29.57; 32.2; 33.6; 36.5; 36.6; 37.0; 40.2; 41.9; 44.3; 46.1; 57.6; 57.62; 58.3; 60.8; 125.6; 125.8; 127.8; 127.9; 130.6; 130.7; 139.0; 139.3; 169.1; 169.2.
Die teilweise doppelten Signalsätze beruhen auf gehinderter Rotation.
LC-MS: m/z: [M+H]⁺ = 315.3, Rₜ = 2.5 min.

### Beispiel Nr. 9

### (8-Benzyl-3-butyl-3-azaspiro[4.5]decan-8-yl)-dimethyl-amin (Beispiel Nr. 9, unpolares Diastereomer)

Eine Lösung von (8-Benzyl-3-azaspiro[4.5]decan-8-yl)-dimethyl-amin (unpolares Diastereomer) (162 mg, 0.59 mmol) in Methanol (5 mL) wurde mit Natriumcyanoborhydrid (177 mg, 2.82 mmol) und Butyraldehyd (86 mg, 106 µL, 1.19 mmol) versetzt und 30 min bei Raumtemperatur gerührt. Das Gemisch wurde mit Eisessig (600 µL) versetzt und weitere 16 h bei Raumtemperatur gerührt. Nach Zugabe von gesättigter Natriumhydrogencarbonat-Lösung (20 mL) wurde das Gemisch mit Dichlormethan (3 × 20 mL) extrahiert. Die vereinigten organischen Phasen wurden mit Natriumsulfat getrocknet und i. Vak. eingeengt. Das Rohprodukt (202 mg) wurde durch Flashchromatographie (10 g, 20 × 1.5 cm) mit Dichlormethan / Methanol (95:5) und 1 % Ammoniak gereinigt.

### Beispiel Nr. 9 (unpolares Diastereoisomer)

Ausbeute: 150 mg (87 %), farbloses Öl
¹H-NMR (CDCl₃): 0.92 (t, 3H, J = 7.3 Hz); 1.10-1.20 (m, 2H); 1.26-1.40 (m, 4H); 1.52-1.65 (m, 4H); 1.67-1.82 (m, 4H); 2.28 (s, 6H); 2.62 (s, 2H); 2.70-2.77 (m, 4H); 2.95 (br t, 2H, J = 6.5 Hz); 7.07-7.11 (m, 2H); 7.16-7.28 (m, 3H).
¹³C-NMR (CDCl₃): 13.6; 20.2; 28.5; 29.2; 31.4; 33.5; 36.5; 37.0; 41.3; 53.8; 56.3; 57.3; 66.7; 125.8; 127.9; 130.6; 138.8.
LC-MS: m/z: [M+H]⁺ = 329.4, Rₜ = 1.2 min.

### Beispiel Nr. 10

### (8-Benzyl-3-butyl-3-azaspiro[4.5]decan-8-yl)-dimethyl-amin (Beispiel Nr. 10, polares Diastereomer)

Zu einer Lösung von (8-Benzyl-3-azaspiro[4.5]decan-8-yl)-dimethyl-amin (polares Diastereomer) (169 mg, 0.62 mmol) und Triethylamin (193 mg, 264 µL, 1.9 mmol) in wasserfreiem Dichlormethan (5 mL) wurde Buttersäurechlorid (134 mg, 132 µL, 1.26 mmol) gegeben und 18 h bei Raumtemperatur gerührt. Das Reaktionsgemisch wurde mit Dichlormethan (20 mL) verdünnt und mit 25 % Kaliumcarbonatlösung (2 × 10 mL) gewaschen. Die organische Phase wurde mit Natriumsulfat getrocknet und i. Vak. eingeengt. Das Rohprodukt (240 mg) wurde durch Flashchromatographie (10 g, 20 × 1.5 cm) mit Dichlormethan / Methanol (95:5) und 1 % Ammoniak (25 % in Wasser) gereinigt.

### Beispiel Nr. 10 (polares Diastereoisomer)

Ausbeute: 159 mg (75 %), farbloses Öl
¹H-NMR (CDCl₃): 0.88 und 0.93 (2 t, 3H, J = 7.4 Hz); 1.04-1.23 (m, 4H); 1.53-1.77 (m, 8H); 2.04 und 2.77 (2 t, 2H, J = 7.5 Hz); 2.28 und 2.30 (2 s, 6H); 2.60 und 2.63 (2 s, 2H); 2.92 und 3.06 (2 s, 2H); 3.36-3.44 (m, 2H); 7.04-7.29 (m, 5H).
¹³C-NMR (CDCl₃): 13.9; 14.0; 18.3; 18.5; 29.59; 29.6; 29.8; 29.9; 36.4; 36.7; 36.8; 36.84; 37.0; 37.1; 37.9; 39.7; 40.0; 42.0; 43.7; 44.9; 54.0; 54.9; 57.61; 57.64; 125.7; 125.9; 127.8; 128.0; 130.5; 130.6; 138.8; 139.1; 171.9.
Die NMR-Spektren zeigen teilweise doppelte Signalsätze (Rotamere).
LC-MS: m/z: [M+H]⁺ = 343.4, Rₜ = 2.7 min.

### Beispiel Nr. 11

### (8-Benzyl-3-butyl-3-azaspiro[4.5]decan-8-yl)-dimethyl-amin (Beispiel Nr. 11, unpolares Diastereomer)

Zu einer Lösung von (8-Benzyl-3-azaspiro[4.5]decan-8-yl)-dimethyl-amin (unpolares Diastereomer) (173 mg, 0.63 mmol) und Triethylamin (193 mg, 264 µL, 1.9 mmol) in wasserfreiem Dichlormethan (5 mL) wurde Buttersäurechlorid (134 mg, 132 µL, 1.26 mmol) gegeben und 18 h bei Raumtemperatur gerührt. Das Reaktionsgemisch wurde mit Dichlormethan (20 mL) verdünnt und mit 25 % Kaliumcarbonatlösung (2 × 10 mL) gewaschen. Die organische Phase wurde mit Natriumsulfat getrocknet und i. Vak. eingeengt. Das Rohprodukt (220 mg) wurde durch Flashchromatographie (10 g, 20 × 1.5 cm) mit Dichlormethan / Methanol (300:5) und 1 % Ammoniak (25 % in Wasser) gereinigt.

### Beispiel Nr. 11 (unpolares Diastereoisomer)

Ausbeute: 157 mg (73 %), weißer Feststoff
Schmelzpunkt: 98-105 °C
¹H-NMR (CDCl₃): 0.93 und 0.94 (2 t, 3H, J = 7.4 Hz); 1.12-1.26 (m, 4H); 1.41 und 1.50 (2 t, 2H, J = 7.1 Hz); 1.59-1.77 (m, 6H); 2.13-2.19 (m, 2H); 2.28 und 2.30 (2 s, 6H); 2.62 (s, 2H); 3.16 und 3.19 (2 s, 2H); 3.33 und 3.38 (2 t, 2H, J = 7.1 Hz); 7.09-7.28 (m, 5H).
¹³C-NMR (CDCl₃): 14.0; 14.02; 18.3; 18.4; 29.0; 29.1; 29.5; 29.6; 32.0; 33.6; 36.2; 36.5; 36.6; 36.8; 37.0; 40.0; 41.9; 44.3; 45.3; 57.6; 57.7; 58.4; 60.0; 125.6; 125.7; 127.8; 127.9; 130.6; 130.7; 139.1; 139.3; 171.6; 171.7.
Die NMR-Spektren zeigen teilweise doppelte Signalsätze (Rotamere).
LC-MS: m/z: [M+H]⁺ = 343.3, Rₜ = 2.9 min.

### Beispiel Nr. 12

### [8-Benzyl-3-(cyclopentyl-methyl)-3-azaspiro[4.5]decan-8-yl]-dimethyl-amin (Beispiel Nr. 12) unpolares Diastereomer,

Eine Lösung von (8-Benzyl-3-azaspiro[4.5]decan-8-yl)-dimethyl-amin (unpolares Diastereomer) (202 mg, 0.74 mmol) in Methanol (5 mL) wurde mit Cyclopentancarbaldehyd (125 mg, 136 µL, 1.3 mmol) und Eisessig (500 µL) versetzt und 2 h bei Raumtemperatur gerührt. Nach Zugabe von Natriumcyanoborhydrid (200 mg, 3.1 mmol) wurde das Gemisch 24 h bei Raumtemperatur gerührt. Anschließend wurde das Reaktionsgemisch mit Dichlormethan (20 mL) verdünnt, mit gesättigter Natriumhydrogencarbonat-Lösung (25 mL) versetzt und die Phasen wurden getrennt. Die wässrige Phase wurde mit Dichlormethan (3 × 20 mL) extrahiert. Die vereinigten organischen Phasen wurden mit Natriumsulfat getrocknet und i. Vak. eingeengt. Das Rohprodukt (283 mg) wurde durch Flashchromatographie (10 g, 20 × 1.5 cm) mit Dichlormethan / Methanol (95:5) und 1 % Ammoniak (25 % in Wasser) gereinigt.

### Beispiel Nr. 12 (unpolares Diastereoisomer)

Ausbeute: 192 mg (73 %), farbloses Öl
¹H-NMR (CDCl₃): 1.11-1.29 (m, 6H); 1.37 (t, 2H, J = 6.9 Hz); 1.45-1.80 (m, 10H); 1.98 (td, 1H, J = 15.4, 7.8 Hz); 2.29 (s, 6H); 2.39 (d, 2H, J = 7.2 Hz); 2.41 (s, 2H); 2.58 (t, 2H, J = 6.9 Hz); 2.62 (s, 2H); 7.09-7.25 (m, 5H).
¹³C-NMR (CDCl₃): 25.1; 29.4; 31.5; 32.5; 34.6; 36.7; 37.1; 38.8; 41.0; 54.7; 57.5; 62.5; 68.5; 125.6; 127.7; 130.7; 139.3.
LC-MS: m/z: [M+H]⁺ = 355.4, Rₜ = 2.0 min.

### Beispiel Nr. 13

### 8-Dimethylamino-8-phenyl-3-azaspiro[4.5]decan-4-on (Beispiel Nr. 13, ein Diastereomer)

Zu einer 2 *M* Lösung von Phenylmagnesiumchlorid in Tetrahydrofuran (2.4 mL, 4.8 mmol) wurde bei 0 °C und unter Argon eine Lösung von Dimethylamino-1-oxo-2-azaspiro[4.5]decan-8-carbonitril (330 mg, 1.49 mmol) in Tetrahydrofuran (15 mL) getropft und danach über Nacht bei Raumtemperatur gerührt. Anschließend wurde die Reaktionslösung mit 20 % Ammoniumchloridlösung (15 mL) versetzt. Die Phasen wurden getrennt und die wässrige Phase wurde mit Ethylacetat (2 × 15 mL) extrahiert. Die vereinigten organischen Phasen wurden mit Natriumsulfat getrocknet und i. Vak. eingeengt. Das Rohprodukt (520 mg) wurde durch Flashchromatographie (45 g, 23 × 2.5 cm) mit Dichlormethan / Methanol (95:5) und 0.5 % Ammoniak (33 % in H₂O) gereinigt.

### Beispiel Nr. 13 (ein Diastereomer)

Ausbeute: 155 mg (38 %), weißer Feststoff
Schmelzpunkt: 183-185 °C
¹H-NMR (CDCl₃): 1.30-1.37 (m, 2H); 1.61 (t, 2H, J = 13.5 Hz); 1.97 (t, 2H, J = 6.9 Hz); 2.05 (s, 6H); 2.21 (dt, 2H, J = 3.1 und 13.1 Hz); 2.62 (br d, 2H, J = 14.4 Hz); 3.26-3.32 (m, 2H); 6.17 (br s, 1H); 7.21-7.30 (m, 1H); 7.30-7.39 (m, 4H).
¹³C-NMR (CDCl₃): 28.2 (2C); 29.3; 32.8; 37.9 (2C); 38.8 (2C); 43.1; 58.7; 126.5; 126.8 (2C); 127.5 (2C); 139.4; 182.9.
LC-MS: m/z: [M+H]⁺= 273.3, Rₜ = 1.4 min.

### Beispiel Nr. 14 und Beispiel Nr. 15

### Stufe 1: (4-Dimethylamino-4-phenylcyclohexyliden)essigsäureethylester

Eine Lösung von Phosphonoessigsäure-triethylester (3.86 g, 3.14 mL, 17.3 mmol) in wasserfreiem *N,N*-Dimethylformamid (20 mL) wurde unter Argon mit Kalium-*tert*-butylat (1.93 g, 17.3 mmol) versetzt. Das Gemisch wurde 10 min bei Raumtemperatur gerührt und anschließend mit einer Lösung von 4-(Dimethylamino)-4-phenylcyclohexanon (2.50 g, 11.5 mmol) in wasserfreiem *N,N*-Dimethylformamid (40 mL) versetzt, danach 1 h bei Raumtemperatur gerührt und anschließend in Eiswasser (50 g) gegossen. Die wässrige Suspension wurde mit Diethylether (4 × 40 mL) extrahiert. Die vereinigten organischen Phasen wurden mit Natriumsulfat getrocknet und i. Vak. eingeengt.
Ausbeute: 3.39 g (100 %)
¹H-NMR (CDCl₃): 1.26 (t, 3H, J = 7.1 Hz); 2.06 (s, 6H); 2.10-2.25 (m, 5H); 2.45 (m, 1H); 2.67 (m, 1H); 3.20 (m, 1H); 4.13 (q, 2H, J = 7.1 Hz); 5.60 (s, 1H); 7.26 (m, 1H); 7.31-7.40 (m, 4H). LC-MS: [M+H]⁺: m/z = 288.3, Rₜ = 2.7 min.

### Stufe 2: (4-Dimethylamino-2-nitromethyl-4-phenylcyclohexyl)essigsäureethylester

Eine Mischung von (4-Dimethylamino-4-phenylcyclohexyliden)essigsäureethylester (500 mg, 1.74 mmol) und Tetra-n-butylammoniumfluorid-Trihydrat (602 mg, 1.91 mmol) in Tetrahydrofuran (30 mL) wurde mit Nitromethan (138 mg, 122 µL, 2.26 mmol) versetzt und 6 h bei 70 °C gerührt. Das Reaktionsgemisch wurde anschließend i. Vak. eingeengt und der Rückstand (1.10 g) durch Flashchromatographie (38 g, 20 × 2.5 cm) mit Ethylacetat / Methanol (95:5) gereinigt.
Ausbeute: 453 mg (75 %), gelbliches Öl
¹H-NMR (DMSO-d₆): Das Spektrum zeigt alle erforderlichen Signale. Es handelt sich um ein Diastereoisomerengemisch im Verhältnis von ca. 3:2.

### Stufe 3: 8-(Dimethyl-amino)-8-phenyl-3-azaspiro[4.5]decan-2-on (Beispiel Nr. 14, polares Diastereomer, Beispiel Nr. 15, unpolares Diastereomer)

Zu einer Mischung aus Eisenpulver (904 mg, 16.2 mmol), Ammoniumchlorid (4.33 g, 81 mmol) und Wasser (3.3 mL) wurde eine Lösung von (4-Dimethylamino-2-nitromethyl-4-phenylcyclohexyl)essigsäureethylester (1.13 g, 3.24 mmol) in Ethanol (32 mL) gegeben und anschließend 5 h bei 80 °C gerührt. Das Gemisch wurde filtriert und der Rückstand mit Ethanol gewaschen. Das Filtrat wurde mit 5 % Natriumhydrogencarbonat-Lösung (1 mL) alkalisch gestellt und dann i. Vak. eingeengt. Das Rohprodukt wurde durch Flashchromatographie (38 g, 20 × 2.5 cm) mit Dichlormethan / Methanol (95:5) und 1 % Ammoniak (25 % in Wasser) gereinigt. Das isolierte Diastereoisomerengemisch wurde durch Mitteldruckchromatographie an einer PuriFlash-Kartusche (PF-15SIHP, 40 g) mit obigem Eluenten getrennt.

### Beispiel Nr. 14(polares Diastereoisomer)

Ausbeute: 330 mg (37 %), weißer Feststoff
Schmelzpunkt: 210-215 °C
¹H-NMR (CDCl₃): 1.35-1.45 (m, 2H); 1.73-1.82 (m, 2H); 1.86-2.01 (m, 2H); 2.02 (s, 6H); 2.05 (s, 2H); 2.10-2.30 (m, 2H); 3.26 (s, 2H); 6.28 (s, 1H); 7.25-7.30 (m, 3H); 7.35-7.40 (m, 2H).
¹³C-NMR (CDCl₃): 30.2; 32.7; 37.98; 38.0; 39.0; 43.3; 52.8; 53.4; 60.1; 126.7; 127.4; 127.7; 136.0; 177.4.
LC-MS: m/z: [M+H]⁺ = 273.3, Rₜ = 1.3 min.

### Beispiel Nr. 15 (unpolares Diastereoisomer)

Ausbeute: 215 mg (24 %), weißer Feststoff
Schmelzpunkt: 218-223 °C
¹H-NMR (CDCl₃): 1.35-1.45 (m, 2H); 1.50-1.58 (m, 1H); 1.73-1.82 (m, 2H); 1.85-2.02 (m, 2H); 2.04 (s, 6H); 2.13-2.18 (m, 1H); 2.30 (s, 2H); 3.02 (s, 2H); 5.41 (br s, 1H); 7.27-7.32 (m, 3H); 7.36-7.41 (m, 2H).
¹³C-NMR (CDCl₃): 30.2; 32.9; 38.0; 38.1; 39.2; 42.4; 53.9; 60.4; 126.6; 127.5; 127.7; 136.3; 177.6.
LC-MS: m/z: [M+H]⁺ = 273.3, Rₜ = 1.6 min.

### Beispiel Nr. 16

### 8-Butyl-8-dimethylamino-3-azaspiro[4.5]decan-4-on (Beispiel Nr. 16, ein Diastereomer)

Zu einer 2 *M* Lösung von *n*-Butylmagnesiumchlorid in Tetrahydrofuran (3.5 mL, 7 mmol) wurde bei 0 °C und unter Argon eine Lösung von Dimethylamino-1-oxo-2-azaspiro[4.5]decan-8-carbonitril (390 mg, 1.76 mmol) in Tetrahydrofuran (15 mL) getropft und danach über Nacht bei Raumtemperatur gerührt. Anschließend wurde die Reaktionslösung mit 20 % Ammoniumchloridlösung (15 mL) versetzt. Die Phasen wurden getrennt und die wässrige Phase wurde mit Ethylacetat (2 × 20 mL) extrahiert. Die vereinigten organischen Phasen wurden mit Natriumsulfat getrocknet und i. Vak. eingeengt. Das Rohprodukt (334 mg) wurde durch Flashchromatographie (32 g, 20 × 2.5 cm) mit Dichlormethan / Methanol [9:1→4:1→4:1 + 1 % Ammoniak (33 % in H₂O)], gereinigt. Danach wurden die Mischfraktionen durch erneute Flashchromatographie (12 g, 18 × 1.6 cm) mit Dichlormethan / Methanol [9:1 + 0.5 % Ammoniak (33 % in H₂O)] gereinigt.

### Beispiel Nr. 16 (ein Diastereomer)

Ausbeute: 185 mg (42 %), weißer Feststoff
Schmelzpunkt: 148-151 °C
¹H-NMR (CDCl₃): 0.89 (t, 3H, J = 7.1 Hz); 1.08-1.35 (m, 10H); 1.71 (br d, 2H, J = 13.0 Hz); 1.98 (t, 2H, J = 6.9 Hz); 2.06 (dt, 2H, J = 13.0 Hz); 2.20 (s, 6H); 3.23-3.31 (m, 2H); 6.85 (s, 1H).
¹³C-NMR (CDCl₃): 14.1; 23.8 (2C); 26.7; 27.3; 28.4; 31.0 (2C); 32.3; 37.1 (2C); 39.0; 43.6; 55.9; 183.5.
LC-MS: m/z: [M+H]⁺= 253.3, Rₜ = 1.5 min.

### Beispiel Nr. 17

### 8-Dimethylamino-8-thiophen-2-yl-3-azaspiro[4.5]decan-4-on (Beispiel Nr. 17, ein Diastereomer)

Zu einer 1 *M* Lösung von 2-Thienylmagnesiumbromid in Tetrahydrofuran (11.5 mL, 11.5 mmol) wurde bei 0 °C und unter Argon eine Lösung von Dimethylamino-1-oxo-2-azaspiro[4.5]decan-8-carbonitril (800 mg, 3.6 mmol) in Tetrahydrofuran (15 mL) getropft und danach über Nacht bei Raumtemperatur gerührt. Anschließend wurde die Reaktionslösung mit 20 % Ammoniumchloridlösung (35 mL) versetzt. Die Phasen wurden getrennt und die wässrige Phase wurde mit Ethylacetat (2 × 30 mL) extrahiert. Die vereinigten organischen Phasen wurden mit Natriumsulfat getrocknet und eingeengt. Das Rohprodukt (950 mg) wurde durch Flashchromatographie (80 g, 17 × 3.7 cm) mit Dichlormethan / Methanol [9:1 + 2 % Ammoniak (33 % in H₂O)] gereinigt.

### Beispiel Nr. 17 (ein Diastereomer)

Ausbeute: 840 mg (84 %), gelblicher Feststoff
Schmelzpunkt: 168-174 °C
¹H-NMR (CDCl₃): 1.26-1.36 (m, 2H); 1.69 (dt, 2H, J = 3.2 und 13.8 Hz); 1.99 (t, 2H, J = 6.9 Hz); 2.10 (s, 6H); 2.20 (dt, 2H, J = 3.2 und 13.1 Hz); 2.45 (br d, 2H, J = 13.6 Hz); 3.25-3.34 (m, 2H); 6.76 (br s, 1H); 6.85 (dd, 1H, J = 1.1 und 3.6 Hz); 7.03 (dd, 1H, J = 3.6 und 5.1 Hz); 7.21 (dd, 1H, J = 1.1 und 5.1 Hz).
¹³C-NMR (CDCl₃): 27.9 (2C); 31.9(2C); 32.5; 38.0 (2C); 38.9; 43.4; 58.4; 122.8; 123.6; 126.0; 145.4; 183.0.
LC-MS: m/z: [M+H]⁺ = 279.2, Rₜ = 1.3 min.

### Beispiel Nr. 18

### Dimethyl-(8-thiophen-2-yl-3-azaspiro[4.5]decan-8-yl)-amin (Beispiel Nr. 18, polares Diastereomer)

Eine Suspension von Lithiumaluminiumhydrid (490 mg, 12.9 mmol) in Tetrahydrofuran (4 mL) wurde bei Raumtemperatur mit einer Lösung von 8-Dimethylamino-8-thiophen-2-yl-3-azaspiro[4.5]decan-4-on (polares Diastereomer) (714 mg, 2.56 mmol) in Tetrahydrofuran (20 mL) versetzt und 18 h bei 60 °C gerührt. Das Reaktionsgemisch wurde auf 0 °C abgekühlt, mit Wasser (0.5 mL), 1 N Natronlauge (1 mL) und erneut Wasser (1 mL) versetzt und anschließend 1 h bei Raumtemperatur gerührt. Der Niederschlag wurde abfiltriert, das Filtrat mit Ethylacetat (20 mL) versetzt und die Phasen wurden getrennt. Die organische Phase wurde mit Natriumsulfat getrocknet und das Lösemittel i. Vak. entfernt. Der Rückstand (570 mg) wurde durch Flashchromatographie (30 g, 19 × 2.5 cm) mit Dichlormethan / Methanol (4:1) und 1 % Ammoniak (25 % in H₂O) gereinigt.

### Beispiel Nr. 18 (polares Diastereomer)

Ausbeute: 280 mg (41 %), weißer öliger Feststoff
Schmelzpunkt: 80-84 °C
¹H-NMR (CDCl₃): 1.38 (ddd, 2H, J =3.6, 13.3 Hz); 1.43-1.50 (m, 1H); 1.58-1.70 (m, 2H); 1.86 -2.01(m, 2H); 2.09 (m, 9H); 2.75 (s, 2H); 2.90 (t, 2H, J = 7.1 Hz); 6.84 (dd, 1H, J = 1.1 und 3.6 Hz); 7.03 (dd, 1H, J = 3.6 und 5.1 Hz); 7.23 (dd, 1H, J =1.1 und 5.1 Hz). Das NH-Proton konnte nicht identifiziert werden.
¹³C-NMR (CDCl₃): 32.8 (2C); 33.7 (2C); 38.1; 39.0; 42.3; 57.8; 59.8; 123.2; 124.9; 126.1; 143.2.
LC-MS: m/z: [M+H]⁺ = 265.2, Rₜ = 0.5 min.

### Beispiel Nr. 19

### Dimethyl-(3-methyl-8-thiophen-2-yl-3-azaspiro[4.5]decan-8-yl)-amin (Beispiel Nr. 19, polares Diastereomer)

Eine Lösung von Dimethyl-(8-thiophen-2-yl-3-azaspiro[4.5]decan-8-yl)-amin (polares Diastereomer) (160 mg, 0.6 mmol) in Methanol (6 mL) wurde mit 37 % wässriger FormalinLösung (1 mL) und Natriumcyanoborhydrid (151 mg, 2.4 mmol) versetzt und 30 min bei Raumtemperatur gerührt. Nach Zugabe von Essigsäure (0.6 mL) wurde 3 h bei Raumtemperatur weitergerührt. Die Reaktionslösung wurde anschließend mit gesättigter Natriumhydrogencarbonat-Lösung (20 mL) verdünnt und mit Dichlormethan (3 × 20 mL) extrahiert. Die vereinigten organischen Phasen wurden mit Natriumsulfat getrocknet, das Lösungsmittel wurde i. Vak. entfernt und der Rückstand (164 mg) durch Flashchromatographie (16 g, 16 × 2 cm) mit Dichlormethan / Methanol (9:1) und 1 % Ammoniak (25 % in H₂O) gereinigt.

### Beispiel Nr. 19 (polares Diastereomer)

Ausbeute: 90 mg (54 %), weißer Feststoff
Schmelzpunkt: 71-72 °C
¹H-NMR (CDCl₃): 1.34-1.43 (m, 2H); 1.52 (t, 2H, J = 6.8 Hz); 1.64-1.75 (m, 2H); 1.84-1.99 (m, 2H); 2.08 (s, 8H); 2.30 (s, 3H); 2.40 (s, 2H); 2.48 (t, 2H, J = 6.8 Hz); 6.83 (dd, 1H, J = 1.0 und 3.5 Hz); 7.02 (dd, 1H, J = 3.5 und 5.1 Hz); 7.21 (dd, 1H, J = 1.0 und 5.1 Hz).
¹³C-NMR (CDCl₃): 33.6(2C); 34.4; 38.1 (2C); 38.6 (br.); 41.7 ; 42.6 (2C); 55.9; 59.6; 68.1 (br.); 74.8; 132.2; 124.9; 126.1; 143.3 (br).
LC-MS: [MH-HNMe₂]⁺: m/z = 234.2 (100 %) und [M+H]⁺: m/z = 279.3 (8 %), Rₜ = 0.2 min.

### Beispiel Nr. 20a

### 1-(8-Dimethylamino-8-thiophen-2-yl-3-azaspiro[4.5]decan-3-yl)-butan-1-on (Beispiel Nr. 20a, polares Diastereomer)

Eine Lösung von Dimethyl-(8-thiophen-2-yl-3-azaspiro[4.5]decan-8-yl)-amin (polares Diastereomer) (142 mg, 0.54 mmol) und Triethylamin (82 mg, 0.81 mmol) in Dichlormethan (5 mL) wurde mit Buttersäurechlorid (69 mg, 68 µL, 0.65 mmol) versetzt und 2.5 h bei Raumtemperatur gerührt. Das Reaktionsgemisch wurde anschließend mit 1 *M* Kaliumcarbonat-Lösung (6 mL) versetzt und 30 min bei Raumtemperatur gerührt. Die Phasen wurden getrennt und die wässrige Phase wurde mit Dichlormethan (2 × 10 mL) extrahiert. Die vereinigten organischen Phasen wurden mit Natriumsulfat getrocknet und i. Vak. eingeengt. Der Rückstand (180 mg) wurde durch Flashchromatographie (18 g, 19 × 2 cm) mit Dichlormethan / Methanol (95:5) gereinigt.

### Beispiel Nr. 20a (polares Diastereomer)

Ausbeute: 140 mg (78 %), farbloses Öl
¹H-NMR (CDCl₃): 0.96 (ddt, 3H, J = 0.6; 4.5 und 7.4 Hz); 1.33 -1.45(m, 2H); 1.57-1.78 (m, 6H); 1.84-2.03 (m, 2H); 2.09 (d, 3H, J = 0.7 Hz); 2.12 (s, 4H); 2.22 (dd, 3H, J = 6.7 und 14.5 Hz); 3.31 (s, 1H); 3.37 (s, 1H); 3.41-3.50 (m, 2H); 6.83-6.88 (m, 1H); 7.01-7.07 (m, 1H); 7.22-7.26 (m, 1H).
¹³C-NMR (CDCl₃): 14.0; 18.3; 18.4; 31.1; 31.2; 32.8; 33.4; 35.6; 36.3; 36.8; 37.1; 38.1; 40.1; 42.9; 45.0; 55.3; 56.4; 59.9; 123.3; 123.6; 124.9; 125.1; 126.1; 126.4; 171.9;.172.0. Einige C-Signale verdoppeln sich auf Grund der Amidstruktur.
LC-MS: [MH-HNMe₂]⁺: m/z = 290.2 (100 %) und [M+H]⁺: m/z = 335.3 (90 %), Rₜ = 2.7 min.

### Beispiel Nr. 20b

### 1-[8-(Dimethyl-amino)-8-thiophen-2-yl-3-azaspiro[4.5]decan-3-yl]-butan-1-on (Beispiel Nr. 20b, unpolares Diastereomer)

Eine Lösung von Dimethyl-(8-thiophen-2-yl-3-azaspiro[4.5]decan-8-yl)-amin (unpolares Diastereomer) (130 mg, 0.49 mmol) und Triethylamin (75 mg, 103 µL, 0.74 mmol) in Dichlormethan (5 mL) wurde mit Buttersäurechlorid (63 mg, 62 µL, 0.59 mmol) versetzt und 1 h bei Raumtemperatur gerührt. Anschließend wurde das Gemisch mit Kaliumcarbonat-Lösung (5 mL) versetzt und 15 min gerührt.
Die Phasen wurden getrennt und die wässrige Phase mit Dichlormethan (3 × 5 mL) nachextrahiert. Die vereinigten organischen Phasen wurden mit Natriumsulfat getrocknet und i. Vak. eingeengt. Das Rohprodukt (160 mg) wurde mittels Flashchromatographie (18 g, 20 × 2.0 cm) mit Dichlormethan / Methanol (95:5) gereinigt.

### Beispiel Nr. 20b (unpolares Diastereoisomer)

Ausbeute: 116 mg (71 %), gelbes Öl
¹H-NMR (CDCl₃): 0.91 (t, 1.5H, J = 7.4 Hz); 0.96 (t, 1.5H, J = 7.4 Hz); 1.37-1.45 (m, 2H); 1.57-1.73 (m, 4H); 1.76 (t, 1H, J = 7.2 Hz); 1.86 (t, 1H, J = 7.2 Hz); 2.00-2.09 (m, 4H); 2.12 (s, 6H); 2.13-2.23 (m, 2H); 3.14 (s, 1H); 3.23 (s, 1H); 3.47 (t, 1H, J = 7.1 Hz); 3.52 (t, 1H, J = 7.1 Hz); 6.84 (dd, 0.5H, J = 0.8, 3.6 Hz); 6.86 (dd, 0.5H, J = 1.1, 3.6 Hz); 7.02 (dd, 0.5H, J = 3.6, 5.2Hz); 7.04 (dd, 0.5H, J = 3.6, 5.2Hz); 7.22 (dd, 0.5H, J = 0.8, 5.1 Hz); 7.24 (dd, 0.5H, J = 1.1, 5.1 Hz).
¹³C-NMR (CDCl₃): 13.97; 13.99; 18.4; 31.0; 33.1; 36.3; 36.7, 38.07; 38.09, 40.1; 42.1; 44.1; 45.2; 56.0; 57.6; 59.9; 123.5; 124.9; 126.21, 126.26; 171.8; 171.9.

Die NMR-Spektren zeigen teilweise doppelte Signalsätze (Rotamere).
LC-MS: m/z: [MH-HNMe₂]⁺ = 290.2 (100 %) und [M+H]⁺ = 335.3 (50 %), Rₜ = 2.7 min.

### Beispiel Nr. 21

### (3-Butyl-8-thiophen-2-yl-3-azaspiro[4.5]decan-8-yl)-dimethyl-amin (Beispiel Nr. 21, polares Diastereomer)

Eine trübe Lösung von Dimethyl-(8-thiophen-2-yl-3-azaspiro[4.5]decan-8-yl)-amin (polares Diastereomer) (137 mg, 0.52 mmol) in Methanol (5 mL) wurde mit Butyraldehyd (49 mg, 61 µL, 0.68 mmol) und Natriumcyanoborhydrid (147 mg, 2.34 mmol) versetzt und 30 min bei Raumtemperatur gerührt. Nach Zugabe von Essigsäure (0.52 mL) wurde noch 2 h bei Raumtemperatur gerührt. Anschließend wurde das Reaktionsgemisch mit Natriumhydrogencarbonat-Lösung (20 mL) verdünnt und mit Dichlormethan (3 × 20 mL) extrahiert. Die vereinigten organischen Phasen wurden mit Natriumsulfat getrocknet und i. Vak. eingeengt. Das Rohprodukt (157 mg) wurde mittels Flashchromatographie (16 g, 16 × 2.0 cm) mit Methanol und 0.5 % Ammoniak (25 % in H₂O) gereinigt.

### Beispiel Nr. 21 (polares Diastereomer)

Ausbeute: 105 mg (63 %), farbloses Öl
¹H-NMR (CDCl₃): 0.91 (t, 3H, J = 7.2 Hz); 1.27-1.53 (m, 8H); 1.64-1.74 (m, 2H); 1.85-1.99 (m, 2H); 2.10 (m, 8H); 2.33-2.40 (m, 2H); 2.42 (s, 2H); 2.50 (t, 2H, J = 6.9 Hz); 6.85 (td, 1H, J = 1.0 und 3.6 Hz); 7.02-7.05 (m, 1H); 7.21-7.24 (m, 1H).
¹³C-NMR (CDCl₃): 14.1; 20.9; 31.0; 33.8; 34.5; 38.2; 40.8; 54.0; 56.8; 59.7; 65.6; 123.2; 124.9; 126.1. Ein Thienyl-C-Signal (ca. 143 ppm) konnte nicht identifiziert werden.
LC-MS: [MH-HNMe₂]⁺: m/z = 276.3 (100 %) und [M+H]⁺: m/z = 321.3 (16 %), Rₜ = 0.3 min.

### Beispiel Nr. 22

### (3-(Cyclopentyl-methyl)-8-thiophen-2-yl-3-azaspiro[4.5]decan-8-yl]-dimethyl-amin (Beispiel Nr. 22, polares Diastereomer)

Eine trübe Lösung von Dimethyl-(8-thiophen-2-yl-3-azaspiro[4.5]decan-8-yl)-amin (polares Diastereomer) (190 mg, 0.72 mmol) in Methanol (6 mL) wurde mit einer Lösung von Cyclopentancarbaldehyd (92 mg, 0.94 mmol) in Methanol (1 mL) und Natriumcyanoborhydrid (204 mg, 3.24 mmol) versetzt und 30 min bei Raumtemperatur gerührt. Nach Zugabe von Essigsäure (0.72 mL) wurde noch 3 h bei Raumtemperatur gerührt. Anschließend wurde das Reaktionsgemisch mit Natriumhydrogencarbonat-Lösung (30 mL) verdünnt und mit Dichlormethan / 2-Propanol (4:1, 2 × 30 mL) und Dichlormethan (30 mL) extrahiert. Die vereinigten organischen Phasen wurden mit Natriumsulfat getrocknet und i. Vak. eingeengt. Das Rohprodukt (357 mg) wurde mittels Flashchromatographie (35 g, 22 × 2.5 cm) mit Methanol und 0.2 % Ammoniak (25 % in H₂O) gereinigt.

### Beispiel Nr. 22 (polares Diastereomer)

Ausbeute: 179 mg (72 %), farbloses Öl
¹H-NMR (CDCl₃): 1.13-1.24 (m, 2H); 1.37 (ddd, 2H, J = 3.5; 10.0 und 13.3 Hz); 1.44-1.63 (m, 6H); 1.64-1.81 (m, 4H); 1.84-2.03 (m, 3H); 2.10 (s, 8H); 2.31 (d, 2H, J = 7.3 Hz); 2.41 (s, 2H); 2.49 (t, 2H, J = 6.9 Hz); 6.85 (dd, 1H, J = 1.1 und 3.6 Hz); 7.03 (dd, 1H, J = 3.6 und 5.1 Hz); 7.22 (dd, 1H, J = 1.1 und 5.1 Hz).
¹³C-NMR (CDCl₃): 25.2 (2C); 31.5 (2C); 33.7 (2C);34.3 (2C); 37.7; 38.2; 39.1; 41.0; 54.3; 59.7; 62.5; 65.6; 123.2; 124.9; 126.1; 143.2.
LC-MS: [MH-HNMe₂]⁺: m/z = 302.3 (100 %) und [M+H]⁺: m/z = 347.3 (30 %), Rₜ = 1.9 min.

### Beispiel Nr. 24a und Beispiel Nr. 24b

### Stufe 1: (4-Dimethylamino-4-thiophen-2-yl-cyclohexyliden)-essigsäureethylester

Eine Lösung von Trietylphosphonoacetat (6.02 g, 5.33 mL, 26.9 mmol) in absolutem *N,N-*Dimethylformamid (30 mL) wurde unter Argon mit Kalium-*tert*-butylat (3.01 g, 26.9 mmol) versetzt. Das Gemisch wurde 10 min bei Raumtemperatur gerührt, bevor es mit einer Lösung von 4-(Dimethylamino)-4-(thiophen-2-yl)cyclohexanon (4.0 g, 17.9 mmol) in absolutem *N,N*-Dimethylformamid (60 mL) versetzt und dann 1 h bei Raumtemperatur gerührt wurde. Anschließend wurde das Reaktionsgemisch in Eiswasser (75 g) gegossen und die wässrige Suspension mit Diethylether (4 × 50 mL) extrahiert. Die vereinigten organischen Phasen wurden mit Natriumsulfat getrocknet und i. Vak. eingeengt.
Ausbeute: 5.20 g (99 %), gelbes Öl
¹H-NMR (CDCl₃): 1.26 (t, 3H, J = 7.1 Hz); 2.03-2.12 (m, 2H); 2.13 (s, 6H); 2.15-2.27 (m, 2H); 2.90-3.09 (m, 4H); 4.13 (q, 2H, J = 7.1 Hz); 5.61 (s, 1H); 6.87 (dd, 1H, J = 1.1, 3.6 Hz); 7.03 (dd, 1H, J = 3.6, 5.1 Hz); 7.23 (dd, 1H, J = 1.1, 5.1 Hz).
LC-MS: m/z: [MH-HNMe₂]⁺ = 249.2 (90 %), Rₜ = 2.8 min.

### Stufe 2: (4-Dimethylamino-1-nitromethyl-4-thiophen-2-yl-cyclohexyl)-essigsäureethylester

Eine Lösung des Rohproduktes von (4-Dimethylamino-4-thiophen-2-yl-cyclohexyliden)-essigsäureethylester (5.20 g, 17.7 mmol) in Tetrahydrofuran (120 mL) wurde mit Tetra-*n-*butylammoniumfluorid-Trihydrat (5.10 g, 19.5 mmol) und Nitromethan (5.40 g, 4.79 mL, 88.5 mmol) versetzt, 3 h bei 70 °C und dann 18 h bei 45 °C gerührt. Anschließend wurde das Reaktionsgemisch i. Vak. eingeengt. Der Rückstand wurde mittels Flashchromatographie (200 g, 20 × 4.0 cm) mit Cyclohexan / Ethylacetat (1:9) gereinigt.
Ausbeute: 4.9 g (78 %), orangefarbenes Öl
¹H-NMR (CDCl₃): 1.20-1.28 (m, 3H); 1.44-1.53 (m, 4H); 1.77-1.88 (m, 4H); 2.09 (s, 6H); 2.46 und 2.61 (2 s, 2H); 4.04-4.22 (m, 2H); 4.62 und 4.77 (s, 2H); 6.82-6.85 (m, 1H); 7.02-7.06 (m, 1H); 7.22-7.25 (m, 1H).
LC-MS: m/z: [M+H]⁺ = 355.2, Rₜ = 2.5 min.
Es handelt sich um ein Diastereoisomerengemisch im Verhältnis von ca. 1:1, das noch mit ca. 15 % Edukt verunreinigt ist.

### Stufe 3: 8-(Dimethyl-amino)-8-thiophen-2-yl-2-azaspiro[4.5]decan-3-on (Beispiel Nr. 30, polares Diastereomer, Beispiel Nr. 31, unpolares Diastereomer)

Eine Lösung des Diastereoisomerengemisches (4-Dimethylamino-1-nitromethyl-4-thiophen-2-yl-cyclohexyl)-essigsäureethylester (4.90 g, 13.8 mmol) in Ethanol (138 mL) wurde zu einem Gemisch von Eisenpulver (3.85 g, 69 mmol) und Ammoniumchlorid (18.5 g, 345 mmol) in Wasser (14 mL) gegeben und 5 h unter Rückfluss erhitzt. Anschließend wurde das Reaktionsgemisch filtriert, das Filtrat mit gesättigter Natriumhydrogencarbonat-Lösung (4 mL) versetzt und i. Vak. eingeengt. Der Rückstand wurde durch Flashchromatographie (200 g, 20 × 5.7 cm) mit Dichlormethan / Methanol (10:1) und 1 % Ammoniak (32 % in Wasser) getrennt.
Ausbeute: 2.33 g (61 %), Diastereoisomerengemisch im Verhältnis von ca. 1:1
Das Diastereoisomerengemisch wurde durch wiederholte Mitteldruckchromatographie (230 g, 3.6 × 46 cm) bzw. Flashchromatographie (100 g, 20 × 4.0 cm) getrennt, wobei als Säulenmaterial sphärisches Kieselgel (PharmPrep 60 CC (40-63 µm) und als Eluent Dichlormethan / Methanol 95:5 und 1 % Ammoniak (32 % in H₂O) verwendet wurde. Das Verhältnis von Proben- zu Kieselgelmasse betrug jeweils ca. 1:200.

### Beispiel Nr. 24a (polares Diastereomer)

Schmelzpunkt: 213-215 °C, weißer Feststoff
¹H-NMR (CDCl₃): 1.47-1.55 (m, 2H); 1.78-1.86 (m, 2H); 1.97-2.09 (m, 4H); 2.10 (s, 6H); 2.12 (s, 2H); 3.23 (s, 2H); 5.69 (br s, 1H); 6.85 (dd, 1H, J = 1.1, 3.6 Hz); 7.05 (dd, 1H, J = 3.6, 5.1 Hz); 7.25 (dd, 1H, J = 1.2, 5.1 Hz).
¹³C-NMR (CDCl₃): 32.6; 32.7, 38.1; 38.8; 43.1; 53.0; 59.3; 123.4; 124.9; 126.3; 142.6; 177.5.
LC-MS: m/z: [MH-HNMe₂]⁺ = 234.2 (100 %) und [M+H]⁺ = 279.2 (16 %), Rₜ = 1.3 min.

### Beispiel Nr. 24b (unpolares Diastereoisomer)

Schmelzpunkt: 213-222 °C, weißer Feststoff
¹H-NMR (CDCl₃): 1.46-1.54 (m, 2H); 1.76-1.84 (m, 2H); 1.93-2.12 (m, 4H); 2.09 (s, 6H); 2.26 (s, 2H); 3.08 (s, 2H); 5.78 (br s, 1H); 6.85 (dd, 1H, J = 1.1, 3.6 Hz); 7.04 (dd, 1H, J = 3.6, 5.1 Hz); 7.24 (dd, 1H, J = 1.1, 5.1 Hz).
¹³C-NMR (CDCl₃): 32.7; 32.8; 38.1; 38.9; 42.5; 53.6; 59.5; 123.4; 124.8; 124.9; 126.3; 142.7; 177.5.
LC-MS: m/z: [MH-HNMe₂]⁺ = 234.2 (100 %) und [M+H]⁺ = 279.2 (22 %), Rₜ = 1.4 min.

### Beispiel Nr. 25

### Dimethyl-(8-thiophen-2-yl-3-azaspiro[4.5]decan-8-yl)-amin (Beispiel Nr. 25, unpolares Diastereomer)

Eine Aufschlämmung von Lithiumaluminiumhydrid (184 mg, 4.85 mmol) in absolutem Tetrahydrofuran (10 mL) wurde unter Eiskühlung mit einer Lösung von 8-Dimethylamino-8-thiophen-2-yl-3-azaspiro[4.5]decan-4-on (unpolares Diastereomer) (270 mg, 0.97 mmol) in absolutem Tetrahydrofuran (15 mL) versetzt und 18 h bei 60 °C gerührt. Anschließend wurde das Gemisch unter Eiskühlung mit Wasser (755 µL), 1 *N* Natronlauge (2.5 mL) und erneut mit Wasser (2.5 mL) versetzt und 1 h bei Raumtemperatur gerührt. Die Suspension wurde durch Seesand filtriert und der Rückstand mit Tetrahydrofuran gewaschen. Das Filtrat wurde mit Natriumsulfat getrocknet und i. Vak. eingeengt. Das Rohprodukt (300 mg) wurde mittels Flashchromatographie (18 g, 20 × 2.0 cm) mit Dichlormethan / Methanol 4:1 + 1 % Ammoniak (25 % in H₂O)→ Methanol + 1 % Ammoniak (25 % in H₂O) gereinigt.

### Beispiel Nr. 25 (unpolares Diastereoisomer)

Ausbeute: 182 mg (71 %), gelbes Öl
¹H-NMR (CDCl₃): 1.37 (ddd, 2H, J = 3.5, 10.1, 13.5 Hz); 1.57-1.65 (m, 4H); 1.89-1.99 (m, 2H); 2.06-2.15 (m, 3H); 2.10 (s, 6H); 2.57 (s, 2H); 2.93 (t, 2H, J = 7.1 Hz); 6.84 (dd, 1H, J = 1.1, 3.6 Hz); 7.02 (dd, 1H, J = 3.6, 5.1 Hz); 7.22 (dd, 1H, J = 1.1, 5.1 Hz).
¹³C-NMR (CDCl₃): 32.9; 33.8; 38.2; 38.4; 42.4; 46.6; 59.4; 59.9; 123.2; 125.0; 126.1; 143.0. LC-MS: m/z: [MH-HNMe₂]⁺ = 220.2 (100 %) und [M+H]⁺ = 265.3 (48 %), Rₜ = 0.2 min.

### Beispiel Nr. 27

### (8-Benzyl-3-butyl-3-azaspiro[4.5]decan-8-yl)-dimethyl-amin (Beispiel Nr. 27, polares Diastereomer)

Eine Lösung von (8-Benzyl-3-azaspiro[4.5]decan-8-yl)-dimethyl-amin (polares Diastereomer) (162 mg, 0.59 mmol) in Methanol (5 mL) wurde mit Natriumcyanoborhydrid (177 mg, 2.82 mmol) und Butyraldehyd (86 mg, 106 µL, 1.19 mmol) versetzt und 30 min bei Raumtemperatur gerührt. Nach Zugabe von Eisessig (600 µL) wurde weitere 2 h bei Raumtemperatur gerührt. Danach wurde gesättigte Natriumhydrogencarbonat-Lösung (20 mL) zugegeben und die Lösung mit Dichlormethan (3 × 20 mL) extrahiert. Die vereinigten organischen Phasen wurden mit Natriumsulfat getrocknet und i. Vak. eingeengt. Das Rohprodukt wurde durch Flashchromatographie (10 g, 20 × 1.5 cm) mit Dichlormethan / Methanol (95:5) und 1 % Ammoniak (25 % in Wasser) gereinigt.

### Beispiel Nr. 27 (polares Diastereoisomer)

Ausbeute: 158 mg (93 %), farbloses Öl
¹H-NMR (CDCl₃): 0.88 (t, 3H, J = 7.3 Hz); 1.06-1.16 (m, 2H); 1.22-1.34 (m, 4H); 1.38-1.47 (m, 2H); 1.60 (t, 2H, J = 6.9 Hz); 1.64-1.74 (m, 4H); 2.21 (s, 2H); 2.29 (s, 6H); 2.35-2.41 (m, 2H); 2.58 (t, 2H, J = 6.9 Hz); 2.62 (s, 2H); 7.10-7.13 (m, 2H); 7.16-7.28 (m, 3H).
¹³C-NMR (CDCl₃): 13.9; 20.7; 30.1; 30.3; 33.0; 36.8; 37.2; 39.7; 40.9; 53.2; 56.7; 57.5; 64.3; 125.6; 127.9; 130.7; 139.3.
LC-MS: m/z: [M+H]⁺ = 329.4, Rₜ = 0.9 min.

### Beispiel Nr. 28

### 1-[8-(Dimethyl-amino)-8-phenyl-3-azaspiro[4.5]decan-3-yl]-butan-1-on (Beispiel Nr. 28, unpolares Diastereomer)

Eine Lösung von N,N-Dimethyl-8-phenyl-3-azaspiro[4.5]decan-8-amin (unpolares Diastereomer) (160 mg, 0.62 mmol) in wasserfreiem Dichlormethan (5 mL) wurde mit Triethylamin (193 mg, 264 µL, 1.9 mmol) und Buttersäurechlorid (134 mg, 132 µL, 1.26 mmol) versetzt und 20 h bei Raumtemperatur gerührt. Nach Zugabe von Dichlormethan (20 mL) wurde die Lösung mit 25 % Kaliumcarbonatlösung (2 × 20 mL) gewaschen, die organische Phase mit Natriumsulfat getrocknet und i. Vak. eingeengt. Das Rohprodukt (205 mg) wurde durch Flashchromatographie (10 g, 20 × 1.5 cm) mit Dichlormethan /

### Beispiel Nr. 28 (unpolares Diastereoisomer)

Methanol (95:5) und 1 % Ammoniak (25 % in Wasser) gereinigt.
Ausbeute: 106 mg (50 %), farbloses Öl
¹H-NMR (CDCl₃): 0.90 und 0.95 (2 t, 3H, J = 7.4 Hz); 1.28-1.39 (m, 2H); 1.56-1.70 (m, 4H); 1.80 und 1.89 (2 t, 2H, J = 7.1 Hz); 1.96-2.04 (m, 2H); 2.05 (s, 6H); 2.09-2.23 (m, 4H); 3.08 und 3.17 (2 s, 2H); 3.45-3.55 (m, 2H); 7.26-7.41 (m, 5H).
¹³C-NMR (CDCl₃): 13.96; 14.0; 18.4; 30.4; 30.5; 31.10; 31.11; 34.3; 36.3; 36.7; 38.0; 40.3; 42.3; 44.1; 45.2; 56.4; 58.0; 60.7; 126.7; 127.4; 127.5; 127.6; 127.7; 136.5; 171.8.
Die NMR-Spektren zeigen teilweise doppelte Signalsätze (Rotamere).
LC-MS: m/z: [M+H]⁺ = 329.4, Rₜ = 2.7 min.

### Beispiel Nr. 29

### 1-[8-(Dimethyl-amino)-8-phenyl-3-azaspiro[4.5]decan-3-yl]-butan-1-on (Beispiel Nr.29, polares Diastereomer)

Eine Lösung von N,N-Dimethyl-8-phenyl-3-azaspiro[4.5]decan-8-amin (polares Diastereomer) (200 mg, 0.77 mmol) in wasserfreiem Dichlormethan (5 mL) wurde mit Triethylamin (235 mg, 322 µL, 2.3 mmol) und Buttersäurechlorid (164 mg, 161 µL, 1.5 mmol) versetzt und 4 h bei Raumtemperatur gerührt. Nach Zugabe von Dichlormethan (20 mL) wurde die Lösung mit 25 % Kaliumcarbonatlösung (2 × 20 mL) gewaschen, die organische Phase mit Natriumsulfat getrocknet und i. Vak. eingeengt. Das Rohprodukt (260 mg) wurde durch Flashchromatographie (10 g, 20 × 1.5 cm) mit Dichlormethan / Methanol (95:5) und 1 % Ammoniak (25 % in Wasser) gereinigt.

### Beispiel Nr. 29 (polares Diastereoisomer)

Ausbeute: 218 mg (86 %), farbloses Öl
¹H-NMR (CDCl₃): 0.97 (t, 3H, J = 7.4 Hz); 1.23-1.35 (m, 2H); 1.52-1.74 (m, 8H); 2.02 und 2.04 (2 s, 6H); 2.18-2.26 (m, 3H); 2.23-2.41 (br s, 1H); 3.34 und 3.41 (2 s, 2H); 3.39-3.48 (m, 2H); 7.23-7.42 (m, 5H).
¹³C-NMR (CDCl₃): 13.7; 14.0; 18.4; 18.5; 18.9; 30.1; 30.9; 31.2; 31.5; 36.4; 36.9; 37.8; 38.0; 38.1; 40.3; 42.3; 43.9; 45.0; 55.1; 56.4; 60.8; 126.5; 126.7; 127.6; 127.63; 127.67; 127.7; 171.9; 172.0.
Die NMR-Spektren zeigen teilweise doppelte Signalsätze (Rotamere).
LC-MS: m/z: [M+H]⁺ = 329.4, Rₜ = 2.8 min.

### Beispiel Nr. 30

### Stufe 1: 8-Cyanomethyl-1,4-dioxaspiro[4.5]decan-8-carbonsäureethylester

Zu einer Lösung von Diisopropylamin (2.78 g, 3.92 mL, 27.5 mmol) in Tetrahydrofuran (50 mL) in einem ausgeheizten Kolben wurde bei -78 °C unter Argon eine 2.5 *M* Lösung von *n-*Butyllithium in *n*-Hexan (11 mL, 27.5 mmol) getropft. Die Reaktionslösung wurde 15 min bei 0 °C gerührt, dann erneut auf -78 °C abgekühlt und innerhalb von 20 min tropfenweise mit einer Lösung von Ethyl-1,4-dioxaspiro[4.5]decan-8-carboxylat (5.36 g, 25 mmol) in Tetrahydrofuran (7.5 mL) versetzt. Das resultierende Gemisch wurde 1.5 h bei -78 °C gerührt und anschließend mit einer Lösung von Bromacetonitril (3.58 g, 1.99 mL, 30 mmol) und 1,3-Dimethyl-3,4,5,6-tetrahydro-2(1*H*) pyrimidon (DMPU, 1.60 g, 1.5 mL, 12.5 mmol) in Tetrahydrofuran (7.5 mL) langsam tropfenweise versetzt. Anschließend wurde die Reaktionslösung innerhalb von ca. 3 h auf Raumtemperatur erwärmt und weitere 20 h bei Raumtemperatur gerührt. Danach wurde das Reaktionsgemisch mit 0.5 *N* Salzsäure (19 mL) versetzt und die Phasen wurden getrennt. Die wässrige Phase wurde mit Diethylether (3 × 50 mL) extrahiert. Die vereinigten organischen Phasen wurden mit Natriumhydrogencarbonat-Lösung (2 × 100 mL) und mit Natriumchlorid-Lösung (4 × 100 mL) gewaschen, mit Natriumsulfat getrocknet und i. Vak. eingeengt. Das Rohprodukt (5.5 g) wurde durch Flashchromatographie (250 g, 27 × 5.4 cm) mit Ethylacetat / Cyclohexan (1:2) gereinigt.
Ausbeute: 3.60 g (57 %), farbloses Öl
¹H-NMR (CDCl₃): 1.29 (t, 3H, J = 7.1 Hz); 1.62-1.76 (m, 6H); 2.17-2.29 (m, 2H); 2.57 (s, 2H); 3.93 (t, 4H, J = 2.2 Hz); 4.23 (q, 2H, J = 7.1 Hz).

### Stufe 2: 1,4-Dioxa-10-aza-dispiro[4.2.4.2]tetradecan-9-on

Zu einer Mischung von 8-Cyanomethyl-1,4-dioxaspiro[4.5]decan-8-carbonsäureethylester (3.6 g, 14.2 mmol) und Cobalt(II)-chlorid (922 mg, 7.1 mmol) in Tetrahydrofuran (50 mL) und Wasser (25 mL) wurde bei 0 °C unter Argon portionsweise Natriumborhydrid (2.68 g, 71 mmol) gegeben. Das Reaktionsgemisch wurde 2 d bei Raumtemperatur gerührt. Anschließend wurde zum Reaktionsgemisch 25 % Ammoniak-Lösung (2.5 mL) gegeben und das entstandene Gemisch filtriert. Der Filterrückstand wurde mit Tetrahydrofuran / Wasser (2:1) gewaschen. Das Filtrat wurde i. Vak. konzentriert und die wässrige Lösung mit Dichlormethan (3 × 50 mL) extrahiert. Die vereinigten organischen Extrakte wurden mit Natriumchlorid-Lösung gewaschen, mit Natriumsulfat getrocknet und i. Vak. eingeengt.
Ausbeute: 2.50 g (83 %), weißer Feststoff, der noch ca. 15 % Edukt enthält.
¹H-NMR (CDCl₃): 1.49-1.64 (m, 5H); 1.82 -1.91(m, 2H); 1.96 -2.04(m, 2H); 2.03-2.08 (m, 2H); 3.29-3.34 (m, 2H); 3.95 (s, 4H); 5.51 (br s, 1H).

### Stufe 3: 3-Azaspiro[4.5]decan-4,8-dion

Eine Lösung von 1,4-Dioxa-10-aza-dispiro[4.2.4.2]tetradecan-9-on (3.48 g, 16.5 mmol) in Aceton (50 ml) wurde mit 5 % wässriger Schwefelsäure (60 ml) versetzt und 1 d bei Raumtemperatur gerührt. Das Reaktionsgemisch wurde mit 1 *M* Kaliumcarbonatlösung (60 ml) versetzt und i. Vak. eingeengt. Die erhaltene wässrige Lösung wurde mit Dichlormethan (4 x 50 mL) extrahiert, die vereinigten organischen Phasen wurden mit gesättigter Natriumchloridlösung (50 ml) gewaschen, mit Natriumsulfat getrocknet und i. Vak. eingeengt.
Ausbeute: 2.72 g (98 %), weißer Feststoff
¹H-NMR (CDCl₃): 1.73-1.89 (m, 2H); 2.08-2.21 (m, 4H); 2.33 (ddd, 2H, J = 5.8, 10.2 und 15.0 Hz); 2.70 (td, 2H, J = 6.3 und 14.8 Hz); 3.41 (dt, 2H, J = 0.8 und 7.1 Hz); 3.72 (s, 1H).

### Stufe 4: Dimethylamino-1-oxo-2-aza-spiro[4.5]decan-8-carbonitril

Zu einer auf 0 °C abgekühlten 40 % wässrigen Dimethylaminlösung (3.3 mL, 26.2 mmol) und Methanol (1.5 mL) wurde 4 *N* Salzsäure (1.97 mL, 7.86 mmol) und anschließend eine Lösung von 3-Azaspiro[4.5]decan-4,8-dion (1.09 mg, 6.55 mmol) in Methanol (12 mL) gegeben. Dieses Gemisch wurde mit Kaliumcyanid (853 mg, 13.1 mmol) versetzt und 20 h bei Raumtemperatur gerührt. Nach Zugabe von Wasser (30 mL) wurde die Lösung mit Diethylether (3 × 30 mL) extrahiert. Die vereinigten organischen Extrakte wurden mit Natriumsulfat getrocknet und eingeengt, wodurch das Produkt (390 mg) isoliert wurde. Anschließend wurde die wässrige Lösung mit Dichlormethan (3 × 20 mL) nachextrahiert. Die vereinigten Dichlormethan-Extrakte wurden mit Natriumsulfat getrocknet und eingeengt, wodurch 820 mg des Produktes erhalten wurden.
Ausbeute: 1.21 g (83 %), weißer Feststoff
¹H-NMR (CDCl₃): 1.35-1.67 (m, 3H); 1.76-2.09 (m, 5H); 2.18-2.31 (m, 2H); 2.33 und 2.35 (2s, 6H); 3.28-3.35 (m, 2H); 6.50 und 6.60 (2s, 1H). Es handelt sich um ein Diastereoisomerengemisch im Verhältnis von ca. 2:1.

### Stufe 5: 8-(Dimethyl-amino)-8-(5-methyl-thiophen-2-yl)-3-azaspiro[4.5]decan-4-on

### (Beispiel Nr. 42, polares Diastereomer)

In einer sekurierten Apparatur wurden unter Argon Magnesium (292 mg, 12 mmol) und ein lodkristall erhitzt bis sich lodgas entwickelte. Nach 10 min wurde absoluter Diethylether (20 mL) und ein weiterer lodkristall zugefügt. Das Gemisch wurde zum Sieden erhitzt und dann langsam eine Lösung von 2-Brom-5-methylthiophen (2.12 g, 1.35 mL, 12 mmol) in absolutem Diethylether (20 mL) so zugetropft, dass die Lösung weiter siedete. Nach Beendigung der Zugabe wurde noch 30 min unter Rückfluss erhitzt und die Lösung dann abgekühlt auf 0°C.
Zu dieser eisgekühlten Lösung wurde unter Argon langsam eine Lösung von Dimethylamino-1-oxo-2-aza-spiro[4.5]decan-8-carbonitril (1.06 g, 4.8 mmol) in absolutem Tetrahydrofuran (50 mL) getropft und anschließend über Nacht bei Raumtemperatur gerührt. Nach Zugabe von gesättigter Ammoniumchlorid-Lösung (50 mL) wurde i. Vak. das Tetrahydrofuran entfernt. Die erhaltene wässrige Lösung wurde mit Dichlormethan (3 × 50 mL) extrahiert, die vereinigten organischen Phasen wurden mit gesättigter Natriumchlorid-Lösung (50 mL) gewaschen, mit Natriumsulfat getrocknet und i. Vak. eingeengt. 160 mg des Rohproduktes (1.56g) wurden zwecks Abgabe mittels Flashchromatographie (10g, 20 × 1.5 cm) mit Ethylacetat / Methanol (4:1) gereinigt, wodurch 139 mg reine Zielverbindung erhalten wurden. Es handelt sich um das polare Diastereoisomer. Der Rest wurde als Rohprodukt weiter umgesetzt.

### Beispiel Nr. 30 (polares Diastereoisomer)

Ausbeute: 1.56 g (Rohprodukt)
Schmelzpunkt: 173-176 °C
¹H-NMR (CDCl₃): 1.27-1.33 (m, 2H); 1.66 (dt, J = 12.9, 3.2 Hz, 2H); 2.00 (t, J = 6.9 Hz, 2H); 2.11 (s, 6H); 2.18 (dt, J = 13.2, 3.1 Hz, 2H); 2.36-2.43 (m, 2H); 2.46 (s, 3H); 3.27-3.31 (m, 2H); 6.21 (br s, 1H); 6.62 (d, J = 3.5 Hz, 1H); 6.65-6.67 (m, 1H).
¹³C-NMR (CDCl₃): 15.2; 28.0; 31.8; 32.6; 37.9; 38.7; 43.3; 58.5; 123.6; 124.7; 137.2; 143.1; 182.8.
LC-MS: m/z: [MH-HNMe₂]⁺ = 248.2, Rₜ = 2.5 min.

### Beispiel Nr. 31

### Dimethyl-[8-(5-methyl-thiophen-2-yl)-3-azaspiro[4.5]decan-8-yl]-amin

### (Beispiel Nr. 31, polares Diastereomer)

In einer ausgeheizten Apparatur wurde eine Aufschlämmung von Lithiumaluminiumhydrid (456 mg, 12 mmol) in wasserfreiem Tetrahydrofuran (20 mL) unter Eiskühlung mit einer Lösung von 8-(Dimethyl-amino)-8-(5-methyl-thiophen-2-yl)-3-azaspiro[4.5]decan-4-on (polares Diastereomer) (1.40 g, 4.8 mmol) in wasserfreiem Tetrahydrofuran (100 mL) versetzt und anschließend bei 60 °C über Nacht gerührt. Die Reaktionslösung wurde unter Eiskühlung mit Wasser (857 µL), 1 *N* Natronlauge (2.1 mL) und erneut mit Wasser (2.1 mL) versetzt und 1 h bei Raumtemperatur gerührt. Das Gemisch wurde durch Seesand filtriert und der Rückstand mit Tetrahydrofuran gewaschen. Das Filtrat wurde mit Natriumsulfat getrocknet und i. Vak. eingeengt. 160 mg des Rohproduktes (1.18 g) wurden zwecks Abgabe mittels Flashchromatographie (10 g, 20 × 1.5 cm) mit Ethylacetat / Methanol (4:1) )→Methanol + 1 % Ammoniak (25 % in Wasser) gereinigt, wodurch 80 mg Zielverbindung erhalten wurden, die immer noch minimale Verunreinigungen enthielt.

### Beispiel Nr. 31 (polares Diastereoisomer)

Ausbeute: 1.18 g (Rohprodukt), gelbes zähes Öl
¹H-NMR (CDCl₃): 1.37-1.41 (m, 2H); 1.47 (t, J = 7.1 Hz, 2H); 1.57-1.65 (m, 2H); 1.85-1.91 (m, 2H); 2.00-2.16 (m, 2H, überlagert); 2.11 (s, 6H); 2.47 (s, 3H); 2.75 (s, 2H); 2.91 (t, J = 7.1 Hz, 2H); 6.62 (d, J = 3.5 Hz, 1H); 6.67-6.68 (m, 1H). Das NH-Proton konnte nicht identifiziert werden.
¹³C-NMR (CDCl₃): 15.3; 32.9; 33.6; 38.2; 42.4; 46.1; 57.9; 59.9; 64.2; 124.3; 124.9; 137.6; 140.8.
LC-MS: m/z: [MH-HNMe₂]⁺ = 234.2, Rₜ = 0.7 min.

### Beispiel Nr. 32

### 1-[8-(Dimethyl-amino)-8-(5-methyl-thiophen-2-yl)-3-azaspiro[4.5]decan-3-yl]-butan-1-on

### (Beispiel Nr. 32, polares Diastereomer)

Eine Lösung von 8-(Dimethyl-amino)-8-(5-methyl-thiophen-2-yl)-3-azaspiro[4.5]decan-4-on (polares Diastereomer) (200 mg, 0.72 mmol) und Triethylamin (110 mg, 152 µL, 1.1 mmol) in absolutem Dichlormethan (10 mL) wurde mit Buttersäurechlorid (91 mg, 90 µL, 0.86 mmol) versetzt und 2 h bei Raumtemperatur gerührt. Das Reaktionsgemisch wurde anschließend mit 1 *M* Kaliumcarbonat-Lösung auf pH 9-10 eingestellt und 15 min gerührt. Die Phasen wurden getrennt und die wässrige Phase mit Dichlormethan (3 × 50 mL) extrahiert. Die vereinigten organischen Phasen wurden mit Natriumsulfat getrocknet und i. Vak. eingeengt. Der Rückstand (230 mg) wurde durch Flashchromatographie an sphärischem Kieselgel (PharmPrep 60 CC, 40-63 mm, 10 g, 20 × 1.5 cm) mit Ethylacetat / Methanol (4:1) gereinigt.

### Beispiel Nr. 32 (polares Diastereoisomer)

Ausbeute: 153 mg (63 %), farbloses zähes Öl
¹H-NMR (CDCl₃): 0.94-0.98 (m, 3H); 1.35-1.46 (m, 2H); 1.58-1.71 (m, 6H); 1.81-1.95 (m, 2H); 2.10 (s, 2H); 2.13 (s, 4H); 2.19 (m, 4H); 2.46 (d, J = 1.0 Hz, 1H); 2.47 (d, J = 1.0 Hz, 2H); 3.30 (s, 1.3H); 3.36 (s, 0.7H); 3.42-3.49 (m, 2H); 6.61 (d, J = 3.5 Hz, 0.3H); 6.62 (d, J = 3.5 Hz, 0.7H); 6.66 (dd, J = 3.4; 1.1 Hz, 0.3H); 6.69 (dd, J = 3.4; 1.1 Hz, 0.7H).
¹³C-NMR (CDCl₃): 14.0; 15.3; 18.3; 18.5; 31.2; 31.3; 32.6; 33.3; 36.3; 36.3; 36.9; 37.3; 38.1; 40.1; 42.0; 43.9; 45.0; 55.2; 56.2; 60.1; 60.4; 124.3, 124.6, 124.9; 125.2; 137.7, 138.0; 171.8; 171.9.
Die NMR-Spektren zeigen teilweise doppelte Signalsätze (Rotamere).
LC-MS: m/z: [MH-HNMe₂]⁺= 304.3, Rₜ 3.0 min.

### Beispiel Nr. 33

### 3-Butyl-8-(dimethyl-amino)-8-thiophen-2-yl-3-azaspiro[4.5]decan-2-on

### (Beispiel Nr. 33, unpolares Diastereomer)

Eine Mischung von 8-(Dimethyl-amino)-8-thiophen-2-yl-2-azaspiro[4.5]decan-3-on (unpolares Diastereoisomer) (200 mg, 0.72 mmol) und Kalium-*tert*-butylat (92 mg, 0.82 mmol) in *N,N*-Dimethylformamid (5 mL) wurde 40 min bei Raumtemperatur gerührt, bevor es mit lodbutan (151 mg, 94 µL, 0.82 mmol) versetzt und weitere 18 h bei Raumtemperatur gerührt wurde. Anschließend wurde das Reaktionsgemisch mit Ethylacetat (50 mL) verdünnt und mit Wasser (3 × 10 mL) gewaschen. Die organische Phase wurde mit Natriumsulfat getrocknet und i. Vak. eingeengt. Das Rohprodukt (190 mg) wurde mittels Flashchromatographie (10 g, 20 × 1.5·cm) mit Dichlormethan / Methanol 95:5 + 1 % Ammoniak (25 % in H₂O) gereinigt.

### Beispiel Nr. 33 (unpolares Diastereoisomer)

Ausbeute: 130 mg (54 %), gelbes Öl
¹H-NMR (CDCl₃): 0.89 (t, 3H, J = 7.3 Hz); 1.22-1.33 (m, 2H); 1.39-1.50 (m, 4H); 1.70-1.79 (m, 2H); 1.92-2.08 (m, 4H); 2.10 (s, 6H); 2.32 (s, 2H); 3.04 (s, 2H); 3.22 (t, 2H, J = 7.3 Hz); 6.84 (dd, 1H, J = 1.1, 3.6 Hz); 7.04 (dd, 1H, J = 3.6, 5.1 Hz); 7.24 (dd, 1H, J = 1.1, 5.1 Hz).
¹³C-NMR (CDCl₃): 13.7; 20.0; 29.3; 32.7; 32.9; 35.6; 38.1; 42.0; 43.8; 58.6; 59.5; 123.4; 124.9; 126.2; 142.8; 173.5.
LC-MS: m/z: [MH-HNMe₂]⁺ = 290.3 (100 %) und [M+H]⁺ = 335.3 (33 %), Rₜ = 2.9 min.

### Beispiel Nr. 34

### 8-(Dimethyl-amino)-3-methyl-8-thiophen-2-yl-3-azaspiro[4.5]decan-2-on

### (Beispiel Nr. 34, unpolares Diastereomer)

Eine Mischung von 8-(Dimethyl-amino)-8-thiophen-2-yl-2-azaspiro[4.5]decan-3-on (unpolares Diastereoisomer) (200 mg, 0.72 mmol) und Kalium-*tert*-butylat (92 mg, 0.82 mmol) in *N,N*-Dimethylformamid (5 mL) wurde 40 min bei Raumtemperatur gerührt, bevor es mit Methyliodid (116 mg, 51 µL, 0.82 mmol) versetzt und weitere 5 h bei Raumtemperatur gerührt wurde. Da die Umsetzung nicht vollständig war, wurden nochmals Kalium-*tert*-butylat (40 mg, 0.36 mmol) und Methyliodid (58 mg, 25 µL, 0.41 mmol) zugesetzt und das Gemisch weitere 18 h bei Raumtemperatur gerührt. Anschließend wurde das Reaktionsgemisch mit Ethylacetat (50 mL) verdünnt und mit Wasser (3 × 10 mL) gewaschen. Die organische Phase wurde mit Natriumsulfat getrocknet und i. Vak. eingeengt. Das Rohprodukt wurde mittels Flashchromatographie (10 g, 20 × 1.5 cm) mit Dichlormethan / Methanol 95:5 + 1% Ammoniak (25 % in H₂O) gereinigt.

### Beispiel Nr. 34 (unpolares Diastereoisomer)

Ausbeute: 124 mg (59 %), weißer Feststoff
Schmelzpunkt: 88-94 °C
¹H-NMR (CDCl₃): 1.40-1.49 (m, 2H); 1.68-1.78 (m, 2H); 1.90-2.07 (m, 4H); 2.08 (s, 6H); 2.29 (s, 2H); 2.77 (s, 3H); 3.04 (s, 2H); 6.82 (dd, 1H, J = 0.9, 3.4 Hz); 7.02 (dd, 1H, J = 3.6, 5.0 Hz); 7.21 (dd, 1H, J = 0.8, 5.1 Hz).
¹³C-NMR (CDCl₃): 29.5; 32.6; 32.9; 35.3; 38.1; 43.4; 59.4; 61.0; 123.4; 124.9; 126.2; 142.7; 173.7.
LC-MS: m/z: [MH-HNMe₂]⁺ = 248.2 (100 %) und [M+H]⁺ = 293.3 (50 %), Rₜ = 2.2 min.

### Beispiel Nr. 35

### [3-Butyl-8-(5-methyl-thiophen-2-yl)-3-azaspiro[4.5]decan-8-yl]-dimethyl-amin

### (Beispiel Nr. 35, polares Diastereomer)

Eine Lösung von Dimethyl-[8-(5-methyl-thiophen-2-yl)-3-azaspiro[4.5]decan-8-yl]-amin (polares Diastereomer) (180 mg, 0.65 mmol) in absolutem Methanol (5 mL) wurde nacheinander mit Butyraldehyd (61 mg, 75 µL, 0.84 mmol), Essigsäure (650 µL) und Natriumcyanoborhydrid (184 mg, 2.9 mmol) versetzt und 4 h bei Raumtemperatur gerührt. Das Reaktionsgemisch wurde anschließend mit gesättigter Kaliumhydrogencarbonat-Lösung (30 mL) versetzt und mit Dichlormethan / 2-Propanol (4:1) extrahiert (3 × 30 mL). Die vereinigten organischen Phasen wurden mit gesättigter Natriumchlorid-Lösung (50 mL) gewaschen, mit Natriumsulfat getrocknet und i. Vak. eingeengt. Der Rückstand (186 mg) wurde durch Flashchromatographie an sphärischem Kieselgel (PharmPrep 60 CC, 40-63 mm, 10 g, 20 × 1.5 cm) mit Methanol, welches 1 % Ammoniak (25 % in H₂O) enthielt, gereinigt.

### Beispiel Nr. 35 (polares Diastereoisomer)

Ausbeute: 106 mg (49 %), farbloses zähes Öl
¹H-NMR (CDCl₃): 0.91 (t, J = 7.3 Hz, 3H); 1.27-1.52 (m, 8H); 1.62-1.69 (m, 2H); 1.78-1.93 (m, 2H); 2.02-2.05 (m, 2H); 2.11 (s, 6H); 2.34-2.38 (m, 2H); 2.41 (s, 2H); 2.46 (d, J = 1.1 Hz, 3H); 2.49 (d, J = 6.9 Hz, 2H); 6.61 (d, J = 3.5 Hz, 1H); 6.67 (qd, J = 3.3, 1.0 Hz, 1H).
¹³C-NMR (CDCl₃): 14.1; 15.3; 20.9; 31.1; 33.6; 34.6; 38.2; 40.8, 54.0; 56.8; 59.8: 65.6; 124.3; 124.9; 137.5.
LC-MS: m/z: [M+H]⁺= 335.2, Rₜ = 1.6 min.

### Beispiel Nr. 41

### 1-[8-Dimethylamino-8-(5-methyl-thiophen-2-yl)-2-aza-spiro[4.5]dec-2-yl]-2-methoxy-ethanon

Dimethyl-[8-(5-methyl-thiophen-2-yl)-3-azaspiro[4.5]decan-8-yl]-amin (Beispiel Nr. 31) (0.28 g, 1.00 mmol) und Triethylamin (0.33 mL, 2.40 mmol) wurden unter Argon in abs. THF (5 mL) gelöst, mit Methoxyacetylchlorid (0.11 mL, 0.13 g, 1.20 mmol) versetzt und bei Raumtemperatur 1 d gerührt. Zur Aufarbeitung wurde der Reaktionsansatz i. Vak. zur Trockene eingeengt, der Rückstand in Dichlormethan aufgenommen, mit gesättigter NaHCO3-Lösung (2x 25 mL) und Wasser (2x 25 mL) gewaschen. Die organische Phase wurde über Na2SO4 getrocknet, filtriert und i. Vak. eingeengt. Der Rückstand wurde mittels Puriflash 430 und Interchim-Kartusche 15 µm x 25 g mit Chloroform/Methanol (100:0→0:100) gereinigt.
Ausbeute: 120 mg (34 %)
1H-NMR (CDCl3): 1.34-1.41 (2 H, m); 1.53-1.68 (4 H, m); 1.94 (2 H, m); 2.10 (2 H, s); 2.17 (6 H, s); 2.44 (3 H, m); 3.27 (1H, s); 3.36-3.41 (4 H, m); 3.47 (2 H, t); 3.97 (2 H, d); 6.64 (2 H, m).

### Stufe 2: [3-(2-Methoxy-ethyl)-8-(5-methyl-thiophen-2-yl)-3-azaspiro[4.5]decan-8-yl]-dimethylamin (Beispiel Nr. 58, polares Diastereomer)

Eine Lösung von 1-[8-Dimethylamino-8-(5-methyl-thiophen-2-yl)-2-aza-spiro[4.5]dec-2-yl]-2-methoxy-ethanon (0.12 g, 0.34 mmol) in abs. THF (5 mL) wurde unter Argon mit LiAlH₄ (26 mg, 0.68 mmol) versetzt und unter Rückfluss 1 h gerührt. Zur Aufarbeitung wurde der Reaktionsansatz bei 0 °C mit einigen Tropfen Wasser hydrolysiert. Anschließend wurde die Suspension 1 h nachgerührt. Die Lösung wurde über eine Seesand-Fritte filtriert, der Sand mit THF und Dichlormethan nachgewaschen und die vereinigten Lösung i. Vak. eingeengt.

### Beispiel Nr. 41 (polares Diastereoisomer)

Ausbeute: 62 mg (54 %)
1H-NMR (CDCl3): 1.34-1.42 (2 H, m); 1.50 (2 H, t); 1.66 (2 H, m); 1.81 (2 H, bs); 2.05 (1H, m); 2.09 (6 H, s); 2.45 (5 H, s); 2.54 (2 H, t); 2.60 (2 H, t); 3.33 (3 H, s); 3.46 (2 H, t); 6.60 (1 H, m); 6.65 (1H, m).
LC-MS: m/z: [M+H]⁺= 337.2, Rₜ = 1.1 min.

### Beispiel Nr. 48

### 2-Cyclopropyl-1-[8-(dimethyl-amino)-8-thiophen-2-yl-3-azaspiro[4.5]decan-3-yl]-ethanon

### (Beispiel Nr. 48, polares Diastereomer)

Eine Lösung von Cyclopropylessigsäure (180 mg, 174 µL, 1.8 mmol) in absolutem Tetrahydrofuran (20 mL) wurde mit Carbonyldiimidazol (365 mg, 2.25 mmol) ver-setzt und 2 h unter Rückfluss gerührt (CO2-Entwicklung). Die Lösung wurde mit einer Lösung von Dimethyl-(8-thiophen-2-yl-3-azaspiro[4.5]decan-8-yl)-amin (Beispiel Nr. 18) (397 mg, 1.5 mmol) in Tetrahydrofuran (10 mL) versetzt und 2 h unter Rückfluss gerührt. Anschließend wurde das Reaktionsgemisch i. Vak. ein-geengt, der Rückstand in Ethylacetat (30 mL) gelöst und die Lösung mit Wasser (3 × 20 mL) gewaschen. Die organische Phase wurde mit Natriumsulfat getrocknet und i. Vak. eingeengt. Das Rohprodukt wurde mittels Flashchromatographie (18 g, 20 × 2.0 cm) mit Dichlormethan / Methanol (95:5) gereinigt.

### Beispiel Nr. 48 (polares Diastereoisomer)

Ausbeute: 280 mg (54 %), braunes Öl
1H-NMR (CDCl3): 0.12-0.19 (2 H, m); 0.55 (2 H, ddd, J = 8.1, 5.8 und 4.6 Hz); 1.03-1.14 (1 H, m); 1.34-1.45 (2 H, m); 1.58-1.63 (1.4 H, m); 1.64-1.72 (3 H, m); 1.85-2.03 (2.6 H, m); 2.09 (3 H, s); 2.13 (3 H, br s); 2.17-2.20 (3 H, m); 3.29 (1.2 H, s), 3.38 (0.8 H, s); 3.41 (0.8 H, t, J = 7.2 Hz); 3.49 (1.2 H, t, J = 7.3 Hz); 6.85 (0.4 H, dd, J = 3.6 und 1.1 Hz); 6.86-6.88 (0.6 H, m); 7.03 (0.4 H, dd, J = 5.1 und 3.5 Hz); 7.06 (0.6 H, dd, J = 5.1 und 3.6 Hz); 7.23 (0.4 H, dd, J = 5.1 und 1.1 Hz); 7.24-7.28 (0.6 H, m).
13C-NMR (CDCl3): 4.4; 4.4; 6.8; 6.9; 31.1; 31.2; 32.8; 33.0; 33.3; 35.6; 37.2; 38.0; 38.1; 39.5; 39.9; 40.0; 42.0; 43.9; 45.0; 55.3; 56.4; 60.0; 123.4; 123.8; 124.9; 125.4; 126.1; 126.4; 143.5; 171.4; 171.5.
Die NMR-Spektren zeigen teilweise einen doppelten Signalsatz (Rotamere).
LC-MS: m/z: [MH-HNMe2]+ = 302.3 (100 %) und [M+H]+ = 347.3 (50 %), Rt = 2.8 min.

### Beispiel Nr. 92

### 8-(Cyclohexyl-methyl)-8-dimethylamino-3-azaspiro[4.5]decan-4-on (Beispiel Nr. 92, ein Distereoisomer)

Eine Lösung von 8-(Dimethylamino)-1-oxo-2-azaspiro[4.5]decane-8-carbonitril (2 g, 9.03 mmol) in wasserfreiem Tetrahydrofuran (75 mL) wurde bei 0 °C tropfenweise mit einer 0.5 M Lösung von Cyclohexylmethyl-Magnesiumbromid in Tetrahydrofuran (63.2 mL, 31.6 mmol) versetzt und 18 h bei Raumtemperatur gerührt. Das Gemisch wurde anschließend unter Eiskühlung mit gesät-tigter Ammoniumchlorid-Lösung (90 mL) versetzt. Die Phasen wurden getrennt und die wässrige mit Ethylacetat (2 x 25 mL) extrahiert. Die vereinigten organischen Phasen wurden mit Natriumsulfat getrocknet und i. Vak. eingeengt. Der Rückstand (2.4 g) wurde durch Flashchromatographie (200 g, 20 x 5.7 cm) mit Dichlormethan / Methanol (98:2) und 1 % Ammoniak (25 % in Wasser) gereinigt.

### Beispiel Nr. 92 (ein Diastereoisomer)

Ausbeute: 1.20 g (46 %), weißer Feststoff
Schmelzpunkt: 190-193 °C
1H-NMR (CDCl3): 0.88-1.00 (2 H, m); 1.06-1.27 (8 H, m); 1.32 (2 H, dt, J = 14.1 und 3.4 Hz); 1.54-1.74 (7 H, m); 2.03 (2 H, t, J = 7.0 Hz); 2.08 (2 H, dt, J = 13.2 und 3.2 Hz); 2.16 (6 H, s); 3.26-3.31 (2 H, m); 6.04 (1H, br s).
13C-NMR (CDCl3): 26.2; 26.7; 27.0; 28.9; 32.0; 32.9; 33.5; 36.0; 36.9; 37.7; 38.1; 38.8; 43.8; 56.4; 183.5.
Es wurde nur ein Diastereoisomer isoliert.
LC-MS: m/z: [M+H]⁺ = 293.2, geringe UV-Aktivität.

### Beispiel Nr. 93

### Stufe 1: (8-Cyclohexylmethyl-2-aza-spiro[4.5]dec-8-yl)dimethylamin

Eine Suspension von Lithiumaluminiumhydrid (683 mg, 18 mmol) in wasserfreiem Tetrahydrofuran (20 mL) wurde unter Eiskühlung tropfenweise mit einer Lösung von 8-(Cyclohexyl-methyl)-8-dimethylamino-3-azaspiro[4.5]decan-4-on (Beispiel Nr. 158, ein Distereoisomer) (1.05 g, 3.59 mmol) in wasserfreiem Tetrahydrofuran (40 mL) versetzt. Das Gemisch wurde 18 h bei 50 °C gerührt, anschließend unter Eiskühlung tropfenweise mit Wasser (700 µL), 1 N Natronlauge (1.4 mL) und erneut Wasser (1.4 mL) versetzt. Die Suspension wurde 1 h bei Raumtemperatur gerührt und danach durch Natriumsulfat filtriert. Der Filterrückstand wurde mit Tetrahydrofuran gewaschen und das Filtrat i. Vak. eingeengt.
Ausbeute: 884 mg (99 %), farbloses Öl
1H-NMR (CDCl₃): 0.89-1.01 (2 H, m); 1.06-1.45 (9 H, m); 1.50-1.74 (10 H, m); 1.80-1.90 (2 H, m); 2.17 (6 H, s); 2.64 (2 H, s); 2.94 (2 H, t, J = 7.1 Hz). Das NH-Proton konnte nicht identifiziert werden.

### Stufe 2: 1-[8-(Cyclohexyl-methyl)-8-dimethylamino-3-azaspiro[4.5]decan-3-yl]-butan-1-on

### (Beispiel Nr. 159, ein Diastereomer)

Eine Lösung von (8-Cyclohexylmethyl-2-aza-spiro[4.5]dec-8-yl)dimethylamin (420 mg, 1.5 mmol) und Triethylamin (230 mg, 315 µL, 2.26 mmol) in wasserfreiem Dichlormethan (15 mL) wurde mit Buttersäurechlorid (193 mg, 190 µL, 1.80 mmol) versetzt und über Nacht bei Raumtemperatur gerührt. Das Gemisch wurde anschließend mit 25 % Kaliumcarbonat-Lösung (15 mL) versetzt und 15 min bei Raumtemperatur gerührt. Die Phasen wurden getrennt und die wässrige mit Dichlormethan (3 x 5 mL) extrahiert. Die vereinigten organischen Phasen wurden mit Natriumsulfat getrocknet und i. Vak. eingeengt. Der Rückstand (515 mg) wurde durch Flashchromatographie (38 g, 20 x 2.8 cm) mit Dichlormethan / Methanol (95:5) und 1 % Ammoniak (25 % in Wasser) gereinigt.

### Beispiel Nr. 93 (ein Diastereoisomer)

Ausbeute: 448 mg (85 %), farbloses Öl
1H-NMR (CDCl3): 0.88-1.01 (5 H, m); 1.05-1.46 (10 H, m); 1.52-1.91 (13 H, m); 2.157 und 2.164 (6 H, 2 s); 2.12-2.25 (2 H, m); 3.17 (1.2 H, s); 3.25 (0.8 H, s); 3.46 (0.8 H, t, J = 7.1 Hz); (1.2 H, t, J = 7.2 Hz).
13C-NMR (CDCl3): 14.0; 18.4; 18.5; 26.2; 26.5; 28.7; 29.2; 29.8; 30.3; 33.1; 33.2; 35.9; 36.1; 36.2; 36.8; 37.1; 37.7; 37.9; 40.3; 42.0; 44.3; 45.2; 57.1; 57.2; 59.5; 171.77; 171.84.
Die NMR-Spektren zeigen teilweise doppelte Signalsätze (Rotamere).
LC-MS: m/z: [M+H]⁺= 349.3, geringe UV-Aktivität.

### Beispiel Nr. 95

### Stufe 1: 8-Cyclopentylmethyl-8-dimethylamino-2-aza-spiro[4.5]decan-1-on

Die Lösung von Cyclopentylmethyl-Magnesiumiodid (ca. 32 mmol) wurde unter Argonatmosphäre bei 0 °C mit einer Lösung von 8-(Dimethylamino)-1-oxo-2-azaspiro[4.5]decane-8-carbonitril (1.96 g, 8.8 mmol) in wasserfreiem Tetrahydrofuran (40 mL) tropfenweise versetzt. Das Reaktionsgemisch wurde 18 h bei Raumtemperatur gerührt und anschließend unter Eiskühlung mit gesättigter Ammoniumchlorid-Lösung (80 mL) versetzt. Die Phasen wurden getrennt und die wässrige Phase mit Ethylacetat (2 x 30 mL) extrahiert. Die vereinigten organischen Phasen wurden mit Natriumsulfat getrocknet und i. Vak. eingeengt. Der Rückstand (1.88 g) wurde durch Flashchromatographie (100 g, 20 x 4.0 cm) mit Dichlormethan / Methanol (95:5) und 1 % Ammoniak (25 % in Wasser) gereinigt.
Ausbeute: 519 mg (21 %), weißer Feststoff
1H-NMR (CDCl3): 0.98-1.10 (2 H, m); 1.10-1.17 (2 H, m); 1.30-1.40 (4 H, m); 1.42-1.84 (9 H, m); 2.01 (2 H, t, J = 6.9 Hz); 2.17 (6 H, s); 3.28 (2 H, dd, J = 13.9 und 0.8 Hz); 6.51 (1H, s).
13C-NMR (CDCl3): 25.2; 27.2; 29.1; 32.2; 35.3; 36.1; 36.9; 38.9; 43.8; 56.2; 183.3.

### Stufe 2: (8-Cyclopentylmethyl-2-aza-spiro[4.5]dec-8-yl)dimethylamin

Eine Suspension von Lithiumaluminiumhydrid (368 mg, 9.7 mmol) in wasserfreiem Tetrahydrofuran (10 mL) wurde unter Eiskühlung tropfenweise mit einer Lösung von 8-Cyclopentylmethyl-8-dimethylamino-2-aza-spiro[4.5]decan-1-on (539 mg, 1.93 mmol) in wasserfreiem Tetrahydrofuran (20 mL) versetzt. Das Gemisch wurde 18 h bei 50 °C gerührt, danach unter Eiskühlung tropfenweise mit Wasser (377 µL), 1 N Natronlauge (754 µL) und erneut Wasser (754 µL) versetzt. Die Suspension wurde 1 h bei Raumtemperatur gerührt, anschließend durch Natriumsulfat filtriert, der Filterrückstand mit Tetrahydrofuran gewaschen und das Filtrat i. Vak. eingeengt.
Ausbeute: 463 mg (90 %), farbloses Öl
1H-NMR (CDCl3): 1.00-1.12 (2 H, m); 1.17-1.27 (2 H, m); 1.31-1.95 (17 H, m); 2.18 (6 H, s); 2.64 (2 H, s); 2.93 (2 H, t, J = 7.0 Hz). Das NH-Signal konnte nicht identifiziert werden. 13C-NMR (CDCl3): 25.0; 29.8; 31.8; 35.1; 36.0; 36.7; 37.2; 37.4; 42.6; 46.6; 56.9; 60.7.

### Stufe 3

### [3-Butyl-8-(cyclopentyl-methyl)-3-azaspiro[4.5]decan-8-yl]-dimethyl-amin (Beispiel Nr. 164, ein Diastereomer)

Eine Lösung von (8-Cyclopentylmethyl-2-aza-spiro[4.5]dec-8-yl)dimethylamin (237 mg, 0.89 mmol) und Triethylamin (136 mg, 187 µL, 1.34 mmol) in wasserfreiem Dichlormethan (10 mL) wurde mit Buttersäurechlorid (114 mg, 112 µL, 1.07 mmol) versetzt und 18 h bei Raumtemperatur gerührt. Das Gemisch wurde mit 25 % Kaliumcarbonat-Lösung (9 mL) versetzt und 15 min bei Raumtemperatur gerührt. Die Phasen wurden getrennt und wässrige mit Dichlor-methan (2 x 15 mL) extrahiert. Die vereinigten organischen Phasen wurden mit Natriumsulfat getrocknet und i. Vak. eingeengt. Der Rückstand (307 mg) wurde durch Flashchromatographie (18 g, 20 x 2.0 cm) mit Dichlormethan / Methanol (95:5) und 1 % Ammoniak (25 % in Wasser) gereinigt.

### Beispiel Nr. 95 (ein Diastereoisomer)

Ausbeute: 206 mg (68 %), farbloses Öl
1H-NMR (CDCl3): 0.94 (1.5 H, t, J = 7.4 Hz); 0.95 (1.5 H, t, J = 7.4 Hz); 1.00-1.14 (2 H, m); 1.20-1.30 (2 H, m); 1.32-1.84 (20 H, m); 2.10-2.24 (7 H, m); 3.17 (1H, s); 3.25 (1H, s); 3.45 (1H, t, J = 7.2 Hz); 3.50 (1H, t, J = 7.2 Hz).
13C-NMR (CDCl3): 14.0; 18.4; 25.1; 28.8; 29.5; 29.8; 30.2; 33.0; 35.0; 35.1; 35.2; 35.5; 35.7; 36.1; 36.2; 36.5; 36.8; 36.9; 37.0; 37.2; 40.3; 42.2; 44.3; 45.3; 56.7; 56.8; 57.1; 59.5; 171.76; 171.84.

Die NMR-Spektren zeigen teilweise doppelte Signalsätze (Rotamere).
LC-MS: m/z: [M+H]⁺ = 335.3, geringe UV-Aktivität.

### Beispiel Nr. 106 und Beispiel Nr. 107

### Stufe 1: 10-Butyl-1,4-dioxa-10-aza-dispiro[4.2.4.2]tetradecan-9-on

Eine Lösung von Substanz D (Schema 1) (5.0 g, 23.7 mmol) in N,N-Dimethylformamid (40 mL) wurde mit Kalium-tert-butylat (3.19 g, 28.4 mmol) versetzt, wobei ein Niederschlag ausfiel und das Gemisch dann 30 min bei Raumtemperatur gerührt wurde. Dann wurde der Suspension n-Butyliodid (5.23 g, 3.23 mL, 28.4 mmol) zugesetzt und 18 h bei Raumtemperatur gerührt. Anschließend wurde das Reaktionsgemisch i. Vak. eingeengt, der Rückstand in Ethylacetat (100 mL) aufgenommen und die Lösung mit Wasser (3 × 40 mL) gewaschen. Die organische Phase wurde mit Natriumsulfat getrocknet und i. Vak. eingeengt. Ausbeute: 5.30 g (84 %), gelbes Öl
1H-NMR (CDCl3): 0.91 (3 H, t, J = 7.3 Hz); 1.22-1.34 (2 H, m); 1.42-1.50 (4 H, m); 1.53-1.62 (2 H, m); 1.81-1.88 (2 H, m); 1.91 (2 H, t, J = 6.9 Hz); 1.93-2.02 (2 H, m); 3.26 (4 H, t, J = 7.0 Hz); 3.92-3.95 (4 H, m).
LC-MS: m/z: [M+H]+ = 268.3, Rt = 3.3 min.

### Stufe 2: 10-Butyl-1,4-dioxa-10-aza-dispiro[4.2.4.2]tetradecan

Eine Aufschlämmung von Lithiumaluminiumhydrid (2.95 g, 77.8 mmol) in absolutem Tetrahydrofuran (20 mL) wurde unter Eiskühlung mit einer Lösung von 10-Butyl-1,4-dioxa-10-aza-dispiro[4.2.4.2]tetradecan-9-on (5.22 g, 19.5 mmol) in absolutem Tetrahydrofuran (40 mL) versetzt und 66 h bei 50 °C gerührt. Anschließend wurde das Gemisch unter Eiskühlung vorsichtig mit Wasser (2.95 mL), 15 % Natronlauge (2.95 mL) und erneut mit Wasser (8.85 mL) versetzt und 1 h bei Raumtemperatur gerührt. Die Suspension wurde durch Seesand filtriert und der Rückstand mit Tetrahydrofuran gewaschen. Das Filtrat wurde mit Natriumsulfat getrocknet und i. Vak. eingeengt.
Ausbeute: 4.83 g (98 %), farbloses Öl
1H-NMR (CDCl3): 0.90 (3 H, t, J = 7.3 Hz); 1.26-1.36 (2 H, m); 1.40-1.49 (2 H, m); 1.55-1.66 (10 H, m); 2.33-2.38 (4 H, m); 2.53 (2 H, t, J = 6.9 Hz); 3.92 (4 H, s).
LC-MS: m/z: [M+H]+ = 254.4, Rt = 2.0 min.

### Stufe 3: 2-Butyl-2-aza-spiro[4.5]decan-8-on

Eine Lösung von 10-Butyl-1,4-dioxa-10-aza-dispiro[4.2.4.2]tetradecan (4.83 g, 19.1 mmol) in 5 % Schwefelsäure (50 mL) wurde 18 h bei Raumtemperatur gerührt. Anschließend wurde die Reaktionslösung mit Diethylether (3 × 20 mL) gewaschen, um vorhandene Neutralstoffe zu entfernen. Dann wurde die wässrige Phase mit 4 N Natronlauge alkalisch (pH ∼9) gestellt und mit Dichlormethan (4 × 30 mL) extrahiert. Die vereinigten organischen Phasen, der alkalischen Extraktion, wurden mit Natriumsulfat getrocknet und i. Vak. eingeengt.
Ausbeute: 3.54 g (89 %), gelbes Öl
1H-NMR (CDCl3): 0.92 (3 H, t, J = 6.9 Hz); 1.34 (2 H, qd, J = 14.3 und 7.2 Hz); 1.43-1.52 (2 H, m); 1.76 (2 H, t, J = 6.9 Hz); 1.87 (2 H, t, J = 6.8 Hz); 2.31-2.38 (4 H, m); 2.39-2.44 (2 H, m); 2.49 (2 H, s); 2.62 (2 H, t, J = 6.9 Hz).

### Stufe 4: 2-Butyl-8-dimethylamino-2-aza-spiro[4.5]decan-8-carbonitril

Zu einer auf 0 °C gekühlten 40 % wässrigen Dimethylaminlösung (10.4 mL, 75.3 mmol) und Methanol (4.7 mL) wurde 4 N Salzsäure (4.23 mL) und dann eine Lösung des Rohproduktes von 2-Butyl-2-aza-spiro[4.5]decan-8-on (3.54 g, 16.9 mmol) in Methanol (20 mL) gegeben. Das Gemisch wurde mit Kaliumcyanid (2.67 g, 40 mmol) versetzt und 18 h bei Raumtemperatur gerührt. Anschließend wurde die Reaktionslösung mit Wasser (75 mL) versetzt und mit Dichlormethan (6 × 15 mL) extrahiert. Die vereinigten organischen Phasen wurden mit Natriumsulfat getrocknet und i. Vak. eingeengt.
Ausbeute: 4.27 g (96 %), gelbes Öl
Es handelt sich um einen Diastereoisomerengemisch.
1H-NMR (CDCl3): Alle charakteristischen Signale sind enthalten.

### Stufe 5: [2-Butyl-8-(iminophenylmethyl)-2-aza-spiro[4.5]dec-8-yl]-dimethylamin

Zu einer Lösung von 2-Butyl-8-dimethylamino-2-aza-spiro[4.5]decan-8-carbonitril (1.0 g, 3.8 mmol) in absolutem Tetrahydrofuran (40 mL) wurde bei 0 °C unter Argon eine 1.8 M Phenyllithium-Lösung in Di-n-butyl-ether (4.2 mL, 7.6 mmol) getropft und dann 3 h bei Raumtemperatur gerührt. An-schließend wurden Wasser (10 mL) und Natriumchloridlösung (10 mL) zugesetzt, die Phasen getrennt und die wässrige Phase mit Dichlormethan (3 × 20 mL) extrahiert. Die vereinigten organischen Phasen wurden mit Natriumsulfat getrocknet und i. Vak. eingeengt.
Ausbeute: 1.59 g (> 100 %, Rohprodukt)
Das Rohprodukt wurde ohne vorherige Reinigung weiter umgesetzt.
1H-NMR (DMSO-d6): Alle charakteristischen Signale sind enthalten (zwei Diastereoisomere).
LC-MS: m/z: [M+H]+ = 342.3, Rt = 0.6 min.

### Stufe 6: [3-Butyl-8-(dimethyl-amino)-3-azaspiro[4.5]decan-8-yl]-phenyl-methanon (Beispiel Nr. 180, polares Diastereomer und Beispiel Nr. 181, unpolares Diastereomer)

Eine Lösung des Rohproduktes von [2-Butyl-8-(iminophenylmethyl)-2-aza-spiro[4.5]dec-8-yl]-dimethylamin (1.57 g, max. 3.8 mmol) in Tetrahydrofuran / Wasser (1:1, ∼20 mL) wurde mit Ameisensäure (5 mL) angesäuert und 18 h bei Raumtemperatur gerührt. Anschließend wurde das Tetrahydrofuran i. Vak. eingeengt und der wässrige Rückstand mit Ethylacetat (3 × 10 mL) extrahiert. Die vereinigten sauren, wässrigen Extrakte wurden mit 1 N Natronlauge alkalisch gestellt und mit Dichlormethan (3 × 20 mL) extrahiert. Die vereinigten organischen Extrakte der alkalischen Extraktion wurden mit Natriumsulfat getrocknet und i. Vak. eingeengt. Das Rohprodukt wurde mittels Flashchromatographie (100 g, 20 × 4.0 cm) mit Chloroform / Methanol (95:5) und 1 % Essigsäure gereinigt. Um die freien Basen, der jeweiligen Produktchargen, zu erhalten, wurden die Fraktionen jeweils i. Vak. eingeengt, der Rückstand in 1 M Kaliumcarbonat-Lösung aufgenommen und die Suspensionen mit Dichlormethan (3 × 10 mL) extrahiert. Die vereinigten organischen Phasen wurden mit Natriumsulfat getrocknet und i. Vak. eingeengt.

### Beispiel Nr. 106 (polares Diastereoisomer)

Ausbeute: 250 mg (19 %), gelbes Öl
1H-NMR (CDCl3): 0.88 (3 H, t, J = 7.3 Hz); 1.24-1.37 (4 H, m); 1.39-1.54 (4 H, m); 1.62-1.71 (4 H, m); 2.03-2.09 (2 H, m); 2.26 (2 H, s); 2.31 (6 H, s); 2.32-2.37 (2 H, m); 2.57 (2 H, t, J = 6.8 Hz); 7.33-7.39 (2 H, m); 7.43-7.49 (1 H, m); 8.21-8.25 (2 H, m).
LC-MS: m/z: [M+H]+ = 343.4, Rt = 2.2 min.

### Beispiel Nr. 107 (unpolares Diastereoisomer)

Ausbeute: 170 mg (13 %), gelbes Öl
1H-NMR (CDCl3): 0.92 (3 H, t, J = 7.3 Hz); 1.27-1.38 (4 H, m); 1.46-1.65 (8 H, m); 2.06-2.12 (2 H, m); 2.32 (6 H, s); 2.47-2.52 (2 H, m); 2.55 (s, 2 H); 2.63 (2 H, t, J = 6.8 Hz); 7.34-7.39 (2 H, m); 7.44-7.49 (1 H, m); 8.20-8.24 (2 H, m).
LC-MS: m/z: [M+H]+ = 343.4, Rt = 2.2 min.

### Beispiel Nr. 117

### 8-(5-Chlor-thiophen-2-yl)-8-dimethylamino-3-azaspiro[4.5]decan-4-on (Beispiel Nr. 117, ein Diastereomer)

Zu einer 0.5 M Aufschlämmung von 5-Chlor-2-thienylmagnesiumbromid (5.29 g, 48 mL, 23.9 mmol) in Tetrahydrofuran wurde unter Argon langsam eine Aufschlämmung von 8-(Dimethylamino)-1-oxo-2-azaspiro[4.5]decane-8-carbonitril (1.76 g, 7.9 mmol) in absolutem Tetrahydrofuran (75 mL) getropft, wobei sich eine klare Lösung bildete. Anschließend wurde über Nacht bei 50 °C gerührt. Nach Zugabe von gesättigter Ammoniumchlorid-Lösung (100 mL) wurde das Tetrahydrofuran i. Vak. entfernt. Die erhaltene wässrige Lösung wurde mit Dichlormethan (3 × 50 mL) extrahiert, die vereinigten organischen Phasen wurden mit gesättigter Natriumchlorid-Lösung (50 mL) gewaschen, mit Natriumsulfat getrocknet und i. Vak. eingeengt. Das Rohprodukt (2.45 g) wurde mittels Flashchromatographie (100 g, 20 × 4.0 cm) mit Ethylacetat / Methanol (97:3) gereinigt.

### Beispiel Nr. 117 (ein Diastereoisomer)

Ausbeute: 1.47 g (59 %), gelber Feststoff
Schmelzpunkt: 198-201 °C
1H-NMR (CDCl3): 1.28-1.34 (2 H, m); 1.61-1.68 (2 H, m); 2.01 (2 H, t, J = 6.9 Hz); 2.12 (6 H, s); 2.17 (2 H, dt, J = 13.1 und 3.1 Hz), 2.32-2.40 (2 H, m); 3.28-3.32 (2 H, m); 5.90 (1 H, br s); 6.60 (1 H, d, J = 3.8 Hz); 6.83 (1 H, d, J = 3.8 Hz).
13C-NMR (CDCl3): 27.9; 31.5; 32.7; 37.9; 38.7; 43.1; 58.9; 123.1; 125.2, 127.4; 144.4; 182.4.
LC-MS: m/z: [MH-HNMe2]+ = 268.2, Rt = 2.6 min.

### Beispiel Nr. 152

### 8-(Dimethyl-amino)-8-(5-methyl-thiophen-2-yl)-3-azaspiro[4.5]decan-2-on (Beispiel Nr. 152, polares Diastereomer)

Eine Lösung von 8-(Dimethyl-amino)-8-(5-methyl-thiophen-2-yl)-2-oxo-3-azaspiro[4.5]decan-3-carbonsäure tert-butyl ester (polares Diastereoisomer) (900 mg, 2.3 mmol) in wasserfreiem Dichlormethan (50 mL) wurde mit Trifluoressigsäure (5 mL) versetzt und 3 h bei Raumtemperatur gerührt. Das Reaktionsgemisch wurde i. Vak. eingeengt, der Rückstand mit Dichlormethan (30 mL) versetzt und mit gesättigter Natrium-hydrogencarbonat-Lösung (3 × 20 mL) gewaschen. Die organische Phase wurde mit Natriumsulfat getrocknet und i. Vak. eingeengt. Das Rohprodukt (622 mg) wurde durch Flashchromatographie (18 g, 20 × 2.0 cm) mit Dichlormethan / Methanol (14:1) und 0.5 % Ammoniak (25 % in Wasser) gereinigt.

### Beispiel Nr. 152 (polares Diastereoisomer)

Ausbeute: 502 mg (75 %), weißer Feststoff
Schmelzpunkt: 198-201 °C
1H-NMR (CDCl3): 1.46-1.54 (2 H, m); 1.72-1.80 (2 H, m); 1.85-2.10 (4 H, m); 2.11 (6 H, s); 2.25 (2 H, s); 2.45 (3 H, d, J = 1.0 Hz); 3.07 (2 H, s); 5.72 (1 H, br s); 6.61 (1 H, d, J = 3.5 Hz); 6.66-6.69 (1 H, m).
13C-NMR (CDCl3): 15.2; 32.6; 32.8; 38.2; 38.9; 42.3; 53.7; 59.7; 124.5; 125.0; 137.9; 177.4. LC-MS: m/z: [MH-HNMe2]+ = 248.3 (100 %) und [M+H]+ = 293.3 (10 %), Rt = 2.2 min.

### Beispiel Nr. 153

### 8-(Dimethyl-amino)-8-(5-methyl-thiophen-2-yl)-3-azaspiro[4.5]decan-2-on (Beispiel Nr. 153, unpolares Diastereomer)

Eine Lösung von 8-(Dimethyl-amino)-8-(5-methyl-thiophen-2-yl)-2-oxo-3-azaspiro[4.5]decan-3-carbonsäure tert-butyl ester (Beispiel Nr. 251, unpolares Diastereoisomer) (820 mg, 2.09 mmol) in wasserfreiem Dichlormethan (50 mL) wurde mit Trifluoressigsäure (5 mL) versetzt und 3 h bei Raumtemperatur gerührt. Das Reaktionsgemisch wurde i. Vak. einge-engt, der Rückstand mit Dichlormethan (30 mL) versetzt und mit gesättigter Natriumhydrogencarbonat-Lösung (3 × 20 mL) gewaschen. Die organische Phase wurde mit Natriumsulfat getrocknet und i. Vak. eingeengt. Das Rohprodukt (530 mg) wurde durch Flashchromatographie (18 g, 20 × 2.0 cm) mit Dichlormethan / Metha-nol (95:5) und 1 % Ammoniak (25 % in Wasser) gereinigt.

### Beispiel Nr. 153 (unpolares Diastereoisomer)

Ausbeute: 425 mg (70 %), weißer Feststoff
1H-NMR (CDCl3): 1.46-1.56 (2 H, m); 1.74-1.84 (2 H, m); 1.86-2.09 (4 H, m); 2.11 (6 H, s); 2.115 (2 H, s); 2.47 (3 H, d, J = 1.1 Hz); 3.22 (2 H, s); 5.78 (1 H, br s); 6.61 (1 H, d, J = 3.5 Hz); 6.67-6.69 (1 H, m).
13C-NMR (CDCl3): 15.2; 32.6; 38.1; 38.8; 43.2; 52.7; 59.4; 124.5; 124.9; 137.9; 140.0; 177.4.
LC-MS: m/z: [M+H]+ = 293.3, Rt = 2.2 min.

### Beispiel Nr. 162

### Stufe 1: 8-Dimethylamino-8-phenyl-2-azaspiro[4.5]decan-3-on

Zu einer auf 0 °C gekühlten 2 M Lösung von Phenylmagnesiumchlorid in Tetrahydrofuran (3 mL, 6 mmol) wurde unter Argon eine Suspension von 8-Dimethylamino-3-oxo-2-azaspiro[4.5]decan-8-carbonitril (536 mg, 2.4 mmol) in wasserfreiem Tetrahydrofuran (30 mL) getropft und anschlie-ßend 18 h bei Raumtemperatur gerührt. Nach Zugabe von gesättigter Ammoniumchlorid-Lösung (15 mL) wurden die Phasen getrennt und die wässrige Phase mit Ethylacetat (3 × 30 mL) extrahiert. Die vereinigten organischen Phasen wurden mit Natriumsulfat getrocknet und i. Vak. eingeengt.
Ausbeute: 601 mg (92 %), weißer Feststoff (Rohprodukt)
Diastereoisomerengemisch: Verhältnis polar : unpolar = 1 : 2.
Das Diastereoisomerenverhältnis wurde anhand der Singuletts der HN-CH2-Gruppe bei 3.27 (polares Diastereoisomer) bzw. 3.02 ppm (unpolares Diastereoisomer) im 1H-NMR-Spektrum bestimmt.

### Stufe 2: 8-Dimethylamino-3-oxo-8-phenyl-2-azaspiro[4.5]decan-2-carbonsäure-tert-butylester

Eine Lösung von 8-Dimethylamino-8-phenyl-2-azaspiro[4.5]decan-3-on (4.60 g, 16.9 mmol) in wasserfreiem Acetonitril (300 mL) und wasserfreiem Tetrahydrofuran (100 mL) wurde mit einer Lösung von Di-tert-butyldicarbonat (4.05 g, 18.6 mmol) in wasserfreiem Tetrahydrofuran (30 mL) und 4-Dimethylaminopyridin (206 mg, 1.69 mmol) versetzt und 3 d bei Raumtemperatur gerührt. Da die Umsetzung nicht vollständig war, wurde nochmals eine Lösung von Di-tert-butyldicarbonat (2.00 g, 9 mmol) in wasserfreiem Acetonitril (10 mL) zugegeben und 3 h bei 50 °C und 18 h bei Raumtemperatur gerührt. Anschließend wurde das Lösungsmittel i. Vak. entfernt, der Rückstand in Dichlormethan (100 mL) gelöst und die Lösung mit Wasser (3 × 50 mL) und gesättigter Natriumchlorid-Lösung (50 mL) gewaschen. Die organische Phase wurde mit Natriumsulfat getrocknet und i. Vak. eingeengt. Das Rohprodukt (7.00 g) wurde durch Flashchromatographie (400 g, 20 × 7.5 cm) mit Ethylacetat / Methanol (9:1) gereinigt.
Unpolares Diastereoisomer
Ausbeute: 1.40 g (22 %), weißer Feststoff
Schmelzpunkt: 174-176 °C
1H-NMR (CDCl3): 1.34-1.42 (2 H, m); 1.53 (9 H, s); 1.72-1.82 (2 H, m); 1.96-2.03 (2 H, m); 2.04 (6 H, s); 2.10-2.24 (2 H, m); 2.25 (2 H, s); 3.61 (2 H, s); 7.26-7.31 (3 H, m); 7.36-7.41 (2 H, m).
13C-NMR (CDCl3): 28.1; 30.0; 32.2; 34.3; 38.0; 45.8; 56.6; 60.1; 82.8; 126.8; 127.4; 127.8; 150.1; 173.4.
LC-MS: m/z: [M+H]+ = 373.4, Rt = 2.6 min.
Polares Diastereoisomer
Ausbeute: 1.26 g (20 %), weißer Feststoff
Schmelzpunkt: 176-181 °C
1H-NMR (CDCl3): 1.34-1.44 (2 H, m); 1.48 (9 H, s); 1.68-1.77 (2 H, m); 1.90-2.03 (2 H, m); 2.04 (6 H, s); 2.15-2.30 (2 H, m); 2.48 (2 H, s); 3.36 (2 H, s); 7.28-7.32 (3 H, m); 7.36-7.42 (2 H, m).
13C-NMR (CDCl3): 28.0; 29.8; 32.3; 34.5; 38.0; 44.9; 57.6; 60.3; 60.5; 82.7; 126.8; 127.5; 127.8; 136.2; 150.1; 173.4.
LC-MS: m/z: [M+H]+ = 373.4, Rt = 3.0 min.

### Stufe 3: 8-Dimethylamino-8-phenyl-2-azaspiro[4.5]decan-3-on (unpolares Diastereomer)

Eine Lösung von 8-Dimethylamino-3-oxo-8-phenyl-2-azaspiro[4.5]decan-2-carbonsäure-tert-butylester (unpolares Diastereoisomer) (1.46 g, 3.9 mmol) in wasserfreiem Dichlormethan (50 mL) wurde mit Trifluoressigsäure (5 mL) versetzt und 3 h bei Raumtemperatur gerührt. Das Reaktionsgemisch wurde i. Vak. eingeengt, der Rückstand in Dichlormethan (50 mL) gelöst und die Lösung mit gesättigter Natriumhydrogencarbonat-Lösung (3 × 20 mL) gewaschen. Die organische Phase wurde mit Natriumsulfat getrocknet und i. Vak. eingeengt.
Ausbeute: 1.03 g (96 %), weißer Feststoff
Schmelzpunkt: > 260 °C
1H-NMR (CDCl3): 1.37-1.46 (2 H, m); 1.76-1.84 (2 H, m); 1.90-2.02 (2 H, br s); 2.04 (6 H, s); 2.06 (2 H, s); 2.15-2.27 (2 H, br s); 3.27 (2 H, s); 5.60 (1 H, s); 7.26-7.32 (3 H, m); 7.36-7.42 (2 H, m).

### Stufe 4: 8-(Dimethyl-amino)-3-methyl-8-phenyl-3-azaspiro[4.5]decan-2-on (Beispiel Nr. 162, unpolares Diastereomer)

Eine Aufschlämmung von 8-Dimethylamino-8-phenyl-2-azaspiro[4.5]decan-3-on (unpolares Diastereomer) (150 mg, 0.55 mmol) in wasserfreiem Tetrahydrofuran (20 mL) und wasserfreiem N,N-Dimethylformamid (3 mL) wurde mit Kalium-tert-butylat (74 mg, 0.66 mmol) versetzt und 30 min bei Raumtemperatur gerührt. Anschließend wurde Methyliodid (94 mg, 41 µL, 0.66 mmol) zugegeben und 5 h bei Raumtemperatur gerührt. Die Lösung wurde anschließend i. Vak. eingeengt. Nach Zugabe von Ethylacetat (50 mL) wurde die Mischung mit Wasser (3 × 20 mL) gewaschen. Die organische Phase wurde anschließend mit 5 % Ameisensäure (3 × 20 mL) extrahiert. Die vereinigten wässrigen, sauren Phasen wurden mit 5 N Natronlauge auf pH 10 eingestellt und mit Ethylacetat (3 × 20 mL) extrahiert. Die vereinigten organischen Phasen wurden mit Natriumsulfat getrocknet und i. Vak. eingeengt.

### Beispiel Nr. 162 (unpolares Diastereoisomer)

Ausbeute: 120 mg (76 %), weißer Feststoff
Schmelzpunkt: 145-148 °C
1H-NMR (CDCl3): 1.33-1.42 (2 H, m); 1.70-1.78 (2 H, m); 1.85-2.02 (2 H, m); 2.03 (6 H, s); 2.12 (2 H, s); 2.13-2.25 (2 H, m); 2.83 (3 H, s); 3.34 (2 H, s); 7.24-7.31 (3 H, m); 7.35-7.41 (2 H, m).
13C-NMR (CDCl3): 29.7; 30.2; 33.1; 35.5; 38.0; 44.2; 60.1; 60.5; 126.7;. 127.4; 127.7; 173.8. LC-MS: m/z: [M+H]+ = 287.4, Rt = 1.3 min.

### Beispiel Nr. 186

### Stufe 1: 2-[3-(tert-Butyldimethylsilanyloxy)-3-methylbutyl]-8-dimethylamino-8-phenyl-2-aza-spiro[4.5]decan-3-on

Eine Suspension von pulverisiertem, i. Vak. getrocknetem Natriumhydroxid (106 mg, 2.64 mmol) in absolutem Dimethylsulfoxid (5 mL) wurde 40 min bei Raumtemperatur gerührt. Dann wurde hierzu 8-Dimethylamino-8-phenyl-2-azaspiro[4.5]decan-3-on (Beispiel Nr. 152, Stufe 3, unpolares Diastereomer) (180 mg, 0.66 mmol) gegeben, bevor es mit einer Lösung von 3-(Tert-butyldimethylsilyloxy)-3-methylbutyl 4-methylbenzenesulfonat (272 mg, 0.73 mmol) in Dimethylsulfoxid (2 mL) versetzt und 2 h bei Raumtemperatur gerührt wurde. Dann wurde nochmals eine Lösung von 3-(Tert-butyldimethylsilyloxy)-3-methylbutyl 4-methylbenzenesulfonat (136 mg, 0.37 mmol) in Dimethylsulfoxid (2 mL) zugesetzt und 18 h bei Raumtemperatur gerührt. Anschließend wurde das Gemisch mit Wasser (100 mL) versetzt und mit Ethylacetat (4 × 20 mL) extrahiert. Die vereinigten organischen Phasen wurden mit Natriumsulfat getrocknet und i. Vak. eingeengt. Der Rückstand wurde wiederholt in Toluol (3 × 10 mL) aufgenommen und jeweils wieder i. Vak. eingeengt.
Das Rohprodukt (420 mg, >100 %) wurde ohne vorherige Reinigung weiter umgesetzt.

### Stufe 2: 8-Dimethylamino-2-(3-hydroxy-3-methylbutyl)-8-phenyl-2-azaspiro[4.5]decan-3-on (Beispiel Nr. 186, unpolares Diastereomer)

Eine Lösung des Rohproduktes von 2-[3-(tert-Butyldimethylsilanyloxy)-3-methylbutyl]-8-dimethylamino-8-phenyl-2-aza-spiro[4.5]decan-3-on (unpolares Diastereomer) (420 mg, max. 0.66 mmol) in Methanol (20 mL) wurde mit 2 N Salzsäure (7.5 mL) versetzt und 2 h bei Raumtemperatur gerührt. Das Reaktionsgemisch wurde mit 1 M Kaliumcarbonat-Lösung alkalisch gestellt und das Methanol i. Vak. entfernt. Der wässrige Rückstand wurde mit Dichlormethan (3 × 20 mL) extrahiert. Die vereinigten organischen Phasen wurden mit Natriumsulfat getrocknet und i. Vak. eingeengt. Das Rohprodukt wurde mittels Flashchromatographie (18 g, 20 × 2.0 cm) mit Dichlormethan / Methanol (4:1) gereinigt.

### Beispiel Nr. 186 (unpolares Diastereoisomer)

Ausbeute: 105 mg (44 %), weißer Feststoff
Schmelzpunkt: 114-119 °C
1H-NMR (CDCl3): 1.25 (6 H, s); 1.30-1.41 (2 H, m); 1.63-1.68 (2 H, m); 1.69-1.79 (2 H, m); 1.91-2.03 (2 H, m); 2.02 (6 H, s); 2.12 (2 H, s); 2.13-2.24 (4 H, m); 2.28-2.45 (1 H, br s); 3.27 (2 H, s); 3.37-3.42 (2 H, m); 7.25-7.30 (3 H, m); 7.35-7.40 (2 H, m).
13C-NMR (CDCl3): 29.6; 30.2; 32.8; 35.8; 38.0; 38.8; 40.1; 44.5; 58.3; 60.1; 69.5; 126.7; 127.4; 127.7; 136.4; 174.0.
LC-MS: m/z: [MH-HNMe2]+ = 314.3 (82 %) und [M+H]+ = 359.3 (100 %), Rt = 2.3 min.

### Beispiel Nr. 187

### Stufe 1: 2-[3-(tert-Butyldimethylsilanyloxy)-3-methylbutyl]-8-dimethylamino-8-phenyl-2-aza-spiro[4.5]decan-3-on

Eine Suspension von pulverisiertem, i. Vak. getrocknetem Natriumhydroxid (106 mg, 2.64 mmol) in absolutem Dimethylsulfoxid (5 mL) wurde 40 min bei Raumtemperatur gerührt. Dann wurde hierzu 8-Dimethylamino-8-phenyl-2-azaspiro[4.5]decan-3-on (180 mg, 0.66 mmol) gegeben und mit einer Lösung von 3-(Tert-butyldimethylsilyloxy)-3-methylbutyl 4-methylbenzenesulfonat (272 mg, 0.73 mmol) in Dimethylsulfoxid (2 mL) versetzt. Nach 2 h bei Raumtemperatur wurde nochmals eine Lösung von 3-(Tert-butyldimethylsilyloxy)-3-methylbutyl 4-methylbenzenesulfonat (136 mg, 0.37 mmol) in Dimethylsulfoxid (2 mL) zugesetzt und 18 h bei Raumtemperatur gerührt. Anschließend wurde das Gemisch mit Wasser (100 mL) versetzt und mit Ethylacetat (4 × 20 mL) extrahiert. Die vereinigten organischen Phasen wurden mit Natriumsulfat getrocknet und i. Vak. eingeengt. Der Rückstand wurde wiederholt in Toluol (3 × 10 mL) aufgenommen und jeweils wieder i. Vak. eingeengt.
Das Rohprodukt (450 mg, >100 %) wurde ohne vorherige Reinigung weiter umge-setzt:

### Stufe 2: 8-Dimethylamino-2-(3-hydroxy-3-methylbutyl)-8-phenyl-2-azaspiro[4.5]decan-3-on (Beispiel Nr. 187, polares Diastereomer)

Eine Lösung des Rohproduktes von 2-[3-(tert-Butyldimethylsilanyloxy)-3-methylbutyl]-8-dimethylamino-8-phenyl-2-aza-spiro[4.5]decan-3-on (polares Diastereomer) (450 mg, max. 0.66 mmol) in Methanol (20 mL) wurde mit 2 N Salzsäure (7.5 mL) versetzt und 2 h bei Raumtemperatur gerührt. Anschließend wurde das Reaktionsgemisch mit 1 M Kaliumcarbonat-Lösung alkalisch gestellt und das Methanol i. Vak. entfernt. Der wässrige Rückstand wurde mit Dichlormethan (3 × 20 mL) extrahiert. Die vereinigten organischen Phasen wurden mit Natriumsulfat getrocknet und i. Vak. eingeengt. Das Rohprodukt wurde mittels Flashchromatographie (18 g, 20 × 2.0 cm) mit Dichlormethan / Methanol (4:1) gereinigt. Die hierdurch gewonnene Produktcharge (150 mg) enthielt als Verunreinigung 3-Methyl-1,3-butandiol und wurde nochmals mittels Flashchromatographie (10 g, 20 × 1.5 cm) mit Dichlormethan / Methanol (9:1) gereinigt.

### Beispiel Nr. 187 (polares Diastereoisomer)

Ausbeute: 120 mg (51 %), farbloses Öl
1H-NMR (CDCl3): 1.21 (6 H, s); 1.32-1.40 (2 H, m); 1.56-1.61 (2 H, m); 1.67-1.74 (2 H, m); 1.88-2.00 (2 H, m); 2.02 (6 H, s); 2.15-2.29 (2 H, m); 2.34 (2 H, s); 3.02 (2 H, s); 3.31-3.36 (2 H, m); 7.24-7.30 (3 H, m); 7.35-7.40 (2 H, m).
13C-NMR (CDCl3): 29.5; 30.0; 33.0; 35.9; 38.0; 38.6; 40.0; 43.6; 59.2; 60.7; 69.5; 126.7; 127.6; 127.8; 136.2; 174.0.
LC-MS: m/z: [MH-HNMe2]+ = 314.3 (100 %) und [M+H]+ = 359.4 (55 %), Rt = 2.6 min.

### Beispiel Nr. 213

### Stufe 1: 3-(8-Dimethylamino-8-thiophen-2-yl-2-azaspiro[4.5]dec-2-yl)propionsäuremethyl-ester

Zu einer Lösung von Beispiel Nr. 21 (320 mg, 1.2 mmol) in wasserfreiem Tetrahydrofuran (10 mL) wurde Methylacrylat (2.00 g, 2.16 mL, 24 mmol) gegeben und 3 d bei 50 °C gerührt. Das Reaktionsgemisch wurde anschließend i. Vak. eingeengt und der Rückstand (365 mg) durch Flashchromatographie (18 g, 20 × 2.0 cm) mit Dichlormethan / Methanol (95:5) und 0.5 % Ammoniak (25 % in Wasser) gereinigt.
Ausbeute: 274 mg (65 %), farbloses Öl
1H-NMR (CDCl3): 1.33-1.41 (2 H, m); 1.51 (2 H, t, J = 6.8 Hz); 1.65-1.74 (2 H, m); 1.87-2.00 (2 H, m); 2.10 (8 H, s); 2.44 (2 H, s); 2.47-2.56 (4 H, m); 2.70-2.75 (2 H, m); 3.68 (3 H, s); 6.85 (1 H, d, J = 3.0 Hz); 7.03 (1 H, dd, J = 5.1 und 3.5 Hz); 7.23 (1 H, br d, J = 4.8 Hz).

### Stufe 2: 1-[8-(Dimethyl-amino)-8-thiophen-2-yl-3-azaspiro[4.5]decan-3-yl]-3-ethyl-pentan-3-ol(Beispiel Nr. 213, polares Diastereoisomer)

Eine Lösung von 3-(8-Dimethylamino-8-thiophen-2-yl-2-azaspiro[4.5]dec-2-yl)propionsäuremethyl-ester (274 mg, 0.78 mmol) in wasserfreiem Tetrahydrofuran (5 mL) wurde unter Argon mit Titan-tetra-isopropylat (45 mg, 48 µL, 0.16 mmol) versetzt. Anschließend wurde innerhalb 1 h eine 0.3 M Lösung von Ethylmagnesiumbromid (7.8 ml, 2.34 mmol) in Diethylether langsam zugetropft und über Nacht bei Raumtemperatur gerührt. Die honiggelbe Lösung wurde mit 5 % wässriger Schwe-felsäure (5 mL) versetzt und kräftig gerührt. Durch Zugabe von 2 M Kaliumcarbonat-Lösung wurde pH 10 eingestellt und das Gemisch mit Dichlormethan (3 × 30 mL) extrahiert. Die vereinigten organischen Phasen wurden mit Natriumsulfat getrocknet und i. Vak. eingeengt. Das Rohprodukt (250 mg) wurde durch Flashchromatogra¬phie (10 g, 20 × 1.5 cm) mit Dichlormethan / Methanol (95:5) und 1 % Ammoniak (25 % in Wasser) gereinigt.

### Beispiel Nr. 213 (polares Diastereoisomer)

Ausbeute: 85 mg (29 %), Öl
1H-NMR (CDCl3): 0.84 (6 H, t, J = 7.5 Hz); 1.31-1.58 (10 H, m); 1.64-1.74 (2 H, m); 1.88-1.98 (2 H, m); 2.00-2.09 (2 H, m); 2.10 (6 H, s); 2.48 (2 H, s); 2.58 (2 H, t, J = 7.0 Hz); 2.64-2.69 (2 H, m); 6.25 (1 H, br s); 6.83 (1 H, dd, J = 3.5 und 1.1 Hz); 7.02 (1 H, dd, J = 5.1 und 3.5 Hz); 7.21 (1 H, dd, J = 5.1 und 1.1 Hz).
13C-NMR (CDCl3): 8.1; 26.9; 30.9; 33.5; 33.9; 37.2; 38.1; 40.9; 52.2; 53.5; 59.6; 65.5; 74.9; 123.2; 124.8; 126.1; 143.1.
LC-MS: m/z: [M+H]+ = 379.4, Rt = 1.9 min.

### Beispiel Nr. 214

### Stufe 1: 8-Dimethylamino-8-thiophen-2-ylmethyl-2-azaspiro[4.5]decan-1-on

In einem Schlenk-Kolben wurde Lithiumchlorid (269 mg, 6.33 mmol) im Hochvakuum mittels Heißluftpistole 10 min erhitzt. Dann wurde Magnesium (220 mg, 9.05 mmol) zugegeben und das Gemisch noch einmal für 10 min mittels Heißluft-pistole im Hochvakuum erhitzt. Anschließend wurden absolutes frisch über Calciumhydrid destilliertes Tetrahydrofuran (1.5 mL) und ein Tropfen einer 25 % Diisobutylaluminiumhydrid-Lösung in Toluol zugegeben und 5 min bei Raumtemperatur gerührt. Dann wurde Substanz F (Schema 1, R1=R2=Methyl) (400 mg, 1.18 mmol) zugegeben und zu dieser Suspension eine für 40 min über Molsieb 4 A getrocknete Lösung von 2-(Brommethyl)thiophen (1.18 g, 6.66 mmol) in absolutem frisch über Calciumhydrid destilliertem Tetrahydrofuran (2 mL) langsam zugetropft, wobei eine Erwärmung der Lösung und das Auflösen des Ausgangsstoffes beobachtet wurde. Das Reaktionsgemisch wurde 30 min bei Raumtemperatur und anschließend 3 h bei 55 °C gerührt. Danach wurde langsam gesättigte Ammoniumchlorid-Lösung (6 mL) zugegeben und 16 h bei Raumtemperatur gerührt. Nach Zugabe von 2 M Natronlauge (8 mL) und Wasser (30 mL) wurde mit Ethylacetat (2 × 100 mL) und Chloroform / Methanol (7:1, 50 mL) extrahiert, die vereinigten organischen Phasen wurden mit gesättigter Natriumchlorid-Lösung (30 mL) gewaschen, mit Magnesiumsulfat getrocknet und das Lösungsmittel i. Vak. entfernt. Der Rückstand (706 mg) wurde in Diethylether (20 mL) aufgenommen, die Suspension filtriert, der Filterrückstand mit Diethylether gewaschen und das Filtrat i. Vak. eingeengt. Dieser Rückstand wurde durch Flashchromatographie (18 g, 20 × 2 cm) mit Ethylacetat / Methanol (2:0.1→2:0.4) und 1 % Ammoniak (25 % in Wasser) gereinigt.

### Unpolares Diastereoisomer

Ausbeute: 240 mg (45 %), farbloses Öl.
1H-NMR (CDCl3): 1.15 (2 H, d, J = 13.1 Hz); 1.32 (2 H, t, J = 14.1 Hz); 1.74-1.87 (4 H, m); 2.02-2.12 (2 H, m); 2.29 (6 H, s); 2.85 (2 H, s); 3.21 (2 H, t, J = 7.1 Hz); 6.16 (1 H, s); 6.75 (1 H, d, J = 2.8 Hz); 6.92 (1 H, dd, J = 5.2 und 3.4 Hz); 7.12 (1 H, dd, J = 5.2 und 1.2 Hz).
LC-MS: [M+H]+: m/z = 293.3, Rt = 2.2 min.

### Polares Diastereoisomer

Ausbeute: 20 mg (3.8 %) farbloses Öl.
1H-NMR (CDCl3): 1.46-1.63 (4 H, m); 1.70-1.85 (4 H, m); 2.00 (2 H, t, J = 6.9 Hz); 2.31 (6 H, s); 3.10 (2 H, s); 3.28 (2 H, t, J = 6.9 Hz); 6.25 (1 H, s); 6.86 (1 H, dd, J = 3.4 Hz); 6.90 (1 H, dd, J = 5.1 Hz); 7.13 (1 H, dd, J = 5.1 Hz).
LC-MS: [M+H]+; m/z = 293.3, Rt = 0.6 min.

### Stufe 2: N,N-Dimethyl-8-(thiophen-2-ylmethyl)-2-azaspiro[4.5]decan-8-amin (unpolares Diastereomer)

In einem Schlenk-Kolben wurde unter Argon zu einer Suspension von Lithiumaluminiumhydrid (150 mg, 3.95 mmol) in absolutem Tetrahydrofuran (5 mL) bei 0 °C langsam 8-Dimethylamino-8-thiophen-2-ylmethyl-2-azaspiro[4.5]decan-1-on (unpolares Diastereoisomer) (230 mg, 0.79 mmol) gelöst in absolutem Tetrahydrofuran (6 mL) gegeben. Im Anschluss wurde das Eisbad entfernt und 16 h bei 50 °C gerührt. Dann wurden bei 0 °C sukzessive Wasser (150 µL), 15 % Natronlauge (150 µL), Tetrahydrofuran (10 mL) und Wasser (450 µL) zugegeben und die Suspension wurde 1 h bei Raumtemperatur gerührt. Die Suspension wurde durch Natriumsulfat filtriert, der Rückstand mit Dichlormethan (2 × 30 mL) gewaschen und das Filtrat i. Vak. zur Trockene eingeengt.
Ausbeute: 207 mg (95 %), gelbes Öl.
1H-NMR (CDCl3): 1.20-1.28 (2 H, m); 1.33-1.45 (4 H, m); 1.60-1.71 (4 H, m); 2.28 (6 H, s); 2.65 (2 H, s); 2.87-2.93 (4 H, m); 6.76 (1 H, dd, J = 3.4 und 1.1 Hz); 6.92 (1 H, dd, J = 5.2 und 3.4 Hz); 7.12 (1 H, dd, J = 5.2 und 1.2 Hz).
LC-MS: [M+H]+: m/z = 279.3, Rt = 0.2 min.

### Stufe 3: 1-(8-Dimethylamino-8-thiophen-2-ylmethyl-2-azaspiro[4.5]dec-2-yl)-butan-1-on (Beispiel Nr. 214, unpolares Diastereomer)

In einem 10 mL Schlenk-Kolben wurde unter Argon zu einer Lösung von N,N-Dimethyl-8-(thiophen-2-ylmethyl)-2-azaspiro[4.5]decan-8-amin (unpolares Diastereomer) (199 mg, 0.72 mmol) in absolutem Dichlormethan (5 mL) bei Raumtemperatur zuerst Triethylamin (116 mg, 160 µL, 1.14 mmol) und dann langsam Buttersäurechlorid (99 mg, 96 µL, 0.93 mmol) gegeben. Das Reaktionsgemisch wurde 16 h bei Raumtemperatur gerührt und anschließend mit gesättigter Natriumcarbonat-Lösung (20 mL) versetzt. Nach Zugabe von Dichlormethan (10 mL) wurden die Phasen getrennt und die wässrige Phase mit Dichlormethan (2 × 25 mL) extrahiert. Die vereinigten organischen Phasen wurden sukzessive mit gesättigter Natriumcarbonat-Lösung (20 mL), 0.2 M Natronlauge (10 mL) und gesättigter Natriumchlorid-Lösung (10 mL) gewaschen, mit Magnesiumsulfat getrocknet und das Lösungsmittel i. Vak. entfernt. Das Rohprodukt (264 mg) wurde zuerst durch Flashchromatographie (PuriFlash PF-15SIHP, 8 g) mit Dichlormethan / Methanol (95:5→80:20) und 1 % Ammoniak (25 % in Wasser) und dann erneut durch Flashchromatographie (7 g, 20 × 1.5 cm) mit Dichlormethan / Methanol (95:5→80:20) und 1 % Ammoniak (25 % in Wasser) gereinigt.

### Beispiel Nr. 217 (unpolares Diastereoisomer)

Ausbeute: 161 mg (65 %), gelber Feststoff.
Schmelzpunkt: 86 °C
1H-NMR (CDCl3):0.94 (3 H, t, J = 7.4 Hz); 1.21-1.47 (4 H, m); 1.56 (1 H, t, J = 7.3 Hz); 1.60-1.77 (7 H, m); 2.18 (2 H, t, J = 7.5 Hz); 2.27 und 2.28 (6 H, s); 2.88 und 2.90 (2 H, 2 s); 3.16 (1 H, s); 3.24 (1 H, s); 3.38-3.46 (2 H, m); 6.80-6.73 (1 H, m); 6.91-6.94 (1 H, m); 7.13 (1 H, d, J = 5.1 Hz). Einige Signale sind als doppelter Signalsatz zu sehen (Rotamere).
13C-NMR (CDCl3): 13.99; 14.01; 18.3; 18.4; 28.5; 29.0; 29.8; 30.1; 31.1; 31.3; 32.7; 34.9; 36.3; 36.7; 37.1; 37.1; 40.1; 42.0; 44.2; 45.3; 57.36; 57.38; 59.4; 123.9; 124.0; 126.3; 126.5; 126.45; 126.60; 126.7; 141.0; 141.2; 171.7; 171.8.
LC-MS: [M+H]+: m/z = 349.3, Rt = 2.8 min.

### Beispiel Nr. 234

### Stufe 1: Cyclopent-1-en-Magnesiumbromid

In einer sekurierten Apparatur wurden Magnesium (1.70 g, 70 mmol) und ein lod-kristall so erhitzt, dass sich lodgas bildete. Das Gemisch wurde auf Raumtemperatur abgekühlt und anschließend mit wasserfreiem Tetrahydrofuran (17 mL) und einem weiteren lodkristall versetzt. Anschließend wurde eine Lösung von 1 Bromcyclopenten (10.3 g, 70 mmol) in wasserfreiem Tetrahydrofuran (23 mL) so zugetropft, dass das Reaktionsgemisch zu sieden begann. Das Gemisch wurde noch 1 h unter Rückfluss gerührt und anschließend auf Raumtemperatur abgekühlt. Die so erhaltene Lösung wurde in in der nächsten Stufe eingesetzt.

### Stufe 2: (8-Cyclopent-1-enyl-1,4-dioxaspiro[4.5]dec-8-yl)-dimethylamin

Zur Lösung von Stufe 1 (max. 70 mmol) wurde eine Lösung des 8-(Dimethylamino)-1,4-dioxaspiro[4.5]decane-8-carbonitrils (6.05 g, 28.7 mmol) in wasserfreiem Tetrahydrofuran (40 mL) getropft. Das Gemisch wurde über Nacht bei Raumtemperatur, anschließend 2 h bei 60 °C gerührt und danach unter Eiskühlung mit gesättigter Ammoniumchlorid-Lösung (50 mL) und Wasser (50 mL) versetzt. Der pH-Wert des Gemisches wurde mit 4 N Natronlauge auf 9 gestellt. Die Phasen wurden getrennt und die wässrige Phase mit Ethylacetat (3 x 50 mL) extrahiert. Die vereinigten organischen Phasen wurden mit Natriumsulfat getrocknet und i. Vak. eingeengt. Das Rohprodukt wurde durch Flashchromatographie (400 g, 20 x 7.5 cm) mit Dichlormethan / Methanol (95:5) und 1 % Ammoniak (25 % in Wasser) gereinigt.
Ausbeute: 2.54 g (35 %), gelbes Öl
1H-NMR (CDCl3): 1.50-1.60 (2 H, m); 1.70-1.94 (8 H, m); 2.20 (6 H, s); 2.24-2.30 (2 H, m); 2.31-2.39 (2 H, m); 3.88-3.96 (4 H, m); 5.53 (1 H, m).
13C-NMR (CDCl3): 23.6; 29.0; 31.4; 32.2; 33.1; 38.5; 58.4; 64.1; 109.0; 128.2; 143.8. LC-MS: [M+H]+: m/z = 252.3, Rt = 1.9 min.

### Stufe 3: (8-Cyclopentyl-1,4-dioxaspiro[4.5]dec-8-yl)-dimethylamin

Eine Lösung von (8-Cyclopent-1-enyl-1,4-dioxaspiro[4.5]dec-8-yl)-dimethylamin (2.53 g, 10 mmol) in wasserfreiem Methanol (220 mL) wurde mit 5 % Rhodium auf aktiviertem Aluminiumoxid (2.05 g, 1 mmol) versetzt. Die Suspension wurde 18 h bei 50 °C und einem Wasserstoffdruck von 4 bar gerührt und anschließend durch Celite, das zuvor mit Methanol gewaschen wurde, filtriert. Das Filtrat wurde i. Vak. eingeengt.
Ausbeute: 2.51 g (100 %), gelbes Öl
1H-NMR (CDCl3): 1.20-1.34 (2 H, m); 1.38-1.64 (10 H, m); 1.68-1.78 (2 H, m); 1.82-1.94 (2 H, m); 2.07 (1 H, m); 2.27 (6 H, s); 3.91-3.94 (4 H, m).
13C-NMR (CDCl3): 25.0; 28.0; 28.5; 30.0; 37.8; 43.8; 57.5; 64.1; 109.6.

### Stufe 4: 4-Cyclopentyl-4-dimethylaminocyclohexanon

Eine Lösung von (8-Cyclopentyl-1,4-dioxaspiro[4.5]dec-8-yl)-dimethylamin (5.21 g, 20.5 mmol) in 1 M wässriger Schwefelsäure (150 mL) wurde 48 h bei Raumtemperatur gerührt. Das Gemisch wurde mit Dichlormethan (2 x 70 mL) gewaschen. Die wässrige Phase wurde mit 4 N Natronlauge alkalisch gestellt und mit Dichlormethan (4 x 50 mL) extrahiert. Die vereinigten organischen Phasen wurden mit Natriumsulfat getrocknet und i. Vak. eingeengt. Ausbeute: 3.52 g (82 %), gelbes Öl
1H-NMR (CDCl3): 1.18-1.34 (2 H, m); 1.40-1.80 (8 H, m); 1.96-2.08 (2 H, m); 2.10-2.22 (3 H, m); 2.34 (6 H, s); 2.51-2.63 (2 H, m).
13C-NMR (CDCl3): 24.9; 28.6; 30.1; 36.6; 37.2; 38.0; 43.4; 57.5.
Der Carbonylkohlenstoff wurde durch ein gHMBC-Spektrum bei 212 ppm nachgewiesen. LC-MS: [M+H]+: m/z = 210.3, Rt = 0.8 min.

### Stufe 5: (4-Cyclopentyl-4-dimethylamino-cyclohexyliden)-essigsäureethylester

Eine Lösung von Phosphonoessigsäure-triethylester (6.74 g, 5.98 mL, 30.1 mmol) in wasserfreiem N,N-Dimethylformamid (30 mL) wurde mit Kalium-tert-butanolat (2.99 g, 26.7 mmol) versetzt und 1 h bei 50 °C gerührt. Die Lösung wurde auf Raumtemperatur abgekühlt und anschließend mit einer Lösung von 4-Cyclopentyl-4-dimethylaminocyclohexanon (3.96 g, 18.9 mmol) in wasserfreiem N,N-Dimethylformamid (50 mL) versetzt. Das Reaktionsgemisch wurde 20 h bei Raumtemperatur gerührt und anschließend in Eiswasser (75 g) gegossen. Die Suspension wurde mit Diethylether (4 x 40 mL) extrahiert. Die vereinigten organischen Phasen wurden mit Natriumsulfat getrocknet und i. Vak. eingeengt. Der Rückstand wurde zuerst wiederholt mit Toluol versetzt, jeweils wieder i. Vak. eingeengt und danach wurde die Prozedur mit Cyclohexan wiederholt. Dieser Rückstand (5.49 g) wurde in Ethylacetat (30 mL) aufgenommen und die Lösung mit 10 % wässriger Ameisensäure (5 x 30 mL) extrahiert. Die vereinigten sauren, wässrigen Phasen wurden mit 4 N Natronlauge alkalisch gestellt und mit Dichlormethan (5 x 30 mL) extrahiert. Die vereinigten organischen Phasen wurden mit Natriumsulfat getrocknet und i. Vak. eingeengt. Ausbeute: 4.36 g (77 %), farbloses Öl
1H-NMR (CDCl3): 1.14-1.70 (13 H, m); 1.78-2.40 (5 H, m); 2.32 (6 H, s); 2.57 (1 H, br t, J = 13.9 Hz); 3.55 (1 H, br d, J = 12.6 Hz); 4.13 (2 H, q, J = 7.0 Hz); 5.58 (1 H, s).

### Stufe 6: (4-Cyclopentyl-4-dimethylamino-1-nitromethylcyclohexyl)-essigsäureethylester

Eine Mischung von (4-Cyclopentyl-4-dimethylamino-cyclohexyliden)-essigsäureethylester (4.35 g, 15.6 mmol) und Tetra-n-butylammonium-fluorid-Trihydrat (5.36 g, 17 mmol) in wasserfreiem Tetrahydrofuran (37 mL) wurde mit Nitromethan (1.22 mL, 1.07 mL, 20 mmol) versetzt. Die Lösung wurde 7.5 h bei 70 °C und anschließend 18 h bei 45 °C gerührt. Das Gemisch wurde i. Vak. eingeengt. Der Rückstand (9.9 g) wurde durch Flashchromatographie (400 g, 20 x 7.5 cm) mit Cyclohexan / Ethylacetat (1:4) gereinigt.
Ausbeute: 3.04 g (57 %), gelbliches Öl
1H-NMR (CDCl3): 1.26 (0.3 H, t, J = 7.0 Hz); 1.27 (2.7 H, t, J = 7.1 Hz); 1.30-1.75 (16 H, m); 2.06 (1 H, m); 2.24 (6 H, s); 2.46 (0.2 H, s); 2.59 (1.8 H, s); 4.15 (2 H, q, J = 7.1 Hz); 4.58 (1.8 H, s); 4.81 (0.2 H, s)
13C-NMR (CDCl3): 14.2; 25.05; 24.14; 25.4; 28.1; 28.45; 28.50; 35.0; 36.8; 37.7; 43.6; 44.0; 44.1; 57.4; 60.1; 60.2; 84.3; 171.3.
Es handelt sich um ein Diastereoisomerengemisch.

### Stufe 7: 8-Cyclopentyl-8-dimethylamino-2-azaspiro[4.5]decan-3-on

Eine Lösung von (4-Cyclopentyl-4-dimethylamino-1-nitromethyl-cyclohexyl)-essigsäureethylester (3.04 g) in Methanol (50 mL) wurde mit 50-%- wässriger Raney-Nickel-Suspension (1.15 mL) versetzt. Die Suspension wurde 5 h bei 60 °C und einem Wasserstoffdruck von 5 bar gerührt. Die Suspension wurde durch Celite filtriert, der Filterrückstand mit Methanol (2 x 10 mL) gewaschen und das Filtrat i. Vak. eingeengt.
Ausbeute: 2.36 g (100 %), weißer Feststoff 1 H-NMR (CDCl3): 1.16-1.80 (16 H, m); 2.05 (1 H, m); 2.12 (0.3 H, s); 2.20 (1.7 H, s); 2.26 (6 H, s); 3.09 (1.7 H, s); 3.18 (0.3 H, s); 6.04 (1 H, br s).
Es handelt sich um ein Diastereoisomerengemisch im Verhältnis von ca. 7:1.

### Stufe 8: 8-Cyclopentyl-8-dimethylamino-3-oxo-2-azaspiro[4.5]decan-2-carbonsäure-tert-butylester

Eine Lösung von 8-Cyclopentyl-8-dimethylamino-2-azaspiro[4.5]decan-3-on (2.36 g, 8.92 mmol) in wasserfreiem Acetonitril (60 mL) wurde mit einer Lösung von Di-tert-butyldicarbonat (2.14 g, 9.83 mmol) in wasser-freiem Acetonitril (20 mL) und 4-Dimethylaminopyridin (69 mg, 0.87 mmol) versetzt und anschließend über Nacht bei 50 °C gerührt. Da der Umsatz (1H-NMR) nicht vollständig war, wurde weiteres Di-tert-butylcarbonat (2.14 g, 9.83 mmol) zugesetzt und weitere 18 h bei 50 °C gerührt. Das Gemisch wurde i. Vak. eingeengt und der Rückstand in Dichlormethan (100 mL) aufgenommen. Die Lösung wurde mit Wasser (3 x 80 mL) und gesättigter Natriumchlorid-Lösung (2 x 50 mL) gewaschen. Die organische Phase wurde mit Natriumsulfat getrocknet und i. Vak. eingeengt. Das Rohprodukt (3.54 g) wurde durch Flashchromatographie (200 g, 20 x 5.7 cm) mit Dichlormethan / Methanol (98: 2-95:5) gereinigt.

### Ausbeute:

### unpolares Diastereoisomer

Ausbeute: 1.74 g (53 %), gelblicher Feststoff
1H-NMR (CDCl3): 1.16-1.36 (6 H, m); 1.38-1.63 (6 H, m); 1.51 (9 H, s); 1.64-1.80 (4 H, m); 2.05 (1 H, m); 2.26 (6 H, s); 2.40 (2 H, s); 3.44 (2 H, s).

### polares Diastereoisomer

Ausbeute: 408 mg (12 %), gelbes Öl
1H-NMR (CDCl3): 1.10-1.85 (25 H, m); 2.06 (1 H, m); 2.25 (6 H, s); 2.32 (2 H, s); 3.54 (2 H, s).

### Stufe 9: 8-Cyclopentyl-8-dimethylamino-2-azaspiro[4.5]decan-3-on (unpolares Diastereoisomer)

Eine Lösung von 8-Cyclopentyl-8-dimethylamino-3-oxo-2-azaspiro[4.5]decan-2-carbonsäure-tert-butylester - unpolares Diastereoisomer (1.74 g, 4.77 mmol) in wasserfreiem Dichlormethan (75 mL) wurde mit Trifluoressigsäure (10 mL) versetzt und über Nacht bei Raumtemperatur gerührt. Das Gemisch wurde i. Vak. eingeengt, der Rückstand in Dichlormethan (150 mL) aufgenommen und die Lösung mit gesättigter Natriumhydrogencarbonat-Lösung (3 x 50 mL) gewaschen. Die wässrige Phase wurde mit einem Dichlormethan-Isopropanol-Gemisch (4:1, 3 x 50 mL) ex-trahiert. Die vereinigten organischen Phasen wurden mit Natriumsulfat getrocknet und i. Vak. eingeengt.
Ausbeute: 1.08 g (86 %), weißer Feststoff
1H-NMR (CDCl3): 1.16-1.82 (16 H, m); 2.06 (1 H, m); 2.21 (2 H, s); 2.26 (6 H, s); 3.10 (2 H, s); 5.86 (1 H, br s).

### Stufe 10: 8-Cyclopentyl-8-dimethylamino-2-azaspiro[4.5]decan-3-on (polares Diastereoisomer)

Eine Lösung von 8-Cyclopentyl-8-dimethylamino-3-oxo-2-azaspiro[4.5]decan-2-carbonsäure-tert-butylester - polares Diastereoisomer (446 mg, max. 1.22 mmol, verunreinigt) in wasserfreiem Dichlormethan (35 mL) wurde mit Trifluoressigsäure (3.38 mL) versetzt und 4 h bei Raumtemperatur gerührt. Das Gemisch wurde i. Vak. eingeengt, der Rückstand wurde in Dichlormethan (40 mL) aufgenommen und die Lösung mit gesättigter Natriumhydrogencarbonat-Lösung (3 x 30 mL) gewaschen. Die wässrige Phase wurde mit einem Dichlormethan-Isopropanol-Gemisch (4:1, 3 x 50 mL) extrahiert. Die vereinigten organischen Phasen wurden mit Natriumsulfat getrocknet und i. Vak. eingeengt. Der Rückstand (326 mg) wurde durch Flashchromatographie (38 g, 20 x 2.5 cm) mit Dichlormethan / Methanol (95:5) und 1 % Am-moniak (25 % in Wasser) gereinigt. Ausbeute: 176 mg (54 %), weißer Feststoff
1H-NMR (CDCl3): 1.16-1.32 (4 H, m); 1.36-1.62 (8 H, m); 1.63-1.82 (4 H, m); 2.06 (1 H, m); 2.14 (2 H, s); 2.26 (6 H, s); 3.20 (2 H, s); 5.81 (1 H, br s).

### Stufe 11: 8-Cyclopentyl-3-(2-cyclopropyl-ethyl)-8-(dimethyl-amino)-3-azaspiro[4.5]decan-2-on (Beispiel Nr. 234, unpolares Diastereoisomer)

Eine Suspension von Natriumhydroxid (96 mg, 2.39 mmol) in wasserfreiem Dimethylsulfoxid (5 mL) wurde 40 min bei Raumtemperatur gerührt, dann mit 8-Cyclopentyl-8-dimethylamino-2-azaspiro[4.5]decan-3-on (unpolares Diastereoisomer) (158 mg, 0.6 mmol) und anschließend mit einer Lösung von 2-Cyclopropylethyl 4-methylbenzensulfonat (144 mg, 0.6 mmol) in wasserfreiem Dimethylsulfoxid (2 mL) versetzt und 4 h bei Raumtemperatur gerührt. Das Reaktionsgemisch wurde erneut mit einer Lösung von 2-Cyclopropylethyl 4-methylbenzensulfonat (72 mg, 0.3 mmol) in wasserfreiem Dimethylsulfoxid (2 mL) versetzt und weitere 18 h bei Raumtemperatur gerührt. Das Gemisch wurde mit Wasser (150 mL) versetzt und mit Ethylacetat (3 x 50 mL) extrahiert. Die vereinigten organischen Phasen wurden mit Natriumsulfat getrocknet und i. Vak. eingeengt. Der Rückstand (300 mg) wurde durch Flashchromatographie (18 g, 20 x 2.0 cm) mit Dichlormethan / Methanol (98:2) gereinigt. Dadurch wurden 60 mg der Zielverbindung rein erhalten. Das durch die Flashchromatographie erhaltene, verunreinigte Produkt (90 mg) wurde in Ethylacetat (10 mL) aufgenommen und die Lösung mit 10 % wässriger Ameisen¬säure (4 x 20 mL) extrahiert. Die vereinigten sauren, wässrigen Phasen wurden mit 4 N Natronlauge alkalisch gestellt und mit Dichlormethan (4 x 20 mL) extrahiert. Die vereinigten organischen Phasen wurden mit Natriumsulfat getrocknet und i. Vak. eingeengt.

### Beispiel Nr. 234 (unpolares Diastereoisomer)

Ausbeute: 131 mg (66 %), gelbliches Öl
1H-NMR (CDCl3): 0.00-0.05 (2 H, m); 0.38-0.45 (2 H, m); 0.61 (1 H, m); 1.10-1.80 (18 H, m); 2.03 (1 H, m); 2.24 (6 H, s), 2.25 (2 H, s); 3.08 (2 H, s); 3.29 (2 H, t, J = 7.2 Hz).
13C-NMR (CDCl3): 4.2; 8.5; 25.1; 25.4; 25.8; 26.0; 26.9; 28.5; 31.6; 32.3; 32.4; 36.3; 37.8; 42.4; 42.5; 44.2; 57.7; 61.1; 173.7.
LC-MS: [M+H]+: m/z = 333.4, Rt = 2.6 min.

### Beispiel Nr. 272

### Stufe 1: 3-(8-Dimethylamino-8-phenyl-2-azaspiro[4.5]dec-2-yl)propionsäuremethylester

Zu einer Lösung von N,N-dimethyl-8-phenyl-2-azaspiro[4.5]decan-8-amin (600 mg, 2.32 mmol) in wasserfreiem Tetrahydrofuran (20 mL) wurde Methylacrylat (4.18 g, 4.20 mL, 46 mmol) gegeben und 18 h bei 50 °C gerührt. Das Reaktionsgemisch wurde im Vakuum eingeengt und der Rückstand (767 mg) durch Flashchromatographie (38 g, 20 × 2.5 cm) mit Dichlormethan / Methanol (95:5) und 0.5 % Ammoniak (25 % in Wasser) gereinigt.

### Stufe 2: 4-(8-Dimethylamino-8-phenyl-2-azaspiro[4.5]dec-2-yl)-2-methylbutan-2-ol (Beispiel Nr.272)

Eine Lösung von 3-(8-Dimethylamino-8-phenyl-2-azaspiro[4.5]dec-2-yl)propionsäuremethyl-ester (150 mg, 0.44 mmol) in wasserfreiem Tetrahydrofuran (5 mL) wurde unter Argon mit einer 1.4 M Lösung von Methylmagnesiumbromid (1.26 mL, 1.76 mmol) in Toluol / Tetrahydrofuran (3:1) versetzt und über Nacht bei Raumtemperatur gerührt. Die Lösung wurde mit 5 % wässriger Schwefelsäure (5 mL) versetzt und kräftig gerührt. Durch Zugabe von 2 M Kaliumcarbonat-Lösung wurde pH 10 eingestellt und das Gemisch mit Dichlormethan (3 × 30 mL) extrahiert. Die vereinigten organischen Phasen wurden mit Natriumsulfat getrocknet und i. Vak. eingeengt. Der Rückstand (130 mg) wurde in Ethylacetat (30 mL) aufgenommen und die Lösung anschließend mit 5 % wässriger Ameisensäure (3 × 20 mL) extrahiert. Die vereinigten wässrigen, sauren Phasen wurden mit 5 M Natronlauge auf pH 10 eingestellt und mit Ethylacetat (3 × 30 mL) extrahiert. Die vereinigten organischen Phasen wurden mit Natriumsulfat getrocknet und i. Vak. eingeengt.

### Analytische Daten von Beispiel 272:

¹H-NMR (CDCl3): 1.22 (6 H, s); 1.23-1.30 (2 H, m); 1.44 (2 H, t, *J* = 7.1 Hz); 1.56-1.60 (2 H, m); 1.60-1.70 (2 H, m); 1.80-1.97 (2 H, m); 2.01 (6 H, s); 2.10-2.26 (2 H, m); 2.52 (2 H, s); 2.57 (2 H, t, *J* = 7.1 Hz); 2.70-2.75 (2 H, m); 6.10-6.80 (1 H, br s); 7.22-7.30 (3 H, m); 7.32-7.39 (2 H, m).
¹³C-NMR (CDCl3): 29.6; 31.0; 34.2; 37.7; 38.0; 38.3; 41.2; 52.8; 53.4; 60.5; 65.4; 71.0; 126.4; 127.6; 127.61.
LC-MS (Methode 1): m/z: [M+H]⁺ = 345.4, Rₜ = 0.4 min.

### Beispiel Nr. 275

### 3-(2-Cyclopropyl-ethyl)-8-methylamino-8-phenyl-3-azaspiro[4.5]decan-2-on (Beispiel Nr. 275, unpolares Diastereoisomer)

Eine Lösung von 3-(2-Cyclopropyl-ethyl)-8-(dimethyl-amino)-8-phenyl-3-azaspiro[4.5]decan-2-on (Bsp. Nr. 184, unpolare Reihe, 350 mg, 1.03 mmol) in wasserfreiem Acetonitril (10 mL) wurde mit N-Iodsuccinimid (348 mg, 1.55 mmol) versetzt und 5 h bei Raumtemperatur gerührt. Anschließend wurde die Reaktionslösung mit 4 M Natronlauge (3 mL) versetzt und 20 min bei Raumtemperatur gerührt. Die Phasen wurden getrennt und die wässrige Phase mit Dichlormethan (2 x 10 mL) extrahiert. Die vereinigten organischen Phasen wurden i. Vak. eingeengt. Der Rückstand (550 mg) wurde in Methanol (5 mL) aufgenommen, die Lösung mit 2 M Salzsäure (2 mL) versetzt und 1 h bei Raumtemperatur gerührt. Anschließend wurde die Lösung mit Wasser (10 mL) verdünnt und mit Diethylether (3 x 10 mL) gewaschen. Die wässrige Lösung wurde mit 2 M Natronlauge alkalisch (pH∼10) gestellt und mit Dichlormethan (3 x 20 mL) extrahiert. Die vereinigten organischen Phasen wurden mit Natriumsulfat getrocknet und i. Vak. eingeengt. Das Rohprodukt (240 mg) wurde mittels Flashchromatographie an PharmPrep 60CC (12 g, 18 x1.5 cm) mit Dichlormethan / Methanol (50:1) + 0.5 % Ammoniak (25 % in Wasser) und anschließende erneute Flashchromatographie an Pharm-Prep 60CC (4 g, 14 x1.0 cm) mit Methanol gereinigt.

### Beispiel Nr. 275 (unpolares Diastereoisomer)

Ausbeute : 155 mg (46 %), farbloses Harz
1H-NMR (CDCl3): 0.04-0.08 (2 H, m); 0.42-0.49 (2 H, m); 0.59-0.70 (1 H, m); 1.42 (2 H, dd, J = 14.4 und 7.01 Hz); 1.45-1.53 (2 H, m); 1.73-1.99 (7 H, m); 2.00 (3 H, s); 2.24 (2 H, s); 3.22 (2 H, s); 3.32-3.37 (2 H, m); 7.18-7.28 (1 H, m); 7.32-7.39 (4 H, m).
13C-NMR (CDCl3): 4.3; 8.6; 28.6; 31.9; 32.4; 32.5; 35.8; 38.0; 42.6; 56.9; 59.7; 125.9; 126.5; 128.4; 173.5.
LC-MS: m/z: [M+H]+ = 327.3, Rt = 2.6 min.

### Beispiel Nr. 337

### Stufe 1: 8-Dimethylamino-8-[1.2.3]triazol-1-yl-2-azaspiro[4.5]decan-3-on

Eine Lösung von 2-Azaspiro[4.5]decane-3,8-dion (500 mg, 3 mmol) in wasserfreiem Tetrahydrofuran (30 mL) wurde mit einer 2 M Dimethylamin-Lösung in Tetrahydrofuran (1.8 mL, 3.6 mmol), 1,2,3-Triazol (228 mg, 191 µL, 3.3 mmol) und Molsieb 4 Å (1.00 g) versetzt und 18 h bei Raumtemperatur gerührt. Das Reaktionsgemisch wurde filtriert und das Filtrat i. Vak. eingeengt.
Ausbeute: 704 mg (89 %), weißer Feststoff

### Stufe 2: 8-(5-Chlorthiophen-2-yl)-8-dimethylamino-2-azaspiro[4.5]decan-3-on

Zu einer 0.5 M Suspension von 5-Chlor-2-thienylmagnesiumbromid (45 mL, 22.5 mmol) in Tetrahydrofuran wurde bei Raumtemperatur eine Suspension von 8-Dimethylamino-8-[1.2.3]triazol-1-yl-2-azaspiro[4.5]decan-3-on (2.10 g, maximal 8 mmol) in wasserfreiem Tetrahydrofuran (60 mL) getropft und dann 6 h bei 50 °C und 18 h bei Raumtemperatur gerührt. Nach Zugabe von gesättigter Ammoniumchlorid-Lösung (100 mL) wurden die Phasen getrennt und die wässrige Phase mit Ethylacetat (3 × 30 mL) extrahiert. Die vereinigten organischen Phasen wurden mit gesättigter Natriumchlorid-Lösung (50 mL) gewaschen, mit Natriumsulfat getrocknet, filtriert und i. Vak. eingeengt.
Ausbeute: 1.95 g (> 100 %), brauner Feststoff
Das 1H-NMR-Spektrum (CDCl3) zeigt alle erforderlichen Signale des Diastereoisomerengemisches.
LC-MS: m/z: [M+H]+ = 313.2, Rt = 2.3 min.

### Stufe 3: 8-(5-Chlorthiophen-2-yl)-8-dimethylamino-3-oxo-2-azaspiro[4.5]decan-2-carbonsäure-tert-butylester (polares und unpolares Diastereoisomer)

Eine Lösung von 8-(5-Chlorthiophen-2-yl)-8-dimethylamino-2-azaspiro[4.5]decan-3-on (1.70 g, maximal 8 mmol) in wasserfreiem Tetrahydrofuran (30 mL) und wasserfreiem Acetonitril (50 mL) wurde mit Di-tert-butyldicarbonat (1.92 g, 8.8 mmol) und 4-Dimethylaminopyridin (100 mg, 0.8 mmol) versetzt und 18 h bei Raumtemperatur gerührt. Nach 4 h und 1 d wurden jeweils Di-tert-butyldi-carbonat (1.92 g, 8.8 mmol) und Dimethylaminopyridin (100 mg, 0.8 mmol) zugegeben und anschließend 4d bei Raumtemperatur gerührt. Das Reaktionsgemisch wurde i. Vak. eingeengt, der Rückstand in Dichlormethan (100 mL) aufgenommen und die Lösung mit Wasser (3 × 50 mL) und gesättigter Natriumchlorid-Lösung (50 mL) gewaschen. Die organische Phase wurde mit Natriumsulfat getrocknet und i. Vak. eingeengt. Das Rohprodukt (1.70 g) wurde durch Flashchromatographie (100 g, 20 × 4.0 cm) mit Ethylacetat / Methanol (30:1) gereinigt.
unpolares Diastereoisomer
Ausbeute: 224 mg (7 %), braunes Öl
1H-NMR (CDCl3): 1.42-1.50 (3 H, m); 1.52 (9 H, s); 1.75-1.83 (2 H, m); 1.90-2.03 (3 H, m); 2.12 (6 H, s); 2.32 (2 H, s); 3.56 (2 H, s); 6.60 (1 H, d, J = 3.8 Hz); 6.85 (1 H, d, J = 3.8 Hz).
polares Diastereoisomer
Ausbeute: 260 mg (8 %), braunes Öl
1H-NMR (CDCl3): 1.44-1.50 (3 H, m); 1.50 (9 H, s); 1.70-1.78 (2 H, m); 1.88-2.03 (3 H, m); 2.11 (6 H, s); 2.43 (2 H, s); 3.44 (2 H, s); 6.61 (1 H, d, J = 3.8 Hz); 6.85 (1 H, d, J = 3.8 Hz).

### Stufe 4: 8-(5-Chlorthiophen-2-yl)-8-dimethylamino-2-azaspiro[4.5]decan-3-on

Eine Lösung von 8-(5-Chlorthiophen-2-yl)-8-dimethylamino-3-oxo-2-azaspiro[4.5]decan-2-carbonsäure-tert-butylester (unpolares Diastereoisomer) (224 mg, 0.54 mmol) in wasserfreiem Dichlormethan (20 mL) wurde mit Trifluoressigsäure (2 mL) versetzt und 3 h bei Raumtemperatur gerührt. Das Reaktionsgemisch wurde i. Vak. eingeengt, der Rückstand mit Dichlormethan (20 mL) versetzt und die Lösung mit gesättigter Natriumhydrogencarbonat-Lösung (3 × 20 mL) gewaschen. Die organische Phase wurde mit Natriumsulfat getrocknet und i. Vak. eingeengt.
Unpolares Diastereoisomer
Ausbeute: 126 mg (74 %), brauner Feststoff
Schmelzpunkt: 170-175 °C
1H-NMR (CDCl3): 1.46-1.55 (2 H, m); 1.76-1.85 (2 H, m); 1.90-2.01 (4 H, m); 2.11 (6 H, s); 2.14 (2 H, s); 3.22 (2 H, s); 5.55 (1 H, br s); 6.61 (1 H, d, J = 3.8 Hz); 6.85 (1 H, d, J = 3.8 Hz).
Eine Lösung von 8-(5-Chlorthiophen-2-yl)-8-dimethylamino-3-oxo-2-azaspiro[4.5]decan-2-car-bonsäure-tert-butylester (polares Diastereoisomer) (155 mg, 0.37 mmol) in wasserfreiem Dichlormethan (20 mL) wurde mit Trifluoressigsäure (2 mL) versetzt und 2 h bei Raumtemperatur gerührt. Das Reaktionsgemisch wurde i. Vak. eingeengt, der Rückstand mit Dichlormethan (20 mL) versetzt und mit gesättigter Natriumhydrogencarbonat-Lösung (3 × 20 mL) gewaschen. Die organische Phase wurde mit Natriumsulfat getrocknet und i. Vak. eingeengt.

### Polares Diastereoisomer

Ausbeute: 66 mg (56 %), brauner Feststoff
1H-NMR (CDCl3): 1.46-1.54 (2 H, m); 1.75-1.83 (2 H, m); 1.89-2.03 (4 H, m); 2.12 (6 H, s); 2.25 (2 H, s); 3.10 (2 H, s); 5.48 (1 H, br s); 6.61 (1 H, d, J = 3.8 Hz); 6.85 (1 H, d, J = 3.8 Hz).

### Stufe 5: 8-(5-Chlor-thiophen-2-yl)-8-(dimethyl-amino)-3-(3-methoxy-3-methyl-butyl)-3-azaspiro[4.5]decan-2-on (Beispiel Nr. 460, unpolares Diastereoisomer)

Eine Suspension von pulverisiertem, i. Vak. getrocknetem Natriumhydroxid (96 mg, 2.4 mmol) in wasserfreiem Dimethylsulfoxid (10 mL) wurde 40 min bei Raumtemperatur gerührt und dann mit 8-(5-Chlorthiophen-2-yl)-8-dimethylamino-2-azaspiro[4.5]decan-3-on (unpolare Reihe, 126 mg, 0.41 mmol) und 3-Methoxy-3-methylbut-1-yl-tosylat (136 mg, 0.5 mmol) versetzt. Danach wurde das Reaktionsgemisch 1 d bei Raumtemperatur gerührt. Nach Zugabe von Wasser (50 mL) wurde das Reaktionsgemisch mit Ethylacetat (3 × 30 mL) extrahiert. Die vereinigten organischen Phasen wurden mit Natriumsulfat getrocknet, filtriert und i. Vak. eingeengt. Der Rückstand wurde wiederholt mit Toluol versetzt und jeweils i. Vak. eingeengt. Das Rohprodukt (152 mg) wurde durch Flashchromatographie (10 g, 20 × 1.5 cm) mit Dichlormethan / Methanol (95:5) und 0.5 % Ammoniak (25 % in Wasser) gereinigt.

### Beispiel Nr. 337 (unpolares Diastereoisomer)

Ausbeute: 98 mg (58 %), gelber Feststoff
Schmelzpunkt: 86-90 °C
1H-NMR (CDCl3): 1.18 (6 H, s); 1.41-1.49 (2 H, m); 1.62-1.69 (2 H, m); 1.70-1.79 (2 H, m); 1.91-2.00 (4 H, m); 2.10 (6 H, s); 2.18 (2 H, s); 3.19 (3 H, s); 3.20 (2 H, s); 3.28-3.34 (2 H, m); 6.60 (1 H, d, J = 3.8 Hz); 6.84 (1 H, d, J = 3.8 Hz).
13C-NMR (CDCl3): 24.9; 32.4; 32.6; 35.5; 36.8; 38.0; 38.2; 44.3; 49.2; 58.0; 59.6; 73.5; 124.2; 124.5; 127.8; 173.2.
LC-MS: m/z: [M+H]+ = 414.3, Rt = 2.8 min.

### Beispiel Nr. 364

### Stufe 1: (8-Butyl-2-azaspiro[4.5]dec-8-yl)-dimethylamin

In einer ausgeheizten Apparatur wurde eine Aufschlämmung von Lithium-aluminiumhydrid (3.01 g, 79.2 mmol) in wasserfreiem Tetrahydrofuran (50 mL) unter Eiskühlung mit einer Lösung von Beispiel Nr. 19 (5.00 g, 19.8 mmol) in wasserfreiem Tetrahydrofuran (50 mL) versetzt, 18 h bei 50 °C und anschließend 72 h bei Raumtemperatur gerührt. Zum Reaktionsgemisch wurden unter Eiskühlung Wasser (3 mL), 15 % Natronlauge (3 mL) und erneut Wasser (9 mL) getropft und 2 h bei Raumtem¬peratur gerührt. Anschließend wurde die Suspension durch Seesand filtriert, der Rückstand mit Tetrahydrofuran gewaschen, das Filtrat mit Natriumsulfat getrocknet und i. Vak. eingeengt. Der Rückstand wurde mehrmals in Dichlormethan aufgenommen (3 x 25 mL) und die Lösung jeweils wieder i. Vak. wieder eingeengt.
Ausbeute: 4.71 g (100 %), gelbes Öl
1H-NMR (CDCl3): 0.87 (3 H, t, J = 7.1 Hz); 1.14-1.33 (10 H, m); 1.44-1.57 (8 H, m); 2.13 (6 H, s); 2.80 (2 H, t, J = 7.1 Hz); 3.65 (1 H, br s).

### Stufe 2: 1-(8-Butyl-8-dimethylamino-3-azaspiro[4.5]decan-3-yl)-2-cyclopropyl-ethanon (Beispiel Nr. 364, polares Diastereoisomer)

Eine Lösung von Cyclopropylessigsäure (137 mg, 1.4 mmol) in absolutem Tetrahydrofuran (10 mL) wurde mit N,N'-Carbonyldiimidazol (122 mg, 0.8 mmol) versetzt und 2 h unter Rückfluss gerührt. Danach wurde eine Lösung von (8-Butyl-2-azaspiro[4.5]dec-8-yl)-dimethylamin (238 mg, 1.0 mmol) in absolutem Tetrahydrofuran (10 mL) zugegeben und weitere 2 h unter Rückfluss gerührt. Die Reaktionslösung wurde anschließend i. Vak. eingeengt und der Rückstand in Ethylacetat (40 mL) aufgenommen. Die erhaltene Lösung wurde mit 1 M Kaliumcarbonat-Lösung (2 x 20 mL), Wasser (3 x 20 mL) und gesättigter Natriumchlorid-Lösung (20 mL) gewaschen, mit Natriumsulfat getrocknet und i. Vak. eingeengt. Das Rohprodukt (210 mg) wurde durch Flashchromatographie (10 g, 20 × 1.5 cm) mit Ethylacetat / Methanol (9:1) gereinigt.

### Beispiel Nr. 364 (polares Diastereoisomer)

Ausbeute: 93 mg (33 %), gelbes Öl
1H-NMR (CDCl3): 0.13-0.17 (2 H, m); 0.52-0.57 (2 H, m); 0.91 (3 H, t, J = 7.1 Hz); 1.04-1.13 (1 H, m); 1.16-1.46 (10 H, m); 1.50-1.67 (4 H, m, vom Wassersignal überlagert), 1.72 (0.8 H, t, J = 7.2 Hz); 1.78 (1.2 H, t, J = 7.1 Hz); 2.19 (2 H, m); 2.21 (3 H, s); 2.22 (3 H, s); 3.18 (1 H, s); 3.30 (1 H, s); 3.43 (0.8 H, t, J = 7.1 Hz); 3.52 (1.2 H, t, J = 7.2 Hz).
13C-NMR (CDCl3): 4.42; 4.44; 6.9; 7.0; 14.17; 14.20; 23.76; 23.81; 26.2, 26.6, 28.1; 28.9; 30.2; 30.5, 30.6; 30.8; 33.8; 36.3; 37.3; 37.4; 39.5; 39.9; 40.3; 42.2; 44.2; 45.3; 56.4; 56.5; 58.8; 171.40; 171.44.
LC-MS: m/z: [M+H]+ = 321.4 (100 %), Rt = 2.8 min.

### Beispiel Nr. 408

### Stufe 1: Dimethyl-(8-phenyl-2-azaspiro[4.5]dec-8-yl)amin

Zu einer Aufschlämmung von Lithiumaluminiumhydrid (114 mg, 3 mmol) in wasserfreiem Tetrahydrofuran (10 mL) wurde unter Argon eine Lösung von Beispiel 431 (8-Dimethylamino-8-phenyl-2-azaspiro[4.5]decan-3-on) (unpolares Diastereoisomer 327 mg, 1.2 mmol) in wasserfreiem Tetrahydrofuran (25 mL) getropft und anschließend 18 h bei Raumtemperatur gerührt. Da die Umsetzung nicht vollständig war, wurde das Gemisch auf 50 °C erwärmt und weitere 3 h gerührt. Nach Zugabe von Wasser (200 µL), 1 M Natronlauge (500 µL) und Wasser (500 µL) wurde 1 h gerührt und die Suspension durch Seesand filtriert. Das Filtrat wurde mit Natriumsulfat getrocknet und i. Vak. eingeengt.
Ausbeute: 310 mg (100 %), farbloses Öl
1H-NMR (CDCl3): 1.23-1.32 (2 H, m); 1.54-1.62 (2 H, m); 1.65 (2 H, t, J = 7.1 Hz); 1.85-1.96 (2 H, m); 2.04 (6 H, s); 2.05-2.11 (1 H, m); 2.23-2.35 (2 H, m); 2.53 (2 H, s); 2.95 (2 H, t, J = 7.1 Hz); 7.26-7.32 (3 H, m); 7.34-7.40 (2 H, m).

### Stufe 2: 2-Cyclopropyl-1-[8-(dimethyl-amino)-8-phenyl-3-azaspiro[4.5]decan-3-yl]-ethanon (Beispiel Nr. 408, unpolares Diastereoisomer)

Eine Lösung von Cyclopropylessigsäure (100 mg, 96 µL, 1 mmol) in wasserfreiem Tetrahydrofuran (20 mL) wurde mit N,N'-Carbonyldiimidazol (130 mg, 0.8 mmol) versetzt und 2 h unter Rückfluss gerührt. Nach Zugabe von Dimethyl-(8-phenyl-2-azaspiro[4.5]dec-8-yl)amin (155 mg, 0.6 mmol) in wasserfreiem Tetrahydrofuran (5 mL) wurde die Lösung weitere 2 h unter Rückfluss gerührt. Das Reaktionsgemisch wurde anschließend i. Vak. eingeengt, der Rückstand in Ethylacetat (30 mL) aufgenommen und die Lösung mit 2 M Kaliumcarbonat-Lösung (3 × 30 mL), Wasser (3 × 30 mL) und gesättigter Natriumchlorid-Lösung (30 mL) gewaschen. Die organische Phase wurde abgetrennt, mit Natriumsulfat getrocknet, filtriert und das Filtrat i. Vak. eingeengt. Das Rohprodukt (210 mg) wurde durch Flashchromatographie (10 g, 20 × 1.5 cm) mit Dichlormethan / Methanol (95:5) und 0.5 % Ammoniak (25 % in Wasser) gereinigt.

### Beispiel Nr. 408 (unpolares Diastereoisomer)

Ausbeute: 65 mg (32 %), farbloses Öl
1H-NMR (CDCl3): 0.06-0.16 (2 H, m); 0.47-0.57 (2 H, m); 0.97-1.12 (1 H, m); 1.24-1.40 (3 H, m); 1.60-1.70 (3 H, m); 1.80 (1 H, t, J = 7.2 Hz); 1.88 (1 H, t, J = 7.1 Hz); 2.04 (6 H, s); 2.07-2.22 (4 H, m); 3.06 und 3.18 (2 H, 2 s); 3.46 (1 H, t, J = 7.1 Hz); 3.54 (1 H, t, J = 7.2 Hz); 7.26-7.32 (3 H, m); 7.33-7.41 (2 H, m).
13C-NMR (CDCl3): 4.36; 4.42; 30.4; 30.5; 31.1; 34.2; 38.1; 39.4; 39.8; 40.3; 42.3; 44.2; 45.3; 58.0; 60.6; 126.7; 127.4; 127.5; 127.7; 171.4.
LC-MS: m/z: [M+H]+ = 341.3, Rt = 2.8 min.

### Beispiel Nr. 417

### Stufe 1: [4-Dimethylamino-4-(5-fluorthiophen-2-yl)-cyclohexyliden]-essigsäureethylester

Eine Lösung von Phosphonoessigsäure-triethylester (2.73 g, 2.42 mL, 12.2 mmol) in wasserfreiem N,N-Dimethylformamid (15 mL) wurde mit Kalium-tert-butanolat (1.21 g, 10.8 mmol) versetzt und 1 h bei 50 °C gerührt. Die Lösung wurde auf Raumtemperatur abgekühlt und anschließend mit einer Lösung von 4-(Dimethylamino)-4-(5-fluorothiophen-2-yl)cyclohexanon (1.85 g, 7.66 mmol) in wasserfreiem N,N-Dimethylformamid (20 mL) versetzt. Das Reaktionsgemisch wurde 20 h bei Raumtemperatur gerührt und anschließend in Eiswasser (30 g) gegossen. Die Suspension wurde mit Diethylether (4 x 20 mL) extrahiert. Die vereinigten organischen Phasen wurden mit Natriumsulfat getrocknet und i. Vak. eingeengt. Der Rückstand wurde wiederholt mit Toluol, anschließend wiederholt mit Cyclohexan versetzt und jeweils wieder i. Vak. eingeengt. Der Rückstand (2.42 g) wurde in Ethylacetat (30 mL) aufgenommen und die Lösung mit 10 % wässriger Ameisensäure (5 x 30 mL) extrahiert. Die vereinigten sauren, wässrigen Phasen wurden mit 4 N Natronlauge alkalisch gestellt und mit Dichlormethan (5 x 30 mL) extrahiert. Die vereinigten organischen Phasen wurden mit Natriumsulfat getrocknet und i. Vak. eingeengt.
Ausbeute: 2.0 g (84 %), gelbliches Öl
1H-NMR (CDCl3): 1.27 (3 H, t, J = 7.1 Hz); 1.90-2.08 (2 H, m); 2.09-2.28 (4 H, m); 2.17 (6 H, s); 2.82-2.96 (1 H, m); 3.00-3.15 (1 H, m); 4.14 (2 H, q, J = 7.1 Hz); 5.62 (1 H, s); 6.38 (1 H, dd, J = 3.9 und 1.6 Hz); 6.47 (1 H, t, J = 3.5 Hz).

### Stufe 2: [4-Dimethylamino-4-(5-fluotthiophen-2-yl)-1-nitromethyl-cyclohexyl]-essigsäureethylester

Eine Lösung von [4-Dimethylamino-4-(5-fluorthiophen-2-yl)-cyclohexyliden]-essigsäureethylester (500 mg, 1.6 mmol) in Tetrahydrofuran (30 mL) wurde mit Tetra-n-butylammoniumfluorid-Trihydrat (555 mg, 1.8 mmol) und Nitromethan (5.40 g, 4.79 mL, 88 mmol) versetzt, 3 h bei 70 °C und dann 18 h bei 45 °C gerührt. Die Reaktionslösung wurde anschließend i. Vak. eingeengt und der Rückstand (1.31 g) durch Flashchromatographie an sphärischem Kieselgel (PuriFlash PF-50SIHP, 50 µm, 100 g, 20 × 4.0 cm) mit Cyclohexan / Ethylacetat (2:1→1:1) gereinigt. Die Zielverbindung fällt als Diastereoisomerengemisch (ca. 1:1) an.
Ausbeute: 415 mg (70 %), hellgelbes zähes Öl
1H-NMR (CDCl3): 1.24 und 1.26 (3 H, 2 t, J = 7.1 Hz); 1.46-1.53 (2 H, m); 1.77-1.86 (2 H, m); 1.93-2.01 (4 H, m); 2.10 (6 H, s); 2.48 (1 H, s); 2.60 (1 H, s); 4.12 und 4.16 (2 H, 2 q, J = 7.2 Hz); 4.64 (1 H, s); 4.76 (1 H, s); 6.38-6.43 (2 H, m).
LC-MS: m/z: [M+H]+= 373.32 (55 %) und [MH-NHMe2]+= 328.2 (100 %), Rt = 2.7 und 2.9 min.

### Stufe 3: 8-Dimethylamino-8-(5-fluorthiophen-2-yl)-2-azaspiro[4.5]decan-3-on

Zu einer Mischung aus Eisenpulver (307 mg, 5.5 mmol), Ammoniumchlorid (1.42 g, 28 mmol) und Wasser (1.1 mL) wurde eine Lösung von [4-Dimethylamino-4-(5-fluorthiophen-2-yl)-1-nitromethyl-cyclohexyl]-essigsäureethylester (415 mg, 1.1 mmol) in Ethanol (10 mL) gegeben und anschließend 4 h bei 80 °C gerührt. Die Reaktionsmischung wurde filtriert und der Rückstand mit Ethanol gewaschen. Die ethanolische Lösung wurde mit 5 % Natriumhydrogencarbonat-Lösung alkalisch gestellt und das Ethanol i. Vak. entfernt. Die wässrige Suspension wurde mit Dichlormethan (3 × 20 mL) extrahiert, die vereinigten organischen Phasen wurden mit gesättigter Natriumchlorid-Lösung (20 mL) gewaschen, durch Phasentrennpapier filtriert und das Filtrat i. Vak. eingeengt. Das Rohprodukt (306 mg) wurde vollständig cyclisiert.

### Stufe 4: 8-Dimethylamino-8-(5-fluorthiophen-2-yl)-2-azaspiro[4.5]decan-3-on

Eine Lösung von Stufe 3 (900 mg, 3.0mmol) in wasserfreiem Tetrahydrofuran (40 mL) wurde mit Kalium-tert-butylat (419 mg, 3.7 mmol) versetzt und 18 h über Nacht bei Raumtemperatur gerührt. Die Reaktionslösung wurde anschließend mit gesättigter Ammoniumchlorid-Lösung (5 mL) versetzt, i. Vak. eingeengt und der Rückstand mit Wasser (50 mL) versetzt. Die wässrige Lösung wurde mit Dichlormethan (4 × 30 mL) extrahiert, die vereinigten organischen Phasen wurden mit gesättigter Natriumchlorid-Lösung (30 mL) gewaschen, durch Phasentrennpapier filtriert und das Filtrat i. Vak. eingeengt.
Ausbeute: 662 mg (66 %), hellgelber, zäher Schaum
1H-NMR (CDCl3): 1.47-1.54 (2 H, m); 1.75-1.83 (2 H, m); 1.90-2.00 (4 H, m); 2.116 (3 H, s); 2.119 (3 H, s); 2.14 (1 H, s); 2.25 (1 H, s); 3.10 (1 H, s); 3.22 (1 H, s); 5.55 (1 H, br s); 6.38-6.40 (1 H, m); 6.42-6.44 (1 H, m).
LC-MS: m/z: [M+H]+= 297.3(60 %) und [MH-NHMe2]+= 252.2 (100 %), Rt = 1.8 min.
Es handelt sich um ein Diastereoisomerengemisch im Verhältnis von ca. 1:1.

### Stufe 5: 8-Dimethylamino-8-(5-fluorthiophen-2-yl)-3-oxo-2-azaspiro[4.5]decan-2-carbonsäure-tert-butylester (polares und unpolares Diastereoisomer)

Eine Lösung von 8-Dimethylamino-8-(5-fluorthiophen-2-yl)-2-azaspiro[4.5]decan-3-on (660 mg, Rohprodukt, maximal 2.2 mmol) in einer Mischung von wasserfreiem Tetrahydrofuran (25 mL) und wasserfreiem Acetonitril (25 mL) wurde mit Di-tert-butyldicarbonat (528 mg, 2.4 mmol) und 4 Dimethylaminopyridin (25 mg, 0.2 mmol) versetzt und 18 h bei 50 °C gerührt. Die Reaktionslösung wurde anschließend i. Vak. eingeengt und das Diastereoisomerengemisch (890 mg) durch Flashchromatographie an sphärischem Kieselgel (PuriFlash PF-50SIHP, 50 µm, 38 g, 20 × 2.5 cm) mit Ethylacetat, welches 1 % Methanol enthielt, getrennt.

### Unpolares Diastereoisomer

Ausbeute: 447 mg (51 %), hellgelber Feststoff
Schmelzpunkt: 130-132 °C
1 H-NMR (CDCl3): 1.43-1.51 (2 H, m); 1.53 (9 H, s); 1.75-1.81 (2 H, m); 1.89-2.02 (4 H, m); 2.12 (6 H, s); 2.33 (2 H, s); 3.56 (2 H, s); 6.39 (1 H, dd, J = 1.6 und 4.1 Hz); 6.42-6.43 (1 H, m).

### Polares Diastereoisomer

Ausbeute: 337 mg (41 %), hellgelber Feststoff
Schmelzpunkt: 152-155 °C
1H-NMR (CDCl3): 1.46-1.51 (2 H, m, überlagert); 1.51 (9 H, s); 1.71-1.77 (2 H, m); 1.88-2.03 (4 H, m); 2.11 (6 H, s); 2.43 (2 H, s); 3.45 (2 H, s); 6.39 (1 H, dd, J = 1.5 und 4.1 Hz); 6.42-6.44 (1 H, m).

### Stufe 6: 8-Dimethylamino-8-(5-fluorthiophen-2-yl)-2-azaspiro[4.5]decan-3-on (unpolares Diastereoisomer)

Eine Lösung von 8-Dimethylamino-8-(5-fluorthiophen-2-yl)-3-oxo-2-azaspiro[4.5]decan-2-carbonsäure-tert-butylester (unpolare Reihe, 430 mg, 1.1 mmol) in absolutem Dichlomethan (10 mL) wurde mit Trifluoressigsäure (2.5 mL, 25 % v / v) versetzt und 1 h bei Raumtemperatur gerührt. Die Reaktionslösung wurde anschließend i. Vak. eingeengt und der Rückstand in Dichlomethan (50 mL) aufgenommen. Die Lösung wurde mit 1 M Kaliumcarbonat-Lösung (3 × 30 mL) und gesättigter Natriumchlorid-Lösung (30 mL) gewaschen, durch Phasentrennpapier filtriert und das Filtrat i. Vak. eingeengt.

### Unpolares Diastereoisomer:

Ausbeute: 248 mg (77 %), hellrosafarbener Feststoff
Schmelzpunkt: 198-204 °C
1H-NMR (CDCl3): 1.48-1.54 (2 H, m); 1.76-1.83 (2 H, m); 1.91-1.97 (4 H, m); 2.11 (6 H, s); 2.14 (2 H, s); 3.22 (2 H, s); 5.70 (1 H, br s); 6.39 (1 H, dd, J = 1.7 und 4.0 Hz); 6.42-6.44 (1 H, m).
13C-NMR (CDCl3): 32.0; 32.1; 32.4; 38.0; 38.9; 43.0; 52.5; 59.5; 106.3; 106.4; 121.1; 162.5; 165.4; 177.2.
LC-MS: m/z: [M+H]+= 297.2 (72 %) und [MH-NHMe2]+= 252.2 (100 %), Rt = 1.7 min.

### Stufe 7: 1-[8-Dimethylamino-8-(5-fluor-thiophen-2-yl)-3-azaspiro[4.5]decan-3-yl]-3-methoxy-3-methyl-butan-1-on (Beispiel Nr. 549, unpolares Diastereoisomer)

Eine Suspension von pulverisiertem, i. Vak. getrocknetem Natriumhydroxid (70 mg, 1.8 mmol) in absolutem Dimethylsulfoxid (5 mL) wurde 20 min bei Raumtemperatur gerührt. Anschließend wurden hierzu eine Lösung von 8-Dimethylamino-8-(5-fluorthiophen-2-yl)-2-azaspiro[4.5]decan-3-on (unpolares Diastereoisomer, 130 mg, 0.44 mmol) in absolutem Dimethylsulfoxid (5 mL) sowie eine Lösung von 3-Methoxy-3-methylbut-1-yl-tosylat (144 mg, 0.53 mmol) in Dimethylsulfoxid (5 mL) gegeben und 4 h bei 80 °C gerührt. Die Reaktionslösung wurde danach mit Wasser (50 mL) versetzt und mit Ethylacetat (4 × 20 mL) extrahiert. Die vereinigten organischen Phasen wurden mit gesättigter Natriumchlorid-Lösung (20 mL) gewaschen, durch Phasentrennpapier filtriert und das Filtrat i. Vak. eingeengt. Der Rückstand wurde mehrmals nacheinander mit Toluol und Dichlormethan (je 3 x 10 mL) versetzt und jeweils wieder i. Vak. bei 60 °C eingeengt, um Dimethylsulfoxid restlos zu entfernen. Das Rohprodukt (154 mg) wurde mittels Flashchromatographie an sphärischem Kieselgel (PuriFlash PF-50SIHP, 50 µm, 5 g, 15 × 0.9 cm) mit Ethylacetat / Methanol (9:1) gereinigt.

### Beispiel Nr. 417 (unpolares Diastereoisomer)

Ausbeute: 116 mg (67 %), weißer Feststoff
Schmelzpunkt: 93 °C
1H-NMR (CDCl3): 1.18 (6 H, m); 1.43-1.49 (2 H, m); 1.64-1.68 (2 H, m); 1.71-1.77 (2 H, m); 1.91-1.97 (4 H, m); 2.11 (6 H, s); 2.19 (2 H, s); 3.19 (3 H, s); 3.21 (2 H, s); 3.30-3.34 (2 H, m); 6.39 (1 H, dd, J = 1.7 und 3.9 Hz); 6.41-6.43 (1 H, m).
13C-NMR (CDCl3): 24.9; 32.2; 32.6; 35.5; 36.8; 38.0; 38.3; 44.4; 49.2; 59.5; 73.6; 106.3; 106.4; 121.1; 162.5; 165.4; 173.3.
LC-MS: m/z: [M+H]+= 397.3 (100 %) und [MH-NHMe2]+= 352.3 (35 %), Rt = 2.6 min.

### Beispiel Nr. 424

### Stufe 1: 8-Cyclopent-1-enyl-8-dimethylamino-2-azaspiro[4.5]decan-1-on

Eine Lösung von 8-(Dimethylamino)-1-oxo-2-azaspiro[4.5]decane-8-carbonitril (958 mg, 4.32 mmol) in wasserfreiem Tetrahydrofuran (20 mL) wurde tropfenweise mit der Lösung von Cyclopentenylmagnesiumbromid (maximal 17 mmol) versetzt und 1 h bei Raumtemperatur gerührt. Das Gemisch wurde auf 60 °C erwärmt und 1 h bei dieser Temperatur gerührt. Die Suspension wurde unter Eiskühlung mit gesättigter Ammoniumchlorid-Lösung (25 mL) und Wasser (20 mL) versetzt. Die Phasen wurden getrennt und die wässrige mit Ethylacetat (2 x 30 mL) extrahiert. Die vereinigten organischen Phasen wurden mit Natriumsulfat getrocknet und i. Vak. eingeengt. Der Rückstand (900 mg) wurde durch Flashchromatographie (85 g, 4.0 x 20 cm) mit Dichlormethan / Methanol (9:1) und 1 % Ammoniak (25 % in Wasser) gereinigt.
Ausbeute: 527 mg (46 %), weißer Feststoff
1H-NMR (CDCl3): 1.18-1.26 (2 H, m); 1.31-1.41 (2 H, m); 1.75-1.85 (2 H, m); 1.97 (2 H, t, J = 6.9 Hz); 2.01-2.10 (2 H, m); 2.11-2.20 (2 H, m); 2.18 (6 H, s); 2.22-2.36 (4 H, m); 3.25-3.30 (2 H, m); 5.44 (1 H, m); 6.38 (1 H, br s).
13C-NMR (CDCl3): 23.6; 28.2; 29.1; 31.9; 32.9; 34.0; 38.3; 38.6; 38.8; 43.2; 56.9; 125.8; 146.0; 183.2.
LC-MS: [M+H]+: m/z = 263.4, Rt = 2.3 min.

### Stufe 2: 8-Cyclopentyl-8-dimethylamino-2-azaspiro[4.5]decan-1-on

Eine Lösung von 8-Cyclopent-1-enyl-8-dimethylamino-2-azaspiro[4.5]decan-1-on (2.5 g, 9.5 mmol) in wasserfreiem Methanol (20 mL) wurde mit 5 % Rhodium auf Aluminiumoxid (960 mg, 0.47 mmol) versetzt und 3 h bei einem Wasserstoffdruck von 2 bar gerührt. Das Gemisch wurde erneut mit Methanol (20 mL) versetzt und weitere 2 h bei einem Wasserstoffdruck von 2 bar gerührt. Da das Edukt noch nicht umgesetzt war, wurde das Reaktionsgemisch mit Methanol (110 mL) verdünnt, erneut mit 5 % Rhodium auf Aluminiumoxid (1.92 g, 0.95 mmol) versetzt und 20 h bei einem Wasserstoffdruck von 4 bar hydriert. Die Suspension wurde durch Celite filtriert, der Rückstand wurde mit Methanol gewa-schen und das Filtrat i. Vak. eingeengt. Der Rückstand wurde zwischen Ethylacetat und 10 % Citronensäure-Lösung (je 40 mL) verteilt. Die organische Phase wurde mit 10 % Citronensäure-Lösung (3 x 80 mL) gewaschen. Die vereinigten sauren, wäss-rigen Phasen wurden mit 4 M Natronlauge alkalisch gestellt und mit Dichlormethan (4 x 50 mL) extrahiert. Die vereinigten organischen Phasen wurden mit Natriumsul¬fat getrocknet und i. Vak. eingeengt. Das Rohprodukt wurde durch Flashchroma-tographie (85 g, 20 x 4.0 cm) mit Dichlormethan / Methanol (95:5) und 1 % Ammo-niak (25 % in Wasser) gereinigt.
Ausbeute: 757 mg (30 %), weißer Feststoff
1H-NMR (CDCl3):1.14 (2 H, dd, J = 11.8 und 1.2 Hz); 1.20-1.34 (4 H, m); 1.40-1.63 (6 H, m); 1.73 (2 H, dd, J = 14.9 und 2.8 Hz); 1.98-2.14 (5 H, m); 2.28 (6 H, s); 3.29-3.30 (2 H, m); 6.20 (1 H, s).
13C-NMR (CDCl3): 25.0; 26.5; 27.3; 28.3; 31.9; 37.9; 38.9; 44.2; 44.4; 57.4; 183.4.
LC-MS: [M+H]+: m/z = 265.4.4, Rt = 2.2 min.

### Stufe 3: 8-Cyclopentyl-8-dimethylamino-2-azaspiro[4.5]decan

Eine Suspension von Lithiumaluminiumhydrid (542 mg, 14.3 mmol) in wasserfreiem Tetrahydrofuran (10 mL) wurde unter Eiskühlung tropfenweise mit einer Lösung von 8-Cyclopentyl-8-dimethylamino-2-azaspiro[4.5]decan-1-on (758 mg, 2.8 mmol) in wasserfreiem Tetrahydrofuran (30 mL) versetzt. Die Suspension wurde 4 h bei 50 °C gerührt. Das Gemisch wurde unter Eiskühlung mit Wasser (560 µL), 1 M Natronlauge (1.1 mL) und erneut mit Wasser (1.1 mL) versetzt. Die Suspension wurde 1 h bei Raumtemperatur gerührt und anschließend durch Natriumsulfat filtriert. Der Rückstand wurde mit Tetrahydrofuran gewaschen und das Filtrat i. Vak. eingeengt.
Ausbeute: 689 mg (96 %), farbloses Öl
1H-NMR (CDCl3): 1.11-1.20 (2 H, m); 1.22-1.36 (4 H, m); 1.40-1.70 (12 H, m); 1.98 (1 H, br s); 2.05 (1 H, m); 2.26 (6 H, s); 2.61 (2 H, s); 2.93 (2 H, t, J = 7.0 Hz).
LC-MS: [M+H]+: m/z = 251.4, Rt = 0.3 min.

### Beispiel Nr. 425

### Stufe 1: 8-(5-Chlor-thiophen-2-yl)-8-dimethylamino-2-azaspiro[4.5]decan-4-on

Zu einer 0.5 M Aufschlämmung von 5-Chlor-2-thienylmagnesiumbromid (5.29 g, 48 mL, 23.9 mmol) in Tetrahydrofuran wurde unter Argon langsam eine Aufschlämmung von 8-(Dimethylamino)-1-oxo-2-azaspiro[4.5]decane-8-carbonitril (1.76 g, 7.9 mmol) in absolutem Tetrahydrofuran (75 mL) getropft, wobei sich eine klare Lösung bildete. Anschließend wurde über Nacht bei 50 °C gerührt. Nach Zugabe von gesättigter Ammoniumchlorid-Lösung (100 mL) wurde das Tetrahydrofuran i. Vak. entfernt. Die erhaltene wässrige Lösung wurde mit Dichlormethan (3 × 50 mL) extrahiert, die vereinigten organischen Phasen wurden mit gesättigter Natriumchlorid-Lösung (50 mL) gewaschen, mit Natriumsulfat getrocknet und i. Vak. eingeengt. Das Rohprodukt (2.45 g) wurde mittels Flashchromatographie (100 g, 20 × 4.0 cm) mit Ethylacetat / Methanol (97:3) gereinigt.
Ausbeute: 1.47 g (59 %), gelber Feststoff
Schmelzpunkt: 198-201 °C
1H-NMR (CDCl3): 1.28-1.34 (2 H, m); 1.61-1.68 (2 H, m); 2.01 (2 H, t, J = 6.9 Hz); 2.12 (6 H, s); 2.17 (2 H, dt, J = 13.1 und 3.1 Hz), 2.32-2.40 (2 H, m); 3.28-3.32 (2 H, m); 5.90 (1 H, br s); 6.60 (1 H, d, J = 3.8 Hz); 6.83 (1 H, d, J = 3.8 Hz).
13C-NMR (CDCl3): 27.9; 31.5; 32.7; 37.9; 38.7; 43.1; 58.9; 123.1; 125.2, 127.4; 144.4; 182.4.
LC-MS: m/z: [MH-HNMe2]+ = 268.2, Rt = 2.6 min.

### Stufe 2: [8-(5-Chlor-2-thiophen-2-yl)-2-aza-spiro[4.5]dec-8-yl]-dimethylamin (Beispiele Nr. 425)

Eine Lösung von 8-(5-Chlor-thiophen-2-yl)-8-dimethylamino-2-azaspiro[4.5]decan-4-on (1.34 g, 4.3 mmol) in absolutem Tetrahydrofuran (150 mL) wurde mit einer 2 M Lösung des Boran-Dimethylsulfid-Komplexes in Tetrahydrofuran (6.42 mL, 12.8 mmol) versetzt und 4 h unter Rückfluss und über Nacht bei 50 °C gerührt. Da die Umsetzung noch nicht vollständig war, wurde nochmals dieselbe Menge 2 M Boran-Dimethylsulfid-Komplex zugegeben und weitere 6 h unter Rückfluss und über das Wochenende bei Raumtemperatur gerührt. Die Reaktionslösung wurde mit Wasser (100 mL) versetzt und i. Vak. eingeengt. Der Rückstand wurde nacheinander mit Toluol, Methanol und Dichlormethan (jeweils 3 × 30 mL) versetzt und wiederum i. Vak. eingeengt. Das Rohprodukt wurde ohne Reinigung weiter umgesetzt.
Ausbeute: 1.95 g (151 %), zähes gelbes Öl
Das 1 H-NMR-Spektrum zeigt alle erwarteten Signale.
LC-MS: m/z: [MH-HNMe2]+ = 254.3, Rt = 2.7 min.
Der Gehalt an Produkt beträgt maximal 66 %.

### Beispiel Nr. 426

### Stufe 1: 8-Dimethylamino-8-(5-fluor-thiophen-2-yl)-2-aza-spiro[4.5]decan-2-carbonsäure-tert-butylester

In einer ausgeheizten Apparatur wurde bei -78 °C unter Argon zu einer Lösung von 8-Dimethylamino-8-thiophen-2-yl-3-azaspiro[4.5]decan-3-carbonsäure tert-butyl ester (Beispiel Nr. 79) (1.55 g, 4.3 mmol) in absolutem Tetrahydrofuran (100 mL) eine 2.5 M Lösung von n-Butyllithium in Hexan (2.2 mL, 5.5 mmol) getropft und 30 min bei dieser Temperatur gerührt. Die Lösung färbte sich gelb. Hierzu wurde eine Lösung von N-Benzolsulfonyl-N-fluorbenzolsulfonamid (1.74 g, 5.5 mmol) in absolutem Tetrahydrofuran (50 mL) getropft, anschießend langsam auf Raumtemperatur erwärmt und 18 h bei dieser Temperatur weiter gerührt. Die Lösung färbte sich rot. Nach Zugabe von gesättigter Ammoniumchlorid-Lösung (50 mL) wurde das Tetrahydrofuran i. Vak. entfernt. Die erhaltene wässrige Lösung wurde mit Dichlormethan (3 × 30 mL) extrahiert, die vereinigten organischen Phasen wurden mit gesättigter Natriumchlorid-Lösung (50 mL) gewaschen, mit Natriumsulfat getrocknet und i. Vak. eingeengt. Das Rohprodukt (2.50 g) wurde durch Flashchromatographie an sphärischem Kieselgel (PharmPrep 60 CC, 40-63 µm, 100 g, 20 × 4.0 cm) mit Ethylacetat / Isopropanol (99:1) gereinigt.
Ausbeute: nicht bestimmbar da verschiedene Mischfraktionen mit unterschiedlichem Reinheitsgrad erhalten wurden, orangefarbenes zähes Öl
1H-NMR (CDCl3): 1.34-1.42 (2 H, m); 1.46 (9 H, s); 1.57-1.66 (4 H, m); 1.78-1.97 (4 H, m); 2.11 (2 H, s); 2.13 (4 H, s); 3.18 (0.7 H, s); 3.22 (1.3 H, s); 3.32 (0.7 H, t, J = 7.1 Hz); 3.37 (1.3 H, t, J = 7.1 Hz); 6.35-6.40 (1 H, m); 6.42 (1 H, t, J = 3.5 Hz).
13C-NMR (DMSO-d6): 28.6; 31.3; 32.1; 32.9; 36.6; 37.0; 38.1; 40.7; 41.5, 44.0; 44.4; 55.6, 60.2; 79.1; 106.3; 121.3; 154.8; 162.5; 165.4.
Einige C-Signale verdoppeln sich aufgrund der Amidstruktur (Rotamere). Aus diesem Grunde wurden auch keine C-F-Kopplungskonstanten bestimmt.
LC-MS: m/z: [MH-NHMe2]+ = 383.4, Rt = 3.3 min.

### Stufe 2: [8-(5-Fluor-thiophen-2-yl)-2-aza-spiro[4.5]dec-8-yl]-dimethylamin (Beispiel 426)

Eine Lösung von 8-Dimethylamino-8-(5-fluor-thiophen-2-yl)-2-aza-spiro[4.5]decan-2-carbonsäure-tert-butylester (1.35 g, max. 3.5 mmol, leicht verunreinigt) in absolutem Dichlormethan (60 mL) wurde mit Trifluoressigsäure (15 mL) versetzt und 1 h bei Raumtemperatur gerührt. Die Reaktionslösung wurde i. Vak. eingeengt und der Rückstand mit Dichlormethan (50 mL) versetzt. Die erhaltene Lösung wurde mit gesättigter Kaliumhydrogencarbonat-Lösung (3 × 30 mL) und gesättigter Natriumchlorid-Lösung (50 mL) gewaschen, mit Natriumsulfat getrocknet und i. Vak. eingeengt. Das Rohprodukt wurde ohne Reinigung weiter umgesetzt.
Ausbeute: 738 mg (Rohprodukt), orangefarbenes zähes Öl
1H-NMR (CDCl3): 1.43 (2 H, ddd, J = 13.1, 8.1 und 4.9 Hz); 1.61 (2 H, t, J = 7.3 Hz); 1.68-1.74 (2 H, m); 1.86-1.99 (4 H, m); 2.10 (6 H, s); 2.88 (2 H, s); 3.09 (2 H, t, J = 7.3 Hz); 5.02 (1 H, br. s); 6.38 (1 H, dd, J = 4.0 und 1.7 Hz); 6.42 (1 H, dd, J = 4.0 und 3.1 Hz).

### Beispiel Nr. 427

### Stufe 1: 2-[4-(Azetidin-1-yl)-4-(2-thienyl)cyclohexyliden]-essigsäureethylester

Eine Lösung von Phosphonoessigsäure-triethylester (5.60 g, 4.8 mL, 25.1 mmol) in wasserfreiem N,N-Dimethylformamid (30 mL) wurde unter Argon mit Kalium-tert-butylat (2.82 g, 25.1 mmol) versetzt und 10 min bei Raumtemperatur gerührt. Anschließend wurde das Gemisch mit einer Lösung von 4-(Azetidin-1-yl)-4-(thiophen-2-yl)cyclohexanon (3.96 g, 16.8 mmol) in wasserfreiem N,N-Dimethylformamid (60 mL) versetzt, 1 h bei Raumtemperatur gerührt und dann in Eiswasser (80 g) gegossen. Die wässrige Suspension wurde mit Diethylether (4 × 40 mL) extrahiert. Die vereinigten organischen Extrakte wurden mit Natriumsulfat getrocknet und i. Vak. eingeengt.
Ausbeute: 4.79 g (93 %), bräunliches Öl
1H-NMR (CDCl3): 1.26 (t, 3H, J = 7.1 Hz); 1.76-1.85 (m, 2H); 1.87-2.02 (m, 4H); 2.12-2.20 (m, 1H); 2.44-2.57 (m, 1H); 2.89-3.05 (m, 2H); 3.11 (t, 4H, J = 6.9 Hz); 4.13 (q, 2H, J = 7.1 Hz); 5.61 (br s, 1H); 6.89 (d, 1H, J = 3.5 Hz); 7.08 (dd, 1H, J = 5.1, 1.5 Hz); 7.25-7.28 (m, 1H, vom CDCl3-Signal überlagert).

### Stufe 2: 2-[4-(Azetidin-1-yl)-1-(nitromethyl)-4-(2-thienyl)cyc/ohexyl]-essigsäureethylester

Eine Mischung von 2-[4-(Azetidin-1-yl)-4-(2-thienyl)cyclohexyliden]-essigsäureethylester (4.79 g, 15.7 mmol) und Tetra-n-butylammoniumfluorid-Trihydrat (5.43 g, 17.2 mmol) in Tetrahydrofuran (150 mL) wurde mit Nitromethan (1.24 g, 1.09 mL, 20.3 mmol) versetzt und 6 h bei 70 °C und 18 h bei 45 °C gerührt. Das Reaktionsgemisch wurde anschließend i. Vak. eingeengt und das Rohprodukt (12.0 g) durch Flashchromatographie (200 g, 20 × 5.7 cm) mit Ethylacetat / Cyclohexan (9:1) gereinigt.

Ausbeute: 4.18 g (74 %), gelbliches Öl
1H-NMR (DMSO-d6): 1.10-1.24 (m, 3H); 1.37-1.47 (m, 2H); 1.63-1.86 (m, 8H); 2.42 und 2.46 (2 s, 2H); 2.92-2.99 (m, 4H); 3.98-4.05 (m, 2H); 4.68 und 4.69 (2 s, 2H); 6.96 (dt, 1H, J = 3.5, 1.1 Hz); 7.09-7.12 (m, 1H); 7.47 (dd, 1H, J = 5.1, 1.0 Hz).
Es handelt sich um ein Diastereoisomerengemisch im Verhältnis von ca. 2:3.

### Stufe 3: 8-(Azetidin-1-yl)-8-(2-thienyl)-3-azaspiro[4.5]decan-2-on

Zu einer Mischung aus Eisenpulver (2.84 g, 53 mmol), Ammoniumchlorid (14.2 g, 265 mmol) und Wasser (10 mL) wurde eine Lösung aus 2-[4-(Azetidin-1-yl)-1-(nitromethyl)-4-(2-thienyl)cyclohexyl]-essigsäureethylester (3.90 g, 10.7 mmol) in Ethanol (100 mL) gegeben und anschließend 4 h bei 80 °C gerührt. Das Gemisch wurde filtriert und der Rückstand mit Ethanol gewaschen. Das Filtrat wurde durch Zugabe von 5 % Natriumhydrogencarbonat-Lösung (8 mL) alkalisch gestellt und dann i. Vak. eingeengt. Das Rohprodukt (6.30 g) wurde durch Flash¬chromatographie (200 g, 20 × 5.7 cm) mit Dichlormethan / Methanol (95:5) und 1 % Ammoniak (25 % in Wasser) gereinigt. Das isolierte Gemisch aus unpolarem und polarem Diastereoisomer (1.60 g) wurde durch Mitteldruckchromatographie bei 8-10 bar an einer puriFlash-Kartusche (PF-15SIHP, 40 g, 15 µm) und 2 puriFlash-Kartuschen (PF-15SIHP, 120 g, 15 µm) mit Dichlormethan / Isopropanol (9:1) und 1 % Ammoniak (25 % in Wasser) gereinigt.

### Unpolares Diastereoisomer

Ausbeute: 504 mg (16 %), weißer Feststoff
Schmelzpunkt: 180-183 °C
1H-NMR (DMSO-d6): 1.31-1.40 (m, 2H); 1.63-1.77 (m, 8H); 2.02 (s, 2H); 2.93 (s, 2H); 2.96 (t, 4H, J = 6.9 Hz); 6.95 (d, 1H, J = 3.5 Hz); 7.10 (dd, 1H, J = 8.6, 3.5 Hz); 7.41 (br s, 1H); 7.46 (d, 1H, J = 5.1 Hz).

### Polares Diastereoisomer

Ausbeute: 772 mg (25 %),weißer Feststoff
Schmelzpunkt: 170-172 °C
1H-NMR (DMSO-d6): 1.30-1.40 (m, 2H); 1.62-1.82 (m, 8H); 1.93 (s, 2H); 2.96 (t, 4H, J = 6.9 Hz); 3.03 (s, 2H); 6.95 (dd, 1H, J = 3.5 Hz, 1.1 Hz); 7.10 (dd, 1H, J = 5.1, 3.5 Hz); 7.45 (br s, 1H); 7.46 (dd, 2H, J = 5.1 Hz, 1.0 Hz).

### Stufe 4: 8-(Azetidin-1-yl)-8-(2-thienyl)-3-azaspiro[4.5]decan (polares Diastereomer) (Beispiel 427)

Eine Lösung von 8-(Azetidin-1-yl)-8-(2-thienyl)-3-azaspiro[4.5]decan-2-on (polares Diastereoisomer) (765 mg, 2.63 mmol) in wasserfreiem Tetrahydrofuran (50 mL) wurde bei 0 °C unter Argon zu einer Aufschlämmung von Lithiumaluminiumhydrid (500 mg, 13.1 mmol) in wasserfreiem Tetrahydrofuran (20 mL) getropft und dann bei 60 °C über Nacht gerührt. Nach Zugabe von Wasser (500 µL), 1 N Natronlauge (1.3 mL) und nochmals Wasser (1.3 mL) wurde das Gemisch eine Stunde bei Raumtemperatur gerührt, danach durch Seesand filtriert, das Filtrat mit Natriumsulfat getrocknet und i. Vak. eingeengt.
Ausbeute: 696 mg (96 %), farbloses Öl
1H-NMR (CDCl3): 1.35 (ddd, 2H, J = 13.1, 9.4, 3.7 Hz); 1.40-1.46 (m, 3H), 1.60-1.90 (m, 8H); 2.75 (s, 2H); 2.89 (t, 2H, J = 7.1 Hz); 3.07 (t, 4H, J = 7.0 Hz); 6.88 (dd, 1H, J = 3.5, 1.1 Hz); 7.09 (dd, 1H, J = 5.1, 3.5 Hz); 7.27 (dd, 1H, J = 5.1, 1.1 Hz).

### Beispiel Nr. 428

### 8-(Azetidin-1-yl)-8-(2-thienyl)-3-azaspiro[4.5]decan (unpolares Diastereomer) (Beispiel Nr. 428)

Eine Lösung von 8-(Azetidin-1-yl)-8-(2-thienyl)-3-azaspiro[4.5]decan-2-on (unpolares Diastereoisomer) (504 mg, 1.73 mmol) in wasserfreiem Tetrahydrofuran (50 mL) wurde bei 0 °C unter Argon zu einer Aufschlämmung von Lithiumaluminiumhydrid (330 mg, 8.65 mmol) in wasserfreiem Tetrahydrofuran (20 mL) getropft und dann über Nacht bei 60 °C gerührt. Nach Zugabe von Wasser (300 µL), 1 N Natronlauge (800 µL) und nochmals Wasser (800 µL) wurde das Gemisch 1 h bei Raumtemperatur gerührt und danach durch Seesand filtriert. Das Filtrat wurde mit Natriumsulfat getrocknet und i. Vak. einge-engt.
Ausbeute: 414 mg (87 %), Öl
1H-NMR (CDCl3): 1.35 (ddd, 2H, J = 13.4, 9.9, 3.7 Hz); 1.56-1.64 (m, 3H); 1.70-1.93 (m, 8H); 2.55 (s, 2H); 2.94 (t, 2H, J = 7.1 Hz); 3.08 (t, 4H, J = 7.1 Hz); 6.87 (dd, 1H, J = 3.5, 1.1 Hz); 7.08 (dd, 1H, J = 5.1, 3.5 Hz); 7.27 (dd, 1H, J = 5.1, 1.1 Hz).

### Beispiel Nr. 429

### Stufe 1: (4-Azetidin-1-yl-4-phenylcyclohexyliden)essigsäureethylester

Eine Lösung von Phosphonoessigsäure-triethylester (7.03 g, 6.2 mL, 31.4 mmol) in wasserfreiem N,N-Dimethylformamid (30 mL) wurde unter Argon mit Kalium-tert-butylat (3.52 g, 31.4 mmol) versetzt und 10 min bei Raumtemperatur gerührt. Anschließend wurde das Gemisch mit einer Lösung von 4-(Azetidin-1-yl)-4-phenylcyclohexanon (4.81 g, 21 mmol) in wasserfreiem N,N-Dimethylformamid (60 mL) versetzt, 1 h bei Raumtemperatur gerührt und dann in Eiswasser (80 g) gegossen. Die wässrige Suspension wurde mit Diethylether (4 × 40 mL) extrahiert. Die vereinigten organischen Extrakte wurden mit Natriumsulfat getrocknet und i. Vak. eingeengt.
Ausbeute: 6.30 g (100 %), gelbliches Öl
1H-NMR (DMSO-d6): 1.18 (t, 3H, J = 7.1 Hz); 1.65 (quin, 2H, J = 7.0 Hz); 1.75-1.90 (m, 2H); 1.96-2.10 (m, 3H); 2.73-2.82 (m, 2H); 2.88-2.96 (m, 1H); 2.90 (t, 4H, J = 6.9 Hz); 4.05 (q, 2H, J = 7.1 Hz); 5.62 (s, 1H); 7.23-7.45 (m, 5H).

### Stufe 2: (4-Azetidin-1-yl-1-nitromethyl-4-phenylcyclohexyl)essigsäureethylester

Eine Mischung von (4-Azetidin-1-yl-4-phenylcyclohexyliden)essigsäureethylester (6.30 g, 21 mmol) und Tetra-n-butylammoniumfluorid-Trihydrat (7.26 g, 23 mmol) in Tetrahydrofuran (150 mL) wurde mit Nitromethan (1.65 g, 1.45 mL, 27.1 mmol) versetzt und 6 h bei 70 °C und 18 h bei 45 °C gerührt. Da die Umsetzung nicht vollständig war, wurden nochmals Tetra-n-butylammonium-fluorid-Trihydrat (2.42 g, 7.6 mmol) und Nitromethan (550 mg, 483 µL, 9 mmol) zugegeben und weitere 5 h bei 70 °C und 18 h bei 45 °C gerührt. Das Reaktionsgemisch wurde i. Vak. eingeengt und der Rückstand (17.0 g) durch Flashchromatographie (200 g, 20 × 5.7 cm) mit Ethylacetat / Methanol (95:5) gereinigt.
Ausbeute: 4.92 g (65 %), bräunliches Öl
1H-NMR (DMSO-d6): 1.10 und 1.18 (2 t, 3H, J = 7.1 Hz); 1.30-1.42 (m, 2H); 1.62 (t, 2H, J = 6.8 Hz); 1.70-1.80 (m, 4H); 1.85-1.95 (m, 2H); 2.36 (s, 1H); 2.84 (t, 4H, J = 6.8 Hz); 3.95-4.08 (m, 2H); 4.63 und 4.73 (m, 2H); 7.26-7.45 (m, 5H).
LC-MS: m/z: [M+H]+ = 361.4, Rt = 2.6 und 2.7 min.
Es liegt ein Diastereoisomerengemisch im Verhältnis von 4:3 vor.

### Stufe 3: 8-Azetidin-1-yl-8-phenyl-2-azaspiro[4.5]decan-3-on

Zu einer Mischung aus Eisenpulver (3.58 g, 67 mmol), Ammoniumchlorid (17.9 g, 334 mmol) und Wasser (13 mL) wurde eine Lösung aus (4-Azetidin-1-yl-1-nitromethyl-4-phenylcyclohexyl)essigsäureethylester (4.92 g, 13.5 mmol) in Ethanol (130 mL) gegeben und anschließend 4 h bei 80 °C und über Nacht bei 65 °C gerührt. Das Gemisch wurde filtriert und der Filterrückstand mit Ethanol gewaschen. Das Filtrat wurde durch Zugabe von 5 % Natriumhydrogenca¬bonat-Lösung (8 mL) alkalisch gestellt und dann i. Vak. eingeengt. Der Rückstand (10.0 g) wurde durch Flashchromatographie (400 g, 20 × 7.5 cm) mit Dichlormethan / Methanol (95:5) und 1 % Ammoniak (25 % in Wasser) gereinigt. Das isolierte Gemisch aus unpolarem und polarem Diastereoisomer (1.80 g) wurde durch Flashchromatographie an zwei Säulen mit PharmPrep (40-63 µm, 200 g, 20 × 5.7 cm) und die dabei erhaltenen Mischfraktionen (670 mg) an einer puriFlash-Kartusche (PF-15SIHP, 200 g, 15 µm) jeweils mit Dichlormethan / Ethanol (95:5) und 1 % Ammoniak (25 % in Wasser) gereinigt.

### Unpolares Diastereoisomer

Ausbeute: 719 mg (19 %), weißer Feststoff
Schmelzpunkt: 180-187 °C
1H-NMR (DMSO-d6): 1.21-1.31 (m, 2H); 1.56-1.84 (m, 8H); 2.06 (s, 2H); 2.85 (t, 4H, J = 6.8 Hz); 2.88 (s, 2H); 7.22-7.46 (m, 6H).
LC-MS: m/z: [M+H]+ = 285.4, Rt = 1.9 min.

### Polares Diastereoisomer

Ausbeute: 907 mg (24 %), weißer Feststoff
Schmelzpunkt: 150-155 °C
1H-NMR (DMSO-d6): 1.20-1.33 (m, 2H); 1.58-1.87 (m, 8H); 1.88 (s, 2H); 2.84 (t, 4H, J = 6.8 Hz); 3.07 (s, 2H); 7.25-7.49 (m, 6H).
LC-MS: m/z: [M+H]+ = 285.4, Rt = 1.8 min.

### Stufe 4: 8-Azetidin-1-yl-8-phenyl-2-azaspiro[4.5]decan (polares Diastereoisomer) (Beispiel 429)

Eine Lösung von 8-Azetidin-1-yl-8-phenyl-2-azaspiro[4.5]decan-3-on (polares Diastereoisomer) (892 mg, 3.14 mmol) in wasserfreiem Tetrahydrofuran (80 mL) wurde bei 0 °C unter Argon zu einer Aufschlämmung von Lithiumaluminiumhydrid (599 mg, 15.7 mmol) in wasserfreiem Tetrahydrofuran (20 mL) getropft und dann bei 60 °C über Nacht gerührt. Nach Zugabe von Wasser (500 µL), 1 N Natronlauge (1.3 mL) und nochmals Wasser (1.3 mL) wurde das Gemisch eine Stunde bei Raumtemperatur gerührt, danach durch Seesand filtriert, das Filtrat mit Natriumsulfat getrocknet und i. Vak. eingeengt.
Ausbeute: 830 mg (98 %), farbloses Öl
1H-NMR (DMSO-d6): 1.12-1.22 (m, 2H); 1.23-1.30 (m, 2H); 1.52-1.66 (m, 4H); 1.70-1.81 (m, 3H); 2.53 (s, 2H); 2.70 (t, 2H, J = 7.1 Hz); 2.82 (t, 4H, J = 6.8 Hz); 3.34-3.42 (m, 2H); 7.24-7.34 (m, 3H); 7.37-7.43 (m, 2H).
LC-MS: m/z: [M+H]+ = 271.4, Rt = 0.4 min.

### Beispiel Nr. 430

### 8-Azetidin-1-yl-8-phenyl-2-azaspiro[4.5]decan (unpolares Diastereomer)

Eine Lösung von 8-Azetidin-1-yl-8-phenyl-2-azaspiro[4.5]decan-3-on (unpolares Diastereoisomer) (701 mg, 2.46 mmol) in wasserfreiem Tetrahydrofuran (100 mL) wurde bei 0 °C unter Argon zu einer Aufschlämmung von Lithiumaluminiumhydrid (470 mg, 12.3 mmol) in wasserfreiem Tetrahydrofuran (20 mL) getropft und dann bei 60 °C über Nacht gerührt. Nach Zugabe von Wasser (500 µL), 1 N Natronlauge (1.3 mL) und nochmals Wasser (1.3 mL) wurde das Gemisch eine Stunde bei Raumtemperatur gerührt, danach durch Seesand filtriert, das Filtrat mit Natriumsulfat getrocknet und i. Vak. eingeengt.
Ausbeute: 663 mg (95 %), farbloses Öl
1H-NMR (DMSO-d6): 1.10-1.20 (m, 2H); 1.48 (t, 2H, J = 7.0 Hz); 1.50-1.66 (m, 4H); 1.70-1.80 (m, 3H); 2.34 (s, 2H); 2.74 (t, 2H, J = 7.0 Hz); 2.84 (t, 4H, J = 6.8 Hz); 3.20-3.40 (m, 2H); 7.23-7.34 (m, 3H); 7.36-7.42 (m, 2H).
LC-MS: m/z: [M+H]+ = 271.4, Rt = 0.2 min.

### Beispiel Nr. 431

### 8-Dimethylamino-8-phenyl-2-azaspiro[4.5]decan-3-on (polares Diastereomer)

Eine Lösung von 8-Dimethylamino-3-oxo-8-phenyl-2-azaspiro[4.5]decan-2-carbonsäure-tert-butylester (polares Diastereoisomer) (1.28 g, 3.43 mmol) in wasserfreiem Dichlormethan (50 mL) wurde mit Trifluoressigsäure (5 mL) versetzt und 3 h bei Raumtemperatur gerührt. Das Reaktionsgemisch wurde i. Vak. eingeengt, der Rückstand in Dichlormethan (50 mL) gelöst und die Lösung mit gesättigter Natriumhydrogencarbonat-Lösung (3 × 20 mL) gewaschen. Die organische Phase wurde mit Natriumsulfat getrocknet und i. Vak. eingeengt.
Ausbeute: 875 mg (94 %), weißer Feststoff
Schmelzpunkt: 220-222 °C
1H-NMR (CDCl3): 1.34-1.44 (2 H, m); 1.72-1.81 (2 H, m); 1.86-2.02 (2 H, br s); 2.04 (6 H, s); 2.16-2.29 (2 H, m); 2.30 (2 H, s); 3.01 (2 H, s); 5.60 (1 H, s); 7.26-7.32 (3 H, m); 7.36-7.41 (2 H, m).

### Beispiel Nr. 432 und 433

### Stufe 1: 8-Butyl-8-dimethylamino-2-azaspiro[4.5]decan-3-on

Zu einer 2 M Lösung von n-Butylmagnesiumchlorid in wasserfreiem Tetrahydrofuran (20 mL, 40 mmol) wurde bei 0 °C unter Argon eine Suspension von 8-(Dimethylamino)-3-oxo-2-azaspiro[4.5]decane-8-carbonitril (2.21 g, 10 mmol) in wasserfreiem Tetrahydrofuran (140 mL) getropft und 20 h bei Raumtemperatur gerührt. Anschließend wurde die Lösung mit gesättigter Ammoniumchlorid-Lösung (50 mL) versetzt. Die Phasen wurden getrennt und die wässrige mit Dichlormethan (3 x 20 mL) extrahiert. Die vereinigten organischen Phasen wurden mit Natriumsulfat getrocknet und i. Vak. eingeengt. Das Rohprodukt (3.97 g) wurde in Dichlormethan aufgenommen und die Suspension mit Kaliumcarbonat-Lösung gewaschen. Anschließend wurde die organische Phase mit Natriumsulfat getrocknet und i. Vak. eingeengt.
Ausbeute: 1.88 g (75 %), farbloses Öl, das mit der Zeit kristallisierte 1H-NMR (CDCl3): 0.90 und 0.91 (3 H, 2 t, J = 7.2 Hz); 1.14-1.47 (10 H, m); 1.51-1.61 (2 H, m); 1.67-1.82 (2 H, m); 2.18 und 2.19 (2 H, 2 s); 2.21 (s, 6 H); 3.15 und 3.18 (2 H, 2 s); 5.90 und 5.93 (1 H, br s).
Es handelt um Diastereoisomerengemisch im Verhältnis von ca. 1:1.
LC/MS: m/z: [M+H]+ = 253.3, Rt = 1.3 min.

### Stufe 2: 8-Butyl-8-dimethylamino-3-oxo-2-azaspiro[4.5]decan-2-carbonsäure-tert-butylester (polares und unpolares Diastereoisomer)

Eine Lösung von 8-Butyl-8-dimethylamino-2-azaspiro[4.5]decan-3-on (1.84 g, 7.3 mmol) in wasserfreiem Acetonitril (60 mL) und wasserfreiem Tetrahydrofuran (20 mL) wurde mit Di-tert-Butyldicarbonat (2.71 g, 12.4 mmol) und 4-Dimethylaminopyridin (90 mg, 0.75 mmol) versetzt. Das Reaktionsgemisch wurde 72 h bei 50 °C gerührt. Anschließend wurde es i. Vak. eingeengt. Der Rückstand wurde in Dichlormethan (100 ml) aufgenommen und die Lösung mit Wasser (3 x 80 mL) und gesättigter Natriumchlorid-Lösung (50 mL) gewaschen. Die organische Phase wurde mit Natriumsulfat getrocknet und i. Vak. eingeengt. Das Rohprodukt (2.37 g) wurde durch Flashchromatographie (220 g, 20 x 5.7 cm) mit Dichlormethan/ Methanol (95:5-9:1-4:1) gereinigt.

### Unpolares Diastereoisomer:

Ausbeute: 819 mg (32 %), orangefarbener Feststoff
1H-NMR (CDCl3): 0.90 (3 H, t, J = 7.1 Hz); 1.17-1.40 (10 H, m); 1.51 (9 H, s); 1.54-1.76 (4 H, m); 2.21 (6 H, s); 2.39 (2 H, s); 3.49 (2 H, s).

### Polares Diastereoisomer:

Ausbeute: 647 mg (25 %), gelbes Öl
1H-NMR (CDCl3): 0.90 (3 H, t, J = 7.1 Hz); 1.22-1.48 (10 H, m); 1.53 (9 H, s); 1.58-1.76 (4 H, m); 2.25 (6 H, s); 2.39 (2 H, s); 3.52 (2 H, s).

### Mischfraktion:

Ausbeute: 310 mg (12 %), gelbes Öl

### Stufe 3: 8-Butyl-8-dimethylamino-2-azaspiro[4.5]decan-3-on (polares Diastereoisomer) (Beispiel Nr. 432)

Eine Lösung von 8-Butyl-8-dimethylamino-3-oxo-2-azaspiro[4.5]decan-2-carbonsäure-tert-butylester - polares Diastereoisomer (603 mg, 1.71 mmol) in wasserfreiem Dichlormethan (50 mL) wurde mit Trifluoressigsäure (12.5 mL) versetzt und 4 h bei Raumtemperatur gerührt. Das Reaktionsgemisch wurde anschließend i. Vak. eingeengt, der Rückstand in Dichlormethan aufgenommen (50 mL) und die Lösung mit 25 % Kaliumcarbonat-Lösung (3 x 20 mL) gewaschen. Die organische Phase wurde mit Natriumsulfat getrocknet und i. Vak. eingeengt.

### Polares Diastereoisomer:

Ausbeute: 365 mg (85 %), gelblicher Feststoff
1H-NMR (CDCl3): 0.90 (3 H, t, J = 7.2 Hz); 1.11-1.48 (10 H, m); 1.53-1.64 (2 H, m); 1.69-1.79 (2 H, m); 2.17 (2 H, s); 2.21 (6 H, s); 3.17 (2 H, s); 6.10 (br s, 1 H).
13C-NMR (CDCl3): 14.1; 23.7; 26.5; 28.3; 30.7 (2 C); 31.9 (2 C); 37.3 (2 C); 39.0; 44.0; 52.6; 56.2; 177.9.

### Stufe 4: 8-Butyl-8-dimethylamino-2-azaspiro[4.5]decan-3-on (unpolares Diastereoisomer) (Beispiel Nr. 433)

Eine Lösung von 8-Butyl-8-dimethylamino-3-oxo-2-azaspiro[4.5]decan-2-carbonsäure-tert-butylester -unpolares Diastereoisomer (740 mg, 2.09 mmol) in wasserfreiem Dichlormethan (50 mL) wurde mit Trifluoressigsäure (12.5 mL) versetzt und 2 h bei Raumtemperatur gerührt. Das Reaktionsgemisch wurde anschließend i. Vak. eingeengt, der Rückstand in Dichlormethan aufgenommen (50 mL) und die Lösung mit 25 % Kaliumcarbonat-Lösung (3 x 20 mL) gewaschen. Die organische Phase wurde mit Natriumsulfat getrocknet und i. Vak. eingeengt.

### Unpolares Diastereoisomer:

Ausbeute: 416 mg (79 %), gelber Feststoff
1H-NMR (CDCl3): 0.90 (3 H, t, J = 7.2 Hz); 1.16-1.43 (10 H, m); 1.58-1.78 (4 H, m); 2.19 (2 H, s); 2.22 (6 H, s); 3.14 (2 H, s); 5.97 (br s, 1 H).
13C-NMR (CDCl3): 14.1; 23.8; 26.6; 28.6; 30.6 (2 C); 31.8 (2 C); 37.3 (2 C); 39.1; 42.1; 54.6; 177.7.

Entsprechend den dargestellten allgemeinen Synthesevorschriften und in Analogie zu den exemplarisch aufgeführten konkreten Synthesebeispielen wurden aus den polaren und unpolaren Vorstufen (8-Benzyl-8-(dimethyl-amino)-3-azaspiro[4.5]decan-4-on, (8-Benzyl-3-azaspiro[4.5]decan-8-yl)-dimethyl-amin, 8-Dimethylamino-8-phenyl-3-azaspiro[4.5]decan-4-on, 8-(Dimethyl-amino)-8-phenyl-3-azaspiro[4.5]decan-2-on, 8-Butyl-8-dimethylamino-3-azaspiro[4.5]decan-4-on, 8-Dimethylamino-8-thiophen-2-yl-3-azaspiro[4.5]decan-4-on, Dimethyl-(8-thiophen-2-yl-3-azaspiro[4.5]decan-8-yl)-amin, 8-(Dimethyl-amino)-8-thiophen-2-yl-2-azaspiro[4.5]decan-3-on, 8-(Dimethyl-amino)-8-(5-methyl-thiophen-2-yl)-3-azaspiro[4.5]decan-4-on, Dimethyl-[8-(5-methyl-thiophen-2-yl)-3-azaspiro[4.5]decan-8-yl]-amin, Dimethyl-(8-phenyl-3-azaspiro[4.5]decan-8-yl)-amin, 8-(Cyclohexyl-methyl)-8-dimethylamino-3-azaspiro[4.5]decan-4-on, 8-(Cyclopentylmethyl)-N, N-dimethyl-2-azaspiro[4.5]decan-8-amin, 8-Cyclopentyl-N,N-dimethyl-2-azaspiro[4.5]decan-8-amin, 8-(Azetidin-1-yl)-8-(thiophen-2-yl)-2-azaspiro[4.5]decan, 8-(Azetidin-1-yl)-8-phenyl-2-azaspiro[4.5]decan, 8-(5-Chlor-thiophen-2-yl)-8-dimethylamino-3-azaspiro[4.5]decan-4-on, 8-(Dimethyl-amino)-8-(5-methyl-thiophen-2-yl)-3-azaspiro[4.5]decan-2-on) die folgenden Beispiele durch Acylierung, Arylierung, Alkylierung, reduktive Aminierung oder Reduktion von Amiden hergestellt.

| **Bsp Nr.** | **Diastereomer*** | **Building Block/ Methode/ Ausbeute** | **LC-MS [M+H]+/ RT** | **NMR-spectrum** |
|---|---|---|---|---|
| 36 | 1 | Bsp. Nr. 31/ Acylierung/ 19% | [M+H]+ = 361.2, Rt = 3.3 min. | 1H-NMR (CDCl3): 1.37 (2 H, m); 1.53-1.65 (4 H, m); 1.81-1.93 (4 H, m); 2.10 (9 H, m); 2.29 (2 H, m); 2.44 (3 H, s); 3.14 (2 H, m); 3.32 (2 H, m); 3.43 (2 H, t); 6.60 (2 H, m). |
| 37 | 1 | Bsp. Nr. 31/ Acylierung/ 18% | [M+H]+ = 347.1. Rt = 2.7 min. | 1H-NMR (CDCl3): 0.73 (2 H, m); 0.96 (2 H, m); 1.40-1.46 (2 H, m); 1.56-1.73 (5 H, m); 1.89 (2 H, m); 2.09 (4 H, s); 2.12 (4 H, s); 2.45 (3 H, s); 3.35 (1 H, s); 3.49 (3 H, t); 3.62 (1 H, t); 6.65 (2 H, m). |
| 38 | 1 | Bsp. Nr. 31/ Acylierung/ 18% | [M+H]+ = 349.2, Rt = 2.9 min. | 1H-NMR (CDCl3): 1.10 (6 H, d); 1.37-1.44 (2 H, m); 1.57-1.71 (4 H, m); 1.80-1.94 (2 H, m); 2.01 (1 H, m); 2.11 (7 H, m); 2.45 (3 H, s); 2.59 (1 H, m); 3.33 (2 H, m); 3.45 (2 H, m); 6.64 (2 H, m). |
| 39 | 1 | Bsp. Nr. 31/ Acylierung/ 28% | [M+H]+ = 363.2, Rt = 3.3 min. | 1H-NMR (DMSO-d6): 0.94 (6 H, m); 1.40 (2 H, m); 1.56-1.69 (4 H, m); 1.95 (2 H, m); 2.05 (3 H, m); 2.12 (8 H, m); 2.45 (3 H, s); 3.30 (1 H, s); 3.35 (1 H, s); 3.45 (2 H, m); 6.62 (1 H, m); 6.67 (1 H, m). |
| 40 | 1 | Bsp. Nr. 31/ Acylierung/ 27% | [M+H]+ = 335.2, Rt = 2.7 min | 1H-NMR (CDCl3): 1.12 (3 H, t); 1.39 (2 H, m); 1.58 (4 H, m); 1.84 (2 H, m); 2.06 (4 H, s); 2.09 (4 H, s); 2.22 (2 H, m); 2.43 (3 H, s); 3.25 (1 H, s); 3.33 (1 H, s); 3.40 (2 H, m); 6.62 (2 H, m). |
| 42 | 1 | Bsp. Nr. 18/ Acylierung/ 90% | [M+H]+ = 337.2, Rt = 2.0 min | 1H-NMR (CDCl3): 1.39 (2 H, m); 1.60-1.70 (4 H, m); 1.94 (2 H, m); 2.09 (7 H, m); 2.15 (1 H, b s); 3.28 (1 H, s); 3.42 (5 H, m); 3.51 (1 H, s); 3.99 (2 H, s); 6.84 (1 H, s); 7.03 (1 H, s); 7.22 (1 H, s). |
| 43 | 1 | Bsp. Nr. 18/ Acylierung/ 88% | [M+H]+ = 347.2, Rt = 1.8 min | 1H-NMR (CDCl3): 1.32 (2 H, m); 1.52 (1 H, t); 1.58 (3 H, t); 1.62-1.93 (4 H, m); 2.00-2.12 (10 H, m); 2.29 (2 H, m); 3.09 (2 H, m); 3.28 (1 H, m); 3.38 (1 H, t); 6.77 (1 H, m); 6.97 (1 H, m); 7.16 (1 H, m). |
| 44 | 1 | Bsp. Nr. 18/ Acylierung/ 37% | [M+H]+ = 333.2, Rt = 2.4 min | 1H-NMR (CDCl3): 0.73 (2 H, m); 0.98 (2 H, m); 1.40-1.75 (6 H, b m); 1.94 (2 H, b s); 2.09 (2 H, s); 2.12 (4 H, s); 2.20 (2 H, b s); 3.38 (1 H, s); 3.49 (2 H, m); 3.64 (1 H, t); 6.86 (1 H, m); 7.04 (1 H, m); 7.27 (1 H, m). |
| 45 | 1 | Bsp. Nr. 18/ Acylierung/ 57% | [M+H]+ = 335.2, Rt = 2.0 min | 1H-NMR (CDCl3): 1.04 (6 H, m); 1.35 (2 H, m); 1.53 (1 H, t); 1.63 (3 H, m); 1.88 (2 H, m); 2.03 (6 H, m); 2.11 (2 H, m); 2.55 (1 H, m); 3.28 (2 H, m); 3.41 (2 H, m); 6.78 (1 H, m); 6.97 (1 H, m); 7.16 (1 H, m). |
| 46 | 1 | Bsp. Nr. 18/ Acylierung/ 51% | [M+H]+ = 349.2, Rt = 2.2 min | 1H-NMR (DMSO-d6): 0.93 (6 H, m); 1.37 (2 H, m); 1.56 (1 H, t); 1.63 (3 H, m); 1.89 (2 H, m); 2.01-2.17 (11 H, b m); 3.27 (1 H, s); 3.33 (1 H, s); 3.41 (2 H, m); 6.08 (1 H, m); 6.97 (1 H, m); 7.18 (1 H, m). |
| 47 | 1 | Bsp. Nr. 70/ Reduktion/ 90% | [M+H]+ = 323.2, Rt = 0.5 min | 1H-NMR (CDCl3): 1.36 (2 H, m); 1.50 (2 H, t); 1.68 (2 H, m); 1.89 (2 H, m); 2.08 (8 H, s); 2.46 (2 H, s); 2.54 (2 H, t); 2.60 (2 H, t); 3.33 (3 H, s); 3.46 (2 H, t); 6.82 (1 H, m); 7.01 (1 H, m); 7.20 (1 H, m). |
| 49 | 1 | Bsp. Nr. 18/ Reduktive Aminierung/ 69% | [MH-HNMe2]+ = 274.3 (100 %) | 1H-NMR (DMSO-d6): 0.10-0.15 (2 H, m); 0.43-0.50 (2 H, m); 0.82-0.92 (1 H, m); 1.27-1.35 (2 H, m); 1.52 (2 H, t, J = 6.8 Hz); 1.62-1.71 (2 H, m); 1.82-2.01 (4 H, m); 1.99 (6 H, s); 2.30-2.45 (2 H, m); 2.51-2.56 (2 H, m); 2.63-2.72 (2 H, m); 6.92 (1 H, dd, J = 3.5 und 1.1 Hz); 7.05 (1 H, dd, J = 5.1 und 3.5 Hz); 7.41 (1 H, dd, J = 5.1 und 1.0 Hz). |
| | | | [M+H]+ = 319.3 (40 %), Rt = 0.4min. | 13C-NMR (DMSO-d6): 3.6; 8.7; 32.7; 33.0; 36.0; 37.7; 40.5; 41.3; 41.6; 52.9; 58.6; 59.9; 65.0; 123.5; 124.7; 126.3; 143.2. |
| 50 | 1 | Bsp. Nr. 18/ Acylierung/ 48% | [MH-HNMe2]+ = 316.3 (100 %) | 1H-NMR (CDCl3): 1.32-1.44 (2 H, m); 1.59 (1 H, t, J = 7.1 Hz); 1.62-1.75 (5 H, m); 1.77-2.01 (4 H, m); 2.02-2.24 (4 H, m); 2.11 (6 H, s); 2.33-2.38 (2 H, m); 2.66-2.78 (1 H, m); 3.29 (1.2 H, s); 3.34 (0.8 H, s); 3.42 (0.8 H, t, J = 7.6 Hz); 3.45 (1.2 H, t, J = 7.8 Hz); 6.82-6.86 (1 H, m); 7.00-7.06 (1 H, m); 7.20-7.26 (1 H, m). |
| | | | [M+H]+ = 361.4 (65 %), Rt = 3.0min. | 13C-NMR (CDCl3): 18.71; 18.74; 28.5; 28.6; 31.1; 31.2; 32.3; 32.8; 33.5; 35.6; 37.0; 38.08; 38.09; 40.1; 41.2; 41.7; 42.0; 43.7; 45.1; 53.4; 55.3; 56.5; 59.8; 123.3; 123.5; 124.8; 125.1; 126.1; 126.3; 142.1; 143.7; 171.0; 171.1. |
| 51 | 1 | Bsp. Nr. 48/ Reduktion/ 55% | [MH-HNMe2]+ = 288.3 (100 %) | 1H-NMR (CDCl3): 0.01-0.06 (2 H, m); 0.38-0.44 (2 H, m); 0.59-0.72 (1 H, m); 1.34-1.43 (4 H, m); 1.52 (2 H, t, J = 6.8 Hz); 1.66-1.74 (2 H, m); 1.84-1.99 (2 H, m); 2.05-2.18 (2 H, m); 2.10 (6 H, s); 2.45 (2 H, s); 2.47-2.57 (4 H, m); 6.84 (1 H, dd, J = 3.6 und 1.1 Hz); 7.03 (1 H, dd, J = 5.1 und 3.6 Hz); 7.22 (1 H, dd, J = 5.1 und 1.0 Hz). |
| | | | [M+H]+ = 333.4 (25 %), Rt = 0.7min. | 13C-NMR (CDCl3): 4.3; 9.1; 33.7; 34.3; 38.2; 40.9; 54.0; 56.9; 59.6; 65.5; 123.2; 125.0; 126.2. |
| 52 | 1 | Bsp. Nr. 50/ Reduktion/ 65% | MH-HNMe2]+ = 302.3 (100 %) | 1H-NMR (CDCl3): 1.38 (2 H, ddd, J = 13.3, 10.0 und 3.5 Hz); 1.50 (2 H, t, J = 6.8 Hz); 1.54-1.64 (4 H, m); 1.65-1.73 (2 H, m); 1.78-1.96 (4 H, m); 1.98-2.16 (4 H, m); 2.10 (6 H, s); 2.20-2.30 (3 H, m); 2.41 (2 H, s); 2.49 (2 H, t, J = 6.8 Hz); 6.85 (1 H, dd, J = 3.6 und 1.1 Hz); 7.03 (1 H, dd, J = 5.1 und 3.6 Hz); 7.22 (1 H, dd, J = 5.1 und 1.1 Hz). |
| | | | [M+H]+ = 347.4 (25 %), Rt = 1.9min. | 13C-NMR (CDCl3): 18.7; 28.4; 33.7; 34.5; 36.1; 38.2; 40.8; 54.0; 54.8; 59.6; 65.8; 123.2; 124.9; 126.1. |
| 53 | 1 | Bsp. Nr. 43/ Reduktion/ 60% | [MH-HNMe2]+ = 288.3 (100 %) | 1H-NMR (CDCl3): 1.38-1.48 (2 H, m); 1.60-1.82 (8 H, m); 1.85-1.94 (1 H, m); 1.94-2.04 (2 H, m); 2.06-2.18 (4 H, m); 2.12 (6 H, s); 2.55-2.95 (6 H, m); 6.85 (1 H, br d, J = 3.0 Hz); 7.03 (1 H, dd, J = 5.1 und 3.6 Hz); 7.22-7.24 (1 H, dd, J = 5.0 und 0.8 Hz). |
| | | | [M+H]+ = 333.4 (20 %), Rt = 1.1 min. | 13C-NMR (CDCl3): 18.7; 27.8; 30.3; 33.3; 33.6; 36.9; 38.1; 41.1; 53.5; 59.9; 62.2; 64.4; 123.6; 125.3; 126.3. |
| 54 | 1 | Bsp. Nr. 18/ Acylierung/ 55% | [M+H]+ = 363.3, Rt = 3.1 min. | 1H-NMR (CDCl3): 0.91 (6 H, t, J = 6.1 Hz); 1.34-1.45 (2 H, m); 1.50-1.73 (8 H, m); 1.80-2.08 (2 H, m); 2.09 und 2.11 (6 H, 2 s); 2.15-2.26 (3 H, m); 3.30 (1.2 H, s); 3.36 (0.8 H, s); 3.41-3.50 (2 H, m); 6.83-6.87 (1 H, m); 7.01-7.07 (1 H, m); 7.23-7.26 (1 H, m). |
| | | | | 13C-NMR (CDCl3): 22.3; 22.38; 22.41; 27.9; 28.0; 31.1; 31.2; 32.4; 32.8; 33.4; 33.7; 33.8; 35.6; 37.2; 38.1; 40.1; 42.0; 44.0; 45.0; 55.5; 56.5; 59.9; 123.3; 123.5; 124.8; 125.0; 126.1; 126.3; 172.17; 172.2. |
| 55 | 1 | Bsp. Nr. 18/ Acylierung/ 36% | [MH-HNMe2]+ = 315.2 (100%) | 1H-NMR (CDCl3): 1.38-1.46 (2 H, m); 1.60 und 1.61 (6 H, 2 s); 1.65 (2 H, t, J = 7.4 Hz); 1.68-1.81 (3 H, m); 1.93-2.09 (3 H, m); 2.10 und 2.11 (6 H, 2 s); 3.43 (0.7 H, s); 3.63 (1.3 H, 3 s); 3.55 (1.3 H, t, J = 7.5 Hz); 3.83 (0.7 H, t, J = 7.0 Hz); 6.85 (1 H, dd, J = 3.5 und 0.9 Hz); 7.05 (1 H, dd, J = 5.1 und 3.5 Hz); 7.24 (1 H, d, J = 5.0 Hz). |
| | | | [M+H]+ = 360.2 (10 %), Rt = 2.6 min. | 13C-NMR (CDCl3): 25.07; 25.1; 30.4; 31.2; 33.0; 33.1; 34.0; 36.7; 38.0; 38.1; 39.2; 43.0; 46.2; 46.4; 57.9; 59.8; 121.7; 121.8; 123.4; 124.8; 124.9; 126.2; 126.3; 165.9. |
| 56 | 1 | Bsp. Nr. 18/ Acylierung/ 50% | [MH-HNMe2]+ = 332.2 (100 %) | 1H-NMR (CDCl3): 1.33-1.45 (2 H, m); 1.50-1.74 (5 H, m); 1.76-2.06 (3 H, m); 2.09 und 2.10 (6 H, 2 s); 2.12-2.40 (2 H, m); 2.30-2.40 (2 H, m); 2.66-2.76 (1 H, m); 3.28 (1.2 H, s); 3.36 (0.8 H, s); 3.40-3.50 (3 H, m); 3.70-3.78 (1 H, m); 3.82-3.89 (1 H, m); 3.93-4.00 (1 H, m); 6.84 (1 H, dt, J = 3.7 und 1.1 Hz); 7.01-7.06 (1 H, m); 7.22 (0.4 H, dd, J = 5.1 und 1.1 Hz); 7.24 (0.6 H, dd, J = 5.1 und 1.1 Hz). |
| | | | [M+H]+ = 377.3 (50 %), Rt = 2.5min. | 13C-NMR (CDCl3): 31.1; 31.2; 32.3; 32.83; 32.85; 33.4; 35.3; 35.5; 37.0; 38.1; 38.5; 40.1; 42.1; 43.9; 45.1; 55.4; 56.4; 59.8; 67.6; 73.31; 73.33; 123.3; 123.5; 124.8; 125.1; 126.1; 126.3; 143.6; 170.40; 170.44. |
| 57 | 1 | Bsp. Nr. 18/ Acylierung/ 41% | [M+H]+ = 349.3, Rt = 2.3 min. | 1H-NMR (CDCl3): 1.33-1.44 (2 H, m); 1.60-1.72 (5 H, m); 1.85-2.08 (2 H, m); 2.09 und 2.11 (6 H, 2s); 2.11-2.18 (1 H, m); 3.06 (1.2 H, s); 3.23 (0.8 H, t, J = 7.2 Hz); 3.40 (0.8 H, s); 3.52 (1.2 H, t, J = 7.2 Hz); 3.87-3.98 (1 H, m); 4.74-4.81 (2 H, m); 4.90-4.96 (2 H, m); 6.83-6.86 (1 H, m); 7.01-7.06 (1 H, m); 7.22-7.26 (1 H, m). |
| | | | | 13C-NMR (CDCl3): 30.9; 31.2; 32.9; 32.3; 35.0; 36.9; 38.04; 38.09; 38.3; 38.5; 40.1; 42.1; 44.2; 44.3; 55.6; 55.9; 59.8; 73.01; 73.03; 123.4; 123.5; 124.9; 125.0; 126.2; 126.3; 169.6; 169.7. |
| 58 | 1 | Bsp. Nr. 18/ Acylierung/ 23% | [MH-HNMe2]+ = 313.2 (100 %) | 1H-NMR (CDCl3): 1.38-1.54 (4 H, m); 1.55-1.81 (8 H, m); 1.95-2.08 (2 H, m); 2.10 und 2.12 (6 H, 2s); 3.38 (0.8 H, s); 3.50 (1.2 H, t, J = 7.3 Hz); 3.71 (1.2 H, s); 3.90 (0.8 H, t, J = 1.1 Hz); 6.86 (1 H, br d, J = 3.5 Hz); 7.03-7.07 (1 H, m); 7.23-7.26 (1 H, m). |
| | | | [M+H]+ = 358.2 (6 %), Rt = 2.6 min. | 13C-NMR (CDCl3): 16.3; 16.7; 30.7; 31.1; 33.2; 38.07; 38.1; 39.9; 42.5; 45.7; 46.1; 120.3; 123.4; 124.9; 126.2; 126.3; 163.0. |
| 59 | 1 | Bsp. Nr. 18/ Acylierung/ 84% | [M+H]+ = 393.3, Rt = 2.8 min. | 1H-NMR (CDCl3): 1.15 (2.4 H, s); 1.16 (3.6 H, s); 1.30-1.46 (2 H, m); 1.60 (1.2 H, t, J = 7.2 Hz); 1.63-1.74 (2.8 H, m); 1.80-2.02 (4 H, m); 2.04-2.22 (8 H, m); 2.23-2.30 (2 H, m); 3.16 (1.2 H, s); 3.18 (1.8 H, s); 3.32 (1.2 H, s); 3.36 (0.8 H, s); 3.44-3.50 (2 H, m); 6.83-6.87 (1 H, m); 7.01-7.07 (1 H, m); 7.21-7.26 (1 H, m). |
| | | | | 13C-NMR (CDCl3): 24.7; 25.0; 28.7; 29.1; 31.0; 31.1; 32.7; 33.4; 34.0; 34.2; 35.6; 37.1; 38.1; 40.0; 42.0; 44.0; 45.0; 49.1; 53.4; 55.5; 56.4; 59.8; 73.90; 73.94; 123.2; 124.7; 126.0; 126.2; 171.91; 171.94. |
| 60 | 1 | Bsp. Nr. 18/ Acylierung/ 57% | [M+H]+ = 377.3, Rt = 2.5 min. | 1H-NMR (CDCl3): 1.34-1.46 (2 H, m); 1.56-1.74 (7 H, m); 1.84-2.02 (4 H, m); 2.09 und 2.12 (6 H, 2 s); 2.16-2.26 (1 H, m); 2.52-2.61 (1 H, m); 3.34-3.54 (6 H, m); 3.99-4.05 (2 H, m); 6.83-6.87 (1 H, m); 7.01-7.07 (1 H, m); 7.21-7.27 (1 H, m). |
| | | | | 13C-NMR (CDCl3): 28.58; 28.6; 31.0; 31.2; 32.8; 33.4; 35.5; 37.1; 38.1; 39.6; 39.8; 39.9; 42.0; 44.2; 44.7; 55.6; 56.0; 59.8; 67.3; 123.3; 123.6; 124.8; 125.1; 126.1; 126.4; 173.0; 173.1. |
| 61 | 1 | Bsp. Nr. 18/ Acylierung/ 68% | [MH-HNMe2]+ = 327.2 (100 %) | 1H-NMR (CDCl3): 1.37-1.46 (2 H, m); 1.63-1.78 (4 H, m); 1.93-2.09 (4 H, m); 2.10 (6 H, s); 2.12-2.15 (1 H, m); 2.20-2.33 (1 H, m); 2.53-2.68 (2 H, m); 2.72-2.82 (2 H, m); 3.35 (1.2 H, s); 3.42 (0.8 H, s); 3.51-3.57 (2 H, m); 6.85 (1 H, dd, J = 3.5 und 0.9 Hz); 7.04 (1 H, td, J = 5.1 und 3.6 Hz); 7.22-7.26 (1 H, m). |
| | | | [M+H]+ = 372.3 (5 %), Rt = 2.7 min. | 13C-NMR (CDCl3): 16.5; 30.5; 30.6; 31.2; 33.0; 33.1; 34.4; 37.2; 38.0; 38.1; 38.8; 39.0; 39.8; 42.7; 44.9; 45.5; 56.7; 57.1; 59.8; 120.8; 120.9; 123.4; 124.9; 126.2; 126.3; 164.4; 164.7. |
| 62 | 1 | Bsp. Nr. 18/ Acylierung/ 61% | [M+H]+ = 361.3, Rt = 3.0 min. | 1H-NMR (CDCl3): 0.03-0.08 (2 H, m); 0.38-0.45 (2 H, m); 0.66-0.76 (1 H, m); 1.34-1.45 (2 H, m); 1.50-1.64 (3 H, m); 1.64-1.72 (3 H, m); 1.85-2.02 (3 H, m); 2.03 und 2.12 (6 H, 2 s); 2.14-2.25 (1 H, m); 2.31-2.38 (2 H, m); 3.32 (1.2 H, s); 3.36 (0.8 H, s); 3.34-3.50 (2 H, m); 6.83-6.87 (1 H, m); 7.01-7.07 (1 H, m); 7.21-7.26 (1 H, m). |
| | | | | 13C-NMR (CDCl3): 4.5; 10.7; 28.5; 30.12; 30.15; 31.1; 31.2; 32.8; 33.4; 34.4; 34.8; 35.6; 37.1; 38.1; 40.1; 42.0; 43.9; 45.0; 55.3; 56.5; 59.9; 123.3; 123.5; 124.9; 125.1; 126.1; 126.3; 143.6; 171.87; 171.9. |
| 63 | 1 | Bsp. Nr. 18/ Acylierung/ 67% | [M+H]+ = 365.3, Rt = 2.5 min. | 1H-NMR (CDCl3): 1.35-1.44 (8 H, m); 1.53-1.58 (1 H, m); 1.64-1.75 (3 H, m); 1.95-2.09 (3 H, m); 2.10 und 2.11 (6 H, 2 s); 2.12-2.18 (1 H, m); 3.18 und 3.19 (3 H, 2 s); 3.42 (0.7 H, s); 3.57 (1.3 H, t, J = 7.4 Hz); 3.62 (1.3 H, s); 3.73 (0.7 H, t, J = 7.0 Hz); 6.85 (1 H, dd, J = 3.5 und 0.7 Hz); 7.04 (1 H, dd, J = 5.0 und 3.6 Hz); 7.22-7.26 (1 H, m). |
| | | | | 13C-NMR (CDCl3): 24.1; 30.3; 30.6; 31.4; 32.9; 33.1; 34.0; 38.07; 38.1; 38.6; 42.4; 45.4; 45.8; 51.57; 51.59; 56.8; 57.7; 59.9; 79.5; 79.8; 123.3; 124.8; 126.1; 126.3; 172.6; 172.9. |
| 64 | 1 | Bsp. Nr. 18/ Acylierung/ 58% | [M+H]+ = 377.3, Rt = 2.7 min. | 1H-NMR (CDCl3): 1.34-1.45 (2 H, m); 1.51-1.73 (6 H, m); 1.84-2.00 (4 H, m); 2.09 und 2.11 (6 H, 2 s); 2.13-2.23 (2 H, m); 2.34-2.43 (1 H, m); 2.57-2.65 (1 H, m); 3.28-3.40 (2 H, m); 3.40-3.55 (2 H, m); 3.70-3.77 (1 H, m); 3.82-3.90 (1 H, m); 4.25-4.32 (1 H, m); 6.84-6.86 (1 H, m); 7.01-7.07 (1 H, m); 7.22-7.26 (1 H, m). |
| | | | | 13C-NMR (CDCl3): 25.63; 25.66; 31.1; 31.15; 31.2; 31.5; 31.6; 32.8; 32.9; 33.3; 38.1; 40.1; 40.5; 40.9; 42.0; 43.9; 45.2; 55.4; 56.6; 59.9; 67.81; 67.84; 75.99; 76.0; 123.3; 124.8; 126.1; 126.3; 169.5; 169.6. |
| 65 | 1 | Bsp. Nr. 18/ Acylierung/ 28% | [MH-HNMe2]+ = 334.3, Rt = 2.5 min. | 1H-NMR (CDCl3): 1.22 (2.6 H, s); 1.23 (3.4 H, s); 1.32-1.47 (2 H, m); 1.57-1.74 (4 H, m); 1.83 (2 H, t, J = 6.8 Hz); 1.87-2.02 (2 H, m); 2.02-2.24 (2 H, m); 2.08 (2.6 H, s); 2.11 (3.4 H, s); 2.36-2.44 (2 H, m); 3.32 (1.2 H, s); 3.36 (0.8 H, s); 3.43-3.50 (2 H, m); 6.84 (0.4 H, dd, J = 3.6 und 1.1 Hz); 6.85 (0.6 H, dd, J = 3.6 und 1.1 Hz); 7.02 (0.4 H, dd, J = 5.1 und 3.6 Hz); 7.05 (0.6 H, dd, J = 5.1 und 3.6 Hz); 7.23 (0.4 H, dd, J = 5.1 und 1.1 Hz); 7.25 (0.6 H, dd, J = 5.1 und 1.1 Hz). Das OH-Signal konnte nicht identifiziert werden. |
| | | | | 13C-NMR (CDCl3): 29.1; 29.4; 29.5; 31.1; 31.2; 32.7; 33.4; 35.5; 37.2; 38.1; 40.0; 42.1; 44.1; 45.2; 55.7; 56.7; 59.7; 69.51; 69.55; 123.2; 123.4; 124.7; 125.1; 126.0; 126.2; 172.6; 172.7. |
| 66 | 1 | Bsp. Nr. 18/ Acylierung/ 50% | [M+H]+ = 363.2 (4%) | 1H-NMR(DMSO-d6): 1.22-1.32 (2 H, m); 1.41-1.48 (1 H, m); 1.52-1.66 (6 H, m); 1.68-1.77 (1 H, m); 1.99 (4 H, s); 2.00 (2 H, s); 1.94-2.06 (4 H, m, überlagert); 2.45-2.52 (2 H, m, überlagert vom DMSO-Signal); 3.17 (0.8 H, s); 3.32 (1.2 H; t, J = 7.1 Hz, überlagert vom Wasser-Signal); 3.35 (1.2 H; s); 3.54 (0.8 H; t, J = 7.1 Hz); 5.747 (0.3 H, s); 5.754 (0.7 H, s); 6.92-6.94 (1 H, m); 7.04-7.07 (1 H, m); 7.40-7.42 (1 H, m). |
| | | | [MH-NHMe2]+ = 318.3 (100%), Rt = 2.6 min. | 13C-NMR (DMSO-d6): 12.2; 29.9; 30.0; 30.7; 32.4; 32.5; 33.4; 33.6; 37.7; 38.7; 41.5; 44.4; 44.5; 54.8; 59.0; 59.1; 75.2; 75.5; 123.6; 124.8; 124.9; 126.3; 171.1; 171.2. |
| 67 | 1 | Bsp. Nr. 18/ Acylierung/ 30% | [MH-HNMe2]+ = 327.2 (100 %) | 1H-NMR (CDCl3): 0.95-1.02 (2 H, m); 1.32-1.37 (2 H, m); 1.37-1.46 (2 H, m); 1.61-1.75 (4 H, m); 1.87-2.06 (3 H, m); 2.10 (2.5H, s); 2.11 (3.5 H, s); 2.13-2.22 (1 H, m); 2.45 (2 H, s); 3.29 (1.1 H, s); 3.39 (0.9 H, s); 3.46 (0.9 H, t, J = 7.1 Hz); 3.52 (1.1 H, t, J = 7.3 Hz); 6.83-6.86 (1 H, m); 7.02 (0.5 H, dd, J = 5.1 und 3.6 Hz); 7.04 (0.5 H, dd, J = 5.1 und 3.6 Hz); 7.22 (0.5 H, dd, J = 5.1 und 1.1 Hz); 7.24 (0.5 H, dd, J = 5.1 und 1.1 Hz). |
| | | | [M+H]+ = 372.3 (98 %), Rt = 2.5 min. | 13C-NMR (CDCl3): 6.6; 6.7; 14.0; 31.0; 31.1; 32.8; 33.3; 35.4; 36.9; 38.1; 38.1; 39.3; 39.7; 40.1; 42.3; 44.2; 45.2; 55.6; 56.5; 59.8; 59.8; 123.1; 123.2; 123.3; 123.6; 124.8; 125.1; 126.1; 126.3; 143.5; 167.2; 167.2. |
| 68 | 1 | Bsp. Nr. 18/ Acylierung/ 44% | [MH-HNMe2]+ = 341.2 (95%) | 1H-NMR (CDCl3): 1.34-1.46 (2 H, m); 1.60-1.75 (4 H, m); 1.85-2.06 (4 H, m); 2.08 (2.5 H, s); 2.11 (3.5 H, s); 2.16-2.30 (4 H, m); 2.58-2.67 (2 H, m); 2.69 (0.8 H, s); 2.70 (1.2 H, s); 3.30 (1.2 H, s); 3.37 (0.8 H, s); 3.45 (0.8 H, t, J = 7.1 Hz); 3.50 (1.2 H, t, J = 7.3 Hz); 6.84 (0.4 H, dd, J = 3.6 und 1.1 Hz); 6.86 (0.6 H, dd, J = 3,5 und 1.0 Hz); 7.02 (0.4 H, dd, J = 5.1 und 3.6 Hz); 7.05 (0.6 H, dd, J = 5.1 und 3.6 Hz); 7.23 (0.4 H, dd, J = 5.1 und 1.1 Hz); 7.25 (0.6 H, dd, J = 5.1 und 1.1 Hz). |
| | | | [M+H]+ = 386.3 (100 %), Rt = 2.7 min. | |
| 69 | 1 | Bsp. Nr. 18/ Acylierung/ 32% | [M+H]+ = 375.3, Rt = 3.2 min. | 1H-NMR (CDCl3): 1.35-1.45 (2 H, m); 1.46-1.76 (10 H, m); 1.78-1.96 (3 H, m); 1.96-2.09 (3 H, m); 2.09 und 2.11 (6 H, 2 s); 2.16-2.32 (3 H, m); 3.29 (1.2 H, s); 3.36 (0.8 H, s); 3.40-3.50 (2 H, m); 6.83-6.87 (1 H, m); 7.01-7.09 (1 H, m); 7.21-7.25 (1 H, m). |
| | | | | 13C-NMR (CDCl3): 18.2; 18.29; 18.30; 27.8; 27.9; 27.96; 28.0; 31.1; 31.2; 31.5; 31.7; 31.97; 31.99; 32.04; 32.5; 32.8; 33.4; 35.2; 35.5; 35.7; 35.73; 37.1; 38.1; 40.1; 42.0; 43.9; 45.0; 55.4; 56.4; 59.9; 77.2; 77.5; 123.3; 123.5; 124.9; 125.1; 126.1; 126.3; 171.9; 172.0. |
| 70 | 1 | Bsp. Nr. 57/ Reduktion/ 44% | [M+H]+ = 335.3, Rt = 0.2 min. | 1H-NMR (CDCl3): 1.32-1.40 (2 H, m); 1.48 (2 H, t, J = 6.9 Hz); 1.63-1.71 (2 H, m); 1.75-2.00 (3 H, m); 2.00-2.08 (1 H, m); 2.09 (6 H, s); 2.35 (2 H, s); 2.46 (2 H, t, J = 6.9 Hz); 2.73 (2 H, d, J = 7.3 Hz); 3.13-3.22 (1 H, m); 4.42 (2 H, t, J = 6.2 Hz); 4.78 (2 H, dd, J = 7.8 und 6.0 Hz); 6.84 (1 H, d, J = 3.5 Hz); 7.03 (1 H, dd, J = 5.1 und 3.5 Hz); 7.22 (1 H, d, J = 5.1 Hz). |
| | | | | 13C-NMR (CDCl3): 19.8; 33.6; 34.3; 34.7; 38.1; 41.0; 53.8; 59.6; 59.8; 65.7; 76.5; 123.2; 124.9; 126.1. |
| 71 | 1 | Bsp. Nr. 13/ Reduktion/ 77% | [M+H]+ = 259.2, Rt = 0.6 min | 1H-NMR (CDCl3): 1.31 (2 H, m); 1.43 (2 H, m); 1.63 (2 H, m); 1.91 (2 H, m); 2.03 (6 H, s); 2.24 (2 H, m); 2.84 (2 H, s); 2.95 (2 H, m); 7.25-7.39 (5 H, m). |
| 72 | 1 | Bsp. Nr. 71/ Acylierung/ 86% | [M+H]+ = 329.4, Rt = 2.8 min. | 1H-NMR (CDCl3): 0.97 (t, 3H, J = 7.4 Hz); 1.23-1.35 (m, 2H); 1.52-1.74 (m, 8H); 2.02 und 2.04 (2 s, 6H); 2.18-2.26 (m, 3H); 2.23-2.41 (br s, 1 H); 3.34 und 3.41 (2 s, 2H); 3.39-3.48 (m, 2H); 7.23-7.42 (m, 5H). |
| | | | | 13C-NMR (CDCl3): 13.7; 14.0; 18.4; 18.5; 18.9; 30.1; 30.9; 31.2; 31.5; 36.4; 36.9; 37.8; 38.0; 38.1; 40.3; 42.3; 43.9; 45.0; 55.1; 56.4; 60.8; 126.5; 126.7; 127.6; 127.63; 127.67; 127.7; 171.9; 172.0. |
| 73 | 1 | Bsp. Nr. 71/ Acylierung/ 56% | [M+H]+ = 301.2, Rt = 1.5 min | 1H-NMR (CDCl3): 1.30 (2 H, m); 1.54 (1 H, m); 1.62 (3 H, m); 1.83-1.96 (2 H, m); 2.00-2.03 (9 H, m); 2.19 (1 H, m); 2.32 (1 H, m); 3.32-3.45 (4 H, m); 7.23-7.39 (5 H, m). |
| 74 | 1 | Bsp. Nr. 71/ Reduktive Aminierung/ 83% | [M+H]+ = 315.3, Rt = 1.4 min | 1H-NMR (CDCl3): 0.90 (3 H, t); 1.30 (4 H, m); 1.44 (4 H, m); 1.66 (2 H, m); 1.85 (2 H, m); 2.02 (6 H, s); 2.26 (2 H, m); 2.37 (2 H, m); 2.46 (4 H, m); 7.29 (5 H, m). |
| 75 | 1 | Bsp. Nr. 71/ Acylierung/ 51% | [M+H]+ = 371.4, Rt = 2.8 min. | 1H-NMR (CDCl3): 1.23-1.34 (2 H, m); 1.51-1.70 (5 H, m); 1.83-1.98 (4 H, m); 2.03 und 2.05 (6 H, 2 s); 2.09-2.25 (2 H, m); 2.25-2.45 (2 H, m); 2.59 (0.6 H, d, J = 6.9 Hz); 2.63 (0.4 H, d, J = 6.9 Hz); 3.33-3.53 (4 H, m); 3.69-3.77 (1 H, m); 3.81-3.91 (1 H, m); 4.24-4.33 (1 H, m); 7.26-7.33 (3 H, m); 7.34-7.41 (2 H, m). |
| | | | | 13C-NMR (CDCl3): 25.6; 30.0; 30.1; 30.7; 31.16; 31.2; 31.4; 31.5; 31.57; 31.6; 36.0; 37.6; 38.0; 38.1; 40.4; 40.5; 40.9; 42.3; 43.9; 45.2; 55.1; 56.5; 60.9; 67.81; 67.85; 76.0; 126.5; 126.7; 127.6; 127.7; 127.8; 137.4; 169.7; 169.7. |
| 76 | 1 | Bsp. Nr. 71/ Acylierung/ 29% | [MH-HNMe2]+ = 335.3 (22 %) | 1H-NMR (CDCl3): 1.24-1.36 (2 H, m); 1.56 (1 H, t, J = 7.3 Hz); 1.62 (3 H, m); 1.83-2.11 (9 H, m); 2.15-2.31 (4 H, m); 2.33-2.44 (1 H, m); 2.59-2.68 (2 H, m); 2.69 (0.8 H, s); 2.71 (1.2 H, s); 3.34 (1.2 H, s); 3.41 (0.8 H, s); 3.43-3.51 (2 H, m); 7.24-7.33 (3 H, m); 7.34-7.42 (2 H, m). |
| | | | [M+H]+ = 380.4 (100%), Rt 2.7 min. | 13C-NMR (CDCl3): 17.1; 30.0; 30.8; 31.2; 31.4; 32.5; 33.2; 33.3; 35.9; 37.5; 38.0; 38.1; 40.4; 41.5; 42.0; 42.5; 43.9; 45.2; 55.3; 56.2; 60.8; 124.5; 124.5; 126.5; 126.9; 127.6; 127.6; 127.7; 127.8; 137.5; 167.0; 167.1. |
| 77 | 1 | Bsp. Nr. 71/ Acylierung/ 25% | [MH-HNMe2]+ = 321.3 (16%) | 1H-NMR (CDCl3): 0.96-1.02 (2 H, m); 1.25-1.37 (4 H, m); 1.57 (1 H, t, J = 7.3 Hz); 1.62-1.70 (3 H, m); 1.83-1.99 (2 H, m); 2.03 (2.6 H, s); 2.04 (3.4 H, s); 2.16-2.25 (1 H, m); 2.29-2.38 (1 H, m); 2.45 (0.8 H, s); 2.46 (1.2 H, s); 3.32 (1.2 H, s); 3.43 (0.8 H, s); 3.41-3.53 (2 H, s); 7.23-7.32 (3 H, m); 7.33-7.41 (2 H, m). |
| | | | [M+H]+ = 366.3 (100 %), Rt = 2.6 min. | 13C-NMR (CDCl3): 6.7; 6.7; 14.0; 30.1; 30.8; 31.1; 31.4; 35.8; 37.5; 38.0; 38.1; 39.3; 39.7; 40.4; 42.5; 44.2; 45.2; 55.4; 56.4; 60.8; 123.1; 123.2; 126.5; 126.8; 127.6; 127.6; 127.7; 127.8; 137.3; 167.2; 167.2. |
| 78 | 1 | Bsp. Nr. 71/ Acylierung/ 25% | [M+H]+ = 357.3, Rt = 2.4 min. | 1H-NMR (CDCl3): 1.26-1.36 (2 H, m); 1.55 (1 H, t, J = 7.3 Hz); 1.59-1.70 (3 H, m); 1.82-2.00 (2 H, m); 2.02 und 2.05 (6 H, 2 s); 2.13-2.40 (2 H, m); 2.67 (2 H, t, J = 7.3 Hz); 3.34 (1.1 H, s); 3.37 (0.9 H, s); 3.38-3.46 (3 H, m); 4.38-4.44 (2 H, m); 4.90 (2 H, dd, J = 7.8 und 6.3 Hz); 7.24-7.32 (3 H, m); 7.34-7.42 (2 H, m). |
| | | | | 13C-NMR (CDCl3): 20.1; 30.1; 30.8; 31.1; 31.4; 31.5; 31.6; 35.8; 37.4; 37.9; 38.0; 38.1; 38.5; 39.8; 40.4; 42.3; 43.8; 44.9; 55.1; 56.3; 60.7; 77.4; 77.5; 126.5; 126.7; 127.5; 127.54; 127.6; 127.7; 137.3; 169.5; 169.6. |
| 79 | 1 | Bsp. Nr. 71/ Acylierung/ 58% | [M+H]+ = 357.4, Rt = 3.1 min. | 1H-NMR (CDCl3): 0.90 und 0.92 (6 H, 2 d, J = 6.3 Hz); 1.24-1.36 (2 H, m); 1.50-1.70 (7 H, m); 1.77-1.99 (2 H, m); 2.02 und 2.04 (6 H, 2 s); 2.16-2.27 (3 H, m); 2.29-2.40 (1 H, m); 3.40 (1.2 H, s); 3.34 (0.8 H, s); 3.41-3.48 (2 H, m); 7.24-7.32 (3 H, m); 7.34-7.41 (2 H, m). |
| | | | | 13C-NMR (CDCl3): 22.37; 22.4; 27.9; 28.0; 31.0; 30.9; 31.2; 31.5; 32.4; 32.9; 33.8; 36.0; 37.6; 38.0; 38.1; 40.3; 42.3; 43.9; 45.0; 55.2; 56.3; 60.8; 126.5; 126.7; 127.6; 127.63; 127.7; 127.8; 172.19; 172.2. |
| 80 | 1 | Bsp. Nr. 71/ Acylierung/ 58% | [M+H]+ = 354.4, Rt = 2.6 min. | 1H-NMR (CDCl3): 1.25-1.38 (2 H, m); 1.54-1.58 (1 H, m); 1.58 und 1.60 (6 H, 2 s); 1.62-1.73 (3 H, m); 1.88-2.00 (2 H, m); 1.98 und 2.02 (6 H, 2 s); 2.17-2.30 (2 H, m); 3.45 (0.7 H, s); 3.65 (1.3 H, s); 3.51 (1.3 H, t, J = 7.0 Hz); 3.79 (0.7 H, t, J = 7.0 Hz); 7.23-7.31 (3 H, m); 7.34-7.40 (2 H, m). |
| | | | | 13C-NMR (CDCl3): 25.01; 25.07; 30.2; 30.5; 30.6; 31.4; 34.4; 36.6; 37.9; 38.0; 39.3; 43.2; 46.1; 46.3; 57.5; 57.9; 60.6; 121.6; 121.8; 126.5; 126.6; 127.4; 127.5; 127.7; 165.6; 165.9. |
| 81 | 1 | Bsp. Nr. 71/ Acylierung/ 40% | [M+H]+ = 371.4, Rt = 2.6 min. | 1H-NMR (CDCl3): 1.24-1.35 (2 H, m); 1.52-1.68 (6 H, m); 1.80-1.98 (4 H, m); 2.02 und 2.04 (6 H, 2 s); 2.15-2.43 (2 H, m); 2.51-2.61 (1 H, m); 3.36-3.51 (6 H, m); 3.98-4.05 (2 H, m); 7.25-7.32 (3 H, m); 7.33-7.41 (2 H, m). |
| | | | | 13C-NMR (CDCl3): 28.6; 30.1; 31.0; 31.1; 31.4; 35.7; 37.6; 38.0; 39.6; 39.8; 40.1; 42.3; 44.1; 44.6; 55.4; 55.9; 60.7; 67.3; 67.32; 126.5; 126.7; 127.5; 127.6; 127.8; 135.2; 137.4; 173.0; 173.1. |
| 82 | 1 | Bsp. Nr. 71/ Acylierung/ 14% | [M+H]+ = 373.3, Rt = 2.5 min. | 1H-NMR (CDCl3):1.23 (2.4 H, s); 1.24 (3.6 H, s); 1.27-1.35 (2 H, m); 1.51-1.57 (1.2 H, m); 1.59-1.70 (2.8 H, m); 1.81-1.98 (4 H, m); 2.03 (2.4 H, s); 2.06 (3.6 H, s); 2.16-2.45 (4 H, m); 3.35 (1.2 H, s); 3.40 (0.8 H, s); 3.41-3.49 (2 H, m); 7.23-7.41 (5 H, m). Das OH-Signal konnte nicht identifiziert werden. |
| | | | | Das 1 H-NMR-Spektrum in DMSO-d6 zeigt das OH-Signal bei 4.22 und 4.25 ppm. |
| | | | | 13C-NMR (CDCl3): 27.6; 29.1; 29.4; 29.5; 30.0; 30.7; 31.2; 31.3; 35.9; 37.2; 37.4; 37.9; 38.0; 40.3; 42.2; 44.2; 45.0; 55.3; 56.2; 60.7; 69.4; 74.7; 74.8; 75.2; 126.4; 126.9; 127.5; 127.7; 129.9; 137.4; 172.5; 172.6. |
| 83 | 1 | Bsp. Nr. 71/ Acylierung/ 77% | [M+H]+ = 387.4, Rt = 2.8 min. | 1H-NMR (CDCl3): 1.15 (2.6 H, s); 1.17 (3.4 H, s); 1.27-1.36 (2 H, m); 1.51-1.57 (1.2 H, m); 1.59-1.70 (2.8 H, m); 1.80-1.99 (4 H, m); 2.02 (2.6 H, s); 2.03 (3.4 H, m); 2.16-2.40 (4 H, m); 3.16 (1.3 H, s); 3.18 (1.7 H, s); 3.35 (1.2 H, s); 3.40 (0.8 H, s); 3.41-3.48 (2 H, m); 7.23-7.42 (5 H, m). |
| | | | | 13C-NMR (CDCl3): 25.0; 25.0; 28.7; 29.1; 30.1; 30.7; 31.3; 31.5; 34.0; 34.0; 34.2; 35.9; 37.5; 38.0; 40.4; 42.3; 44.0; 44.9; 49.1; 55.3; 56.2; 60.7; 73.90; 73.94; 126.5; 126.7; 127.6; 127.7; 171.92; 171.94. |
| 84 | 1 | Bsp. Nr. 71/ Acylierung/ 54% | [M+H]+= 357.3 (29 %) | 1H-NMR (DMSO-d6): 1.13-1.24 (2 H, m); 1.42-1.49 (2 H, m); 1.53-1.63 (2 H, m); 1.68-1.78 (1 H, m); 1.90 (4 H, s); 1.92 (2 H, s); 1.82-1.91 (1 H, m, überlagert); 1.95-2.13 (6 H, m); 2.46-2.54 (2 H, m, überlagert vom DMSO-Signal); 3.21 (0.8 H, s); 3.24 (1.2 H; t, J = 7.1 Hz, überlagert vom Wasser-Signal); 3.39 (1.2 H; s); 3.52 (0.8 H; t, J = 7.1 Hz); 5.70 (0.3 H, s); 5.77 (0.7 H, s); 7.23-7.26 (1 H, m); 7.31-7.39 (4 H, m). |
| | | | [MH-NHMe2]+=312.3 (100 %), Rt = 2.7 min. | 13C-NMR (DMSO-d6): 12.2; 29.9; 30.2; 30.9; 33.4; 33.6; 37.7; 41.7; 44.4; 59.8; 60.0; 75.2; 75.5; 126.2; 127.2; 127.4; 171.1; 171.3. |
| 85 | 1 | Bsp. Nr. 71/ Acylierung/ 70% | [M+H]+ = 341.4, Rt = 2.8 min. | 1H-NMR (CDCl3): 0.10-0.19 (2 H, m); 0.51-0.58 (2 H, m); 1.08 (1 H, m); 1.22-1.35 (2 H, m); 1.50-1.67 (4 H, m); 1.75-1.98 (2 H, m); 2.02 (2.4 H, s); 2.03 (3.6 H, s); 3.31 (1.2 H, s); 2.17 (0.8 H, d, J = 6.7 Hz); 2.20 (1.2 H, d, J = 6.7 Hz); 2.18-2.40 (2 H, m); 3.38 (0.8 H, t, J = 7.2 Hz); 3.41 (0.8 H, s); 3.46 (1.2 H, t, J = 7.2 Hz); 7.22-7.40 (5 H, m). |
| | | | | 13C-NMR (CDCl3): 4.3; 6.9; 30.1; 30.9; 31.2; 31.3; 35.9; 37.7; 37.9; 38.1; 39.5; 39.9; 40.2; 42.3; 43.9; 44.9; 55.0; 56.3; 60.8; 126.3; 126.7; 127.4; 127.5; 127.6; 135.6; 137.4; 171.38; 171.44. |
| 86 | 1 | Bsp. Nr. 71/ Acylierung/ 79% | [M+H]+ = 355.4, Rt = 3.1 min. | 1H-NMR (CDCl3): 1.24-1.35 (2 H, m); 1.53 (1 H, m); 1.56-1.76 (5 H, m); 1.78-1.98 (4 H, m); 2.02 (2.5 H, s); 2.04 (3.5 H, s); 2.10-2.24 (3 H, m); 2.30-2.42 (3 H, m); 2.74 (1 H, m); 3.33 (1.8 H, s); 3.37 (0.8 H, s); 3.38-3.46 (2 H, m); 7.22-7.42 (5 H, m). |
| | | | | 13C-NMR (CDCl3): 18.6; 28.6; 30.0; 30.8; 31.2; 31.3; 32.2; 32.4; 35.8; 37.5; 38.0; 38.1; 40.2; 41.3; 41.6; 42.3; 43.6; 45.1; 53.4; 55.1; 56.3; 60.8; 126.3; 126.7; 127.6; 171.06; 171.09. |
| 87 | 1 | Bsp. Nr. 83/ Reduktion/ 55% | [M+H]+ = 373.4, Rt = 2.1 min. | 1H-NMR (CDCl3): 1.13 (6 H, s); 1.20-1.32 (2 H, m); 1.40-1.55 (6 H, m); 1.60-1.70 (2 H, m); 1.75-1.92 (2 H, m); 2.02 (6 H, s); 2.27 (2 H, br s); 2.34-2.40 (2 H, m); 2.48-2.54 (4 H, m); 3.17 (3 H, s); 7.24-7.40 (5 H, m). |
| | | | | 13C-NMR (CDCl3): 23.2; 24.9; 31.0; 34.7; 37.6; 38.1; 38.2; 41.0; 49.1; 53.4; 53.9; 57.4; 60.4; 65.4; 74.3; 126.4; 127.4; 127.6. |
| 88 | 3 | Bsp. Nr. 425/ Acylierung/ 41% | [MH-HNMe2]+ = 324.3, Rt = 3.0 min. | 1H-NMR (CDCl3): 0.96 (3 H, dt, J = 7.4 und 2.8 Hz); 1.34-1.44 (2 H, m); 1.61-1.73 (6 H, m); 1.83-2.02 (4 H, m); 2.10 (3 H, s); 2.12 (3 H, s); 2.21 (2 H, dt, J = 7.8 und 1.9 Hz); 3.29 (1 H, s); 3.36 (1 H, s); 3.46 (2 H, td, J = 14.6 und 7.2 Hz); 6.61 (1 H, t, J = 4.0 Hz); 6.84 (1 H, dd, J = 10.5 und 3.8 Hz). |
| | | | | 13C-NMR (CDCl3): 18.3; 18.4; 31.0; 31.2; 32.4; 33.0; 35.5; 36.3; 36.8; 37.2; 37.9; 38.1; 40.1; 42.0; 43.9; 45.0; 55.2; 55.5; 124.3; 124.5; 125.4; 125.6; 127.7; 127.9; 142.7. |
| 89 | 3 | Bsp. Nr. 425/ Reduktive Aminierung/ 44% | [M-HNMe2]+ = 310.3, Rt = 2.0 min. | 1H-NMR (CDCl3): 0.91 (3 H, t, J = 7.3 Hz); 1.28-1.35 (2 H, m); 1.37-1.41 (2 H, m); 1.43-1.47 (2 H, m); 1.51 (2 H, t, J = 6.9 Hz); 1.65-1.71 (2 H, m); 1.81-1.87 (2 H, m); 1.98-2.06 (2 H, m); 2.11 (6 H, s); 2.34-2.38 (2 H, m); 2.39 (2 H, s); 2.50 (2 H, t, J = 6.8 Hz); 6.60 (1 H, dd, J = 3.8 und 0.9 Hz); 6.83 (1 H, dd, J = 3.8 und 0.9 Hz). |
| | | | | 13C-NMR (CDCl3): 14.1; 20.9; 31.0; 33.3; 33.8; 34.4; 38.1; 38.2; 40.8; 53.9; 56.8; 60.1; 124.3; 125.4, 126.1; 127.5. |
| 90 | 1 | Bsp. Nr. 426/ Acylierung/ 42% | [MH-HNMe2]+ = 308.3, Rt = 2.9 min. | 1H-NMR (CDCl3): 0.96 (3 H, dt, J = 7.4 und 2.6 Hz); 1.35-1.44 (2 H, m); 1.61-1.73 (6 H, m); 1.81-1.99 (4 H, m); 2.10 (3 H, s); 2.12 (3 H, s); 2.19-2.23 (2 H, m); 3.28 (1 H, s); 3.36 (1 H, s); 3.46 (2 H, td, J = 14.6 und 7.2 Hz); 6.38 (1 H, ddd, J = 12.6, 4.0 und1.7 Hz); 6.42 (1 H, ddd, J = 4.8, 4.0 und 3.2 Hz). |
| | | | | 13C-NMR (CDCl3): 14.0; 18.3; 18.4; 31.0; 31.2; 32.1; 32.8; 35.6; 36.3; 36.8; 37.2; 37.9; 38.05; 38.06; 40.1; 42.0; 43.9; 45.0; 55.2; 56.4; 60.0; 60.1; 106.0; 106.2; 160.3; 160.4; 121.0; 121.1; 121.3; 162.5; 165.4; 171.87; 171.91. |
| 91 | 1 | Bsp. Nr. 426/ Reduktive Aminierung/ 49% | [MH-HNMe2]+ = 294.3, Rt = 1.0 min | 1H-NMR (CDCl3): 0.91 (3 H, t, J = 7.3 Hz); 1.28-1.48 (6 H, m); 1.52 (2 H, t, J = 6.9 Hz); 1.64-1.70 (2 H, m); 1.83 (2 H, t, J = 11.4 Hz); 1.93-2.04 (2 H, m); 2.11 (6 H, s); 2.34-2.38 (2 H, m); 2.40 (2 H, s); 2.50 (2 H, t, J = 6.9 Hz,); 6.37 (1 H, dd, J = 4.0 und 1.7 Hz,); 6.42 (1 H, dd, J = 4.0 und 3.2 Hz). |
| | | | | 13C-NMR (CDCl3): 14.1; 20.9: 31.0; 33.1; 34.4; 38.0; 38.1; 40.8, 53.9; 56.8; 59.9: 65.6; 106.1 (d, J = 11 Hz); 121.1; 127.3; 129.2; 132.7; 133.1; 163.8 (d, J = 289 Hz). |
| 94 | 3 | Bsp. Nr. 93/ Reduktion/ 72% | [M+H]+ = 335.3, geringe UV-Aktivität | 1H-NMR (CDCl3): 0.90 (3 H, t, J = 7.3 Hz); 0.92-1.00 (2 H, m); 1.06-1.75 (25 H, m); 2.17 (6 H, s); 2.30-2.39 (4 H, m); 2.53 (2 H, t, J = 6.8 Hz). |
| | | | | 13C-NMR (CDCl3): 14.1; 20.9; 26.2; 26.7; 29.4; 31.0; 33.1; 33.5; 36.1; 36.3; 37.3; 37.8; 41.0; 54.4; 56.8; 56.9; 68.3. |
| 96 | 3 | Bsp. Nr. 95/ Reduktion/ 66% | [M+H]+ = 321.3, geringe UV-Aktivität | 1H-NMR (CDCl3): 0.89 (3 H, t, J = 7.3 Hz); 1.00-1.10 (2 H, m); 1.22-1.84 (23 H, m); 2.18 (6 H, s); 2.30-2.40 (4 H, m); 2.55 (2 H, t, J = 6.7Hz). |
| | | | | 13C-NMR (CDCl3): 14.1; 20.9; 25.0; 29.5; 30.8; 33.3; 35.1; 36.0; 36.3; 36.7; 37.1; 37.3; 41.0; 54.2; 56.6; 56.7; 68.1. |
| 97 | 3 | Bsp. Nr. 424/ Acylierung/ 89% | [M+H]+ = 321.4, Rt = 2.8 min. | 1H-NMR (CDCl3): 0.948 und 0.953 (3 H, 2 t, J = 7.4); 1.10-1.39 (6 H, m); 1.40-1.84 (14 H, m); 2.00-2.12 (1 H, m); 2.16-2.23 (2 H, m); 2.25 (2.4 H, s); 2.26 (3.6 H, s); 3.15 (1.2 H, s); 3.22 (0.8 H, s); 3.46 (0.8 H, t , J = 7.1 Hz); 3.50 (1.2 H, t, J = 7.2 Hz). |
| 98 | 3 | Bsp. Nr. 97/ Reduktion/ 84% | [M+H]+ = 307.4, Rt = 1.3 min. | 1H-NMR (CDCl3): 0.90 (3 H, t, J = 7.3 Hz); 1.20-1.36 (8 H, m); 1.38-1.70 (14 H, m); 2.04 (1 H, tt, J = 10.9 und 7.6 Hz); 2.26 (6 H, s); 2.30-2.40 (4 H, m); 2.57 (2 H, t, J = 6.5 Hz). |
| | | | | 13C-NMR (CDCl3): 14.1; 20.7; 25.0; 27.2; 28.4; 30.7; 33.1; 34.9; 38.0; 41.5; 44.5; 54.6; 56.9; 57.6; 69.2. |
| 99 | 3 | Bsp. Nr. 424/ Acylierung/ 86% | [M+H]+ = 333.4, Rt = 2.9 min. | 1H-NMR (CDCl3): 1.12-1.38 (6 H, m); 1.40-2.18 (17 H, m); 2.25 (2 H, s); 2.26 (4 H, s); 2.28-2.37 (2 H, m); 3.05 (1.3 H, s); 3.15 (1 H, m); 3.21 (0.7 H, s); 3.37 (0.7 H, t, J = 7.1 Hz); 3.49 (1.3 H, t, J = 7.1 Hz). |
| | | | | 13C-NMR (CDCl3): 18.0; 18.1; 24.7; 25.0; 25.1; 26.9; 27.1; 28.48; 28.53; 29.8; 29.9; 31.7; 33.4; 37.7; 37.9; 38.0; 38.4; 40.5; 42.5; 44.0; 44.1; 44.5; 44.7; 57.8; 57.8; 58.8; 59.4; 173.1; 173.3. |
| 100 | 3 | Bsp. Nr. 424/ Acylierung/ 75% | [M+H]+ = 319.4, Rt = 2.7 min. | 1H-NMR (CDCl3): 0.68-0.75 (2 H, m); 0.94-1.00 (2 H, m); 1.14-1.40 (6 H, m); 1.41-1.78 (12 H, m); 1.84 (1 H, t, J = 7.1 Hz); 2.07 (1 H, m); 2.26 (3.6 H, s), 2.28 (2.4 H, s); 3.23 (0.8 H, s); 3.35 (1.2 H, s); 3.51 (1.2 H, t, J = 7.1 Hz); 3.66 (0.8 H, t, J = 7.1 Hz). |
| | | | | 13C-NMR (CDCl3): 7.2; 12.0; 12.3; 25.0; 25.2; 27.05; 27.1; 27.6; 28.51; 28.54; 29.7; 29.8; 32.1; 33.4; 37.7; 37.9; 37.8; 40.6; 42.4; 44.2; 44.3; 44.8; 45.3; 57.7; 57.9; 59.2; 60.3; 172.0; 172.1. |
| 101 | 3 | Bsp. Nr. 100/ Reduktion/ 84% | [M+H]+ = 305.4, Rt = 0.6 min. | 1H-NMR (CDCl3): 0.07-0.13 (2 H, m); 0.44-0.50 (2 H, m); 0.90 (1 H, m); 1.20-1.35 (6 H, m), 1.38-1.74 (12 H, m); 2.04 (1 H, tt, J = 10.8 und 7.6 Hz); 2.26 (6 H, s); 2.28 (2 H, d, J = 6.6 Hz); 2.42 (2 H, s); 2.63 (2 H, t, J = 6.8 Hz). |
| | | | | 13C-NMR (CDCl3): 3.8; 9.7; 25.0; 27.2; 28.4; 33.0; 34.9; 37.9; 41.5; 44.3; 54.6; 57.7; 61.8; 69.4. |
| 102 | 3 | Bsp. Nr. 99/ Reduktion/ 89% | [M+H]+ = 319.4, Rt = 1.4 min. | 1H-NMR (CDCl3): 1.18-1.34 (6 H, m); 1.40-1.92 (16 H, m); 1.98-2.10 (3 H, m); 2.26 (6 H, s); 2.31 (2 H, s); 2.44 (2 H, d, J = 6.9 Hz); 2.48-2.60 (3 H, m). |
| | | | | 13C-NMR (CDCl3): 18.6; 25.0; 27.2; 27.7; 28.4; 33.1; 34.9; 35.4; 37.9; 41.6; 44.3; 54.8; 57.6; 63.3; 69.3. |
| 103 | 3 | Bsp. Nr. 424/ Acylierung/ 68% | [M+H]+ = 333.4, Rt = 2.8 min. | 1H-NMR (CDCl3): 0.11-0.16 (2 H, m); 0.46-0.60 (2 H, m); 1.08 (1 H, m); 1.12-1.84 (18 H, m); 2.05 (1 H, tt, J = 11.0 und 7.6 Hz); 2.17 (2 H, dd, J = 6.7 und 4.8 Hz); 2.24 (2.5 H, s); 2.25 (3.5 H, s); 3.13 (1.2 H, s); 3.23 (0.8 H, s); 3.42 (0.8 H, t, J = 7.1 Hz); 3.51 (1.2 H, t, J = 7.2 Hz). |
| | | | | 13C-NMR (CDCl3): 4.4; 6.9; 7.0; 25.10; 25.12; 26.9; 27.1; 28.46; 28.50; 29.8; 29.79; 29.83; 31.9; 33.6; 37.81; 37.83; 39.3; 39.7; 40.4; 42.6; 44.0; 44.1; 44.3; 45.4; 53.4; 57.76; 57.85; 58.8; 60.3; 171.1; 171.2. |
| 104 | 3 | Bsp. Nr. 103/ Reduktion/ 58% | [M+H]+ = 319.4, Rt = 1.8 min. | 1H-NMR (CDCl3): 0.00-0.05 (2 H, m); 0.32-0.44 (2 H, m); 0.64 (1 H, m); 1.20-1.70 (20 H, m); 2.04 (1 H, tt, J = 10.7 und 7.6 Hz); 2.26 (6 H, s); 2.31 (2 H, s); 2.42-2.48 (2 H, m); 2.54 (2 H, t, J = 6.8 Hz). |
| | | | | 13C-NMR (CDCl3): 4.3; 9.2; 25.0; 27.2; 28.4; 33.2; 34.0; 35.1; 37.9; 41.3; 44.3; 54.6; 57.0; 57.7; 69.5. |
| 105 | 3 | Bsp. Nr. 424/ Acylierung/ 54% | [M+H]+ = 347.4, Rt = 3.1 min. | 1H-NMR (CDCl3): 1.13-1.95 (20 H, m); 2.00-2.19 (3 H, m); 2.25 (2.7 H, s); 2.27 (3.3 H, s); 2.34 (1.2 H, d, J = 7.4 Hz); 2.35 (0.8 H, d, J = 7.4 Hz); 2.73 (1 H, m); 3.16 (1.2 H, s); 3.20 (0.8 H, s), 3.46 (0.8 H, t, J = 7.1 Hz); 3.48 (1.2 H, t, J = 7.2 Hz). |
| | | | | 13C-NMR (CDCl3): 18.6; 18.8; 25.11; 2515; 26.9; 27.1; 28.48; 28.54; 28.6; 29.7; 32.1; 32.37; 32.39; 33.6; 37.7; 37.9; 40.6; 41.1; 41.5; 42.4; 44.0; 44.1; 44.3; 45.6; 57.7; 57.8; 58.5; 60.3; 170.8; 171.0. |
| 108 | 1 | Bsp. Nr. 427/ Acylierung/ 68% | [M+H]+ = 347.4, Rt = 2.8 min. | 1H-NMR (CDCl3): 0.97 und 0.975 (2 t, 3H, J = 7.4 Hz); 1.30-1.42 (m, 2H); 1.58 (t, 1H, J = 7.2Hz); 1.61-2.00 (m, 11 H); 2.18-2.25 (m, 2H); 3.05 und 3.06 (2 t, 4H, J = 7.0 Hz); 3.30 und 3.36 (2 s, 2H); 3.40-3.49 (m, 2H); 6.87 und 6.89 (2 t, 1H, J = 3.6 Hz); 7.07 und 7.11 (2 dd, 1H, J = 5.1, 1.5 Hz); 7.26 und 7.29 (2 dd, 1 H, J = 5.1, 1.5 Hz). |
| | | | | 13C-NMR (CDCl3): 13.7; 14.0; 15.9; 16.1; 18.3; 18.4; 18.9; 30.8; 31.0; 31.04; 31.7; 36.4; 36.9; 37.8; 40.2; 42.2; 43.9; 45.0; 46.7; 46.8; 55.6; 56.7; 58.7; 59.0; 123.5; 123.7; 124.6; 125.0; 126.4; 126.6; 171.8; 171.9. |
| 109 | 2 | Bsp. Nr. 428/ Acylierung/ 70% | [M+H]+ = 347.2, Rt = 1.8 min. | 1H-NMR (CDCl3): 0.87-0.99 (m, 3H); 1.33-1.42 (m, 2H); 1.57-1.87 (m, 12H); 2.14 (t, 1 H, J = 7.6 Hz); 2.20 (t, 1 H, J = 7.5 Hz); 3.07 (t, 4H, J = 6.8 Hz); 3.11 (s, 1 H); 3.20 (s, 1H); 3.44-3.58 (m, 2H); 6.84 (d, 0.5H, J = 3.5 Hz); 6.87 (d, 0.5H, J = 3.5 Hz); 7.05-7.11 (m, 1 H); 7.24-7.30 (m, 1H). |
| | | | | 13C-NMR (CDCl3): 13.96; 14.0; 15.9; 18.4; 30.76; 30.81; 36.3; 36.7; 40.1; 42.2; 44.0; 45.1; 46.71; 46.72; 123.6; 124.7; 126.4; 126.5; 171.8. |
| 110 | 1 | Bsp. Nr. 429/ Acylierung/ 73% | [M+H]+ = 341.4, Rt = 2.8 min. | 1H-NMR (CDCl3): 0.92-1.00 (m, 3H); 1.21-1.32 (m, 2H); 1.50 (t, 1H, J = 7.2 Hz); 1.58-1.82 (m, 9H); 1.94-2.14 (m, 2H); 2.17-2.26 (m, 2H); 2.92-2.98 (m, 4H); 3.25 (s, 1H); 3.38-3.48 (m, 3H); 7.26-7.34 (m, 3H); 7.36-7.46 (m, 2H). |
| | | | | 13C-NMR (CDCl3): 13.7; 14.01; 14.04; 16.5; 16.8; 18.3; 18.5; 18.9; 28.5; 29.4; 30.9; 31.2; 36.3; 36.9; 37.7; 40.4; 42.4; 43.8; 45.0; 46.6; 46.7; 55.3; 56.6; 59.3; 59.6; 126.5; 126.7; 127.5; 127.7; 127.87; 127.94; 171.8; 171.9. |
| 194 | 2 | Bsp. Nr. 430/ Acylierung/ 60% | [M+H]+ = 341.4, Rt = 2.7 min. | 1H-NMR (CDCl3): 0.89 und 0.94 (2 t, 3H, J = 7.4 Hz); 1.25-1.35 (m, 2H); 1.54-2.00 (m, 12H); 2.10 und 2.19 (2 t, 2H, J = 7.5 Hz); 2.96 (t, 4H, J = 6.9 Hz); 3.14 und 3.05 (2s, 2H); 3.49 (td, 2H, J = 14.0, 7.1 Hz); 7.25-7.34 (m, 3H); 7.36-7.45 (m, 2H). |
| | | | | 13C-NMR (CDCl3): 13.9; 14.0; 16.6; 18.4; 28.8; 28.9; 30.88; 30.9, 34.4; 36.3; 36.7; 40.4; 42.4; 44.1; 45.2; 46.6; 56.1; 57.7; 59.3; 126.6; 126.7; 127.5; 127.6; 127.8; 127.9; 137.6; 171.8. |
| 112 | 1 | Bsp. Nr. 429/ Reduktive Aminierung/ 52% | [M+H]+ = 327.4, Rt = 0.7 min. | 1H-NMR (CDCl3): 0.95 (t, 3H, J = 7.3 Hz); 1.28-1.43 (m, 4H); 1.55-1.66 (m, 4H); 1.72-1.86 (m, 6H); 2.05 (br s, 2H); 2.66-2.73 (m, 2H); 2.80 (s, 2H); 2.83-2.90 (m, 2H); 3.10 (t, 4H, J = 7.0 Hz); 7.31-7.38 (m, 3H); 7.42-7.49 (m, 2H). |
| | | | | 13C-NMR (CDCl3): 13.8; 16.4; 20.4; 29.0; 29.2; 33.2; 41.5; 46.9; 56.4; 64.5; 127.4; 127.7; 128.3. |
| 113 | 2 | Bsp. Nr. 430/ Reduktive Aminierung/ 42% | [M+H]+ = 327.4, Rt = 1.9 min. | 1H-NMR (CDCl3): 0.78-0.94 (m, 3H); 1.20-1.50 (m, 6H); 1.54-1.80 (m, 8H); 1.90-2.10 (m, 2H); 2.17 (s, 2H); 2.28-2.43 (m, 2H); 2.46-2.58 (m, 2H); 2.93-3.00 (m, 4H); 7.25-7.34 (m, 3H); 7.40 (t, 2H, J = 7.6 Hz). |
| | | | | 13C-NMR (CDCl3): 14.0; 16.7; 20.8; 26.9; 27.0; 29.1; 30.9; 34.2; 41.3; 46.7; 54.4; 56.7; 126.5; 127.8; 127.9. |
| 114 | 3 | Bsp. Nr. 17 / Alkylierung/ 56% | [M+H]+ = 293.3, Rt = 2.2 min. | 1H-NMR (CDCl3):1.20-1.28 (m, 2H); 1.68 (dt, 2H, J = 3.0 und 14.0 Hz); 1.89 (t, 2H, J = 7.0 Hz); 2.11 (s, 6H); 2.20 (dt, 2H, J = 3.1 und 13.0 Hz); 2.45 (br d, 2H, J = 13.8 Hz,); 2.85 (s, 3H); 3.23-3.28 (m, 2H); 6.85 (br d, 1 H, J = 3.4 Hz); 7.02 (dd, 1H, J = 3.6 und 5.1 Hz); 7.20 (dd, 1 H, J = 1.0 und 5.1 Hz). |
| | | | | 13C-NMR (CDCl3): 28.3 (2C); 29.8 (2C); 30.3; 31.7; 38.0 (2C); 44.3; 46.1; 58.4; 122.7; 123.6; 125.9; 145.8; 178.9. |
| 115 | 3 | Bsp. Nr. 17 / Alkylierung/ 67% | [M+H]+ = 335.3, Rt = 2.8 min. | 1H-NMR (CDCl3): 0.92 (dt, 3H, J = 7.4 Hz); 1.20-1.35 (m, 4H); 1.44-1.53 (m, 2H); 1.61-1.73 (m, 2H); 1.87 (t, 2H, J = 6.9 Hz); 2.10 (m, 6H); 2.20 (dt, 2H, J = 13.1 Hz); 2.44 (d, 2H, J = 2.6 und 13.6 Hz); 3.21-3.30 (m, 4H); 6.83-6.86 (m, 1 H); 6.99-7.03 (m, 1 H); 7.18-7.21 (m, 1H). |
| | | | | 13C-NMR (CDCl3): 13.7; 19.9; 28.2 (2C); 29.3; 30.4; 31.8 (2C); 37.9 (2C); 42.2; 43.7; 44.6; 58.4; 122.7; 123.6; 125.9; 145.9; 178.7. |
| 116 | 3 | Bsp. Nr. 17 / Alkylierung/ 18% | [M+H]+ = 361.3, Rt = 3.0 min. | 1H-NMR (CDCl3): 1.16 (m, 4H); 1.47-1.58 (m, 2H); 1.60-1.74(m, 6H); 1.87 (t, 2H, J = 6.9 Hz); 2.11 (s, 6H); 2.12-2.25 (m, 3H); 2.45 (d, 2H, J = 13.8 Hz); 3.21 (d, 2 H, J = 7.8 Hz); 3.25-3.30 (m, 2H); 6.86 (d, 1H, J = 3.5 Hz); 7.02 (dd, 1H, J = 3.6 und 5.1 Hz); 7.20 (dd, 1H, J = 1.1 und 5.1 Hz). |
| | | | | 13C-NMR (CDCl3): 25.1 (2C); 28.2; 30.3 (2C); 30.6; 31.8; 37.9 (2C); 38.0; 44.1; 44.6; 47.5; 58.5; 122.8; 123.6; 126.0; 178.8. |
| 118 | 2 | Bsp. Nr. 2 / Alkylierung/ 71% | [M+H]+ = 301.3, Rt = 2.4 min. | 1H-NMR (DMSO-d6): 0.93 (d, 2H, J = 13.0 Hz); 1.04 (dt, 2H, J = 14.0, 3.3 Hz); 1.45 (t, 2H, J = 6.9 Hz); 1.71 (dd, 2H, J = 14.8, 2.6 Hz); 1.82 (dt, 2H, J = 13.6, 3.0 Hz); 2.25 (s, 6H); 2.58 (s, 2H); 2.65 (s, 3H); 3.06-3.11 (m, 2H); 7.12-7.20 (m, 3H); 7.23-7.29 (m, 2H). |
| | | | | 13C-NMR (DMSO-d6): 26.7; 27.9; 28.8; 29.1; 36.2; 36.8; 43.5; 45.1; 56.6; 125.5; 127.6; 130.5, 139.0; 177.7. |
| 119 | 1 | Bsp. Nr. 1 / Alkylierung/ 45% | [M+H]+ = 301.3, Rt = 2.1 min. | 1H-NMR (CDCl3): 1.52-1.60 (m, 4H); 1.67-1.75 (m, 2H); 1.83-1.94 (m, 4H); 2.28 (s, 6H); 2.78 (s, 3H); 2.79 (s, 2H); 3.21-3.42 (m, 2H); 7.14-7.20 (m, 1 H); 7.21-7.25 (m, 4H). |
| | | | | 13C-NMR (CDCl3): 28.4; 29.4; 29.6; 32.8; 36.8; 37.4; 42.6; 46.0; 57.6; 125.7; 127.7; 130.7; 139.2; 179.0. |
| 120 | 2 | Bsp. Nr. 2 / Alkylierung/ 34% | [M+H]+ = 343.3, Rt = 3.0 min | 1H-NMR (CDCl3): 0.90 (t, 3H, J = 7.3 Hz); 1.02 (d, 2H, J = 13.3 Hz); 1.13 (dt, 2H, J = 14.0, 3.3 Hz); 1.26 (qd, 2H, J = 14.0, 7.3 Hz); 1.39-1.48 (m, 2H); 1.58 (t, 2H, J = 7.0 Hz); 1.71-1.78 (m, 2H); 2.10 (dt, 2H, J = 13.4, 2.9 Hz); 2.30 (s, 6H); 2.61 (s, 2H); 3.11 (t, 2H, J = 6.9 Hz); 3.22 (t, 2H, J = 7.2 Hz); 7.09-7.13 3 (m, 2H); 7.15-7.28 (m, 3H). |
| | | | | 13C-NMR (CDCl3): 13.7; 19.9; 27.2; 28.6; 29.3; 29.6; 36.9; 37.0; 42.1; 43.6; 44.8; 57.2; 125.6; 127.6; 127.7; 130.7; 139.5; 179.0. |
| 121 | 1 | Bsp. Nr. 1 / Alkylierung/ 35% | [M+H]+ = 343.3, Rt = 2.8 min. | 1H-NMR (CDCl3): 0.91 (t, 3H, J = 7.3 Hz); 1.23-1.34 (m, 2H); 1.41-1.61 (m, 6H); 1.69-1.77 (m, 2H); 1.82-1.92 (m, 4H); 2.28 (s, 6H); 2.81 (s, 2H); 3.19-3.25 (m, 4H); 7.13-7.19 (m, 1H); 7.20-7.25 (m, 4H). |
| | | | | 13C-NMR (CDCl3): 13.8; 20.0; 28.5; 29.3; 29.4; 32.7; 36.9; 37.5; 42.1; 43.1; 43.6; 57.6; 125.7; 127.8; 130.8; 139.4; 178.8. |
| 222 | 2 | Bsp. Nr. 2 / Alkylierung/ 31% | [M+H]+ = 369.3, Rt = 3.2 min. | 1H-NMR (CDCl3): 1.00-1.20 (m, 6H); 1.45-1.65 (m, 8H); 1.70-1.78 (m, 2H); 2.00-2.20 (m, 3H); 2.31 (s, 6H); 2.62 (s, 2H); 3.11-3.18 (m, 4H); 7.09-7.28 (m, 5H). |
| | | | | 13C-NMR (CDCl3): 25.1; 27.1; 28.6; 29.7; 30.2; 36.9; 37.0; 44.1; 44.7; 47.5; 57.3; 125.6; 127.7; 130.7; 139.4; 179.2. |
| 123 | 1 | Bsp. Nr. 1 / Alkylierung/ 16% | [M+H]+ = 369.4, Rt = 3.0 min. | 1H-NMR (CDCl3): 1.16-1.26 (m, 2H); 1.48-1.80 (m, 13H); 1.84-1.94 (m, 3H); 2.09-2.18 (m, 1H); 2.29 (s, 6H); 2.83 (s, 2H); 3.17 (d, 2H, J = 7.8 Hz); 3.26 (t, 2H, J = 6.9 Hz); 7.15-7.29 (m, 5H). |
| | | | | 13C-NMR (CDCl3): 25.1; 28.6; 29.4; 30.3; 32.7; 37.0; 37.6; 38.0; 43.2; 44.0; 47.4; 57.7; 125.7; 127.8; 130.8; 139.4; 179.0. |
| 21512 4 | 1 | Bsp. Nr. 24a / Alkylierung/ 38% | [MH-HNMe2]+ = 248.3 (100 %) | 1H-NMR (CDCl3): 1.43-1.51 (m, 2H); 1.73-1.82 (m, 2H); 1.96-2.09 (m, 4H); 2.10 (s, 6H); 2.18 (s, 2H); 2.83 (s, 3H); 3.21 (s, 2H); 6.85 (dd, 1H, J = 1.1, 3.6 Hz); 7.04 (dd, 1H, J = 3.6, 5.1 Hz); 7.24 (dd, 1H, J = 1.1, 5.1 Hz). |
| | | | [M+H]+ = 293.3 (70%), Rt = 1.4 min. | 13C-NMR (CDCl3): 29.7; 32.7; 32.9; 35.3; 38.0; 44.0; 59.3; 62.4; 123.4; 124.9; 126.3; 173.8. |
| 125 | 1 | Bsp. Nr. 24a / Alkylierung/ 52% | [M+H]+ = 335.3, Rt = 2.1 min. | 1H-NMR (CDCl3): 0.93 (t, 3H, J = 7.3 Hz); 1.26-1.36 (m, 2H); 1.42-1.52 (m, 4H); 1.72-1.81 (m, 2H); 1.95-2.09 (m, 4H); 2.10 (s, 6H); 2.18 (s, 2H); 3.20 (s, 2H); 3.25 (t, 2H, J = 7.3 Hz); 6.85 (dd, 1H, J = 1.1, 3.6 Hz); 7.04 (dd, 1 H, J = 3.6, 5.1 Hz); 7.24 (dd, 1H, J = 1.1, 5.1 Hz). |
| | | | | 13C-NMR (CDCl13): 13.8; 20.0; 29.3; 32.7; 32.8; 35.5; 38.1; 42.1; 44.3; 57.9; 59.3; 123.4; 124.9; 126.3; 142.5; 173.5. |
| 126 | 1 | Bsp. Nr. 24a / Alkylierung/ 33% | [MH-HNMe2]+ = 316.3 (100 %) | 1H-NMR (CDCl3): 1.16-1.26 (m, 2H); 1.42-1.59 (m, 4H); 1.60-1.82 (m, 7H); 1.97-2.17 (m, 4H); 2.12 (s, 6H); 2.19 (s, 2H); 3.19 (d, 2H, J = 7.9 Hz); 3.24 (s, 2H); 6.87 (br s, 1H); 7.03-7.07 (m, 1H); 7.23-7.28 (m, 1H). |
| | | | [M+H]+ = 361.4 (70 %), Rt = 2.7 min. | 1H-NMR (DMSO-d6): 1.09-1.19 (m, 2H); 1.30-1.38 (m, 2H); 1.44-1.74 (m, 9H); 1.77-1.94 (m, 2H); 2.01 (s, 6H); 2.08 (s, 2H); 2.09-2.18 (m, 2H); 3.07 (d, 2H, J = 7.8 Hz); 3.16 (s, 2H); 6.94 (s, 1H); 7.04-7.08 (m, 1H); 7.40-7.45 (m, 1H). |
| | | | | 13C-NMR (DMSO-d6): 24.6; 29.7; 32.0; 35.1; 37.3; 37.6; 38.9; 43.0; 46.3; 57.8; 58.7; 123.5; 124.7; 126.3; 143.3; 172.4. |
| 127 | 2 | Bsp. Nr. 24b / Alkylierung/ 25% | [MH-HNMe2]+ = 316.3 (100 %) | 1H-NMR (CDCl3): 1.12-1.22 (m, 2H); 1.42-1.56 (m, 4H); 1.57-1.70 (m, 4H); 1.73-1.81 (m, 2H); 2.01-2.17 (m, 5H); 2.18 (s, 6H); 2.36 (s, 2H); 3.06 (s, 2H); 3.15 (d, 2H, J = 7.8 Hz); 6.91 (br d, 1 H, J = 3.4 Hz); 7.07 (dd, 1H, J = 3.6, 5.1 Hz); 7.29 (br d, 1 H, 5.0 Hz). |
| | | | [M+H]+ = 361.4 (8 %), Rt = 3.1 min. | 13C-NMR (CDCl3): 25.1; 30.4; 32.4; 32.8; 35.6; 38.0; 38.1; 43.4; 47.4; 59.0; 124.2; 125.8; 126.5; 173.6. |
| 128 | 1 | Bsp. Nr. 24a / Alkylierung/ 61% | [M+H]+ = 361.4, Rt = 2.7 min. | 1H-NMR (CDCl3): 1.41-1.50 (2 H, m); 1.55-1.70 (5 H, m); 1.72-1.91 (4 H, m); 1.93-2.04 (5 H, m); 2.10 (6 H, s); 2.17 (2 H, s); 2.25 (1 H, td, J = 15.6 und 7.9 Hz); 3.14-3.20 (4 H, m); 6.85 (1 H, dd, J = 3.5 und 1.0 Hz); 7.04 (1 H, dd, J = 5.1 und 3.5 Hz); 7.24 (1 H, dd, J = 5.1 und 1.0 Hz). |
| | | | | 13C-NMR (CDCl3): 18.6; 28.2; 32.7; 32.8; 33.7; 34.3; 35.5; 38.1; 40.5; 44.3; 58.2; 59.3; 123.4; 124.9; 126.3; 173.4. |
| 129 | 2 | Bsp. Nr. 24b / Alkylierung/ 50% | [M+H]+ = 361.3, Rt = 3.1 min. | 1H-NMR (CDCl3): 1.42-1.65 (6 H, m); 1.70-1.91 (4 H, m); 1.93-2.09 (6 H, m); 2.10 (6 H, s); 2.22 (1 H, td, J = 15.6 und 7.9 Hz); 2.31 (2 H, s); 3.03 (2 H, s); 3.13 (2 H, t, J = 7.4 Hz); 6.85 (1 H, dd, J = 3,5 und 1.0 Hz); 7.04 (1 H, dd, J = 5.1 und 3.5 Hz); 7.24 (1 H, dd, J = 5.1 und 1.0 Hz). |
| | | | | 13C-NMR (CDCl3): 18.5; 28.2; 32.7; 32.9; 33.6; 34.2; 35.5; 38.1; 40.5; 43.8; 58.7; 59.5; 123.4; 125.0; 126.2; 173.4. |
| 130 | 1 | Bsp. Nr. 24a / Alkylierung/ 58% | [M+H]+ = 347.3, Rt = 2.5 min. | 1H-NMR (CDCl3): 0.03-0.09 (2 H, m); 0.44-0.49 (2 H, m); 0.60-0.70 (1 H, m); 1.38-1.50 (4 H, m); 1.72-1.80 (2 H, m); 1.90-2.09 (4 H, m); 2.10 (6 H, s); 2.18 (2 H, s); 3.23 (2 H, s); 3.34 (2 H, t, J = 7.3 Hz); 6.85 (1 H, d, J = 3.4 Hz); 7.04 (1 H, dd, J = 5.0 und 3.5 Hz); 7.24 (1 H, d, J = 5.0 Hz). |
| | | | | 13C-NMR (CDCl3): 4.3; 8.6; 32.5; 32.7; 32.8; 35.5; 38.0; 42.6; 44.3; 58.5; 59.3; 123.4; 124.8; 126.3; 173.5. |
| 131 | 2 | Bsp. Nr. 24b / Alkylierung/ 38% | [M+H]+ = 347.3, Rt = 2.9 min. | 1H-NMR (CDCl3): 0.02 (2 H, q, J = 5.4 Hz); 0.39-0.45 (2 H, m); 0.55-0.65 (1 H, m); 1.36 (2 H, dd, J = 14.4 und 7.0 Hz); 1.42-1.51 (2 H, m); 1.70-1.79 (2 H, m); 1.92-2.09 (4 H, m); 2.10 (6 H, s); 2.32 (2 H, s); 3.08 (2 H, s); 3.30 (2 H, t, J = 7.3 Hz); 6.85 (1 H, d, J = 3.5 Hz); 7.04 (1 H, dd, J = 5.1 und 3.8 Hz); 7.24 (1 H, d, J = 5.1 Hz). |
| | | | | 13C-NMR (CDCl3): 4.3; 8.6; 32.4; 32.7; 32.9; 35.5; 38.1; 42.6; 43.9; 59.0; 59.5; 123.4; 124.9; 126.2; 173.5. |
| 132 | 1 | Bsp. Nr. 24a / Alkylierung/ 69% | [M+H]+ = 347.3, Rt = 2.5 min. | 1H-NMR (CDCl3): 1.40-1.49 (2 H, m); 1.63-1.78 (4 H, m); 1.85-2.09 (8 H, m); 2.10 (6 H, s); 2.17 (2 H, s); 2.52 (1 H, td, J = 15.6 und 7.8 Hz); 3.17 (2 H, s); 3.29 (2 H, d, J = 7.6 Hz); 6.84 (1 H, d, J = 3.4 Hz); 7.04 (1 H, dd, J = 5.0 und 3.6 Hz); 7.24 (1 H, d, J = 5.0 Hz). |
| | | | | 13C-NMR (CDCl3): 18.4; 26.4; 32.6; 32.8; 33.9; 35.7; 38.1; 44.1; 47.8; 58.2; 59.3; 123.4; 124.9; 126.3; 173.6. |
| 133 | 2 | Bsp. Nr. 24b / Alkylierung/ 83% | [M+H]+ = 347.3, Rt = 2.9 min. | 1H-NMR (CDCl3): 1.40-1.48 (2 H, m); 1.63-1.77 (4 H, m); 1.80-2.09 (8 H, m); 2.10 (6 H, s); 2.31 (2 H, s); 2.47 (1 H, sept, J = 7.7 Hz); 3.01 (2 H, s); 3.25 (2 H, d, J = 7.6 Hz); 6.85 (1 H, d, J = 3.5 Hz); 7.04 (1 H, dd, J = 5.1 und 3.5 Hz); 7.24 (1 H, d, J = 5.1 Hz). |
| | | | | 13C-NMR (CDCl3): 18.4; 26.4; 32.7; 32.8; 33.9; 35.7; 38.1; 43.6; 47.8; 58.9; 59.6; 123.4; 125.0; 126.2; 173.6. |
| 134 | 1 | Bsp. Nr. 24a / Alkylierung/ 61% | [M+H]+ = 333.3, Rt = 2.2 min. | 1H-NMR (CDCl3): 0.18-0.23 (2 H, m); 0.49-0.55 (2 H, m); 0.83-0.94 (1 H, m); 1.44-1.52 (2 H, m); 1.74-1.83 (2 H, m); 1.90-2.08 (4 H, m); 2.11 (6 H, s); 2.19 (2 H, s); 3.13 (2 H, d, J = 7.1 Hz); 3.32 (2 H, s); 6.85 (1 H, d, J = 3.5 Hz); 7.05 (1 H, dd, J = 5.1 und 3.5 Hz); 7.24 (1 H, d, J = 4.4 Hz). |
| | | | | 13C-NMR (CDCl3): 3.4; 9.1; 19.8; 32.7; 32.9; 35.6; 38.1; 44.2; 47.0; 58.2; 59.3; 123.4; 124.9; 126.3; 173.4. |
| 135 | 2 | Bsp. Nr. 24b / Alkylierung/ 40% | [M+H]+ = 333.3, Rt = 2.7 min. | 1H-NMR (CDCl3): 0.14-0.19 (2 H, m); 0.44-0.50 (2 H, m); 0.78-0.88 (1 H, m); 1.44-1.53 (2 H, m); 1.72-1.81 (2 H, m); 1.93-2.09 (4 H, m); 2.10 (6 H, s); 2.33 (2 H, s); 3.10 (2 H, d, J = 7.1 Hz); 3.16 (2 H, s); 6.86 (1 H, dd, J = 3.5 und 1.0 Hz); 7.05 (1 H, dd, J = 5.1 und 3.5 Hz); 7.25 (1 H, dd, J = 5.1 und 1.0 Hz). |
| | | | | 13C-NMR (CDCl3): 3.4; 9.1; 32.7; 32.9; 35.7; 38.1; 43.7; 47.0; 58.7; 59.6; 123.4; 125.0; 126.2; 173.4. |
| 136 | 1 | Bsp. Nr. 24a / Alkylierung/ 57% | [M+H]+ = 377. 3, Rt = 2.3 min. | 1H-NMR (CDCl3): 1.40-1.52 (3 H, m); 1.63-1.93 (6 H, m); 1.95-2.08 (5 H, m); 2.10 (6 H, s); 2.17 (2 H, s); 3.20-3.27 (2 H, m); 3.28-3.41 (2 H, m); 3.67-3.74 (1 H, m); 3.75-3.88 (2 H, m); 6.84 (1 H, dd, J = 3.5 und 1.1 Hz); 7.04 (1 H, dd, J = 5.1 und 3.1 Hz); 7.23 (1 H, dd, J = 5.1 und 1.1 Hz). |
| | | | | 13C-NMR (CDCl3): 25.6; 31.4; 32.67; 32.72; 32.74; 33.3; 35.6; 38.0; 40.0; 44.2; 58.4; 59.2; 67.7; 77.0; 123.4; 124.8; 126.2; 173.6. |
| 137 | 2 | Bsp. Nr. 24b / Alkylierung/ 53% | [MH-HNMe2]+ = 332.3 (100 %) | 1H-NMR (CDCl3): 1.39-1.50 (3 H, m); 1.60-1.80 (4 H, m); 1.80-1.90 (2 H, m); 1.94-2.09 (5 H, m); 2.10 (6 H, s); 2.31 (2 H, s); 3.05-3.12 (2 H, m); 3.24-3.38 (2 H, m); 3.64-3.71 (1 H, m); 3.72-3.85 (2 H, m); 6.84 (1 H, dd, J = 3.5 und 1.1 Hz); 7.04 (1 H, dd, J = 5.1 und 3.1 Hz); 7.23 (1 H, dd, J = 5.1 und 1.0 Hz). |
| | | | [M+H]+ = 377.4 (10 %), Rt = 2.6 min. | 13C-NMR (CDCl3): 25.6; 31.4; 32.7; 32.73; 32.8; 33.2; 35.6; 39.9; 43.6; 58.9; 59.5; 67.7; 76.9; 123.4; 124.9; 126.2; 173.6. |
| 138 | 1 | Bsp. Nr. 24a / Alkylierung/ 51% | [M+H]+ = 377.4, Rt = 2.2 min. | 1H-NMR (CDCl3): 1.28-1.40 (2 H, m); 1.43-1.53 (4 H, m); 1.62-1.90 (3 H, m); 1.95-2.09 (4 H, m); 2.10 (6 H, s); 2.20 (2 H, s); 3.14 (2 H, d, J = 7.3 Hz); 3.23 (2 H, s); 3.35 (2 H, dt, J = 11.8 und 1.9 Hz); 3.96 (2 H, br dd, J = 11.5 und 2.6 Hz); 6.85 (1 H, dd, J = 3.3 und 1.1 Hz); 7.04 (1 H, dd, J = 4.9 und 3.6 Hz); 7.24 (1 H, d, J = 4.9 Hz). |
| | | | | 13C-NMR (CDCl3): 30.7; 32.7; 32.8; 33.7; 35.8; 38.0; 44.1; 48.4; 59.2; 59.9; 67.5; 67.7; 123.5; 124.8; 126.3; 174.1. |
| 139 | 2 | Bsp. Nr. 24b / Alkylierung/ 45% | [M+H]+ = 377.4, Rt = 2.5 min. | 1H-NMR (CDCl3): 1.31 (2 H, ddd, J = 17.6, 11.8 und 4.4 Hz); 1.43-1.54 (4 H, m); 1.71-1.85 (3 H, m); 1.93-2.09 (4 H, m); 2.10 (6 H, s); 2.34 (2 H, s); 3.05-3.12 (4 H, m); 3.32 (2 H, dt, J = 11.7 und 2.2 Hz); 3.91-3.97 (2 H, m); 6.85 (1 H, dd, J = 3.6 und 1.1 Hz); 7.04 (1 H, dd, J = 5.1 und 3.6 Hz); 7.24 (1 H, dd, J = 5.1 und 1.1 Hz). |
| | | | | 13C-NMR (CDCl3): 30.7; 32.7; 32.9; 33.7; 35.8; 38.1; 43.3; 48.3; 59.6; 59.9; 67.5; 123.5; 125.0; 126.3; 174.1. |
| 140 | 2 | Bsp. Nr. 24b / Alkylierung/ 42% | [M+H]+ = 377.2, geringe UV-Aktivität | 1H-NMR (CDCl3): 1.40-1.61 (5 H, m); 1.70-1.80 (2 H, m); 1.91-2.09 (5 H, m); 2.10 (6 H, s); 2.11-1.26 (1 H, m); 2.32 (2 H, s); 3.05 (2 H, s); 3.24 (2 H, t, J = 7.4 Hz); 3.32 (1 H, dd, J = 8.3 und 6.9 Hz); 3.72 (1 H, dd, J = 15.4 und 7.7 Hz); 3.79-3.90 (2 H, m); 6.84 (1 H, dd, J = 3.5 und 1.0 Hz); 7.04 (1 H, dd, J = 5.1 und 3.5 Hz); 7.24 (1 H, dd, J = 5.1 und 0.9 Hz). |
| | | | | 13C-NMR (CDCl3): 30.7; 32.2; 32.7; 32.72; 32.9; 35.6; 36.9; 38.1; 41.3; 43.7; 58.6; 59.5; 67.8; 73.1; 123.5; 125.0; 126.2; 173.6. |
| 141 | 1 | Bsp. Nr. 24a / Alkylierung/ 49% | [M+H]+ = 377.2, geringe UV-Aktivität | 1H-NMR (CDCl3): 1.41-1.70 (5 H, m); 1.72-1.81 (2 H, m); 2.00-2.09 (4 H, m); 2.10 (6 H, s); 2.11-1.18 (2 H, m); 2.19 (2 H, s); 3.20 (2 H, s); 3.28 (2 H, t, J = 7.4 Hz); 3.36 (1 H, dd, J = 8.3 und 6.9 Hz); 3.71-3.78 (1 H, m); 3.82-3.93 (2 H, m); 6.85 (1 H, dd, J = 3.5 und 1.0 Hz); 7.04 (1 H, dd, J = 5.1 und 3.5 Hz); 7.24 (1 H, dd, J = 5.1 und 1.0 Hz). |
| | | | | 13C-NMR (CDCl3):): 30.7; 32.2; 32.7; 32.79; 35.6; 36.1; 36.9; 38.1; 41.4; 44.2; 58.1; 59.2; 67.9; 73.1; 123.5; 124,8; 126.3; 173.6. |
| 142 | 1 | Bsp. Nr. 24a / Alkylierung/ 25% | [M+H]+ = 363.2, geringe UV-Aktivität | 1H-NMR (CDCl3): 1.43-1.51 (2 H, m); 1.57-1.67 (2 H, m); 1.73-1.82 (2 H, m); 1.95-2.09 (4 H, m); 2.10 (6 H, s); 2.20 (2 H, s); 2.47-2.56 (1 H, m); 3.19-3.26 (3 H, m); 3.36 (1 H, dd, J = 13.6 und 7.6 Hz); 3.47 (1 H, dd, J = 8.6 und 6.3 Hz); 3.76 (1 H, td, J = 8.5 und 7.3 Hz); 3.82-3.91 (2 H, m); 6.85 (1 H, dd, J = 3.5 und 0.9 Hz); 7.05 (1 H, dd, J = 5.1 und 3.5 Hz); 7.25 (1 H, dd, J = 5.0 und 0.8 Hz). |
| | | | | 13C-NMR (CDCl3): 30.1; 32.6; 32.8; 35.8; 38.0; 38.1; 44.0; 45.3; 58.8; 59.2; 67.7; 71.4; 123.5; 124.8; 126.3; 174.0. |
| 143 | 2 | Bsp. Nr. 24b / alkylierung/ 22% | [M+H]+ = 363.2, geringe UV-Aktivität | 1H-NMR (CDCl3): 1.40-1.51 (2 H, m); 1.52-1.62 (1 H, m); 1.70-1.79 (2 H, m); 1.89-2.09 (5 H, m); 2.10 (6 H, s); 2.32 (2 H, s); 2.42-2.50 (1 H, m); 3.07 (2 H, s); 3.18 (1 H, dd, J = 13.7 und 7.5 Hz); 3.31 (1 H, dd, J = 13.7 und 7.6 Hz); 3.42 (1 H, dd, J = 8.6 und 6.2 Hz); 3.68-3.88 (3H, m); 6.84 (1 H, dd, J = 3.5 und 1.1 Hz); 7.03 (1 H, dd, J = 5.1 und 3.5 Hz); 7.24 (1 H, dd, J = 5.1 und 1.1 Hz). |
| | | | | 13C-NMR (CDCl3):0.1; 32.7; 32.8; 35.8; 37.9; 38.1; 43.5; 45.2; 59.2; 59.5; 67.6; 71.3; 123.5; 125.0; 126.3; 174.0. |
| 144 | 1 | Bsp. Nr. 24a / Alkylierung/ 49% | [MH-HNMe2]+ = 327.2 (95 %) | 1H-NMR (CDCl3): 87 (2 H, m); 1.26 (2 H, m); 1.44-1.52 (2 H, m); 1.70 (2 H, t, J = 7.0 Hz); 1.76-1.84 (2 H, m); 1.96-2.09 (4 H, m); 2.10 (6 H, s); 2.21 (2 H, s); 3.31 (2 H, s); 3.48 (2 H, t, J = 7.1 Hz); 6.85 (1 H, dd, J = 3.6 und 0.8 Hz); 7.04 (1 H, dd, J = 5.0 und 3.6 Hz); 7.24 (1 H, dd, J = 5.1 und 0.7 Hz). |
| | | | [M+H]+ = 372.2 (100 %), Rt = 2.3 min. | 13C-NMR (CDCl3): 7.6; 14.1; 32.6; 32.7; 35.7; 38.0; 41.2; 44.0; 59.2; 123.0; 123.4; 124.8; 126.3; 174.1. |
| 145 | 2 | Bsp. Nr. 24b / Alkylierung/ 50% | [MH-HNMe2]+ = 327.2 (100 %) | 1H-NMR (CDCl3): 0.85 (2 H, m); 1.23 (2 H, m); 1.49 (2 H, ddd, J = 13.2, 9.1 und 4.0 Hz); 1.66 (2 H, t, J = 7.1 Hz); 1.72-1.80 (2 H, m); 1.92-2.09 (4 H, m); 2.10 (6 H, s); 2.33 (2 H, s); 3.16 (2 H, s); 3.44 (2 H, t, J = 7.1 Hz); 6.84 (1 H, dd, J = 3.6 und 1.1 Hz); 7.04 (1 H, dd, J = 5.1 und 3.6 Hz); 7.24 (1 H, dd, J = 5.1 und 1.1 Hz). |
| | | | [M+H]+ = 372.2 (90 %), Rt = 2.5 min. | 13C-NMR (CDCl3): 7.5; 14.0; 32.5; 32.7; 32.9; 35.7; 38.1; 41.1; 43.4; 59.5; 122.9; 123.5; 125.0; 126.3; 174.1. |
| 146 | 1 | Bsp. Nr. 24a / Alkylierung/ 51% | [M+H]+ = 349.2, Rt = 2.1 min. | 1H-NMR (CDCl3): 1.42-1.53 (2 H, m); 1.73-1.85 (3 H, m); 1.97-2.08 (3 H, m); 2.09 (6 H, s); 2.21 (2 H, d, J = 2.8 Hz); 2.41-2.52 (1 H, m); 2.62-2.70 (1 H, m); 3.34 (1 H, d, J = 10.1 Hz); 3.44-3.57 (3 H, m); 4.46-4.53 (1 H, m); 4.62-4.69 (1 H, m); 4.93-5.00 (1 H, m); 6.84 (1 H, dd, J = 3.5 und 1.1 Hz); 7.04 (1 H, dd, J = 5.1 und 3.5 Hz); 7.23 (1 H, dd, J = 5.1 und 1.1 Hz). |
| | | | | 13C-NMR (CDCl3): 24.8; 32.6; 32.64; 32.7; 35.8; 38.0; 43.8; 48.1; 59.3; 60.0; 68.5; 81.3; 123.4; 124.8; 126.2; 142.8; 174.3. |
| 147 | 2 | Bsp. Nr. 24b / Alkylierung/ 32% | [MH-HNMe2]+ = 304.2, Rt = 2.3 min. | 1H-NMR (CDCl3): 1.41-1.56 (2 H, m); 1.64-1.83 (3 H, m); 1.90-2.09 (3 H, m); 2.10 (6 H, s); 2.35 (2 H, d, J = 5.0 Hz); 2.39-2.49 (1 H, m); 2.58-2:60 (1 H, m); 3.20 (1 H, d, J = 10.0 Hz); 3.32 (1 H, d, J = 10.0 Hz); 3.42-3.53 (2 H, m); 4.41-4.48 (1 H, m); 4.59-4.65 (1 H, m); 4.90-4.97 (1 H, m); 6.83 (1 H, dd, J = 3.5 und 1.1 Hz); 7.03 (1 H, dd, J = 5.1 und 3.5 Hz); 7.23 (1 H, dd, J = 5.1 und 1.1 Hz). |
| | | | | 13C-NMR (CDCl3): 24.8; 32.66; 32.7; 32.8; 35.9; 38.1; 43.4; 48.1; 59.5; 60.4; 68.5; 81.5; 123.4; 124.9; 126.2; 142.7; 174.3. |
| 148 | 1 | Bsp. Nr. 24a / Alkylierung/ 44% | [MH-HNMe2]+ = 304.2 (100 %) | 1H-NMR (CDCl3): 1.40-1.50 (2 H, m); 1.67 (1 H, br s); 1.70-1.80 (2 H, m); 1.93-2.09 (3 H, m); 2.10 (6 H, s); 2.18 (2 H, s); 3.16 (2 H, s); 3.17-3.25 (1 H, m); 3.59 (2 H, d, J = 7.2 Hz); 4.46 (2 H, t, J = 6.2 Hz); 4.78 (2 H, dd, J = 7.8 und 6.2 Hz); 6.84 (1 H, dd, J = 3.6 und 1.1 Hz); 7.04 (1 H, dd, J = 5.1 und 3.6 Hz); 7.25 (1 H, dd, J = 5.1 und 1.1 Hz). |
| | | | [M+H]+ = 349.3 (100 %), Rt = 20 mn. | 13C-NMR (CDCl3): 32.6; 32.7; 34.1; 35.9; 38.0; 43.9; 45.4; 59.2; 75.4; 123.5; 124.8; 126.3; 174.0. |
| 149 | 2 | Bsp. Nr. 24b / Alkylierung/ 46% | [MH-HNMe2]+ = 304.2 (100 %) | 1H-NMR (CDCl3): 1.40-1.49 (2 H, m); 1.62-1.77 (3 H, m); 1.90-2.09 (3 H, m); 2.10 (6 H, s); 2.32 (2 H, s); 3.01 (2 H, s); 3.13-3.21 (1 H, m); 3.55 (2 H, d, J = 7.2 Hz); 4.42 (2 H, t, J = 6.2 Hz); 4.74 (2 H, dd, J = 7.8 und 6.2 Hz); 6.84 (1 H, dd, J = 3.5 und 1.1 Hz); 7.04 (1 H, dd, J = 5.1 und 3.5 Hz); 7.25 (1 H, dd, J = 5.1 und 1.1 Hz). |
| | | | [M+H]+ = 349.2 (100 %), Rt = 2.2 min. | 13C-NMR (CDCl3): 32.7; 32.8; 34.0; 35.9; 38.1; 43.2; 45.4; 59.5; 75.4; 123.5; 125.0; 126.3; 174.0. |
| 150 | 1 | Bsp. Nr. 24a / Alkylierung/ 58% | [MH-HNMe2]+ = 346.3 (95% | 1H-NMR (CDCl3): 1.43-1.51 (2 H, m); 1.74-1.83 (2 H, m); 1.93-1.98 (2 H, m); 1.99-2.07 (4 H, m); 2.10 (6 H, s); 2.11-2.27 (6 H, m); 2.49-2.59 (2 H, m); 3.25 (2 H, s); 3.36-3.41 (2 H, m); 6.84 (1 H, dd, J = 3.5 und 0.9 Hz); 7.04 (1 H, dd, J = 5.1 und 3.6 Hz); 7.24 (1 H, dd, J = 5.1 und 0.9 Hz). |
| | | | [M+H]+ = 386.3 (100 %), Rt = 2.5 min. | 13C-NMR (CDCl3): 16.8; 32.1; 32.7; 33.8; 34.8; 35.6; 38.0; 39.0; 44.0; 58.5; 59.2; 123.4; 124.1; 124.8; 126.3; 174.0. |
| 151 | 2 | Bsp. Nr. 24b / Alkylierung/ 85% | [MH-HNMe2]+ = 341.2 (100 %) | 1H-NMR (CDCl3): 1.43-1.52 (2 H, m); 1.72-1.80 (2 H, m); 1.89-1.94 (2 H, m); 1.95-2.07 (4 H, m); 2.11 (6 H, s); 2.12-2.23 (4 H, m); 2.33 (2 H, s); 2.47-2.56 (2 H, m); 3.11 (2 H, s); 3.33-3.38 (2 H, m); 6.85 (1 H, dd, J = 3.6 und 1.1 Hz); 7.04 (1 H, dd, J = 5.1 und 3.6 Hz); 7.24 (1 H, dd, J = 5.1 und 1.1 Hz). |
| | | | [M+H]+ = 386.3 (85 %), Rt = 2.7 min. | 13C-NMR (CDCl3): 16.8; 32.1; 32.7; 32.9; 33.8; 34.8; 35.7; 38.1; 39.0; 59.0; 59.6; 123.5; 124.1; 125.0; 126.3; 174.0. |
| 154 | 2 | Bsp. Nr. 153 / Alkylierung/ 64% | [M+H]+ = 307.3, Rt = 2.2 min. | 1H-NMR (CDCl3): 1.42-1.51 (2 H, m); 1.66-1.79 (2 H, m); 1.82-2.09 (4 H, m); 2.11 (6 H, s); 2.17 (2 H, s); 2.47 (3 H, d, J = 1.1 Hz); 2.82 (3 H, s); 3.20 (2 H, s); 6.51 (1 H, d, J = 3.5 Hz); 6.66-6.69 (1 H, m). |
| | | | | 13C-NMR (CDCl3): 15.2; 29.7; 32.6; 33.0; 35.3; 38.1; 44.1; 59.4; 60.5; 124.5; 124.9; 137.9; 173.8. |
| 155 | 1 | Bsp. Nr. 152 / Alkylierung/ 63% | [MH-HNMe2]+ = 262.3 (100 %) | 1H-NMR (CDCl3): 1.43-1.52 (2 H, m); 1.68-1.76 (2 H, m); 1.85-2.09 (4 H, m); 2.10 (6 H, s); 2.30 (2 H, s); 2.46 (3 H, d, J = 1.1 Hz); 2.79 (3 H, s); 3.05 (2 H, s); 6.60 (1 H, d, J = 3.5 Hz); 6.66-6.68 (1 H, m). |
| | | | [M+H]+ = 307.3 (10 %), Rt = 2.4 min. | 1H-NMR (CDCl3): 15.2; 29.6; 32.6; 33.1; 35.4; 38.2; 43.3; 59.6; 61.2; 124.4; 124.9; 137.8; 173.8. |
| 156 | 2 | Bsp. Nr. 153 / Alkylierung/ 68% | [M+H]+ = 349.4, Rt = 2.6 min. | 1H-NMR (CDCl3): 0.92 (3 H, t, J = 7.3 Hz); 1.25-1.36 (2 H, m); 1.43-1.52 (4 H, m); 1.68-1.77 (2 H, m); 1.85-2.10 (4 H, m); 2.11 (6 H, s); 2.18 (2 H, s); 2.46 (3 H, d, J = 1.1 Hz); 3.19 (2 H, s); 3.25 (2 H, t, J = 7.3 Hz); 6.61 (1 H, d, J = 3.5 Hz); 6.66-6.69 (1 H, m). |
| | | | | 13C-NMR (CDCl13): 13.8; 15.2; 20.0; 29.3; 32.7; 32.8; 35.5; 38.1; 42.0; 44.5; 57.8; 59.4; 124.5; 124.9; 137.8; 173.6. |
| 157 | 1 | Bsp. Nr. 152 / Alkylierung/ 68% | [MH-HNMe2]+ = 304.3 (100 %) | 1H-NMR (CDCl3): 0.90 (3 H, t, J = 7.3 Hz); 1.22-1.33 (2 H, m); 1.39-1.51 (4 H, m); 1.66-1.76 (2 H, m); 1.86-2.09 (4 H, m); 2.10 (6 H, s); 2.31 (2 H, s); 2.46 (3 H, d, J = 1.0 Hz); 3.04 (2 H, s); 3.21 (2 H, t, J = 7.3 Hz); 6.60 (1 H, d, J = 3.5 Hz); 6.65-6.68 (1 H, m). |
| | | | [M+H]+ = 349.4 (10 %), Rt = 3.0 min. | 13C-NMR (CDCl3): 13.7; 15.2; 20.0; 29.3; 32.6; 32.9; 35.6; 38.2; 42.0; 43.6; 58.7; 59.6; 124.4; 124.9; 137.8; 173.6. |
| 158 | 2 | Bsp. Nr. 153 / Alkylierung/ 13% | [M+H]+ = 375.4, Rt = 2.9 min. | 1H-NMR (CDCl3): 1.16-1.27 (2 H, m); 1.42-1.80 (11 H, m); 1.84-2.20 (12 H, m); 2.48 (3 H, s); 3.19 (2 H, d, J = 7.8 Hz); 3.24 (2 H, br s); 6.63 (1 H, br s); 6.70 (1 H, br s). |
| | | | | 13C-NMR (CDCl3): 15.3; 25.1; 30.4; 32.3; 32.7; 35.5; 38.0; 47.4; 124.7; 173.6. Alle anderen Signale konnten auf Grund geringer Probenkonzentration nicht identifiziert werden. |
| 159 | 1 | Bsp. Nr. 152 / Alkylierung/ 32% | [MH-HNMe2]+ = 330.3 (100 %) | 1H-NMR (CDCl3): 1.11-1.22 (2 H, m); 1.42-1.76 (10 H, m); 1.85-1.97 (2 H, m); 2.00-2.09 (3 H, m); 2.10 (6 H, s); 2.31 (2 H, s); 2.46 (3 H, d, J = 1.0 Hz); 3.06 (2 H, s); 3.15 (2 H, d, J = 7.8 Hz); 6.61 (1 H, d, J = 3.5 Hz); 6.65-6.68 (1 H, m). |
| | | | [M+H]+ = 375.4 (10 %), Rt = 3.2 min. | 13C-NMR (CDCl3): 15.2; 25.1; 30.4; 32.6; 32.9; 35.7; 38.0; 38.2; 43.5; 47.4; 59.1; 59.7; 126.4; 125.0; 137.8; 173.8. |
| 160 | 1 | Bsp. Nr. 152 / Alkylierung/ 66% | [M+H]+ = 347.3, Rt = 2.9 min. | 1H-NMR (CDCl3):0.12-0.18 (2 H, m); 0.43-0.49 (2 H, m); 0.77-0.87 (1 H, m); 1.43-1.52 (2 H, m); 1.68-1.77 (2 H, m); 1.85-2.08 (4 H, m); 2.10 (6 H, s); 2.31 (2 H, s); 2.45 (3 H, s); 3.08 (2 H, d, J = 7.1 Hz); 3.14 (2 H, s); 6.60 (1 H, d, J = 3.4 Hz); 6.65-6.69 (1 H, m). |
| | | | | 13C-NMR (CDCl3): 3.4; 9.0; 15.2; 32.5; 32.9; 35.6; 38.1; 43.5; 46.9; 58.8; 59.6; 124.4; 124.9; 137.8; 173.4. |
| 161 | 1 | Bsp. Nr. 152 / Alkylierung/ 69% | [MH-HNMe2]+ = 316.3 (100 %) | 1H-NMR (CDCl3): 1.40-1.49 (2 H, m); 1.65-1.75 (4 H, m); 1.80-2.10 (8 H, m); 2.11 (6 H, s); 2.30 (2 H, s); 2.42-2.51 (1 H, m); 2.47 (3 H, d, J = 1.0 Hz); 3.01 (2 H, s); 3.25 (2 H, d, J = 7.6 Hz); 6.61 (1 H, d, J = 3.5 Hz); 6.66-6.69 (1 H, m). |
| | | | [M+H]+ = 361.3 (10 %), Rt = 3.1 min. | 13C-NMR (CDCl3): 15.2; 18.4; 18.5; 24.8; 26.4; 26.5; 32.6; 32.8; 33.9; 35.7; 38.2; 43.4; 47.8; 59.0; 59.7; 124.4; 124.9; 137.8; 173.7. |
| 163 | 2 | Bsp. Nr. 162 Stufe 3 / Alkylierung/ 87% | [M+H]+ = 329.4, Rt = 2.4 min. | 1H-NMR (CDCl3): 0.93 (3 H, t, J = 7.3 Hz); 1.27-1.42 (4 H, m); 1.45-1.53 (2 H, m); 1.70-1.78 (2 H, m); 1.85-2.20 (2 H, m); 2.03 (6 H, s); 2.13 (2 H, s); 2.14-2.25 (2 H, m); 3.23 (2 H, s); 3.26 (2 H, t, J = 7.3 Hz); 7.26-7.32 (3 H, m); 7.35-7.41 (2 H, m). |
| | | | | 13C-NMR (CDCl3): 13.8; 20.0; 29.4; 30.2; 32.9; 35.7; 38.0; 42.1; 44.6; 57.9; 60.2; 126.7; 127.4; 127.8; 173.6. |
| 164 | 1 | Bsp. Nr. 431 / Alkylierung/ 61% | [M+H]+ = 329.4, Rt = 2.9 min. | 1H-NMR (CDCl3): 0.88 (3 H, t, J = 7.3 Hz); 1.22-1.46 (6 H, m); 1.66-1.75 (2 H, m); 1.85-2.00 (2 H, m); 2.03 (6 H, s); 2.15-2.31 (2 H, m); 2.35 (2 H, s); 2.97 (2 H, s); 3.20 (2 H, t, J = 7.3 Hz); 7.24-7.32 (3 H, m); 7.35-7.40 (2 H, m). |
| | | | | 13C-NMR (CDCl3): 13.7; 20.0; 29.3; 30.1; 30.11; 30.2; 30.25; 32.9; 33.0; 35.8; 38.0; 41.9; 43.6; 58.9; 60.5; 126.6; 126.7; 127.6; 127.7; 136.4; 173.6. |
| 165 | 2 | Bsp. Nr. 162 Stufe 3 / Alkylierung/ 26% | [M+H]+ = 355.4, Rt = 2.7 min. | 1H-NMR (CDCl3): 1.14-1.27 (2 H, m); 1.33-1.43 (2 H, m); 1.50-1.80 (9 H, m); 1.90-2.20 (2 H, m); 2.03 (6 H, s); 2.08-2.20 (4 H, m); 3.19 (2 H, d, J = 7.8 Hz); 3.26 (2 H, s); 7.27-7.31 (3 H, m); 7.35-7.41 (2 H, m). |
| | | | | 13C-NMR (CDCl3): 25.1; 30.2; 30.5; 32.8; 35.8; 38.0; 38.1; 44.5; 47.4; 58.3; 60.1; 126.7; 127.4; 127.7; 173.8. |
| 166 | 1 | Bsp. Nr. 431 Alkylierung/ 27% | [M+H]+ = 355.4, Rt = 3.1 min. | 1H-NMR (CDCl3): 1.10-1.20 (2 H, m); 1.30-1.40 (2 H, m); 1.42-1.75 (8 H, m); 1.84-2.20 (3 H, m); 2.02 (6 H, s); 2.10-2.30 (2 H, br s); 2.35 (2 H, s); 3.00 (2 H, s); 3.11 (2 H, d, J = 7.8 Hz); 7.24-7.31 (3 H, m); 7.34-7.40 (2 H, m). |
| | | | | 13C-NMR (CDCl3): 25.1; 30.0; 30.2; 30.3; 33.0; 35.9; 37.9; 38.0; 43.6; 47.3; 59.2; 60.4; 126.6; 127.5; 127.7; 173.7. |
| 167 | 2 | Bsp. Nr. 162 Stufe 3 / Alkylierung/ 58% | [M+H]+ = 369.4, Rt = 2.5 min. | 1H-NMR (CDCl3): 1.30-1.39 (2 H, m); 1.65-1.73 (2 H, m); 1.80-1.95 (2 H, m); 2.00 (6 H, s); 2.13-2.25 (2 H, m); 2.16 (2 H, s); 3.20 (2 H, s); 4.62 (2 H, s); 6.95-6.98 (2 H, m); 7.23-7.30 (4 H, m); 7.35-7.40 (2 H, m). |
| | | | | 13C-NMR (CDCl3): 30.2; 32.7; 35.7; 38.0; 41.0; 44.6; 56.9; 60.1; 125.5; 126.72; 126.74; 126.8; 127.4; 127.8; 139.0; 173.4. |
| 167 | 2 | Bsp. Nr. 162 Stufe 3 / Alkylierung/ 45% | [M+H]+ = 341.4, Rt = 2.5 min. | 1H-NMR (CDCl3): 0.03-0.09 (2 H, m); 0.43-0.49 (2 H, m); 0.59-0.70 (1 H, m); 1.33-1.45 (4 H, m); 1.69-1.78 (2 H, m); 1.85-2.01 (2 H, m); 2.03 (6 H, d, J = 1.3 Hz); 2.13 (2 H, s); 2.15-2.26 (2 H, m); 3.26 (2 H, s); 3.34 (2 H, t, J = 7.2 Hz); 7.27-7.31 (3 H, m); 7.35-7.41 (2 H, m). |
| | | | | 13C-NMR (CDCl3): 4.7; 8.9; 30.6; 32.8; 33.4; 36.0; 38.3; 43.0; 44.9; 58.6; 60.4; 127.0; 127.7; 128.0; 173.9. |
| 168 | 1 | Bsp. Nr. 431 / Alkylierung/ 50% | [M+H]+ = 341.4, Rt = 2.9 min. | 1H-NMR (CDCl3): 0.01-0.04 (2 H, m); 0.38-0.44 (2 H, m); 0.54-0.64 (1 H, m); 1.30-1.40 (4 H, m); 1.66-1.76 (2 H, m); 1.85-2.00 (2 H, m); 2.03 (6 H, s); 2.16-2.32 (2 H, m); ); 2.36 (2 H, s); 3.01 (2 H, s); 3.38 (2 H, t, J = 7.2 Hz); 7.24-7.32 (3 H, m); 7.34-7.40 (2 H, m). |
| | | | | 13C-NMR (CDCl3): 4.3; 8.6; 30.1; 32.4; 33.1; 35.8; 38.1; 42.6; 43.7; 59.2; 60.6; 126.6; 127.6; 127.7; 173.6. |
| 169 | 2 | Bsp. Nr. 162 Stufe 3 / Alkylierung/ 64% | [M+H]+ = 341.3, Rt = 2.5 min. | 1H-NMR (CDCl3): 1.32-1.41 (2 H, m); 1.65-1.79 (5 H, m); 1.85-1.95 (3 H, m); 2.03 (6 H, s); 2.03-2.09 (2 H, m); 2.12 (2 H, s); 2.12-2.27 (2 H, m); 2.53 (1 H, td, J = 15.6, 7.8 Hz); 3.20 (2 H, s); 3.29 (2 H, d, J = 7.6 Hz); 7.26-7.30 (3 H, m); 7.35-7.41 (2 H, m). |
| | | | | 13C-NMR (CDCl3): 18.4; 26.4; 30.3; 32.8; 34.0; 35.8; 38.0; 44.3; 47.9; 58.1; 60.1; 126.7; 127.4; 127.7; 173.7. |
| 170 | 1 | Bsp. Nr. 431 / Alkylierung/ 45% | [M+H]+ = 341.5, Rt = 3.0 min. | 1H-NMR (CDCl3): 1.30-1.40 (2 H, m); 1.60-1.73 (4 H, m); 1.80-2.02 (6 H, m); 2.03 (6 H, s); 2.15-2.33 (2 H, m); 2.35 (2 H, s); 2.45 (1 H, td, J = 15.6, 7.8 Hz); 2.95 (2 H, s); 3.24 (2 H, d, J = 7.6 Hz); 7.27-7.32 (3 H, m); 7.35-7.41 (2 H, m). |
| | | | | 13C-NMR (CDCl3): 18.3; 26.3; 30.1; 33.0; 33.8; 36.0; 38.1; 43.5; 47.8; 59.3; 60.5; 126.6; 127.6; 127.7; 173.7. |
| 171 | 2 | Bsp. Nr. 162 Stufe 3 / Alkylierung/ 56% | [M+H]+ = 327.4, Rt = 2.3 min. | 1H-NMR (CDCl3): 0.18-0.23 (2 H, m); 0.49-0.55 (2 H, m); 0.83-0.94 (1 H, m); 1.35-1.43 (2 H, m); 1.71-1.79 (2 H, m); 1.85-2.02 (2 H, m); 2.04 (6 H, s); 2.13 (2 H, s); 2.16-2.30 (2 H, m); 3.13 (2 H, d, J = 7.1 Hz); 3.35 (2 H, s); 7.26-7.31 (3 H, m); 7.36-7.41 (2 H, m). 13C-NMR (CDCl3): 3.4; 9.1; 30.3; 32.9; 35.8; 38.0; 44.6; 47.0; 57.9; 60.2; 126.7; 127.5; 127.7; 173.4. |
| 172 | 1 | Bsp. Nr. 431 / Alkylierung/ 58% | [M+H]+ = 327.4, Rt = 2.8 min. | 1H-NMR (CDCl3): 0.11-0.16 (2 H, m); 0.42-0.47 (2 H, m); 0.75-0.85 (1 H, m); 1.32-1.41 (2 H, m); 1.67-1.76 (2 H, m); 1.87-2.00 (2 H, m); 2.03 (6 H, s); 2.15-2.33 (2 H, m); 2.36 (2 H, s); 3.05-3.10 (4 H, m); 7.24-7.31 (3 H, m); 7.35-7.40 (2 H, m). |
| | | | | 13C-NMR (CDCl3): 3.3; 9.0; 30.1; 33.0; 35.9; 38.0; 43.5; 46.9; 59.0; 60.4; 126.6; 127.5; 127.7; 173.4. |
| 173 | 2 | Bsp. Nr. 162 Stufe 3 / Alkylierung/ 60% | [M+H]+ = 355.4, Rt = 2.7 min. | 1H-NMR (CDCl3): 1.33-1.42 (2 H, m); 1.56-1.68 (5 H, m); 1.68-2.02 (6 H, m); 2.03 (6 H, s); 2.04-2.10 (2 H, m); 2.12 (2 H, s); 2.13-2.30 (2 H, m); 3.14-3.20 (2 H, m); 3.22 (2 H, s); 7.26-7.31 (3 H, m); 7.35-7.41 (2 H, m). |
| | | | | 13C-NMR (CDCl3): 18.6; 28.2; 30.2; 32.9; 33.7; 34.3; 35.6; 38.0; 40.5; 44.6; 58.0; 60.1; 126.7; 127.4; 127.7; 173.5. |
| 174 | 1 | Bsp. Nr. 431 / Alkylierung/ 51% | [M+H]+ = 355.4, Rt = 3.2 min. | 1H-NMR (CDCl3): 1.31-1.40 (2 H, m); 1.47-1.53 (4 H, m); 1.66-1.85 (5 H, m); 1.88-2.02 (3 H, m); 2.03 (6 H, s); 2.14-2.30 (3 H, m); 2.35 (2 H, s); 2.97 (2 H, s); 3.08-3.14 (2 H, m); 7.26-7.32 (3 H, m); 7.35-7.41 (2 H, m). |
| | | | | 13C-NMR (CDCl3): 18.5; 28.2; 30.1; 33.1; 33.6; 34.2; 35.8; 38.1; 40.4; 43.7; 59.0; 60.5; 126.6; 127.6; 127.7; 173.5. |
| 175 | 2 | Bsp. Nr. 162 Stufe 3 / Alkylierung/ 47% | [MH-HNMe2]+ = 321.3 (20%) | 1H-NMR (CDCl3): 0.87 (2 H, dd, J = 5.1 Hz); 1.26 (2 H, dd, J = 5.0 Hz); 1.35-1.44 (2 H, m); 1.71 (2 H, t, J = 7.1 Hz); 1.74-1.82 (2 H, m); 2.03 (8 H, s); 2.10-2.21 (4 H, s); 3.35 (2 H, s); 3.48 (2 H, t, J = 7.1 Hz); 7.29 (3 H, d, J = 7.5 Hz); 7.35-7.41 (2 H, m). |
| | | | [M+H]+ = 366.3 (100 %), Rt = 2.3 min. | 13C-NMR (CDCl3): 7.6; 14.1; 30.1; 32.6; 32.9; 35.8; 38.0; 41.2; 44.2; 58.9; 60.1; 123.0; 126.7; 127.4; 127.7; 174.2. |
| 176 | 1 | Bsp. Nr. 431 / Alkylierung/ 54% | [MH-HNMe2]+ = 321.3 (25 %) | 1H-NMR (CDCl3): 0.84 (2 H, dd, J = 5.2 Hz); 1.22 (2 H, dd, J = 4.9 Hz); 1.34-1.42 (2 H, m); 1.65 (2 H, t, J = 7.1 Hz); 1.69-1.77 (2 H, m); 1.87-2.00 (2 H, m); 2.03 (6 H, s); 2.18-2.33 (2 H, m); 2.37 (2 H, s); 3.10 (2 H, s); 3.42 (2 H, t, J = 7.1 Hz); 7.25-7.31 (3 H, m); 7.35-7.40 (2 H, m). |
| | | | [M+H]+ = 366.3 (100 %), Rt = 2.6 min. | 13C-NMR (CDCl3): 7.5; 14.0; 30.1; 32.5; 33.1; 36.0; 38.0; 41.1; 43.4; 59.9; 60.5; 122.9; 126.7; 127.6; 127.8; 174.2. |
| 177 | 2 | Bsp. Nr. 162 Stufe 3 / Alkylierung/ 58% | [MH-HNMe2]+ = 335.3 (12 %) | 1H-NMR (CDCl3): 1.34-1.43 (2 H, m); 1.72-1.80 (2 H, m); 1.93-1.98 (2 H, m); 1.99-2.07 (3 H, m); 2.03 (6 H, s); 2.11-2.25 (7 H, m); 2.49-2.58 (2 H, m); 3.29 (2 H, s); 3.37-3.41 (2 H, m); 7.27-7.30 (3 H, m); 7.35-7.40 (2 H, m). |
| | | | [M+H]+ = 380.3 (100%), Rt = 2.3 min. | 13C-NMR (CDCl3): 16.8; 30.1; 32.1; 32.9; 33.8; 34.9; 35.8; 38.0; 39.0; 44.3; 58.3; 60.2; 124.1; 126.8; 127.4; 127.8; 174.0. |
| 178 | 1 | Bsp. Nr. 431 / Alkylierung/ 65% | [MH-HNMe2]+ = 335.3 (20 %) | 1H-NMR (CDCl3): 1.32-1.41 (2 H, m); 1.68-1.76 (2 H, m); 1.87-1.92 (2 H, m); 1.94-2.32 (8 H, m); 2.05 (6 H, s); 2.37 (2 H, s); 2.46-2.55 (2 H, m); 3.04 (2 H, s); 3.30-3.36 (2 H, m); 7.28-7.32 (3 H, m); 7.36-7.41 (2 H, m). |
| | | | [M+H]+ = 380.3 (100%), Rt = 2.6 min. | 13C-NMR (CDCl3): 16.8; 30.1; 32.1; 33.1; 33.8; 34.8; 36.0; 38.1; 39.0; 43.4; 59.3; 60.5; 124.1; 126.7; 127.6; 127.8; 174.1. |
| 179 | 2 | Bsp. Nr. 162 Stufe 3 / Alkylierung/ 83% | [M+H]+= 357.3 (100%) | 1H-NMR (CDCl3): 1.37-1.43 (2 H, m); 1.49-1.59 (1 H, m); 1.72-1.81 (4 H, m); 2.04 (6 H, s); 2.03-2.09 (5 H, m, überlagert); 2.18 (2 H, s); 2.12-2.22 (2 H, m, überlagert); 3.44 (2 H, s); 3.46 (2 H, s); 4.09 (1 H, s); 7.27-7.31 (3 H, m); 7.37-7.41 (2 H, m). |
| | | | [MH-NHMe2]+=312.3 (92%), Rt = 2.3 min. | 13C-NMR (CDCl3): 11.9; 30.1; 32.7; 34.5; 36.7; 38.0; 44.1; 51.7; 61.3; 75.6; 126.9; 127.4; 127.8; 176.3. |
| 180 | 1 | Bsp. Nr. 431 / Alkylierung/ 63% | [M+H]+= 357.3 (43%) | 1H-NMR (CDCl3): 1.35-1.43 (2 H, m); 1.45-1.50 (1 H, m); 1.67-1.77 (4 H, m); 2.04 (6 H, s); 2.00-2.08 (5 H, m, überlagert); 2.11-2.33 (2 H, m); 2.41 (2 H, s); 3.20 (2 H, s); 3.38 (2 H, s); 4.06 (1 H, br s); 7.28-7.30 (3 H, m); 7.36-7.40 (2 H, m). |
| | | | [MH-NHMe2]+=312.3 (100%), Rt = 2.5 min. | 13C-NMR (CDCl3): 11.9; 30.1; 32.9; 34.5; 36.8; 38.1; 43.2; 51.6; 62.3; 75.6; 126.7; 127.6; 127.8; 176.3. |
| 181 | 2 | Bsp. Nr. 21/ Alkylierung/ 78% | [M+H]+ = 345.4, Rt = 2.2 min. | 1H-NMR (CDCl3): 1.23 (6 H, s); 1.36-1.46 (2 H, m); 1.72-1.82 (2 H, m); 1.90-2.01 (2 H, m); 2.02 (6 H, s); 2.04-2.16 (2 H, m); 2.18 (2 H, s); 3.26 (2 H, s); 3.45 (2 H, s); 3.53 (1 H, br s); 7.26-7.31 (3 H, m); 7.35-7.41 (2 H, m). |
| | | | | 13C-NMR (CDCl3): 27.6; 29.9; 30.0; 32.7; 35.8; 36.3; 37.9; 43.9; 55.0; 60.0; 61.4; 71.9; 126.6; 127.3; 127.6; 136.3; 174.6; 175.7. |
| 182 | 2 | Bsp. Nr. 162 Stufe 3 / Alkylierung/ 55% | [MH-HNMe2]+ = 328.3 (72%) | 1H-NMR (CDCl3): 1.18 (6 H, s); 1.32-1.41 (2 H, m); 1.64-1.69 (2 H, m); 1.70-1.77 (2 H, m); 1.91-2.01 (2 H, m); 2.02 (6 H, s); 2.11 (2 H, s); 2.13-2.27 (2 H, m); 3.20 (3 H, s); 3.25 (2 H, s); 3.29-3.34 (2 H, m); 7.25-7.31 (3 H, m); 7.35-7.41 (2 H, m). |
| | | | [M+H]+ = 373.4 (100 %), Rt = 2.5 min. | 13C-NMR (CDCl3): 24.9; 30.2; 32.9; 35.7; 36.7; 38.0; 38.2; 44.6; 49.2; 58.0; 60.2; 73.6; 126.7; 127.4; 127.7; 173.4. |
| 183 | 1 | Bsp. Nr. 431 / Alkylierung/ 82% | [MH-HNMe2]+ = 328.3 (100 %) | 1H-NMR (CDCl3): 1.14 (6 H, s); 1.31-1.40 (2 H, m); 1.57-1.62 (2 H, m); 1.67-1.74 (2 H, m); 1.90-2.02 (2 H, m); 2.03 (6 H, s); 2.14-2.29 (2 H, m); 2.34 (2 H, s); 3.00 (2 H, s); 3.15 (3 H, s); 3.23-3.29 (2 H, m); 7.25-7.31 (3 H, m); 7.35-7.40 (2 H, m). |
| | | | [M+H]+ = 373.4 (75 %), Rt = 2.9 min. | 13C-NMR (CDCl3): 24.8; 30.1; 33.0; 35.8; 36.8; 38.1; 38.1; 43.7; 49.1; 59.1; 60.5; 73.5; 126.6; 127.6; 127.7; 173.5. |
| 184 | 2 | Bsp. Nr. 162 Stufe 3 / Alkylierung/ 47% | [M+H]+ = 385.4 | 1H-NMR (CDCl3) 1.32-1.44 (2 H, m); 1.52-1.66 (1 H, m); 1.69-1.80 (3 H, m); 1.82-1.93 (4 H, m); 1.94- 2.02 (2 H, m); 2.03 (6 H, s); 2.07-2.18 (5 H, m); 2.19-2.31 (1 H, m); 3.15 (3 H, s); 3.23-3.30 (4 H, m); 7.26-7.32 (3 H, m); 7.34-7.43 (2 H, m). |
| | | | [MH-HNMe2]+ = 340.3, Rt = 2.5 min. | 13C-NMR (CDCl3): 12.4; 30.2; 31.3; 31.8; 32.9; 35.6; 37.8; 38.0; 44.7; 49.3; 58.3; 60.2; 78.2; 126.7; 127.4; 127.7; 173.4. |
| 185 | 1 | Bsp. Nr. 431 / Alkylierung/ 54% | [M+H]+= 385.4 | 1 H-NMR (CDCl3) 1.32-1.41 (2 H, m); 1.54 (1 H, dq, J = 11.6 und 8.9 Hz); 1.66-1.75 (3 H, m); 1.76-1.89 (4 H, m); 1.91-2.01 (2 H, m); 2.03-2.13 (8 H, m); 2.16-2.30 (2 H, m); 2.35 (2 H, s); 3.02 (2 H, s); 3.11 (3 H, s); 3.21 (2 H, ddd, J = 15.9, 7.9 und 5.1 Hz); 7.25-7.32 (3 H, m); 7.35-7.40 (2 H, m). |
| | | | [MH-HNMe2]+ = 340.3, Rt = 2.9min. | 13C-NMR (CDCl3): 12.4; 30.1; 31.2; 31.9; 33.1; 35.8; 37.7; 38.1; 43.7; 49.3; 59.4; 60.5; 78.2; 126.6; 127.6; 127.7; 136.4; 173.5. |
| 188 | 1 | Bsp. Nr. 71/ Acylierung/ 57% | [M+H]+ = 371.4, Rt = 2.7 min. | 1 H-NMR (CDCl3): 1.24-1.34 (2 H, m); 1.50-1.68 (5 H, m); 1.78-1.98 (2 H, m); 2.01 und 2.03 (6 H, 2 s); 2.10-2.23 (2 H, m); 2.29-2.40 (3 H, m); 2.64-2.76 (1 H, m); 3.31 (1 H, s); 3.36-3.47 (4 H, m); 3.70-3.78 (1 H, m); 3.81-3.89 (1 H, m); 3.93-4.00 (1 H, m); 7.24-7.31 (3 H, m); 7.33-7.40 (2 H, m). |
| | | | | 13C-NMR (CDCl3): 30.0; 30.8; 31.1; 31.2; 31.4; 32.3; 35.3; 35.33; 35.8; 37.5; 38.0; 38.04; 38.06; 38.5; 40.3; 42.3; 43.9; 45.0; 55.2; 56.4; 60.7; 67.6; 73.29; 73.31; 126.5; 126.7; 127.5; 127.6; 127.65; 127.7; 135.6; 137.4; 170.39; 170.4. |
| 189 | 1 | Bsp. Nr. 71/ Acylierung/ 35% | [M+H]+ = 343.3, Rt = 2.5 min. | 1 H-NMR (CDCl3): 1.22-1.35 (2 H, m); 1.54-1.67 (4 H, m); 1.70-2.02 (2 H, m); 2.03 und 2.04 (6 H, 2 s); 2.05-2.36 (2 H, m); 3.09 (1.2 H, s); 3.20 (0.8 H, t, J = 7.2 Hz); 3.43 (0.8 H, s); 3.49 (1.2 H, t, J = 7.3 Hz); 3.87-3.98 (1 H, m); 4.73-4.83 (2 H, m); 4.91-4.96 (2 H, m); 7.26-7.34 (3 H, m); 7.34-7.42 (2 H, m). |
| | | | | 13C-NMR (CDCl3): 30.1; 30.8; 31.0; 31.4; 35.5; 38.0; 38.1; 38.3; 38.5; 40.4; 42.3; 44.16; 44.2; 55.4; 55.7; 60.7; 73.03; 73.04; 126.5; 126.8; 127.5; 127.6; 127.7; 127.8; 169.6; 169.7. |
| 190 | 1 | Bsp. Nr. 71/ Acylierung/ 66% | [M+H]+ = 359.4, Rt = 2.8 min. | 1H-NMR (CDCl13): 1.25-1.35 (2 H, m); 1.41 und 1.42 (6 H, 2 s); 1.49 (1 H, t, J = 7.4 Hz); 1.55-1.70 (3 H, m); 1.85-2.03 (2 H, m); 2.04 (6 H, s); 2.16-2.34 (2 H, m); 3.18 (1 H, s); 3.20 (2 H, s); 3.46 (0.6 H, s); 3.50 (1.4 H, t, J = 7.2 Hz); 3.66 (1.4 H, s); 3.71 (0.6 H, t, J = 7.0 Hz); 7.26-7.33 (3 H, m); 7.34-7.41 (2 H, m). |
| | | | | 13C-NMR (CDCl3): 24.1; 30.2; 30.6; 30.8; 31.7; 34.6; 38.0; 38.1; 38.8; 42.6; 45.3; 45.7; 51.5; 51.6; 56.6; 57.4; 60.8; 79.5; 79.8; 126.5; 126.6; 127.5; 127.6; 127.68; 127.7; 172.6; 172.9. |
| 191 | 1 | Bsp. Nr. 85/ Reduktion/ 100% | [M+H]+ = 327.4, Rt = 1.3 min. | 1 H-NMR (CDCl3): 0.01-0.06 (2 H, m); 0.38-0.44 (2 H, m); 0.66 (1 H, m); 1.22-1.32 (2 H, m); 1.34-1.48 (4 H, m); 1.60-1.70 (2 H, m); 1.76-1.94 (2 H, m); 2.02 (6 H, s); 2.26 (2 H, br s); 2.44-2.52 (6 H, m); 7.22-7.40 (5 H, m). |
| | | | | 13C-NMR (CDCl3): 4.3; 9.2; 31.0; 34.1; 34.6; 38.1; 38.3; 41.0; 53.9; 56.9; 60.4; 65.6; 70.6; 126.4; 127.4; 127.6; 136.9. |
| 192 | 1 | Bsp. Nr. 86/ Reduktion/ 88% | [M+H]+ = 341.4, Rt = 2.2 min. | 1 H-NMR (CDCl3): 1.22-1.33 (2 H, m); 1.44 (2 H, t, J = 6.8 Hz); 1.54-1.72 (6 H, m); 1.74-1.92 (4 H, m); 1.95-2.08 (2 H, m); 2.02 (6 H, s); 2.20-2.32 (5 H, m); 2.41-2.50 (4 H, m); 7.22-7.40 (5 H, m). |
| | | | | 13C-NMR (CDCl3): 18.7; 28.2; 31.1; 34.5; 34.7; 36.0; 38.1; 41.0; 53.8; 54.7; 65.5; 70.6; 126.4; 127.4; 127.6; 136.8. |
| 193 | 1 | Bsp. Nr. 84/ Reduktion/ 69% | [M+H]+= 343.4, Rt = 0.5 min. | 1H-NMR (DMSO-d6): 1.14-1.24 (2 H, m); 1.30 (1 H, t, J = 7.0 Hz); 1.36 (2 H, t, J = 6.9 Hz); 1.40-1.47 (1 H, m); 1.57-1.67 (4 H, m); 1.90 (6 H, s); 1.82-1.92 (2 H, m, überlagert); 1.94-2.03 (4 H, m); 2.448 (2 H, s); 2.453 (2 H, s); 2.53-2.57 (2 H, m); 4.58 (1 H, br s); 7.21-7.25 (1 H, m); 7.30-7.37 (4 H, m). |
| | | | | 13C-NMR (DMSO-d6): 12.0; 26.8; 30.3; 33.8; 33.8; 37.7; 40.7; 54.4; 63.2; 73.4; 126.1; 127.2; 127.3. |
| 194 | 1 | Bsp. Nr. 71/ Reduktive Aminierung/ 56% | [MH-HNMe2]+ = 321.3 (100 %) | 1 H-NMR (CDCl3): 1.24-1.32 (2 H, m); 1.46 (2 H, t, J = 6.9 Hz); 1.63-1.70 (2 H, m); 1.79-1.95 (4 H, m); 2.03 (6 H, s); 2.05-2.22 (4 H, m); 2.23-2.34 (2 H, m); 2.48-2.55 (8 H, m); 7.24-7.32 (3 H, m); 7.34-7.39 (2 H, m); |
| | | | [M+H]+ = 366.4 (80 %), Rt = 0.5 min. | 13C-NMR (CDCl3): 17.0; 31.1; 32.3; 34.5; 36.7; 38.1; 41.3; 50.8; 52.6; 53.4; 53.8; 60.6; 65.6; 124.5; 126.5; 127.6; 127.7. |
| 195 | 1 | Bsp. Nr. 71/ Acylierung/ 49% | [M+H]+ = 369.4, Rt = 3.2 min. | 1H-NMR (CDCl3): 1.24-1.35 (2 H, m); 1.51-1.68 (6 H, m); 1.69-1.76 (3 H, m); 1.78-1.99 (4 H, m); 2.03 und 2.05 (6 H, 2s); 2.06-2.17 (3 H, m); 2.18-2.40 (3 H, m); 3.32 (1 H, s); 3.32-3.49 (3 H, m); 7.26-7.33 (3 H, m); 7.34-7.42 (2 H, m). |
| | | | | 13C-NMR (CDCl3): 18.28; 18.3; 27.97; 28.0; 30.0; 30.9; 31.2; 31.5; 32.0; 32.1; 32.5; 35.71; 35.74; 36.0; 37.6; 38.0; 38.1; 40.3; 42.3; 43.9; 45.0; 55.2; 56.4; 60.8; 126.5; 126.7; 127.6; 127.63; 127.67; 127.8; 171.98; 172.0. |
| 196 | 1 | Bsp. Nr. 189/ Reduktion/ 23% | [M+H]+ = 329.4, Rt = 0.6 min. | 1H-NMR (CDCl3): 1.20-1.30 (2 H, m); 1.41 (2 H, t, J = 6.8 Hz); 1.58-1.70 (2 H, m); 1.79-1.99 (2 H, m); 2.01 (6 H, s); 2.10-2.30 (2 H, m); 2.39 (2 H, s); 2.43 (2 H, t, J = 6.8 Hz); 2.72 (1 H, s); 2.74 (1 H, s); 3.12-3.22 (1 H, m); 4.41 (2 H, dd, J = 6.2 und 6.2 Hz); 4.77 (2 H, dd, J = 7.8 und 6.0 Hz); 7.22-7.39 (5 H, m). |
| | | | | 13C-NMR (CDCl3): 31.0; 34.5; 34.7; 38.0; 41.2; 53.7; 59.9; 60.4; 65.7; 76.5; 126.4; 126.5; 127.5; 127.6; 127.7. |
| 197 | 1 | Bsp. Nr. 71/ Acylierung/ 76% | [M+H]+ = 355.4, Rt = 3.0 min. | 1H-NMR (CDCl3): 0.02-0.09 (2 H, m); 0.38-0.46 (2 H, m); 0.65-0.78 (1 H, m); 1.24-1.35 (2 H, m); 1.50-1.70 (6 H, m); 1.80-2.00 (2 H, m); 2.02 und 2.04 (6 H, 2 s); 2.16-2.26 (1 H, m); 2.28-2.40 (3 H, m); 3.32 (1 H, s); 3.40 (1 H, s); 3.45 (2 H, dt, J = 7.3 und 1.9 Hz); 7.25-7.32 (3 H, m); 7.34-7.41 (2 H, m). |
| | | | | 13C-NMR (CDCl3): 4.41; 4.45; 10.5; 10.71; 10.76; 28.5; 30.0; 30.1; 30.5; 30.9; 31.2; 31.4; 34.4; 34.8; 35.9; 37.6; 38.0; 38.1; 40.4; 42.3; 43.9; 45.0; 55.2; 56.4; 60.8; 126.5; 126.7; 127.6; 127.62; 127.66; 127.7; 137.4; 171.87; 171.92. |
| 198 | 1 | Bsp. Nr. 71/ Acylierung/ 92% | [M+H]+ = 352.3, Rt = 2.6 min. | 1H-NMR (CDCl3): 1.32 (1 H, dd, J = 10.6 und 3.1 Hz); 1.35 (1 H, dd, J = 10.6 und 3.1 Hz); 1.45-1.54 (2 H, m); 1.56-1.74 (6 H, m); 1.85-2.01 (2 H, m); 2.02 und 2.04 (6 H, 2 s); 2.16-2.35 (2 H, m); 3.41 (0.8 H, s); 3.48 (1.2 H, t, J = 7.3 Hz); 3.73 (1.2 H, s); 3.87 (0.8 H, t, J = 7.1 Hz); 7.26-7.32 (3 H, m); 7.35-7.42 (2 H, m). |
| | | | | 13C-NMR (CDCl3): 13.2; 13.7; 16.2; 16.6; 30.2; 30.7; 30.8; 31.2; 35.1; 37.6; 38.0; 38.04; 40.1; 42.7; 45.6; 46.0; 57.2; 57.4; 60.7; 120.0; 120.2; 126.6; 126.7; 127.4; 127.5; 127.71; 127.73; 136.0; 137.0; 162.8; 163.0. |
| 199 | 1 | Bsp. Nr. 71/ Acylierung/ 84% | [M+H]+ = 366.3, Rt = 2.7 min. | 1H-NMR (CDCl3): 1.28-1.37 (2 H, m); 1.56-1.74 (5 H, m); 1.88-2.00 (2 H, m); 2.028 und 2.03 (6 H, 2 s); 2.17-2.33 (3 H, m); 2.53-2.69 (2 H, m); 2.73-2.82 (2 H, m); 3.38 (1.2 H, s); 3.45 (0.8 H, s); 3.49-3.55 (2 H, m); 7.26-7.32 (3 H, m); 7.35-7.41 (2 H, m). |
| | | | | 13C-NMR (CDCl3): 16.47; 16.5; 30.3; 30.5; 30.56; 30.6; 30.7; 31.4; 34.9; 37.7; 38.0; 38.05; 38.8; 38.98; 40.0; 42.9; 44.9; 45.5; 56.4; 56.9; 60.7; 120.8; 120.9; 126.6; 126.7; 127.5; 127.6; 127.71; 127.75; 137.0; 164.4; 164.7. |
| 200 | 1 | Bsp. Nr. 71/ Reduktive Aminierung/ 51% | [MH-HNMe2]+ = 307.3 (100 %) | 1H-NMR (CDCl3): 0.79-0.83 (2 H, m); 1.20-1.23 (2 H, m); 1.24-1.32 (2 H, m); 1.45 (2 H, t, J = 6.9 Hz); 1.60-1.70 (4 H, m); 1.77-1.92 (2 H, m); 2.02 (6 H, s); 2.16-2.34 (2 H, m); 2.47 (2 H, s); 2.52 (2 H, t, J = 6.9 Hz); 2.62-2.67 (2 H, m); 7.23-7.32 (3 H, m); 7.34-7.39 (2 H, m). |
| | | | [M+H]+ = 352.4 (82 %), Rt = 0.8 min. | 13C-NMR (CDCl3): 8.2; 13.9; 31.1; 34.3; 34.5; 38.1; 41.3; 53.8; 54.5; 60.5; 65.5; 123.3; 126.4; 126.5; 127.7. |
| 201 | 1 | Bsp. Nr. 188/ Reduktion/ 20% | [M+H]+ = 357.4, Rt = 1.3 min. | 1H-NMR (CDCl3): 1.23-1.32 (2 H, m); 1.45 (2 H, t, J = 6.8 Hz); 1.49-1.62 (3 H, m); 1.62-1.70 (2 H, m); 1.80-1.93 (2 H, m); 2.03 (6 H, s); 2.03-2.09 (1 H, m); 2.15-2.31 (3 H, m); 2.34-2.45 (2 H, m); 2.45 (2 H, s); 2.48 (2 H, t, J = 6.8 Hz); 3.33 (1 H, t, J = 7.9 Hz); 3.74 (1 H, dd, J = 15.4 und 7.9 Hz); 3.84 (1 H, dt, J = 8.2 und 4.7 Hz); 3.89-3.95 (1 H, m); 7.23-7.40 (5 H, m). |
| | | | | 13C-NMR (CDCl3): 31.1; 32.4; 32.5; 34.6; 37.8; 38.0; 38.5; 41.08; 53.8; 55.8; 60.5; 65.6; 67.8; 73.4; 126.4; 127.5; 127.7; 136.6. |
| 202 | 1 | Bsp. Nr. 190/ Reduktion/ 10% | [M+H]+ = 345.4, Rt = 2.0 min. | 1H-NMR (CDCl3): 1.16 (6 H, s); 1.22-1.30 (2 H, m); 1.40 (2 H, t, J = 6.9 Hz); 1.63-1.70 (2 H, m); 1.80-1.95 (2 H, m); 2.03 (6 H, s); 2.15-2.30 (2 H, m); 2.43 (2 H, s); 2.57 (2 H, s); 2.60 (2 H, t, J = 6.9 Hz); 3.21 (3 H, s); 7.23-7.40 (5 H, m). |
| | | | | 13C-NMR (CDCl3): 23.6; 30.9; 31.0; 34.2; 38.1; 41.5; 49.3; 55.4; 60.7; 64.5; 67.3; 75.9; 126.4; 127.5; 127.8. |
| 203 | 1 | Bsp. Nr. 195/ Reduktion/ 67% | [M+H]+ = 355.4, Rt = 2.7 min. | 1H-NMR (CDCl3): 1.22-1.32 (2 H, m); 1.34-1.48 (6 H, m); 1.52-1.70 (4 H, m); 1.74-1.90 (4 H, m); 1.98-2.01 (1 H, m); 2.03 (6 H, s); 2.04-2.08 (1 H, m); 2.19-2.38 (5 H, m); 2.46 (2 H, s); 2.48 (2 H, t, J = 6.9 Hz); 7.23-7.40 (5 H, m). |
| | | | | 13C-NMR (CDCl3):18.4; 26.5; 28.3; 31.1; 34.7; 35.0; 36.1; 38.1; 38.4; 41.1; 53.9; 57.1; 60.5; 65.5; 126.4; 127.3; 127.7. |
| 204 | 1 | Bsp. Nr. 197/ Reduktion/ 72% | [M+H]+ = 341.4, Rt = 2.3 min. | 1H-NMR (CDCl3): -0.02-0.03 (2 H, m); 0.37-0.43 (2 H, m); 0.60-0.70 (1 H, m); 1.18-1.52 (4 H, m); 1.44 (2 H, t, J = 6.8 Hz); 1.54-1.70 (4 H, m); 1.75-1.95 (2 H, m); 2.03 (6 H, s); 2.15-2.35 (2 H, m); 2.37-2.43 (2 H, m); 2.45-2.51 (4 H, m); 7.24-7.40 (5 H, m). |
| | | | | 13C-NMR (CDCl3): 4.4; 10.8; 28.4; 28.9; 31.1; 32.8; 33.7; 34.7; 38.1; 38.3; 41.1; 53.9; 56.8; 56.9; 60.5; 65.6; 126.4; 127.5; 127.7. |
| 205 | 1 | Bsp. Nr. 71/ Acylierung/ 59% | [M+H]+= 343.3 (99 %) | 1H-NMR (DMSO-d6): 0.73-0.81 (2 H, m); 0.91-0.97 (2 H, m); 1.14-1.24 (2 H, m); 1.49 (1 H, t, J = 6.9 Hz); 1.54-1.64 (3 H, m); 1.92 (6 H, s); 1.93-2.13 (4 H, m, über-lagert); 3.20 (0.8 H, s); 3.26-3.30 (1.2 H, m, überlagert vom Wasser-Signal); 3.63 (1.2 H; s); 3.74-3.78 (0.8 H, m); 6.02 (0.3 H, s); 6.08 (0.7 H, s); 7.23-7.28 (1 H, m); 7.32-7.39 (4 H, m). |
| | | | [MH-NHMe2]+=298.3 (100 %),Rt = 2.6 min. | 13C-NMR (DMSO-d6): 13.9; 14.1; 29.1; 30.4; 30.7; 33.9; 36.6; 37.7; 41.6; 44.7; 45.2; 55.4; 56.9; 59.9; 126.2; 127.2; 127.4; 170.5. |
| 206 | 1 | Bsp. Nr. 205/ Reduktion/ 85% | [M+H]+= 329.3 (100%) | 1H-NMR (DMSO-d6): 0.37 (2 H, dd, J = 6.7 und 4.6 Hz); 0.54 (2 H, dd, J = 6.9 und 4.6 Hz); 1.16-1.23 (2 H, m); 1.38 (2 H, t, J = 6.9 Hz); 1.60-1.66 (2 H, m); 1.86-2.08 (4 H, m, überlagert); 1.91 (6 H, s), 2.41 (2 H, s); 2.44 (2 H, s); 2.51-2.53 (2 H, m, überlagert vom DMSO-Signal); 4.82 (1 H, s); 7.21-7.26 (1 H, m); 7.30-7.38 (4 H, m). |
| | | | [MH-NHMe2]+=284.3 (58 %), Rt = 0.3 min. | 13C-NMR (DMSO-d6): 12.1; 30.4; 34.0; 37.7; 40.6; 52.8; 53.6; 54.8; 59.5; 63.1; 126.1; 127.2; 127.4. |
| 207 | 1 | Bsp. Nr. 66/ Reduktion/ 73% | [M+1]+= 349.3 (43%) | 1H-NMR (DMSO-d6): 1.25-1.31 (2 H, m); 1.40-1.47 (4 H, m); 1.57-1.67 (4 H, m); 1.86-2.02 (6 H, m, überlagert); 1.99 (6 H, s); 2.42 (2 H, s); 2.44 (2 H, s); 2.56 (2 H, t, J = 6.9 Hz); 4.57 (1 H, br s); 6.91 (1 H, dd, J = 3.6 und 1.2 Hz); 7.04 (1 H, dd, J = 5.1 und 3.5 Hz); 7.40 (1 H, dd, J = 5.1 und 1.1 Hz). |
| | | | [MH-NHMe2]+=304.3 (100 %), Rt = 0.3 min. | 13C-NMR (DMSO-d6): 12.0; 26.8; 32.9; 33.6; 34.8; 37.7; 40.4; 54.5; 58.7; 63.1; 73.4; 123.4; 124.6; 126.2. |
| 208 | 1 | Bsp. Nr. 18/ Reduktive Aminierung/ 40% | [MH-HNMe2]+ = 327.3 (100 %) | 1H-NMR (CDCl3): 1.38 (2 H, ddd, J = 13.2, 9.8 und 3.5 Hz); 1.51 (2 H, t, J = 6.9 Hz); 1.65-1.73 (2 H, m); 1.86-1.97 (4 H, m); 1.98-2.21 (2 H, m); 2.10 (6 H, s); 2.45 (2 H, s); 2.47-2.56 (6 H, m); 6.85 (1 H, dd, J = 3.5 und 1.1 Hz); 7.02 (1 H, dd, J = 5.1 und 3.5 Hz); 7.22 (1 H, dd, J = 5.1 und 1.0 Hz). |
| | | | [M+H]+ = 372.3 (15 %), Rt = 0.4min. | 13C-NMR (CDCl3): 17.0; 32.3; 33.7; 34.3; 34.5; 36.7; 38.1; 41.0; 52.6; 53.9; 59.6; 65.7; 123.3; 124.4; 124.9; 126.1. |
| 209 | 1 | Bsp. Nr. 18/ Acylierung/ 49% | [M+H]+= 349.3 (3%) | 1H-NMR (DMSO-d6): 0.72-0.80 (2 H, m); 0.91-0.97 (2 H, m); 1.24-1.35 (2 H, m); 1.54-1.69 (4 H, m); 1.93-1.99 (4 H, m); 2.01 (6 H, s); 3.17 (0.8 H, s); 3.28-3.37 (1.2 H, m, überlagert vom Wasser-Signal); 3.61 (1.2 H; s); 3.75-3.82 (0.8 H, m); 6.06 (1 H, br s); 6.94 (1 H, dd, J = 3.6 und 1.1 Hz); 7.06 (1 H, dd, J = 5.1 und 3.5 Hz); 7.42 (1 H, dd, J = 5.1 und 1.1 Hz). |
| | | | [MH-NHMe2]+=304.2 (100%), Rt = 2.5 min. | 13C-NMR (DMSO-d6): 13.9; 14.1; 30.3; 30.4; 32.5; 33.5; 37.7; 41.4; 44.7; 45.3; 57.1; 59.1; 123.6; 124.8; 126.3; 170.5. |
| 210 | 1 | Bsp. Nr. 209/ Reduktion/ 69% | [M+H]+= 335.3 (40%) | 1H-NMR (DMSO-d6): 0.36 (2 H, dd, J = 6.7 und 4.6 Hz); 0.54 (2 H, dd, J = 6.9 und 4.6 Hz); 1.26-1.32 (2 H, m); 1.44 (2 H, t, J = 6.8 Hz); 1.62-1.68 (2 H, m); 1.83-1.97 (4 H, m); 1.99 (6 H, s), 2.41 (4 H, s); 2.51-2.55 (2 H, m, überlagert vom DMSO-Sig-nal); 4.82 (1 H, s); 6.91 (1 H, dd, J = 3.5 und 1.1 Hz); 7.05 (1 H, dd, J = 5.1 und 3.5 Hz); 7.40 (1 H, dd, J = 5.1 und 1.0 Hz). |
| | | | [MH-NHMe2]+=290.3 (100%), Rt = 0.2 min. | 13C-NMR (DMSO-d6): 12.6; 33.4; 34.2; 36.9; 38.2; 40.9; 53.2; 54.2; 59.2; 63.5; 123.9; 125.2; 126.7. |
| 211 | 1 | Bsp. Nr. 18/ Reduktive Aminierung/ 45% | [MH-HNMe2]+ = 313.3 (100 %) | 1H-NMR (CDCl3): 0.79-0.83 (2 H, m); 1.19-1.23 (2 H, m); 1.38 (2 H, ddd, J = 13.3, 9.9 und 3.5 Hz); 1.51 (2 H, t, J = 6.9 Hz); 1.60-1.65 (2 H, m); 1.66-1.73 (2 H, m); 1.84-1.97 (2 H, m); 2.04-2.18 (8 H, m); 2.43 (2 H, s); 2.59 (2 H, t, J = 6.9 Hz); 2.61-2.67 (2 H, m); 6.84 (1 H, dd, J = 3.6 und 1.1 Hz); 7.03 (1 H, dd, J = 5.1 und 3.6 Hz); 7.22 (1 H, dd, J = 5.1 und 1.1 Hz). |
| | | | [M+H]+ = 358.3 (10 %), Rt = 0.7min. | 13C-NMR (CDCl3): 8.2; 13.9; 33.7; 34.3; 37.8; 38.1; 41.0; 53.9; 54.4; 59.6; 65.7; 123.2; 123.3; 124.9; 126.1; 143.2. |
| 212 | 1 | Bsp. Nr. 18/ Acylierung/ 33% | [M+H]+ = 363.3, Rt = 2.3 min. | 1H-NMR (CDCl3): 1.33-1.46 (2 H, m); 1.59-1.80 (5 H, m); 1.83-2.07 (3 H, m); 2.08 und 2.12 (6 H, 2 s); 2.66 (2 H, dd, J = 7.8 und 2.5 Hz); 3.30 (1.2 H, s); 3.33 (0.8 H, s); 3.36-3.50 (3 H, m); 4.40 (0.8 H, dd, J = 6.3 und 2.5 Hz); 4.41 (1.2 H, dd, J = 6.3 und 2.5 Hz); 4.88 (1.2 H, dd, J = 6.3 und 1.4 Hz); 4.90 (0.8 H, dd, J = 6.3 und 1.4 Hz); 6.83-6.87 (1 H, m); 7.01-7.07 (1 H, m); 7.22-7.26 (1 H, m). |
| | | | | 13C-NMR (CDCl3): 19.8; 31.1; 31.2; 31.5; 31.6; 32.8; 33.0; 33.4; 35.4; 38.03; 38.07; 38.09; 38.2; 38.5; 40.1; 42.1; 43.9; 45.0; 55.4; 56.4; 59.8; 77.5; 77.54; 123.3; 123.6; 124.9; 125.1; 126.1; 126.4; 169.58; 169.64. |
| 215 | 2 | Bsp. Nr. 24b / Alkylierung/ 79% | [MH-NHMe2]+= 318.2 , Rt = 2.5 min. | 1H-NMR (CDCl3): 1.44-1.54 (4 H, m); 1.68-1.81 (4 H, m); 1.98-2.08 (6 H, m); 2.10 (6 H, s); 2.37 (2 H, s); 3.27 (2 H, s); 3.39 (2 H, s); 4.03 (1 H, br. s); ); 6.85 (1 H, dd, J = 3.6 und 1.0 Hz); 7.04 (1 H, dd, J = 5.1 und 3.6 Hz); 7.24 (1 H, dd, J = 5.1 und 1.0 Hz). |
| | | | | 13C-NMR (CDCl3): 11.9; 30.3; 32.7; 34.5; 36.5; 38.1; 43.3; 51.5; 59.5; 60.4; 75.6; 123.5; 125.0; 126.3; 176.2. |
| 216 | 1 | Bsp. Nr. 24a / Alkylierung/ 81% | [M+1]+= 363.3 (61 %) | 1H-NMR (CDCl3); 1.46-1.58 (4 H, m); 1.71-1.83 (4 H, m); 2.04-2.09 (6 H, m); 2.10 (6 H, s); 2.23 (2 H, s); 3.42 (2 H, s); 3.43 (2 H, s); 4.07 (1 H, br s); ); 6.85 (1 H, dd, J = 3.6 und 1.1 Hz); 7.05 (1 H, dd, J = 5.1 und 3.6 Hz); 7.25 (1 H, dd, J = 5.1 und 1.0 Hz). |
| | | | [MH-NHMe2]+ = 318.3 (100 %), Rt = 2.2 min. | 13C-NMR (CDCl3): 11.9; 32.5; 32.8; 34.5; 36.5; 38.1; 43.8; 51.6; 59.3; 75.6; 123.5; 124.9; 126.3; 176.2. |
| 217 | 1 | Bsp. Nr. 24a / Alkylierung/ 52% | [M+H]+ = 391.3, Rt = 2.5 min. | 1H-NMR (CDCl3): 1.47 (2 H, ddd, J = 13.1, 7.6 und 4.9 Hz); 1.54-1.64 (1 H, m); 1.72-1.80 (3 H, m); 1.83-1.93 (4 H, m); 1.99-2.13 (12 H, m); 2.17 (2 H, s); 3.15 (3 H, s); 3.22-3.30 (4 H, m); 6.85 (1 H, d, J = 2.9 Hz); 7.05 (1 H, dd, J = 5.1 und 3.6 Hz); 7.25 (1 H, d, J = 4.8 Hz). |
| | | | | 13C-NMR (CDCl3): 12.4; 31.2; 31.8; 32.7; 32.8; 35.4; 37.7; 38.0; 44.3; 49.3; 58.3; 59.3; 78.2; 123.4; 124.9; 126.3; 173.3. |
| 218 | 2 | Bsp. Nr. 24b / Alkylierung/ 47% | [M+H]+ = 391.4, | 1H-NMR (CDCl3): 1.43-1.51 (2 H, m); 1.52-1.62 (1 H, m); 1.68-1.90 (7 H, m); 1.98-2.14 (12 H, m); 2.31 (2 H, s); 3.09 (2 H, s); 3.12 (3 H, s); 3.23 (2 H, ddd, J = 16.0, 7.9 und 5.1 Hz); 6.85 (1 H, dd, J = 3.6 und 1.1 Hz); 7.04 (1 H, dd, J = 5.1 und 3.6 Hz); 7.24 (1 H, dd, J = 5.1 und 1.1 Hz). |
| | | | [M-HNMe2]+ =346.4, Rt = 2.8 min. | 13C-NMR (CDCl3): 12.4; 31.2; 31.9; 32.7; 32.9; 35.5; 37.8; 38.1; 43.8; 49.3; 59.1; 59.5; 78.2; 123.5; 125.0; 126.2; 173.4. |
| 219 | 2 | Bsp. Nr. 24b / Alkylierung/ 54% | [MH-HNMe2]+= 334.2 (100 %) | 1H-NMR (CDCl3):1.14 (6 H, s); 1.41-1.49 (2 H, m); 1.58-1.63 (2 H, m); 1.69-1.78 (2 H, m); 1.93-2.08 (4 H, m); 2.09 (6 H, s), 2.30 (2 H, s); 3.06 (2 H, s); 3.15 (3 H, s); 3.25-3.30 (2 H, m); 6.83-6.85 (1 H, m); 7.03 (1 H, dd, J = 5.1 und 3.5 Hz); 7.22-7.24 (1 H, m). |
| | | | [M+H]+ = 379.3 (2 %), Rt = 2.8 min. | 13C-NMR (CDCl3): 24.8; 32.7; 32.8; 35.5; 36.8; 38.1; 38.2; 43.7; 49.1; 58.7; 59.5; 73.5; 123.4; 124.9; 126.2; 142.9; 173.4. |
| 220 | 1 | Bsp. Nr. 24a / Alkylierung/ 70% | [MH-HNMe2]+ = 334.2 (100 %) | 1H-NMR (CDCl3): 1.17 (6 H, s); 1.41-1.49 (2 H, m); 1.63-1.68 (2 H, m); 1.71-1.79 (2 H, m); 1.93-2.08 (4 H, m); 2.09 (6 H, s); 2.16 (2 H); 3.19 (3 H, s); 3.21 (2 H, s); 3.28-3.33 (2 H, m); 6.84 (1 H, dd, J = 3.6 und 1.0 Hz); 7.04 (1 H, dd, J = 5.1 und 3.6 Hz); 7.23 (1 H, dd, J = 5.1 und 1.0 Hz). |
| | | | [M+H]+ = 379.3 (55 %), Rt = 2.5 min. | 13C-NMR (CDCl3): 24.9; 32.7; 32.8; 35.5; 36.7; 38.1; 38.2; 44.3; 49.2; 58.1; 59.3; 73.5; 123.4; 124.9; 126.3; 142.5; 173.4. |
| 221 | 1 | Bsp. Nr. 24a / Alkylierung/ 37% | [MH-HNMe2]+ = 320.3 (95%) | 1H-NMR (CDCl3): 1.23 (6 H, s); 1.38-1.47 (2 H, m); 1.62-1.67 (2 H, m); 1.73-1.81 (2 H, m); 2.05-2.20 (4 H, m); 2.15 (2 H, s, überlagert); 2.17 (6 H, s, überlagert); 2.52 (1 H, br s); 3.27 (2 H, s); 3.36-3.41 (2 H, m); 6.89 (1 H, dd, J = 3.5 und 0.9 Hz); 7.05 (1 H, dd, J = 5.1 und 3.6 Hz); 7.28 (1 H, dd, J = 5.1 und 0.8 Hz). |
| | | | [M+H]+ = 365.3 (100 %), Rt 2.3 min. | 13C-NMR (CDCl3): 29.5; 32.3; 32.6; 35.5; 37.9; 38.7; 40.0; 44.4; 57.8; 60.5; 69.5; 124.3; 125.7; 126.6; 173.8. |
| 222 | 2 | Bsp. Nr. 24b / Alkylierung/ 56% | [MH-HNMe2]+ = 320.2 (100 %) | 1H-NMR (CDCl3): 1.21 (6 H, s); 1.41-1.49 (2 H, m); 1.57-1.62 (2 H, m); 1.70-1.78 (2 H, m); 1.98-2.17 (4 H, m); 2.14 (6 H, s, überlagert); 2.32 (2 H, s); 2.44 (1 H, s); 3.07 (2 H, s); 3.32-3.37 (2 H, m); 6.86-6.89 (1 H, m); 7.04 (1 H, dd, J = 5.1 und 3.6 Hz); 7.24-7.27 (1 H, m). |
| | | | [M+H]+ = 365.3 (1 %), Rt = 2.5 min. | 13C-NMR (CDCl3): 29.5; 32.4; 32.8; 35.6; 38.0; 38.6; 40.0; 43.4; 58.9; 69.5; 124.0; 125.4; 126.4; 173.9. |
| 223 | 1 | Bsp. Nr. 18/ Acylierung/ 58% | m/z: [M+H]+= 351.3 (43%) | 1H-NMR (DMSO-d6): 1.28 (2 H, s); 1.29 (6 H, s); 1.51 (1 H, t, J = 7.4 Hz); 1.57-1.67 (3 H, m); 1.93-1.98 (4 H, m); 2.00 (6 H, s); 3.18 (0.7 H, s); 3.33 (1.3 H; t, J = 7.0 Hz, überlagert vom Wasser-Signal); |
| | | | [MH-NHMe2]+= 306.3 (100 %), Rt = 2.4 min. | 3.59 (1.3 H, s); 3.76 (0.7 H, t, J = 6.8 Hz); 5.07 (0.4 H, s); 5.14 (0.6 H, s); 6.94 (1 H, dd, J = 3.5 und 1.0 Hz); 7.06 (1 H, dd, J = 4.8 und 3.7 Hz); 7.42 (1 H, dd, J = 5.1 und 0.7 Hz). 13C-NMR (DMSO-d6): 27.8; 30.0; 30.6; 32.5; 32.9; 37.7; 38.0; 41.8; 45.5; 45.8; 57.4; 59.0; 59.1; 72.6; 72.8; 123.6; 124.8; 126.3; 142.9; 173.7. |
| 224 | 2 | Bsp. Nr. 24b/ Alkylierung/ 55% | [M+H]+ = 351.3, Rt = 2.2 min. | 1H-NMR (CDCl3): 1.22 (6 H, s); 1.45-1.54 (2 H, m); 1.77-1.85 (2 H, m); 2.00-2.10 (4 H, m); 2.11 (6 H, s); 2.23 (2 H, s); 3.25 (2 H, s); 3.33 (1 H, s); 3.41 (2 H, s); 6.85 (1 H, dd, J = 3.5 und 1.0 Hz); 7.05 (1 H, dd, J = 5.1 und 3.5 Hz); 7.25 (1 H, dd, J = 5.1 und 1.0 Hz). |
| | | | | 13C-NMR (CDCl3): 27.7; 32.6; 32.7; 36.3; 38.0; 43.8; 55.1; 59.4; 61.4; 72.1; 123.6; 124.9; 126.3; 175.8. |
| 225 | 1 | Bsp. Nr. 24a/ Alkylierung/ 71% | m/z: [MH-HNMe2]+ = 306.2, Rt = 2.3 min. | 1H-NMR (CDCl3): 1.19 (6 H, s); 1.45-1.54 (2 H, m); 1.74-1.83 (2 H, m); 1.96-2.09 (4 H, m); 2.10 (6 H, s); 2.37 (2 H, s); 3.21 (2 H, s); 3.25 (2 H, s); 3.26 (1 H, s); 6.85 (1 H, dd, J = 3.5 und 1.0 Hz); 7.02 (1 H, dd, J = 5.1 und 3.5 Hz); 7.24 (1 H, dd, J = 5.1 und 1.0 Hz). |
| | | | | 13C-NMR (CDCl3): 27.7; 32.6; 32.8; 36.3; 38.1; 43.2; 55.0; 59.6; 62.0; 72.1; 123.5; 125.0; 126.3; 175.8. |
| 226 | 1 | Bsp. Nr. 18/ Alkylierung/ Reduktion/ 2 Stufen 27% | m/z: [M+H]+ = 351.3, Rt = 0.3 min. | 1H-NMR (CDCl3): 1.21 (6 H, s); 1.30-1.40 (2 H, m); 1.48-1.52 (2 H, m); 1.55-1.60 (2 H, m); 1.60-1.70 (2 H, m); 1.80-2.05 (3 H, m); 2.08 (6 H, s); 2.10 (2 H, s); 2.49 (2 H, s); 2.56-2.61 (2 H, m); 2.70-2.74 (2 H, m); 6.83 (1 H, dd, J = 3.6 und 1.2 Hz); 7.02 (1 H, dd, J = 5.2 und 3.6 Hz); 7.21 (1 H, dd, J = 5.2 und 1.2 Hz). |
| | | | | 13C-NMR (CDCl3): 29.6; 33.6; 33.9; 37.4; 38.1; 38.3; 40.9; 52.7; 53.5; 59.6; 65.5; 71.0; 123.2; 124.8; 126.1. |
| 227 | 1 | Bsp. Nr. 223/ Reduktion/ 79% | m/z: [M+H]+= 337.3 (42 %) | 1H-NMR (DMSO-d6): 1.04 (6 H, s) 1.20-1.28 (2 H, m); 1.38 (2 H, t, J = 6.9 Hz); 1.57-1.63 (2 H, m); 1.79-1.92 (4 H, m); 1.95 (6 H, s); 2.25 (2 H, s); 2.41 (2 H, s); 2.54 (2 H, t, J = 7.0 Hz); 3.93 (1 H, s); 6.87 (1 H, dd, J = 3.6 und 1.1 Hz); 7.01 (1 H, dd, J = 5.1 und 3.5 Hz); 6.87 (1 H, dd, J = 5.1 und 1.1 Hz). |
| | | | [MH-NHMe2]+=292.2 (100 %), Rt = 0.3 min. | 13C-NMR (DMSO-d6): 28.2; 32.9; 33.4; 37.7; 40.6; 55.1; 58.8; 67.2; 69.8; 123.4; 124.6; 126.2. |
| 228 | 1 | Bsp. Nr. 71/ Acylierung/ 74% | m/z: [M+H]+= 345.4 (100 %) | 1H-NMR (DMSO-d6): 1.14-1.24 (2 H, m); 1.28 (2 H, s); 1.30 (4 H, s); 1.44 (1 H, t, J = 7.3 Hz); 1.54-1.64 (3 H, m); 1.91 (6 H, s); 1.95-2.15 (4 H, m); 3.21 (0.7 H, s); 3.30 (1.3 H, t, J = 7.3 Hz, überlagert vom Wasser-Signal); 3.63 (1.3 H; s); 3.74 (0.7 H, t, J = 7.0 Hz); 5.07 (0.3 H, s); 5.15 (0.7 H, s); 7.23-7.27 (1 H, m); 7.32-7.39 (4 H, m). |
| | | | [MH-NHMe2]+=303.3 (73 %), Rt= 2.5 min. | 13C-NMR (DMSO-d6): 27.7; 27.8; 29.9; 30.2; 30.8; 33.4; 37.1; 37.7; 38.1; 42.0; 45.4; 45.8; 57.3; 59.8; 59.9; 72.6; 72.8; 126.2; 127.2; 127.4, 136.8, 173.7. |
| 229 | 1 | Bsp. Nr. 228/ Reduktion/ 82% | m/z: [M+H]+= 331.3 (100 %) | 1H-NMR (DMSO-d6): 1.09 (6 H, s); 1.16-1.24 (2 H, m); 1.35 (2 H, t, J = 6.9 Hz); 1.58-1.65 (2 H, m); 1.90 (6 H, s); 1.92-2.02 (4 H, m); 2.30 (2 H, s); 2.48 (2 H, s); 2.56 (2 H, t, J = 6.9 Hz); 3.97 (1 H, s); 7.22-7.25 (1 H, m); 7.30-7.37 (4 H, m). |
| | | | [MH-NHMe2]+=236.3 Rt (36 %), = 0.3 min. | 13C-NMR (DMSO-d6): 28.2; 30.3; 33.7; 37.1; 37.7; 40.8; 55.1; 67.2; 67.7; 69.8; 126.1; 127.2; 127.3; 137.4. |
| 230 | 2 | Bsp. Nr. 162 Stufe 3/ Alkylierung/ 48% | m/z: [MH-HNMe2]+ = 323.3 (14 %) | 1H-NMR (CDCl13): 1.34-1.43 (8 H, m); 1.72-1.80 (4 H, m); 1.96-2.07 (8 H, m); 2.12-2.24 (4 H, m); 3.29 (2 H, s); 3.43-3.49 (2 H, m); 7.25-7.30 (3 H, m); 7.35-7.40 (2 H, m). |
| | | | [M+H]+ = 368.4 (100 %), Rt = 24 min. | |
| 231 | 1 | Bsp. Nr. 431/ Alkylierung/ 60% | [MH-HNMe2]+ = 323.3 (20 %) | 1H-NMR (CDCl3): 1.33-1.41 (8 H, m); 1.65-1.76 (4 H, m); 1.88-2.01 (2 H, m); 2.04 (6 H, s); 2.15-2.32 (2 H, m); 2.37 (2 H, s); 3.04 (2 H, s); 3.37-3.42 (2 H, m); 7.25-7.31 (3 H, m); 7.35-7.40 (2 H, m). |
| | | | [M+H]+ = 368.4 (100 %), Rt = 2.7 min. | 13C-NMR (CDCl3): 26.6; 30.0; 30.5; 33.0; 35.9; 37.4; 38.0; 38.9; 43.3; 59.0; 60.6; 124.4; 126.7; 127.6; 127.8; 136.2; 173.9. |
| 232 | 1 | Bsp. Nr. 71/ Acylierung/ 54% | m/z: [MH-HNMe2]+ = 323.3 (10 %) | 1H-NMR (CDCl3): 1.27-1.36 (2 H, m); 1.52 (6 H, s); 1.54-1.59 (1.2 H, m); 1.62-1.70 (2.8 H, m); 1.82-2.00 (2 H, m); 2.03 (2.4 H, s); 2.05 (3.6 H, s); 2.14-2.24 (0.8 H, m); 2.31-2.43 (1.2 H, m); 2.48 (0.8 H, s); 2.49 (1.2 H, s); 3.36 (1.2 H, s); 3.43 (0.8 H, s); 3.46 (0.8 H, t, J = 7.2 Hz); 3.50 (1.2 H, t, J = 7.3 Hz); 7.27-7.32 (3 H, m); 7.34-7.42 (2 H, m). |
| | | | [M+H]+ = 368.4 (100%), Rt = 2.7 min. | 13C-NMR (CDCl3): 27.05; 27.11; 30.0; 30.3; 30.4; 30.8; 31.2; 31.3; 35.9; 37.5; 37.95; 38.03; 40.3; 42.5; 43.7; 44.1; 45.4; 55.4; 56.4; 60.8; 61.0; 124.6; 124.7; 126.5; 126.8; 127.58; 127.59; 127.7; 127.8; 135.1; 137.4; 166.9; 167.0. |
| 233 | 1 | Bsp. Nr. 18/ Acylierung/ 39% | m/z: [MH-HNMe2]+ = 329.3 (63 %) | 1H-NMR (CDCl3): 1.34-1.46 (2 H, m); 1.51 (6 H, s); 1.60-1.64 (1.3 H, m); 1.65-1.75 (2.7 H, m); 1.86-2.06 (2.7 H, m); 2.09 (2.4 H, s); 2.12 (3.6 H, s); 2.17-2.26 (1.3 H, m); 2.48 (2 H, s); 3.32 (1.2 H, s); 3.39 (0.8 H, s); 3.45-3.54 (2 H, m); 6.84 (0.4 H, dd, J = 3.6 und 1.1 Hz); 6.86 (0.6 H, dd, J = 3.6 und 1.0 Hz); 7.03 (0.4 H, dd, J = 5.1 und 3.6 Hz); 7.05 (0.6 H, dd, J = 5.1 und 3.6 Hz); 7.23 (0.4 H, dd, J = 5.1 und 1.1 Hz); 7.24-7.26 (0.6 H, m). |
| | | | [M+H]+ = 374.3 (100 %), Rt = 2.6 min. | 13C-NMR (CDCl3): 27.05; 27.11; 30.65; 30.44; 31.09; 31.11; 32.8; 33.5; 35.5; 36.9; 38.1; 40.1; 42.3; 43.7; 44.0; 44.08; 44.11; 45.5; 55.7; 56.6; 59.8; 67.7; 123.3; 123.6; 124.6; 124.7; 124.8; 125.2; 126.1; 126.4; 166.8; 166.9. |
| 235 | 1 | Bsp. Nr. 18/ Acylierung/ 48% | m/z: [M+H]+= 391.3 (42%) | 1H-NMR (CDCl3):1.35-1.44 (2 H, m); 1.58-1.63 (2 H, m), 1.66-1.76 (4 H, m); 1.81-2.05 (4 H, m); 2.04 (2 H, s); 2.11 (4 H, s); 2.14-2.22 (2 H, m); 2.23-2.34 (2 H, m); 2.58 (0.7 H, s); 2.60 (1.3 H, s); 3.23 (1 H, s); 3.24 (2 H, s); 3.37 (0.7 H, s); 3.41 (1.3 H, s), 3.48 (1.3 H, t, J = 7.2 Hz); 3.54 (0.7 H, t, J = 7.2 Hz); 6.84-6.86 (1 H, m); 7.04 (1 H, ddd, J = 9.7, 5.1 und 3.6 Hz); 7.22-7.25 (1 H, m). |
| | | | [MH-NHMe2]+=346.3 (100 %),Rt = 2.9 min. | 13C-NMR (CDCl3): 12.5; 12.6; 30.5; 30.7; 31.1; 31.2; 32.8; 33.4; 35.8; 37.1; 38.1; 40.1; 40.4; 41.0; 42.0; 43.9; 45.7; 50.19; 50.22; 55.5; 56.9; 59.9; 76.9; 77.2; 77.5; 79.48; 79.50; 123.3; 123.5; 124.8; 125.1; 126.1; 126.3; 169.5; 169.6. |
| 236 | 2 | Bsp. Nr. 234 . Stufe Alkylierung/ 77% | [M+H]+: m/z = 347.4, Rt = 2.9 min. | 1H-NMR (CDCl3): 1.12-1.36 (4 H, m); 1.38-1.92 (17 H, m); 1.98-2.12 (3 H, m); 2.18-2.30 (2 H, m); 2.25 (8 H, s); 3.06 (2 H, s); 3.15 (2 H, t, J = 7.3 Hz). |
| | | | | 13C-NMR (CDCl3): 18.6; 25.1; 26.9; 28.1; 28.5; 31.8; 33.7; 34.3; 36.3; 37.9; 40.3; 42.4; 44.2; 57.7; 60.8; 173.6. |
| 237 | 1 | Bsp. Nr. 71/ Acylierung/ 53% | m/z: [M+H]+= 385.4 (83%) | 1H-NMR (CDCl3): 1.25-1.33 (2 H, m); 1.51-1.55 (1 H, m); 1.58-1.66 (3 H, m); 1.68-1.79 (1 H, m); 1.81-1.88 (2.6 H, m); 1.91-1.99 (1.4 H, m); 2.03 (2 H, s); 2.04 (4 H, s); 2.11-2.19 (2 H, m); 2.23-2.34 (2 H, m); 2.35-2.42 (1 H, m); 2.58 (0.7 H, s); 2.60 (1.3 H, s); 3.23 (1.2 H, s); 3.25 (1.8 H, s); 3.41 (0.7 H, s); 3.45 (1.3 H, s); 3.46 (1.3 H, t, J = 7.2 H); 3.51 (0.7 H, t, J = 7.2 Hz); 7.27-7.32 (3 H, m); 7.35-7.40 (2 H, m). |
| | | | [MH-NHMe2]+=340.3 (100 %), Rt = 3.0 min. | 13C-NMR (CDCl3): 12.5; 12.6; 30.0; 30.5; 30.7; 30.8; 31.2; 31.4; 36.1; 37.7; 38.0; 38.1; 40.3; 40.4, 41.0; 42.3; 43.9; 45.6; 50.19; 50.22; 55.2; 56.9; 60.8; 76.8; 77.2; 77.5; 79.48; 79.50; 126.5; 126.7; 127.6; 127.66; 127.75; 137.7; 169.5; 169.6. |
| 238 | 1 | Bsp. Nr. 235/ Reduktion/ 79% | m/z: [M+H]+= 377.4 (100 %) | 1H-NMR (CDCl3): 1.39 (2 H, ddd, J = 13.4, 10.2 und 3.4 Hz); 1.53 (2 H, t, J = 6.8 Hz); 1.58-1.63 (1 H, m); 1.65-1.89 (4 H, m); 1.83-1.94 (5 H, m); 2.03-2.09 (2 H, m); 2.10 (6 H, s); 2.11-2.19 (2 H, m); 2.39-2.43 (2 H, m); 2.46 (2 H, s); 2.54 (2 H, t, J = 6.8 Hz); 3.12 (3 H, s); 6.85 (1 H, dd, J = 3.6 und 1.1 Hz); 7.04 (1 H, dd, J = 5.1 und 3.6 Hz); 7.23 (1 H, dd, J = 5.1 und 1.1 Hz). |
| | | | [MH-NHMe2]+=332.3 (38 %), Rt = 0.5 min. | 13C-NMR (CDCl3): 12.5; 28.4; 31.7; 33.3; 33.8; 34.6; 38.2; 40.9; 49.2; 51.3; 54.3; 59.7; 77.5; 78.6; 123.2; 125.0; 126.2. |
| 239 | 1 | Bsp. Nr. 237/ Reduktion/ 78% | m/z: [M+H]+= 371.4 (100 %) | 1H-NMR (CDCl3): 1.26-1.32 (2 H, m); 1.47 (2 H, t, J = 6.8 Hz); 1.53-1.63 (1 H, m); 1.64-1.71 (2 H, m); 1.72-1.80 (2 H, m); 1.84-1.93 (5 H, m); 2.03 (6 H, s); 2.04-2.11 (2 H, m); 2.22-2.36 (2 H, m); 2.40-2.44 (2 H, m); 2.49-2.55 (4 H, m); 3.13 (3 H, s); 7.25-7.39 (5 H, m). |
| | | | [MH-NHMe2]+=326.3 (22 %), Rt= 2.0 min. | 13C-NMR (CDCl3): 12.5; 31.2; 31.7; 33.3; 34.7; 38.1; 38.4; 41.2; 49.2; 51.3; 54.2; 60.6; 65.8; 76.8; 77.1; 77.2; 77.4; 78.6; 126.4; 127.6; 127.8. |
| 240 | 1 | Bsp. Nr. 24a/ Alkylierung/ 93% | m/z: [MH-HNMe2]+ = 329.3 (100 %) = 374.3 | 1H-NMR (CDCl3): 1.37 (6 H, s); 1.43-1.51 (2 H, m); 1.67-1.72 (2 H, m); 1.73-1.80 (2 H, m); 1.98-2.18 (4 H, m); 2.14 (6 H, s); 2.34 (2 H, s); 3.10 (2 H, s); 3.39-3.44 (2 H, m); 6.86-6.89 (1 H, m); 7.05 (1 H, dd, J = 5.1 und 3.6 Hz); 7.25-7.28 (1 H, m). |
| | | | [M+H]+ (98 %), Rt = 2.6 min. | 13C-NMR (CDCl3): 26.6; 30.5; 32.5; 32.9; 35.6; 37.4; 37.9; 38.1; 38.9; 43.3; 58.7; 123.9; 124.4; 125.4; 126.4; 173.8. |
| 241 | 2 | Bsp. Nr. 24b/ Alkylierung/ 55% | m/z: [M+H]+ = 363.3, Rt = 2.2 min. | 1H-NMR (CDCl3): 1.42-1.50 (2 H, m); 1.72-1.82 (2 H, m); 1.90 (2 H, dd, J = 14.4 und 7.5 Hz); 1.97-2.09 (4 H, m); 2.10 (6 H, s); 2.18 (2 H, s); 2.90-3.01 (1 H, m); 3.17-3.23 (4 H, m); 4.38 (2 H, t, J = 6.1 Hz); 4.79 (2 H, dd, J = 7.8 und 6.0 Hz); 6.85 (1 H, d, J = 3.2 Hz); 7.04 (1 H, dd, J = 5.1 und 3.6 Hz); 7.24 (1 H, d, J = 5.0 Hz). |
| | | | | 13C-NMR (CDCl3): 31.2; 32.7; 33.0; 35.5; 38.0; 40.2; 44.1; 58.2; 59.2; 77.3; 123.5; 124.9; 126.3; 174.0. |
| 242 | 1 | Bsp. Nr. 24a/ Alkylierung/ 66% | m/z: [M+H]+ = 363.3, Rt = 2.4 min. | 1H-NMR (CDCl3): 1.42-1.50 (2 H, m); 1.70-1.80 (2 H, m); 1.85 (2 H, dd, J = 14.4 und 7.5 Hz); 1.94-2.10 (4 H, m); 2.11 (6 H, s); 2.32 (2 H, s); 2.90-2.99 (1 H, m); 3.04 (2 H, s); 3.14-3.20 (2 H, m); 4.36 (2 H, t, J = 6.1 Hz); 4.77 (2 H, dd, J = 7.8 und 6.0 Hz); 6.86 (1 H, dd, J = 3.5 und 0.7 Hz); 7.05 (1 H, dd, J = 5.1 und 3.5 Hz); 7.25 (1 H, dd, J = 5.0 und 0.9 Hz). |
| | | | | 13C-NMR (CDCl3): 31.2; 32.7; 32.9; 33.0; 35.6; 38.1; 38.1; 40.1; 43.5; 58.8; 77.2; 123.6; 125.1; 126.3; 173.6. |
| 243 | 1 | Bsp. Nr. 234 Stufe 10/ Alkylierung/ 86% | [M+H]+: m/z = 333.4, Rt = 2.9 min. | 1H-NMR (CDCl3): 0.03-0.09 (2 H, m); 0.40-0.48 (2 H, m); 0.64 (1 H, m); 1.10-1.85 (18 H, m); 2.07 (1 H, m); 2.21 (2 H, s); 2.26 (6 H, s); 3.19 (2 H, s); 3.33 (2 H, t, J = 7.3 Hz). |
| | | | | 13C-NMR (CDCl3): 4.2; 8.5; 25.1; 26.9; 28.4; 31.8; 32.3; 35.8; 37.8; 41.0; 42.6; 44.0; 46.9; 56.8; 57.2; 173.8. |
| 244 | 2 | Bsp. Nr. 234 Stufe 9/ Alkylierung/ 89% | [M+H]+: m/z = 335.4, Rt = 2.1 min. | 1H-NMR (CDCl3): 1.14-1.34 (6 H, m); 1.36-1.62 (6 H, m); 1.62-1.76 (4 H, m); 2.04 (1 H, m); 2.25 (6 H, s); 2.26 (2 H, s); 3.03 (2 H, s); 3.20 (1 H, m); 3.57 (2 H, d, J = 7.3 Hz); 4.45 (2 H, t, J = 6.1 Hz); 4.76 (2 H, dd, J = 6.1 und 7.7 Hz). |
| | | | | 13C-NMR (CDCl3): 25.0; 26.7; 28.5; 31.6; 34.0; 36.7; 37.9; 41.9; 44.1; 45.3; 57.5; 61.4; 75.4; 174.2. |
| 245 | 2 | Bsp. Nr. 234 Stufe 9/ Alkylierung/ 59% | M+H]+: m/z = 358.4, Rt = 2.4 min. | 1H-NMR (CDCl3): 0.85 (2 H, m); 1.14-1.36 (8 H, m); 1.38-1.62 (6 H, m); 1.62-1.80 (6 H, m); 2.04 (1 H, m); 2.26 (6 H, s); 2.28 (2 H, s); 3.18 (2 H, s); 3.45 (2 H, t, J = 7.0 Hz). |
| | | | | 13C-NMR (CDCl3): 7.5; 14.0; 25.0; 26.9; 28.3; 31.6; 32.5; 36.3; 37.7; 40.9; 42.0; 44.0; 57.6; 61.8; 122.8; 174.4. |
| 246 | 2 | Bsp. Nr. 234 Stufe 9/ Alkylierung/ 87% | [M+H]+: m/z = 349.4, Rt = 2.2 min. | 1H-NMR (CDCl3): 1.16-1.36 (6 H, m); 1.38-1.62 (6 H, m); 1.62-1.82 (4 H, m); 1.87 (2 H, q, J = 7.3 Hz); 2.05 (1 H, m); 2.22-2.30 (2 H, m); 2.26 (6 H, s); 2.96 (1 H, m); 3.06 (2 H, s); 3.18 (2 H, t, J = 7.1 Hz); 4.37 (2 H, t, J = 6.1 Hz); 4.77 (2 H, dd, J = 6.2 und 7.6 Hz). |
| | | | | 13C-NMR (CDCl3): 25.0; 26.7; 28.5; 31.1; 31.8; 32.9; 36.2; 37.9; 40.0; 41.0; 42.2; 44.0; 57,5; 60.8; 77.3; 173.9. |
| 247 | 3 | Bsp. Nr. 424/ Acylierung/ 72% | m/z: [M+H]+= 377.4 (100 %) | 1H-NMR (CDCl3): 1.17-1.37 (6 H, m); 1.41-1.60 (6 H, m); 1.63-2.21 (7 H, m); 2.02-2.21 (5 H, m); 2.24-2.30 (1 H, m, überlagert); 2.26 (6 H, s); 2.570 (1 H, s), 2.575 (1 H, s); 3.221 (1.8 H, s); 3.224 (1.2 H, s); 3.23 (0.8 H, s); 3.26 (1.2 H, s); 3.52 (0.8 H, t, J = 7.3 Hz); 3.56 (1.2 H, t, J = 7.3 Hz). |
| | | | [MH-NHMe2]+=332.4 (56 %), Rt = 2.9 min. | 13C-NMR (CDCl3): 12.6; 25.1; 25.2; 27.0; 27.1; 28.49; 28.52; 29.78; 29.82; 30.8; 32.1; 33.6; 37.9; 40.3; 40.57; 40.60; 40.61, 42.6; 44.2; 44.3; 44.5; 46.1, 50.21; 50.24; 57.9; 58.8; 61.0; 76.8; 77.16; 77.21; 79.46; 79.52; 169.3; 169.4. |
| 248 | 1 | Bsp. Nr. 18/ reduktive Aminierung/ 33% | m/z: [MH-HNMe2]+ = 315.2 (100 %) | 1 H-NMR: 1.34-1.42 (2 H, m); 1.37 (6 H, s); 1.51 (2 H, t, J = 6.9 Hz); 1.66-1.74 (4 H, m); 1.82-1.97 (2 H, m); 2.06-2.16 (2 H, m); 2.10 (6 H, s); 2.45 (2 H, s); 2.54 (2 H, t, J = 6.9 Hz); 2.56-2.61 (2 H, m); 6.84-6.86 (1 H, m); 7.03 (0.4 H, dd, J = 3.6 und 0.4 Hz); 7.04 (0.6 H, dd, J = 3.6 und 0.4 Hz); 7.21-7.24 (1 H, m). |
| | | | [M+H]+ = 360.3 (17 %), Rt = 0.6 min. | 13C-NMR (CDCl3): 27.0; 31.2; 33.7; 34.3; 38.1; 39.4; 40.9; 52.7; 53.9; 59.6; 65.6; 123.2; 124.8; 124.9; 126.2. |
| 249 | 1 | Bsp. Nr. 71/ Reduktive Aminierung/ 54% | m/z: [MH-HNMe2]+ = 309.3 (70 %) | 1H-NMR (CDCl3): 1.24-1.32 (2 H, m); 1.37 (6 H, s); 1.46 (2 H, t, J = 6.8 Hz); 1.63-1.70 (2 H, m); 1.70-1.75 (2 H, m); 1.79-1.95 (2 H, m); 2.03 (6 H, s); 2.20-2.35 (2 H, m); 2.49 (2 H, s); 2.53 (2 H, t, J = 6.8 Hz); 2.57-2.62 (2 H, m); 7.24-7.34 (3 H, m); 7.35-7.40 (2 H, m). |
| | | | [M+H]+ = 354.4 (100 %), Rt = 0.5 min. | 13C-NMR (CDCl3): 27.0; 31.1; 31.2; 34.5; 38.1; 39.5; 41.2; 52.8; 53.8; 60.5; 65.6; 124.8; 126.5; 127.6; 127.7. |
| 250 | 2 | Bsp. Nr. 24b/ Alkylierung/ 64% | m/z: [MH-HNMe2]+ = 329.2 (67 %) | 1H-NMR (CDCl3): 1.40 (6 H, s); 1.42-1.50 (2 H, m); 1.72-1.83 (4 H, m); 2.00-2.11 (4 H, m); 2.12 (6 H, s); 2.19 (2 H, s); 3.27 (2 H, s); 3.43-3.48 (2 H, m); 6.85-6.87 (1 H, m); 7.05 (1 H, dd, J = 5.1 und 3.6 Hz); 7.24-7.26 (1 H, m). |
| | | | [M+H]+ = 374.3 (100 %), Rt = 2.3 min. | 13C-NMR (CDCl3): 26.6; 30.5; 32.7; 35.6; 37.5; 38.0; 39.0; 44.0; 58.0; 59.6; 123.6; 124.4; 125.0; 126.4; 173.9. |
| 251 | 2 | Bsp. Nr. 162 Stufe3/ Alkylierung/ 49% | m/z: [M+H]+ = 343.3, Rt = 2.1 min. | 1H-NMR (CDCl3): 1.33-1.41 (2 H, m); 1.68-1.77 (2 H, m); 1.90-2.02 (2 H, m); 2.03 (6 H, s); 2.12 (2 H, s); 2.13-2.22 (2 H, m); 3.19 (2 H, s); 3.20-3.26 (1 H, m); 3.59 (2 H, d, J = 7.2 Hz); 4.46 (2 H, t, J = 6.2 Hz); 4.78 (2 H, dd, J = 7.8 und 6.2 Hz); 7.25-7.31 (3 H, m); 7.35-7.41 (2 H, m). |
| | | | | 13C-NMR (CDCl3): 30.2; 32.7; 34.0; 36.0; 37.9; 44.0; 45.4; 58.7; 60.1; 75.4; 126.8; 127.4; 127.8; 174.0. |
| 252 | 1 | Bsp. Nr. 431/ Alkylierung/ 55% | m/z: [M+H]+ = 343.3, Rt = 2.3 min. | 1H-NMR (CDCl3): 1.29-1.38 (2 H, m); 1.65-1.73 (2 H, m); 1.85-2.00 (2 H, m); 2.03 (6 H, s); 2.15-2.31 (2 H, m); 2.35 (2 H, s); 2.94 (2 H, s); 3.10-3.19 (1 H, m); 3.53 (2 H, d, J = 7.2 Hz); 4.40 (2 H, t, J = 6.2 Hz); 4.72 (2 H, dd, J = 7.8 und 6.2 Hz); 7.25-7.31 (3 H, m); 7.35-7.41 (2 H, m). |
| | | | | 13C-NMR (CDCl3): 30.0; 32.9; 34.0; 36.2; 38.0; 43.1; 45.3; 59.6; 60.6; 75.3; 126.7; 127.5; 127.8; 136.1; 174.0. |
| 253 | 1 | Bsp. Nr. 18/ Alkylierung/ 2Stufen 19% | [M+H]+ = 349.3, Rt = 0.5 min. | 1H-NMR (CDCl3): 0.35-0.40 (2 H, m); 0.70-0.74 (2 H, m); 1.32-1.41 (2 H, m); 1.53 (2 H, t, J = 7.0 Hz); 1.62-1.74 (4 H, m); 1.90-2.09 (5 H, m); 2.09 (6 H, s); 2.54 (2 H, s); 2.64 (2 H, t, J = 6.8 Hz); 2.79 (2 H, t, J = 5.5 Hz); 6.84 (1 H, dd, J = 3.5 und 0.8 Hz); 7.03 (1 H, dd, J = 5.1 und 3.5 Hz); 7.22 (1 H, dd, J = 5.0 und 0.8 Hz). |
| | | | | 13C-NMR (CDCl3): 12.8; 33.5; 34.0; 34.3; 37.5; 38.1; 41.0; 53.6; 55.5; 57.7; 59.6; 65.6; 123.2; 124.9; 126.2. |
| 254 | 2 | Bsp. Nr. 162 Stufe3/ Alkylierung/ 57% | m/z: [M+H]+ = 357.3, Rt = 2.2 min. | 1H-NMR (CDCl3): 1.34-1.43 (2 H, m); 1.70-1.79 (2 H, m); 1.91 (2 H, dd, J = 14.4 und 7.4 Hz); 2.05 (8 H, br s); 2.12 (2 H, s); 2.14-2.27 (2 H, m); 2.93-3.03 (1 H, m); 3.18-3.26 (4 H, m); 4.40 (2 H, t, J = 6.1 Hz); 4.80 (2 H, dd, J = 7.8 und 6.0 Hz); 7.26-7.33 (3 H, m); 7.36-7.42 (2 H, m). |
| | | | | 13C-NMR (CDCl3): 30.1; 31.2; 32.9; 33.0; 35.7; 38.0; 40.2; 44.4; 58.0; 77.3; 126.9; 127.4; 127.8; 147.2; 173.7. |
| 255 | 1 | Bsp. Nr. 431/ Alkylierung/ 58% | m/z: [M+H]+ = 357.4, Rt = 2.5 min. | 1H-NMR (CDCl3): 1.32-1.41 (2 H, m); 1.62-1.75 (2 H, m); 1.83 (2 H, dd, J = 14.4 und 7.4 Hz); 1.91-2.04 (2 H, m); 2.05 (6 H, s); 2.17-2.33 (2 H, m); 2.35 (2 H, s); 2.88-2.96 (1 H, m); 2.98 (2 H, s); 3.15 (2 H, t, J = 7.1 Hz); 4.34 (2 H, t, J = 6.1 Hz); 4.76 (2 H, dd, J = 7.8 und 6.0 Hz); 7.25-7.32 (3 H, m); 7.36-7.41 (2 H, m). |
| | | | | 13C-NMR (CDCl3): 30.0; 31.2; 33.0; 33.1; 35.9; 38.0; 40.1; 43.4; 59.0; 77.2; 126.8; 127.6; 127.8; 173.7. |
| 256 | 2 | Bsp. Nr. 234 Stufe9/ Alkylierung/ 42% | [M+H]+: m/z = 372.4, Rt = 2.6 min. | 1H-NMR (CDCl3): 1.14-1.36 (6 H, m); 1.38-1.62 (6 H, m); 1.62-1.78 (4 H, m); 1.91-1.97 (2 H, m); 1.98-2.22 (5 H, m); 2.26 (6 H, s); 2.28 (2 H, s); 2.47-2.58 (2 H, m); 3.12 (2 H, s); 3.33-3.40 (2 H, m). |
| | | | | 13C-NMR (CDCl3): 16.9; 25.1; 26.9; 28.5; 31.6; 32.1; 33.7; 34.9; 36.3; 37.9; 38.8; 42.0; 44.2; 57.5; 61.1; 124.0; 174.3. |
| 257 | 3 | Bsp. Nr. 247/ Reduktion/ 87% | m/z: [M+H]+= 363.4 (100 %) | 1H-NMR (CDCl3): 1.21-1.34 (6 H, m); 1.43-1.76 (14 H, m); 1.85-1.92 (4 H, m); 2.00-2.10 (3 H, m); 2.27 (6 H, s); 2.37-2.44 (4 H, m); 2.57-2.64 (2 H, m); 3.12 (3 H, s). |
| | | | [MH-NHMe2]+= 318.4 (13 %), Rt = 1.9 min. | 13C-NMR (CDCl3): 12.5; 25.1; 27.4; 28.5; 31.6; 33.1; 35.1; 38.0; 41.5, 44.3; 49.2; 51.4; 54.8; 57.6; 69.5; 76.9, 77.2; 77.5; 78.6. |
| 258 | 3 | . Bsp. Nr. 424/ Reduktive Aminierung/ 52% | m/z: [MH-HNMe2]+ = 301.3 (15%) | 1H-NMR (CDCl3): 1.20-1.34 (6 H, m); 1.35 (6 H, s); 1.39-1.67 (12 H, m); 1.68-1.74 (2 H, m); 1.99-2.10 (1 H, m); 2.26 (6 H, s); 2.33 (2 H, s); 2.53-2.60 (4 H, m). |
| | | | [M+H]+ = 346.4 (100 %), Rt = 0.3 min. | 13C-NMR (CDCl3): 25.1; 27.0; 27.3; 28.5; 31.2; 33.0; 35.0; 38.0; 39.4; 41.5; 44.3; 52.8; 54.5; 57.7; 69.4; 124.9. |
| 259 | 3 | Bsp. Nr. 424/ Acylierung/ 26% | m/z: [MH-HNMe2]+ = 315.3 (2 %) | 1H-NMR (CDCl3): 1.16-1.38 (6 H, m); 1.43-1.61 (4 H, m); 1.49 (3.6 H, s); 1.50 (2.4 H, s); 1.63-1.77 (5.2 H, m); 1.83 (0.8 H, t, J = 7.1 Hz); 2.01-2.12 (1 H, m); 2.26 (6 H, s); 2.47 (1.2 H, s); 2.48 (0.8 H, s); 3.17 (1.2 H, s); 3.24 (0.8 H, s); 3.49 (0.8 H, t, J = 7.1 Hz); 3.54 (1.2 H, t, J = 7.2 Hz). |
| | | | [M+H]+ = 360.4 (100%), Rt = 2.7 min. | 13C-NMR (CDCl3): 25.09; 25.12; 27.00; 27.03; 28.48; 28.51; 29.8; 30.36; 30.43; 32.0; 33.5; 37.8; 40.6; 42.8; 43.6; 43.9; 44.1; 44.2; 44.6; 45.9; 57.77; 57.84; 58.8; 60.5; 124.7; 124.8; 166.7; 166.8. |
| 260 | 2 | Bsp. Nr. 162 Stufe3/ Alkylierung/ 45% | m/z: [M+H]+ = 357.3, Rt = 2.2 min. | 1H-NMR (CDCl3): 1.34-1.43 (2 H, m); 1.58-1.67 (1 H, m); 1.70-1.79 (2 H, m); 1.95-2.02 (3 H, m); 2.04 (6 H, s); 2.14 (2 H, s); 2.15-2.30 (2 H, m); 2.47-2.57 (1 H, m); 3.23 (1 H, dd, J = 13.7 und 7.5 Hz); 3.28 (2 H, s); 3.36 (1 H, dd, J = 13.6 und 7.6 Hz); 3.47 (1 H, dd, J = 8.6 und 6.3 Hz); 3.73-3.80 (1 H, m); 3.82-3.91 (2 H, m); 7.26-7.32 (3 H, m); 7.36-7.42 (2 H, m). |
| | | | | 13C-NMR (CDCl3): 30.2; 32.7; 32.8; 35.9; 38.0; 44.2; 45.3; 58.6; 60.1; 71.4; 126.8; 127.4; 127.8; 174.0. |
| 261 | 1 | Bsp. Nr. 431/ Alkylierung/ 42% | m/z: [M+H]+ = 357.3, Rt = 2.5 min. | 1H-NMR (CDCl3): 1.32-1.41 (2 H, m); 1.58-1.62 (1 H, m); 1.67-1.76 (2 H, m); 1.85-2.03 (3 H, m); 2.05 (6 H, s); 2.15-2.35 (2 H, m); 2.37 (2 H, s); 2.40-2.49 (1 H, m); 3.01 (2 H, s); 3.17 (1 H, dd, J = 13.7 und 7.6 Hz); 3.30 (1 H, dd, J = 13.7 und 7.6 Hz); 3.41 (1 H, dd, J = 8.6 und 6.3 Hz); 3.68-3.88 (3 H, m); 7.26-7.32 (3 H, m); 7.36-7.42 (2 H, m). |
| | | | | 13C-NMR (CDCl3): 30.0; 31.1; 32.9; 33.0; 36.1; 37.9; 38.0; 43.3; 45.2; 59.6; 60.7; 67.7; 71.3; 126.8; 127.6; 127.8; 136.1; 174.0. |
| 262 | 2 | Bsp. Nr. 162 Stufe3/ Alkylierung/ 58% | m/z: [M+H]+ = 371.3, Rt = 2.5 min. | 1H-NMR (CDCl3): 1.32-1.41 (2 H, m); 1.44-1.58 (1 H, m); 1.64-1.93 (7 H, m); 1.95-2.03 (2 H, m); 2.04 (6 H, br s); 2.12 (2 H, s); 2.13-2.30 (2 H, m); 3.24-3.42 (4 H, m); 3.67-3.74 (1 H, m); 3.76-3.89 (2 H, m); 7.26-7.33 (3 H, m); 7.35-7.41 (2 H, m). |
| | | | | 13C-NMR (CDCl3): 25.6; 30.1; 31.4; 32.9; 33.3; 35.7; 38.0; 40.0; 44.6; 58.3; 67.7; 77.02; 126.8; 127.5; 128.0; 173.6. |
| 263 | 1 | Bsp. Nr. 431/ Alkylierung/ 57% | m/z: [M+H]+ = 371.4, Rt = 2.7 min. | 1H-NMR (CDCl3): 1.31-1.49 (3 H, m); 1.58-1.76 (4 H, m); 1.78-1.89 (2 H, m); 1.90-2.03 (3 H, m); 2.04 (6 H, s); 2.15-2.33 (2 H, m); 2.35 (2 H, s); 2.99-3.06 (2 H, m); 3.23-3.38 (2 H, m); 3.63-3.84 (3 H, m); 7.27-7.32 (3 H, m); 7.35-7.41 (2 H, m). |
| | | | | 13C-NMR (CDCl3): 25.6; 30.0; 30.01; 31.3; 33.0; 33.2; 35.8; 38.0; 39.8; 43.6; 59.2; 60.6; 67.6; 76.9; 126.7; 127.5; 127.7; 173.6. |
| 264 | 2 | Bsp. Nr. 162 Stufe3/ Alkylierung/ 50% | m/z: [M+H]+ = 371.4, Rt = 2.4 min. | 1H-NMR (CDCl3): 1.33-1.42 (2 H, m); 1.47-1.67 (4 H, m); 1.69-1.77 (2 H, m); 1.90-2.01 (3 H, m); 2.03 (6 H, s); 2.05-2.21 (4 H, m); 3.23 (2 H, s); 3.28 (2 H, t, J = 7.4 Hz); 3.35 (1 H, dd; J = 8.2 und 6.9 Hz); 3.70-3.77 (1 H, m); 3.81-3.93 (2 H, m); 7.26-7.32 (3 H, m); 7.35-7.41 (2 H, m). |
| | | | | 13C-NMR (CDCl3): 30.2; 30.7; 32.2; 32.8; 35.7; 36.9; 38.0; 41.4; 44.4; 57.9; 60.1; 67.8; 73.1; 126.8; 127.4; 127.7; 173.6. |
| 265 | 3 | Bsp. Nr. 424/ Reduktive Aminierung/ 68% | [M+H]+: m/z = 349.4, Rt = 0.6 min. | 1H-NMR (CDCl3): 1.18-1.36 (10 H, m); 1.38-1.74 (15 H, m); 2.04 (1 H, m); 2.12-2.36 (2 H, m); 2.26 (6 H, s); 2.53 (2 H, br s); 3.35 (2 H, dt, J = 1.8 und 12.1 Hz); 3.94 (2 H, dd, J = 3.7 und 10.9 Hz). |
| | | | | 13C-NMR (CDCl3): 25.0; 27.2; 28.4; 31.7; 33.0; 34.3; 34.8; 38.0; 41.6; 44.2; 55.0; 57.8; 63.2; 68.0; 69.2. |
| 266 | 3 | Bsp. Nr. 424/ Reduktive Aminierung/ 30% | [M+H]+: m/z = 321.4, Rt = 1.9 min. | 1H-NMR (CDCl3): 0.91 (6 H, d, J = 6.5 Hz); 1.16-1.40 (6 H, m); 1.40-1.78 (13 H, m); 1.81 (2 H, t, J = 7.1 Hz); 2.05 (1 H, m); 2.28 (6 H, s); 2.60-2.76 (4 H, m); 2.96 (2 H, br s). |
| | | | | 13C-NMR (CDCl3): 22.3; 25.1; 26.3; 27.2; 28.4; 31.9; 33.7; 35.6; 37.8; 41.7; 44.0; 54.0; 55.1; 67.3. |
| 267 | 3 | Bsp. Nr. 424/ Reduktive Aminierung/ 74% | [M+H]+: m/z = 344.4, Rt = 0.6 min. | 1H-NMR (CDCl3): 0.81 (2 H, dd, J = 5.0 und 7.1 Hz); 1.20 (2 H, teilweise überlagert dd, J = 4.9 und 7.1 Hz); 1.16-1.36 (6 H, m); 1.38-1.74 (14 H, m); 2.04 (1 H, m); 2.26 (6 H, s); 2.33 (2 H, s); 2.54-2.67 (4 H, m). |
| | | | | 13C-NMR (CDCl3): 8.1; 13.9; 25.0; 27.3; 28.4; 32.9; 34.1; 34.9; 38.0; 41.4; 44.3; 54.4; 57.7; 69.3; 123.4. |
| 268 | 3 | Bsp. Nr. 424/ Reduktive Aminierung/ 83% | [M+H]+: m/z = 358.4, Rt = 1.3 min. | 1H-NMR (CDCl3): 1.57-1.36 (6 H, m); 1.38-1.75 (12 H, m); 1.87-1.95 (2 H, m); 1.97-2.21 (5 H, m); 2.26 (6 H, s); 2.34 (2 H, s); 2.45-2.54 (4 H, m); 2.58 (2 H, t, J = 6.8 Hz). |
| | | | | 13C-NMR (CDCl3): 17.0; 25.0; 27.3; 28.4; 32.2; 33.0; 34.6; 35.0; 36.6; 37.9; 41.4; 44.3; 52.5; 54.6; 57.7; 69.4; 124.5. |
| 269 | 2 | Bsp. Nr. 162 Stufe3/ Alkylierung/ 44% | m/z: [M+H]+ = 371.4, Rt = 2.4 min. | 1H-NMR (CDCl3): 1.25-1.45 (4 H, m); 1.50-1.58 (2 H, m); 1.70-1.80 (2 H, m); 1.80-1.93 (1 H, m); 2.00 (8 H, s); 2.14 (2 H, s); 2.15-2.22 (2 H, m); 3.14 (2 H, d, J = 7.3 Hz); 3.26 (2 H, s); 3.35 (2 H, dt, J = 11.7 und 2.1 Hz); 3.94-3.99 (2 H, m); 7.26-7.32 (3 H, m); 7.35-7.41 (2 H, m). |
| | | | | 13C-NMR (CDCl3): 30.1; 30.7; 32.8; 33.7; 35.9; 37.9; 44.3; 48.4; 59.2; 60.0; 67.5; 126.8; 127.4; 127.8; 174.1. |
| 270 | 1 | Bsp. Nr. 431/ Alkylierung/ 35% | m/z: [M+H]+ = 371.3, Rt = 2.6 min. | 1H-NMR (CDCl3): 1.23-1.40 (4 H, m); 1.45-1.52 (2 H, m); 1.68-1.84 (3 H, m); 1.90-2.02 (2 H, m); 2.05 (6 H, s); 2.18-2.35 (2 H, m); 2.38 (2 H, s); 3.01 (2 H, s); 3.09 (2 H, d, J = 7.3 Hz); 3.30 (2 H, dt, J = 11.7 und 2.2 Hz); 3.90-3.96 (2 H, m); 7.26-7.32 (3 H, m); 7.36-7.41 (2 H, m). |
| | | | | 13C-NMR (CDCl3): 30.0; 30.7; 33.1; 33.7; 36.1; 38.0; 43.4; 48.3; 60.2; 67.5; 126.8; 127.6; 127.8; 174.1. |
| 271 | 1 | Bsp. Nr. 431/ Alkylierung/ 40% | m/z: [M+H]+ = 371.4, Rt = 2.6 min. | 1H-NMR (CDCl3): 1.30-1.40 (2 H, m); 1.41-1.59 (3 H, m); 1.67-1.76 (2 H, m); 1.90-2.01 (2 H, m); 2.04 (6 H, s); 2.05-2.15 (2 H, m); 2.16-2.33 (2 H, m); 2.36 (2 H, s); 2.98 (2 H, s); 3.23 (2 H, t, J = 7.4 Hz); 3.30 (1 H, dd; J = 8.2 und 6.9 Hz); 3.68-3.75 (1 H, m); 3.78-3.90 (2 H, m); 7.26-7.32 (3 H, m); 7.35-7.41 (2 H, m). |
| | | | | 13C-NMR (CDCl3): 30.0; 30.6; 32.2; 33.1; 35.9; 36.9; 38.0; 41.3; 43.5; 58.9; 60.7; 67.8; 73.1; 126.7; 127.6; 127.8; 173.6. |
| 272 | 1 | | | |
| 273 | 3 | Bsp. Nr. 424/ Reduktive Aminierung/ 62% | [M+H]+: m/z = 307.4, Rt = 0.6 min. | 1H-NMR (CDCl3): 0.89 (6 H, d, J = 6.5 Hz); 1.18-1.36 (6 H, m); 1.38-1.80 (13 H, m); 2.64 (1 H, m); 2.13 (2 H, d, J = 7.3 Hz); 2.25 (2 H, s); 2.27 (6 H, s); 2.51 (2 H, t, J = 6.5 Hz). |
| | | | | 13C-NMR (CDCl3): 21.0; 25.1; 27.2; 27.4; 28.4; 33.1; 34.7; 38.0; 41.5; 44.4; 54.8; 57.8; 65.3; 69.1. |
| 274 | 2 | Bsp. Nr. 234 Stufe9/ Alkylierung/ 90% | [M+H]+: m/z = 333.4, Rt = 2.6 min. | 1H-NMR (CDCl3): 1.14-1.36 (6 H, m); 1.38-1.60 (6 H, m); 1.60-1.78 (6 H, m); 1.80-1.94 (2 H, m); 1.96-2.10 (3 H, m); 2.25 (8 H, s); 2.51 (1 H, m); 3.03 (2 H, s); 7.57 (2 H, d, J = 7.6 Hz). |
| | | | | 13C-NMR (CDCl3): 18.4; 25.1; 26.4; 26.9; 28.5; 31.5; 33.8; 36.5; 37.9; 42.0; 44.0; 47.6; 57.7; 61.2; 173.8. |
| 276 | 2 | Bsp. Nr. 234 Stufe9/ Alkylierung/ 50% | [M+H]+: m/z = 319.4, Rt = 2.4 min. | 1H-NMR (CDCl3): 0.19 (2 H, td, J = 4.6 und 5.8 Hz); 0.49 (2 H, m); 8.58 (1 H, m); 1.16-1.37 (6 H, m); 1.38-1.62 (6 H, m); 1.64-1.79 (4 H, m); 2.06 (1 H, m); 2.27 (6 H, s); 2.28 (2 H, s); 3.11 (2 H, d, J = 7.1 Hz); 3.19 (2 H, s). |
| | | | | 13C-NMR (CDCl3): 3.3; 9.0; 25.1; 26.8; 28.5; 31.6; 36.3; 37.9; 42.3; 44.2; 46.8; 57.7; 60.9; 173.6. |
| 277 | 2 | Bsp. Nr. 234 Stufe9/ Alkylierung/ 51% | [M+H]+: m/z = 361.4, Rt = 3.0 min. | 1H-NMR (CDCl3): 0.84-1.02 (2 H, m); 1.06-1.36 (10 H, m); 1.38-1.84 (15 H, m); 2.04 (1 H, m); 2.26 (6 H, s); 2.28 (2 H, s); 3.06 (2 H, d, J = 7.9 Hz), 3.07 (2 H, s). |
| | | | | 13C-NMR (CDCl3): 25.1; 25.7; 26.3; 26.9; 28.5; 30.1; 30.8; 31.8; 35.9; 36.4; 37.9; 42.3; 44.2; 48.6; 57.7; 61.6; 174.3. |
| 278 | 2 | Bsp. Nr. 234 Stufe9/ Alkylierung/ 61% | [M+H]+: m/z = 347.4, Rt = 2.8 min. | 1H-NMR (CDCl3): 1.10-1.36 (8 H, m); 1.38-1.80 (16 H, m); 1.98-2.18 (2 H, m); 2.26 (6 H, s); 2.28 (2 H, s); 3.09 (2 H, s); 3.17 (2 H, d, J = 7.9 Hz). |
| | | | | 13C-NMR (CDCl3): 25.07; 25.14; 26.9; 28.5; 30.3; 31.7; 36.3; 37.9; 38.0; 42.2; 44.2; 47.2; 57.7; 61.1; 174.0. |
| 279 | 2 | Bsp. Nr. 24b/ Alkylierung/ 58% | m/z: [M+H]+ = 363.3, Rt = 2.2 min. | Drehwert: [α]Δ24 = +4.74 °, (c = 1.0, MeOH) |
| | | | Der Enantiomerenüberschuss beträgt nach HPLC an Chiracel OD [(Cyclohexan / 2-PrOH / Et2NH (70:30:0.1)] > 98 %. | 1H-NMR (CDCl3): 1.41-1.50 (2 H, m); 1.57-1.67 (1 H, m); 1.72-1.82 (2 H, m); 1.94-2.07 (5 H, m); 2.10 (6 H, s); 2.19 (2 H, s); 2.45-2.57 (1 H, m); 3.18-3.26 (3 H, m); 3.35 (1 H, dd, J = 13.6 und 7.6 Hz); 3.46 (1 H, dd, J = 8.6 und 6.3 Hz); 3.72-3.79 (1 H, m); 3.81-3.91 (2 H, m); 6.82-6.86 (1 H, m); 7.04 (1 H, dd, J = 5.0 und 3.6 Hz); 7.22-7.26 (1 H, m). |
| | | | | 13C-NMR (CDCl3): 30.1; 32.6; 32.7; 35.8; 38.0; 38.02; 44.0; 45.3; 58.7; 59.2; 67.7; 71.4; 123.4; 124.8; 126.3; 173.9. |
| 280 | 2 | Bsp. Nr. 24b/ Alkylierung/ 60% | m/z: [M+H]+ = 363.3, Rt = 2.2 min | Drehwert: [α]Δ24 = -3.28°, (c = 1.0, MeOH) |
| | | | Der Enantiomerenüberschuss beträgt nach HPLC an Chiracel OD [(Cyclohexan / 2-PrOH / Et2NH (70:30:0.1)] 91 %. | 1H-NMR (CDCl3): 1.42-1.50 (2 H, m); 1.57-1.67 (1 H, m); 1.73-1.83 (2 H, m); 1.94-2.08 (5 H, m); 2.10 (6 H, s); 2.19 (2 H, s); 2.46-2.56 (1 H, m); 3.19-3.27 (3 H, m); 3.35 (1 H, dd, J = 13.6 und 7.6 Hz); 3.46 (1 H, dd, J = 8.6 und 6.3 Hz); 3.72-3.79 (1 H, m); 3.82-3.92 (2 H, m); 6.83-6.88 (1 H, m); 7.03-7.07 (1 H, m); 7.22-7.27 (1 H, m). |
| | | | | 13C-NMR (CDCl3): 30.1; 32.6; 32.7; 35.8; 37.9; 38.0; 44.0; 45.3; 58.8; 59.2; 67.7; 71.4; 123.5; 124.8; 126.3; 173.9. |
| 281 | 2 | Bsp. Nr. 347 Stufe9/ Alkylierung/ 65% | [M+H]+: m/z = 321.4, Rt = 2.6 min. | 1H-NMR (CDCl3): 0.91 (3 H, t, J = 7.3 Hz); 1.16-1.36 (8 H, m); 1.40-1.80 (12 H, m); 2.04 (1 H, m); 2.26 (6 H, s); 2.27 (2 H, s); 3.06 (2 H, s); 3.23 (2 H, t, J = 7.3 Hz). |
| | | | | 13C-NMR (CDCl3): 13.6; 19.9; 25.1; 26.9; 28.5; 29.2; 31.5; 31.6; 36.2; 41.8; 42.2; 44.2; 57.7; 60.7; 173.9. |
| 282 | 2 | Bsp. Nr. 234 Stufe9/ Alkylierung/ 78% | [M+H]+: m/z = 279.3, Rt = 1.4 min. | 1H-NMR (CDCl3): 1.12-1.34 (6 H, m); 1.38-1.60 (6 H, m); 1.61-1.78 (4 H, m); 2.04 (1 H, s); 2.25 (8 H, s); 2.80 (3 H, s); 3.07 (2 H, s). |
| | | | | 13C-NMR (CDCl3): 25.0; 26.9; 28.3; 29.5; 31.9; 36.0; 37.9; 42.0; 44.0; 57.6; 63.3; 174.0. |
| 283 | 1 | Bsp. Nr. 71/ Reduktive Aminierung/ 9% | m/z: [M+H]+ = 343.3, Rt = 0.3 min. | 1H-NMR (CDCl3): 1.23-1.32 (2 H, m); 1.44 (2 H, t, J = 6.9 Hz); 1.61-1.69 (2 H, m); 1.81-1.89 (4 H, m); 1.98-2.02 (1 H, m); 2.03 (6 H, s); 2.18-2.33 (3 H, m); 2.43 (2 H, s); 2.47 (2 H, t, J = 6.9 Hz); 2.97-3.07 (1 H, m); 4.41 (2 H, t, J = 6.2 Hz); 4.78 (2 H, dd, J = 7.9 und 5.9 Hz); 7.27-7.33 (3 H, m); 7.34-7.39 (2 H, m). |
| | | | | 13C-NMR (CDCl3): 31.1; 32.8; 34.0; 37.9; 37.93; 38.1; 41.2; 53.9; 54.3; 60.5; 65.7; 77.8; 126.4; 127.6; 127.7. |
| 284 | 1 | Bsp. Nr. 71/ Reduktive Aminierung/ 10% | m/z: [M+H]+ = 343.3, Rt = 0.7 min. | 1H-NMR (CDCl3): 1.23-1.31 (2 H, m); 1.40-1.46 (2 H, m); 1.56-1.70 (3 H, m); 1.80-1.95 (2 H, m); 1.97-2.02 (1 H, m); 2.04 (6 H, s); 2.17-2.33 (2 H, m); 2.34-2.42 (3 H, m); 2.42-2.54 (4 H, m); 3.47-3.52 (1 H, m); 3.70-3.77 (1 H, m); 3.80-3.90 (2 H, m); 7.24-7.40 (5 H, m). |
| | | | | 13C-NMR (CDCl3): 31.0; 31.02; 34.5; 37.9; 38.0; 38.7; 41.2; 53.9; 59.8; 65.6; 67.7; 72.4; 126.5; 127.6; 127.7. |
| 285 | 1 | Bsp. Nr. 71/ Alkylierung/ 2Stufen 6% | m/z: [M+H]+ = 343.4, Rt = 0.5 min. | 1H-NMR (CDCl3): 0.38 (2 H, dd, J = 6.3 und 5.1 Hz); 0.73 (2 H, dd, J = 6.3 und 5.1 Hz); 1.24-1.33 (2 H, m); 1.44-1.51 (2 H, m); 1.63-1.72 (4 H, m); 1.87-2.00 (2 H, m); 2.05 (6 H, s); 2.15-2.28 (2 H, m); 2.55-2.67 (4 H, m); 2.78-2.84 (2 H, m); 6.40-6.80 (1 H, br s); 7.26-7.34 (3 H, m); 7.35-7.40 (2 H, m). |
| | | | | 13C-NMR (CDCl3): 12.7; 30.9; 34.3; 38.0; 41.2; 51.0; 53.4; 53.5; 55.6; 57.7; 65.4; 126.5; 127.6; 127.65. |
| 286 | 2 | Bsp. Nr. 234 Stufe9/ Alkylierung/ 33% | [M+H]+: m/z = 333.4, Rt = 2.6 min. | 1H-NMR (CDCl3): 1.16-1.34 (6 H, m); 1.38-1.86 (18 H, m); 2.04 (1 H, m); 2.26 (6 H, s); 2.27 (2 H, s); 3.03 (2 H, s); 4.48 (1 H, m). |
| | | | | 13C-NMR (CDCl3): 24.3; 25.1; 26.9; 28.5; 28.8; 31.3; 36.4; 37.9; 42.5; 44.2; 51.9; 56.6; 57.6; 173.6. |
| 287 | 2 | Bsp. Nr. 234 Stufe9/ Alkylierung/ 79% | [M+H]+: m/z = 377.4, Rt = 2.7 min. | 1H-NMR (CDCl3): 1.14-1.34 (6 H, m); 1.38-1.78 (12 H, m); 1.80-1.94 (4 H, m); 1.98-2.16 (3 H, m); 2.25 (6 H, s); 2.26 (2 H, s); 3.10 (2 H, s); 3.13 (3 H, s); 3.20-3.27 (2 H, m). |
| | | | | 13C-NMR (CDCl3): 12.3; 25.0; 26.9; 28.3; 31.3; 31.6; 31.9; 36.3; 37.5; 37.9; 42.4; 44.2; 49.2; 57.5; 61.2; 78.1; 173.6. |
| 288 | 2 | Bsp. Nr. 24b/ Alkylierung/ 14% | m/z: [MH-HNMe2]+ = 315.3 (100 %) | 1H-NMR (CDCl3): 1.37 (6 H, s); 1.44-1.53 (2 H, m); 1.80-1.88 (2 H, m); 1.95-2.15 (4 H, m); 2.10 (6 H, s); 2.23 (2 H, s); 3.39 (2 H, s); 3.53 (2 H, s); 6.86 (1 H, dd, J = 3.6 und 1.0 Hz); 7.04 (1 H, dd, J = 5.1 und 3.6 Hz); 7.24 (1 H, dd, J = 5.1 und 1.1 Hz). |
| | | | [M+H]+ = 360.3 (35 %), Rt = 2.5 min. | 13C-NMR (CDCl3): 24.8; 32.4; 32.5; 33.0; 36.1; 38.0; 43.4; 51.2; 59.4; 59.7; 123.5; 124.6; 124.8; 126.3; 142.8; 175.2. |
| 289 | 1 | Bsp. Nr. 24a/ Alkylierung/ 28% | m/z: [MH-HNMe2]+ = 315.2 (100 %) | 1H-NMR (CDCl3): 1.34 (6 H, s); 1.52 (2 H, ddd, J = 13.3, 9.4 und 4.0 Hz); 1.73-1.81 (2 H, m); 1.94-2.05 (2 H, m); 2.07-2.16 (2 H, m); 2.10 (6 H, s); 2.36 (2 H, s); 3.35 (2 H, s); 3.37 (2 H, s); 6.84 (1 H, dd, J = 3.6 und 1.0 Hz); 7.03 (1 H, dd, J = 5.1 und 3.5 Hz); 7.24 (1 H, dd, J = 5.1 und 0.9 Hz). |
| | | | [M+H]+ = 360.3 (50 %), Rt = 2.6 min. | 13C-NMR (CDCl3): 24.7; 32.6; 32.9; 36.1; 38.1; 43.0; 51.2; 59.6; 60.0; 123.6; 124.6; 125.1; 126.3; 142.3; 175.2. |
| 290 | 2 | Bsp. Nr. 130/ N-Demethylier ung/ 23% | m/z: [M+H]+ = 333.3 und 302.2, Rt = 2.6 min. | 1H-NMR (CDCl3): 0.03-0.07 (2 H, m); 0.41-0.48 (2 H, m); 0.59-0.69 (1 H, m); 1.41 (2 H, dd, J = 14.4 und 7.0 Hz); 1.47-1.57 (2 H, m); 1.71-2.03 (6 H, m); 2.11 (3 H, s); 2.24 (2 H, s); 3.21 (2 H, s); 3.31-3.36 (2 H, m); 6.87 (1 H, dd, J = 3.5 und 1.0 Hz); 6.95 (1 H, dd, J = 5.1 und 3.5 Hz); 7.21 (1 H, dd, J = 5.1 und 1.0 Hz). Das austauschbare Proton wurde nicht identifiziert. |
| | | | | 13C-NMR (CDCl3): 4.3; 8.6; 28.7; 32.4; 32.5; 33.7; 35.8; 42.6; 43.3; 56.3; 59.3; 123.6; 126.4; 173.4. |
| 291 | 3 | Bsp. Nr. 424/ Acylierung/ 37% | m/z : [M+H]+ = 335.4, Rt = 2.4 min. | 1H-NMR (CDCl3): 1.10-1.38 (6 H, m); 1.40-1.88 (10 H, m); 2.04 (1 H, m); 2.25 (6 H, s); 2.91 (2 H, s); 3.22-3.27 (2 H, m); 3.53 (2 H, t, J = 7.3 Hz); 3.90 (1 H, m); 4.75 (2 H, dd, J = 5.6 und 8.6 Hz); 4.89-4.93 (2 H, m). |
| | | | | 13C-NMR (CDCl3): 25.08; 25.10; 26.8; 27.0; 28.5; 29.7; 31.6; 33.4; 37.7; 38.3; 38.4; 40.6; 42.6; 44.0; 44.2; 44.5; 44.6; 57.6; 57.7; 58.8; 59.6; 73.03; 73.07; 169.3; 169.4. |
| 292 | 3 | Bsp. Nr. 424/ Alkylierung/ 60% | m/z : [M+H]+ = 351.4, Rt = 2.0 min. | 1H-NMR (CDCl3): 1.15 (6 H, s); 1.19-1.35 (6 H, m); 1.39-1.75 (14 H, m); 2.04 (1 H, m); 2.26 (6 H, s); 2.31 (2 H, s); 2.36-2.43 (2 H, m); 2.54 (2 H, t, J = 6.8 Hz); 3.17 (3 H, s). |
| | | | | 13C-NMR (CDCl3): 25.0; 25.2; 27.2; 28.5; 33.1; 35.0; 37.9; 38.0; 41.4; 44.2; 49.1; 52.0; 54.8; 57.7; 69.5; 73.9. |
| 293 | 2 | Bsp. Nr. 234 Stufe9/ Alkylierung/ 60% | m/z: [M+H]+ = 349.4, Rt = 2.3 min. Der Enantiomerenüb erschuss beträgt nach HPLC an Chiralcel OD [(Cyclohexan / 2-PrOH / Et2NH (90:10:0.1) ≥ 97 %. | 1H-NMR (CDCl3): 1.17-1.35 (5 H, m); 1.40-1.78 (12 H, m); 1.93-2.10 (2 H, m); 2.26 (6 H, s); 2.29 (2 H, s); 2.51 (1 H, td, J = 14.2 und 6.9 Hz); 3.10 (2 H, s); 3.20 (1 H, dd, J = 13.6 und 7.6 Hz); 3.35 (1 H, dd, J = 13.6 und 7.6 Hz); 3.46 (1 H, dd, J = 8.6 und 6.2 Hz); 3.70-3.79 (1 H, m), 3.80-3.90 (2 H, m). |
| | | | | 13C-NMR (CDCl3): 25.0; 26.9; 28.5; 30.1; 31.62; 31.65; 36.6; 37.9; 37.94; 42.1; 44.1; 45.2; 57.7; 61.5; 67.7; 71.4; 174.3. |
| | | | | Drehwert: [α]D24 = 4.57 °, (c = 1.13, MeOH). |
| 294 | 2 | Bsp. Nr. 234 Stufe9/ Alkylierung/ 44% | m/z: [M+H]+ = 349.4, Rt = 2.3 min. | 1H-NMR (CDCl3): 1.16-1.33 (6 H, m); 1.39-1.76 (11 H, m); 1.91-2.09 (2 H, m); 2.25 (6 H, s); 2.27 (2 H, s); 2.49 (1 H, td, J = 14.1 und 7.0 Hz); 3.09 (2 H, s); 3.18 (1 H, dd, J = 13.6 und 7.6 Hz); 3.33 (1 H, dd, J = 13.6 und 7.6 Hz); 3.44 (1 H, dd, J = 8.6 und 6.3 Hz); 3.73 (1 H, dd, J = 15.4 und 7.6 Hz); 3.78-3.88 (2 H, m). |
| | | | Der Enantiomerenüb erschuss beträgt nach HPLC an Chiralcel OD [(Cyclohexan / 2-PrOH / Et2NH (90:10:0.1) = 93 %. | 13C-NMR (CDCl3): 25.0; 26.8; 28.4; 30.1; 31.57; 31.59; 36.5; 37.8; 37.9; 42.0; 44.1; 45.1; 57.6; 61.4; 67.6; 71.3; 174.2. |
| | | | | LC-MS (Methode 1): m/z: [M+H]+ = 349.4, Rt = 2.3 min. |
| | | | | Drehwert: [α]D24 = -6.24 °, (c = 1.0, MeOH). |
| 295 | 1 | Bsp. Nr. 18/ Reduktive Aminierung/ 10% | m/z: [M+H]+ = 349.3, Rt = 0.3 min. | 1H-NMR (CDCl3): 1.33-1.42 (2 H, m); 1.50 (2 H, t, J = 6.9 Hz); 1.64-1.73 (2 H, m); 1.86 (2 H, dd, J = 15.0 und 7.6 Hz); 1.89-1.97 (2 H, m); 2.04-2.10 (2 H, m); 2.10 (6 H, s); 2.26-2.34 (2 H, m); 2.41 (2 H, s); 2.50 (2 H, t, J = 6.8 Hz); 2.96 (1 H, m); 4.41 (2 H, t, J = 6.2 Hz); 4.78 (2 H, dd, J = 7.9 und 6.0 Hz); 6.85 (1 H, dd, J = 3.5 und 1.0 Hz); 7.04 (1 H, dd, J = 5.1 und 3.6 Hz); 7.23 (1 H, dd, J = 5.1 und 1.0 Hz). |
| | | | | 13C-NMR (CDCl3): 32.9; 33.7; 34.0; 34.4; 38.1; 40.9; 53.9; 54.3; 59.7; 65.7; 77.8; 123.2; 124.9; 126.2. |
| 296 | 1 | Bsp. Nr. 18/ Reduktive Aminierung/ 12% | m/z: [M+H]+ = 349.3, Rt = 0.4 min. | 1H-NMR (CDCl3): 1.32-1.76 (8 H, m); 1.85-2.06 (4 H, m); 2.13 (6 H, s); 2.34-2.57 (7 H, m); 3.47-3.52 (1 H, m); 3.69-3.77 (1 H, m); 3.80-3.90 (2 H, m); 6.87(1 H, dd, J = 3.1 Hz); 7.04 (1 H, dd, J = 5.1 und 3.5 Hz); 7.24 (1 H, br d, J = 5.2 Hz). |
| | | | | 13C-NMR (CDCl3): 31.0; 33.6; 34.2; 38.1; 38.6; 41.0; 53.9; 59.8; 67.8; 72.4; 123.4; 125.2; 126.2. |
| 297 | 3 | Bsp. Nr. 424/ Acylierung/ 70% | m/z: [M+H]+ = 351.4, Rt = 2.8 min. | 1H-NMR (CDCl3): 1.08-1.38 (6 H, m); 1.40 und 1.41 (6 H, 2 s); 1.42-1.82 (12 H, m); 2.06 (1 H, m); 2.26 (6 H, m); 3.17 (2 H, s); 3.18 (1 H, s); 3.28 (0.5 H, s); 3.48 (1.5 H, s); 3.54 (1.6 H, t, J = 7.4 Hz); 3.74 (0.4 H, t, J = 7.0 Hz). |
| | | | | 13C-NMR (CDCl3): 24.1; 25.0; 26.9; 27.0; 28.4; 29.4; 30.0; 30.6; 30.9; 34.4; 37.7; 39.1; 42.8; 44.1; 45.8; 46.3; 51.5; 51.7; 57.8; 57.9; 60.3; 60.9; 79.6; 79.8; 172.5. |
| 298 | 3 | Bsp. Nr. 297/ Reduktion/ 84% | m/z: [M+H]+ = 337.4, Rt = 1.3 min. | 1H-NMR (CDCl3): 1.15 (6 H, s); 1.20-1.35 (6 H, m); 1.39-1.72 (12 H, m); 2.04 (1 H, m); 2.26 (6 H, s); 2.39 (2 H, s) 2.41 (2 H, s); 2.65 (2 H, t, J = 6.9 Hz); 3.20 (3 H, s). |
| | | | | 13C-NMR (CDCl3): 23.7; 25.2; 27.3; 28.4; 32.6; 34.9; 37.9; 41.7; 44.4; 49.3; 56.0; 57.9; 64.5; 71.1; 75.9. |
| 299 | 1 | Bsp. Nr. 18/ Alkylierung/ 2Stufen 62% | m/z: [M+H]+ = 365.3, Rt = 1.9 min. | 1H-NMR (CDCl3): 1.16 (6 H, s); 1.39 (2 H, ddd, J = 13.3 und 10.2 und 3.4 Hz); 1.52 (2 H, t, J = 6.7 Hz); 1.65-1.74 (4 H, m); 1.85-1.96 (2 H, m); 2.10 (6 H, s); 2.11-2.18 (2 H, m); 2.41-2.49 (4 H, m); 2.50-2.57 (2 H, m); 3.18 (3 H, s); 6.84 (1 H, dd, J = 3.5 und 1.1 Hz); 7.03 (1 H, dd, J = 5.1 und 3.5 Hz); 7.22 (1 H, dd, J = 5.1 und 1.1 Hz). |
| | | | | 13C-NMR (CDCl3): 25.3; 33.7; 34.4; 38.0; 38.1; 40.9; 49.1; 52.0; 54.1; 59.6; 65.8; 73.9; 123.2; 124.9; 126.1. |
| 300 | 1 | Bsp. Nr.71/ Alkylierung/ 2Stufen 60% | m/z: [M+H]+ = 359.4, Rt = 2.0 min. | 1H-NMR (CDCl3): 1.16 (6 H, s); 1.24-1.33 (2 H, m); 1.47 (2 H, t, J = 6.6 Hz); 1.63-1.74 (4 H, m); 1.80-1.93 (2 H, m); 2.03 (6 H, s); 2.19-2.33 (2 H, m); 2.43-2.58 (6 H, m); 3.19 (3 H, s); 7.23-7.40 (5 H, m). |
| | | | | 13C-NMR (CDCl3): 25.3; 31.1; 34.6; 37.9; 38.0; 38.1; 41.2; 49.1; 52.1; 54.0; 60.5; 65.7; 73.9; 126.4; 127.6; 127.7. |
| 301 | 3 | Bsp. Nr. 291/ Reduktion/ 25% | m/z: [M+H]+ = 323.4, Rt = 0.4 min. | 1H-NMR (CDCl3): 0.71 (3 H, d, J = 6.8 Hz); 1.12-1.32 (6 H, m); 1.36-1.74 (12 H; m); 1.96-2.12 (2 H, m); 2.22 (1 H, d, J = 9.3 Hz); 2.24 (6 H, s); 2.32 (1 H, td, J = 2.8 und 11.9 Hz); 2.46-2.54 (2 H, m); 2.64 (1 H, t, J = 11.8 Hz); 2.69-2.77 (1 H, m); 3.47 (1 H, t, J = 10.3 Hz); 3.66 (1 H, ddd, J = 2.6, 3.7 und 10.4 Hz); 6.53 (1 H, br s). |
| | | | | 13C-NMR (CDCl3): 15.0; 25.0; 27.15; 27.20; 28.5; 32.3; 32.5; 32.6; 34.7; 37.8; 41.5; 44.3; 54.4; 57.6; 64.8; 69.5; 71.7. |
| 302 | 3 | Bsp. Nr. 291/ Reduktion/ 20% | m/z: [M+H]+ = 321.4, Rt = 0.3 min. | 1H-NMR (CDCl3): 1.15-1.34 (6 H, m); 1.38-1.74 (12 H, m); 2.04 (1 H, m); 2.23 (2 H, s); 2.26 (6 H, s); 2.49 (2 H, t, J = 6.8 Hz); 2.69 (2 H, d, J = 7.2 Hz); 3.18 (1 H, m); 4.41 (2 H, t, J = 6.2 Hz); 4.76 (2 H, dd, J = 6.0 und 7.9 Hz). |
| | | | | 13C-NMR (CDCl3): 25.0; 27.3; 28.4; 33.1; 34.9; 35.0; 37.9; 41.6; 44.3; 54.6; 57.6; 59.9; 69.4; 76.6. |
| 303 | 1 | Bsp. Nr. 71/ Reduktive Aminierung/ 51% | m/z: [M+H]+ = 357.3, Rt = 1.3 min. | 1H-NMR (CDCl3): 1.21-1.34 (4 H, m); 1.43 (2 H, t, J = 6.7 Hz); 1.62-1.73 (5 H, m); 1.80-2.00 (2 H, m); 2.05 (6 H, s); 2.23-2.37 (4 H, m); 2.41-2.56 (4 H, m); 3.37 (2 H, dt, J = 12.0 und 1.9 Hz); 3.96 (2 H, dd, J = 10.8 und 3.5 Hz); 7.25-7.33 (3 H, m); 7.35-7.41 (2 H, m). |
| | | | | 13C-NMR (CDCl3): 31.1; 31.8; 34.4; 34.5; 38.0; 41.3; 54.2; 63.1; 65.2; 68.0; 126.6; 127.6; 127.8. |
| 304 | 1 | Bsp. Nr. 18/ Reduktive Aminierung/ 70% | m/z: [M+H]+ = 363.4, Rt = 0.6 min. | 1H-NMR (CDCl3): 1.20-1.42 (4 H, m); 1.50 (2 H, t, J = 6.5 Hz); 1.63-1.73 (5 H, m); 1.85-1.97 (2 H, m); 2.11 (6 H, s); 2.12-2.18 (2 H, m); 2.27 (2 H, d, J = 6.3 Hz); 2.40 (2 H, s); 2.45-2.54 (2 H, m); 3.37 (2 H, dt, J = 12.1 und 1.9 Hz); 3.95 (2 H, dd, J = 10.7 und 3.4 Hz); 6.85 (1 H, d, J = 3.4 Hz); 7.04 (1 H, dd, J = 5.1 und 3.5 Hz); 7.23 (1 H, dd, J = 5.1 und 0.7 Hz). |
| | | | | 13C-NMR (CDCl3): 31.8; 33.8; 34.3; 34.4; 37.8; 38.1; 41.0; 54.3; 60.0; 63.1; 65.4; 68.0; 123.3; 125.0; 126.2. |
| 305 | 1 | Bsp. Nr. 71/ Reduktive Aminierung/ 36% | m/z: [M+H]+= = 357.4, Rt = 1.9 min. | 1H-NMR (CDCl3): 1.25-1.53 (2 H, m); 1.43-1.53 (3 H, m); 1.65-1.80 (4 H, m); 1.82-1.93 (4 H, m); 1.95-2.02 (1 H, m); 2.04 (6 H, s); 2.13-2.32 (2 H, m); 2.50-2.65 (6 H, m); 3.67-3.74 (1 H, m); 3.80-3.89 (2 H, m); 7.23-7.33 (3 H, m); 7.34-7.40 (2 H, m). |
| | | | | 13C-NMR (CDCl3): 25.6; 30.9; 31.5; 34.3; 34.5; 38.0; 41.2; 53.7; 54.0; 60.7; 65.4; 67.6; 77.6; 126.6; 127.6; 127.7. |
| 306 | 1 | Bsp. Nr. 18/ Reduktive Aminierung/ 34% | m/z: [M+H]+ = 363.3, Rt = 1.3 min. | 1H-NMR (CDCl3): 1.35-1.59 (5 H, m); 1.65-2.05 (9 H, m); 2.10 (6 H, s); 2.05-2.15 (2 H, m); 2.48-2.65 (6 H, m); 3.67-3.74 (1 H, m); 3.79-3.89 (2 H, m); 6.84 (1 H, dd, J = 3.5 und 1.0 Hz); 7.03 (1 H, dd, J = 5.1 und 3.5 Hz); 7.22 (1 H, dd, J = 5.1 und 1.0 Hz). |
| | | | | 13C-NMR (CDCl3): 25.6; 31.5; 33.6; 34.2; 34.5; 37.6; 38.1; 41.0; 53.8; 54.0; 59.6; 65.6; 67.6; 77.6; 123.3; 124.9; 126.2. |
| 307 | 1 | Bsp. Nr. 129/ N-Demethylier ung/18% | m/z: [MH-HNMe]+ = 316.3, Rt = 3.1 min. | 1H-NMR (CDCl3): 1.33 (1 H, br s); 1.47-1.68 (6 H, m); 1.70-1.92 (6 H, m); 1.93-2.09 (4 H, m); 2.11 (3 H, s); 2.23 (1 H, td, J = 15.4, 7.8 Hz); 2.29 (2 H, s); 3.10 (2 H, s); 3.11-3.18 (2 H, m); 6.88 (1 H, dd, J = 3.5 und 1.1 Hz); 6.96 (1 H, dd, J = 5.1 und 3.5 Hz); 7.22 (1 H, dd, J = 5.1 und 1.1 Hz). |
| | | | | 13C-NMR (CDCl3): 18.5; 28.2; 28.7; 32.7; 33.6; 33.8; 34.2; 35.7; 38.1; 40.5; 44.6; 56.3; 57.7, 123.7; 126.3; 173.3. |
| 308 | 2 | Bsp. Nr. 128/ N-Demethylier ung/35% | | 1H-NMR (CDCl3): 1.37 (1H, s); 1.47-1.69 (6 H, m); 1.69-2.11 (10 H, m); 2.12 (3 H, s); 2.22-2.25 (2 H, m); 2.21-2.30 (1 H, m); 3.15-3.20 (4 H, m); 6.86-6.90 (1 H, m); 6.96 (1 H, dd, J = 5.0 und 3.6 Hz); 7.22 (1 H, dd, J = 5.0 und 0.6 Hz). |
| | | | | 13C-NMR (CDCl3): 18.5; 28.2; 28.7; 32.5; 336.; 33.7; 34.3; 35.7; 40.5; 43.3; 56.3; 58.9; 132.6; 126.4; 173.3. |
| 309 | 1 | Bsp. Nr. 18/ Reduktive Aminierung/ 63% | m/z: [M+H]+ = 363.3, Rt = 0.6 min. | 1H-NMR (CDCl3): 1.38 (2 H, ddd, J = 13.2 und 10.0 und 3.4 Hz); 1.46-1.62 (5 H, m); 1.65-1.74 (2 H, m); 1.84-1.98 (2 H, m); 1.99-2.08 (2 H, m); 2.10 (6 H, s); 2.11-2.27 (2 H, m); 2.33-2.47 (4 H, m); 2.52 (2 H, t, J = 6.6 Hz); 3.30-3.36 (1 H, m); 3.74 (1 H, dd, J = 15.8 und 7.5 Hz); 3.83 (1 H, dt, J = 8.2 und 4.7 Hz); 3.91 (1 H, dd, J = 8.1 und 7.4 Hz); 6.85 (1 H, dd, J = 3.5 und 0.9 Hz); 7.03 (1 H, dd, J = 5.1 und 3.5 Hz); 7.22 (1 H, dd, J = 5.1 und 0.6 Hz). |
| | | | | 13C-NMR (CDCl3): 32.3; 32.5; 33.7; 34.3; 37.8; 38.1; 40.9; 53.9; 55.8; 59.6; 65.6; 67.8; 73.4; 77.2; 123.2; 125.0; 126.2; 143.1. |
| 310 | 1 | Bsp. Nr. 431/ Alkylierung/ 56% | m/z: [M+H]+ = 354.3, Rt = 2.6 min. | 1H-NMR (CDCl3): 1.33 (6 H, s); 1.37-1.47 (2 H, m); 1.69-1.79 (2 H, m); 1.85-2.02 (2 H, m); 2.03 (6 H, s); 2.18-2.30 (2 H, m); 2.39 (2 H, s); 3.31 (2 H, s); 3.34 (2 H, s); 7.24-7.31 (3 H, m); 7.34-7.41 (2 H, m). |
| | | | | 13C-NMR (CDCl3): 24.7; 30.1; 32.78; 32.84; 36.2; 38.0; 42.9; 51.0; 60.3; 124.5; 126.6; 127.5; 127.8; 175.3. |
| 311 | 2 | Bsp. Nr. 24b/ Alkylierung/ 43% | m/z: [M+H]+= 377.3 (100 %) | 1H-NMR (CDCl3): 1.42-1.49 (2 H, m); 1.64-1.81 (4 H, m); 1.86-1.92 (2 H, m); 1.94-2.05 (4 H, m); 2.06-2.14 (2 H, m, überlagert); 2.10 (6 H, s); 2.18 (2H, s); 3.19 (3 H, s); 3.34 (2 H, s); 3.50 (2 H, s); 6.84 (1 H, dd, J = 3.5 und 0.8 Hz); 7.05 (1 H, dd, J = 5.1 und 3.5 Hz); 7.24 (1 H, dd, J = 5.1 und 0.7 Hz). |
| | | | [MH-NHMe2]+=332.3 (99 %), Rt= 2.5 min. | 13C-NMR (CDCl3): 12.2; 29.6; 32.6; 32.8; 35.8; 38.1; 43.9; 45.0; 49.6; 59.4; 79.8; 123.4; 124.9; 126.3, 174.6. |
| 312 | 1 | Bsp. Nr. 431/ Alkylierung/ 55% | m/z: [M+H]+= 371.3 (47%) | 1H-NMR (CDCl3): 1.32-1.39 (2 H, m); 1.66-1.78 (4 H, m); 1.82-1.88 (2 H, m); 1.90-1.99 (2 H, m); 2.01-2.09 (2 H, m, überlagert); 2.03 (6 H, s); 2.11-2.33 (2 H, m); 2.36 (2 H, s); 3.11 (5 H, s); 3.44 (2 H, s); 7.28-7.31 (3 H, m); 7.36-7.40 (2 H, m). |
| | | | [MH-NHMe2]+=326.3 (100 %), Rt = 2.9 min. | 13C-NMR (CDCl3): 12.2; 29.5; 30.1; 32.9; 36.2; 38.1; 44.9; 49.5; 60.2; 79.7; 126.6; 127.6; 127.7, 174.6. |
| 313 | 2 | Bsp. Nr. 24b/ Alkylierung/ 79% | m/z: [M+H]+ = 351.3, Rt = 2.3 min. | 1H-NMR (CDCl3): 1.19 (3 H, t, J = 7.0 Hz); 1.42-1.51 (2 H, m); 1.73-1.82 (2 H, m); 1.93-2.07 (4 H, m); 2.11 (6 H, s); 2.18 (2 H, s); 3.35 (2 H, s); 3.44 (2 H, t, J = 5.3 H); 3.48 (2 H, q, J = 7.0 Hz); 3.52-3.57 (2 H, m); 6.85 (1 H, dd, J = 3.5 und 1.1 Hz); 7.05 (1 H, dd, J = 5.1 und 3.5 Hz); 7.24 (1 H, dd, J = 5.1 und 1.1 Hz). |
| | | | | 13C-NMR (CDCl3): 15.2; 32.6; 32.7; 35.8; 38.1; 42.4; 44.2; 59.3; 66.2; 68.5; 123.4; 124.9; 126.2; 173.8. |
| 314 | 1 | Bsp. Nr. 18/ Acylierung/ 47% | m/z: [M+H]+ = 379.3, Rt = 2.8 min. | 1H-NMR (CDCl3): 1.25 (2 H, s); 1.32 (6 H, s); 1.36-1.46 (2 H, m); 1.58-1.74 (4 H, m); 1.84-2.06 (1.5 H, m); 2.09 (2.5 H, s); 2.11 (3.5 H, s); 2.14-2.24 (0.5 H, m); 2.45 (0.7 H, s); 2.46 (1.3 H, s); 3.21 (1.4 H, s); 3.22 (1.6 H, s); 3.36 (0.8 H, s); 3.40 (1.2 H, s); 3.47 (1.2 H, t, J = 7.3 Hz); 3.53 (0.8 H, t, J = 7.2 Hz); 6.83 (1 H, m); 7.04 (1 H, m); 7.24 (1 H, m). |
| | | | | 13C-NMR (CDCl3): 19.1; 24.6; 24.8; 29.7; 31.0; 32.8; 33.2; 35.7; 37.0; 38.08; 38.10; 40.0; 42.0; 43.9; 45.0; 45.5; 45.8; 49.2; 49.3; 53.4; 55.6; 57.0; 59.7; 59.8; 74.9; 75.0; 123.3; 123.4; 124.8; 125.0; 126.1; 126.3; 143.7; 169.3; 169.4. |
| 315 | 1 | Bsp. Nr. 18/ Acylierung/ 48% | m/z: [M+H]+ = 351.3, Rt = 2.6 min. | 1H-NMR (CDCl3): 1.32-1.46 (2 H, m); 1.56-1.74 (4 H, m); 1.84-2.22 (4 H, m); 2.09 (3.5 H, s); 2.11 (2.5 H, s); 2.51 (2 H, t, J = 6.4 Hz); 3.34 (1.2 H, s); 3.35 (3 H, s); 3.37 (0.8 H, s); 3.47 (2 H, dd, J = 6.8 und 13.8 Hz); 3.60-3.73 (2 H, m); 6.83-6.86 (1 H, m); 7.01-7.06 (1 H, m); 7.21-7.25 (1 H, m). |
| | | | | 13C-NMR (CDCl3): 31.0; 31.2; 32.9; 33.3; 34.6; 35.0; 35.4; 36.9; 38.1; 40.0; 42.0; 43.9; 45.1; 56.6; 58.8; 58.9; 59.9; 68.5; 123.2; 123.5; 124.9; 125.0; 126.0; 126.3; 169.6; 169.7. |
| 316 | 1 | Bsp. Nr. 131/ N-Demethylier ung/ 12% | m/z: [MH-HNMe]+= 302.3, Rt = 2.8 min. | 1H-NMR (CDCl3): 0.00-0.05 (2 H, m); 0.40-0.45 (2 H, m); 0.55-0.66 (1 H, m); 1.38 (2 H, dd, J = 14.4 und 7.0 Hz); 1.43-1.57 (3 H, m); 1.69-1.81 (2 H, m); 1.82-2.04 (4 H, m); 2.11 (3 H, s); 2.30 (2 H, s); 3.13 (2 H, s); 3.28 -3.34 (2 H, m); 6.88 (1 H, dd, J = 3.5 und 1.1 Hz); 6.95 (1 H, dd, J = 5.1 und 3.5 Hz); 7.21 (1 H, dd, J = 5.1 und 1.1 Hz). |
| | | | | 13C-NMR (CDCl3): 4.3; 8.5; 28.7; 32.4; 32.7; 33.7; 35.7; 38.1; 42.6; 44.5; 56.3; 58.1; 123.7; 126.3; 173.4. |
| 317 | 2 | Bsp. Nr. 24b/ Alkylierung/ 72% | m/z: [M+H]+ = 365.3, Rt = 2.4 min. | 1H-NMR (CDCl3): 1.16 (3 H, d, J = 6.1 Hz); 1.42-1.51 (2 H, m); 1.56-1.81 (4 H, m); 1.95-2.09 (4 H, m); 2.10 (6 H, s); 2.18 (2 H, s); 3.21 (2 H, s); 3.30-3.38 (3 H, m); 3.31 (3 H, s); 6.85 (1 H, d, J = 3.5 Hz); 7.04 (1 H, dd, J = 5.0 und 3.6 Hz); 7.24 (1 H, d, J = 5.0 Hz). |
| | | | | 13C-NMR (CDCl3): 19.0; 32.8; 34.1; 35.6; 38.1; 39.4; 44.3; 56.1; 58.4; 59.3; 74.7; 123.4; 124.8; 126.3; 173.6. |
| 318 | 2 | Bsp. Nr. 24b/ Alkylierung/ 54% | m/z: [M+H]+ = 351.3, Rt = 2.2 min. | 1H-NMR (CDCl3): 1.12 (3 H, d, J = 6.2 Hz); 1.40-1.51 (2 H, m); 1.72-1.82 (2 H, m); 1.95-2.09 (4 H, m); 2.10 (6 H, s); 2.18 (2 H, s); 3.23 (1 H, dd, J = 14.1 und 6.8 Hz); 3.31-3.37 (3 H, m); 3.32 (3 H, s); 3.48-3.55 (1 H, m); 6.85 (1 H, d, J = 3.3 Hz); 7.05 (1 H, dd, J = 5.1 und 3.5 Hz); 7.24 (1 H, br d, J = 5.1 Hz). |
| | | | | 13C-NMR (CDCl3): 16.8; 32.6; 32.7; 32.8; 35.8; 39.1; 44.0; 47.4; 56.1; 59.4; 75.9; 123.4; 124.9; 126.3; 174,1. |
| 319 | 2 | Bsp. Nr. 162 Stufe3/ Alkylierung/ 12% | m/z: [M+H]+ = 354.3, Rt = 2.5 min. | 1H-NMR (CDCl3): 1.35-1.48 (4 H, m); 1.37 (6 H, s); 1.64-1.88 (2 H, m); 2.00-2.16 (2 H, m); 2.02 (6 H, s); 2.17 (2 H, s); 3.39 (2 H, s); 3.55 (2 H, s); 7.23-7.32 (3 H, m); 7.34-7.41 (2 H, m). |
| | | | | 13C-NMR (CDCl3): 24.8; 29.9; 32.4; 33.0; 36.3; 37.9; 43.5; 49.0; 50.2; 51.1; 59.5; 59.7; 124.6; 126.7; 127.3; 127.7; 175.3. |
| 320 | 1 | Bsp. Nr. 71/ Acylierung/ 40% | m/z: [M+H]+ = 373.4, Rt = 2.9 min. | 1H-NMR (CDCl3): 1.24-1.31 (2 H, m); 1.33 (6 H, s); 1.48-1.70 (4 H, m); 1.74-2.00 (2 H, m); 2.02 (2.6 H, s); 2.04 (3.4 H, s); 2.13-2.25 (0.8 H, m); 2.28-2.44 (1.2 H, m); 2.45 (0.8 H, s); 2.47 (1.2 H, s); 3.20 (1.2 H, s); 3.22 (1.8 H, s); 3.38-3.54 (4 H, m); 7.22-7.42 (5 H, m). |
| | | | | 13C-NMR (CDCl3): 24.6; 24.8; 29.9; 30.6; 31.0; 31.4; 36.1; 37.6; 38.0; 40.3; 42.3; 43.8; 44.9; 45.5; 45.6; 49.19; 49.23; 55.2; 57.0; 60.6; 60.7; 74.7; 74.8; 126.3; 126.5; 127.57; 127.59; 127.63; 127.70; 137.5; 169.3; 169.4. |
| 321 | 1 | Bsp. Nr. 24a/ Alkylierung/ 64% | m/z: [M+H]+= 377.3 (1 %) | 1H-NMR (CDCl3): 1.42-1.49 (2 H, m); 1.64-1.78 (4 H, m); 1.82-1.89 (2 H, m); 1.92-2.01 (2 H, m); 2.02-2.09 (4 H, m); 2.10 (6 H, s); 2.32 (2 H, s); 3.13 (3 H, s); 3.18 (2 H, s); 3.46 (2 H, s); 6.85 (1 H, dd, J = 3.6 und 1.1 Hz); 7.04 (1 H, dd, J = 5.1 und 3.6 Hz); 7.24 (1 H, dd, J = 5.1 und 1.1 Hz). |
| | | | [MH-NHMe2]+= 332.3 (100 %), Rt = 2.8 min. | 13C-NMR (CDCl3): 12.2; 29.5; 32.7; 35.9; 38.1; 45.0; 49.5; 59.9; 79.7; 123.5; 125.0; 126.3, 174.6. |
| 322 | 2 | Bsp. Nr. 162 Stufe3/ Alkylierung/ 40% | m/z: [M+H]+= 371.3 (100 %) | 1H-NMR (CDCl3): 1.31-1.38 (2 H, m); 1.66-1.81 (4 H, m); 1.87-1.93 (2 H, m); 1.95-2.03 (2 H, m); 2.06 (6 H, s); 2.09-2.15 (4 H, m); 2.35-2.46 (2 H, m); 3.20 (3 H, s); 3.40 (2 H, s); 3.50 (2 H, s); 7.28-7.32 (3 H, m); 7.38-7.41 (2 H, m). |
| | | | [MH-NHMe2]+= 326.3 (73 %), Rt = 2.5 min. | 13C-NMR (CDCl3): 12.2; 29.6; 30.0; 32.8; 35.9; 37.9; 44.2; 45.0; 49.6; 58.7; 79.8; 126.9; 127.7; 127.9, 174.6. |
| 323 | 2 | Bsp. Nr. 162 Stufe3/ Alkylierung/ 51% | m/z: [M+H]+ = 345.4, Rt = 2.3 min. | 1H-NMR (CDCl3): 1.13 (3 H, d, J = 6.2 Hz); 1.32-1.42 (2 H, m); 1.70-1.79 (2 H, m); 1.86-2.03 (2 H, m); 2.04 (6 H, s); 2.12 (2 H, d, J = 1.3 Hz); 2.15-2.26 (2 H, m); 3.24 (1 H, dd, J = 14.0 und 6.8 Hz); 3.30-3.43 (3 H, m); 3.33 (3 H, s); 3.48-3.56 (1 H, m); 7.26-7.32 (3 H, m); 7.36-7.42 (2 H, m). |
| | | | | 13C-NMR (CDCl3): 16.8; 30.1; 32.8; 36.0; 38.0; 44.4; 47.3; 56.1; 59.7; 75.9; 126.8; 127.5; 127.8; 174.1. |
| 324 | 2 | Bsp. Nr. 162 Stufe3/ Alkylierung/ 69% | m/z: [M+H]+ = 359.4, Rt = 2.5 min. | 1H-NMR (CDCl3): 1.16 (3 H, d, J = 6.1 Hz); 1.32-1.42 (2 H, m); 1.55-1.80 (5 H, m); 1.90-2.00 (2 H, m); 2.04 (6 H, s); 2.12 (2 H, s); 2.14-2.28 (1 H, m); 3.26 (2 H, s); 3.30-3.37 (3 H, m); 3.32 (3 H, s); 7.26-7.32 (3 H, m); 7.35-7.42 (2 H, m). |
| | | | | 13C-NMR (CDCl3): 19.0; 30.1; 32.9; 34.1; 35.7; 38.0; 39.4; 44.6; 56.1; 58.1; 74.7; 126.6; 127.4; 127.8; 173.6. |
| 325 | 2 | Bsp. Nr. 162 Stufe3/ Alkylierung/ 53% | m/z: [M+H]+ = 331.3, Rt = 2.2 min. | 1H-NMR (CDCl3): 1.32-1.41 (2 H, m); 1.470-1.79 (2 H, m); 1.70-2.02 (2 H, m); 2.04 (6 H, s); 2.12 (2 H, s); 2.13-2.30 (2 H, m); 3.34 (3 H, s); 3.37 (2 H, s); 3.42-3.47 (2 H, m); 3.48-3.53 (2 H, m); 7.27-7.32 (3 H, m); 7.35-7.41 (2 H, m). |
| | | | | 13C-NMR (CDCl3): 30.1; 32.8; 36.0; 38.0; 42.2; 44.4; 58.6; 59.0; 70.7; 126.7; 127.5; 127.8; 173.9. |
| 326 | 2 | Bsp. Nr. 162 Stufe3/ Alkylierung/ 65% | m/z: [M+H]+ = 345.4, Rt = 2.3 min. | 1H-NMR (CDCl3): 1.20 (3 H, t, J = 7.0 Hz); 1.30-1.41 (2 H, m); 1.54-1.56 (2 H, m); 1.99-2.00 (2 H, m); 2.00 (6 H, s); 2.10 (2 H, s); 2.20 (2 H, br s); 3.38 (2 H, s); 3.44 (2 H, t, J = 5.3 Hz); 3.48 (2 H, q, J = 7.0 Hz); 3.52-3.57 (2 H, m); 7.26-7.32 (3 H, m); 7.35-7.41 (2 H, m). |
| | | | | 13C-NMR (CDCl3): 15.2; 30.1; 32.8; 36.0; 38.0; 42.4; 44.5; 59.2; 60.2; 66.2.; 68.6; 126.7; 127.5; 127.8; 173.8. |
| 327 | 1 | Bsp. Nr. 18/ Acylierung/ 42% | m/z: [M+H]+ = 365.3, Rt = 2.7 min. | 1H-NMR(CDCl3): 1.20 (3 H, d, J = 6.1 Hz); 1.33-1.46 (2 H, m); 1.57-1.74 (4 H, m); 1.82-2.20 (4 H, m); 2.08 (2.5 H, s); 2.11 (3.5 H, s); 2.20-2.27 (1 H, m); 2.58 (1 H, dd, J = 7.2 und 14.8 Hz); 3.24-3.58 (7 H, m); 3.82-3.94 (1 H, m); 6.83-6.86 (1 H, m); 7.01-7.06 (1 H, m); 7.21-7.25 (1 H, m). |
| | | | | 13C-NMR (CDCl3): 19.46; 19.52; 30.9; 31.0; 31.2; 32.7; 33.1; 33.4; 38.1; 40.0; 41.5; 41.9; 42.0; 43.9; 45.3; 56.4; 56.5; 59.7; 74.26; 74.29; 123.3; 123.4; 124.7; 124.9; 126.0; 126.3; 169.9. |
| 328 | 1 | Bsp. Nr. 71/ Acylierung/ 40% | m/z: [M+H]+: = 359.4, Rt = 2.8 min. | 1H-NMR (CDCl3): 1.21 (1.3 H, d, J = 6.1 Hz); 1.22 (1.7 H, J = 6.1 Hz); 1.24-1.36 (2 H, m); 1.54 (1.2 H, t, J = 7.3 Hz); 1.58-1.70 (2.8 H, m); 1.72-2.00 (2 H, m); 2.02 (2.6 H, s); 2.03 (3.4 H, s); 2.12-2.40 (3 H, m); 2.52-2.64 (1 H, m); 3.24-3.56 (7 H, m); 3.80-3.94 (1 H, m); 7.20-7.44 (5 H, m). |
| | | | | 13C-NMR (CDCl3): 19.46; 19.53; 30.0; 30.1; 30.5; 30.8; 31.0; 31.3; 31,36; 31.40; 35.9; 37.5; 38.0; 38.1; 40.4; 41.5; 42.0; 42.3; 43.8; 45.1; 55.2; 56.3; 56.5; 60.8; 74.3; 77.5; 126.5; 126.7; 127.58; 127.60; 127.67; 127.73; 169.8; 169.9. |
| 329 | 1 | Bsp. Nr. 71/ Acylierung/ 50% | m/z: [M+H]+ = 345.3, Rt = 2.7 min. | 1H-NMR (CDCl3): 1.22-1.36 (2 H, m); 1.50-1.70 (4 H, m); 1.78-2.00 (2 H, m); 2.02 (2.8 H, s); 2.03 (3.2 H, s); 2.14-2.40 (2 H, m); 2.51 (2 H, dd, J = 12.5 und 6.3 Hz); 3.348 (1.4 H, s); 3.353 (1.6 H, s); 3.37 (1.2 H, s); 3.41 (0.8 H, s); 3.35 (2 H, dd, J = 14.5 und 7.3 Hz); 3.68-3.73 (2 H, m); 7.22-7.40 (5 H, m). |
| | | | | 13C-NMR (CDCl3): 30.1; 30.6; 31.2; 31.4; 34.6; 35.0; 38.0; 38.1; 40.4; 42.3; 43.8; 45.1; 58.7; 58.8; 68.58; 68.60; 126.5; 126.6; 127.55; 127.60; 127.65; 127.71; 169.7. |
| 330 | 1 | Bsp. Nr. /168 N-Demethylier ung/ 31% | [M+H]+ = 327.3 (22 %) | 1H-NMR (CDCl3): 0.04 (2 H, m); 0.39-0.45 (2 H, m); 0.56-0.66 (1 H, m); 1.37 (3 H, dd, J = 14.4 und 7.0 Hz); 1.43-1.53 (2 H, m); 1.73-1.88 (4 H, m); 1.94 (1 H, s); 1.99 (3 H, s); 2.32 (2 H, s); 3.13 (2 H, s); 3.27-3.33 (2 H, m); 7.20-7.26 (1 H, m); 7.31-7.37 (4 H, m). |
| | | | [MH-HNMe]+ = 296.3 (100 %),Rt = 2.9 min. | 13C-NMR (CDCl3): 4.3; 8.5; 28.6; 31.9; 32.4; 32.6; 35.3; 35.7; 42.6; 45.0; 56.7; 57.8; 126.0; 126.5; 128.3; 145.1; 173.5. |
| 331 | 2 | Bsp. Nr. 173/ N-Demethylier ung/ 60% | m/z: [M+H]+ = 341.4 (22 %) | 1H-NMR (CDCl3): 1.40 (1 H ,br s); 1.44-1.53 (2 H, m); 1.56-1.69 (4 H, m); 1.72-1.98 (8 H, m); 2.00 (3 H, s); 2.02-2.12 (1 H, m); 2.23 (2 H, s); 2.24-2.31 (1 H, m); 3.15-3.20 (4 H, m); 7.19-7.27 (2 H, m); 7.34-7.38 (4 H, m). |
| | | | [MH-HNMe]+ = 310.3 (100 %),Rt = 2.5 min. | 13C-NMR (CDCl3): 18.6; 28.2; 28.7; 31.9; 32.5; 33.7; 34.3; 35.8; 40.5; 43.1; 56.8; 59.5; 125.9; 126.5; 128.3; 173.4. |
| 332 | 2 | Bsp. Nr. 24b/ Alkylierung/ 36% | m/z: [M+H]+= 365.3 (98 %) | 1H-NMR (CDCl3): 1.15 (6 H, s); 1.43-1.49 (2 H, m); 1.74-1.80 (2 H, m); 1.97-2.08 (4 H, m); 2.10 (6 H, s); 2.18 (2 H, s); 3.20 (3 H; s); 3.26 (2 H; s); 3.41 (2 H, s); 6.85 (1 H, dd, J = 3.6 und 0.9 Hz); 7.04 (1 H, dd, J = 5.1 und 3.6 Hz); 7.24 (1 H, dd, J = 5.0 und 0.8 Hz). |
| | | | [MH-NHMe2]+= 320.3 (100 %), Rt = 2.4 min. | 13C-NMR (CDCl3): 22.7; 32.65; 32.74; 35.6; 38.1; 43.9; 49.2; 50.5; 59.4; 76.2; 123.4; 124.9; 126.3; 174.4. |
| 333 | 1 | Bsp. Nr.50/ N-Demethylier ungl 29% | m/z: [M+H]+ = 347.3 (26 %) | 1H-NMR (CDCl3):1.39-1.51 (3 H, m); 1.64-1.93 (10 H, m); 1.94-2.09 (2 H, m); 2.11 (1,4 H, s); 2.13 (1,6 H, s); 2.13-2.20 (2 H, m); 2.36 (1 H, d, J = 7.4 Hz); 2.37 (1 H, d, J = 7.4 Hz); 2.68-2.80 (1 H, m); 3.28 (1,2 H, s); 3.33 (0,8 H, s); 3.43-3.51 (2 H, m); 6.88-6.91 (1 H, m); 6.96 (1 H, dt, J = 5.1 und 3.5 Hz); 7.22 (1 H, ddd, J = 5.1, 3.3 und 1.15Hz). |
| | | | [MH-HNMe]+ = 316.3 (100 %), Rt = 2.9 min. | 13C-NMR (CDCl3): 18.74; 18.76; 28.58; 28.62; 28.65; 28.68; 30.79; 30.95; 32.23; 32.37; 34.17; 34.18; 35.82; 38.10; 40.25; 41.24; 41.68; 42.31; 43.90; 45.20; 56.19; 56.76; 56.83; 57.6; 123.70; 123.72; 126.29; 126.44; 171.08. |
| 334 | 2 | Bsp. Nr. 24b/ Alkylierung/ 69% | m/z: [M+H]+ = 365.3, Rt = 2.4 min. | 1H-NMR (CDCl3): 1.12 (3 H, d, J = 6.2 Hz); 1.19 (3 H, t, J = 7.0 Hz); 1.41-1.51 (2 H, m); 1.73-1.82 (2 H, m); 1.95-2.09 (4 H, m); 2.10 (6 H, s); 2.18 (2 H, s); 3.17 (1 H, dd, J = 14.0 und 7.2 Hz); 3.31-3.44 (4 H, m); 3.52-3.67 (2 H, m); 6.85 (1 H, br d, J = 3.0 Hz); 7.04 (1 H, dd, J = 5.1 und 3.5 Hz); 7.24 (1 H, br d, J = 5.2 Hz). |
| | | | | 13C-NMR (CDCl3): 15.7; 17.6; 32.7; 35.8; 38.1; 44.0; 47.9; 59.3; 60.2; 63.9; 74.1; 123.4; 124.8; 126.3; 174.0. |
| 335 | 2 | Bsp. Nr. 162 Stufe3/ Alkylierung/ 47% | m/z: [M+H]+ = 359.4, Rt = 2.5 min. | 1H-NMR (CDCl3): 1.13 (3 H, d, J = 6.2 Hz); 1.20 (3 H, t, J = 7.0 Hz); 1.30-1.44 (2 H, m); 1.71-1.79 (2 H, m); 1.90-2.02 (2 H, m); 2.03 (6 H, s); 2.12 (2 H, d, J = 1.7 Hz); 2.13-2.25 (2 H, m); 3.18 (1 H, dd, J = 14.0 und 7.2 Hz); 3.33-3.47 (4 H, m); 3.53-3.68 (2 H, m); 7.26-7.32 (3 H, m); 7.35-7.41 (2 H, m). |
| | | | | 13C-NMR (CDCl3): 15.7; 17.6; 30.1; 32.8; 35.9; 38.0; 44.4; 47.9; 60.1; 63.9; 74.1; 126.7; 127.5; 127.7; 174.0. |
| 336 | 2 | Bsp. Nr. 153/ Alkylierung/ 71% | m/z: [M+H]+ = 393.4, Rt = 2.7 min. | 1H-NMR (CDCl3): 1.18 (6 H, s); 1.41-1.50 (2 H, m); 1.63-1.76 (4 H, m); 1.85-1.97 (2 H, m); 1.99-2.09 (2 H, m); 2.12 (6 H, s); 2.16 (2 H, s); 2.46 (3 H, d, J = 1.0 Hz); 3.19 (3 H, s); 3.21 (2 H, s); 3.28-3.34 (2 H, m); 6.61 (1 H, d, J = 3.5 Hz); 6.66-6.69 (1 H, m). |
| | | | | 13C-NMR (CDCl3): 15.2; 24.9; 32.7; 32.8; 35.5; 36.7; 38.1; 38.2; 44.5; 49.1; 49.2; 57.9; 59.4; 73.5; 124.5; 124.9; 137.9; 173.4. |
| 338 | 1 | Bsp. Nr. 71/ Acylierung/ 30% | m/z: [M+H]+ = 373.4, Rt = 3.0 min. | 1H-NMR (CDCl3): 1.15 (1.4 H, t, J = 7.0 Hz); 1.17 (1.6 H, t, J = 7.0 Hz); 1.21 (1.4 H, d, J = 6.1 Hz); 1.22 (1.6 H, d, J = 6.1 Hz); 1.24-1.36 (2 H, m); 1.50-1.70 (4 H, m); 1.80-2.0 (2 H, m); 2.02 (3 H, s); 2.03 (3 H, s); 2.14-2.40 (2 H, m); 2.23 (0.5 H, dd, J = 5.7 und 2.2 Hz, überlagert); 2.26 (0.5 H, dd, J = 5.7 und 2.1 Hz, überlagert); 2.58 (0.6 H, dd, J = 13.8 und 6.4 Hz); 2.60 (0.4 H, dd, J = 13.9 und 7.0 Hz); 3.26-3.70 (6 H, m); 3.90-4.01 (1 H, m); 7.20-7.42 (5 H, m). |
| | | | | 13C-NMR (CDCl3): 15.61; 15.63; 20.28; 20.32; 29.5; 30.0; 30.7; 30.8; 31.0; 31.4; 36.0; 37.4; 38.0; 38.1; 40.4; 42.0; 42.1; 42.3; 43.8; 45.3; 53.4; 55.1; 56.7; 60.7; 64.09; 64.13; 72.7; 72.8; 126.5; 126.6; 127.58; 127.61; 127.67; 127.72; 137.5; 169.9; 170.1. |
| 339 | 1 | Bsp. Nr. 18/ Acylierung/ 40% | m/z: [M+H]+ = 379.3, Rt = 2.9 min. | 1H-NMR (CDCl3): 1.151 (1.4 H, t, J = 7.0 Hz); 1.158 (1.6 H, t, J = 7.0 Hz); 1.21 (3 H, d, J = 6.1 Hz); 1.33-1.46 (2 H, m); 1.57-1.74 (4 H, m); 1.76-2.20 (4 H, m); 2.09 (2.5 H, s); 2.10 (3.5 H, s); 2.22 (0.4 H, dd, J = 5.7 und 2.1 Hz); 2.26 (0.6 H, dd, J = 5.7 und 2.1 Hz); 2.58 (1 H, dd, J = 7.4 und 14.5 Hz); 3.20-3.70 (6 H, m); 3.90-4.00 (1 H, m); 6.83-6.87 (1 H, m); 7.00-7.06 (1 H, m); 7.21-7.25 (1 H, m). |
| | | | | 13C-NMR (CDCl3): 15.61; 15.63; 20.27; 20.32; 30.9; 31.1; 31.2; 32.80; 32.83; 33.28; 33.35; 35.6; 36.8; 38.08; 38.09; 40.0; 41.9; 42.0; 42.3; 43.8; 45.2; 53.4; 55.4; 56.7; 59.9; 64.09; 64.13; 72.6; 72.8; 123.3; 123.4; 124.7; 124.9; 126.0; 126.3; 169.9; 170.1. |
| 340 | 2 | Bsp. Nr. 162 Stufe3/ Alkylierung/ 33% | m/z: [M+H]+= 359.4 (100 %) | 1H-NMR (CDCl3): 1.15 (6 H, s); 1.34-1.41 (2 H, m); 1.72-1.78 (2 H, m); 1.92-2.06 (2 H, m, überlagert); 2.04 (6 H, s); 2.12 (2 H, s); 2.15-2.25 (2 H, m); 3.21 (3 H; s); 3.27 (2 H; s); 3.45 (2 H, s); 7.28-7.30 (3 H, m); 7.37-7.41 (2 H, m). |
| | | | [MH-NHMe2]+= 314.3 (99 %), Rt= 2.4 min. | 13C-NMR (CDCl3): 22.7; 30.2; 32.8; 35.8; 38.0; 44.2; 49.2; 50.4; 60.3; 76.2; 126.7; 127.5; 127.7; 174.5. |
| 341 | 2 | Bsp. Nr. 217/ N-Demethylier ung/ 40% | m/z: [M+H]+ = 377.3 (100 %) | 1H-NMR (CDCl3): 1.43 (1 H, s); 1.49-1.66 (3 H, m); 1.68-2.05 (11 H, m); 2.05-2.19 (2 H, m); 2.12 (3 H, s); 2.24 (2 H, s); 3.16 (3 H, s); 3.23 (2 H, s); 3.24-3.30 (2 H, m); 6.88 (1 H, dd, J = 3.5 und 1.1 Hz); 6.97 (1 H, dd, J = 5.1 und 3.5 Hz); 7.22 (1 H, dd, J = 5.1 und 1.0 Hz). |
| | | | [MH-HNMe]+ = 346.3 (50 %), Rt = 2.5 min. | 13C-NMR (CDCl3): 12.5; 28.8; 31.3; 32.0; 32.5; 33.7; 35.8; 37.8; 43.5; 49.4; 56.3; 78.3; 123.7; 126.5; 173.4. |
| 342 | 1 | Bsp. Nr. 71/ Acylierung/ 71% | [M+H]+: m/z = 341.3, Rt = 2.9 min. | 1H-NMR (CDCl3): 1.22-1.36 (2 H, m); 1.48-1.68 (4 H, m); 1.72-2.00 (4 H, m); 2.02 (2.6 H, s); 2.03 (3.4 H, s); 2.06-2.42 (6 H, m); 3.01-3.21 (1 H, m); 3.23 (1.2 H, s); 3.32 (0.8 H, t, J = 7.1 Hz); 3.39 (0.8 H, s); 3.44 (1.2 H, t, J = 7.2 Hz); 7.20-7.42 (5 H, m). |
| | | | | 13C-NMR (CDCl3): 18.1; 24.7; 24.65; 24.71; 25.4; 25.4; 30.1; 30.9; 31.0; 31.5; 35.7; 38.0; 38.1; 38.2; 38.3; 39.1; 40.1; 42.2; 43.8; 44.2; 47.8; 55.3; 55.5; 60.7; 126.5; 126.7; 127.59; 127.63, 127.66; 127.73; 137.3; 173.4. |
| 343 | 1 | Bsp. Nr. 71/ Acylierung/ 86% | [M+H]+: m/z = 329.4, Rt = 2.8 min. | 1H-NMR (CDCl3): 1.11 und 1.13 (6 H, 2 d, J = jeweils 4.5 Hz); 1.20-1.38 (2 H, m); 1.54 (1.2 H, t, J = 7.3 Hz); 1.58-1.70 (2.8 H, m); 1.74-2.00 (2 H, m); 2.02 (2.6 H, s); 2.04 3.4 H, s); 2.14-2.44 (2 H, m); 2.54-2.68 (1 H, m); 3.28 (1.2 H, s); 3.40 (0.8 H, s); 3.42-3.50 (2 H, m); 7.22-7.44 (5 H, m). |
| | | | | 13C-NMR (CDCl3): 19.07; 19.08; 30.1; 30.9; 31.0; 31.5; 32.0; 32.2; 35.8; 37.7; 38.0; 38.1; 40.2; 42.1; 44.0; 44.7; 53.4; 55.2; 56.0; 60.8; 126.5; 126.7; 127.58; 127.62; 127.66; 127.75; 137.5; 175.79; 175.83. |
| 344 | 2 | Bsp. Nr. 24b/ Alkylierung/ 68% | m/z: [M+H]+ = 358.3, Rt = 2.2 min. | 1H-NMR (CDCl3): 1.07 (2 H, dd, J = 7.3 und 5.1 Hz); 1.28 (2 H, dd, J = 7.3 und 5.1 Hz); 1.46-1.56 (2 H, m); 1.80-1.92 (2 H, m); 2.00-2.15 (4 H, m); 2.10 (6 H, s); 2.23 (2 H,s); 3.35 (2 H, s); 3.47 (2 H, s); 6.85 (1 H, dd, J = 3.5 und 1.0 Hz); 7.05 (1 H, dd, J = 5.1 und 3.5 Hz); 7.24 (1 H, dd, J = 5.1 und 1.0 Hz). |
| | | | | 13C-NMR (CDCl3): 9.3; 13.4; 32.5; 32.6; 36.1; 38.0; 41.0; 43.6; 46.9; 58.6; 59.1; 122.3; 123.3; 124.7; 126.3; 142.8; 174.4. |
| 345 | 1 | Bsp. Nr. 71/ Acylierung/ 19% | [M+H]+ = 357.3, Rt = 2.7 min. | 1H-NMR (CDCl3): 0.88-0.98 (2 H, m); 1.08-1.16 (2 H, m); 1.27-1.37 (2 H, m); 1.50-1.58 (1 H, m); 1.58-1.72 (3 H, m); 1.85-2.01 (2 H, m); 2.05 (6 H, s); 2.16-2.39 (2 H, m); 3.28 (3 H, s); 3.44 (0.8 H, s); 3.49 (1.2 H, t, J = 7.4 Hz); 3.67 (1.2 H, s); 3.74 (0.8 H, t, J = 7.2 Hz); 7.26-7.43 (5 H, m). |
| | | | | 13C-NMR (CDCl3): 12.3; 12.5; 29.7; 30.2; 30.6; 31.0; 31.5; 35.3; 38.0; 38.1; 39.7; 42.1; 44.8; 45.2; 56.1; 56.2; 56.5; 64.1; 1266.; 127.6; 127.7; 169.7. |
| 346 | 1 | Bsp. Nr. 333/ Reduktion/ 48% | m/z: [M+H]+ = 333.3, | 1H-NMR (CDCl3): 1.43-1.51 (2 H, m); 1.54-1.71 (8 H, m); 1.77-1.88 (4 H, m); 1.90-2.08 (5 H, m); 2.11 (3 H, s); 2.21-2.30 (3 H, m); 2.39 (2 H, s); 2.52 (2 H, t, J = 6.9 Hz); 6.88 (1 H, dd, J = 3.5 und1.1 Hz); 6.94 (1 H, dd, J = 5.1 und 3.5 Hz); 7.20 (1 H, dd, J = 5.1 und 1.1 Hz). |
| | | | [MH-HNMe]+ = 302.3, Rt = 1.3 und 1.7 min. | 13C-NMR (CDCl3): 18.7; 28.4; 28.7; 34.1; 34.5; 36.1; 36.4; 41.0; 54.2; 54.8; 56.5; 66.9; 123.5; 126.2. |
| 347 | 2 | Bsp. Nr. 24b/ Alkylierung/ 67% | m/z: [M+H]+ = 391.3, Rt = 2.4 min. | 1H-NMR (CDCl3): 1.22-1.35 (2 H, m); 1.40-1.50 (5 H, m); 1.60-1.68 (2 H, m); 1.70-1.81 (2 H, m); 1.95-2.09 (4 H, m); 2.10 (6 H, s); 2.13 (2 H, s); 3.19 (2 H, s); 3.30 (2 H, t, J = 7.2 Hz); 3.36 (2 H, dt, J = 11.8 und 2.0 Hz); 3.94 (2 H, br dd, J = 10.5 und 3.2 Hz); 6.85 (1 H, d, J = 3.2 Hz); 7.05 (1 H, dd, J = 5.0 und 3.6 Hz); 7.24 (1 H, d, J = 4.9 Hz). |
| | | | | 13C-NMR (CDCl3): 32.7; 32.8; 32.9; 34.1; 35.6; 38.0; 39.6; 44.2; 57.8; 59.2; 67.9; 123.4; 124.9; 126.3; 173.5. |
| 348 | 2 | Bsp. Nr. 162 Stufe3/ Alkylierung/ 52% | m/z: [M+H]+ = 385.4, Rt = 2.4 min. | 1H-NMR (CDCl3): 1.23-1.52 (7 H, m); 1.60-1.68 (2 H, m); 1.69-1.79 (2 H, m); 1.80-2.03 (2 H, m); 2.05 (6 H, s); 2.13 (2 H, s); 2.14-2.30 (2 H, m); 3.24 (2 H, s); 3.30 (2 H, t, J = 7.2 Hz); 3.36 (2 H, dt, J = 11.8 und 2.2 Hz); 3.95 (2 H, br dd, J = 10.6 und 4.5 Hz); 7.27-7.33 (3 H, m); 7.36-7.42 (2 H, m). |
| | | | | 13C-NMR (CDCl3): 30.2; 32.7; 32.8; 32.9; 34.1; 35.7; 38.0; 39.6; 44.5; 57.7; 60.1; 67.9; 126.8; 127.5; 127.8; 173.6. |
| 349 | 2 | Bsp. Nr. 162 Stufe3/ Alkylierung/ 70% | m/z: [M+H]+ = 352.3, Rt = 2.3 min. | 1H-NMR (CDCl3): 1.07 (2 H, dd, J = 7.5 und 5.1 Hz); 1.29 (2 H, dd, J = 7.4 und 5.1 Hz); 1.37-1.49 (2 H, m); 1.76-1.89 (2 H, m); 1.96-2.12 (2 H, m); 2.03 (6 H, s); 2.12-2.25 (4 H, m); 3.36 (2 H, s); 3.51 (2 H, s); 7.24-7.32 (3 H, m); 7.35-7.42 (2 H, m). |
| | | | | 13C-NMR (CDCl3): 9.3; 13.4; 30.2; 32.7; 36.3; 37.9; 43.7; 46.9; 58.5; 60.0; 122.3; 126.6; 127.4; 127.6; 174.3. |
| 350 | 1 | Bsp. Nr. 71/ Acylierung/ 62% | m/z: [M+H]+ = 385.4, Rt = 2.7 min. | 1H-NMR (CDCl3): 1.23-1.39 (4 H, m); 1.52-1.73 (6 H, m); 1.80-2.01 (2 H, m); 2.03 und 2.06 (6 H, 2 s); 2.10-2.26 (4 H, m); 2.32-2.43 (1 H, m); 3.35 (1 H, s); 3.38-3.49 (5 H, m); 3.90-3.97 (2 H, m); 7.27-7.42 (5 H, m). |
| | | | | 13C-NMR (CDCl3): 30.1; 30.8; 31.2; 31.4; 32.0; 33.10; 33.13; 35.9; 37.5; 38.0; 38.1; 40.3; 41.2; 41.7; 42.3; 43.9; 45.2; 55.2; 56.5; 60.9; 67.9; 126.5; 126.8; 127.6; 127.7; 127.9; 170.4; 170.5. |
| 351 | 1 | Bsp. Nr. 18/ Acylierung/ 78% | m/z: [M+H]+ = 391.3, Rt = 2.7 min. | 1H-NMR (CDCl3): 1.20-1.45 (4 H, m); 1.58-1.64 (1 H, m); 1.64-1.74 (5 H, m); 1.80-2.08 (3 H, m); 2.10 und 2.12 (6 H, 2 s); 2.14-2.26 (4 H, m); 3.31 (1 H, s); 3.36-3.50 (5 H, m); 3.90-3.97 (2 H, m); 6.83-6.88 (1 H, m); 7.01-7.08 (1 H, m); 7.22-7.26 (1 H, m). |
| | | | | 13C-NMR (CDCl3): 31.1; 31.2; 31.9; 32.0; 32.8; 33.10; 33.14; 33.4; 35.5; 37.0; 38.1; 40.1; 41.3; 41.7; 42.0; 43.9; 45.2; 55.4; 56.6; 59.9; 67.9; 123.3; 123.6; 124.9; 125.1; 126.2; 126.4; 170.40; 170.44. |
| 352 | 1 | Bsp. Nr. 71/ Reduktive Aminierung/ 37% | m/z: [M+H]+ = 338.3, Rt = 0.4 min. | 1H-NMR (CDCl3): 0.84 (2 H, dd, J = 7.1 und 5.1 Hz); 1.24 (2 H, dd, J = 7.1 und 5.0 Hz); 1.26-1.35 (2 H, m); 1.47 (2 H, t; J = 6.9 Hz); 1.62-1.76 (2 H, m); 1.77-1.96 (2 H, m); 2.03 (6 H, s); 2.12-2.40 (2 H, m); 2.47 (2 H, s); 2.54 (2 H, s); 2.59 (2 H, t, J = 6.9 Hz); 7.23-7.41 (5 H, m). |
| | | | | 13C-NMR (CDCl3): 9.7; 13.1; 28.4; 31.2; 34.3; 38.1; 41.4; 53.4; 60.2; 60.6; 65.4; 123.2; 126.3; 127.6. |
| 353 | 2 | Bsp. Nr. 24b/ Alkylierung/ 73% | [M+H]+: m/z = 372.3, Rt = 2.4 min. | 1H-NMR (CDCl3): 1.43-1.53 (2 H, m); 1.77-1.87 (2 H, m); 1.90-2.32 (8 H, m); 2.10 (6 H, s); 2.22 (2 H, s); 2.43-2.53 (2 H, m); 3.42 (2 H, s); 3.62 (2 H, s); 6.84 (1 H, dd, J = 3.5 und 1.1 Hz); 7.04 (1 H, dd, J = 5.1 und 3.5 Hz); 7.24 (1 H, dd, J = 5.1 und 1.0 Hz). |
| | | | | 13C-NMR (CDCl3): 16.7; 30.4; 32.4; 32.6; 35.3; 36.0; 38.0; 42.7; 43.3; 48.2; 53.4; 59.3; 123.3; 123.9; 124.8; 126.3; 175.0. |
| 354 | 1 | Bsp. Nr. 71/ Acylierung/ 51% | m/z: [M+H]+= 354.3 (100 %) [MH-NHMe2]+= 399.4 (87 %), Rt = 4.5 min. | 1H-NMR (CDCl3): 1.25-1.35 (2 H, m); 1.53-1.57 (1 H, m); 1.58-1.69 (3 H, m); 1.70-1.80 (2 H, m); 1.83-1.89 (3 H, m); 1.92-2.00 (1 H, m); 2.01-2.14 (4 H, m, überla-gert); 2.03 (4 H, s); 2.04 (2 H, s); 2.20-2.26 (3 H, m); 2.28-2.37 (1 H, m); 3.11 (1 H, s); 3.13 (2 H, s); 3.35 (1 H, s); 3.41 (1 H, s); 3.46 (2 H, dd, J = 13.4 und 7.0 Hz); 7.27-7.32 (3 H, m); 7.35-7.40 (2 H, m). |
| | | | | 13C-NMR (CDCl3): 12.3; 12.4; 28.1; 28.5; 29.3; 29.4; 30.1; 30.7; 31.2; 31.29; 31.33; 31.5; 36.0; 37.7; 38.0; 38.1; 40.4; 42.3; 44.1; 45.0; 49.2; 49.3, 55.3, 55.4; 78.87; 78.89; 126.5, 126.7; 127.60; 127.65, 127.70; 127.8; 171.9; 172.0. |
| 355 | 1 | Bsp. Nr. 18/ Acylierung/ 36% | m/z: [M+H]+= 405.3 (26 %) [MH-NHMe2]+= 360.3 (100 %), Rt = 4.5 min. | 1H-NMR (CDCl3): 1.35-1.45 (2 H, m); 1.56-1.80 (8 H, m); 1.83-1.89 (2 H, m); 1.92-1.99 (2 H, m); 2.01-2.06 (2 H, m); 2.07-2.13 (2 H, m, überlagert); 2.10 (3 H, s); 2.11 (3 H, s); 2.20-2.26 (2 H, m); 3.11 (1.3 H, s); 3.13 (1.7 H, s); 3.32 (1 H, s); 3.38 (1 H, s); 3.47 (2 H, td, J = 12.0 und 7.2 Hz); 6.85-6.87 (1 H, m); 7.02-7.06 (1 H, m); 7.22-7.26 (1 H, m). |
| | | | | 13C-NMR (CDCl3): 12.4; 28.1; 28.5; 29.3; 29.4; 31.15; 31.22; 31.3; 32.9; 33.3; 38.1; 40.1; 42.1; 44.1; 45.0; 49.2; 78.9; 123.4; 123.5; 124.9; 126.2; 126.3; 171.9. |
| 356 | 1 | Bsp. Nr. 18/ Acylierung/ 21% | [M+H]+: m/z = 363.3, Rt = 2.6 min. | 1H-NMR (CDCl3): 0.89-0.96 (2 H, m); 1.08-1.15 (2 H, m); 1.35-1.46 (2 H, m); 1.57-1.64 (1 H, m); 1.64-1.76 (3 H, m); 1.90-2.05 (2 H, m); 2.05-2.20 (2 H, m); 2.10 und 2.11 (6 H, 2s); 3.28 (3 H, s); 3.40 (0.8 H, s); 3.51 (1.2 H, t, J = 7.4 Hz); 3.62 (1.2 H, s); 3.76 (0.8 H, t, J = 7.1 Hz); 6.85 (1 H, dd, J = 3.6 und 1.1 Hz); 7.02-7.07 (1 H, m); 7.22-7.26 (1 H, m). |
| | | | | 13C-NMR (CDCl3): 12.3; 12.5; 30.8; 31.2; 32.9; 33.2; 34.8; 37.2; 38.1; 39.5; 41.9; 44.8; 45.2; 56.1; 56.2; 56.7; 59.9; 64.1; 64.2; 123.3; 123.4; 124.8; 126.1; 126.2; 169.6. |
| 357 | 2 | Bsp. Nr. 162 Stufe3/ Alkylierung/ | [M+H]+: m/z = 366.3, Rt = 2.4 min. | 1H-NMR (CDCl3): 1.33-1.45 (2 H, m); 1.72-1.85 (2 H, m); 2.01 (6 H, s); 2.03-2.30 (8 H, m); 2.16 (2 H, s); 2.42-2.53 (2 H, m); 3.44 (2 H, s); 3.62 (2 H, s); 7.24-7.31 (3 H, m); 7.34-7.41 (2 H, m). |
| | | 67% | | 13C-NMR (CDCl3): 16.5; 30.0; 30.4; 32.4; 35.3; 36.3; 37.9; 41.0; 43.7; 48.2; 59.0; 59.9; 123.9; 126.7; 127.2; 127.7; 175.1. |
| 358 | 1 | Bsp. Nr. 356/ Reduktion/ 80% | [M+H]+: m/z = 349.3, Rt = 0.3 min. | 1H-NMR (CDCl3): 0.44-0.48 (2 H, m); 0.74-0.80 (2 H, m); 1.34-1.44 (2 H, m); 1.52 (2 H, t, J = 6.8 Hz); 1.66-1.77 (2 H, m); 1.85-2.00 (2 H, m, 2H); 2.1 (6 H, s); 2.06-2.15 (2 H, m); 2.53 (2 H, s); 2.58 (2 H, s); 2.61 (2 H, t, J = 6.8 Hz); 3.34 (3 H, s); 6.85 (1 H, dd, J = 3.6 und 1.1 Hz); 7.03 (1 H, dd, J = 5.1 und 3.6 Hz); 7.22 (1 H, dd, J = 5.1 und 1.1 Hz,). |
| | | | | 13C-NMR (CDCl3): 11.7; 29.7; 33.7; 34.2; 38.1; 41.0; 54.2; 54.6; 59.4; 59.8; 60.3; 66.1; 123.3; 125.0; 126.1. |
| 359 | 1 | Bsp. Nr. 71/ Reduktive Aminierung/ 19% | [M+H]+: m/z = 352.4, Rt = 0.6 min. | 1H-NMR (CDCl3): 1.23-1.33 (2 H, m); 1.44 (2 H, t, J = 6.9 Hz); 1.65-1.75 (2 H, m); 1.80-2.35 (8 H, m); 2.05 (6 H, br s); 2.43-2.52 (2 H, m); 2.57 (2 H, s); 2.62 (2 H, t, J = 6.9 Hz); 2.72 (2 H; s); 7.25-7.42 (5 H, m). |
| | | | | 13C-NMR (CDCl3): 17.00; 25.9; 30.9; 31.3; 34.1; 36.5; 38.0; 41.6; 54.1; 62.1; 66.1; 124.7; 126.5; 127.5; 127.6. |
| 360 | 1 | Bsp. Nr. 18/ Reduktive Aminierung/ 28% | m/z: [M+H]+ = 344.3, Rt = 0.5 min. | 1H-NMR (CDCl3): 0.83 (2 H, dd, J = 7.1 und 5.0 Hz); 1.24 (2 H, dd, J = 7.1 und 5.0 Hz); 1.34-1.44 (2 H, m); 1.53 (2 H, t, J = 6.9 Hz); 1.68-1.80 (2 H, m); 1.84-2.20 (2 H, m); 2.30-2.22 (8 H, m); 2.47 (2 H, s); 2.51 (2 H, s); 2.61 (2 H, t, J = 6.9 Hz); 6.86 (1 H, d, J = 3.2 Hz); 7.04 (1 H, d, J = 5.1 und 3.6 Hz); 7.23 (1 H, d, J = 4.9 Hz). |
| | | | | 13C-NMR (CDCl3): 9.7; 13.0; 33.7; 34.1; 38.0; 41.1; 53.6; 60.0; 123.2; 124.9; 126.2. |
| 361 | 1 | Bsp. Nr. 18/ Reduktive Aminierung/ 20% | [M+H]+: m/z = 358.3, Rt = 0.4 min. | 1H-NMR (CDCl3): 1.33-1.43 (2 H, m); 1.56 (2 H, t, J = 6.9 Hz); 1.65-1.78 (2 H, m); 1.80-2.26 (8 H, m); 2.10 (6 H, s); 2.42-2.55 (2 H, m); 2.52 (2 H, s); 2.64 (2 H, t, J = 6.9 Hz); 3.80 (2 H, s); 6.85 (1 H, br d, J = 3 Hz); 7.03 (1 H, dd, J = 5.0 und 3.6 Hz); 7.23 (1 H, br d, J = 5 Hz). |
| | | | | 13C-NMR (CDCl3):16.6; 16.9; 28.4; 31.3; 33.4; 33.7; 36.6; 37.4; 38.1; 41.3; 54.0; 62.0; 65.9; 123.3; 124.8; 126.0. |
| 362 | 1 | Bsp. Nr. 71/ Acylierung/ 51% | [M+H]+: m/z = 394.4, Rt = 2.9 min. | 1H-NMR (CDCl3): 1.23-1.37 (2 H, m); 1.56 (1 H, t, J = 7.2 Hz); 1.59-1.72 (3 H, m); 1.84-1.99 (2 H, m); 2.024 (2.7 H, s); 2.035 (3.3 H, s); 2.05-2.40 (10 H, m); 2.44-2.57 (2 H, m); 3.34 (1.1 H, s); 3.40 (0.9 H, s); 3.44 (2 H, m); 7.23-7.32 (3 H, m); 7.33-7.41 (2 H, m). |
| | | | | 13C-NMR (CDCl3): 16.7; 30.1; 30.3; 30.7; 31.0; 31.4; 31.8; 32.6; 32.7; 35.3; 35.95; 38.03; 37.5; 37.8; 40.4; 42.2; 44.0; 44.8; 55.2; 56.3; 60.7; 124.23; 124.33; 126.4; 126.7; 127.5; 127.6; 127.67; 127.73; 169.8. |
| 363 | 1 | Bsp. Nr. 18/ Acylierung/ 60% | [M+H]+: m/z = 400.3, Rt = 2.9 min. | 1H-NMR (CDCl3): 1.32-1.46 (2 H, m); 1.58-1.76 (4 H, m); 1.85-2.22 (16 H, m); 2.30-2.38 (2 H, m); 2.42-2.58 (2 H, m); 3.30 (1.1 H, s); 3.36 (0.9 H, s); 3.47 (2 H, m); 6.81-6.87 (1 H, m); 7.00-7.06 (1 H, m), 7.20-7.25 (1 H, m). |
| | | | | 13C-NMR (CDCl3): 16.6; 30.1; 30.3; 30.9; 31.2; 31.80; 31.83; 32.71;32.77; 33.1; 35.3; 35.2; 36.8; 38.0; 38.1; 40.0; 42.1; 44.2; 45.0; 55.4; 56.4; 59.9; 64.1; 123.4; 123.5; 124.28; 124.33; 124.9; 126.0; 126.3; 169.8. |
| 365 | 1 | Bsp. Nr. 71/ Acylierung/ 64% | m/z: [M+H]+ = 327.3, Rt = 2.7 min. | 1H-NMR (CDCl3): 0.70-0.78 (2 H, m); 0.95-1.01 (2 H, m); 1.26-1.37 (2 H, m); 1.52-1.73 (5 H, m); 1.85-2.00 (2 H, m); 2.03 und 2.08 (6 H, 2 s); 2.15-2.30 (1 H, m); 2.33-2.45 (1 H, m); 3.41 (1 H, s); 3.44-3.49 (1 H, m); 3.56 (1 H, s); 3.62 (1 H, t, J = 7.2 Hz); 7.24-7.43 (5 H, m). |
| | | | | 13C-NMR (CDCl3): 7.3; 7.33; 12.2; 12.5; 30.0; 30.7; 31.3; 31.4; 36.1; 37.6; 38.0; 38.1; 40.3; 42.2; 44.2; 44.9; 54.5; 56.1; 126.5; 127.6; 127.7; 127.9; 172.1; 172.2. |
| 366 | 1 | Bsp. Nr. 71/ Acylierung/ 63% | m/z: [M+H]+ = 343.4, Rt = 3.0 min. | 1H-NMR (CDCl3): 0.97 (6 H, t, J =6.5 Hz); 1.24-1.35 (2 H, m); 1.51-1.56 (1 H, m); 1.59-1.68 (3 H, m); 1.75-2.03 (2 H, m); 2.04 und 2.07 (6 H, 2 s); 2.10-2.25 (4 H, m); 2.30-2.45 (1 H, m); 3.36 (1 H, s); 3.40-3.49 (3 H, m); 7.24-7.43 (5 H, m). |
| | | | | 13C-NMR (CDCl3): 22.7; 22.73; 25.57; 25.6; 30.0; 30.8; 31.2; 31.4; 36.1; 38.0; 38.1; 40.3; 42.2; 43.3; 43.75; 43.8; 45.2; 55.1; 56.4; 126.5; 127.6; 127.7; 127.8; 171.4; 171.5. |
| 367 | 1 | Bsp. Nr. 364 Stufe1/ Acylierung/ 45% | m/z: [M+H]+= 335.4 (100%), Rt = 3.0 min. | 1H-NMR (CDCl3): 0.03-0.07 (2 H, m); 0.39-0.43 (2 H, m); 0.67-0.77 (1H, m); 0.91 (3H, t, J = 7.1 Hz); 1.16-1.68 (16 H, m); 1.71 (1 H, t, J = 7.2 Hz); 1.78 (1 H, t, J = 7.1 Hz); 2.20 (3 H, s); 2.22 (3 H, s); 2.31-2.36 (2 H, m); 3.23 (1 H, s); 3.28 (1 H, s); 3.49 (2 H, dt, J = 7.2 und 2.7 Hz). |
| | | | | 13C-NMR (CDCl3): 4.5; 10.73; 10.74; 14.17; 14.19; 23.7; 23.8; 26.1; 26.6; 28.0; 28.9; 30.17; 30.20; 30.5; 30.6; 30.8; 33.8; 34.4; 34.8; 36.3; 37.3; 37.4; 40.3; 42.2; 44.2; 45.3; 56.5; 58.7; 171.85; 171.89. |
| 368 | 1 | Bsp. Nr. 364Stufe1/ Acylierung/ 17% | m/z: [M+H]+= 367.4 (100%), Rt = 2.9 min. | 1H-NMR (CDCl3): 0.88 (3 H, J = 7.1 Hz); 1.125 (3 H, s); 1.128 (3 H, s); 1.15-1.42 (10 H, m); 1.49-1.65 (4 H, m); 1.68 (1 H, t, J = 7.3 Hz); 1.76 (1 H, t, J = 7.1 Hz); 1.79-1.83 (2 H, m); 2.17 (3 H, s); 2.18 (3 H, s); 2.21-2.26 (2 H, m); 3.14 (3 H, s); 3.19 (1.2 H, s); 3.25 (0.8 H, s); 3.44-3.48 (2 H, m). |
| | | | | 13C-NMR (CDCl3): 14.1; 14.2; 23.7; 23.8; 25.0; 25.1, 26.1; 26.6, 28.0; 28.6, 28.8; 29.1; 30.2; 30.46; 30.52; 30.7; 33.8; 34.0; 34.1; 36.2; 37.29; 37.34; 40.3; 42.2; 44.3; 45.2; 49.15; 49.17; 56.4; 56.5; 56.6; 58.6; 73.90; 73.93; 171.88; 171.91. |
| 389 | 1 | Bsp. Nr. 345/ Reduktion/ 68% | [M+H]+: m/z = 343.4, Rt = 0.3 min. | 1H-NMR (CDCl3): 0.44-0.48 (2 H, m); 0.75-0.80 (2 H, m); 1.22-1.34 (2 H, m); 1.45 (2 H, t, J = 6.8 Hz); 1.64-1.73 (2 H, m); 1.78-1.96 (2 H, m); 2.03 (6 H, s); 2.20-2.34 (2 H, m); 2.55-2.62 (6 H, m); 3.34 (3 H, s); 7.23-7.40 (5 H, m). |
| | | | | 13C-NMR (CDCl3): 11.6 (2 C); 31.13 (2 C); 34.5 (2 C); 38.1 (2 C); 38.3; 41 2; 54.2; 54.7; 59.6; 60.3; 60.6; 66.2; 126.4; 127.6 (2 C); 127.8 (2C); 136.6. |
| 370 | 2 | Bsp. Nr. 24b/ Alkylierung/ 65% | m/z: [M+H]+ = 349.3, Rt = 2.6 min. | 1H-NMR (CDCl3): 0.92 (6 H, d, J = 6.6 Hz); 1.34-1.60 (5 H, m); 1.70-1.80 (3 H, m); 1.90-2.09 (3 H, m); 2.11 (6 H, s); 2.17 (2 H, s); 3.20 (2 H, s); 3.24-3.30 (2 H, m); 6.85 (1 H, d, J = 3.1 Hz); 7.05 (1 H, dd, J = 5.1 und 3.5 Hz); 7.24 (1 H, d, J = 5.0 Hz). |
| | | | | 13C-NMR (CDCl3): 22.5; 25.9; 32.7; 32.8; 35.5; 36.0; 38.1; 40.7; 44.4; 57.8; 59.3; 123.5; 124.9; 126.3; 173.4. |
| 371 | 1 | Bsp. Nr. 71/ Acylierung/ 62% | [M+H]+: m/z = 380.4, Rt = 2.7 min. | 1H-NMR (CDCl3): 0.84-0.89 (2 H, m); 1.14-1.22 (2 H, m); 1.23-1.36 (2 H, m); 1.51-1.71 (4 H, m); 1.79-1.85 (2 H, m); 1.86-1.98 (2 H, m); 2.00 (2.8 H, s); 2.03 (3.2 H, s); 2.12-2.40 (2 H, m); 2.46-2.55 (2 H, m); 3.37 (1.1 H, s); 3.38 (0.9 H, s); 3.40-3.50 (2 H, m); 7.20-7.40 (5 H, m). |
| | | | | 13C-NMR (CDCl3): 9.4; 13.97; 13.99; 30.1; 30.2; 30.5; 30.6; 31.0; 31.2; 32.0; 32.5; 35.7; 37.5; 37.9; 38.0; 40.4; 42.2; 44.0; 45.0; 55.2; 56.5; 60.7; 123.16; 123.25; 126.4; 126.6; 127.5; 127.6; 127.69; 127.74; 137.0; 169.8; 169.8. |
| 372 | 1 | Bsp. Nr. 18/ Acylierung/ 59% | [M+H]+: m/z = 386.3, Rt = 2.7 min. | 1H-NMR (CDCl3): 0.85-0.90 (2 H, m); 1.17-1.26 (2 H, m); 1.32-1.46 (2 H, m); 1.56-1.77 (4 H, m); 1.80-1.87 (2 H, m); 1.89-2.05 (4 H, m); 2.06-2.25 (6 H, m); 2.49-2.57 (2 H, m); 3.31 (0.4 H, s); 3.36 (1.6 H, s); 3.41-3.55 (2 H, m); 6.83-6.88 (1 H, m); 7.01-7.07 (1 H, m); 7.21-7.26 (1 H, m). |
| | | | | 13C-NMR (CDCl3): 9.4; 30.1; 30.2; 31.0; 32.1; 32.5; 32.9; 33.2; 37.1; 38.05; 38.12; 40.0; 40.2; 42.0; 42.1; 43.9; 44.2; 45.1; 45.8; 55.4; 56.6; 59.8; 114.6; 123.2; 123.3; 123.4; 123.5; 124.8; 126.13; 126.18; 126.3; 169.7. |
| 373 | 1 | Bsp. Nr. 18/ Acylierung/ 80% | m/z: [M+H]+= 377.3 (77%) [MH-NHMe2]+= 332.3 (100%). Rt = 2.7 min. | 1H-NMR (CDCl3): 0.64-0.68 (2 H, m); 0.83-0.87 (2 H, m); 1.35-1.45 (2 H, m); 1.63 (1.2 H, t, J = 7.2 Hz); 1.66-1.78 (2 H, m, überlagert); 1.72 (0.8 H, t, J = 7.2 Hz); 1.94-2.21 (4 H, m, überlagert); 2.09 (2.5 H, s); 2.11 (3.5 H, s); 2.67 (2 H, d, J = 3.3 Hz); 3.28 (1.2 H, s), 3.30 (1.8 H, s); 3.37 (0.8 H, s); 3.40 (1.2 H, s); 3.49 (0.8H.t.J=7.1 Hz); 3.56 (1.2 H, t, J = 7.1 Hz); 6.84-686 (1 H, m); 7.02-7.06 (1 H, m); 7.22-725 (1 H, m). |
| | | | | 13C-NMR (CDCl3): 11.8; 11.87; 11.90; 31.0; 31.2; 32.8; 33.2; 35.4; 37.0; 38.1; 38.4; 38.7; 40.1; 42.1; 44.1; 45.5; 54.42; 54.44; 55.6; 57.2; 59.7; 59.76; 59.81; 123.3; 123.4; 124.8; 125.0; 126.1; 126.3; 169.2; 169.3. |
| 374 | 1 | Bsp. Nr. 71/ Acylierung/ 65% | m/z: [M+H]+= 371.4 (100 %) [MH-NHMe2]+= 326.3 (87%), Rt = 2.8 min. | 1H-NMR (CDCl3): ): 0.64-0.68 (2 H, m); 0.83-0.88 (2 H, m); 1.32 (2 H, ddd, J = 13.6, 8.0 und 3.0 Hz); 1.56 (1.2 H, t, J = 7.2 Hz); 1.62-1.70 (2 H, m, überlagert); 1.65 (0.8 H, t, J = 7.2 Hz); 1.86-1.99 (2 H, m); 2.03 (2 H, s); 2.04 (4 H, s); 2.16-2.25 (0.8 H, m); 2.27-2.35 (1.2 H, m); 2.66 (0.8 H, s), 2.68 (1.2 H, s); 3.28 (1.2 H, s); 3.31 (1.8 H, s); 3.41 (0.8 H, s); 3.44 (1.2 H; s); 3.47 (1.2 H, t, J = 7.2 Hz); 3.54 (0.8 H, t, J = 7.2 Hz); 7.26-7.32 (3 H, m); 7.35-7.41 (2 H, m). |
| | | | | 13C-NMR (CDCl3): 11.90; 30.0; 30.7; 31.2; 31.5; 35.8; 37.6; 38.0; 38.1; 38.4; 38.8; 40.3; 42.4; 44.1; 45.5; 54.45; 54.46; 55.4; 57.0; 59.80; 59.83; 60.8; 126.5; 126.7; 127.56; 127.62; 127.68; 127.75; 169.27; 169.31. |
| 375 | 1 | Bsp. Nr. 364 Stufe1/ Acylierung/ 37% | m/z: [M+H]+= 335.4 (100 %), Rt = 3.1 min. | 1H-NMR (CDCl3): 0.91 (3 H, t, J = 7.2 Hz); 1.16-1.46 (10 H, m); 1.51-1.94 (10 H, m); 2.10-2.17 (2 H, m); 2.20 (3 H, s); 2.22 (3 H, s); 2.35 (2 H, dd; J = 7.4-2.7 Hz); 2.66-2.78 (1 H, m); 3.19 (1.2 H, s); 3.26 (0.8 H, s); 3.43-3.49 (2 H, m). |
| | | | | 13C-NMR (CDCl3): 14.16; 14.18; 18.74; 18.75; 23.7; 23.8, 26.1; 26.6; 28.0; 28.57; 28.62; 28.9; 30.2; 30.4; 30.6; 30.8; 32.3; 32.4; 33.8; 36.3; 37.3; 37.4; 40.3; 41.2; 41.6; 42.2; 44.1; 45.3; 56.4; 56.6; 58.7; 171.06; 171.08. |
| 376 | 2 | Bsp. Nr. 162 Stufe3/ Alkylierung/ 78% | m/z: [M+H]+ = 343.4, Rt = 2.7 min. | 1H-NMR (CDCl3): 0.93 (6 H, d, J = 6.6 Hz); 1.33-1.42 (4 H, m); 1.57 (1 H, td, J = 13.4 und 6.7 Hz); 1.61-1.78 (3 H, m); 1.85-2.03 (1 H, m); 2.05 (6 H, s); 2.12 (2 H, s); 2.14-2.32 (2 H, m); 3.23 (2 H, s); 3.25-3.30 (2 H, m); 7.27-7.32 (3 H, m); 7.36-7.42 (2 H, m). |
| | | | | 13C-NMR (CDCl3): 22.5; 25.9; 30.2; 32.9; 35.7; 36.0; 38.0; 40.7; 44.6; 57.7; 126.8; 127.5; 127.8; 173.5. |
| 377 | 2 | Bsp. Nr. 24b/ Alkylierung/ 50% | m/z: [M+H]+ = 335.3, Rt = 2.4 min. | 1H-NMR (CDCl3): 0.90 (6 H, d, J = 6.7 Hz); 1.43-1.52 (2 H, m); 1.56-1.71 (1 H, m); 1.72-1.82 (2 H, m); 1.84-1.95 (1 H, m); 1.97-2.10 (3 H, m); 2.11 (6 H, s); 2.20 (2 H, s); 3.06 (2 H, d, J = 7.5 Hz); 3.21 (2 H, s); 6.86 (1 H, d, J = 3.0 Hz); 7.05 (1 H, dd, J = 5.1 und 3.5 Hz); 7.25 (1 H, d, J = 4.7 Hz). |
| | | | | 13C-NMR (CDCl3): 20.1; 26.7; 32.7; 35.6; 38.0; 40.7; 44.2; 50.0; 59.3; 123.5; 124.9; 126.3; 173.9. |
| 378 | 2 | Bsp. Nr. 162 Stufe3/ Alkylierung/ 63% | m/z: [M+H]+ = 329.4, Rt = 2.4 min. | 1H-NMR (CDCl3): 0.90 (6 H, d, J = 6.6 Hz); 1.34-1.43 (2 H, m); 1.71-1.79 (3 H, m); 1.85-2.02 (3 H, m); 2.04 (6 H, s); 2.15 (2 H, s); 2.16-2.25 (1 H, m); 3.07 (2 H, d, J = 7.5 Hz); 3.24 (2 H, s); 7.26-7.31 (3 H, m); 7.36-7.41 (2 H, m). |
| | | | | 13C-NMR (CDCl3): 20.1; 26.7; 30.2; 32.9; 35.7; 38.0; 44.5; 50.0; 58.6; 126.8; 127.4; 127.8; 174.0. |
| 379 | 1 | Bsp. Nr. 71/ Acylierung/ 71% | [M+H]+: m/z = 371.4, Rt = 2.7 min. | 1H-NMR (CDCl3): 0.75 (2 H, br s); 0.94-1.00 (2 H, m); 1.20-1.35 (2 H, m); 1.44-1.70 (4 H, m); 1.76-1.98 (2 H, m); 2.04 (6 H, s); 2.21 (0.6 H, br s); 2.37 (1.4 H, br s); 3.32 (3 H, s); 3.36-3.48 (2 H, m); 3.45 (2 H, s); 3.61 (1.7 H, s); 3.69 (0.3 H, br s); 7.20-7.44 (5 H, m). |
| | | | | 13C-NMR (CDCl3): 10.5; 26.5; 29.5; 30.0; 30.6; 30.7; 31.7; 35.9; 38.0; 42.0; 44.3; 44.8; 56.1; 58.7; 77.4; 126.7; 127.7; 170.9. |
| 380 | 1 | Bsp. Nr. 71/ Acylierung/ 76% | [M+H]+: m/z = 373.4, Rt = 2.8 min. | 1H-NMR (CDCl3): 1.20-1.35 (2 H, m); 1.27 (6 H, s); 1.49 (2 H, br s); 1.58-1.68 (2 H, m); 1.91 (2 H, br s); 2.04 (6 H, s); 2.30 (2 H, br s); 3.35 (3 H, s); 3.45 (2 H, s); 3.47-3.60 (4 H, m); 7.24-7.44 (5 H, m). |
| | | | | 13C-NMR (CDCl3): 22.9; 30.9; 38.0; 43.4; 46.1; 59.3; 80.2; 126.5; 127.7; 127.6; 174.8. |
| 381 | 1 | Bsp. Nr. 71/ Acylierung/ 65% | [M+H]+: m/z = 385.4, Rt = 2.9 min. | 1H-NMR (CDCl3): 1.17-1.33 (2 H, m); 1.42-1.48 (2 H, m); 1.49-2.00 (8 H, m); 2.03 (6 H, s); 2.14-2.38 (2 H, m); 2.39-2.50; (2 H, m); 3.30-3.50 (7 H, m); 3.61 (0.6 H, s); 3.62 (1.4 H, s); 7.22-7.44 (5 H, m). |
| | | | | 13C-NMR (CDCl3): 15.3; 28.3; 28.5; 30.1; 30.6; 30.8; 31.6; 35.7; 38.0; 38.1; 38.2; 39.5; 42.4; 44.3; 44.9; 48.7; 48.8; 55.3; 56.3; 59.1; 59.3; 61.1; 77.6; 77.7; 126.5; 127.7; 136.1; 137.3; 174.8; 175.2. |
| 382 | 1 | Bsp. Nr. 18/ Acylierung/ 58% | m/z: [M+H]+ = 377.3, Rt = 2.9 min. | 1H-NMR (CDCl3): 1.34-1.44 (2 H, m); 1.55-1.76 (6 H, m); 1.77-1.90 (1 H, m); 1.90-2.10 (3 H, m); 2.11 (6 H, 2 s); 2.14-2.22 (2 H, m); 2.50-2.60 (2 H, m); 3.091 und 3.094 (3 H, 2 s); 3.41 und 3.42 (2 H, 2 s); 3.50-3.55 (2 H, m); 6.85-6.89 (1 H, m); 7.02-7.07 (1 H, m); 7.23-7.26 (1 H, m). |
| | | | | 13C-NMR (CDCl3): 12.7; 12.8; 29.8; 30.0; 30.1; 30.6; 31.3; 32.9; 33.0; 38.0; 38.1; 39.2; 42.1; 44.6; 44.7; 51.3; 51.4; 55.9; 56.7; 59.9; 81.8; 82.1; 123.3; 123.4; 124.9; 125.0; 126.1; 126.2; 170.5; 170.8. |
| 383 | 1 | Bsp. Nr. 18/ Acylierung/ 78% | [M+H]+: m/z = 377.3, Rt = 2.6 min. | 1H-NMR (CDCl3): 0.74 (2 H, brs); 0.96 (2 H, m); 1.37 (2 H, ddd, J = 13.7, 10.4 und 3.5 Hz); 1.50-1.74 (4 H, m); 1.80-2.02 (2 H, m); 2.11 (6 H, s); 2.13-2.28 (2 H, m); 3.31 (3 H, s); 3.33-3.39 (2 H, m); 4.49 (2 H, s); 3.57 (1.4 H, br s); 3.71 (0.6 H, br s); 6.84 (1 H, br d, J = 3.2 Hz); 6.99-7.08 (1 H, m); 7.23 (1 H, br d, J = 4.6 Hz). |
| | | | | 13C-NMR (CDCl3): 10.5; 26.6; 30.6; 31.4; 32.9; 33.1; 35.5; 37.1; 38.1; 39.6; 41.7; 44.5; 44.9; 56.1; 58.7; 60.2; 77.3; 123.6; 125.2; 126.3; 142.0; 143.7; 170.9. |
| 384 | 1 | Bsp. Nr. 18/ Acylierung/ 76% | [M+H]+: m/z = 379.3, Rt = 2.8 min. | 1H-NMR (CDCl3): 1.25 (6 H, s); 1.32-1.43 (2 H, m); 1.47-1.61 (2 H, br s); 1.62-1.71 (2 H, m); 1.95 (2 H, br s); 2.02-2.20 (8 H, br s); 3.34 (3 H, s); 3.43 (2 H, s); 3.46 (2 H, br s); 3.54 (2 H, sehr br s); 6.84 (1 H, d, J = 3.0 Hz); 7.03 (1 H, dd, J = 5.1 und 3.5 Hz); 7.23 (1 H, d, J = 4.9 Hz). |
| | | | | 13C-NMR (CDCl3): 22.7; 30.7; 33.0; 38.1; 43.4; 46.0; 57.2; 59.1; 59.8; 80.2; 123.4; 124.8; 126.1; 174.6. |
| 385 | 1 | Bsp. Nr. 18/ Acylierung/ 65% | [M+H]+: m/z = 391.3, Rt = 2.9 min. | 1H-NMR (CDCl3): 1.28-1.42 (2 H, m); 1.49-1.55 (2 H, m); 1.56-1.76 (4 H, m); 1.76-2.24 (12 H, m); 2.35-2.48 (2 H, m); 3.32 (1.4 H, s); 3.33 (3 H, s); 3.37 (0.6 H, s); 3.42-3.52 (2 H, m); 3.61 (2 H, s); 6.83 (1 H, dd, J = 3.5 und 0.9 Hz); 7.00-7.05 (1 H, m); 7.20-7.25 (1 H, m). |
| | | | | 13C-NMR (CDCl3): 15.24; 15.27; 28.4; 28.5; 30.5; 31.3; 32.9; 33.0; 35.0; 38.1; 39.1; 42.1; 44.3; 44.7; 48.7; 48.8; 55.3; 56.5; 59.0; 59.3; 59.9; 60.1; 77.57; 77.63; 123.1; 123.3; 124.7; 125.1; 126.1; 126.3; 174.8; 175.0. |
| 386 | 2 | Bsp. Nr. 24b/ Alkylierung/ 67% | m/z: [M+H]+= 377.3 (100 %) [MH-NHMe2]+= 332.3 (65 %), Rt = 2.4 min. | 1H-NMR (CDCl3): 0.40-0.44 (2 H, m); 0.75-0.78 (2 H, m); 1.43-1.49 (2 H, m); 1.74-1.80 (4 H, m); 1.93-2.13 (4 H, m, überlagert); 2.10 (6 H, s); 2.17 (2 H, s); 3.25 (3 H, s); 3.29 (2 H, s); 3.40-3.43 (2 H, m); 6.85 (1 H, dd, J = 3.6 und 1.1 Hz); 7.04 (1 H, dd, J = 5.1 und 3.6 Hz); 7.24 (1 H, dd, J = 5.1 und 1.1 Hz). |
| | | | | 13C-NMR (CDCl3): 12.0; 30.4; 32.8; 34.5; 38.1; 39.9; 44.4; 53.8; 59.3; 60.0; 123.5; 123.9; 126.3; 173.6. |
| 387 | 2 | Bsp. Nr. 162 Stufe3/ Alkylierung/ 66% | m/z: [M+H]+= 371.4 (100 %) [MH-NHMe2]+= 326.3 (51 %), Rt = 2.5 min. | 1H-NMR (CDCl3): 0.41-0.44 (2 H, m); 0.76-0.79 (2 H, m); 1.37 (2 H, ddd; J = 13.1, 10.4 und 3.0 Hz); 1.71-1.79 (4 H, m); 1.89-2.04 (2 H, m, überlagert); 2.03 (6 H, s); 2.12 (2 H, s); 2.15-2.30 (2 H, m); 3.26 (3 H, s); 3.33 (2 H, s); 3.40-3.44 (2 H, m); 7.28-7.30 (3 H, m); 7.36-7.40 (2 H, m). |
| | | | | 13C-NMR (CDCl3): 12.1; 30.3; 30.4; 33.0; 35.7; 38.0; 39.9; 44.7; 53.9; 58.8; 60.0; 126.7; 127.5; 127.8; 173.6. |
| 388 | 1 | Bsp. Nr. 364Stufe1/ Acylierung/ 29% | m/z: [M+H]+= 365.4 (100 %), Rt = 3.0 min. | 1H-NMR (CDCl3): 0.92 (3 H, dt, J = 7.2 und 2.0 Hz); 1.22-1.33 (8 H, m);); 1.37-1.44 (2 H, m); 1.55-1.74 (8 H, m, überlagert); 1.70 (1 H, t, J = 7.3 Hz, überlagert); 1.77 (1 H, t, J = 7.1 Hz); 2.12-2.19 (2 H, m); 2.22-2.30 (6 H, m); 2.58 (0.8 H, s); 2.60 (1.2 H, br. s); 3.23 (3 H, s); 3.30 (1 H, s); 3.35 (1 H, br s); 3.49-3.57 (2 H, m). |
| | | | | 13C-NMR (CDCl3): 12.57; 12.59; 14.1; 14.2; 23.71; 23.74; 26.0; 26.6; 27.9; 28.7; 30.1; 30.5; 30.6; 30.8; 34.2; 36.5; 37.4; 40.2; 40.4; 40.6; 42.1; 44.2, 45.8; 50.20; 50.23; 79.47; 79.52; 169.5; 169.6. |
| 389 | 1 | Bsp. Nr. 364Stufe1/ Acylierung/ 41% | m%z: [M+H]+= 351.4 (100 %), Rt = 2.7 min. | 1H-NMR (CDCl3): 0.91 (3 H, J = 7.3 =Hz); 1.18-1.69 (14 H, m); 1.71 (1 H, t, J = 7.2 Hz); 1.79(1 H, t, J = 7.1 Hz); 2.10-2.19 (2 H, m); 2.21 (3 H, s), 2.25 (3 H, br s); 2.28-2.39 (2 H, m); 2.64-2.76 (1 H, m); 3.21 (1 H, br s); 3.28 (1 H, s); 3.40-3.36 (2 H, m); 3.50 (1 H, t, J = 7.3 Hz); 3.71-3.77 (1 H, m); 3.82-3.88 (1 H, m); 3.94-3.98 (1 H, m). |
| | | | | 13C-NMR (CDCl3): 14.1; 14.2, 23.7; 23.8; 26.1; 26.7; 28.0; 28.91; 28.93; 30.1; 30.2; 30.4; 30.5, 30.7; 32.3; 33.9; 35.36; 35.41; 36.1; 37.4; 38.1; 38.4, 40.3; 42.2; 44.2; 45.3, 56.5; 58.5; 67.6; 73.32; 73.35; 170.4; 170.5. |
| 390 | 1 | Bsp. Nr. 71/ Acylierung/ 62% | m/z: [M+H]+ = 371.4, Rt = 3.0 min. | 1H-NMR (CDCl3): 1.22-1.35 (2 H, m); 1.47-1.53 (1 H, m); 1.55-1.75 (4 H, m); 1.80-2.00 (4 H, m); 2.03 und 2.04 (6 H, 2 s); 2.06-2.20 (2 H, m); 2.23-2.34 (1 H, m); 2.51-2.60 (2 H, m); 3.09 (3 H, s); 3.43 und 3.45 (2 H, 2 s); 3.47-3.53 (2 H, m); 7.27-7.33 (3 H, m); 7.34-7.40 (2 H, m). |
| | | | | 13C-NMR (CDCl3): 12.7; 12.8; 30.0; 30.1; 30.2; 30.5; 30.8; 31.6; 35.1; 38.0; 38.1; 39.5; 42.4; 44.6; 44.7; 51.3; 51.4; 55.8; 56.5; 60.9; 81.8; 82.1; 126.5; 126.7; 127.6; 127.7; 127.74; 170.5; 170.8. |
| 391 | 1 | Bsp. Nr. 31/ Acylierung/ 65% | m/z: [M+H]+ = 391.3, Rt = 2.9 min. | 1H-NMR (CDCl3): 0.70-0.78 (2 H, m); 0.94-0.99 (2 H, m); 1.35-1.44 (2 H, m); 1.53-1.70 (4 H, m); 1.73-1.97 (3 H, m); 2.00-2.10 (1 H, m); 2.13 (6 H, br s); 2.46 (3 H, s); 3.32 (3 H, s); 3.42-3.50 (2 H, m); 3.44 (2 H, s); 3.57 (2 H, br s); 6.59-6.64 (1 H, m); 6.65-6.70 (1 H, m). |
| | | | | 13C-NMR (CDCl3): 10.5; 15.2; 26.6; 30.8; 31.5; 32.7; 33.1; 35.7; 38.1; 41.8; 44.5; 45.0; 50.8; 56.1; 58.7; 60.3; 77.4; 124.5; 125.2; 137.9; 171.0. |
| 392 | 1 | Bsp. Nr. 31/ Acylierung/ 62% | m/z: [M+H]+ = 405.3, Rt = 3.0 min. | 1H-NMR(CDCl3): 1.32-1.42 (2 H, m); 1.49-1.62 (3 H, m); 1.63-1.96 (6 H, m); 1.97-2.08 (3 H, m); 2.10 und 2.13 (6 H, 2 s); 2.37-2.45 (2 H, m); 2.46 (3 H, s); 3.33 (3 H, s); 3.32 und 3.37 (2 H, 2 s); 3.42-3.52 (2 H, m); 3.62 (2 H, s); 6.57-6.63 (1 H, m); 6.64-6.69 (1 H, m). |
| | | | | 13C-NMR (CDCl3): 15.2; 28.4; 28.5; 30.7; 31.6; 32.7; 33.0; 35.4; 38.1; 39.2; 42.3; 44.3; 44.9; 48.7; 48.8; 55.3; 56.5; 59.1; 59.3; 60.0; 77.6; 124.3; 124.5; 124.8; 125.1; 137.7; 175.0; 175.2. |
| 393 | 1 | Bsp. Nr. 31/ Acylierung/ 60% | m/z: [M+H]+ = 393.3, Rt = 3.0 min. | 1H-NMR (CDCl3): 1.26 (6 H, s); 1.39 (2 H, ddd, J = 13.6 und 10.3 und 3.5 Hz); 1.50-1.72 (5 H, m); 1.81-1.97 (2 H, m); 1.81-1.97 (2 H, m); 2.00-2.10 (1 H, m); 2.12 (6 H, s); 2.47 (3 H, s); 3.35 (3 H, s); 3.44 (2 H, br s); 3.54 (2 H, br s); 6.60-6.64 (1 H, m); 6.66-6.70 (1 H, m). |
| | | | | 13C-NMR (CDCl3): 15.2; 22.9; 30.9; 33.0; 38.1; 43.5; 46.2; 56.8; 59.2; 60.1; 80.2; 124.4; 125.0; 137.8; 174.8. |
| 394 | 1 | Bsp. Nr. 71/ Acylierung/ 51% | [M+H]+: m/z = 382.4, Rt = 2.8 min. | 1H-NMR (CDCl3): 1.24-1.34 (2 H, m); 1.36 (2.7 H, s); 1.38 (3.3 H, s); 1.53-1.59 (1.1 H, m); 1.60-1.72 (2.9 H, m); 1.84-1.98 (4 H, m); 2.03 (2.7 H, s); 2.05 (3.3 H, s); 2.14-2.37 (2 H, m); 2.38-2.48 (2 H, m); 3.36 (1.1 H, s); 3.40 (0.9 H s); 3.46 (2 H, t, J = 7.0 Hz); 7.22-7.42 (5 H, m). |
| | | | | 13C-NMR (CDCl3): 26.5; 30.1; 30.2; 30.6; 30.7; 31.2; 31.4; 31.8; 31.9; 32.00; 32.02; 35.5; 35.7; 37.5; 37.9; 38.0; 40.37; 40.41;42.2; 44.2; 44.9; 55.2; 56.4; 60.9; 124.5; 124.7; 126.4; 126.8; 127.4; 127.6; 127.7; 127.8; 137.0; 169.8. |
| 395 | 1 | Bsp. Nr. 18/ Acylierung/ 64% | [M+H]+: m/z = 388.3, Rt = 2.8 min. | 1H-NMR (CDCl3): 1.34-1.46 (2 H, m); 1.36 (2.7 H, s); 1.37 (3.3 H, s); 1.58-1.76 (4 H, m); 1.85-2.04 (4 H, m); 2.05-2.21 (2 H, m); 2.09 (2.7 H, s); 2.12 (3.3 H, s); 2.38-2.47 (2 H, m); 3.32 (1.2 H, s); 3.36 (0.8 H, s); 3.48 (2 H, t, J = 7.2 Hz); 6.82-6.88 (1 H, m); 7.01-7.07 (1 H, m); 7.21-7.26 (1 H, m). |
| | | | | 13C-NMR (CDCl3): 26.4; 26.5; 30.0; 30.2; 30.6; 31.0; 31.2; 31.8; 31.9; 32.0; 32.9; 33.1; 35.1; 35.6; 35.7; 36.8; 37.9; 38.1; 40.0; 42.1; 44.2; 44.9; 55.4; 56.4; 59.9; 123.4; 123.6; 124.60; 124.64; 124.9; 126.2; 126.2; 169.8. |
| 396 | 1 | Bsp. Nr. 425/ Acylierung/ 24% | m/z: [MH-HNMe2]+ = 366.2 (100 %), Rt = 3.0 min. | 1H-NMR (CDCl3): 0.70-0.78 (2 H, m); 0.93-1.00 (2 H, m); 1.32-1.42 (2 H, m); 1.54-1.70 (4H, m); 1.80-2.00 (4 H, m); 2.04-2.10 (1 H, m); 2.13 (6 H, br s); 3.32 (3 H, s); 3.42 (2 H, s); 3.44-3.50 (1 H, m); 3.54-3.76 (2 H, m); 6.61 (1 H, br d, J = 3.6 Hz); 6.84 (1 H, br d, J = 2.7 Hz). |
| | | | | 13C-NMR (CDCl3): 10.5; 26.6; 30.6; 31.3; 32.4; 32.8; 35.4; 38.0; 41.8; 44.4; 44.9; 56.1; 58.7; 60.4; 77.5; 124.6; 125.5; 171.0. |
| 397 | 1 | Bsp. Nr. 425/ Acylierung/ 24% | m/z: [MH-HNMe2]+ = 380.2 (100 %), Rt = 3.2 min. | 1H-NMR (CDCl3): 1.30-1.41 (2 H, m); 1.52-1.77 (6 H, m); 1.78-2.08 (6 H, m); 2.10 und 2.11 (6 H, 2 s); 2.37-2.48 (2 H, m); 3.33 und 3.37 (5 H, 2 s); 3.44-3.57 (2 H, m); 3.62 (2 H, s); 6.60 (1 H, d, J = 3.8 Hz); 6.82-6.86 (1 H, m). |
| | | | | 13C-NMR (CDCl3): 15.3; 28.4; 28.5; 30.5; 31.4; 32.5; 32.7; 35.2; 38.1; 39.2; 42.2; 44.3; 44.9; 48.7; 48.8; 55.4; 56.5; 59.1; 59.3; 60.5; 77.6; 77.7; 124.5; 125.4; 125.5; 127.8; 175.0; 175.2. |
| 398 | 1 | Bsp. Nr. 425/ Acylierung/ 28% | m/z: [MH-HNMe2]+ = 368.2 (100 %), Rt = 3.1 min. | 1H-NMR (CDCl3): 1.26 (6 H, s); 1.34-1.42 (2 m); 1.50-1.70 (5 H, m); 1.80-1.95 (2 H, m); 1.95-2.10 (2 H, m); 2.11 (6 H, s); 3.35 (3 H, s); 3.44 (2 H, s); 3.44-3.60 (3 H, m); 6.60 (1 H, br d, J = 3.5 Hz); 6.84 (1 H, br d, J = 3.6 Hz). |
| | | | | 13C-NMR (CDCl3): 22.9; 30.7; 31.5; 32.7; 37.9; 38.0; 43.6; 46.2; 57.5; 59.2; 60.4; 80.2; 124.4; 125.2; 125.5; 174.8. |
| 399 | 1 | Bsp. Nr. 432/ Alkylierung/ 76% | [M+H]+: m/z = 351.4, Rt = 2.6 min. | 1H-NMR (CDCl3): 0.90 (3 H, t, J = 7.2 Hz); 1.10-1.45 (10 H, m); 1.46-1.80 (7 H, m); 2.20-2.18 (2 H, m); 2.19 (6 H, s); 2.23 (2 H, m); 3.13 (2 H, s); 3.26-3.30 (2 H, m); 3.35 (1 H, dd; J = 8.3 und 6.9 Hz); 3.72 (1 H, dd, J = 15.4 und 7.7 Hz); 3.80-3.92 (2 H, m). |
| | | | | 13C-NMR (CDCl3): 14.2; 23.6; 26.5; 28.2; 30.7; 30.6; 32.1; 32.4; 35.5; 36.8; 37.3; 41.5; 45.3; 55.8; 57.6; 67.7; 73.0; 76.7; 77.0; 77.2; 173.9. |
| 400 | 2 | Bsp. Nr. 433/ Alkylierung/ 59% | [M+H]+: m/z = 351.4, Rt = 2.4 min. | 1H-NMR (CDCl3): 0.90 (3 H, t, J = 7.1 Hz); 1.10-1.42 (10 H, m); 1.46-1.80 (7 H, m); 2.02-2.19 (2 H, m); 2.20 (6 H, s); 2.26 (2 H, s); 3.10 (2 H, s); 3.26 (2 H, t, J = 7.4 Hz); 3.35 (1 H, dd, J = 8.3 und 6.9 Hz); 3.70-3.77 (1 H, m); 3.80-3.93 (2 H, m). |
| | | | | 13C-NMR (CDCl3): 14.0; 23.8; 26.5; 28.5; 30.4; 30.6; 31.8; 32.3; 35.9; 36.8; 37.3; 41.2; 43.2; 56.0; 59.8; 67.9; 73.2; 173.7. |
| 401 | 1 | Bsp. Nr. 432/ Alkylierung/ 67% | [M+H]+: m/z = 346.4, Rt = 2.6 min. | 1H-NMR (CDCl3): 0.87 (2 H, m); 0.91 (3 H, t, J = 7.2 Hz); 1.10-1.44 (12 H, m); 1.52-1.64 (2 H, m); 1.66-1.80 (4 H, m); 2.20 (6 H, s); 2.25 (2 H, s); 3.26 (2 H, s); 3.46 (2 H, m). |
| | | | | 13C-NMR (CDCl3): 7.6; 13.9; 14.2; 23.8; 26.4; 28.4; 30.7; 30.8; 31.8; 32.1; 32.6; 35.7; 37.2; 41.1; 45.2; 55.8; 58.7; 123.0; 174.4. |
| 402 | 2 | Bsp. Nr. 433/ Alkylierung/ 37% | [M+H]+: m/z = 346.4, Rt = 2.3 min. | 1H-NMR (CDCl3): 0.83-0.88 (2 H, m); 0.90 (3 H, t, J = 7.1 Hz); 1.12-1.45 (12 H, m); 1.50-1.84 (6 H, m); 2.20 (6 H, s); 2.27 (2 H, s); 3.22 (2 H, s); 3.47 (2 H, t, J = 7.2 Hz). |
| | | | | 13C-NMR (CDCl3): 7.5; 14.1; 14.0; 23.8; 26.5; 28.6; 30.6; 31.8; 32.5; 36.0; 37.3; 41.1; 43.0; 56.0; 60.7; 122.8; 174.2. |
| 403 | 1 | Bsp. Nr. 432/ Alkylierung/ 57% | [M+H]+: m/z = 360.4, Rt = 2.8 min. | 1H-NMR (CDCl3): 0.90 (3 H, t, J = 7.2 Hz); 1.12-1.44 (8 H, m); 1.52-1.64 (2 H, m); 1.67-1.77 (2 H, m); 1.92-1.98 (2 H, m); 1.92-1.98 (2 H, m); 1.99-2.18 (4 H, m); 2.19 (6 H, s); 2.24 (2 H, s); 2.43-2.58 (2 H, m); 3.19 (2 H, s); 3.34-3.40 (2 H, m). |
| | | | | 13C-NMR (CDCl3): 14.2; 16.9; 23.8; 26.4; 28.4; 30.5; 32.0; 33.7; 34.7; 35.6; 37.3; 39.1; 45.2; 53.4; 55.9; 58.0; 124.2; 174.1. |
| 404 | 2 | Bsp. Nr. 433/ Alkylierung/ 57% | [M+H]+: m/z = 360.4, Rt = 2.6 min. | 1H-NMR (CDCl3): 0.90 (3 H, t, J = 7.1 Hz); 1.10-1.45 (10 H, m); 1.46-1.82 (4 H, m); 1.86-2.24 (6 H, m); 2.21 (6 H, s); 2.27 (2 H, s); 2.48-2.58 (2 H, m); 3.16 (2 H, s); 3.33-3.41 (2 H, m). |
| | | | | 13C-NMR (CDCl3): 14.5; 17.2; 24.1; 26.8; 28.9; 30.9; 32.1; 32.3; 34.0; 35.2; 36.2; 37.6; 39.3; 43.4; 56.2; 60.5; 124.5; 174.4. |
| 405 | 1 | Bsp. Nr. 71/ Acylierung/ 60% | m/z: [M+H]+ = 385.4, Rt = 2.9 min. | 1H-NMR (CDCl3): 0.40-0.46 (2 H, m); 0.70-0.77 (2 H, m); 1.20-1.36 (2 H, m); 1.52-1.57 (2 H, m); 1.60-1.70 (2 H, m); 1.85-2.00 (4 H, m); 2.03 und 2.05 (6 H, 2 s); 2.17-2.36 (2 H, m); 2.41-2.48 (2 H, m); 3.23 und 3.25 (3 H, 2 s); 3.39 und 3.40 (2 H, 2 s); 3.43-3.50 (2 H, m); 7.26-7.33 (3 H, m); 7.34-7.41 (2 H, m). |
| | | | | 13C-NMR (CDCl3): 12.27; 12.3; 28.0; 28.1; 30.1; 30.6; 30.64; 31.0; 31.3; 31.4; 36.0; 37.6; 38.0; 38.1; 40.4; 42.3; 44.0; 45.0; 53.75; 55.2; 56.4; 60.9; 61.56; 61.59; 126.5; 126.7; 127.6; 127.63; 127.7; 127.74; 171.7. |
| 406 | 1 | Bsp. Nr. 432/ Alkylierung/ 66% | [M+H]+: m/z = 365.4, Rt = 2.9 min. | 1H-NMR (CDCl3): 0.91 (3 H, t, J = 7.1 Hz); 1.10-1.40 (10 H, m); 1.52-1.80 (6 H, m); 1.82-1.94 (4 H, m); 2.06-2.18 (2 H, m); 2.21 (6 H, s); 2.24 (2 H, s); 3.16 (3 H, s); 3:18 (2 H, s); 3.23-3.29 (2 H, m). |
| | | | | 13C-NMR (CDCl3): 12.5; 14.2; 23.8; 26.5; 28.4; 30.8; 31.2; 32.0; 32.2; 35.5; 37.4; 37.9; 45.4; 49.3; 56.8; 58.4; 78.3; 173.8. |
| 407 | 1 | Bsp. Nr. 426/ Acylierung/ 91% | m/z: [M+H]+= 409.3(58%) [MH-NHMe2]+= 364.3(100%). Rt = 3.0 min. | 1H-NMR (CDCl3): 1.31-1.42 (2 H, m); 1.54-2.04 (14 H, m); 2.11 (2 H, s); 2.12 (4 H, s); 2.39-2.46 (2 H, m); 3.33 (3 H, s); 3.44-3.53 (2 H, m); 3.62 (2 H, s); 6.36-6.39 (1 H, m); 6.41-6.43 (1 H, m). |
| | | | | 13C-NMR (CDCl3): 15.3; 28.45; 28.52; 30.6; 31.4; 32.2; 32.5; 35.2, 38.1; 39.3; 42.3; 44.3; 44.9; 45.3, 47.3, 48.7, 48.8, 55.4; 56.5, 59.1; 59.3; 77.6; 77.7; 106.0; 106.2; 106.3; 121.4; 162.5, 165.4, 175.2. |
| 409 | 2 | Bsp. Nr. 433/ Alkylierung/ 43% | [M+H]+: m/z = 337.3, Rt = 2.1 min. | 1H-NMR (CDCl3): 0.89 (3 H, t, J = 7.2 Hz); 1.10-1.42 (10 H, m); 1.52-1.62 (2 H, m); 1.66-1.78 (2 H, m); 1.87 (2 H, dd, J = 14.5 und 7.4 Hz); 2.19 (6 H, s); 2.24 (2 H, s); 2.90-3.02 (1 H, m); 3.09 (2 H, s); 3.18 (2 H, t, J = 7.1 Hz); 4.37 (2 H, t, J = 6.1 Hz); 4.77 (2 H, dd, J = 7.7 und 5.9 Hz). |
| | | | | 13C-NMR (CDCl3): 14.0; 23.6; 26.6; 28.6; 30.5; 31.2; 31.8; 32.9; 35.7; 37.1; 40.0; 43.1; 55.9; 59.9; 77.5; 173.9. |
| 410 | 1 | Bsp. Nr. 432/ Alkylierung/ 35% | [M+H]+: m/z = 337.4, Rt = 2.5 min. | 1H-NMR (CDCl3): 0.91 (3 H, t, J = 7.2 Hz); 1.10-1.40 (10 H, m); 1.52-1.64 (2 H, m); 1.66-1.78 (2 H, m); 1.89 (2 H, dd, J = 14.5 und 7.5 Hz); 2.20 (6 H, s); 2.23 (2 H, s); 2.90-3.02 (1 H, m); 3.14 (2 H, s); 3.19 (2 H, t, J = 7.1 Hz); 4.38 (2 H, t, J = 6.1 Hz); 4.78 (2 H, dd, J = 7.7 und 6.0 Hz). |
| | | | | 13C-NMR (CDCl3): 14.2; 23.8; 26.4; 28.4; 30.7; 31.1; 32.0; 33.0; 35.5; 37.2; 40.2; 45.1; 55.8; 57.7; 77.6; 173.9. |
| 411 | 1 | Bsp. Nr. 426/ Acylierung/ 45% | m/z: [M+H]+= 395.3 (55 %) [MH-NHMe2]+= 350.2 (100 %), Rt = 2.7 min. | 1H-NMR (CDCl3): 0.72-0.76 (2 H, m); 0.94-0.98 (2 H, m); 1.35-1.42 (2 H, m); 1.58-1.68 (4 H, m); 1.82-1.95 (2 H, m); 1.95-2.15 (2 H, m, überlagert); 2.12 (6 H, s); 3.32 (3 H, s); 3.33-3.40 (0.7 H, m); 3.43-3.50 (1.1 H, m, überlagert); 3.44 (2 H, s); 3.56 (1.3 H, br. s); 3.72 (0.7 H, br. s); 6.36-6.40 (1 H, m); 6.41-6.43 (1 H, m). |
| | | | | 13C-NMR (CDCl3): 10.5; 26.6; 30.6; 31.4; 32.1; 32.6; 35.5; 38.1; 41.9; 44.5; 56.2; 58.7; 60.2; 77.5; 106.3; 121.3; 126.5; 165.4; 171.0. |
| 412 | 1 | Bsp. Nr. 18/ Acylierung/ 29% | m/z: [M+H]+ = 391.3, Rt = 2.8 min. | 1H-NMR (CDCl3): 0.41-0.46 (2 H, m); 0.71-0.77 (2 H, m); 1.23-1.45 (2 H, m); 1.58-1.74 (5 H, m); 1.87-2.08 (4 H, m); 2.09 und 2.11 (6 H, 2 s); 2.13-2.22 (1 H, m); 2.41-2.48 (2 H, m); 3.23 und 3.25 (3 H, 2 s); 3.36 und 3.37 (2 H, 2 s); 3.49 (2 H, q, J = 7.2 Hz); 6.84-6.87 (1 H, m); 7.02-7.06 (1 H, m); 7.22-7.26 (1 H, m). |
| | | | | 13C-NMR (CDCl3): 12.28; 12.3; 28.0; 28.1; 30.6; 31.0; 31.1; 31.2; 32.9; 33.2; 35.6; 37.1; 38.1; 38.11; 40.2; 42.0; 44.0; 45.1; 53.75; 53.77; 55.4; 56.5; 59.9; 61.57; 61.6; 123.4; 124.9; 126.2; 126.3; 171.7. |
| 413 | 2 | Bsp. Nr. 433/ Alkylierung/ 36% | [M+H]+: m/z = 365.4, Rt = 2.7 min. | 1H-NMR (CDCl3): 0.90 (3 H, t, J = 7.1 Hz); 1.10-1.46 (10 H, m); 1.50-1.92 (12 H, m); 2.05-2.16 (2 H, m); 2.20 (6 H, s); 2.26 (2 H, s); 3.15 (3 H, s); 3.22-3.28 (2 H, m). |
| | | | | 13C-NMR (CDCl3): 12.5; 14.0; 23.8; 26.5; 28.7; 29.7; 30.4; 31.3; 31.8; 35.7; 37.2; 37.6; 41.0; 43.4; 49.4; 56.1; 60.1; 78.3; 173.7. |
| 414 | 2 | Bsp. Nr. 408 Stufe1/ Acylierung/ 44% | m/z: [M+H]+ = 355.4, Rt = 3.0 min. | 1H-NMR (CDCl3): 1.27-1.37 (2 H, m); 1.57-1.75 (4 H, m); 1.75-1.92 (4 H, m); 1.93-2.03 (1 H, m); 2.04 (6H, s); 2.06-2.22 (5 H, m); 2.26 (1 H, d, J = 7.4 Hz); 2.34 (1 H, d, J = 7.4 Hz); 2.62-2.76 (1 H, m); 3.07 und 3.14 (2 H, 2 s); 3.48 (2 H, t, J = 7.2 Hz); 3.50 (2 H, t, J = 7.2 Hz); 7.26-7.32 (3 H, m); 7.33-7.41 (2 H, m). |
| | | | | 13C-NMR (CDCl3): 18.69; 18.74; 28.5; 28.6; 30.4; 30.5; 31.1; 31.11; 32.27; 32.34; 34.3; 36.0; 38.0; 40.2; 41.2; 41.6; 42.3; 44.0; 45.3; 56.2; 58.1; 60.7; 126.6; 127.4; 127.5; 127.66; 127.72; 171.0. |
| 415 | 2 | Bsp. Nr. 25/ Acylierung/ 46% | m/z: [M+H]+ = 351.3, Rt = 2.4 min. | 1H-NMR (CDCl3): 1.36-1.46 (2 H, m); 1.63-1.74 (2 H, m); 1.77 (1 H, t, J = 7.2 Hz); 1.85 (1 H, t, J = 7.2 Hz); 1.96-2.09 (4 H, m); 2.10 (6 H, s); 2.45 (1 H, t, J = 6.4 Hz); 2.51 (1 H, t, J = 6.6 Hz); 3.18 und 3.24 (2 H, 2 s); 3.31 und 3.34 (3 H, 2 s); 3.48-3.55 (2 H, m); 3.66 (1 H, t, J = 6.4 Hz); 3.69 (1 H, t, J = 6.6 Hz); 6.80-6.86 (1 H, m); 7.00-7.06 (1 H, m); 7.20-7.25 (1 H, m). |
| | | | | 13C-NMR (CDCl3): 30.9; 33.1; 34.7; 35.1; 36.3; 38.1; 40.1; 42.1; 44.1; 45.2; 55.9; 57.5; 58.8; 58.9; 59.7; 68.6; 123.4; 124.8; 126.17; 126.24; 143.0; 169.58; 169.61. |
| 416 | 2 | Bsp. Nr. 25/ Acylierung/ 65% | m/z: [M+H]+ = 349.3, Rt = 2.9 min. | 1H-NMR (CDCl3): 0.92 (3 H, d, J = 6.3 Hz); 0.95 (3 H, d, J = 6.3 Hz); 1.37-1.45 (2 H, m); 1.62-1.74 (2 H, m); 1.77 (1 H, t, J = 7.2 Hz); 1.85 (1 H, t, J = 7.1 Hz); 2.00-2.10 (5 H, m); 2.109 und 2.11 (6 H, 2 s); 2.12-2.20 (2 H, m); 3.15 und 3.24 (2 H, 2 s); 3.46-3.55 (2 H, m); 6.82-6.87 (1 H, m); 7.00-7.06 (1 H, m); 7.21-7.26 (1 H, m). |
| | | | | 13C-NMR (CDCl3): 22.6; 22.7; 25.5; 25.6; 30.9; 33.1; 34.4; 36.3; 38.1; 40.0; 42.1; 43.3; 43.7; 44.1; 45.4; 56.0; 57.7; 59.8; 123.4; 124.9; 126.2; 126.3; 143.0; 171.38; 171.44. |
| 418 | 1 | Bsp. Nr. 426/ Acylierung/ 73% | m/z: [M+H]+= 397.3 (41 %) [MH-NHMe2]+= 352.3 (100 %), Rt = 2.9 min. | 1H-NMR (CDCl3): 1.26 (6 H, s); 1.34-1.42 (2 H, m); 1.55-1.68 (4 H, m); 1.84-1.91 (2 H, m); 1.96-2.05 (2 H, m); 2.12 (6 H, s); 3.35 (3 H, s); 3.43 (2 H, s); 3.45 (2 H, s); 3.56 (2 H, br. s); 6.37-6.39 (1 H, m); 6.41-6.43 (1 H, m). |
| | | | | 13C-NMR (CDCl3): 22.9; 30.7; 32.5; 38.1; 43.6; 46.2; 53.4; 57.2; 59.3; 60.1; 80.2; 106.2; 106.3; 110.0; 121.1; 162.5; 165.4; 174.8. |
| 552 | 1 | Bsp. Nr. 86/ N-Demethylier ung/ 34% | m/z: [M+H]+ = 341.3 (88 %) [MH-H2NMe]+ = 310.3 (100 %), Rt = 3.0 min. | 1H-NMR (CDCl3): 1.34-1.46 (2 H, m); 1.45-1.64 (1 H, m); 1.64-1.98 (11 H, m); 2.00 (1.3 H, s); 2.01 (1.7 H, s); 2.02-2.10 (1 H, m); 2.20-2.10 (2 H, m); 2.36 (1 H, d, J = 7.3 Hz); 2.38 (1 H, d, J = 7,3 Hz); 2.70-2.80 (1 H, m); 3.30 (1.2 H, s); 3.34 (0.8 H, s); 3.43-3.51 (2 H, m); 7.20-7.28 (1 H, m); 7.32-7.44 (4 H, m). |
| | | | | 13C-NMR (CDCl3): 4.4; 6.88; 6.99; 28.6; 30.7; 31.0; 32.2; 32.3; 34.0; 35.6; 39.5; 39.9; 40.3; 42.4; 44.1; 45.2; 56.8; 57.3; 58.1; 126.1; 126.3; 126.5; 128.3; 128.4; 128.5; 171.41; 171.45. |
| 521 | 2 | Bsp. Nr. 417 Stufe6/ Alkylierung/ 85% | m/z: [M+H]+= 395.3 (100 %) [MH-NHMe2]+= 350.3 (37 %), Rt = 2.6 min. | 1H-NMR (CDCl3): 0.40-0.43 (2 H, m); 0.75-0.78 (2 H, m); 1.43-1.49 (2 H, m); 1.72-1.78 (4 H, m); 1.91-1.97 (4 H, m); 2.11 (6 H, s); 2.19 (2 H, s); 3.25 (3 H, s); 3.28 (2 H, s); 3.39-3.43 (2 H, m); 6.39 (1 H, dd, J = 4.0 und 1.7 Hz); 6.42 (1 H, m). |
| | | | | 13C-NMR (CDCl3): 12.1; 30.4; 32.2; 32.7; 35.5; 38.0; 39.9; 44.4; 53.9; 58.9; 59.5; 60.0; 106.3; 106.4; 121.1; 162.5; 165.4; 173.5. |
| 422 | 1 | Bsp. Nr. 85/ N-Demethylier ung/ 30% | m/z: [M+H]+ = 327.3 (76 %) [MH-H2NMe]+ = 296.3 (100 %), Rt = 2.9 min. | 1H-NMR (CDCl3): 0.12-0.21 (2 H, m); 0.53-0.59 (2 H, m); 1.05-1.15 (1 H, m); 1.34-1.46 (2 H, m); 1.64-1.79 (4 H, m); 1.79-1.87 (2 H, m); 1.96-2.10 2H, m); 2.01 (3 H, s); 2.20 (2 H, t, J = 6.5 Hz); 3.28 (1.1 H, s); 3.38 (0.9 H, s); 3.44 (1.1 H, t, J = 7.1 Hz); 3.52 (0.9 H, t, J = 7.3 Hz); 7.20-7.29 (1 H, m); 7.32-7.43 (4 H, m). |
| | | | | 13C-NMR (CDCl3): 18.75; 18.76; 28.58; 28.62; 30.7; 30.9; 32.3; 32.4; 34.0; 35.5; 40.3; 41.2; 41.7; 42.3; 44.0; 45.3; 56.7; 57.2; 57.3; 58.2; 126.1; 126.2; 126.4; 128.2; 128.3; 171.1. |
| 423 | 1 | Bsp. Nr. 74/ N-Demethylier ung/ 33% | m/z: [M+H]+ = 335.3 (46 %) [MH-H2NMe]+ = 304.2 (100 %), Rt = 2.9 min. | 1H-NMR (CDCl3): 0.96 (3 H, d, J = 2.1 Hz); 0.97 (3 H, d, J = 2.1 Hz); 1.34-1.52 (3 H, m); 1.63-1.73 (3 H, m); 1.74-1.80 (1 H, m); 1.80-1.90 (2 H, m); 1.95-2.10 (2 H, m); 2.12 (3 H, d, J = 2.2 Hz); 2.13-2.15 (2 H, m); 2.15-2.23 (1 H, m); 3.30 (1.1 H, s); 3.36 (0.9 H, s); 3.49 (2 H, td, J = 13.9, 7.2 Hz); 6.88-6.91 (1 H, m); 6.94-6.98 (1 H, m); 7.20-7.24 (1 H, m). |
| | | | | 13C-NMR (CDCl3): 22.7; 22.8; 25.59; 25.62; 28.6; 28.7; 30.8; 30.9; 34.2; 40.2; 42.3; 43.3; 43.7; 44.0; 45.3; 563.; 56.7; 56.8; 57.8; 132.7; 123.8; 126.3; 126.4; 171.5. |

| | | | | |
|---|---|---|---|---|
| * 1= polar, 2= unpolar, 3 = ein Diastereomer | | | | |

### Untersuchungen zur Wirksamkeit der erfindungsgemäßen Verbindungen

### Messung der ORL1-Bindung

Die Verbindungen wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer Kᵢ-Wert in oder% Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 µM bestimmt.

### Analgesieprüfung im Tail-Flick-Test an der Ratte

Die analgetische Wirksamkeit der Testverbindungen wurde im Brennstrahl (Tail-flick)-Test an der Ratte nach der Methode von D'Amour and Smith (J. Pharm. Exp. Ther. 72, 74 79 (1941)) untersucht. Dazu wurden weibliche Sprague Dawley Ratten mit einem Gewicht zwischen 130 und 190 g verwendet. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel: [(T₁ - T₀)/(T₂ - T₀)] x 100. Dabei ist die T₀ die Latenzzeit vor und T₁ die Latenzzeit nach Substanzapplikation, T₂ ist die maximale Expositionszeit (12 sec).Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die ED₅₀-Werte mit Hilfe der Regressionsanalyse bestimmt. Die ED₅₀-Berechnung erfolgte im Wirkmaximum, 20 Minuten nach intravenöser Substanzgabe.

### Chung-Modell: Mononeuropathieschmerz nach spinaler Nervenligatur

Tiere: Männliche Sprague Dawley Ratten (140-160g), von einem kommerziellen Züchter (Janvier, Genest St. Isle, Frankreich), wurden unter einem 12:12h Licht-Dunkel Rhythmus gehalten. Die Tiere wurden mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren ®, 60 mg/kg i.p., Merial GmbH, Hallbergmoos, Deutschland), wurden die linken L5, L6 Spinalnerven exponiert, indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black,USP 5/0, metric 1, Braun Melsungen AG, Melsungen, Deutschland) (Kim and Chung 1992). Nach Ligatur wurden Muskel und benachbarte Gewebe zugenäht und die Wunde mittels Metallklammern geschlossen.

Nach einer Woche Erholungszeit wurden die Tiere zur Messung der mechanischen Allodynie in Käfige mit einem Drahtboden gesetzt. An ipsi- und/oder kontralateraler Hinterpfote wurde die Wegziehschwelle mittels eines elektronischen von Frey Filamentes (Somedic AB, Malmö, Schweden) bestimmt. Der Median von fünf Stimulierungen ergab einen Datenpunkt. Die Tiere wurden 30 min vor und zu verschiedenen Zeiten nach Applikation von Testsubstanz- oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt. Alternativ wurden die Wegziehschwellen in Gramm dargestellt.

Statistische Auswertung: ED₅₀ Werte und 95% Vertrauensbereiche wurden über semilogarithmische Regressionsanalyse zum Zeitpunkt der maximalen Wirkung bestimmt. Die Daten wurden über eine Varianzanalyse mit wiederholten Messungen sowie einer post hoc Analyse nach Bonferroni analysiert. Die Gruppengröße betrug üblicherweise n=10.

Referenzen: Kim, S.H. and Chung,J.M., An experimental model for peripheral neuropathy produced by segmental spinal nerve ligation in the rat, Pain, 50 (1992) 355-363.

**Ergebnisse**

| **Nr**. | **Diastereomer** | **% Hemmung (ORL1) [1 µM]** | **Ki (ORL1) Mittel [µM]** | **% Hemmung (µ) [1 µM]** | **Ki (µ) Mittel [µM]** | **Tail flick Ratte, i. v. ED₅₀ᵣₐₜ [µg/kg] or %MPE (@µg/kg)** | **SNL Ratte, i. v. ED₅₀ᵣₐₜ [µg/kg] or %MPE (@µg/kg)** |
|---|---|---|---|---|---|---|---|
| 1 | 1 | 9 | | 26 | 1.06 | nd | nd |
| 2 | 2 | 11.5 | | 4 | 4.79 | nd | nd |
| 3 | 1 | 15 | | 31.5 | 1.83 | nd | nd |
| 4 | 2 | 9 | | 28.5 | 0.92 | nd | nd |
| 5 | 1 | 15 | | 33 | 1.45 | nd | nd |
| 6 | 2 | 23.5 | | 49 | 1.1 | nd | nd |
| 7 | 1 | 16 | 2.16 | 37.5 | 1.59 | nd | nd |
| 8 | 2 | 25 | | 45.5 | 1.05 | nd | nd |
| 9 | 2 | 92.5 | 0.17 | 7.4 | 0.0045 | nd | nd |
| 10 | 1 | 27.67 | 2.02 | 67 | 0.41 | nd | nd |
| 113 | 2 | 43.5 | 0.16 | 71.5 | 0.034 | nd | nd |
| 12 | 2 | 74.33 | 0.12 | 100.5 | 0.0079 | nd | nd |
| 13 | 3 | 13 | | 34 | 1.885 | nd | nd |
| 14 | 1 | 57.5 | 0.068 | 76.5 | 0.026 | 83%@1000 | nd |
| 15 | 2 | 37.5 | 0.49 | 50.5 | 0.22 | nd | nd |
| 16 | 1 | 10 | | 0 | | nd | nd |
| 17 | 3 | 19 | | 28 | 0.895 | nd | nd |
| 18 | 1 | 47 | 0.175 | 90 | 0.018 | nd | nd |
| 19 | 1 | 70 | 0.08 | 85 | 0.044 | nd | nd |
| 20 | 1 | 82.5 | 0.0096 | 98.5 | 0.00227 | 26 | 30 |
| 21 | 1 | 89.5 | 0.0029 | 98.5 | 0.0028 | 414 | 105 |
| 22 | 1 | 94 | 0.0008 | 98.5 | 0.00114 | 100%@1000 | nd |
| 23 | 1 | 58 | 0.068 | 84.5 | 0.05 | nd | nd |
| 24 | 2 | 49.5 | 0.74 | 70 | 0.13 | nd | nd |
| 25 | 2 | 43.5 | 0.21 | 65.5 | 0.17 | nd | nd |
| 26 | 2 | 63 | 0.38 | 77.5 | 0.12 | nd | nd |
| 27 | 1 | 21.67 | | 61.5 | 1105 | nd | nd |
| 28 | 2 | 49 | 0.15 | 70.5 | 0.2 | nd | nd |
| 29 | 1 | 81.5 | 0.00585 | 93 | 0.0305 | 157 | 100 |
| 30 | 1 | 16.5 | | 32 | 4.04 | nd | nd |
| 31 | 1 | 57.5 | 1135 | 79.5 | 0.13 | nd | nd |
| 32 | 1 | 77.5 | 0.0155 | 93.5 | 0.00765 | 6.54 | 44%@6.81 |
| 33 | 2 | 31 | 0.425 | 16 | 1.2 | nd | nd |
| 34 | 2 | 57.5 | 0.805 | 41 | 12420 | nd | nd |
| 35 | 1 | 91 | 0.039 | 98.5 | 0.00815 | 0%@100 | nd |
| 36 | 1 | 73.5 | 0.023 | 98.5 | 0.00805 | nd | nd |
| 37 | 1 | 34.5 | 0.26 | 86.5 | 0.077 | nd | nd |
| 38 | 1 | 26 | 0.15 | 86 | 0.045 | nd | nd |
| 39 | 1 | 84.5 | 0.027 | 100.5 | 0.0048 | 100%@100 | nd |
| 40 | 1 | 34.5 | 0.15 | 80.5 | 0.125 | nd | nd |
| 41 | 1 | 64.5 | 0.064 | 83.5 | 0.078 | nd | nd |
| 42 | 1 | 63 | | 60 | | nd | nd |
| 43 | 1 | 73 | 0.00845 | | 0.00805 | 100%@100 | nd |
| 44 | 1 | 48 | 0.085 | 80.5 | 0.0115 | nd | nd |
| 45 | 1 | 61 | 0.102 | 85 | 0.077 | nd | nd |
| 46 | 1 | 94 | 0.00235 | 98 | 0.0048 | 3.53 | 28%@3 |
| 47 | 1 | 71.5 | 0.018 | 80.5 | 0.0465 | 5260 | nd |
| 48 | 1 | 94 | | 99.5 | | 3.36 | nd |
| 49 | 1 | 92.33 | 0.0145 | 98.5 | 0.00895 | nd | nd |
| 50 | 1 | 97.5 | 0.00024 | 99.5 | 0.00037 | 94%@10 | nd |
| 51 | 1 | 98 | 0.00145 | 99 | 0.0015 | nd | nd |
| 52 | 1 | 98 | 0.00052 | 101.5 | 0.0006 | 52.8 | nd |
| 53 | 1 | 97 | 0.0017 | 100 | 0.00102 | 63%@1000 | nd |
| 54 | 1 | 93.5 | 0.00044 | 100 | 0.0004 | nd | nd |
| 55 | 1 | 43.5 | 0.105 | 87 | 0.047 | nd | nd |
| 56 | 1 | 89 | 0.0034 | 99 | 0.00175 | 7.16 | nd |
| 57 | 1 | 64 | 0.0715 | 92 | 0.037 | 25%@100 | nd |
| 58 | 1 | 52.67 | 0.155 | 93.5 | 0.0535 | nd | nd |
| 59 | 1 | 97 | 0.00116 | 99.5 | 0.00062 | nd | nd |
| 60 | 1 | 77 | 0.036 | 93.5 | 0.0215 | 90%@1000 | nd |
| 61 | 1 | 78 | 0.02 | 96.5 | 0.00715 | 87%@100 | nd |
| 62 | 1 | 98 | 0.00072 | 99 | 0.00052 | nd | nd |
| 63 | 1 | 76 | 0.0825 | 90.5 | 0.0365 | nd | nd |
| 64 | 1 | 96 | 0.00205 | 100.5 | 0.0013 | 1.86 | nd |
| 65 | 1 | 89.5 | 0.0165 | 99.5 | 0.00305 | nd | nd |
| 66 | 1 | 81.5 | 0.0185 | 99.5 | 0.00545 | nd | nd |
| 67 | 1 | 92 | 0.00295 | 99.5 | 0.00107 | 14.6 | nd |
| 68 | 1 | 97.5 | 0.00073 | 100.5 | 0.00039 | nd | nd |
| 69 | 1 | 97.5 | 0.00076 | 99.5 | 0.00035 | nd | nd |
| 70 | 1 | 84.5 | 0.0305 | 94.5 | 0.022 | nd | nd |
| 71 | 1 | 62.5 | 0.057 | 65.5 | 0.215 | nd | nd |
| 72 | 1 | 81.5 | 0.00585 | 93 | 0.0305 | nd | nd |
| 73 | 1 | 43 | 0.161 | 58.5 | 0.275 | nd | nd |
| 74 | 1 | 80 | 0.00975 | 93 | 0.0065 | 40%@1000 | nd |
| 75 | 1 | 97 | 0.00111 | 99.5 | 0.00115 | 16.2 | 69%@21.5 |
| 76 | 1 | 96.5 | 0.00075 | 100.5 | 0.00104 | 15.2 | 65%@21.5 |
| 77 | 1 | 94.5 | 0.00247 | 99 | 0.00165 | 78.6 | nd |
| 78 | 1 | 86.5 | | 97.5 | | nd | nd |
| 79 | 1 | 98.5 | 0.0003 | 100 | 0.00036 | nd | nd |
| 80 | 1 | 60.5 | 0.24 | 90 | 0.0795 | nd | nd |
| 81 | 1 | 78.5 | 0.054 | 92.5 | 0.064 | 55%@1000 | nd |
| 82 | 1 | 90 | 0.015 | 97.5 | 0.00935 | 0%@100 | nd |
| 83 | 1 | 97.5 | 0.00056 | 99.5 | 0.0004 | 8.03 | nd |
| 84 | 1 | 81 | 0.0215 | 95.5 | 0.0134 | nd | nd |
| 85 | 1 | 94.5 | 0.0018 | 99.5 | 0.00113 | 16.3 | nd |
| 86 | 1 | 95.5 | 0.00045 | 101.5 | 0.00063 | 4.17 | nd |
| 87 | 1 | 96 | 0.00106 | 100 | 0.00075 | 0%@100 | nd |
| 88 | 3 | 72 | 0.0735 | 97.5 | 0.0125 | nd | nd |
| 89 | 3 | 89 | | 100 | | nd | nd |
| 90 | 1 | 86.5 | 0.03 | 97.5 | 0.026 | nd | nd |
| 91 | 1 | 86.5 | 0.0068 | 95.5 | 0.00805 | nd | nd |
| 92 | 3 | 41395 | | 23 | 3585 | nd | nd |
| 93 | 3 | 12 | 24108 | 92 | 0.0265 | nd | nd |
| 94 | 3 | 35.5 | 0.8 | 102.5 | 0.0053 | nd | nd |
| 95 | 3 | 46 | 0.44 | 96.5 | 0.0265 | nd | nd |
| 96 | 3 | 57.5 | 0.275 | 103 | 0.0068 | nd | nd |
| 97 | 3 | 67.5 | | 94.5 | | nd | nd |
| 98 | 3 | 85 | 0.019 | 99.5 | 0.0096 | nd | nd |
| 99 | 3 | 67.5 | 0.028 | 97 | 0.0052 | 100%@100 | nd |
| 100 | 3 | 45 | 0.185 | 86.5 | 0.052 | nd | nd |
| 101 | 3 | 82.5 | 0.011 | 88.67 | 0.017 | 0%@1000 | nd |
| 102 | 3 | 94.5 | 0.00435 | 99.5 | 0.00465 | nd | nd |
| 103 | 3 | 79 | 0.0295 | 99.5 | 0.00405 | 5.43 | 2.44 |
| 104 | 3 | 88.5 | 0.0155 | 98.5 | 0.00245 | 162 | 107 |
| 105 | 3 | 90 | 0.00625 | 99.5 | 0.00069 | nd | nd |
| 106 | 1 | 78 | 0.62 | 65.5 | 1775 | nd | nd |
| 107 | 2 | 13.5 | | 54.5 | 0.95 | nd | nd |
| 108 | 1 | 21.5 | 0.91 | 54 | 0.64 | nd | nd |
| 109 | 2 | 8 | | 6 | | nd | nd |
| 110 | 1 | 24.5 | 1.52 | 43.5 | 1.21 | nd | nd |
| 111 | 2 | 0 | | 5 | | nd | nd |
| 112 | 1 | 59.5 | | 54 | | nd | nd |
| 113 | 2 | 38.5 | 1.34 | 44 | 2.54 | nd | nd |
| 114 | 3 | 30 | | 34 | 0.71 | nd | nd |
| 115 | 3 | 28 | 0.6 | 65 | 0.17 | nd | nd |
| 116 | 3 | 20 | 1.57 | 67.5 | 0.145 | nd | nd |
| 117 | 3 | 15 | | 31.5 | 2.55 | nd | nd |
| 118 | 2 | 6.5 | | 3.5 | 5.5 | nd | nd |
| 119 | 1 | 29 | | 23 | 3.57 | nd | nd |
| 120 | 2 | 13 | | 31 | 7.07 | nd | nd |
| 121 | 1 | 26 | 1.51 | 58 | 0.23 | nd | nd |
| 122 | 2 | 21 | | 45 | 0.67 | nd | nd |
| 123 | 1 | 22 | 1.88 | 71.5 | 0.15 | nd | nd |
| 124 | 1 | 55 | 0.089 | 79.5 | 0.17 | nd | nd |
| 125 | 1 | 87 | 0.0039 | 98 | 0.00615 | 7.09 | nd |
| 126 | 1 | 97 | 0.00088 | 99 | 0.00185 | nd | nd |
| 127 | 2 | 30 | 0.36 | 25.5 | 3.97 | nd | nd |
| 128 | 1 | 98 | 0.00042 | 100.5 | 0.00034 | nd | nd |
| 129 | 2 | 47 | 0.022 | 51.5 | 0.36667 | nd | nd |
| 130 | 1 | 90.5 | 0.00075 | 99 | 0.00064 | nd | nd |
| 131 | 2 | 47 | 0.22 | 37 | 1.18 | nd | nd |
| 132 | 1 | 91.5 | 0.00079 | 99 | 0.0014 | nd | nd |
| 133 | 2 | 47.5 | 0.175 | 32 | 1.4 | nd | nd |
| 134 | 1 | 87 | | 98 | | 100%@100 | nd |
| 135 | 2 | 47.5 | 0.195 | 35.5 | 1.51 | nd | nd |
| 136 | 1 | 87.5 | 0.00135 | 100 | 0.00102 | nd | nd |
| 137 | 2 | 43 | 0.475 | 44 | 0.595 | 100%@100 | nd |
| 138 | 1 | 74 | 0.0215 | 97 | 0.00705 | 30.1 | nd |
| 139 | 2 | 28.5 | 0.68 | 23 | 3.88 | nd | nd |
| 140 | 2 | 34 | 0.235 | 44 | 1.25 | nd | nd |
| 141 | 1 | 90.5 | 0.0027 | 97.5 | 0.0019 | 4.84 | nd |
| 142 | 1 | 85 | 0.00575 | 94 | 0.0074 | 126 | 96 |
| 143 | 2 | 33 | 0.35 | 24 | 1.38 | nd | nd |
| 144 | 1 | 91 | 0.0041 | 99.5 | 0.00205 | 11.5 | 64%@21.5 |
| 145 | 2 | 53 | 0.0675 | 77 | 0.161 | nd | nd |
| 146 | 1 | 80.5 | 0.016 | 94.5 | 0.03 | nd | nd |
| 147 | 2 | 46 | 0.225 | 50.5 | 0.59 | nd | nd |
| 148 | 1 | 78 | 0.032 | 91 | 0.0425 | 70%@1000 | nd |
| 149 | 2 | 22.5 | 0.785 | 54.5 | 3.41 | nd | nd |
| 150 | 1 | 95 | 0.00205 | 99 | 0.0004 | 90%@10 | nd |
| 151 | 2 | 63 | 0.022 | 95 | 0.029 | nd | nd |
| 152 | 1 | 14 | 1.2 | 42 | 0.885 | nd | nd |
| 153 | 2 | 36.5 | 0.41 | 72 | 0.28 | nd | nd |
| 154 | 2 | 45 | 0.19 | 63.5 | 0.145 | nd | nd |
| 155 | 1 | 19 | 0.79 | 21.5 | 2.82 | nd | nd |
| 156 | 2 | 86 | 0.006 | 98 | 0.0036 | nd | nd |
| 157 | 1 | 24 | 0.805 | 11.5 | 4.77 | nd | nd |
| 158 | 2 | 94 | 0.00056 | 100 | 0.00054 | nd | nd |
| 159 | 1 | 31.5 | 0.0915 | 32.5 | 0.185 | nd | nd |
| 160 | 1 | 33 | 0.455 | 44 | 1.13 | nd | nd |
| 161 | 1 | 63 | 0.345 | 97 | 0.0535 | nd | nd |
| 162 | 2 | 82 | 0.029 | 95 | 0.038 | 422 | nd |
| 163 | 2 | 93.5 | 0.0022 | 97 | 0.003 | nd | nd |
| 164 | 1 | 39 | 0.28 | 73 | 0.365 | nd | nd |
| 165 | 2 | 96 | | 100 | | nd | nd |
| 166 | 1 | 76 | 0.101 | 83 | 0.17 | nd | nd |
| 167 | 2 | 97 | 0.00071 | 100.67 | 0.00108 | nd | nd |
| 168 | 1 | 37.5 | 0.235 | 70 | 2.06 | nd | nd |
| 169 | 2 | 95.5 | 0.00103 | 102 | 0.0012 | nd | nd |
| 170 | 1 | 56.5 | 0.0825 | 37 | 0.63 | nd | nd |
| 171 | 2 | 91 | 0.0047 | 99 | 0.003 | 26.8 | nd |
| 172 | 1 | 39.5 | 0.083 | 35.5 | 0.63 | nd | nd |
| 173 | 2 | 96.5 | 0.00032 | 97.5 | 0.00061 | nd | nd |
| 174 | 1 | 49.5 | 0.135 | 28 | 1.67 | nd | nd |
| 175 | 2 | 96 | 0.00395 | 99.5 | 0.0024 | 55 | 72%@46.4 |
| 176 | 1 | 63 | 0.1045 | 72 | 0.175 | nd | nd |
| 177 | 2 | 97.33 | 0.00165 | 99.5 | 0.0012 | 100%@100 | nd |
| 178 | 1 | 82.5 | 0.0395 | 96.5 | 0.0185 | nd | nd |
| 179 | 2 | 93 | 0.0067 | 99 | 0.0075 | nd | nd |
| 180 | 1 | 49.5 | 0.18 | 66.5 | 0.3 | nd | nd |
| 181 | 2 | 77 | 0.01015 | 91.5 | 0.044 | nd | nd |
| 182 | 2 | 96.5 | 0.0013 | 97 | 0.00075 | 6.92 | nd |
| 183 | 1 | 39.5 | 0.3 | 23.5 | 0.795 | nd | nd |
| 184 | 2 | 97 | 0.00066 | 95.5 | 0.0003 | 3.25 | nd |
| 185 | 1 | 49 | 0.1045 | 60.5 | 0.255 | nd | nd |
| 186 | 2 | 98.5 | 0.00205 | 99.5 | 0.0032 | nd | nd |
| 187 | 1 | 59 | 0.34 | 58.5 | 41579 | nd | nd |
| 188 | 1 | 91.5 | 0.0026 | 98 | 0.0026 | nd | nd |
| 189 | 1 | 77.5 | 0.0425 | 93.5 | 0.036 | nd | nd |
| 190 | 1 | 57 | 0.18 | 85.5 | 0.049 | nd | nd |
| 191 | 1 | 93 | 0.0015 | 98 | 0.00195 | 0%@100 | nd |
| 192 | 1 | 96 | 0.0007 | 97.5 | 0.00044 | nd | nd |
| 193 | 1 | 89 | 0.0097 | 97 | 0.0061 | nd | nd |
| 194 | 1 | 92.5 | 0.0023 | 98 | 0.00135 | nd | nd |
| 195 | 1 | 93.5 | 0.0005 | 99.5 | 0.00053 | 100%@100 | nd |
| 196 | 1 | 71.5 | | 95 | | nd | nd |
| 197 | 1 | 95.5 | 0.00046 | 99.5 | 0.00048 | 100%@100 | nd |
| 198 | 1 | 48.5 | 0.1025 | 87 | 0.084 | nd | nd |
| 199 | 1 | 72 | 0.073 | 93 | 0.03 | nd | nd |
| 200 | 1 | 89 | 0.0119 | 98.5 | 0.00325 | nd | nd |
| 201 | 1 | 94 | 0.0067 | 97.5 | 0.0039 | 0%@100 | nd |
| 202 | 1 | 88.5 | 0.0125 | 98 | 0.0054 | nd | nd |
| 203 | 1 | 97.33 | 0.00127 | 98 | 0.00072 | nd | nd |
| 204 | 1 | 95 | 0.00063 | 98 | 0.00145 | nd | nd |
| 205 | 1 | 48.5 | 0.185 | 82.5 | 0.0745 | nd | nd |
| 206 | 1 | 79 | 0.035 | 92 | 0.0255 | nd | nd |
| 207 | 1 | 94.5 | 0.00417 | 99.5 | 0.00475 | nd | nd |
| 208 | 1 | 96 | 0.00205 | 100 | 0.00067 | nd | nd |
| 209 | 1 | 60 | 0.1125 | 94 | 0.0315 | nd | nd |
| 210 | 1 | 80 | 0.023 | 94.5 | 0.0108 | nd | nd |
| 211 | 1 | 95.5 | 0.0053 | 99.5 | 0.00073 | nd | nd |
| 212 | 1 | 82.5 | 0.01967 | 99.33 | 0.00305 | nd | nd |
| 213 | 1 | 98 | 0.00034 | 101.5 | 0.00072 | nd | nd |
| 214 | 2 | 41.5 | 0.23 | 79.5 | 0.0645 | nd | nd |
| 215 | 2 | 42 | 0.305 | 59 | 0.285 | nd | nd |
| 216 | 1 | 85 | 0.00955 | 98.5 | 0.00615 | 52%@100 | nd |
| 217 | 1 | 97 | 0.00115 | 100 | 0.0004 | 0.7 | nd |
| 218 | 2 | 50.5 | 0.185 | 76.5 | 0.165 | nd | nd |
| 219 | 2 | 36 | 0.315 | 46 | 0.945 | nd | nd |
| 220 | 1 | 99 | 0.0012 | 97 | 0.00041 | nd | nd |
| 221 | 1 | 96.5 | 0.002 | 101 | 0.0048 | nd | nd |
| 222 | 2 | 46.5 | 0.265 | 62 | 0.605 | nd | nd |
| 223 | 1 | 55 | 0.082 | 90.5 | 0.052 | nd | nd |
| 224 | 2 | 73.5 | 0.036 | 97 | 0.02 | nd | nd |
| 225 | 1 | 49 | 0.23 | 63 | 0.545 | nd | nd |
| 226 | 1 | 94 | 0.0035 | 98 | 0.00255 | nd | nd |
| 227 | 1 | 81.5 | 0.0165 | 94 | 0.0175 | nd | nd |
| 228 | 1 | 46 | 0.23 | 79 | 0.082 | nd | nd |
| 229 | 1 | 66 | 0.025 | 87 | 0.0445 | nd | nd |
| 230 | 2 | 96.5 | 0.00315 | 98.5 | 0.0013 | nd | nd |
| 231 | 1 | 43 | 0.18 | 91.5 | 0.0585 | nd | nd |
| 232 | 1 | 97.5 | 0.00285 | 97.5 | 0.0031 | nd | nd |
| 233 | 1 | 96 | 0.00225 | 98.5 | 0.0013 | nd | nd |
| 234 | 2 | 81.5 | 0.0225 | 99.5 | 0.00435 | nd | nd |
| 235 | 1 | 98.5 | 0.00047 | 100.5 | 0.00044 | 1.44 | nd |
| 236 | 2 | 91.5 | 0.016 | 100 | 0.00155 | nd | nd |
| 237 | 1 | 97.5 | 0.00058 | 99.5 | 0.00055 | 100%@10 | nd |
| 238 | 1 | 98 | 0.00095 | 103 | 0.00082 | nd | nd |
| 239 | 1 | 98 | 0.00083 | 103.5 | 0.00069 | nd | nd |
| 240 | 1 | 45 | 0.195 | 91 | 0.08 | nd | nd |
| 241 | 2 | 89.5 | 0.015 | 99 | 0.00875 | nd | nd |
| 242 | 1 | 38.5 | 0.45 | 51 | 0.87 | nd | nd |
| 243 | 1 | 36 | 41518 | 78 | 0.12 | nd | nd |
| 244 | 2 | 26 | 0.255 | 74.5 | 0.0985 | nd | nd |
| 245 | 2 | 59.5 | 0.115 | 95 | 0.0066 | nd | nd |
| 246 | 2 | 40 | 0.205 | 87.5 | 0.034 | nd | nd |
| 247 | 3 | 91.5 | 0.01015 | 98.5 | 0.00205 | 100%@100 | nd |
| 248 | 1 | 90.5 | 0.00875 | 100 | 0.0012 | nd | nd |
| 249 | 1 | 88 | 0.0117 | 99 | 0.002 | nd | nd |
| 250 | 2 | 94.5 | 0.0019 | 99.5 | 0.00079 | nd | nd |
| 251 | 2 | 82 | 0.0215 | 87 | 0.054 | nd | nd |
| 252 | 1 | 46.5 | 0.3 | 37.5 | 14.305 | nd | nd |
| 253 | 1 | 97.5 | 0.00165 | 98.5 | 0.0025 | nd | nd |
| 254 | 2 | 94.5 | 0.00875 | 94 | 0.0185 | nd | nd |
| 255 | 1 | 37.5 | 0.26 | 58.5 | 4385 | nd | nd |
| 256 | 2 | 70.5 | 0.064 | 97.33 | 0.00565 | nd | nd |
| 257 | 3 | 89 | 0.01365 | 98 | 0.0028 | nd | nd |
| 258 | 3 | 63.5 | 0.084 | 97.5 | 0.00465 | nd | nd |
| 259 | 3 | 67 | 0.062 | 95.5 | 0.062 | nd | nd |
| 260 | 2 | 90 | 0.00865 | 93.5 | 0.0535 | nd | nd |
| 261 | 1 | 42.5 | 0.395 | 40 | 1.4 | nd | nd |
| 262 | 2 | 98.5 | 0.00155 | 98 | 0.00067 | 6.6 | nd |
| 262 | 1 | 50.5 | 0.245 | 37 | 1.51 | nd | nd |
| 264 | 2 | 96.5 | 0.00275 | 98.5 | 0.0034 | 36.7 | nd |
| 265 | 3 | 71 | 0.029 | 95.5 | 0.006 | nd | nd |
| 266 | 3 | 91 | 0.00633 | 100 | 0.00145 | nd | nd |
| 267 | 3 | 64 | 0.039 | 97.7 | 0.0016 | nd | nd |
| 268 | 3 | 76.5 | 0.093 | 99 | 0.01225 | nd | nd |
| 269 | 2 | 89.5 | 0.0125 | 93 | 0.01005 | nd | nd |
| 270 | 1 | 33.5 | 0.31 | 34 | 1.16 | nd | nd |
| 271 | 1 | 54 | 0.245 | 53.5 | 1.41 | nd | nd |
| 272 | 1 | 91 | 0.00705 | 95.5 | 0.00745 | nd | nd |
| 273 | 3 | 81 | | 89 | | nd | nd |
| 274 | 2 | 0 | 0.00745 | 0 | 0.002 | nd | nd |
| 275 | 2 | 92 | 0.0079 | 96.5 | 0.00435 | nd | nd |
| 276 | 2 | 73.5 | 0.021 | 95 | 0.0165 | nd | nd |
| 277 | 2 | 91 | 0.0064 | 99 | 0.0008 | nd | nd |
| 278 | 2 | 88.5 | 0.0079 | 99 | 0.0014 | nd | nd |
| 279 | 2 | 93 | 0.01225 | 98.5 | 0.0106 | nd | nd |
| 280 | 2 | 93.5 | 0.0088 | 97 | 0.0076 | nd | nd |
| 281 | 2 | 75.5 | 0.0635 | 99.5 | 0.0087 | nd | nd |
| 282 | 2 | 19.5 | 1.38 | 67.5 | 0.295 | nd | nd |
| 283 | 1 | 91.5 | 0.031 | 99.5 | 0.011 | nd | nd |
| 284 | 1 | 92.5 | 0.0195 | 95.5 | 0.00765 | nd | nd |
| 285 | 1 | 95 | 0.0034 | 99 | 0.00255 | nd | nd |
| 286 | 2 | 88 | 0.02 | 99.5 | 0.0013 | nd | nd |
| 287 | 2 | 83 | 0.027 | 99 | 0.00105 | nd | nd |
| 288 | 2 | 87.5 | 0.00595 | 98.5 | 0.0046 | nd | nd |
| 289 | 1 | 45.5 | 0.17 | 69 | 0.27 | nd | nd |
| 290 | 2 | 82 | 0.019 | 97 | 0.00565 | nd | nd |
| 291 | 3 | 0.5 | 1225 | 66 | 0.175 | nd | nd |
| 292 | 3 | 69 | 0.163 | 98 | 0.0084 | nd | nd |
| 293 | 2 | 43.5 | 0.265 | 92 | 0.029 | nd | nd |
| 294 | 2 | 52.5 | 0.07733 | 95 | 0.0275 | nd | nd |
| 295 | 1 | 89 | 0.018 | 98.5 | 0.00635 | nd | nd |
| 296 | 1 | 87 | 0.0111 | 98 | 0.0053 | nd | nd |
| 297 | 3 | 26 | 0.91 | 90 | 0.055 | nd | nd |
| 298 | 3 | 85 | 0.0205 | 98 | 0.00305 | nd | nd |
| 299 | 1 | 94.5 | 0.00395 | 101 | 0.00061 | nd | nd |
| 300 | 1 | 92 | 0.0073 | 100 | 0.00155 | nd | nd |
| 301 | 3 | 66 | 0.071 | 90.5 | 0.0495 | nd | nd |
| 302 | 3 | 48 | 0.1225 | 83 | 0.06 | nd | nd |
| 303 | 1 | 93 | 0.0051 | 97.5 | 0.007 | nd | nd |
| 304 | 1 | 95 | 0.00445 | 99 | 0.0049 | nd | nd |
| 305 | 1 | 93 | 0.00245 | 99.5 | 0.00093 | nd | nd |
| 306 | 1 | 97 | 0.00114 | 96.5 | 0.00057 | nd | nd |
| 307 | 1 | 14.5 | 0.535 | 19 | 0.54 | nd | nd |
| 308 | 2 | 92 | 0.01005 | 99 | 0.00155 | nd | nd |
| 309 | 1 | 94 | 0.00113 | 100 | 0.0015 | nd | nd |
| 310 | 1 | 52 | 0.195 | 29.5 | 1.03 | nd | nd |
| 311 | 2 | 95.5 | 0.0021 | 98 | 0.00125 | nd | nd |
| 312 | 1 | 54.5 | 0.12 | 74.5 | 0.295 | nd | nd |
| 313 | 2 | 92.5 | 0.00345 | 99.5 | 0.00415 | nd | nd |
| 314 | 1 | 94 | 0.0011 | 100 | 0.00073 | nd | nd |
| 315 | 1 | 90 | 0.01055 | 100 | 0.0045 | 19.4 | nd |
| 316 | 1 | 10.5 | 1.23 | 27 | 2.15 | nd | nd |
| 317 | 2 | 92.5 | 0.0027 | 99 | 0.0015 | nd | nd |
| 318 | 2 | 88.5 | 0.0092 | 97.5 | 0.00715 | nd | nd |
| 319 | 2 | 91 | 0.005 | 96.5 | 0.005 | nd | nd |
| 320 | 1 | 93 | 0.0019 | 99.5 | 0.0018 | nd | nd |
| 321 | 1 | 52 | 0.086 | 83 | 0.1495 | nd | nd |
| 322 | 2 | 97 | 0.00165 | 101 | 0.00138 | nd | nd |
| 323 | 2 | 93 | 0.00275 | 98 | 0.00905 | nd | nd |
| 324 | 2 | 95 | 0.00135 | 100 | 0:0024 | nd | nd |
| 325 | 2 | 88 | 0.0084 | 95 | 0.013 | nd | nd |
| 326 | 2 | 96 | 0.00375 | 97.5 | 0.0043 | nd | nd |
| 327 | 1 | 93 | 0.00555 | 101.5 | 0.00155 | nd | nd |
| 328 | 1 | 96 | 0.00325 | 99 | 0.0024 | nd | nd |
| 329 | 1 | 92 | 0.0088 | 97.5 | 0.0059 | nd | nd |
| 330 | 1 | 24 | 0.69 | 60 | 0.97 | nd | nd |
| 331 | 2 | 91 | 0.0076 | 100 | 0.00165 | nd | nd |
| 332 | 2 | 96 | 0.0018 | 104 | 0.0027 | nd | nd |
| 333 | 1 | 96 | 0.0055 | 102.5 | 0.0024 | nd | nd |
| 334 | 2 | 95.5 | 0.0021 | 101 | 0.0052 | nd | nd |
| 335 | 2 | 95.5 | 0.0015 | 99 | 0.0043 | nd | nd |
| 336 | 2 | 93.5 | 0.0035 | 101 | 0.0014 | nd | nd |
| 337 | 2 | 91 | 0.0021 | 101.5 | 0.00041 | nd | nd |
| 338 | 1 | 96.5 | 0.002 | 97.5 | 0.0026 | nd | nd |
| 339 | 1 | 93 | 0.0046 | 101 | 0.001 | nd | nd |
| 340 | 2 | 95.5 | 0.00175 | 99 | 0.00285 | nd | nd |
| 341 | 2 | 90.5 | 0.00935 | 99 | 0.0013 | nd | nd |
| 342 | 1 | 94 | 0.0021 | 99.5 | 0.0032 | nd | nd |
| 343 | 1 | 66 | 0.0595 | 92 | 0.024 | nd | nd |
| 344 | 2 | 95.5 | 0.00143 | 99.5 | 0.00135 | nd | nd |
| 345 | 1 | 48.5 | 0.15 | 90.5 | 0.0655 | nd | nd |
| 346 | 1 | 93.5 | 0.00435 | 101 | 0.00165 | nd | nd |
| 347 | 2 | 95.5 | 0.013 | 99 | 0.0037 | nd | nd |
| 348 | 2 | 94 | 0.013 | 98.5 | 0.0047 | nd | nd |
| 349 | 2 | 96.5 | 0.0018 | 98 | 0.0036 | nd | nd |
| 350 | 1 | 92 | 0.01 | 95 | 0.0019 | nd | nd |
| 351 | 1 | 93.5 | 0.0098 | 98.5 | 0.0016 | nd | nd |
| 352 | 1 | 82 | 0.047 | 93.5 | 0.031 | nd | nd |
| 353 | 2 | 98.5 | 0.00046 | 100 | 0.00098 | nd | nd |
| 354 | 1 | 99 | 0.00052 | 100.5 | 0.00044 | nd | nd |
| 355 | 1 | 98.5 | 0.00047 | 100 | 0.00025 | nd | nd |
| 356 | 1 | 65 | 0.0855 | 91.5 | 0.0235 | nd | nd |
| 357 | 2 | 98 | 0.00061 | 100.5 | 0.00155 | nd | nd |
| 358 | 1 | 94 | 0.0039 | 98.5 | 0.0039 | nd | nd |
| 359 | 1 | 92 | 0.0076 | 98 | 0.0025 | nd | nd |
| 360 | 1 | 85 | 0.019 | 96 | 0.013 | nd | nd |
| 361 | 1 | 91 | 0.0032 | 99 | 0.00145 | nd | nd |
| 362 | 1 | 94.5 | 0.00086 | 98.5 | 0.00083 | nd | nd |
| 363 | 1 | 95.5 | 0.00086 | 101 | 0.00037 | nd | nd |
| 364 | 1 | 61.5 | 0.044 | 90.5 | 0.017 | nd | nd |
| 365 | 1 | 71.5 | 0.02 | 89.5 | 0.0205 | nd | nd |
| 366 | 1 | 95 | 0.00064 | 99.5 | 0.00135 | nd | nd |
| 367 | 1 | 66.5 | 0.0405 | 98 | 0.016 | nd | nd |
| 368 | 1 | 60.5 | 0.058 | 96.5 | 0.01215 | nd | nd |
| 369 | 1 | 88 | 0.0115 | 97.5 | 0.011 | nd | nd |
| 370 | 2 | 93.5 | 0.00053 | 97.5 | 0.00043 | nd | nd |
| 371 | 1 | 96 | 0.00073 | 99 | 0.00106 | nd | nd |
| 372 | 1 | 95 | 0.00145 | 99 | 0.00125 | nd | nd |
| 373 | 1 | 93.5 | 0.00037 | 100.5 | 0.00066 | nd | nd |
| 374 | 1 | 96 | 0.00038 | 100.5 | 0.00057 | nd | nd |
| 375 | 1 | 77 | 0.043 | 99 | 0.0135 | nd | nd |
| 376 | 2 | 93 | 0.00199 | 99 | 0.0005 | nd | nd |
| 377 | 2 | 90 | 0.0065 | 99.5 | 0.00405 | nd | nd |
| 378 | 2 | 90.5 | 0.00525 | 98 | 0.00615 | nd | nd |
| 379 | 1 | 58 | 0.1045 | 93.5 | 0.045 | nd | nd |
| 38009 | 1 | 79 | 0.01685 | 97.5 | 0.00635 | nd | nd |
| 381 | 1 | 74 | 0.0275 | 96 | 0.0195 | nd | nd |
| 382 | 1 | 87 | 0.07 | 99 | 0.00915 | nd | nd |
| 383 | 1 | 78.5 | 0.0865 | 96 | 0.0135 | nd | nd |
| 384 | 1 | 95.5 | 0.00945 | 99 | 0.00165 | nd | nd |
| 385 | 1 | 74 | 0.055 | 99.5 | 0.00735 | nd | nd |
| 386 | 2 | 96.5 | 0.00091 | 100.5 | 0.00098 | 1.45 | nd |
| 387 | 2 | 97 | 0.00077 | 100 | 0.00067 | nd | nd |
| 388 | 1 | 76 | 0.03 | 97.33 | 0.00825 | nd | nd |
| 389 | 1 | 20.5 | 0.405 | 90 | 0.051 | nd | nd |
| 390 | 1 | 66.5 | 0.0545 | 96 | 0.0195 | nd | nd |
| 391 | 1 | 23.5 | 0.355 | 92 | 0.037 | nd | nd |
| 392 | 1 | 42 | 0.114 | 96.5 | 0.00635 | nd | nd |
| 393 | 1 | 57.5 | 0.048 | 98.5 | 0.00225 | nd | nd |
| 394 | 1 | 98 | 0.0015 | 100 | 0.00094 | nd | nd |
| 395 | 1 | 96.5 | 0.0017 | 101 | 0.00056 | nd | nd |
| 396 | 1 | 37 | 0.355 | 97.5 | 0.0115 | nd | nd |
| 397 | 1 | 48.5 | 0.1485 | 98 | 0.00525 | nd | nd |
| 398 | 1 | 55 | 0.098 | 99 | 0.0016 | nd | nd |
| 399 | 1 | 0 | | 20 | 5.77 | nd | nd |
| 400 | 2 | 34 | 0.825 | 86 | 0.0515 | nd | nd |
| 401 | 1 | 0 | | 23 | 2.76 | nd | nd |
| 402 | 2 | 34.5 | 0.51 | 89 | 0.038 | nd | nd |
| 403 | 1 | 6.5 | | 23.5 | 4.125 | nd | nd |
| 404 | 2 | 52.5 | 0.1385 | 96.5 | 0.0063 | nd | nd |
| 405 | 1 | 99.5 | 0.00034 | 101.5 | 0.00044 | nd | nd |
| 406 | 1 | 5 | | 31 | 4.65 | nd | nd |
| 407 | 1 | | 0.135 | 94.5 | 0.0099 | nd | nd |
| 408 | 2 | | 0.139 | 82.5 | 0.08 | nd | nd |
| 409 | 2 | | 1.27 | 78.5 | 0.081 | nd | nd |
| 410 | 1 | 8 | | 20.5 | 5.77 | nd | nd |
| 411 | 1 | | 0.1695 | 93 | 0.0225 | nd | nd |
| 412 | 1 | | 0.00051 | 102.5 | 0.00044 | nd | nd |
| 413 | 2 | | 0.0605 | 98 | 0.00345 | nd | nd |
| 414 | 2 | 67.5 | 0.0665 | 83.5 | 0.0595 | nd | nd |
| 415 | 2 | 46.5 | 0.245 | 81 | 0.0685 | nd | nd |
| 416 | 2 | 53 | 0.165 | 87.5 | 0.069 | nd | nd |
| 417 | 2 | 93.5 | 0.0022 | 99.5 | 0.0004 | nd | nd |
| 418 | 1 | 72 | 0.049 | 99 | 0.00265 | nd | nd |
| 419 | 1 | 44.5 | 0.12 | 92 | 0.0385 | nd | nd |
| 420 | 1 | 92.5 | 0.00605 | 100.5 | 0.0013 | nd | nd |
| 421 | 2 | 95.5 | 0.00129 | 99.5 | 0.00031 | nd | nd |
| 422 | 1 | 73.5 | 0.01125 | 98.5 | 0.00265 | nd | nd |
| 423 | 1 | 72 | 0.018 | 98 | 0.00415 | nd | nd |
| 424 | | | | | | | |
| 425 | | | | | | | |
| 426 | | | | | | | |
| 427 | | | | | | | |
| 428 | | | | | | | |
| 429 | | | | | | | |
| 430 | | | | | | | |
| 431 | | | | | | | |
| 432 | | | | | | | |
| 433 | | | | | | | |

| | | | | | | | |
|---|---|---|---|---|---|---|---|
| nd = not determined * 1= polar, 2= unpolar, 3 = ein Diastereomer | | | | | | | |

Sofern die in vorstehender Tabelle zusammengefassten experimentellen Daten den Anschein erwecken, dass einzelne erfindungsgemäße Verbindungen eine vergleichsweise nur geringe Rezeptoraffinität aufweisen, kann daraus nicht gefolgert werden, dass diese Verbindungen pharmakologisch völlig unwirksam sind. Vielmehr hängen diese Messergebnisse mit der prinzipiell willkürlich gewählten Testkonzentration von 1 µM zusammen. Es kann davon ausgegangen werden, dass bei entsprechend höherer Testkonzentration, z.B. bei 10 µM, auch deutlich größere Werte für die Rezeptoraffinität gemessen werden würden.

## Patentansprüche

1. Verbindung der allgemeinen Formel (1), 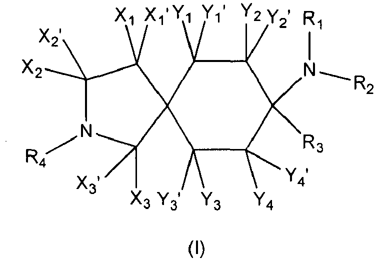 worin
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂ -CHO, -R₀, -C(=O)R₀, -C(=O)H, -C(=O)-OH, -C(=O)OR₀, -C(=O)NH₂ -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NHC(=O)N(R₀)₂; oder Y₁ und Y₁', oder Y₂ und Y₂', oder Y₃ und Y₃', oder Y₄ und Y₄' gemeinsam für =O stehen;
X₁, X₁', X₂, X₂', X₃ und X₃' jeweils unabhängig voneinander für -H, -F, -Cl, -Br, -I, -NO₂ -CF₃, -OR₅, -SR₅, -SO₂R₅, -S(=O)₂OR₅, -CN, -COOR₅, -CONR₅, -NR₆R₇, oder -R₀ stehen; oder X₁ und X₁', oder X₂ und X₂', oder X₃ und X₃' gemeinsam für =O stehen;
oder X₁ und X₂, oder X₂ und X₃ gemeinsam für -(CH₂)₂₋₆- stehen, wobei einzelne Wasserstoffatome auch durch -F, -Cl, -Br, -I, -NO₂ -CF₃, -OR₅, -CN oder -C₁₋₆-Aliphat ersetzt sein können;
oder X₁ und X₁' oder X₂ und X₂' oder X₃ und X₃' gemeinsam für ein C₃₋₆-Cycloaliphat stehen, wobei einzelne Wasserstoffatome auch durch -F, -Cl, -Br, -I, -NO₂ -CF₃, -OR₅, -CN oder -C₁₋₆-Aliphat ersetzt sein können;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₈CH₂CH₂- oder -(CH₂)₃₋₆- stehen;
R₃ für -R₀ steht;
R₄ für H oder -Z-R₁₁ steht,
wobei
Z abwesend oder -C(=O)-, -S(=O)- oder -S(=O)₂- sein kann, und
R₁₁ für -C₁₋₆-Alkyl, -C₃₋₆-Cycloalkyl, oder -C₁₋₃-Alkyl-C₃₋₆-Cycloalkyl steht, wobei in der C₃₋₆-Cycloalkylgruppe ein Ringkohlenstoffatom durch ein Sauerstoffatom ersetzt sein kann und -C₁₋₆-Alkyl, -C₃₋₆-Cycloalkyl oder -C₁₋₃-Alkyl-C₃₋₆-Cycloalkyl unsubstituiert, einfach oder mehrfach substituiert sein können, mit Substituenten ausgesucht aus der Gruppe bestehend aus -F, -Cl, -Br, -I, -CN, -OH, -SH, -O-C₁₋₃-Alkyl, und -S-C₁₋₃-Alkyl, wobei -C₁₋₃-Alkyl substituiert sein kann mit einem oder mehreren Substituenten aus der Gruppe umfassend Substituenten -F, -Cl, -Br, -I, -CN, -OH, -SH;
R₅ jeweils unabhängig für -H oder -R₀ steht;
R₆ und R₇ unabhängig voneinander für -H oder R₀ stehen; oder R₆ und R₇ zusammen für -CH₂CH₂OCH₂CH₂-, -CH₂CH₂NR,_{O}CH₂CH₂- oder -(CH₂)₃₋₆- stehen;
R₈ für -H, -R₀ oder -C(=O)R₀ steht;
R₁₀ für -H oder -C₁₋₆-Aliphat steht;
wobei
"Aliphat" jeweils ein verzweigter oder unverzweigter, gesättigter oder ein ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist;
wobei in Bezug auf "Aliphat" und "Cycloaliphat" unter "ein- oder mehrfach substituiert" die ein- oder mehrfache Substitution eines oder mehrerer Wasserstoffatome durch -F, -Cl, -Br, -I, -CN, -NO₂, -CHO, =O -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂ verstanden wird; wobei ggf. vorhandene N-Ringatome jeweils oxidiert sein können (N-Oxid);
in Form eines einzelnen Stereoisomers oder deren Mischung, der freien Verbindungen und/oder ihrer physiologisch verträglichen Salze und/oder Solvate.

2. Verbindung nach Anspruch 1, wobei Y₁', Y₂', Y₃' und Y₄' jeweils für -H stehen.

3. Verbindung nach Anspruch 1 oder 2, wobei
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂;

4. Verbindung nach einem der Ansprüche 1 bis 3, wobei
R₃ für -C₁₋₈-Aliphat, -Aryl, -Heteroaryl, -C₁₋₃-Aliphat-Aryl, -C₁₋₃-Aliphat-Heteroaryl oder -C₁₋₃-Aliphat-C₅₋₆-Cycloaliphat steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂;
und
X₁, X₁', X₂, X₂', X₃, X₃' jeweils unabhängig voneinander für -H, -F, -Cl, -Br, -I, -NO₂, -CF₃, -OR₅, -SR₅, -SO₂R₅, -S(=O)₂OR₅, -CN, -COOR₅, -CONR₅, -NR₆R₇, oder -R₀ stehen; oder einer der Reste X₁ und X₁' für H und der andere für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, - OCH₃, -OC₂H₅ und -N(CH₃)₂; oder X₁ und X₁', oder X₂ und X₂', oder X₃ und X₃' gemeinsam für =O stehen; oder X₁ und X₁' gemeinsam für C₃₋₆-Cycloalkyl stehen, das unsubstituiert oder mit einem oder mehreren unabhängig voneinander ausgesuchten Substituenten aus der Gruppe bestehend aus -F, -Cl, -Br, -I, -OR₅, SR₅, C₁₋₃-Alkyl oder -CN substituiert sein kann.

5. Verbindung nach einem der Ansprüche 1 bis 4, wobei Y₁, Y₁', Y₂, Y₂', Y₃, Y₃', Y₄ und Y₄' jeweils für -H stehen.

6. Verbindung nach einem der Ansprüche 1 bis 5, welche die allgemeine Formel (3.1) 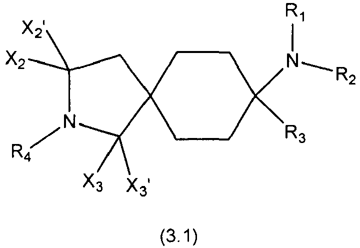 aufweist.

7. Verbindung nach einem der Ansprüche 1 bis 6, wobei
wobei Y₁, Y₁', Y₂, Y₂', Y₃, Y₃', Y₄ und Y₄' jeweils für -H stehen;
X₁, X₁', X₂', X₃ und X₃' für H stehen; oder X₂ und X₂', oder X₃ und X₃' gemeinsam für =O stehen; oder X₁ und X₁' gemeinsam für ein C₃₋₆-Cycloalkyl stehen;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂;
R₁ für -CH₃ steht;
R₂ für -H oder -CH₃ steht; oder
R₁ und R₂ gemeinsam einen Ring bilden und für -(CH₂)₃₋₄- stehen; und
R₃ für -C₁₋₈-Aliphat, -Aryl, -Heteroaryl, -C₁₋₃-Aliphat-Aryl, -C₁₋₃-Aliphat-Heteroaryl oder -C₁₋₃-Aliphat-C₅₋₆-Cycloaliphat steht; wobei diese unsubstituiert oder ein- oder mehrfach substituiert sind mit Substituenten unabhängig voneinander ausgewählt aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂; und
R₄ für H oder -Z-R₁₁ steht,
wobei
-Z- abwesend oder -C(=O)- sein kann, und
R₁₁ für -C₁₋₆-Alkyl, -C₃₋₆-Cycloalkyl, oder -C₁₋₃-Alkyl-C₃₋₆-Cycloalkyl steht, wobei in der C₃₋₆-Cycloalkylgruppe ein Ringkohlenstoffatom durch ein Sauerstoffatom ersetzt sein kann und -C₁₋₆-Alkyl, -C₃₋₆-Cycloalkyl oder -C₁₋₃-Alkyl-C₃₋₆-Cycloalkyl unsubstituiert, einfach oder mehrfach substituiert sein können, mit Substituenten unabhängig voneinander ausgesucht aus der Gruppe bestehend aus -F, -Cl, -Br, -I, -CN, -OH, - SH, -O-C₁₋₃-Alkyl, und -S-C₁₋₃-Alkyl, wobei -C₁₋₃-Alkyl substituiert sein kann mit einem oder mehreren Substituenten aus der Gruppe bestehend aus den Substituenten -F, - Cl, -Br, -I, -CN, -OH und -SH;

8. Verbindung nach einem der Ansprüche 1 bis 7, wobei R₁ und R₂ jeweils für -CH₃ stehen.

9. Verbindung nach einem der Ansprüche 1 bis 8, wobei R₃ ausgesucht ist aus der Gruppe bestehend aus Phenyl, Benzyl, und 2-Thienyl, jeweils unsubstituiert oder ein oder mehrfach substituiert mit Substituenten unabhängig voneinander ausgesucht aus der Gruppe bestehend aus -F, -Cl, -Br, -CN, -CH₃, -C₂H₅, -NH₂, -NO₂, -SH, -CF₃, OH, -OCH₃, -OC₂H₅ und -N(CH₃)₂.

10. Verbindung nach einem der Ansprüche 1 bis 9, wobei R4 ein ausgesucht ist aus der Gruppe bestehend aus H, CH₃, Ethyl, n-Propyl, i-Propyl, n-Butyl, s-Butyl, iso-Butyl, t-Butyl, n-Pentyl, s-Pentyl, iso-Pentyl, 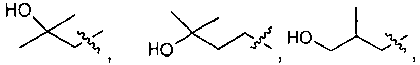 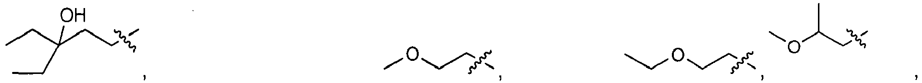 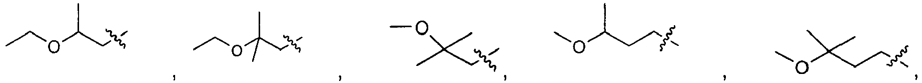 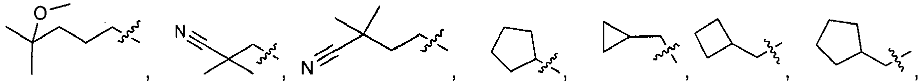 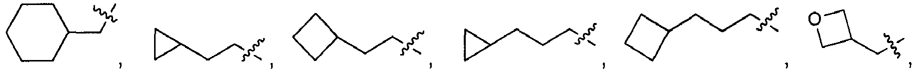 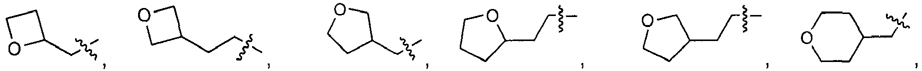 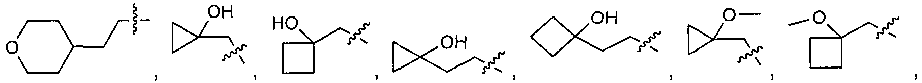 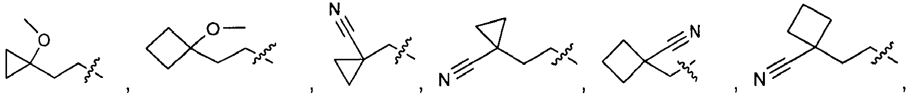 Acetyl,

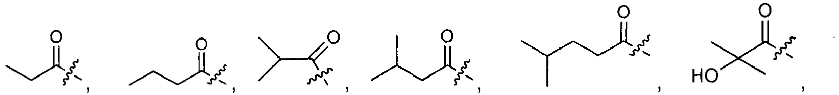

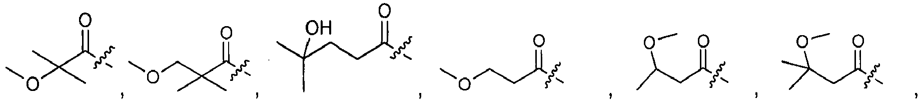

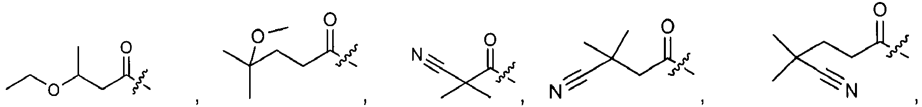

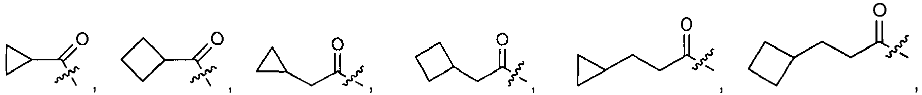

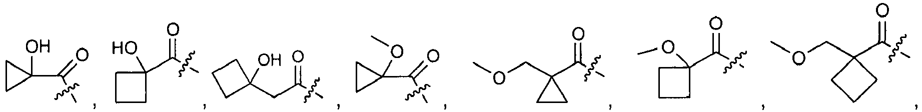

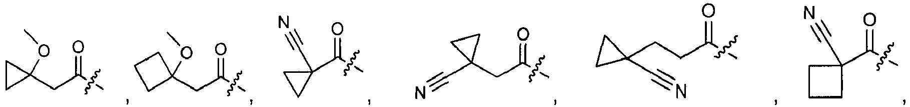

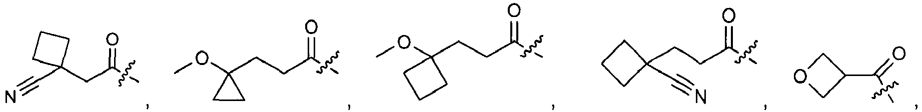

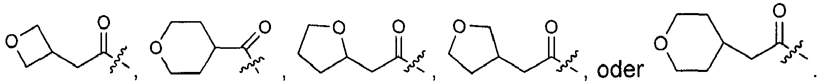

11. Verbindung gemäß einem der Ansprüche 1 bis 9 aus der Gruppe:
| | |
|---|---|
| **1; 2** | 8-Benzyl-8-(dimethyl-amino)-3-azaspiro[4.5]decan-4-on |
| **3; 4** | (8-Benzyl-3-azaspiro[4.5]decan-8-yl)-dimethyl-amin |
| **5; 6** | (8-Benzyl-3-methyl-3-azaspiro[4.5]decan-8-yl)-dimethyl-amin |
| **7; 8** | 1-[8-Benzyl-8-(dimethyl-amino)-3-azaspiro[4.5]decan-3-yl]-ethanon |
| **9** | (8-Benzyl-3-butyl-3-azaspiro[4.5]decan-8-yl)-dimethyl-amin |
| **10; 11** | 1-[8-Benzyl-8-(dimethyl-amino)-3-azaspiro[4.5]decan-3-yl]-butan-1-on |
| **12** | [8-Benzyl-3-(cyclopentyl-methyl)-3-azaspiro[4.5]decan-8-yl]-dimethyl-amin |
| **13** | 8-Dimethylamino-8-phenyl-3-azaspiro[4.5]decan-4-on |
| **14; 15** | 8-(Dimethyl-amino)-8-phenyl-3-azaspiro[4.5]decan-2-on |
| **16** | 8-Butyl-8-dimethylamino-3-azaspiro[4.5]decan-4-on |
| **17** | 8-Dimethylamino-8-thiophen-2-yl-3-azaspiro[4.5]decan-4-on |
| **18; 25** | Dimethyl-(8-thiophen-2-yl-3-azaspiro[4.5]decan-8-yl)-amin |
| **19** | Dimethyl-(3-methyl-8-thiophen-2-yl-3-azaspiro[4.5]decan-8-yl)-amin |
| **20** | 1-(8-Dimethylamino-8-thiophen-2-yl-3-azaspiro[4.5]decan-3-yl)-butan-1-on |
| **21** | (3-Butyl-8-thiophen-2-yl-3-azaspiro[4.5]decan-8-yl)-dimethyl-amin |
| **22** | [3-(Cyclopentyl-methyl)-8-thiophen-2-yl-3-azaspiro[4.5]decan-8-yl]-dimethylamin |
| **23** | 8-(Dimethyl-amino)-8-thiophen-2-yl-2-azaspiro[4.5]decan-3-on |
| **24a/b** | 8-(Dimethyl-amino)-8-thiophen-2-yl-2-azaspiro[4.5]decan-3-on |
| **26** | 1-[8-(Dimethyl-amino)-8-thiophen-2-yl-3-azaspiro[4.5]decan-3-yl]-butan-1-on |
| **27** | (8-Benzyl-3-butyl-3-azaspiro[4.5]decan-8-yl)-dimethyl-amin |
| **28; 29** | 1-[8-(Dimethyl-amino)-8-phenyl-3-azaspiro[4.5]decan-3-yl]-butan-1-on |
| **30** | 8-(Dimethyl-amino)-8-(5-methyl-thiophen-2-yl)-3-azaspiro[4.5]decan-4-on |
| **31** | Dimethyl-[8-(5-methyl-thiophen-2-yl)-3-azaspiro[4.5]decan-8-yl]-amin |
| **32** | 1-[8-(Dimethyl-amino)-8-(5-methyl-thiophen-2-yl)-3-azaspiro[4.5]decan-3-yl]-butan-1-on |
| **33** | 3-Butyl-8-(dimethyl-amino)-8-thiophen-2-yl-3-azaspiro[4.5]decan-2-on |
| **34** | 8-(Dimethyl-amino)-3-methyl-8-thiophen-2-yl-3-azaspiro[4.5]decan-2-on |
| **35** | [3-Butyl-8-(5-methyl-thiophen-2-yl)-3-azaspiro[4.5]decan-8-yl]-dimethyl-amin |
| **36** | Cyclobutyl-[8-dimethylamino-8-(5-methyl-thiophen-2-yl)-3-azaspiro[4.5]decan-3-yl]-methanon |
| **37** | Cyclopropyl-[8-dimethylamino-8-(5-methyl-thiophen-2-yl)-3-azaspiro[4.5]decan-3-yl]-methanon |
| **38** | 1-[8-Dimethylamino-8-(5-methyl-thiophen-2-yl)-3-azaspiro[4.5]decan-3-yl)-2-methyl-propan-1-on |
| **39** | 1-[8-Dimethylamino-8-(5-methyl-thiophen-2-yl)-3-azaspiro[4.5]decan-3-yl]-3-methyl-butan-1-on |
| **40** | 1-[8-Dimethylamino-8-(5-methyl-thiophen-2-yl)-3-azaspiro[4.5]decan-3-yl]-propan-1-on |
| **41** | [3-(2-Methoxy-ethyl)-8-(5-methyl-thiophen-2-yl)-3-azaspiro[4.5]decan-8-yl]-dimethyl-amin |
| **42** | 1-[8-(Dimethyl-amino)-8-thiophen-2-yl-3-azaspiro[4.5]decan-3-yl]-2-methoxy-ethanon |
| **43** | Cyclobutyl-[8-(dimethyl-amino)-8-thiophen-2-yl-3-azaspiro[4.5]decan-3-yl]-methanon |
| **44** | Cyclopropyl-[8-(dimethyl-amino)-8-thiophen-2-yl-3-azaspiro[4.5]decan-3-yl]-methanon |
| **45** | 1-[8-(Dimethyl-amino)-8-thiophen-2-yl-3-azaspiro[4.5]decan-3-yl]-2-methyl-propan-1-on |
| **46** | 1-[8-(Dimethyl-amino)-8-thiophen-2-yl-3-azaspiro[4.5]decan-3-yl]-3-methyl-butan-1-on |
| **47** | [3-(2-Methoxy-ethyl)-8-thiophen-2-yl-3-azaspiro[4.5]decan-8-yl]-dimethyl-amin |
| **48** | 2-Cyclopropyl-1-[8-(dimethyl-amino)-8-thiophen-2-yl-3-azaspiro[4.5]decan-3-yl]-ethanon |
| **49** | [3-(Cyclopropyl-methyl)-8-thiophen-2-yl-3-azaspiro[4.5]decan-8-yl]-dimethylamin |
| **50** | 2-Cyclobutyl-1-[8-(dimethyl-amino)-8-thiophen-2-yl-3-azaspiro[4.5]decan-3-yl]-ethanon |
| **51** | [3-(2-Cyclopropyl-ethyl)-8-thiophen-2-yl-3-azaspiro[4.5]decan-8-yl]-dimethylamin |
| **52** | [3-(2-Cyclobutyl-ethyl)-8-thiophen-2-yl-3-azaspiro[4.5]decan-8-yl]-dimethyl-amin |
| **53** | [3-(Cyclobutyl-methyl)-8-thiophen-2-yl-3-azaspiro[4.5]decan-8-yl]-dimethyl-amin |
| **54** | 1-[8-(Dimethyl-amino)-8-thiophen-2-yl-3-azaspiro[4.5]decan-3-yl]-4-methyl-pentan-1-on |
| **55** | 3-[8-(Dimethyl-amino)-8-thiophen-2-yl-3-azaspiro[4.5]decan-3-yl]-2,2-dimethyl-3-oxo-propionitril |
| **56** | 1-[8-(Dimethyl-amino)-8-thiophen-2-yl-3-azaspiro[4.5]decan-3-yl]-2-tetrahydrofuran-3-yl-ethanon |
| **57** | (8-Dimethylamino-8-thiophen-2-yl-3-azaspiro[4.5]decan-3-yl)-(oxetan-3-yl)-methanon |
| **58** | 1-(8-Dimethylamino-8-thiophen-2-yl-3-azaspiro[4.5]decan-3-carbonyl)-cyclopropan-1-carbonitril |
| **59** | 1-(8-Dimethylamino-8-thiophen-2-yl-3-azaspiro[4.5]decan-3-yl)-4-methoxy-4-methyl-pentan-1-on |
| **60** | [8-(Dimethyl-amino)-8-thiophen-2-yl-3-azaspiro[4.5]decan-3-yl]-tetrahydropyran-4-yl-methanon |
| **61** | 1-[8-(Dimethyl-amino)-8-thiophen-2-yl-3-azaspiro[4.5]decan-3-carbonyl]-cyclobutan-1-carbonitril |
| **62** | 3-Cyclopropyl-1-[8-(dimethyl-amino)-8-thiophen-2-yl-3-azaspiro[4.5]decan-3-yl]-propan-1-on |
| **63** | 1-[8-(Dimethyl-amino)-8-thiophen-2-yl-3-azaspiro[4.5]decan-3-yl]-2-methoxy-2-methyl-propan-1-on |
| **64** | 1-[8-(Dimethyl-amino)-8-thiophen-2-yl-3-azaspiro[4.5]decan-3-yl]-2-tetrahydrofuran-2-yl-ethanon |
| **65** | 1-[8-(Dimethyl-amino)-8-thiophen-2-yl-3-azaspiro[4.5]decan-3-yl]-4-hydroxy-4-methyl-pentan-1-on |
| **66** | [8-(Dimethyl-amino)-8-thiophen-2-yl-3-azaspiro[4.5]decan-3-yl)-(1-hydroxy-cyclobutyl)-methanon |
| **67** | 1-[2-[8-(Dimethyl-amino)-8-thiophen-2-yl-3-azaspiro[4.5]decan-3-yl]-2-oxo-ethyl]-cyclopropan-1-carbonitril |
| **68** | 1-[2-[8-(Dimethyl-amino)-8-thiophen-2-yl-3-azaspiro[4.5]decan-3-yl]-2-oxo-ethyl]-cyclobutan-1-carbonitril |
| **69** | 3-Cyclobutyl-1-[8-(dimethyl-amino)-8-thiophen-2-yl-3-azaspiro[4.5]decan-3-yl]-propan-1-on |
| **70** | Dimethyl-[3-(oxetan-3-yl-methyl)-8-thiophen-2-yl-3-azaspiro[4.5]decan-8-yl]-amin |
| **71** | Dimethyl-(8-phenyl-3-azaspiro[4.5]decan-8-yl)-amin |
| **72** | 1-[8-(Dimethyl-amino)-8-phenyl-3-azaspiro[4.5]decan-3-yl]-butan-1-on |
| **73** | 1-(8-Dimethylamino-8-phenyl-3-azaspiro[4.5]decan-3-yl)-ethanon |
| **74** | (3-Butyl-8-phenyl-3-azaspiro[4.5]decan-8-yl)-dimethyl-amin |
| **75** | 1-[8-(Dimethyl-amino)-8-phenyl-3-azaspiro[4.5]decan-3-yl]-2-tetrahydro-furan-2-yl-ethanon |
| **76** | 1-[2-[8-(Dimethyl-amino)-8-phenyl-3-azaspiro[4.5]decan-3-yl]-2-oxo-ethyl]-cyclobutan-1-carbonitril |
| **77** | 1-[2-[8-(Dimethyl-amino)-8-phenyl-3-azaspiro[4.5]decan-3-yl]-2-oxo-ethyl]-cyclopropan-1-carbonitril |
| **78** | 1-[8-(Dimethyl-amino)-8-phenyl-3-azaspiro[4.5]decan-3-yl]-2-(oxetan-3-yl)-ethanon |
| **79** | 1-[8-(Dimethyl-amino)-8-phenyl-3-azaspiro[4.5]decan-3-yl]-4-methyl-pentan-1-on |
| **80** | 3-[8-(Dimethyl-amino)-8-phenyl-3-azaspiro[4.5]decan-3-yl]-2,2-dimethyl-3-oxo-propionitril |
| **81** | [8-(Dimethyl-amino)-8-phenyl-3-azaspiro[4.5]decan-3-yl]-tetrahydro-pyran-4-yl-methanon |
| **82** | 1-[8-(Dimethyl-amino)-8-phenyl-3-azaspiro[4.5]decan-3-yl]-4-hydroxy-4-methyl-pentan-1-on |
| **83** | 1-[8-(Dimethyl-amino)-8-phenyl-3-azaspiro[4.5]decan-3-yl]-4-methoxy-4-methyl-pentan-1-on |
| **84** | [8-(Dimethyl-amino)-8-phenyl-3-azaspiro[4.5]decan-3-yl]-(1-hydroxy-cyclobutyl)-methanon |
| **85** | 2-Cyclopropyl-1-[8-(dimethyl-amino)-8-phenyl-3-azaspiro[4.5]decan-3-yl]-ethanon |
| **86** | 2-Cyclobutyl-1-[8-(dimethyl-amino)-8-phenyl-3-azaspiro[4.5]decan-3-yl]-ethanon |
| **87** | [3-(4-Methoxy-4-methyl-pentyl)-8-phenyl-3-azaspiro[4.5]decan-8-yl]-dimethylamin |
| **88** | 1-[8-(5-Chlor-thiophen-2-yl)-8-dimethylamino-3-azaspiro[4.5]decan-3-yl]-butan-1-on |
| **89** | [3-Butyl-8-(5-chlor-thiophen-2-yl)-3-azaspiro[4.5]decan-8-yl]-dimethyl-amin |
| **90** | 1-[8-Dimethylamino-8-(5-fluor-thiophen-2-yl)-3-azaspiro[4.5]decan-3-yl]-butan-1-on |
| **91** | [3-Butyl-8-(5-fluor-thiophen-2-yl)-3-azaspiro[4.5]decan-8-yl]-dimethyl-amin |
| **92** | 8-(Cyclohexyl-methyl)-8-dimethylamino-3-azaspiro[4.5]decan-4-on |
| **93** | 1-[8-(Cyclohexyl-methyl)-8-dimethylamino-3-azaspiro[4.5]decan-3-yl]-butan-1-on |
| **94** | [3-Butyl-8-(cyclohexyl-methyl)-3-azaspiro[4.5]decan-8-yl]-dimethyl-amin |
| **95** | 1-[8-(Cyclopentyl-methyl)-8-dimethylamino-3-azaspiro[4.5]decan-3-yl]-butan-1-on |
| **96** | [3-Butyl-8-(cyclopentyl-methyl)-3-azaspiro[4.5]decan-8-yl]-dimethyl-amin |
| **97** | 1-(8-Cyclopentyl-8-dimethylamino-3-azaspiro[4.5]decan-3-yl)-butan-1-on |
| **98** | (3-Butyl-8-cyclopentyl-3-azaspiro[4.5]decan-8-yl)-dimethyl-amin |
| **99** | Cyclobutyl-(8-cyclopentyl-8-dimethylamino-3-azaspiro[4.5]decan-3-yl)-methanon |
| **100** | (8-Cyclopentyl-8-dimethylamino-3-azaspiro[4.5]decan-3-yl)-cyclopropyl-methanon |
| **101** | [8-Cyclopentyl-3-(cyclopropyl-methyl)-3-azaspiro[4.5]decan-8-yl]-dimethyl-amin |
| **102** | [3-(Cyclobutyl-methyl)-8-cyclopentyl-3-azaspiro[4.5]decan-8-yl]-dimethyl-amin |
| **103** | 1-(8-Cyclopentyl-8-dimethylamino-3-azaspiro[4.5]decan-3-yl)-2-cyclopropyl-ethanon |
| **104** | [8-Cyclopentyl-3-(2-cyclopropyl-ethyl)-3-azaspiro[4.5]decan-8-yl]-dimethyl-amin |
| **105** | 2-Cyclobutyl-1-(8-cyclopentyl-8-dimethylamino-3-azaspiro[4.5]decan-3-yl)-ethanon |
| **106; 107** | [3-Butyl-8-(dimethyl-amino)-3-azaspiro[4.5]decan-8-yl]-phenyl-methanon |
| **108; 109** | 1-[8-(Azetidin-1-yl)-8-thiophen-2-yl-3-azaspiro[4.5]decan-3-yl]-butan-1-on |
| **110; 111** | 1-[8-(Azetidin-1-yl)-8-phenyl-3-azaspiro[4.5]decan-3-yl]-butan-1-on |
| **112; 113** | 8-(Azetidin-1-yl)-3-butyl-8-phenyl-3-azaspiro[4.5]decan |
| **114** | 8-Dimethylamino-3-methyl-8-thiophen-2-yl-3-azaspiro[4.5]decan-4-on |
| **115** | 3-Butyl-8-dimethylamino-8-thiophen-2-yl-3-azaspiro[4.5]decan-4-on |
| **116** | 3-(Cyclopentyl-methyl)-8-dimethylamino-8-thiophen-2-yl-3-azaspiro[4.5]decan-4-on |
| **117** | 8-(5-Chlor-thiophen-2-yl)-8-dimethylamino-3-azaspiro[4.5]decan-4-on |
| **118; 119** | 8-Benzyl-8-(dimethyl-amino)-3-methyl-3-azaspiro[4.5]decan-4-on |
| **120; 121** | 8-Benzyl-3-butyl-8-(dimethyl-amino)-3-azaspiro[4.5]decan-4-on |
| **122; 123** | 8-Benzyl-3-(cyclopentyl-methyl)-8-(dimethyl-amino)-3-azaspiro[4.5]decan-4-on |
| **124** | 8-(Dimethyl-amino)-3-methyl-8-thiophen-2-yl-3-azaspiro[4.5]decan-2-on |
| **125** | 3-Butyl-8-(dimethyl-amino)-8-thiophen-2-yl-3-azaspiro[4.5]decan-2-on |
| **126; 127** | 3-(Cyclopentyl-methyl)-8-(dimethyl-amino)-8-thiophen-2-yl-3-azaspiro[4.5]decan-2-on |
| **128; 129** | 3-(2-Cyclobutyl-ethyl)-8-(dimethyl-amino)-8-thiophen-2-yl-3-azaspiro[4.5]decan-2-on |
| **130; 131** | 3-(2-Cyclopropyl-ethyl)-8-(dimethyl-amino)-8-thiophen-2-yl-3-azaspiro[4.5]decan-2-on |
| **132; 133** | 3-(Cyclobutyl-methyl)-8-(dimethyl-amino)-8-thiophen-2-yl-3-azaspiro[4.5]decan-2-on |
| **134; 135** | 3-(Cyclopropyl-methyl)-8-(dimethyl-amino)-8-thiophen-2-yl-3-azaspiro[4.5]decan-2-on |
| **136; 137** | 8-(Dimethyl-amino)-3-(2-tetrahydro-furan-2-yl-ethyl)-8-thiophen-2-yl-3-azaspiro[4.5]decan-2-on |
| **138; 139** | 8-(Dimethyl-amino)-3-(tetrahydro-pyran-4-yl-methyl)-8-thiophen-2-yl-3-azaspiro[4.5]decan-2-on |
| **140; 141** | 8-(Dimethyl-amino)-3-(2-tetrahydro-furan-3-yl-ethyl)-8-thiophen-2-yl-3-azaspiro[4.5]decan-2-on |
| **142; 143** | 8-(Dimethyl-amino)-3-[2-(oxetan-3-yl)-ethyl]-8-thiophen-2-yl-3-azaspiro[4.5]decan-2-on |
| **144; 145** | 1-[2-[8-(Dimethyl-amino)-2-oxo-8-thiophen-2-yl-3-azaspiro[4.5]decan-3-yl]-ethyl]-cyclopropan-1-carbonitril |
| **146; 147** | 8-(Dimethyl-amino)-3-(oxetan-2-yl-methyl)-8-thiophen-2-yl-3-azaspiro[4.5]decan-2-on |
| **148; 149** | 8-(Dimethyl-amino)-3-(oxetan-3-yl-methyl)-8-thiophen-2-yl-3-azaspiro[4.5]decan-2-on |
| **150; 151** | 1-[2-[8-(Dimethyl-amino)-2-oxo-8-thiophen-2-yl-3-azaspiro[4.5]decan-3-yl]-ethyl]-cyclobutan-1-carbonitril |
| **152; 153** | 8-(Dimethyl-amino)-8-(5-methyl-thiophen-2-yl)-3-azaspiro[4.5]decan-2-on |
| **154; 155** | 8-(Dimethyl-amino)-3-methyl-8-(5-methyl-thiophen-2-yl)-3-azaspiro[4.5]decan-2-on |
| **156; 157** | 3-Butyl-8-(dimethyl-amino)-8-(5-methyl-thiophen-2-yl)-3-azaspiro[4.5]decan-2-on |
| **158; 159** | 3-(Cyclopentyl-methyl)-8-(dimethyl-amino)-8-(5-methyl-thiophen-2-yl)-3-azaspiro[4.5]decan-2-on |
| **160** | 3-(Cyclopropyl-methyl)-8-(dimethyl-amino)-8-(5-methyl-thiophen-2-yl)-3-azaspiro[4.5]decan-2-on |
| **161** | 3-(Cyclobutyl-methyl)-8-(dimethyl-amino)-8-(5-methyl-thiophen-2-yl)-3-azaspiro[4.5]decan-2-on |
| **162** | 8-(Dimethyl-amino)-3-methyl-8-phenyl-3-azaspiro[4.5]decan-2-on |
| **163; 164** | 3-Butyl-8-(dimethyl-amino)-8-phenyl-3-azaspiro[4.5]decan-2-on |
| **165; 166** | 3-(Cyclopentyl-methyl)-8-(dimethyl-amino)-8-phenyl-3-azaspiro[4.5]decan-2-on |
| **167; 168** | 3-(2-Cyclopropyl-ethyl)-8-(dimethyl-amino)-8-phenyl-3-azaspiro[4.5]decan-2-on |
| **169; 170** | 3-(Cyclobutyl-methyl)-8-(dimethyl-amino)-8-phenyl-3-azaspiro[4.5]decan-2-on |
| **171; 172** | 3-(Cyclopropyl-methyl)-8-(dimethyl-amino)-8-phenyl-3-azaspiro[4.5]decan-2-on |
| **173; 174** | 3-(2-Cyclobutyl-ethyl)-8-(dimethyl-amino)-8-phenyl-3-azaspiro[4.5]decan-2-on |
| **175; 176** | 1-[2-[8-(Dimethyl-amino)-2-oxo-8-phenyl-3-azaspiro[4.5]decan-3-yl]-ethyl]-cyclopropan-1-carbonitril |
| **177; 178** | 1-[2-[8-(Dimethyl-amino)-2-oxo-8-phenyl-3-azaspiro[4.5]decan-3-yl]-ethyl]-cyclobutan-1-carbonitril |
| **179; 180** | 8-(Dimethyl-amino)-3-[(1-hydroxy-cyclobutyl)-methyl]-8-phenyl-3-azaspiro[4.5]decan-2-on |
| **181** | 8-(Dimethyl-amino)-3-(2-hydroxy-2-methyl-propyl)-8-phenyl-3-azaspiro[4.5]decan-2-on |
| **182; 183** | 8-(Dimethyl-amino)-3-(3-methoxy-3-methyl-butyl)-8-phenyl-3-azaspiro[4.5]decan-2-on |
| **184; 185** | 8-(Dimethyl-amino)-3-[2-(1-methoxy-cyclobutyl)-ethyl]-8-phenyl-3-azaspiro[4.5]decan-2-on |
| **186; 187** | 8-(Dimethyl-amino)-3-(3-hydroxy-3-methyl-butyl)-8-phenyl-3-azaspiro[4.5]decan-2-on |
| **188** | 1-[8-(Dimethyl-amino)-8-phenyl-3-azaspiro[4.5]decan-3-yl]-2-tetrahydro-furan-3-yl-ethanon |
| **189** | [8-(Dimethyl-amino)-8-phenyl-3-azaspiro[4.5]decan-3-yl]-(oxetan-3-yl)-methanon |
| **190** | 1-[8-(Dimethyl-amino)-8-phenyl-3-azaspiro[4.5]decan-3-yl]-2-methoxy-2-methyl-propan-1-on |
| **191** | [3-(2-Cyclopropyl-ethyl)-8-phenyl-3-azaspiro[4.5]decan-8-yl]-dimethyl-amin |
| **192** | [3-(2-Cyclobutyl-ethyl)-8-phenyl-3-azaspiro[4.5]decan-8-yl]-dimethyl-amin |
| **193** | 1-[[8-(Dimethyl-amino)-8-phenyl-3-azaspiro[4.5]decan-3-yl]-methyl]-cyclobutan-1-ol |
| **194** | 1-[2-[8-(Dimethyl-amino)-8-phenyl-3-azaspiro[4.5]decan-3-yl]-ethyl]-cyclobutan-1-carbonitril |
| **195** | 3-Cyclobutyl-1-[8-(dimethyl-amino)-8-phenyl-3-azaspiro[4.5]decan-3-yl]-propan-1-on |
| **196** | Dimethyl-[3-(oxetan-3-yl-methyl)-8-phenyl-3-azaspiro[4.5]decan-8-yl]-amin |
| **197** | 3-Cyclopropyl-1-[8-(dimethyl-amino)-8-phenyl-3-azaspiro[4.5]decan-3-yl]-propan-1-on |
| **198** | 1-[8-(Dimethyl-amino)-8-phenyl-3-azaspiro[4.5]decan-3-carbonyl]-cyclopropan-1-carbonitril |
| **199** | 1-[8-(Dimethyl-amino)-8-phenyl-3-azaspiro[4.5]decan-3-carbonyl]-cyclobutan-1-carbonitril |
| **200** | 1-[2-[8-(Dimethyl-amino)-8-phenyl-3-azaspiro[4.5]decan-3-yl]-ethyl]-cyclopropan-1-carbonitril |
| **201** | Dimethyl-[8-phenyl-3-(2-tetrahydro-furan-3-yl-ethyl)-3-azaspiro[4.5]decan-8-yl]-amin |
| **202** | [3-(2-Methoxy-2-methyl-propyl)-8-phenyl-3-azaspiro[4.5]decan-8-yl]-dimethylamin |
| **203** | [3-(3-Cyclobutyl-propyl)-8-phenyl-3-azaspiro[4.5]decan-8-yl]-dimethyl-amin |
| **204** | [3-(3-Cyclopropyl-propyl)-8-phenyl-3-azaspiro[4.5]decan-8-yl)-dimethyl-amin |
| **205** | [8-(Dimethyl-amino)-8-phenyl-3-azaspiro[4.5]decan-3-yl]-(1-hydroxy-cyclopropyl)-methanon |
| **206** | 1-[[8-(Dimethyl-amino)-8-phenyl-3-azaspiro[4.5]decan-3-yl]-methyl]-cyclopropan-1-ol |
| **207** | 1-[[8-(Dimethyl-amino)-8-thiophen-2-yl-3-azaspiro[4.5]decan-3-yl]-methyl]-cyclobutan-1-ol |
| **208** | 1-[2-[8-(Dimethyl-amino)-8-thiophen-2-yl-3-azaspiro[4.5]decan-3-yl]-ethyl]-cyclobutan-1-carbonitril |
| **209** | [8-(Dimethyl-amino)-8-thiophen-2-yl-3-azaspiro[4.5]decan-3-yl]-(1-hydroxy-cyclopropyl)-methanon |
| **210** | 1-[[8-(Dimethyl-amino)-8-thiophen-2-yl-3-azaspiro[4.5]decan-3-yl]-methyl]-cyclopropan-1-ol |
| **211** | 1-[2-[8-(Dimethyl-amino)-8-thiophen-2-yl-3-azaspiro[4.5]decan-3-yl]-ethyl]-cyclopropan-1-carbonitril |
| **212** | 1-(8-Dimethylamino-8-thiophen-2-yl-3-azaspiro[4.5]decan-3-yl)-2-(oxetan-3-yl)-ethanon |
| **213** | 1-[8-(Dimethyl-amino)-8-thiophen-2-yl-3-azaspiro[4.5]decan-3-yl]-3-ethyl-pentan-3-ol |
| **214** | 1-[8-(Dimethyl-amino)-8-(thiophen-2-yl-methyl)-3-azaspiro[4.5]decan-3-yl]-butan-1-on |
| **215; 216** | 8-(Dimethyl-amino)-3-[(1-hydroxy-cyclobutyl)-methyl]-8-thiophen-2-yl-3-azaspiro[4.5]decan-2-on |
| **217; 218** | 8-(Dimethyl-amino)-3-[2-(1-methoxy-cyclobutyl)-ethyl]-8-thiophen-2-yl-3-azaspiro[4.5]decan-2-on |
| **219; 220** | 8-(Dimethyl-amino)-3-(3-methoxy-3-methyl-butyl)-8-thiophen-2-yl-3-azaspiro[4.5]decan-2-on |
| **221; 222** | 8-(Dimethyl-amino)-3-(3-hydroxy-3-methyl-butyl)-8-thiophen-2-yl-3-azaspiro[4.5]decan-2-on |
| **223** | 1-[8-(Dimethyl-amino)-8-thiophen-2-yl-3-azaspiro[4.5]decan-3-yl]-2-hydroxy-2-methyl-propan-1-on |
| **224; 225** | 8-(Dimethyl-amino)-3-(2-hydroxy-2-methyl-propyl)-8-thiophen-2-yl-3-azaspiro[4.5]decan-2-on |
| **226** | 4-[8-(Dimethyl-amino)-8-thiophen-2-yl-3-azaspiro[4.5]decan-3-yl]-2-methylbutan-2-ol |
| **227** | 1-[8-(Dimethyl-amino)-8-thiophen-2-yl-3-azaspiro[4.5]decan-3-yl]-2-methyl-propan-2-ol |
| **228** | 1-[8-(Dimethyl-amino)-8-phenyl-3-azaspiro[4.5]decan-3-yl]-2-hydroxy-2-methyl-propan-1-on |
| **229** | 1-[8-(Dimethyl-amino)-8-phenyl-3-azaspiro[4.5]decan-3-yl]-2-methyl-propan-2-ol |
| **230; 231** | 4-[8-(Dimethyl-amino)-2-oxo-8-phenyl-3-azaspiro[4.5]decan-3-yl]-2,2-dimethyl-butyronitril |
| **232** | 4-[8-(Dimethyl-amino)-8-phenyl-3-azaspiro[4.5]decan-3-yl]-2,2-dimethyl-4-oxo-butyronitril |
| **233** | 4-[8-(Dimethyl-amino)-8-thiophen-2-yl-3-azaspiro[4.5]decan-3-yl]-2,2-dimethyl-4-oxo-butyronitril |
| **234** | 8-Cyclopentyl-3-(2-cyclopropyl-ethyl)-8-(dimethyl-amino)-3-azaspiro[4.5]decan-2-on |
| **235** | 1-[8-(Dimethyl-amino)-8-thiophen-2-yl-3-azaspiro[4.5]decan-3-yl]-2-(1-methoxy-cyclobutyl)-ethanon |
| **236** | 3-(2-Cyclobutyl-ethyl)-8-cyclopentyl-8-(dimethyl-amino)-3-azaspiro[4.5]decan-2-on |
| **237** | 1-[8-(Dimethyl-amino)-8-phenyl-3-azaspiro[4.5]decan-3-yl]-2-(1-methoxy-cyclobutyl)-ethanon |
| **238** | [3-[2-(1-Methoxy-cyclobutyl)-ethyl]-8-thiophen-2-yl-3-azaspiro[4.5]decan-8-yl]-dimethyl-amin |
| **239** | [3-[2-(1-Methoxy-cyclobutyl)-ethyl]-8-phenyl-3-azaspiro[4.5]decan-8-yl]-dimethyl-amin |
| **240** | 4-[8-(Dimethyl-amino)-2-oxo-8-thiophen-2-yl-3-azaspiro[4.5]decan-3-yl]-2,2-dimethyl-butyronitril |
| **241; 242** | 8-(Dimethyl-amino)-3-[2-(oxetan-3-yl)-ethyl]-8-thiophen-2-yl-3-azaspiro[4.5]decan-2-on |
| **243** | 8-Cyclopentyl-3-(2-cyclopropyl-ethyl)-8-(dimethyl-amino)-3-azaspiro[4.5]decan-2-on |
| **244** | 8-Cyclopentyl-8-(dimethyl-amino)-3-(oxetan-3-yl-methyl)-3-azaspiro[4.5]decan-2-on |
| **245** | 1-[2-[8-Cyclopentyl-8-(dimethyl-amino)-2-oxo-3-azaspiro[4.5]decan-3-yl]-ethyl]-cyclopropan-1-carbonitril |
| **246** | 8-Cyclopentyl-8-(dimethyl-amino)-3-[2-(oxetan-3-yl)-ethyl]-3-azaspiro[4.5]decan-2-on |
| **247** | 1-(8-Cyclopentyl-8-dimethylamino-3-azaspiro[4.5]decan-3-yl)-2-(1-methoxy-cyclobutyl)-ethanon |
| **248** | 4-[8-(Dimethyl-amino)-8-thiophen-2-yl-3-azaspiro[4.5]decan-3-yl]-2,2-dimethyl-butyronitril |
| **249** | 4-[8-(Dimethyl-amino)-8-phenyl-3-azaspiro[4.5]decan-3-yl]-2,2-dimethyl-butyronitril |
| **250** | 4-[8-(Dimethyl-amino)-2-oxo-8-thiophen-2-yl-3-azaspiro[4.5]decan-3-yl]-2,2-dimethyl-butyronitril |
| **251; 252** | 8-(Dimethyl-amino)-3-(oxetan-3-yl-methyl)-8-phenyl-3-azaspiro[4.5]decan-2-on |
| **253** | 1-[2-[8-(Dimethyl-amino)-8-thiophen-2-yl-3-azaspiro[4.5]decan-3-yl]-ethyl]-cyclopropan-1-ol |
| **254; 255** | 8-(Dimethyl-amino)-3-[2-(oxetan-3-yl)-ethyl]-8-phenyl-3-azaspiro[4.5]decan-2-on |
| **256** | 1-[2-[8-Cyclopentyl-8-(dimethyl-amino)-2-oxo-3-azaspiro[4.5]decan-3-yl]-ethyl]-cyclobutan-1-carbonitril |
| **257** | [8-Cyclopentyl-3-[2-(1-methoxy-cyclobutyl)-ethyl]-3-azaspiro[4.5]decan-8-yl]-dimethyl-amin |
| **258** | 4-(8-Cyclopentyl-8-dimethylamino-3-azaspiro[4.5]decan-3-yl)-2,2-dimethyl-butyronitril |
| **259** | 4-(8-Cyclopentyl-8-dimethylamino-3-azaspiro[4.5]decan-3-yl)-2,2-dimethyl-4-oxo-butyronitril |
| **260; 261** | 8-(Dimethyl-amino)-8-phenyl-3-(tetrahydro-furan-3-yl-methyl)-3-azaspiro[4.5]decan-2-on |
| **262; 263** | 8-(Dimethyl-amino)-8-phenyl-3-(2-tetrahydro-furan-2-yl-ethyl)-3-azaspiro[4.5]decan-2-on |
| **264** | 8-(Dimethyl-amino)-8-phenyl-3-(2-tetrahydro-furan-3-yl-ethyl)-3-azaspiro[4.5]decan-2-on |
| **265** | [8-Cyclopentyl-3-(tetrahydro-pyran-4-yl-methyl)-3-azaspiro[4.5]decan-8-yl]-dimethyl-amin |
| **266** | [8-Cyclopentyl-3-(3-methyl-butyl)-3-azaspiro[4.5]decan-8-yl]-dimethyl-amin |
| **267** | 1-[2-(8-Cyclopentyl-8-dimethylamino-3-azaspiro[4.5]decan-3-yl)-ethyl]-cyclopropan-1-carbonitril |
| **268** | 1-[2-(8-Cyclopentyl-8-dimethylamino-3-azaspiro[4.5]decan-3-yl)-ethyl]-cyclobutan-1-carbonitril |
| **269; 270** | 8-(Dimethyl-amino)-8-phenyl-3-(tetrahydro-pyran-4-yl-methyl)-3-azaspiro[4.5]decan-2-on |
| **271** | 8-(Dimethyl-amino)-8-phenyl-3-(2-tetrahydro-furan-3-yl-ethyl)-3-azaspiro[4.5]decan-2-on |
| **272** | 4-[8-(Dimethyl-amino)-8-phenyl-3-azaspiro[4.5]decan-3-yl]-2-methyl-butan-2-ol |
| **273** | [8-Cyclopentyl-3-(2-methyl-propyl)-3-azaspiro[4.5]decan-8-yl]-dimethyl-amin |
| **274** | 3-(Cyclobutyl-methyl)-8-cyclopentyl-8-(dimethyl-amino)-3-azaspiro[4.5]decan-2-on |
| **275** | 3-(2-Cyclopropyl-ethyl)-8-methylamino-8-phenyl-3-azaspiro[4.5]decan-2-on |
| **276** | 8-Cyclopentyl-3-(cyclopropyl-methyl)-8-(dimethyl-amino)-3-azaspiro[4.5]decan-2-on |
| **277** | 3-(Cyclohexyl-methyl)-8-cyclopentyl-8-(dimethyl-amino)-3-azaspiro[4.5]decan-2-on |
| **278** | 8-Cyclopentyl-3-(cyclopentyl-methyl)-8-(dimethyl-amino)-3-azaspiro[4.5]decan-2-on |
| **279; 280** | 8-(Dimethyl-amino)-3-[[(3S)-tetrahydro-furan-3-yl]-methyl]-8-thiophen-2-yl-3-azaspiro[4.5]decan-2-on |
| **281** | 3-Butyl-8-cyclopentyl-8-(dimethyl-amino)-3-azaspiro[4.5]decan-2-on |
| **282** | 8-Cyclopentyl-8-(dimethyl-amino)-3-methyl-3-azaspiro[4.5]decan-2-on |
| **283** | Dimethyl-[3-[2-(oxetan-3-yl)-ethyl]-8-phenyl-3-azaspiro[4.5]decan-8-yl]-amin |
| **284** | Dimethyl-[8-phenyl-3-(tetrahydro-furan-3-yl-methyl)-3-azaspiro[4.5]decan-8-yl]-amin |
| **285** | 1-[2-[8-(Dimethyl-amino)-8-phenyl-3-azaspiro[4.5]decan-3-yl]-ethyl]-cyclopropan-1-ol |
| **286** | 3,8-Dicyclopentyl-8-(dimethyl-amino)-3-azaspiro[4.5]decan-2-on |
| **287** | 8-Cyclopentyl-8-(dimethyl-amino)-3-[2-(1-methoxy-cyclobutyl)-ethyl]-3-azaspiro[4.5]decan-2-on |
| **288; 289** | 3-[8-(Dimethyl-amino)-2-oxo-8-thiophen-2-yl-3-azaspiro[4.5]decan-3-yl]-2,2-dimethyl-propionitril |
| **290** | 3-(2-Cyclopropyl-ethyl)-8-methylamino-8-thiophen-2-yl-3-azaspiro[4.5]decan-2-on |
| **291** | (8-Cyclopentyl-8-dimethylamino-3-azaspiro[4.5]decan-3-yl)-(oxetan-3-yl)-methanon |
| **292** | [8-Cyclopentyl-3-(3-methoxy-3-methyl-butyl)-3-azaspiro[4.5]decan-8-yl]-dimethyl-amin |
| **293; 294** | 8-Cyclopentyl-8-(dimethyl-amino)-3-[[(3S)-tetrahydro-furan-3-yl]-methyl]-3-azaspiro[4.5]decan-2-on |
| **295** | Dimethyl-[3-[2-(oxetan-3-yl)-ethyl]-8-thiophen-2-yl-3-azaspiro[4.5]decan-8-yl]-amin |
| **296** | Dimethyl-[3-(tetrahydro-furan-3-yl-methyl)-8-thiophen-2-yl-3-azaspiro[4.5]decan-8-yl]-amin |
| **297** | 1-(8-Cyclopentyl-8-dimethylamino-3-azaspiro[4.5]decan-3-yl)-2-methoxy-2-methyl-propan-1-on |
| **298** | [8-Cyclopentyl-3-(2-methoxy-2-methyl-propyl)-3-azaspiro[4.5]decan-8-yl]-dimethyl-amin |
| **299** | [3-(3-Methoxy-3-methyl-butyl)-8-thiophen-2-yl-3-azaspiro[4.5]decan-8-yl]-dimethyl-amin |
| **300** | [3-(3-Methoxy-3-methyl-butyl)-8-phenyl-3-azaspiro[4.5]decan-8-yl]-dimethylamin |
| **301** | 3-(8-Cyclopentyl-8-dimethylamino-3-azaspiro[4.5]decan-3-yl)-2-methyl-propan-1-ol |
| **302** | [8-Cyclopentyl-3-(oxetan-3-yl-methyl)-3-azaspiro[4.5]decan-8-yl]-dimethyl-amin |
| **303** | Dimethyl-[8-phenyl-3-(tetrahydro-pyran-4-yl-methyl)-3-azaspiro[4.5]decan-8-yl]-amin |
| **304** | Dimethyl-[3-(tetrahydro-pyran-4-yl-methyl)-8-thiophen-2-yl-3-azaspiro[4.5]decan-8-yl]-amin |
| **305** | Dimethyl-[8-phenyl-3-(2-tetrahydro-furan-2-yl-ethyl)-3-azaspiro[4.5]decan-8-yl]-amin |
| **306** | Dimethyl-[3-(2-tetrahydro-furan-2-yl-ethyl)-8-thiophen-2-yl-3-azaspiro[4.5]decan-8-yl]-amin |
| **307; 308** | 3-(2-Cyclobutyl-ethyl)-8-methylamino-8-thiophen-2-yl-3-azaspiro[4.5]decan-2-on |
| **309** | Dimethyl-[3-(2-tetrahydro-furan-3-yl-ethyl)-8-thiophen-2-yl-3-azaspiro[4.5]decan-8-yl]-amin |
| **310** | 3-(8-Dimethylamino-2-oxo-8-phenyl-3-azaspiro[4.5]decan-3-yl)-2,2-dimethyl-propionitril |
| **311** | 8-Dimethylamino-3-[(1-methoxy-cyclobutyl)-methyl]-8-thiophen-2-yl-3-azaspiro[4.5]decan-2-on |
| **312** | 8-Dimethylamino-3-[(1-methoxy-cyclobutyl)-methyl]-8-phenyl-3-azaspiro[4.5]decan-2-on |
| **313** | 8-Dimethylamino-3-(2-ethoxy-ethyl)-8-thiophen-2-yl-3-azaspiro[4.5]decan-2-on |
| **314** | 1-(8-Dimethylamino-8-thiophen-2-yl-3-azaspiro[4.5]decan-3-yl)-3-methoxy-3-methyl-butan-1-on |
| **315** | 1-(8-Dimethylamino-8-thiophen-2-yl-3-azaspiro[4.5]decan-3-yl)-3-methoxypropan-1-on |
| **316** | 3-(2-Cyclopropyl-ethyl)-8-methylamino-8-thiophen-2-yl-3-azaspiro[4.5]decan-2-on |
| **317** | 8-(Dimethyl-amino)-3-(3-methoxy-butyl)-8-thiophen-2-yl-3-azaspiro[4.5]decan-2-on |
| **318** | 8-(Dimethyl-amino)-3-(2-methoxy-propyl)-8-thiophen-2-yl-3-azaspiro[4.5]decan-2-on |
| **319** | 3-[8-(Dimethyl-amino)-2-oxo-8-phenyl-3-azaspiro[4.5]decan-3-yl]-2,2-dimethyl-propionitril |
| **320** | 1-[8-(Dimethyl-amino)-8-phenyl-3-azaspiro[4.5]decan-3-yl]-3-methoxy-3-methylbutan-1-on |
| **321** | 8-(Dimethyl-amino)-3-[(1-methoxy-cyclobutyl)-methyl]-8-thiophen-2-yl-3-azaspiro[4.5]decan-2-on |
| **322** | 8-(Dimethyl-amino)-3-[(1-methoxy-cyclobutyl)-methyl]-8-phenyl-3-azaspiro[4.5]decan-2-on |
| **323** | 8-(Dimethyl-amino)-3-(2-methoxy-propyl)-8-phenyl-3-azaspiro[4.5]decan-2-on |
| **324** | 8-(Dimethyl-amino)-3-(3-methoxy-butyl)-8-phenyl-3-azaspiro[4.5]decan-2-on |
| **325** | 8-(Dimethyl-amino)-3-(2-methoxy-ethyl)-8-phenyl-3-azaspiro[4.5]decan-2-on |
| **326** | 8-(Dimethyl-amino)-3-(2-ethoxy-ethyl)-8-phenyl-3-azaspiro[4.5]decan-2-on |
| **327** | 1-[8-(Dimethyl-amino)-8-thiophen-2-yl-3-azaspiro[4.5]decan-3-yl]-3-methoxy-butan-1-on |
| **328** | 1-[8-(Dimethyl-amino)-8-phenyl-3-azaspiro[4.5]decan-3-yl]-3-methoxy-butan-1-on |
| **329** | 1-[8-(Dimethyl-amino)-8-phenyl-3-azaspiro[4.5]decan-3-yl]-3-methoxy-propan-1-on |
| **330** | 3-(2-Cyclopropyl-ethyl)-8-methylamino-8-phenyl-3-azaspiro[4.5]decan-2-on |
| **331** | 3-(2-Cyclobutyl-ethyl)-8-methylamino-8-phenyl-3-azaspiro[4.5]decan-2-on |
| **332** | 8-(Dimethyl-amino)-3-(2-methoxy-2-methyl-propyl)-8-thiophen-2-yl-3-azaspiro[4.5]decan-2-on |
| **333** | 2-Cyclobutyl-1-(8-methylamino-8-thiophen-2-yl-3-azaspiro[4.5]decan-3-yl)-ethanon |
| **334** | 8-(Dimethyl-amino)-3-(2-ethoxy-propyl)-8-thiophen-2-yl-3-azaspiro[4.5]decan-2-on |
| **335** | 8-(Dimethyl-amino)-3-(2-ethoxy-propyl)-8-phenyl-3-azaspiro[4.5]decan-2-on |
| **336** | 8-(Dimethyl-amino)-3-(3-methoxy-3-methyl-butyl)-8-(5-methyl-thiophen-2-yl)-3-azaspiro[4.5]decan-2-on |
| **337** | 8-(5-Chlor-thiophen-2-yl)-8-(dimethyl-amino)-3-(3-methoxy-3-methyl-butyl)-3-azaspiro[4.5]decan-2-on |
| **338** | 1-[8-(Dimethyl-amino)-8-phenyl-3-azaspiro[4.5]decan-3-yl]-3-ethoxy-butan-1-on |
| **339** | 1-[8-(Dimethyl-amino)-8-thiophen-2-yl-3-azaspiro[4.5]decan-3-yl)-3-ethoxy-butan-1-on |
| **340** | 8-(Dimethyl-amino)-3-(2-methoxy-2-methyl-propyl)-8-phenyl-3-azaspiro[4.5]decan-2-on |
| **341** | 3-[2-(1-Methoxy-cyclobutyl)-ethyl]-8-methylamino-8-thiophen-2-yl-3-azaspiro[4.5]decan-2-on |
| **342** | Cyclobutyl-[8-(dimethyl-amino)-8-phenyl-3-azaspiro[4.5]decan-3-yl]-methanon |
| **343** | 1-[8-(Dimethyl-amino)-8-phenyl-3-azaspiro[4.5]decan-3-yl]-2-methyl-propan-1-on |
| **344** | 1-[[8-(Dimethyl-amino)-2-oxo-8-thiophen-2-yl-3-azaspiro[4.5]decan-3-yl]-methyl]-cyclopropan-1-carbonitril |
| **345** | [8-(Dimethyl-amino)-8-phenyl-3-azaspiro[4.5]decan-3-yl]-(1-methoxy-cyclopropyl)-methanon |
| **346** | [3-(2-Cyclobutyl-ethyl)-8-thiophen-2-yl-3-azaspiro[4.5]decan-8-yl]-methyl-amin |
| **347** | 8-(Dimethyl-amino)-3-(2-tetrahydro-pyran-4-yl-ethyl)-8-thiophen-2-yl-3-azaspiro[4.5]decan-2-on |
| **348** | 8-(Dimethyl-amino)-8-phenyl-3-(2-tetrahydro-pyran-4-yl-ethyl)-3-azaspiro[4.5]decan-2-on |
| **349** | 1-[[8-(Dimethyl-amino)-2-oxo-8-phenyl-3-azaspiro[4.5]decan-3-yl]-methyl]-cyclopropan-1-carbonitril |
| **350** | 1-[8-(Dimethyl-amino)-8-phenyl-3-azaspiro[4.5]decan-3-yl]-2-tetrahydro-pyran-4-yl-ethanon |
| **351** | 1-[8-(Dimethyl-amino)-8-thiophen-2-yl-3-azaspiro[4.5]decan-3-yl]-2-tetrahydropyran-4-yl-ethanon |
| **352** | 1-[[8-(Dimethyl-amino)-8-phenyl-3-azaspiro[4.5]decan-3-yl]-methyl]-cyclopropan-1-carbonitril |
| **353** | 1-[[8-(Dimethyl-amino)-2-oxo-8-thiophen-2-yl-3-azaspiro[4.5]decan-3-yl]-methyl]-cyclobutan-1-carbonitril |
| **354** | 1-[8-(Dimethyl-amino)-8-phenyl-3-azaspiro[4.5]decan-3-yl]-3-(1-methoxy-cyclobutyl)-propan-1-on |
| **355** | 1-[8-(Dimethyl-amino)-8-thiophen-2-yl-3-azaspiro[4.5]decan-3-yl]-3-(1-methoxy-cyclobutyl)-propan-1-on |
| **356** | [8-(Dimethyl-amino)-8-thiophen-2-yl-3-azaspiro[4.5]decan-3-yl]-(1-methoxy-cyclopropyl)-methanon |
| **357** | 1-[[8-(Dimethyl-amino)-2-oxo-8-phenyl-3-azaspiro[4.5]decan-3-yl]-methyl]-cyclobutan-1-carbonitril |
| **358** | [3-[(1-Methoxy-cyclopropyl)-methyl]-8-thiophen-2-yl-3-azaspiro[4.5]decan-8-yl]-dimethyl-amin |
| **359** | 1-[[8-(Dimethyl-amino)-8-phenyl-3-azaspiro[4.5]decan-3-yl]-methyl]-cyclobutan-1-carbonitril |
| **360** | 1-[[8-(Dimethyl-amino)-8-thiophen-2-yl-3-azaspiro[4.5]decan-3-yl]-methyl]-cyclopropan-1-carbonitril |
| **361** | 1-[[8-(Dimethyl-amino)-8-thiophen-2-yl-3-azaspiro[4.5]decan-3-yl]-methyl]-cyclobutan-1-carbonitril |
| **362** | 1-[3-[8-(Dimethyl-amino)-8-phenyl-3-azaspiro[4.5]decan-3-yl]-3-oxo-propyl]-cyclobutan-1-carbonitril |
| **363** | 1-[3-[8-(Dimethyl-amino)-8-thiophen-2-yl-3-azaspiro[4.5]decan-3-yl]-3-oxo-propyl]-cyclobutan-1-carbonitril |
| **364** | 1-(8-Butyl-8-dimethylamino-3-azaspiro[4.5]decan-3-yl)-2-cyclopropyl-ethanon |
| **365** | Cyclopropyl-(8-dimethylamino-8-phenyl-3-azaspiro[4.5]decan-3-yl)-methanon |
| **366** | 1-(8-Dimethylamino-8-phenyl-3-azaspiro[4.5]decan-3-yl)-3-methyl-butan-1-on |
| **367** | 1-(8-Butyl-8-dimethylamino-3-azaspiro[4.5]decan-3-yl)-3-cyclopropyl-propan-1-on |
| **368** | 1-(8-Butyl-8-dimethylamino-3-azaspiro[4.5]decan-3-yl)-4-methoxy-4-methyl-pentan-1-on |
| **369** | [3-[(1-Methoxy-cyclopropyl)-methyl]-8-phenyl-3-azaspiro[4.5]decan-8-yl]-dimethyl-amin |
| **370** | 8-(Dimethyl-amino)-3-(3-methyl-butyl)-8-thiophen-2-yl-3-azaspiro[4.5]decan-2-on |
| **371** | 1-[3-[8-(Dimethyl-amino)-8-phenyl-3-azaspiro[4.5]decan-3-yl]-3-oxo-propyl]-cyclopropan-1-carbonitril |
| **372** | 1-[3-[8-(Dimethyl-amino)-8-thiophen-2-yl-3-azaspiro[4.5]decan-3-yl]-3-oxo-propyl]-cyclopropan-1-carbonitril |
| **373** | 1-(8-Dimethylamino-8-thiophen-2-yl-3-azaspiro[4.5]decan-3-yl)-2-(1-methoxy-cyclopropyl)-ethanon |
| **374** | 1-(8-Dimethylamino-8-phenyl-3-azaspiro[4.5]decan-3-yl)-2-(1-methoxy-cyclopropyl)-ethanon |
| **375** | 1-(8-Butyl-8-dimethylamino-2-azaspiro[4.5]decan-2-yl)-2-cyclobutyl-ethanon |
| **376** | 8-Dimethylamino-3-(3-methyl-butyl)-8-phenyl-3-azaspiro[4.5]decan-2-on |
| **377** | 8-Dimethylamino-3-(2-methyl-propyl)-8-thiophen-2-yl-3-azaspiro[4.5]decan-2-on |
| **378** | 8-Dimethylamino-3-(2-methyl-propyl)-8-phenyl-3-azaspiro[4.5]decan-2-on |
| **379** | (8-Dimethylamino-8-phenyl-2-azaspiro[4.5]decan-2-yl)-[1-(methoxymethyl)-cyclopropyl]-methanon |
| **380** | 1-(8-Dimethylamino-8-phenyl-2-azaspiro[4.5]decan-2-yl)-3-methoxy-2,2-dimethyl-propan-1-on |
| **381** | (8-Dimethylamino-8-phenyl-2-azaspiro[4.5]decan-2-yl)-[1-(methoxymethyl)-cyclobutyl]-methanon |
| **382** | (8-Dimethylamino-8-thiophen-2-yl-3-azaspiro[4.5]decan-3-yl)-(1-methoxy-cyclobutyl)-methanon |
| **383** | (8-Dimethylamino-8-thiophen-2-yl-3-azaspiro[4.5]decan-3-yl)-[1-(methoxymethyl)-cyclopropyl]-methanon |
| **384** | 1-(8-Dimethylamino-8-thiophen-2-yl-3-azaspiro[4.5]decan-3-yl)-3-methoxy-2,2-dimethyl-propan-1-on |
| **385** | (8-Dimethylamino-8-thiophen-2-yl-3-azaspiro[4.5]decan-3-yl)-[1-(methoxymethyl)-cyclobutyl]-methanon |
| **386** | 8-Dimethylamino-3-[2-(1-methoxy-cyclopropyl)-ethyl]-8-thiophen-2-yl-3-azaspiro[4.5]decan-2-on |
| **387** | 8-Dimethylamino-3-[2-(1-methoxy-cyclopropyl)-ethyl]-8-phenyl-3-azaspiro[4.5]decan-2-on |
| **388** | 1-(8-Butyl-8-dimethylamino-3-azaspiro[4.5]decan-3-yl)-2-(1-methoxy-cyclobutyl)-ethanon |
| **389** | 1-(8-Butyl-8-dimethylamino-3-azaspiro[4.5]decan-3-yl)-2-tetrahydro-furan-3-yl-ethanon |
| **390** | (8-Dimethylamino-8-phenyl-3-azaspiro[4.5]decan-3-yl)-(1-methoxy-cyclobutyl)-methanon |
| **391** | [8-Dimethylamino-8-(5-methyl-thiophen-2-yl)-3-azaspiro[4.5]decan-3-yl]-[1-(methoxymethyl)-cyclopropyl]-methanon |
| **392** | [8-Dimethylamino-8-(5-methyl-thiophen-2-yl)-3-azaspiro[4.5]decan-3-yl]-[1-(methoxymethyl)-cyclobutyl]-methanon |
| **393** | 1-[8-Dimethylamino-8-(5-methyl-thiophen-2-yl)-3-azaspiro[4.5]decan-3-yl]-3-methoxy-2,2-dimethyl-propan-1-on |
| **394** | 5-(8-Dimethylamino-8-phenyl-3-azaspiro[4.5]decan-3-yl)-2,2-dimethyl-5-oxo-pentannitril |
| **395** | 5-(8-Dimethylamino-8-thiophen-2-yl-3-azaspiro[4.5]decan-3-yl)-2,2-dimethyl-5-oxo-pentannitril |
| **396** | [8-(5-Chlor-thiophen-2-yl)-8-dimethylamino-3-azaspiro[4.5]decan-3-yl]-[1-(methoxymethyl)-cyclopropyl]-methanon |
| **397** | [8-(5-Chlor-thiophen-2-yl)-8-dimethylamino-3-azaspiro[4.5]decan-3-yl]-[1-(methoxymethyl)-cyclobutyl]-methanon |
| **398** | 1-[8-(5-Chlor-thiophen-2-yl)-8-dimethylamino-3-azaspiro[4.5]decan-3-yl]-3-methoxy-2,2-dimethyl-propan-1-on |
| **399; 400** | 8-Butyl-8-(dimethyl-amino)-3-(2-tetrahydro-furan-3-yl-ethyl)-3-azaspiro[4.5]decan-2-on |
| **401; 402** | 1-[2-[8-Butyl-8-(dimethyl-amino)-2-oxo-3-azaspiro[4.5]decan-3-yl]-ethyl]-cyclopropan-1-carbonitril |
| **403; 404** | 1-[2-[8-Butyl-8-(dimethyl-amino)-2-oxo-3-azaspiro[4.5]decan-3-yl]-ethyl]-cyclobutan-1-carbonitril |
| **405** | 1-[8-(Dimethyl-amino)-8-phenyl-3-azaspiro[4.5]decan-3-yl]-3-(1-methoxy-cyclopropyl)-propan-1-on |
| **406** | 8-Butyl-8-(dimethyl-amino)-3-[2-(1-methoxy-cyclobutyl)-ethyl]-3-azaspiro[4.5]decan-2-on |
| **407** | [8-(Dimethyl-amino)-8-(5-fluor-thiophen-2-yl)-3-azaspiro[4.5]decan-3-yl]-[1-(methoxymethyl)-cyclobutyl]-methanon |
| **408** | 2-Cyclopropyl-1-[8-(dimethyl-amino)-8-phenyl-3-azaspiro[4.5]decan-3-yl]-ethanon |
| **409; 410** | 8-Butyl-8-(dimethyl-amino)-3-[2-(oxetan-3-yl)-ethyl]-3-azaspiro[4.5]decan-2-on |
| **411** | [8-(Dimethyl-amino)-8-(5-fluor-thiophen-2-yl)-3-azaspiro[4.5]decan-3-yl]-[1-(methoxymethyl)-cyclopropyl]-methanon |
| **412** | 1-[8-(Dimethyl-amino)-8-thiophen-2-yl-3-azaspiro[4.5]decan-3-yl]-3-(1-methoxy-cyclopropyl)-propan-1-on |
| **413** | 8-Butyl-8-(dimethyl-amino)-3-[2-(1-methoxy-cyclobutyl)-ethyl]-3-azaspiro[4.5]decan-2-on |
| **414** | 2-Cyclobutyl-1-[8-(dimethyl-amino)-8-phenyl-3-azaspiro[4.5]decan-3-yl]-ethanon |
| **415** | 1-[8-(Dimethyl-amino)-8-thiophen-2-yl-3-azaspiro[4.5]decan-3-yl]-3-methoxypropan-1-on |
| **416** | 1-[8-(Dimethyl-amino)-8-thiophen-2-yl-3-azaspiro[4.5]decan-3-yl]-3-methylbutan-1-on |
| **417** | 8-(Dimethyl-amino)-8-(5-fluor-thiophen-2-yl)-3-(3-methoxy-3-methyl-butyl)-3-azaspiro[4.5]decan-2-on |
| **418** | 1-[8-Dimethylamino-8-(5-fluor-thiophen-2-yl)-3-azaspiro[4.5]decan-3-yl]-3-methoxy-3-methyl-butan-1-on |
| **419** | 3-Methoxy-1-(8-methylamino-8-thiophen-2-yl-3-azaspiro[4.5]decan-3-yl)-propan-1-on |
| **420** | 2-Cyclobutyl-1-(8-methylamino-8-phenyl-3-azaspiro[4.5]decan-3-yl)-ethanon |
| **421** | 8-(Dimethyl-amino)-8-(5-fluor-thiophen-2-yl)-3-[2-(1-methoxy-cyclopropyl)-ethyl]-3-azaspiro[4.5]decan-2-on |
| **422** | 2-Cyclopropyl-1-(8-methylamino-8-phenyl-3-azaspiro[4.5]decan-3-yl)-ethanon |
| **423** | 3-Methyl-1-(8-methylamino-8-thiophen-2-yl-3-azaspiro[4.5]decan-3-yl)-butan-1-on |
| **424** | 8-Cyclopentyl-8-dimethylamino-2-azaspiro[4.5]decan |
| **425** | 8-(5-Chlor-2-thiophen-2-yl)-2-aza-spiro[4.5]dec-8-yl]-dimethylamin |
| **426** | [8-(5-Fluor-thiophen-2-yl)-2-aza-spiro[4.5]dec-8-yl]-dimethylamin |
| **427; 428** | 8-(Azetidin-1-yl)-8-(2-thienyl)-3-azaspiro[4.5]decan |
| **429,430** | 8-Azetidin-1-yl-8-phenyl-2-azaspiro[4.5]decan |
| **431** | 8-Dimethylamino-8-phenyl-2-azaspiro[4.5]decan-3-on |
| **432; 433** | 8-Butyl-8-dimethylamino-2-azaspiro[4.5]decan-3-on |
in Form eines einzelnen Stereoisomers oder deren Mischung, der freien Verbindungen und/oder ihrer physiologisch verträglichen Salze und/oder Solvate.

12. Arzneimittel enthaltend wenigstens eine Verbindung nach einem der Ansprüche 1 bis 11 in Form eines einzelnen Stereoisomers oder deren Mischung, der freien Verbindungen und/oder ihrer physiologisch verträglichen Salze und/oder Solvate, sowie ggf. geeignete Zusatz- und/oder Hilfsstoffe und/oder gegebenenfalls weiterer Wirkstoffe.

13. Verbindung nach einem der Ansprüche 1 bis 11 in Form eines einzelnen Stereoisomers oder deren Mischung, der freien Verbindung und/oder ihrer physiologisch verträglichen Salze und/oder Solvate zur Anwendung bei der Behandlung von Schmerz.

14. Verbindung nach einem der Ansprüche 1 bis 1 in Form eines einzelnen Stereoisomers oder deren Mischung, der freien Verbindungen und/oder ihrer physiologisch verträglichen Salze und/oder Solvate zur Anwendung bei der Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. A compound of the general formula (1), 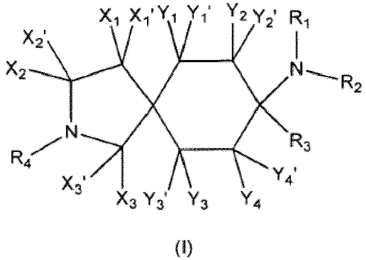 wherein
Y₁, Y₁' , Y₂, Y₂' , Y₃, Y₃', Y₄ and Y₄' in each case are selected independently of each other from the group consisting of -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, - NHC(=O)NH₂, -NHC(=O)NHR₀ and -NHC(=O)N(R₀)2; or Y₁ and Y₁' , or Y₂ and Y₂' , or Y₃ and Y₃', or Y₄ and Y₄' together represent =O;
X₁, X₁' , X₂' , X₃ and X₃' in each case independently of each other represent -H, -F, -Cl, -Br, -I, -NO₂, -CF₃, -OR₅, -SR₅, -SO₂R₅, -S(=O)₂OR₅, -CN, -COOR₅, -CONR₅, -NR₆R₇ or -R₀; or X₁ and X₁' , or X₂ and X₂' , or X₃ and X₃' together represent =O;
or X₁ and X₂ or X₂ and X₃ together represent -(CH₂)₂₋₆-, wherein individual hydrogen atoms can also be replaced by -F, -Cl, -Br, - I, -NO₂, -CF₃, -OR₅, -CN or -C₁₋₆-aliphatic;
or X₁ and X₁' or X₂ and X₂' or X₃ and X₃' together represent a C₃₋₆-cycloaliphatic, wherein individual hydrogen atoms can also be replaced by -F, -Cl, -Br, -I, -NO₂, -CF₃, -OR₅, -CN or -C₁₋₆-aliphatic;
R₀ in each case independently represents -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, - C₃₋₈-cycloaliphatic-C₁₋₈-aliphatic, -C₃₋₈-cycloaliphatic-aryl or -C₃₋₈-cycloaliphatic-heteroaryl;
R₁ and R₂ independently of each other represent -H or -R₀; or R₁ and R₂ together represent -CH₂CH₂OCH₂CH₂-, -CH₂CH₂NR₈CH₂CH₂- or -(CH₂)₃₋₆-;
R₃ represents -R₀;
R₄ represents H or -Z-R₁₁,
wherein
Z can be absent or -C(=O)-, -S(=O)- or -S(=O)₂-, and R₁₁ represents -C₁₋₆-alkyl, -C₃₋₆-cycloalkyl, or -C₁₋₃-alkyl-C₃₋₆-cycloalkyl, wherein in the C₃₋₆-cycloalkyl group a carbon ring atom can be replaced by an oxygen atom and -C₁₋₆-alkyl, -C₃₋₆-cycloalkyl or unsubstituted -C₁₋₃-alkyl-C₃₋₆-cycloalkyl can be substituted one or more times with substituents selected from the group consisting of -F, -Cl, -Br, -I, -CN, -OH, -SH, -O-C₁₋₃-alkyl, and -S-C₁₋₃-alkyl, wherein -C₁₋₃-alkyl can be substituted with one or more substituents from the group comprising substituents -F, -Cl, -Br, -I, -CN, -OH, -SH;
R₅ in each case independently represents -H or -R₀;
R₆ and R₇ independently of each other represent -H or -R₀; or R₆ and R₇ together represent -CH₂CH₂OCH₂CH₂-, -CH₂CH₂NR₁₀CH₂CH₂- or -(CH₂)₃₋₆-;
R₈ represents -H, -R₀ or -C(=O)R₀;
R₁₀ represents -H or -C₁₋₆-aliphatic;
wherein
"aliphatic" in each case is a branched or unbranched, saturated or mono- or polyunsaturated, unsubstituted or mono- or polysubstituted, aliphatic hydrocarbon radical;
"cycloaliphatic" in each case is a saturated or mono- or polyunsaturated, unsubstituted or mono- or polysubstituted, alicyclic, mono- or polycyclic hydrocarbon radical;
wherein with respect to "aliphatic" and "cycloaliphatic", "mono- or polysubstituted" is understood as meaning the substitution once or several times of one or more hydrogen atoms by -F, -Cl, -Br, - I, -CN, -NO₂, -CHO, =O, -R₀,
-C(=O)R₀, -C(=O)H, -C(=O)-OH, -C(=O)OR₀, -C(=O)NH₂, -C(=O)NHR₀, - C(=O)N(R₀)₂, -OH, -OR₀, -OC(=O)H,
-OC(=O)R₀, -OC(=O)OR₀, -OC(=O)NHR₀, -OC(=O)N(R₀)₂, -SH, -SR₀, -SO₃H, -S(=O)₁₋₂-R₀,
-S(=O)₁₋₂NH₂, -NH₂, -NHR₀, -N(R₀)₂, -N⁺(R₀)₃, -N⁺(R₀)₂O⁻-, -NHC(=O)R₀, - NHC(=O)OR₀,
-NHC(=O)NH₂, -NHC(=O)NHR₀, -NH-C(=O)N(R₀)₂, -Si(R₀)₃, -PO(OR₀)₂;
"aryl" in each case independently represents a carbocyclic ring system having at least one aromatic ring, but without heteroatoms in this ring, wherein the aryl radicals can optionally be fused with further saturated, (partially) unsaturated or aromatic ring systems and each aryl radical can be unsubstituted or mono- or polysubstituted, wherein the substituents on aryl can be identical or different and can be in any desired and possible position of the aryl;
"heteroaryl" represents a 5-, 6- or 7-membered cyclic aromatic radical which contains 1, 2, 3, 4 or 5 heteroatoms, wherein the heteroatoms are identical or different and are nitrogen, oxygen or sulfur, and the heterocycle can be unsubstituted or mono- or polysubstituted; wherein in the case of substitution on the heterocycle, the substituents can be identical or different and can be in any desired and possible position of the heteroaryl; and wherein the heterocycle can also be part of a bi- or polycyclic system;
wherein with respect to "aryl" and "heteroaryl", "mono- or polysubstituted" is understood as meaning the substitution once or several times of one or more hydrogen atoms of the ring system by substituents selected from the group consisting of -F, -Cl, -Br, - I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, - OC(=O)H, -OC(=O)R₀, -OC(=O)OR₀, -OC(=O)NHR₀, -OC(=O)N(R₀)2, -SH, - SR₀, -SO₃H, -S(=O)₁₋₂-R₀, -S(=O)₁₋₂NH₂, -NH₂, -NHR₀, -N(R₀)₂, -N+(R₀)₃, -N⁺(R₀)₂O⁻, -NHC(=O)R₀, -NHC(=O)OR₀, -NH-C(=O)NH₂, -NHC(=O)NHR₀, - NHC(=O)N(R₀)₂, -Si(R₀)₃, -PO(OR₀)₂; wherein optionally present N ring atoms can in each case be oxidized (N-oxide);
in the form of a single stereoisomer or mixture thereof, the free compounds and/or the physiologically acceptable salts and/or solvates thereof.

2. A compound according to claim 1, wherein Y₁' , Y₂' , Y₃' and Y₄' each represent -H.

3. A compound according to claim 1 or 2, wherein
R₀ in each case independently represents -C₁₋₈-aliphatic, -C₃₋₁₂-cycloaliphatic, -aryl,
-heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl; wherein these are unsubstituted or mono- or polysubstituted with substituents selected independently of each other from the group consisting of -F, -Cl, -Br, -CN, -CH₃, -C₂H₅, -NH₂,
-NO₂, -SH, -CF₃, OH, -OCH₃, -OC₂H₅ and -N(CH₃)₂.

4. A compound according to one of claims 1 to 3, wherein
R₃ represents -C₁₋₈-aliphatic, -aryl, -heteroaryl, -C₁₋₃-aliphatic-aryl, -C₁₋₃-aliphatic- heteroaryl or -C₁₋₃-aliphatic-C₅₋₆-cycloaliphatic; wherein these are unsubstituted or mono- or polysubstituted with substituents selected independently of each other from the group consisting of -F, -Cl, -Br, -CN, -CH₃, -C₂H₅, -NH₂, -NO₂, -SH, -CF₃, OH, -OCH₃, -OC₂H₅ and -N(CH₃)₂;
and
X₁, X₁' , X₂' , X₃ and X₃' in each case independently of each other represent -H, -F, -Cl, -Br, -I, -NO₂, -CF₃, -OR₅, -SR₅, - SO₂R₅, -S(=O)₂OR₅, -CN, -COOR₅, -CONR₅, -NR₆R₇ or -R₀; or one of the radicals X₁ and X₁' represents H and the other represents -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl; wherein these are unsubstituted or mono- or polysubstituted with substituents selected independently of each other from the group consisting of -F, -Cl, -Br, -CN, -CH₃, -C₂H₅, -NH₂, -NO₂, -SH, -CF₃, OH, -OCH₃, -OC₂H₅ and
-N(CH₃)₂; or X₁ and X₁', or X₂ and X₂' , or X₃ and X₃' together represent =O; or X₁ and X₁' together represent C₃₋₆-cycloalkyl, which can be unsubstituted or substituted with one or more substituents selected independently of each other from the group consisting of -F, -Cl, -Br, -I, -OR₅, SR₅, C₁₋₃-alkyl or -CN.

5. A compound according to one of claims 1 to 4, wherein Y₁, Y₁', Y₂, Y₂', Y₃, Y₃', Y₄ and Y₄' each represent -H.

6. A compound according to one of claims 1 to 5, which has the general formula (3.1) .

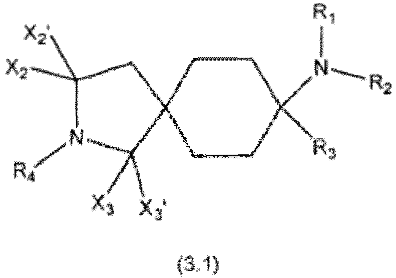

7. A compound according to one of claims 1 to 6, wherein
Y₁, Y₁', Y₂, Y₂', Y₃, Y₃', Y₄ and Y₄' each represent -H;
X₁, X₁', X₂, X₂', X₃ and X₃' represent H; or X₂ and X₂', or X₃ and X₃' together represent =O; or X₁ and X₁' together represent a C₃₋₆-cycloalkyl;
R₀ in each case independently represents -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl,-C₃₋₈-cycloaliphatic-C₁₋₈-aliphatic, -C₃₋₈-cycloaliphatic-aryl or -C₃₋₈-cycloaliphatic-heteroaryl; wherein these are unsubstituted or mono- or polysubstituted with substituents selected independently of each other from the group consisting of -F, -Cl, -Br, -CN, -CH₃, -C₂H₅, -NH₂,
-NO₂, -SH, -CF₃, OH, -OCH₃, -OC₂H₅ and -N(CH₃)₂;
R₁ represents -CH₃;
R₂ represents -H or -CH₃; or
R₁ and R₂ together form a ring and represent -(CH₂)₃₋₄; and
R₃ represents -C₁₋₈-aliphatic, -aryl, -heteroaryl, -C₁₋₃-aliphatic-aryl, -C₁₋₃-aliphatic-heteroaryl or -C₁₋₃-aliphatic-C₅₋₆-cycloaliphatic; wherein these are unsubstituted or mono- or polysubstituted with substituents selected independently of each other from the group consisting of -F, -Cl, -Br, -CN, -CH₃, -C₂H₅,
-NH₂, -NO₂, -SH, -CF₃, OH, -OCH₃, -OC₂H₅ and
-N(CH₃)₂; and
R₄ represents H or -Z-R₁₁,
wherein
-Z- can be absent or -C(=O)-, and
R₁₁ represents -C₁₋₆-alkyl, -C₃₋₆-cycloalkyl, or -C₁₋₃-alkyl-C₃₋₆-cycloalkyl, wherein a carbon ring atom in the C₃₋₆-cycloalkyl group can be replaced by an oxygen atom, and -C₁₋₆-alkyl, -C₃₋₆-cycloalkyl, or -C₁₋₃-alkyl-C₃₋₆-cycloalkyl can be unsubstituted or substituted one or more times with substituents selected independently of each other from the group consisting of -F, -Cl,
-Br, -I, -CN, -OH, -SH, -O-C₁₋₃-alkyl, and -S-C₁₋₃-alkyl, wherein-C₁₋₃-alkyl can be substituted with one or more substituents from the group consisting of the substituents -F, -Cl, -Br, -I, -CN, - OH and -SH;

8. A compound according to one of claims 1 to 7, wherein R₁ and R₂ each represent -CH₃.

9. A compound according to one of claims 1 to 8, wherein R₃ is selected from the group consisting of phenyl, benzyl, and 2-thienyl, in each case unsubstituted or substituted one or more times with substituents selected independently of each other from the group consisting of -F, -Cl, -Br, -CN, -CH₃, -C₂H₅, -NH₂, -NO₂, -SH, -CF₃, OH, -OCH₃, -OC₂H₅ and -N(CH₃)₂.

10. A compound according to one of claims 1 to 9, wherein R4 is one selected from the group consisting of H, CH₃, ethyl, n-propyl, i-propyl, n-butyl, s-butyl, isobutyl, t-butyl, n-pentyl, s-pentyl, isopentyl, 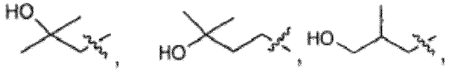 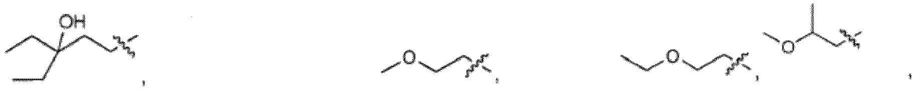 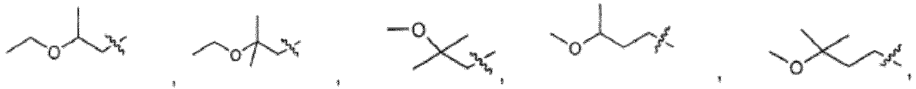 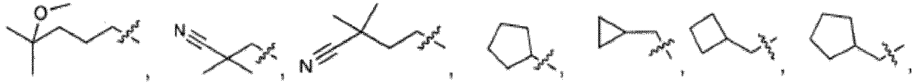 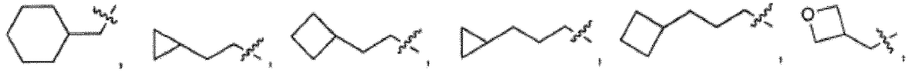 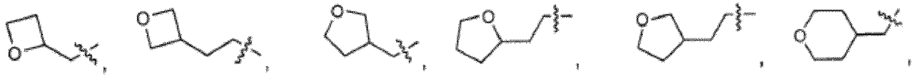 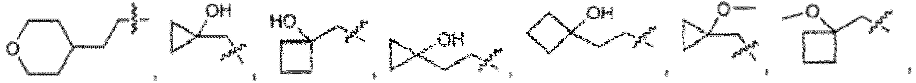 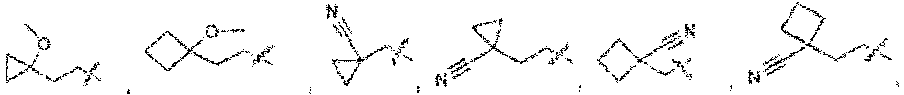 Acetyl,

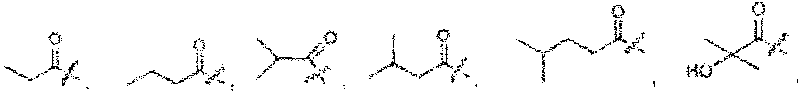

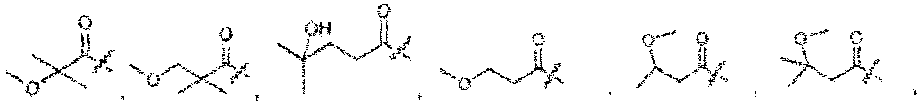

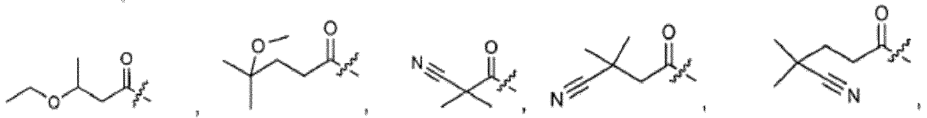

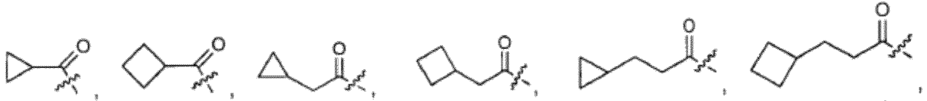

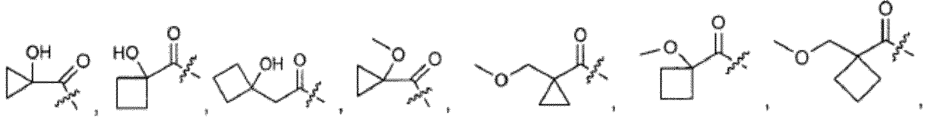

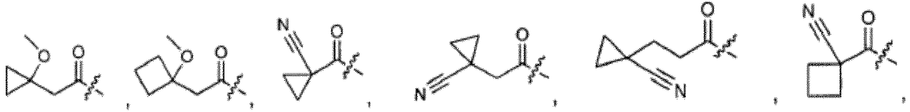

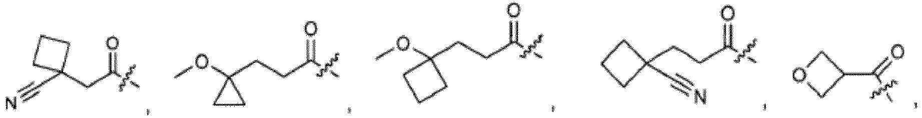

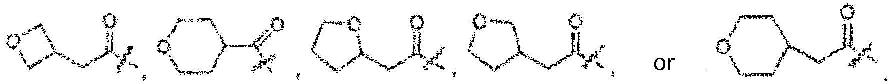

11. A compound according to one of claims 1 to 9 from the following group:
| | |
|---|---|
| **1; 2** | 8-Benzyl-8-(dimethyl-amino)-3-azaspiro[4.5]decane-4-one |
| **3; 4** | (8-Benzyl-3-azaspiro[4.5]decane-8-yl)-dimethyl-amine |
| **5; 6** | (8-Benzyl-3-methyl-3-azaspiro[4.5]decane-8-yl)-dimethyl-amine |
| **7; 8** | 1-[8-Benzyl-8-(dimethyl-amino)-3-azaspiro[4.5]decane-3-yl]-ethanone |
| **9** | (8-Benzyl-3-butyl-3-azaspiro[4.5]decane-8-yl)-dimethyl-amine |
| **10; 11** | 1-[B-Benzyl-8-(dimethyl-amino)-3-azaspiro[4.5]decane-3-yl]-butan-1-one |
| **12** | [8-Benzyl-3-(cyclopentyl-methyl)-3-azaspiro[4.5]decane-8-yl]-dimethyl-amine |
| **13** | 8-Dimethylamino-8-phenyl-3-azaspiro[4.5]decane-4-one |
| **14; 15** | 8-(Dimethyl-amino)-8-phenyl-3-azaspiro[4.5]decane-2-one |
| **16** | 8-Butyl-8-dimethylamino-3-azaspiro[4.5]decane-4-one |
| **17** | 8-Dimethylamino-8-thiophen-2-yl-3-azaspiro[4.5]decane-4-one |
| **18; 25** | Dimethyl-(8-thiophen-2-yl-3-azaspiro[4.5]decane-8-yl)-amine |
| **19** | Dimethyl-(3-methyl-8-thiophen-2-yl-3-azaspiro[4.5]decane-8-yl)-amine |
| **20** | 1-(8-Dimethylamino-8-thiophen-2-yl-3-azaspiro[4.5]decane-3-yl)butane-1-one |
| **21** | (3-Butyl-8-thiophen-2-yl-3-azaspiro[4.5]decane-8-yl)-dimethyl-amine |
| **22** | [3-(Cydopentyl-methyl)-8-thiophen-2-yl-3-azaspiro[4.5]decane-8-yl]-dimethylamine |
| **23** | 8-(Dimethyl-amino)-8-thiophen-2-yl-2-azaspiro[4.5]decane-3-one |
| **24a/b** | 8-(Dimethy)-amino)-8-thiophen-2-yl-2-azaspiro[4.5]decane-3-one |
| **26** | 1-[8-(Dimethyl-amino)-8-thiophen-2-yl-3-azaspiro[4.5]decane-3-yl]butane-1-one |
| **27** | (8-Benzyl-3-butyl-3-azaspiro[4.5]decane-8-yl)-dimethyl-amine |
| **28; 29** | 1-(8-(Dimethyl-amino)-8-phenyl-3-azaspiro[4.5]decane-3-yl]butane-1-one |
| **30** | 8-(Dimethyl-amino)-8-(5-methyl-thiophen-2-yl)-3-azaspiro[4.5]decane -4-one |
| **31** | Dimethyl-[8-(5-methyl-thiophen-2-yl)-3-azaspiro[4.5]decane-8-yl]-amine |
| **32** | 1-[8-(Dimethyl-amino)-8-(5-methyl-thiophen-2-yl)-3-azaspiro[4.5]decane-3-yl]-butane-1-one |
| | 3-Butyl-8-(dimethyl-amino)-8-thiophen-2-yl-3-azaspiro[4.5]decane-2-one |
| **34** | 8-(Dimethyl-amino)-3-methyl-8-thiophen-2-yl-3-azaspiro[4.5]decane-2-one |
| **35** | [3-Butyl-8-(5-methyl-thiophen-2-yl)-3-azaspiro[4.5]decane-8-yl]-dimethyl-amine |
| **36** | Cyclobutyl-[8-dimethylamino-8-(5-methyl-thiophen-2-yl)-3-azaspiro[4.5]decane-3-yl]-methanone |
| **37** | Cyclopropyl-[8-dimethylamino-8-(5-methyl-thiophen-2-yl)-3-azaspiro[4.5]decane--3-yl]-methanone |
| **38** | 1-[8-Dimethylamino-8-(5-methy-thiophen-2-yl)-3-azaspiro[4.5]decane-3-yl]-2-methyl-propane-1-one |
| **39** | 1-[8-Dimethylamino-8-(5-methyl-thiophen-2-yl)-3-azaspiro[4.5]decane-3-yl]-3-methyl-butane-1-one |
| **40** | 1-[8-Dimethylamino-8-(5-methyl-thiophen-2-yl)-3-azaspiro[4.5]decane-3-yl]-propane-1-one |
| **41** | [3-(2-Methoxy-ethyl)-8-(5-methyl-thiophen-2-yl)-3-azaspiro[4.5]decane-8-yl]-dimethyl-amine |
| **42** | 1-[8-(Dimethyl-amino)-8-thiophen-2-yl-3-azaspiro[4.5]decane-3-yl]-2-methoxy-ethanone |
| **43** | Cyclobutyl-[8-(dimethyl-amino)-8-thiophen-2-yl-3-azaspiro[4.5]decane-3-yl]-methanone |
| **44** | Cyclopropyl-[8-(dimethyl-amino)-8-thiophen-2-yl-3-azaspiro[4.5]decane-3-yl]-methanone |
| **45** | 1-[8-(Dimethyl-amino)-8-thiophen-2-yl-3-azaspiro[4.5]decane-3-yl]-2-methyl-propane-1-one |
| **46** | 1-[8-(Dimethyl-amino)-8-thiophen-2-yl-3-azaspiro[4.5]decane-3-yl]-3-methyl-butane-1-one |
| **47** | [3-(2-Methoxy-ethyl)-8-thiophen-2-yl-3-azaspiro[4-5]decane-8-yl]-dimethyl-amine |
| **48** | 2-Cyclopropyl-1-[8-(dimethyl-amino)-8-thiophen-2-yl-3-azaspiro[4.5]decane-3-yl]-ethanone |
| **49** | [3-(Cyclopropyl-methyl)-8-thiophen-2-yl-3-azaspiro[4.5]decane-8-yl]-dimethyl-amine |
| **50** | 2-Cyclobutyl-1-[8-(dimethyl-amino)-8-thiophen-2-yl-3-azaspiro[4.5]decane-3-yl]-ethanone |
| **51** | [3-(2-Cyclopropyl-ethyl)-8-thiophen-2-yl-3-azaspiro[4.5]decane-8-yl]-dimethyl-amine |
| **52** | [3-(2-Cyclobutyl-ethyl)-8-thiophen-2-yl-3-azaspiro[4.5]decane-8-yl]-dimethyl-amine |
| **53** | [3-(Cyclobutyl-methyl)-8-thiophen-2-yl-3-azaspiro[4.5]decane-8-yl]-dimethyl-amine |
| **54** | 1-[8-(Dimethyl-amino)-8-thiophen-2-yl-3-azaspiro[4.5]decane-3-yl]-4-methyl-pentan-1-one |
| **55** | 3-[8-(Dimethyl-amino)-8-thiophen-2-yl-3-azaspiro[4.5]decan-3-yl]-2,2-dimethyl-3-oxo-propionitrile |
| **56** | 1-[8-(Dimethyl-amino)-8-thiophen-2-yl-3-azaspiro[4.5]decane-3-yl]-2-tetrahydro-furan-3-yl-ethanone |
| **57** | (8-Dimethylamino-8-thiophen-2-yl-3-azaspiro[4.5]decane-3-yl)-(oxetan-3-yl)-methanone |
| **58** | 1-(8-Dimethylamino-8-thiophen-2-yl-3-azaspiro[4.5]decane-3-carbonyl)-cyclopropane-carbonitrile |
| **59** | 1-(8-Dimethylamino-8-thiophen-2-yl-3-azaspiro[4.5]decane-3-yl)-4-methoxy-4-methyl-pentan-1-one |
| **60** | [8-(Dimethyl-amino)-8-thiophen-2-yl-3-azaspiro[4.5]decane-3-yl]-tetrahydro-pyran-4-yl-methanone |
| **61** | 1-[8-(Dimethyl-amino)-8-thiophen-2-yl-3-azaspiro[4.5]decane-3-carbonyl]-cyclobutane-1-carbonitrile |
| **62** | 3-Cyclopropyl-1-[8-(dimethyl-amino)-8-thiophen-2-yl-3-azaspiro[4.5]decane-3-yl]-propane-1-one |
| **63** | 1-[8-(Dimethyl-amino)-8-thiophen-2-yl-3-azaspiro[4.5]decane-3-yl]-2-methoxy-2-methyl-propane-1-one |
| **64** | 1-[8-(Dimethyl-amino)-8-thiophen-2-yl-3-azaspiro[4.5]decane-3-yl]-2-tetrahydro-furar-2-yl-ethanone. |
| **65** | 1-[8-(Dimethyl-amino)-8-thiophen-2-yl-3-azaspiro[4.5]decane-3-yl]-4-hydroxy-4-methyl-pentan-1-one |
| **66** | [8-(Dimethyl-amino)-8-thiophen-2-yl-3-azaspiro[4.5]decane-3-yl]-(1-hydroxy-cyclobutyl)-methanone |
| **67** | 1-[2-[8-(Dimethyl-amino)-8-thiophen-2-yl-3-azaspiro[4.5]decane-3-yl]-2-oxo-ethyl]-cyclopropane-1-carbonitrile |
| **68** | 1-[2-[8-(Dimethyl-amino)-8-thiophen-2-yl-3-azaspiro[4.5]decane-3-yl]-2-oxo-ethyl-cyclobutane-1-carbonitrile |
| **69** | 3-Cyctobutyl-1-[8-(dimethyl-amino)-8-thiophen-2-yl-3-azaspiro[4.5]decane-3-yl]-propane-1-one |
| **70** | Dimethyl-[3-(oxetan-3-yl-methyl)-8-thiophen-2-yl-3-azaspiro[4.5]decane-8-yl]-amine |
| **71** | Dimethyl-(8-phenyl-3-azaspiro[4.5]decane-8-yl)-amine |
| **72** | 1-[8-(Dimethyl-amino)-8-phenyl-3-azaspiro[4.5]decane-3-yl]-butane-1-one |
| **73** | 1-(8-Dimethylamino-8-phenyl-3-azaspiro[4.5]decane-3-yl)-ethanone |
| **74** | (3-Butyl-8-phenyl-3-azaspiro[4.5]decan decane -8-yl)-dimethyl-amine |
| **75** | 1-[8-(Dimethyl-amino)-3-phenyl-3-azaspiro[4.5]decane-3-yl]-2-tetrahydro-furan-2-yl-ethanone |
| **76** | 1-[2-[8-(Dimethyl-amino)-8-phenyl-3-azaspiro[4.5]decane-3-yl]-2-oxo-ethyl]-cycto butane-1-carbonitrile |
| **77** | 1-[2-[8-(Dimethyl-amino)-8-phenyl-3-azaspiro[4.5]decane-3-yl]-2-oxo-ethyl]-cyclopropane-1-carbonitrile |
| **78** | 1-[8-(Dimethyl-amino)-8-phenyl-3-azaspiro[4.5]decane-3-yl]-2-(oxetan-3-yl)-ethanone |
| **79** | 1-[8-(Dimethyl-amino)-8-phenyl-3-azaspiro[4.5]decane-3-yl]-4-methyl-pentan-1-one |
| **80** | 3-[8-(Dimethyl-amino)-8-phenyl-3-azaspiro[4.5]decane3-yl]-2,2-dimethyl-3-oxo-propionitrile |
| **81** | [8-(Dimethyl-amino)-8-phenyl-3-azaspiro[4.5]decane-3-yl]-tetrahydro-pyran-4-yl-methanone |
| **82** | 1-[8-(Dimethyl-amino)-8-phenyl-3-azaspiro[4.5]decane-3-yl]-4-hydroxy-4-methyl-pentan-1-one |
| **83** | 1-[8-(Dimethyl-amino)-8-phenyl-3-azaspiro[4.5]decane-3-yl]-4-methoxy-4-methyl-pentan-1-one |
| **84** | [8-(Dimethyl-amino)-8-phenyl-3-azaspiro[4.5]decane-3-yl]-(1-hydroxy-cyclobutyl)-methanone |
| **85** | 2-Cyclopropyl-1-[8-(dimethyl-amino)-8-phenyl-3-azaspiro[4.5]decane-3-yl]-et ha none |
| **86** | 2-Cyclobutyl-1-[8-(dimethyl-amino)-8-phenyl-3-azaspiro[4.5]decane-3-yl]-ethanone |
| **87** | [3-(4-Methoxy-4-methyl-pentyl)-8-phenyl-3-azaspiro[4.5]decane-8-yl]-dimethyl-amine |
| **88** | 1-[8-(5-Chlor-thiophen-2-yl)-8-dimethylamino-3-azaspiro[4.5]decane-3-yl]-butane-1-one |
| **89** | [3-Butyl-8-(5-chlor-thiophen-2-yl)-3-azaspiro[4.5]decane-8-y]-dimethyl-amine |
| **90** | 1-[8-Dimethylamino-8-(5-fluor-thiophen-2-yl)-3-azaspiro[4.5]decane-3-yl]-butan-1-one |
| **91** | [3-Butyl-8-(5-fluor-thiophen-2-yl)-3-azaspiro[4.5]decane-8-yl]-dimethyl-amine |
| **92** | 8-(Cyclohexyl-methyl)-8-dimethylamino-3-azaspiro[4.5]decane-4-one |
| **93** | 1-[8-(Cyclohexyl-methyl)-8-dimethylamino-3-azaspiro[4.5]decan-3-yl]-decane-1-one |
| **94** | [3-Butyl-8-(cyclohexyl-methyl)-3-azaspiro[4.5]decane-8-yl]-dimethyl-amine |
| **95** | 1-[8-(Cyclopentyl-methyl)-8-dimethylamino-3-azaspiro[4.5]decane-3-yl]-butane-1-one |
| **96** | [3-Butyl-8-(cyclopentyl-methyl)-3-azaspiro[4.5]decane-8-yl]-dimethyl-amine |
| **97** | 1-(8-Cyclopentyl-8-dimethylamino-3-azaspiro[4.5]decane-3y)butane-1-one |
| **98** | (3-Butyl-8-cyclopentyl-3-azaspiro[4.5]decane-8-yl)-dimethyl-amine |
| **99** | Cyclobutyl-(8-cyclopentyl-8-dimethylamino-3-azaspiro[4.5]decane-3-yl)-methanone |
| **100** | (8-Cyclopentyl-8-dímethylamino-3-azaspiro[4.5]decane-3-yl)-cyclopropyl-methanone |
| **101** | [8-Cyclopentyl-3-(cyclopropyl-methyl)-3-azaspiro[4.5]decane-8-yl]-dimethyl-amine |
| **102** | [3-(Cyclobutyl-methyl)-8-cyclopentyl-3-azaspiro[4.5]decane-8-yl]-dimethyl-amine |
| **103** | 1-(8-Cyclopentyl-8-dimethylamino-3-azaspiro[4.5]decane-3-yl)-2-cyclopropyl-ethanone |
| **104** | [8-Cyclopentyl-3-(2-cyclopropyl-ethyl)-3-azaspiro[4.58]decane-8-yl]-dimethyl-amine |
| **105** | 2-Cyclobutyl-1-(8-cyclopentyl-8-dimethylamino-3-azaspiro[4.5]decane-3-yl)-ethanone |
| **106; 107** | [3-Butyl-8-(dimethyl-amino)-3-azaspiro[4.5]decane-8-yl]-phenyl-methanone |
| **108; 109** | 1-[8-(Azetidin-1-yl)-8-thiophen-2-yl-3-azaspiro[4.5]decane-3-yl]-butane-1-one |
| **110; 111** | 1-[8-(Azetidin-1-yl)-8-phenyl-3-azaspiro[4.5]decane-3-y]-butane-3-one |
| **112; 113** | 8-(Azetidin-1-yl)-3-bulyl-8-phenyl-3-azaspiro[4.5]decane |
| **114** | 8-Dimethylamino-3-methyl-8-thiophen-2-yl-3-azaspiro[4.5]decane-4-one |
| **115** | 3-Butyl-8-dimethylamino-8-thiophen-2-yl-3-azaspiro[4.5]decane-4-one |
| **116** | 3-(Cyclopentyl-methyl)-8-dimethylamino-8-thiophen-2-yl-3-azaspiro[4.5]decane-4-one |
| **117** | 8-(5-Chlor-thiophen-2-yl)-8-dimethylamino-3-azaspiro[4.5]decane-4-one |
| **118; 119** | 8-Benzyl-8-(dimethyl-amino)-3-methyl-3-azaspiro[4.5]decane-4-one |
| **120; 121** | 8-Benzyl-3-butyl-8-(dimethyl-amino)-3-azaspiro[4.5]decane-4-one |
| **122; 123** | 8-Benzyl-3-(cyclopentyl-methyl)-8-(dimethyl-amino)-3-azaspiro[4.5]decane-4-one |
| **124** | 8-(Dimethyl-amino)-3-methyl-8-thiophen-2-yl-3-azaspiro[4.5]decane-2-one |
| **125** | 3-Butyl-8-(dimethyl-amino)-8-thiophen-2-yl-3-azaspiro[4.5]decane-2-one |
| **126; 127** | 3-(Cyclopentyl-methyl)-8-(dimethyl-amino)-8-thiophen-2-yl-3-azaspiro[4.5]decane-2-one |
| **128; 129** | 3-(2-Cyclobutyl-ethyl)-8-(dimethyl-amino)-8-thiophen-2-yl-3-azaspiro[4.5]decane-2-one |
| **130; 131** | 3-(2-Cyclopropyl-ethyl)-8-(dimethyl-amino)-8-thiophen-2-yl-3-azaspíro[4.5]decane-2-one |
| **132; 133** | 3-(Cyclobutyl-methyl)-8-(dimethyl-amino)-8-thiophen-2-yl-3-azaspiro[4.5]decane-2-one |
| **134; 135** | 3-(Cyclopropyl-methyl)-8-(dimethyl-amino)-6-thiophen-2-yl-3-azaspiro[4.5]decane-2-one |
| **136; 137** | 8-(Dimethyl-amino)-3-(2-tetrahydro-furan-2-yl-ethyl)-8-thiophen-2-yl-3-azaspiro[4.5]decane-2-one |
| **138; 139** | 8-(Dimethyl-amino)-3-(tetrahydro-pyran-4-yl-methyl)-8-thiophen-2-yl-3-azaspiro[4.5]decane-2-one |
| **140; 141** | 8-(Dimethyl-amino)-3-(2-tetrahydro-furan-3-yl-ethyl)-8-thiophen-2-yl-3-azaspiro[4.5]decane-2-one |
| **142; 143** | 8-(Dimethyl-amino)-3-[2-(oxetan-3-yl)-ethyl]-8-thiophen-2-yl-3-azaspiro[4.5]decane-2-one |
| **144; 145** | 1-[2-[8-(Dimethyl-amino)-2-oxo-8-thiophen-2-yl-3-azaspiro[4.5]decane-3-yl]-ethyl]-cyclopropane-1-carbonitrile |
| **146; 147** | 8-(Dimethyl-amino)-3-(oxetan-2-yl-methyl)-8-thiophen-2-yl-3-azaspiro[4.5]decane -2-one |
| **148; 149** | 8-(Dimethyl-amino)-3-(oxetan-3-yl-methyl)-8-thiophen-2-yl-3-azaspiro[4.5]decane-2-one |
| **150; 151** | 1-[2-[8-(Dimethyl-amino)-2-oxo-thiophen-2-yl-3-azaspiro[4.5]decane-3-yl]-ethyl]-cyclobutane-1-carbonitrile |
| **152; 153** | 8-(Dimethyl-amino)-8-(5-methyl-thiophen-2-yl)-3-azaspiro[4.5]decane -2-one |
| **154; 155** | 8-(Dimethyl-amino)-3-methyl-8-(5-methyl-thiophen-2-yl)-3-azaspiro[4.5]decane-2-one |
| **156; 157** | 3-Butyl-8-(dimethyl-amino)-8-(5-methyl-thiophen-2-yl)-3-azaspiro[4.5]decane-2-one |
| **158; 159** | 3-(Cyclopentyl-methyl)-8-(dimethyl-amino)-8-(5-methyl-thiophen-2-yl)-3-azaspiro[4.5]decane-2-one |
| **160** | 3-(Cyclopropyl-methyl)-8-(dimethyl-amino)-8-(5-methyl-thiophen-2-yl)-3-azaspiro[4.5]decane-2-one |
| **161** | 3-(Cyclobutyl-methyl)-8-(dimethyl-amino)-9-(5-methyl-thiophen-2-yl)-3-azaspiro[4.5]decane-2-one |
| **162** | 8-(Dimethyl-amino)-3-methyl-8-phenyl-3-azaspiro[4.5]decane-2-one |
| **163; 164** | 3-Butyl-8-(dimethyl-amino)-8-phenyl-3-azaspiro[4.5]decane-2-one |
| **165; 166** | 3-(Cyclopentyl-methyl)-8-(dimethyl-amino)-8-phenyl-3-azaspiro[4.5]-decane-2-one |
| **167; 168** | 3-(2-Cyclopropyl-ethyl)-8-(dimethyl-amino)-8-phenyl-3-azaspiro[4.5]decane-2-one |
| **169;170** | 3-(Cyclobutyl-methyl)-8-(dimethyl-amino)-8-phenyl-3-azaspiro[4.5]decane-2-one |
| **171; 172** | 3-(Cyclopropyl-methyl)-8-(dimethyl-amino)-8-phenyl-3-azaspiro[4.5]decane-2-one |
| **173; 174** | 3-(2-Cyclobutyl-ethyl)-8-(dimethyl-amino)-8-phenyl-3-azaspiro[4.5]decane-2-one |
| **175; 176** | 1-[2-[8-(Dimethyl-amino)-2-oxo-8-phenyl-3-azaspiro[4.5]decane-3-yl]-ethyl]-cyclopropane-1-carbonitrile |
| **177; 178** | 1-[2-[8-(Dimethyl-amino)-2-oxo-8-phenyl-3-azaspiro[4.5]decane-3-yl]-ethyl]-cyclobutane-1-carbonitrile |
| **179; 180** | 8-(Dimethyl-amino)-3-[(1-hydroxy-cyclobutyl)-methyl]-8-phenyl-3-azaspiro[4.5]decane-2-one |
| **181** | 8-(Dimethyl-amino)-3-(2-hydroxy-2-methyl-propyl)-8-phenyl-3-azaspiro[4.5]decane-2-one |
| **182; 183** | 8-(Dimethyl-amino)-3-(3-methoxy-3-methyl-butyl)-8-phenyl-3-azaspiro[4.5]decane-2-one |
| **184; 185** | 8-(Dimethyl-amino)-3-[2-(1-methoxy-cyclobutyl)-ethyl]-8-phenyl-3-azaspiro[4.5]decane-2-one |
| **186; 187** | 8-(Dimethyl-amino)-3-(3-hydroxy-3-methyl-butyl)-8-phenyl-3-azaspiro[4.5]decane-2-one |
| **188** | 1-[8-(Dimethyl-amino)-8-phenyl-3-azaspiro[4.5]decane-3-yl]-2-tetrahydro-furan-3-yl-ethanone |
| **189** | [8-(Dimethyl-amino)-8-phenyl-3-azaspiro[4.5]decane-3-yl]-(oxetan-3-yl)-methanone |
| **190** | 1-[8-(Dimethyl-amino)-8-phenyl-3-azaspiro[4.5]decane-3-yl]-2-methoxy-2-methyl-propane-1-one |
| **191** | [3-(2-Cyclopropyl-ethyl)-8-phenyl-3-azaspiro[4.5]decane-8-yl]-dimethyl-amine |
| **192** | [3-(2-Cyclobutyl-ethyl)-8-phenyl-3-azaspiro[4.5]decane-6-yl]-dimethyl-amine |
| **193** | 1-[[8-(Dimethyl-amino)-8-phenyl-3-azaspiro[4.5]decane-3-yl]-methyl]-cyclobutane-1-ol |
| **194** | 1-[2-[8-(Dimethyl-amino)-8-phenyl-3-azaspiro[4.5]decane-3-yl]-ethyl]-cyclobutane-1-carbonitrile |
| **195** | 3-Cyclobutyl-1-[8-(dimethyl-amino)-8-phenyl-3-azaspiro[4.5]decane-3-yl]-propane-1-one |
| **196** | Dimethyl-[3-(oxetan-3-yl-methyl)-8-phenyl-3-azaspiro[4.5]decane-8-yl]-amine |
| **197** | 3-Cyclopropyl-1-[8-(dimethyl-amino)-8-phenyl-3-azaspiro[4.5]decane-3-yl]-propane-1-one |
| **198** | 1-[8-(Dimethyl-amino)-8-phenyl-3-azaspiro[4.5]decane-3-carbonyl]-cyclopropane-1-carbonitrile |
| **199** | 1-[8-(Dimethyl-amino)-8-phenyl-3-azaspiro[4.5]decane-3-carbonyl]-cyclobutane-1-carbonitrile |
| **200** | 1-[2-[8-(Dimethyl-amino)-8-phenyl-3-azaspiro[4.5]decane-3-yl]-ethyl]-cyclopropane-1-carbonitrile |
| **201** | Dimethyl-[8-phenyl-3-(2-tetrahydro-furan-3-yl-ethyl)-3-azaspiro[4.5]decane-8-yl]-amine |
| **202** | [3-(2-Methoxy-2-methyl-propyl)-8-phenyl-3-azaspiro[4.5]decane-8-yl]-dimethyl-amine |
| **203** | [3-(3-Cyclobutyl-propyl)-8-phenyl-3-azaspiro[4.5]decane-8-yl]-dimethyl-amine |
| **204** | [3-(3-Cyclopropyl-propyl)-8-phenyl-3-azaspiro[4.5]decane-8-yl]-dimethyl-amine |
| **205** | [8-(Dimethyl-amino)-8-phenyl-3-azaspiro[4.5]decane-3-yl]-(1-hydroxy-cyclopropyl)-methanone |
| **206** | 1-[[8-Dimethyl-amino)-8-phenyl-3-azaspiro[4.5]decane-3-yl]-methyl]-cyclopropane-1-ol |
| **207** | 1-[[8-Dimethyl-amino)-8-phenyl-3-azaspiro[4.5]decane-3-yl]-methyl]-cyclobutane-1-ol |
| **208** | 1-[2-[8-(Dimethyl-amino)-8-thiophen-2-yl-3-azaspiro[4.5]decane-3-yl]-ethyl]-cyclobutane-1-carbonitrile |
| **209** | [8-(Dimethyl-amino)-8-thiophen-2-yl-3-azaspiro[4.5]decane-3-yl]-(1-hydroxy-cyclopropyl)-methanone |
| **210** | 1-[[8-(Dimethyl-amino)-8-thiophen-2-yl-3-azaspiro[4.5]decane-3-yl]-methyl]-cyclopropane-1-ol |
| **211** | 1-[2-[8-(Dimethyl-amino)-8-thiophen-2-yl-3-azaspiro[4.5]decane-3-yl]-ethyl]-cyclopropane-1-carbonitrile |
| **212** | 1-(8-Dimethylamino-8-thiophen-2-yl-3-azaspiro[4.5]decane-3-yl)-2-(oxetan-3-yl)-ethanone |
| **213** | 1-[8-(Dimethyl-amino)-8-thiophen-2-yl-3-azaspiro[4.5]decane-3-yl]-3-ethyl-pentan-3-ol |
| **214** | 1-[8-(Dimethyl-amino)-8-(thiophen-2-yl-methyl)-3-azaspiro[4.5]decane--3-yl] butane-1-one |
| **215; 216** | 8-(Dimethyl-amino)-3-[(1-hydroxy-cyclobutyl)-methyl]-8-thiophen-2-yl-3-azaspiro[4.5]decane-2-one |
| **217; 218** | 8-(Dimethyl-amino)-3-[2-(1-methoxy-cyclobutyl)-ethyl]-8-thiophen-2-yl-3-azaspiro[4.5]decane-2-one |
| **219; 220** | 8-(Dimethyl-amino)-3-(3-methoxy-3-methyl-butyl)-8-thiophen-2-yl-3-azaspiro[4.5]decane-2-one |
| **221; 222** | 8-(Dimethyl-amino)-3-(3-hydroxy-3-methyl-butyl)-8-thiophen-2-yl-3-azaspiro[4.5]decane-2-one |
| **223** | 1-[8-(Dimethyl-amino)-8-thiophen-2-yl-3-azaspiro[4.5]decan-3-yl]-2-hydroxy-2-methyl-propane-1-one |
| **224; 225** | 8-(Dimethyl-amino)-3-(2-hydroxy-2-methyl-propyl)-8-thiophen-2-yl-3-azaspiro[4.5]decane-2-one |
| **226** | 4-[8-(Dimethyl-amino)-8-thiophen-2-yl-3-azaspiro[4.5]decane-3-yl]-2-methyl-butane-2-ol |
| **227** | 1-[8-(Dimethyl-amino)-8-thiophen-2-yl-3-azaspiro[4.5]decane-3-yl]-2-methyl-propane-2-ol |
| **228** | 1-[8-(Dimethyl-amino)-8-pheny-3-azaspiro[4.5]decane-3-yl]-2-hydroxy-2-methyl-propane-1-one |
| **229** | 1-[8-(Dimethyl-amino)-8-phenyl-3-azaspiro[4.5]decane-3-yl]-2-methyl-propan-2-ol |
| **230; 231** | 4-[8-(Dimethyl-amino)-2-oxo-8-phenyl-3-azaspiro[4.5]decane-3-yl]-2,2-dimethyl-butyronitrile |
| **232** | 4-[8-(Dimethyl-amino)-8-phenyl-3-azaspiro[4.5]decane-3-yl]-2,2-dimethyl-4-oxo-butyronitrile |
| **233** | 4-[8-(Dimethyl-amino)-8-thiophen-2-yl-3-azaspiro[4.5]decane-3-yl]-2,2-dimethyl-4-oxo-butyronitrile |
| **234** | 8-Cyclopentyl-3-(2-cyclopropyl-ethyl)-8-(dimethyl-amino)-3-azaspiro[4.5]decane-2-one |
| **235** | 1-[8-(Dimethyl-amino)-8-thiophen-2-yl-3-azaspiro[4.5]decane-3-yl]-2-(1-methoxy-cyclobutyl)-ethanone |
| **236** | 3-(2-Cyclobutyl-ethyl)-8-cyclopentyl-8-(dimethyl-amino)-3-azaspiro[4.5]decane-2-one |
| **237** | 1-[8-(Dimethyl-amino)-8-phenyl-3-azaspiro[4.5]decane-3-yl]-2-(1-methoxy-cyclobutyl)-ethanone |
| **238** | [3-[2-(1-Methoxy-cyclobutyl)-ethyl]-8-thiophen-2-yl-3-azaspiro[4.5]decane-8-yl]-dimethyl-amine |
| **239** | [3-[2-(1-Methoxy-cyclobutyl)-ethyl]-8-phenyl-3-azaspiro[4.5]decane-8-yl]-dimethyl-amine |
| **240** | 4-[8-(Dimethyl-amino]-2-oxo-8-thiophen-2-yl-3-azaspiro[4.5]decane-3-yl]-2,2-dimethyl-butyronitrile |
| **241; 242** | 8-(Dimethyl-amino)-3-[2-(oxetan-3-yl)-ethyl]-8-thiophen-2-yl-3-azaspiro[4.5]decane-2-one |
| **243** | 8-Cyclopentyl-3-(2-cyclopropyl-ethyl)-8-(dimethyl-amino)-3-azaspiro[4.5]decane-2-one |
| **244** | 8-Cyclopentyl-8-(dimethyl-amino)-3-(oxetan-3-yl-methyl)-3-azaspiro[4.5]decane-2-one |
| **245** | 1-[2-[8-Cyclopentyl-8-(dimethyl-amino)-2-oxo-3-azaspiro[4.5]decane-3-yl]-ethyl]-cyclopropane-1-carbonitrile |
| **246** | 8-Cyclopentyl-8-(dimethyl-amino)-3-[2-(oxetan-3-yl)-ethyl]-3-azaspiro[4.5]decane-2-one |
| **247** | 1-(8-Cyclopentyl-8-dimethylamino-3-azaspiro[4.5]decane-3-yl)-2-(1-methoxy-cyclobutyl)-ethanone |
| **248** | 4-[8-(Dimethyl-amino)-8-thiophen-2-yl-3-azaspiro[4.5]decane-3-yl]-2,2-dimethyl-butyronitrile |
| **249** | 4-[8-(Dimethyl-amino)-8-phenyl-3-azaspiro[4.5]decane-3-yl]-2,2-dimethyl-butyronitrile |
| **250** | 4-[8-(Dimethyl-amino)-2-oxo-8-thiophen-2-yl-3-azaspiro[4.5]decane-3-yl]-2,2-dimethyl-butyronitrile |
| **251; 252** | 8-(Dimethyl-amino)-3-(oxetan-3-yl-methyl)-8-phenyl-3-azaspiro[4.5]decane -2-one |
| **253** | 1-[2-[8-(Dimethyl-amino)-8-thiophen-2-yl-3-azaspiro[4.5]decane-3-yl-ethyl]-cyclopropane-1-ol |
| **254; 255** | 8-(Dimethyl-amino)-3-[2-(oxetan-3-yl)-ethyl]-8-phenyl-3-azaspiro[4.5]decane-2-one |
| **256** | 1-[2-[8-Cyclopentyl-8-(dimethyl-amino)-2-oxo-3-azaspiro[4.5]decane-3-yl]-ethyl]-cyclobutane-1-carbonitrile |
| **257** | [8-Cyclopentyl-3-[2-(1-methoxy-cyclobutyl)-ethyl]-3-azaspiro[4.5]decane-8-yl]-dimethyl-amine |
| **258** | 4-(8-Cyclopentyl-8-dimethylamino-3-azaspiro[4.5]decane-3-yl)-2,2-dimethyl-butyronitrile |
| **259** | 4-(8-Cyclopentyl-8-dimethylamino-3-azaspiro[4.5]decane-3-yl)-2,2-dimethyl-4-oxo-butyronitrile |
| **260; 261** | 8-(Dimethyl-amino)-8-phenyl-3-(tetrahydro-furan-3-yl-methyl)-3-azaspiro[4.5]decane-2-one |
| **262; 263** | 8-(Dimethyl-amino)-8-phenyl-3-(2-tetrahydro-furan-2-yl-ethyl)-3-azaspiro[4.5]decane-2-one |
| **264** | 8-(Dimethyl-amino)-8-phenyl-3-(2-tetrahydro-furan-3-yl-ethyl)-3-azaspiro[4.5]decane-2-one |
| **265** | [8-Cyclopentyl-3-(tetrahydro-pyran-4-yl-methyl)-3-azaspiro[4.5]decane-8-yl]-dimethyl-amine |
| **266** | [8-Cyclopentyl-3-(3-methyl-butyl)-3-azaspiro[4.5]decane-8-yl]dimethyl-amine |
| **267** | 1-[2-(8-Cyclopentyl-8-dimethylamino-3-azaspiro[4.5]-decane-3-yl)-ethyl]-cyclopropane-1-carbonitrile |
| **268** | 1-[2-(8-Cyclopentyl-8-dimethylamino-3-azaspiro[4.5]decane-3-yl)-ethyl]-cyclobutane-1-carbonitrile |
| **269; 270** | 8-(Dimethyl-amino)-8-phenyl-3-(tetrahydro-pyran-4-yl-methyl)-3-azaspiro[4.5]decane-2-one |
| **271** | 8-(Dimethyl-amino)-8-phenyl-3-(2-tetrahydro-furan-3-yl-ethyl)-3-azaspiro[4.5]decane-2-one |
| **272** | 4-[8-(Dimethyl-amino)-8-phenyl-3-azaspiro[4.5]decane-3-yl]-2-methyl-butane-2-ol |
| **273** | [8-Cyclopentyl-3-(2-methyl-propyl)-3-azaspiro[4.5]decane-8-yl]-dimethyl-amine |
| **274** | 3-(Cyclobutyl-methyl)-8-cyclopentyl-8-(dimethyl-amino)-3-azaspiro[4.5]decane-2-one |
| **275** | 3-(2-Cyclopropyl-ethyl)-8-methylamino-8-phenyl-3-azaspiro[4.5]decane-2-one |
| **276** | 8-Cyclopentyl-3-(cyclopropyl-methyl)-8-(dimethyl-amino)-3-azaspiro[4.5]decane-2-one |
| **277** | 3-(Cyclohexyl-methyl)-8-cyclopentyl-8-(dimethyl-amino)-3-azaspiro[4.5]decane-2-one |
| **278** | 8-Cyclopentyl-3-(cyclopentyl-methyl)-8-(dimethyl-amino)-3-azaspiro[4.5]decane-2-one |
| **279; 280** | 8-(Dimethyl-amino)-3-[[(3S)-tetrahydro-furan-3-yl]-methyl]-8-thiophen-2-yl-3-azaspiro[4.5]decane-2-one |
| **281** | 3-Butyl-8-cyclopentyl-8-(dimethyl-amino)-3-azaspiro[4.5]decane-2-one |
| **282** | 8-Cyclopentyl-8-(dimethyl-amino)-3-methyl-3-azaspiro[4.5]decane-2-one |
| **283** | Dimethyl-[3-[2-(oxetan-3-yl)-ethyl]-8-phenyl-3-azaspiro[4.5]decane-8-yl]-amine |
| **284** | Dimethyl-[8-phenyl-3-(tetrahydro-furan-3-yl-methyl)-3-azaspiro[4.5]decane-8-yl]-amine |
| **285** | 1-[2-[8-(Dimethyl-amino)-8-phenyl-3-azaspiro[4.5]decane-3-yl]-ethyl]-cyclopropane-1-ol |
| **286** | 3,8-Dicyclopentyl-8-(dimethyl-amino)-3-azaspiro[4.5]decane-2-one |
| **287** | 8-Cyclopentyl-8-(dimethyl-amino)-3-[2-(1-methoxy-cyclobutyl)-ethyl]-3-azaspiro[4.5]decane-2-one |
| **288; 289** | 3-[8-(Dimethyl-amino)-2-oxo-8-thiophen-2-yl-3-azaspiro[4.5]decane-3-yl]-2,2-dimethyl-propionitrile |
| **290** | 3-(2-Cyclopropyl-ethyl)-8-methylamino-8-thiophen-2-yl-3-azaspiro[4.5]decane-2-one |
| **291** | (8-Cyclopentyl-8-dimethylamino-3-azaspiro[4.5]decane-3-yl)-(oxetan-3-yl)-methanone |
| **292** | [8-Cyclopentyl-3-(3-methoxy-3-methyl-butyl)-3-azaspiro[4.5]decane-8-yl]-dimethyl-amine |
| **293; 294** | 8-Cyclopentyl-8-(dimethyl-amino)-3-[[(3S)-tetrahydro-furan-3-yl]-methyl]-3-azaspiro[4.5]decane-2-one |
| **295** | Dimethyl-[3-[2-(oxetan-3-yl)-ethyl]-8-thiophen-2-yl-3-azaspiro[4.5]decane-8-yl]-amine |
| **296** | Dimethyl-[3-(tetrahydro-furan-3-yl-methyl)-8-thiophen-2-yl-3-azaspiro[4.5]decane-8-yl]-amine |
| **297** | 1-(8-Cyclopentyl-8-dimethylamino-3-azaspiro[4.5]decane-3-yl)-2-methoxy-2-methyl-propane-1-one |
| **298** | [8-Cyclopentyl-3-(2-methoxy-2-methyl-propyl)-3-azaspiro[4.5]decane-8-yl]-dimethyl-amine |
| **299** | [3-(3-Methoxy-3-methyl-butyl)-8-thiophen-2-yl-3-azaspiro[4.5]decane-8-yl]-dimethyl-amine |
| **300** | [3-(3-Methoxy-3-methyl-butyl)-8-phenyl-3-azaspiro[4.5]decane-8-yl]-dimethyl-amine |
| **301** | 3-(8-Cyclopentyl-8-dimethylamino-3-azaspiro[4.5]decane-3-yl)-2-methyl-propane-1-ol |
| **302** | [8-Cyclopentyl-3-(oxetan-3-yl-methyl)-3-azaspiro[4.5]decane-8-yl]-dimethyl-amine |
| **303** | Dimethyl-[8-phenyl-3-(tetrahydro-pyran-4-yl-methyl)-3-azaspiro[4.5]decane-8-yl]-amine |
| **304** | Dimethyl-[3-(tetrahydro-pyran-4-yl-methyl)-8-thiophen-2-yl-3-azaspiro[4.5]decane-8-yl]-amine |
| **305** | Dimethyl-[8-phenyl-3-(2-tetrahydro-furan-2-yl-ethyl)-3-azaspiro[4.5]decane-8-yl]-amine |
| **306** | Dimethyl-[3-(2-tetrahydro-furan-2-yl-ethyl)-8-thiophen-2-yl-3-azaspiro[4.5]decane-8-yl]-amine |
| **307; 308** | 3-(2-Cyclobutyl-ethyl)-8-methylamino-8-thiophen-2-yl-3-azaspiro[4.5]decane-2-one |
| **309** | Dimethyl-[3-(2-tetrahydro-furan-3-yl-ethyl)-8-thiophen-2-yl-3-azaspiro[4.5]decane-8-yl]-amine |
| **310** | 3-(8-Dimethylamino-2-oxo-8-phenyl-3-azaspiro[4.5]decane-3-yl)-2,2-dimethyl-propionitrile |
| **311** | 8-Dimethylamino-3-[(1-methoxy-cyclobutyl)-methyl]-8-thiophen-2-yl-3-azaspiro[4.5]decane-2-one |
| **312** | 8-Dimethylamino-3-[(1-methoxy-cyclobutyl)-methyl]-8-phenyl-3-azaspiro[4.5]decane-2-one |
| **313** | 8-Dimethylamino-3-(2-ethoxy-ethyl)-8-thiophen-2-yl-3-azaspiro[4.5]decane-2-one |
| **314** | 1-(8-Dimethylamino-8-thiophen-2-yl-3-azaspiro[4.5]decane-3-yl)-3-methoxy-3-methyl-propane-1-one |
| **315** | 1-(8-Dimethylamino-8-thiophen-2-yl-3-azaspiro[4.5]decane-3-yl)-3-methoxy-propane-1-one |
| **316** | 3-(2-Cyclopropyl-ethyl-8-methylamino-8-thiophen-2-yl-3-azaspiro[4.5]decane-2-one |
| **317** | 8-(Dimethyl-amino)-3-(3-methoxy-butyl)-8-thiophen-2-yl-3-azaspiro[4.5]decane-2-one |
| **318** | 8-(Dimethyl-amino)-3-(2-methoxy-propyl)-8-thiophen-2-yl-3-azaspiro[4.5]decane-2-one |
| **319** | 3-[8-(Dimethyl-amino)-2-oxo-8-phenyl-3-azaspiro[4.5]decane-3-yl]-2,2-dimethyl-propionitrile |
| **320** | 1-[8-(Dimethyl-amino)-8-phenyl-3-azaspiro[4.5]decane-3-yl]-3-methoxy-3-methyl-butane-1-one |
| **321** | 8-(Dimethyl-amino)-3-[(1-methoxy-cyclobutyl)-methyl]-8-thiophen-2-yl-3-azaspiro[4.5]decane -2-one |
| **322** | 8-(Dimethyl-amino)-3-[(1-methoxy-cyclobutyl)-methyl]-8-phenyl-3-azaspiro[4.5]decane-2-one |
| **323** | 8-(Dimethyl-amino)-3-(2-methoxy-propyl)-8-phenyl-3-azaspiro[4.5]decane-2-one |
| **324** | 8-(Dimethyl-amino)-3-(3-methoxy-butyl)-8-phenyl-3-azaspiro[4.5]decane-2-one |
| **32S** | 8-(Dimethyl-amino)-3-(2-methoxy-ethyl)-8-phenyl-3-azaspiro[4.5]decane-2-one |
| **326** | 8-(Dimethyl-amino)-3-(2-ethoxy-ethyl)-8-phenyl-3-azaspiro[4.5]decane-2-one |
| **327** | 1-[8-(Dimethyl-amino)-8-thiophen-2-yl-3-azaspiro[4.5]decane-3-yl]-3-methoxy-butane-1-one |
| **328** | 1-[8-(Dimethyl-amino)-8-phenyl-3-azaspiro[4.5]decane-3-yl]-3-methoxy-butane-1-one |
| **329** | 1-[8-(Dimethyl-amino)-8-phenyl-3-azaspiro[4.5]decane-3-yl]-3-methoxy-propane-1-one |
| **330** | 3-(2-Cyclopropyl-ethyl)-8-methylamino-8-phenyl-3-azaspiro[4.5]decane-2-one |
| **331** | 3-(2-Cyclobutyl-ethyl)-8-methylamino-8-phenyl-3-azaspiro[4.5]decane-2-one |
| **332** | 8-(Dimethyl-amino)-3-(2-methoxy-2-methyl-propyl)-8-thiophen-2-yl-3-azaspiro[4.5]decane-2-one |
| **333** | 2-Cyclobutyl-1-(8-methylamino-8-thiophen-2-yl-3-azaspiro[4.5]decane-3-yl)-ethanone |
| **334** | 8-(Dimethyl-amino)-3-(2-ethoxy-propyl)-8-thiophen-2-yl-3-azaspiro[4.5]decane-2-one |
| **335** | 8-(Dimethyl-amino)-3-(2-ethoxy-propyl)-8-phenyl-3-azaspiro[4.5]decane-2-one |
| **336** | 8-(Dimethyl-amino)-3-(3-methoxy-3-methyl-butyl)-8-(5-methyl-thiophen-2-yl)-3-azaspiro[4.5]decane-2-one |
| **337** | 8-(5-Chlor-thiophen-2-yl)-8-(dimethyl-amino)-3-(3-methoxy-3-methyl-butyl)-3-azaspiro[4.5]decane-2-one |
| **338** | 1-[8-(Dimethyl-amino)-8-phenyl-3-azaspiro[4.5]decane -3-yl]-3-ethoxy-butane-1-one |
| **339** | 1-[8-(Dimethyl-amino)-8-thiophen-2-yl-3-azaspiro[4.5]decane-3-yl]-3-ethoxy-butane-1-one |
| **340** | 8-(Dimethyl-amino)-3-(2-methoxy-2-methylpropyl)-8-phenyl-3-azaspiro[4.5]decane-2-one |
| **341** | 3-[2-(1-Methoxy-cyclobutyl)-ethyl]-8-methylamino-8-thiophen-2-yl-3-azaspiro[4.5]decane-2-one |
| **342** | Cyclobutyl-[8-(dimethyl-amino)-8-phenyl-3-azaspiro[4.5]decane-3-yl]-methanone |
| **343** | 1-[8-(Dimethyl-amino)-8-phenyl-3-azaspiro[4.5]decane-3-yl]-2-methyl-propane-1-one |
| **344** | 1-[[8-(Dimethyl-amino)-2-oxo-8-thiophen-2-yl-3-azaspiro[4.5]decane-3-yl]-methyl]-cyclopropane-1-carbonitrile |
| **345** | [8-(Dimethyl-amino)-8-phenyl-3-azaspiro[4.5]decane-3-yl]-(1-methoxy-cyclopropyl)-methanone |
| **346** | [3-(2-Cyclobutyl-ethyl)-8-thiophen-2-yl-3-azaspiro[4.5]decane-8-yl]-methyl-amine |
| **347** | 8-(Dimethyl-amino)-3-(2-tetrahydro-pyran-4-yl-ethyl)-8-thiophen-2-yl-3-azaspiro[4.5]decane-2-one |
| **348** | 8-(Dimethyl-amino)-8-phenyl-3-(2-tetrahydro-pyran-4-yl-ethyl)-3-azaspiro[4.5]decane-2-one |
| **349** | 1-[[8-(Dimethyl-amino)-2-oxo-8-phenyl-3-azaspiro[4.5]decane-3-yl]-methyl]-cyclopropane-1-carbonitrile |
| **350** | 1-[8-(Dimethyl-amino)-8-phenyl-3-azaspiro[4.5]decane-3-yl]-2-tetrahydro-pyran-4-yl-ethanone |
| **351** | 1-[8-(Dimethyl-amino)-8-thiophen-2-yl-3-azaspiro[4.5]decane-3-yl]-2-tetrahydro-pyran-4-yl-ethanone |
| **352** | 1-[[8-(Dimethyl-amino)-8-phenyl-3-azaspiro[4.5]decane-3-yl]-methyl]-cyclopropane-1-carbonitrile |
| **353** | 1-[[8-(Dimethyl-amino)-2-oxo-8-thiophen-2-yl-3-azaspiro[4.5]decane-3-yl]-methyl]-cyclobutane-1-carbonitrile |
| **354** | 1-[8-(Dimethyl-amino)-8-phenyl-3-azaspiro[4.5]decane-3-yl]-3-(1-methoxy-cyclobutyl)-propane-1-one |
| **355** | 1-[8-(Dimethyl-amino)-8-thiophen-2-yl-3-azaspiro[4.5]decane-3-yl]-3-(1-methoxy-cyclobutyl)-propane-1-one |
| **356** | [8-(Dimethyl-amino)-8-thiophen-2-yl-3-azaspiro[4.5]decane-3-yl]-(1-methoxy-cyclopropyl)-methanone |
| **357** | 1-[[8-(Dimethyl-amino)-2-oxo-8-phenyl-3-azaspiro[4.5]decane-3-yl]-methyl]-cyclobutane-1-carbonitrile |
| **358** | [3-[(1-Methoxy-cyctopropyl)-methyl]-8-thiophen-2-yl-3-azaspiro[4.5]decane-8-yl]-dimethyl-amine |
| **359** | 1-[[8-(Dimethyl-amino)-8-phenyl-3-azaspiro[4.5]decane-3-yl]-methyl]-cyclobutane-1-carbonitrile |
| **360** | 1-[[8-(Dimethyl-amino)-8-thiophen-2-yl-3-azaspiro[4.5]decane-3-yl]-methyl]-cyclopropane-1-carbonitrile |
| **361** | 1-[[8-(Dimethyl-amino)-8-thiophen-2-yl-3-azaspiro[4.5]decane-3-yl]-methyl]-cyclcbutane-1-carbonitrile |
| **362** | 1-[3-[8-(Dimethyl-amino)-8-phenyl-3-azaspiro[4.5]decane-3-yl]-3-oxo-propyl]-cyclobutane-1-carbonitrile |
| **363** | 1-[3-[8-(Dimethyl-amino)-8-thiophen-2-yl-3-azaspiro[4,5]decade-3-yl]-3-oxo-propyl]-cyclobutane-1-carbonitrile |
| **364** | 1-(8-Butyl-8-dimethylamino-3-azaspiro[4.5]decane-3-yl)-2-cyclopropyl-ethanone |
| **365** | Cyclopropyl-(8-dimethylamino-8-phenyl-3-azaspiro[4.5]decane-3-yl)-methanone |
| **366** | 1-(8-Dimethylamino-8-phenyl-3-azaspiro[4.5]decane-3-yl)-3-methyl-butane-1-one |
| **367** | 1-(8-Butyl-8-dimethylamino-3-azaspiro[4.5]decane-3-yl)-3-cyclopropyl-propane-1-one |
| **368** | 1-(8-Butyl-8-dimethylamino-3-azaspiro[4.5]decane-3-yl)-4-methoxy-4-methyl-pentan-1-one |
| **369** | [3-[(1-Methoxy-cyclopropyl)-methyl]-8-phenyl-3-azaspiro[4.5]decane-8-yl]-dimethyl-amine |
| **370** | 8-(Dimethyl-amino)-3-(3-methyl-butyl)-8-thiophen-2-yl-3-azaspiro[4.5]decane-2-one |
| **371** | 1-[3-[8-(Dimethyl-amino)-8-phenyl-3-azaspiro[4.5]decane-3-yl]-3-oxo-propyl]-cyclopropane-1-carbonitrile |
| **372** | 1-[3-[8-(Dimethyl-amino)-8-thiophen-2-yl-3-azaspiro[4.5]decane-3-yl]-3-oxo-propyl]-cyclopropane-1-carbonitrile |
| **373** | 1-(8-Dimethylamino-8-thiophen-2-yl-3-azaspiro[4.5]decane-3-yl)-2-(1-methoxy-cyclopropyl)-ethanone |
| **374** | 1-(8-Dimethylamino-8-phenyl-3-azaspiro[4.5]decane-3-yl)-2-(1-methoxy-cyclopropyl)-ethanone |
| **375** | 1-(8-Butyl-8-dimethylamino-2-azaspiro[4.5]decane-2-yl)-2-cyclobutyl-ethanone |
| **376** | 8-Dimethylamino-3-(3-methyl-butyl)-8-phenyl-3-azaspiro[4.5]decane-2-one |
| **377** | 8-Dimethylamino-3-(2-methyl-propyl)-8-thiophen-2-yl-3-azaspiro[4.5]decane-2-one |
| **378** | 8-Dimethylamino-3-(2-methyl-propyl)-8-phenyl-3-azaspiro[4.5]decane-2-one |
| **379** | (8-Dimethylamino-8-phenyl-2-azaspiro[4.5]decance-2-yl)-[1-(methoxymethyl)-cydopropyl]-methanone |
| **380** | 1-(8-Dimethylamino-8-phenyl-2-azaspiro[4.5]decane-2-yl)-3-methoxy-2,2-dimethyl-propane-1-one |
| **381** | (8-Dimethylamino-8-phenyl-2-azaspiro[4.5]decane 2-yl)-[1-(methoxymethyl)-cyclobutyl]-methanone |
| **382** | (8-Dimethylamino-8-thiophen-2-yl-3-azaspiro[4.5]decane-3-yl)-(1-methoxy-cyclobutyl)-methanone |
| **383** | (8-Dimethylamino-8-thiophen-2-yl-3-azaspiro[4.5]decane-3-yl)-[1-(methoxymethyl)-cyclopropyl]-methanone |
| **384** | 1-(8-Dimethylamino-8-thiophen-2-yl-3-azaspiro[4.5]decane-3-yl)-3-methoxy-2.2-dimethyl-propane-1-one |
| **385** | (8-Dimethylamino-8-thiophen-2-yl-3-azaspiro[4.5]decane-3-yl)-[1-(methoxymethyl)-cyclobutyl]-methanone |
| **386** | 8-Dimethylamino-3-[2-(1-methoxy-cyclopropyl)-ethyl]-8-thiopben-2-yl-3-azaspiro[4.5]decane-2-one |
| **387** | 8-Dimethylamino-3-[2-(1-methoxy-cyclopropyl)-ethyl]-8-phenyl-3-azaspiro[4.5]decane-2-one |
| **388** | 1-(8-Butyl-8-dimethylamino-3-azaspiro[4.5]decane-3-yl)-2-(1-methoxy-cyclobutyl)-ethanone |
| **389** | 1-(8-Butyl-8-dimethylamino-3-azaspiro[4.5]decane-3-yl)-2-tetrahydro-furan-3-yl-ethanone |
| **390** | (8-Dimethylamino-8-ohenyl-3-azaspiro[4.5]decane-3-yl)-(1-methoxy-cyclobutyl)-methanone |
| **391** | [8-Dimethylamino-8-(5-methyl-thiophen-2-yl)-3-azaspiro[4.5]decane-3-yl]-[1-(methoxymethyl)-cyclopropyl]-methanone |
| **392** | [8-Dimethylamino-8-(5-methyl-thiophen-2-yl)-3-azaspiro[4.5]decane-3-yl]-[1-(methoxymethyl)-cyclobutyl]-methanone |
| **393** | 1-[8-Dimethylamino-8-(5-methyl-thiophen-2-yl)3-azaspiro[4.5]decane-3-yl]-3-methoxy-2,2-dimethyl-propane-1-one |
| **394** | 5-(8-Dimethylamino-8-phenyl-3-azaspiro[4.5]decane-3-yl)-2,2-dimethyl-5-oxo-*pentannitril* |
| **395** | 5-(8-Dimethylamino-8-thiophen-2-yl-3-azaspiro[4.5]decane-3-yl)-2,2-dimethyl-5-oxo-*pentannitril* |
| **396** | [8-(5-Chlor-thiophen-2-yl)-8-dimethylamino-3-azaspiro[4.5]decane-3-yl]-[1-(methoxymethyl)-cyclopropyl]-methanone |
| **397** | [8-(5-Chlor-thiophen-2-yl)-8-dimethylamino-3-azaspiro[4.5]decane-3-yl]-[1-(methoxymethyl)-cyclobutyl]-methanone |
| **398** | 1-[8-(5-Chlor-thiophen-2-yl)-8-dimethylamino-3-azaspiro[4.5]decane-3-yl]-3-methoxy-2,2-dimethyl-propane-1-one |
| **399;400** | 8-Butyl-8-(dimethyl-amino)-3-(2-tetrahydro-furan-3-yl-ethyl)-3-azaspiro[4.5]decane-2-one |
| **401; 402** | 1-[2-[8-Butyl-8-(dimethyl-amino)-2-oxo-3-azaspiro[4.5]decane-3-yl]-ethyl]-cyclopropane-1-carbonitrile |
| **403; 404** | 1-[2-[8-Butyl-8-(dimethyl-amino)-2-oxo-3-aspiro[4.5]decane-3-yl]-ethyl]-cyclobutane-1-carbonitrile |
| **405** | 1-[8-(Dimethyl-amino)-8-phenyl-3-azaspiro[4.5]decane-3-yl]-3-(1-methoxy-cyctopropyl)-propane-1-one |
| **406** | 8-Butyl-8-(dimethyl-amino)-3-[2-(1-methoxy-cyclobutyl)-ethyl]-3-azaspiro[4.5]decane-2-one |
| **407** | [8-(Dimethyl-amino)-8-(5-fluor-thiophen-2-yl)-3-azaspiro[4.5]decane-3-yl]-[1-(methoxymethyl)-cyclobutyl]-methanone |
| **408** | 2-Cyclopropyl-1-[8-(dimethyl-amino)-8-phenyl-3-azaspiro[4.5]decane-3-yl]-ethanone |
| **409; 410** | 8-Butyl-8-(dimethyl-amino)-3-[2-(oxetan-3-yl)-ethyl]-3.azaspiro[4.5]decane -2-one |
| **411** | [8-(Dimethyl-amino)-8-(5-fluor-thiophen-2-yl)-3-azaspiro[4.5]decane-3-yl]-(methoxymethyl)-cyclopropyl]-methanone |
| **412** | 1-[8-(Dimethyl-amino)-8-thiophen-2-yl-3-azaspiro[4.5]decane-3-yl]-3-(1-methoxy-cyclopropyl)-propane-1-one |
| **413** | 8-Butyl-8-(dimethyl-amino)-3-[2-(1-methoxy-cyclobutyl)-ethyl]-3-azaspiro[4.5]decane-2-one |
| **414** | 2-Cyclobutyl-1-[8-(dimethyl-amino)-8-phenyl-3-azaspiro[4.5]decane-3-yl]-ethanone |
| **415** | 1-[8-(Dimethy-amino)-8-thiophen-2-yl-3-azaspiro[4.5]decane-3-yl]-3-methoxy-propane-1-one |
| **416** | 1-[8-(Dimethyl-amino)-6-thiophen-2-yl-3-azaspiro[4.5]decane-3-yl]-3-methyl-butane-1-one |
| **417** | 8-(Dimethyl-amino)-8-(5-fluor-thiophen-2-yl)-3-(3-methoxy-3-methyl-butyl)-3-azaspiro[4.5]decane-2-one |
| **418** | 1-[8-Dimethylamino-8-(5-fluor-thiophen-2-yl)-3-azaspiro[4.5]decane-3-yl]-methoxy-3-methyl-butane-1-one |
| **419** | 3-Methoxy-1-(8-methylamino-8-thiophen-2-yl-3-azaspiro[4.5]decane-3-yl]-propane-1-one |
| **420** | 2-Cyclobutyl-1-(8-methylamino-8-phenyl-3-azaspiro[4.5]decane-3-yl)-ethanone |
| **421** | 8-(Dimethyl-amino)-8-(5-fluor-thiophen-2-yl)-3-[2-(1-methoxy-cyclopropyl)-ethyl]-3-azaspiro[4.5]decane-2-one |
| **422** | 2-Cyclopropyl-1-(8-methylamino-8-phenyl-3-azaspiro[4.5]decane-3-yl)-ethanone |
| **423** | 3-Methyl-1-(8-methylamino-8-thiophen-2-yl-3-azaspiro[4.5]decan-3-yl)-butane-1-one |
| **424** | 8-Cyclopentyl-8-dimethylamino-2-azaspiro[4.5]decane |
| **425** | 8-(5-Chlor-2-thiophen-2-yl)-2-aza-spiro[4.5]dec-8-yl]-dimethylamine |
| **426** | [8-(5-Fluor-thiophen-2-yl)-2-aza-spiro[4.5]dec-8-yl]-dimethylamine |
| **427; 428** | 8-(Azetidin-1-yl)-8-(2-thienyl)-3-azaspiro[4.5]decane |
| **429, 430** | 8-Azetidin-1-yl-8-phenyl-2-azaspiro[4.5]decane |
| **431** | 8-Dimethylamino-8-phenyl-2-azaspiro[4.5]decane-3-one |
| **432; 433** | 8-Butyl-8-dimethylamino-2-azaspiro[4.5]decane-3-one |
in the form of an individual stereoisomer or mixture thereof, the free compounds and/or their physiologically acceptable salts and/or solvates.

12. A medicinal product containing at least one compound according to one of claims 1 to 11 in the form of an individual stereoisomer or mixture thereof, the free compounds and/or their physiologically acceptable salts and/or solvates, and optionally suitable additives and/or auxiliary substances and/or optionally further active substance.

13. A compound according to one of claims 1 to 11 in the form of an individual stereoisomer or mixture thereof, the free compound and/or its physiologically acceptable salts and/or solvates for use in the treatment of pain.

14. A compound according to one of claims 1 to 1 in the form of an individual stereoisomer or mixture thereof, the free compounds and/or their physiologically acceptable salts and/or solvates for use in the treatment of anxiety, of stress and syndromes associated with stress, depressions, epilepsy, Alzheimer's disease, senile dementia, general cognitive dysfunctions, learning and memory disorders (as a nootropic), withdrawal symptoms, alcohol and/or drug and/or medical substance abuse and/or dependency, sexual dysfunctions, cardiovascular diseases, hypotension, hypertension, tinnitus, pruritus, migraines, impaired hearing, lack of intestinal motility, impaired food intake, anorexia, obesity, locomotor disorders, diarrhea, cachexia, urinary incontinence, or as a muscle relaxant, anticonvulsant or anesthetic, or for coadministration in treatment with an opioid analgesic or with an anesthetic, for diuresis or antinatriuresis, anxiolysis, for modulation of motor activity, for modulation of neurotransmitter release and treatment of neurodegenerative diseases associated therewith, for treatment of withdrawal symptoms and/or for reducing the addiction potential of opioids.

## Revendications

1. Composé de formule générale (1), 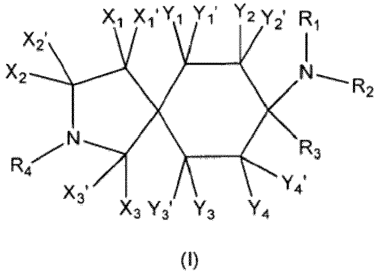 dans laquelle
Y₁, Y₁', Y₂, Y₂', Y₃, Y₃', Y₄ et Y₄' sont choisis respectivement indépendamment les uns des autres dans le groupe constitué de-H, -F, -Cl, -Br, -I, -CN, -NO₂, -CHO, -R₀, -C(=O)R₀, -C(=O)H,-C(=O)-OH, -C(=O)OR₀, -C(=O)NH₂, -C(=O)NHR₀, -C(=O)N(R₀)₂, -OH,-ORS₀, -OC(=O)H, -OC(=O)R₀, -OC(=O)OR₀, -OC(=O)NHR₀, OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂,-NHC(=O)NHR₀ et -NHC(=O)N(R₀)₂ ; ou Y₁ et Y₁', ou Y₂ et Y₂', ou Y₃ et Y₃' ou Y₄ et Y₄' représentent conjointement =O ;
X₁, X₁', X₂, X₂', X₃ et X₃' représentent respectivement indépendamment les uns des autres -H, -F, -Cl, -Br, -I, -NO₂,-CF₃, -OR₅, -SR₅, -SO₂R₅, -S(=O)₂OR₅, -CN, -COOR₅, -CONR₅, -NR₆R₇ ou -R₀ ; ou X₁ et X₁' , ou X₂ et X₂', ou X₃ et X₃' représentent conjointement =O ;
ou X₁ et X₂, ou X₂ et X₃ représentent conjointement -(CH₂)₂₋₆-, dans lequel des atomes d'hydrogène individuels peuvent être remplacés également par -F, -Cl, -Br, -I, -NO₂, -CF₃, -OR₅, -CN ou -un groupe aliphatique en C₁ à C₆ ;
ou X₁ et X₁' ou X₂ et X₂' ou X₃ et X₃' représentent conjointement un groupe cycloaliphatique en C₃ à C₆, dans lequel des atomes d'hydrogène individuels peuvent également être remplacés par-F, -Cl, -Br, -I, -NO₂, -CF₃, -OR₅, -CN ou -un groupe aliphatique en C₁ à C₆ ;
R₀ représente respectivement indépendamment un groupe aliphatique en C₁ à C₈, cycloaliphatique en C₃ à C₁₂, -aryle,-hétéroaryle, -aliphatique en C₁ à C₈-cycloaliphatique en C₃ à C₁₂, -aliphatique en C₁ à C₈-aryle, -aliphatique en C₁ à C₈-hétéroaryle, -cycloaliphatique en C₃ à C₈-aliphatique en C₁ à C₈,-cycloaliphatique en C₃ à C₈-aryle ou -cycloaliphatique en C₃ à C₈-hétéroaryle ;
R₁ et R₂ représentent indépendamment l'un de l'autre -H ou -R₀ ; ou R₁ et R₂ représentent conjointement -CH₂CH₂OCH₂CH₂-,-CH₂CH₂NR₈CH₂CH₂- ou - (CH₂)₃₋₆- ;
R₃ représente -R₀ ;
R₄ représente H ou -Z-R₁₁,
dans lequel
Z peut être absent ou peut être -C(=O)-, -S(=O)- ou -S(=O)₂-, et R₁₁ représente un groupe -alkyle en C₁ à C₆, -cycloalkyle en C₃ à C₆ ou -alkyle en C₁ à C₃-cycloalkyle en C₃ à C₆, dans lequel dans le groupe cycloalkyle en C₃ à C₆, un atome de carbone cyclique peut être remplacé par un atome d'oxygène et les groupes -alkyle en C₁ à C₆, -cycloalkyle en C₃ à C₆ ou -alkyle en C₁ à C₃-cycloalkyle en C₃ à C₆ peuvent être non substitués, monosubstitués ou poly-substitués, par des substituants choisis dans le groupe constitué de -F, -Cl, -Br, -I, -CN, -OH, -SH,-O-alkyle en C₁ à C₃ et -S-alkyle en C₁ à C₃, dans lequel le groupe -alkyle en C₁ à C₃ peut être substitué par un ou plusieurs substituants du groupe comprenant les substituants -F, -Cl,-Br, -I, -CN, -OH, -SH ;
R₅ représente respectivement indépendamment -H ou -R₀ ;
R₆ et R₇ représentent indépendamment l'un de l'autre -H ou R₀ ; ou R₆ et R₇ représentent conjointement -CH₂CH₂OCH₂CH₂-,-CH₂CH₂NR₁₀CH₂CH₂- ou - (CH₂)₃₋₆- ;
R₈ représente -H, -R₀ ou -C(=O)R₀ ;
R₁₀ représente -H ou un groupe -aliphatique en C₁ à C₆ ;
dans lequel
« aliphatique » est respectivement un radical hydrocarbure aliphatique ramifié ou non ramifié, saturé ou monoinsaturé ou poly-insaturé, non substitué ou monosubstitué ou polysubstitué ;
« cycloaliphatique » est respectivement un radical hydrocarbure alicyclique, monocyclique ou polycyclique saturé ou monoinsaturé ou poly-insaturé, non substitué ou monosubstitué ou polysubstitué ;
dans lequel par rapport à « aliphatique » et « cycloaliphatique », on comprend par « monosubstitué ou polysubstitué » la substitution unique ou multiple d'un ou plusieurs atomes d'hydrogène par -F, -Cl, -Br, -I, -CN, -NO₂,-CHO, =O, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀,-OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻,-NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂ ;
« aryle » représente respectivement indépendamment un système cyclique carbocyclique comportant au moins un cycle aromatique, mais sans hétéroatomes dans ce cycle, dans lequel les radicaux aryle peuvent être condensés éventuellement avec d'autres systèmes cycliques saturés, (partiellement) insaturés ou aromatiques et chaque radical aryle peut se présenter sous forme non substituée ou monosubstituée ou polysubstituée, dans lequel les substituants aryle peuvent être identiques ou différents et à chaque position quelconque et possible du groupe aryle ;
« hétéroaryle » représente un radical aromatique cyclique de 5, 6 ou 7 chaînons qui contient 1, 2, 3, 4 ou 5 hétéroatomes, dans lequel les hétéroatomes, identiques ou différents, sont des atome d'azote, d'oxygène ou de soufre, et l'hétérocycle peut être non substitué ou monosubstitué ou polysubstitué ; dans lequel dans le cas de la substitution sur l'hétérocycle, les substituants peuvent être identiques ou différents et à chaque position quelconque et possible du groupe hétéroaryle ; et dans lequel l'hétérocycle peut également faire partie d'un système bicyclique ou polycyclique ;
dans lequel par rapport à « aryle » et « hétéroaryle », on comprend par « monosubstitué ou polysubstitué » la substitution unique ou multiple d'un ou plusieurs atomes d'hydrogène du système cyclique par des substituants choisis dans le groupe constitué de -F, -Cl, -Br, -I, -CN, -NO₂, -CHO, =O, -R₀, -C(=O)R₀, -C(=O)H, -C(=O)-OH, -C(=O)OR₀, -C(=O)NH₂, -C(=O)NHR₀,-C(=O)N(R₀)₂, -OH, -O(CH₂)₁₋₂O-*,* -OR₀, -OC(=O)H, -OC(=O)R₀,-OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O-,-NHC(=O)R₀, -NHC(=O)OR₀, -NH-C(=O)NH₂, -NHC(=O)NHR₀,-NHC(=O)N(R₀)₂, -Si(R₀)₃, -PO(OR₀)₂ ; dans lequel des atomes d'azote cyclique éventuellement présents peuvent être oxydés respectivement (N-oxyde) ;
sous la forme d'un stéréo-isomère individuel ou son mélange, de composés libres et/ou leurs sels et/ou solvates physiologiquement acceptables.

2. Composé selon la revendication 1, dans lequel Y₁', Y₂', Y₃' et Y₄' représentent respectivement -H.

3. Composé selon la revendication 1 ou 2, dans lequel
R₀ représente respectivement indépendamment un groupe-aliphatique en C₁ à C₈, -cycloaliphatique en C₃ à C₁₂, -aryle,-hétéroaryle, -aliphatique en C₁ à C₈-cycloaliphatique en C₃ à C₁₂, -aliphatique en C₁ à C₈-aryle, -aliphatique en C₁ à C₈-hétéroaryle, -cycloaliphatique en C₃ à C₈-aliphatique en C₁ à C₈,-cycloaliphatique en C₃ à C₈-aryle ou -cycloaliphatique en C₃ à C₈-hétéroaryle ; dans lequel ceux-ci sont non substitués ou monosubstitués ou polysubstitués par des substituants choisis indépendamment les uns des autres dans le groupe constitué de-F, -Cl, -Br, -CN, -CH₃, -C₂H₅, -NH₂, -NO₂, -SH, -CF₃, OH, -OCH₃, -OC₂H₅ et -N(CH₃)₂.

4. Composé selon l'une des revendications 1 à 3, dans lequel
R₃ représente un groupe -aliphatique en C₁ à C₈, -aryle,-hétéroaryle, -aliphatique en C₁ à C₃-aryle, -aliphatique en C₁ à C₃-hétéroaryle ou -aliphatique en C₁ à C₃-cycloaliphatique en C₅ à C₆ ; dans lequel ceux-ci sont non substitués ou monosubsitués ou polysubstitués par des substituants choisis indépendamment dans le groupe constitué de -F, -Cl, -Br, -CN, -CH₃, -C₂H₅, -NH₂, -NO₂, -SH, -CF₃, OH, -OCH₃, -OC₂H₅ et -N(CH₃)₂ ;
et
X₁, X₁', X₂, X₂', X₃ et X₃' représentent respectivement indépendamment les uns des autres -H, -F, -Cl, -Br, -I, -NO₂,-CF₃, -OR₅, -SR₅, -SO₂R₅, -S(=O)₂OR₅, -CN, -COOR₅, -CONR₅, -NR₆R₇ ou -R₀ ; ou l'un des radicaux X₁ et X₁' représente H et l'autre représente un groupe -aliphatique en C₁ à C₈, -cycloaliphatique en C₃ à C₁₂, -aryle, -hétéroaryle, aliphatique en C₁ à C₈-cycloaliphatique en C₃ à C₁₂, -aliphatique en C₁ à C₈-aryle,-aliphatique en C₁ à C₈-hétéroaryle, -cyclaliphatique en C₃ à C₈-aliphatique en C₁ à C₈, cycloaliphatique en C₃ à C₈-aryle ou-cycloaliphatique en C₃ à C₈-hétéroaryle ; dans lequel ceux-ci sont non substitués ou monosubstitués ou polysubstitués par des substituants choisis indépendamment les uns des autres dans le groupe constitué de -F, -Cl, -Br, -CN, -CH₃, -C₂H₅, -NH₂, -NO₂,-SH, -CF₃, OH, -OCH₃, -OC₂H₅ et -N(CH₃)₂ ; ou X₁ et X₁' , ou X₂ et X₂', ou X₃ et X₃' représentent conjointement =O ; ou X₁ et X₁' représentent conjointement un groupe cycloalkyle en C₃ à C₆ qui peut être non substitué ou substitué par un ou plusieurs substituants choisis indépendamment les uns des autres dans le groupe constitué de -F, -Cl, -Br, -I, -OR₅, SR₅, un groupe alkyle en C₁ à C₃ ou -CN.

5. Composé selon l'une des revendications 1 à 4, dans lequel Y₁, Y₁', Y₂, Y₂', Y₃, Y₃', Y₄ et Y₄' représentent respectivement -H.

6. Composé selon l'une des revendications 1 à 5, qui présente la formule générale (3.1)

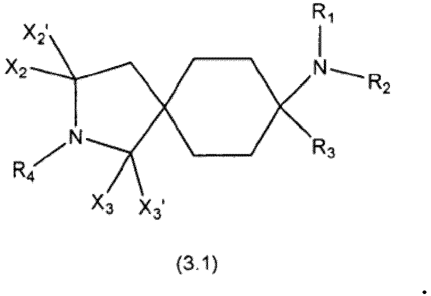

7. Composé selon l'une des revendications 1 à 6, dans lequel Y₁, Y₁', Y₂, Y₂', Y₃, Y₃', Y₄ et Y₄' représentent respectivement -H ;
X₁, X₁', X₂, X₂', X₃ et X₃' représentent H ; ou X₂, X₂', ou X₃ et X₃' représentent conjointement =O ; ou X₁ et X₁' représentent conjointement un groupe cycloalkyle en C₃ à C₆ ;
R₀ représente respectivement indépendamment un groupe-aliphatique en C₁ à C₈, -cycloaliphatique en C₃ à C₁₂, -aryle,-hétéroaryle, -aliphatique en C₁ à C₈-cycloaliphatique en C₃ à C₁₂, -aliphatique en C₁ à C₈-aryle, -aliphatique en C₁ à C₈-hétéroaryle, -cycloaliphatique en C₃ à C₈-aliphatique en C₁ à C₈,-cycloaliphatique en C₃ à C₈-aryle ou -cycloaliphatique en C₃ à C₈-hétéroaryle ; dans lequel ceux-ci sont non substitués ou monosubstitués ou polysubstitués par des substituants choisis indépendamment les uns des autres dans le groupe constitué de-F, -Cl, -Br, -CN, -CH₃, -C₂H₅, -NH₂, -NO₂, -SH, -CF₃, OH, -OCH₃, -OC₂H₅ et -N(CH₃)₂ ;
R₁ représente -CH₃ ;
R₂ représente -H ou -CH₃ ; ou
R₁ et R₂ forment conjointement un cycle et représentent -(CH₂)₃₋₄ ; et
R₃ représente un groupe -aliphatique en C₁ à C₈, -aryle,-hétéroaryle -aliphatique en C₁ à C₃-aryle, -aliphatique en C₁ à C₃-hétéroaryle ou -aliphatique en C₁ à C₃-cycloaliphatique en C₅ à C₆ ; dans lequel ceux-ci sont non substitués ou monosubsitués ou polysubstitués par des substituants choisis indépendamment les uns des autres dans le groupe constitué de -F, -Cl, -Br,-CN, -CH₃, -C₂H₅, -NH₂, -NO₂, -SH, -CF₃, OH, -OCH₃, -OC₂H₅ et-N(CH₃)₂ ; et
R₄ représente H ou -Z-R₁₁,
dans lequel
-Z- peut être absent ou peut être -C(=O), et
R₁₁ représente un groupe -alkyle en C₁ à C₆, -cycloalkyle en C₃ à C₆ ou -alkyle en C₁ à C₃-cycloalkyle en C₃ à C₆, dans lequel dans le groupe cycloalkyle en C₃ à C₆, un atome de carbone cyclique peut être remplacé par un atome d'oxygène et les groupes -alkyle en C₁ à C₆, -cycloalkyle en C₃ à C₆ ou -alkyle en C₁ à C₃-cycloalkyle en C₃ à C₆ peuvent être non substitués, monosubstitués ou poly-substitués, avec des substituants choisis indépendamment les uns des autres dans le groupe constitué de-F, -Cl, -Br, -I, -CN, -OH, -SH, -O-alkyle en C₁ à C₃ et -S-alkyle en C₁ à C₃ ; dans lequel le groupe -alkyle en C₁ à C₃ peut être substitué par un ou plusieurs substituants du groupe constitué des substituants -F, -Cl, -Br, -I, -CN, -OH et -SH.

8. Composé selon l'une des revendications 1 à 7, dans lequel R₁ et R₂ représentent respectivement -CH₃.

9. Composé selon l'une des revendications 1 à 8, dans lequel R₃ est choisi dans le groupe constitué de groupes phényle, benzyle et 2-thiényle, respectivement non substitués ou monosubstitués ou polysubstitués par des substituants choisis indépendamment les uns des autres dans le groupe constitué de -F, -Cl, -Br,-CN, -CH₃, -C₂H₅, -NH₂, -NO₂, -SH, -CF₃, OH, -OCH₃, -OC₂H₅ et-N(CH₃)₂.

10. Composé selon l'une des revendications 1 à 9, dans lequel R₄ est choisi dans le groupe constitué de H, CH₃, un groupe éthyle, n-propyle, i-propyle, n-butyle, s-butyle, iso-butyle, t-butyle, n-pentyle, s-pentyle, iso-pentyle, 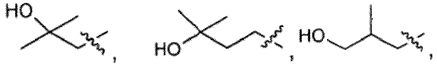 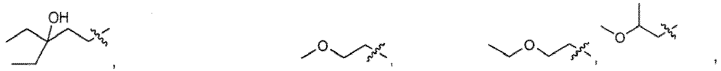 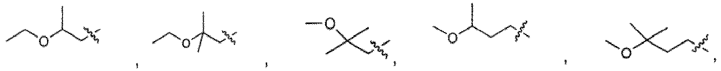 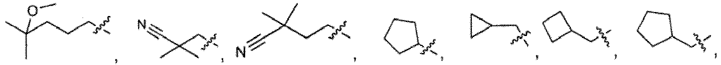 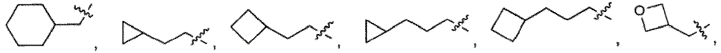 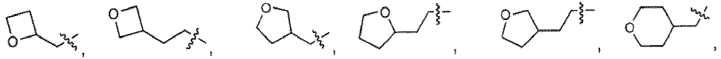 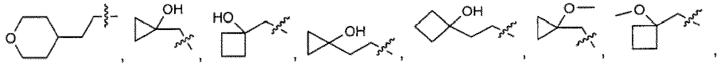 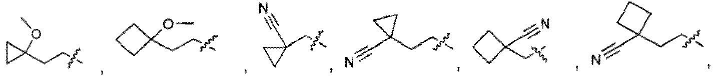 acétyle,

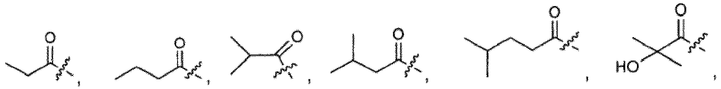

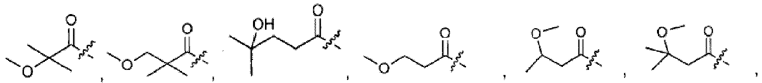

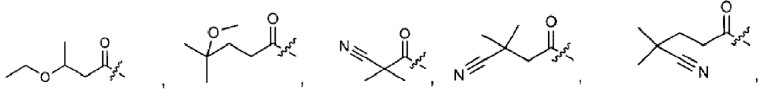

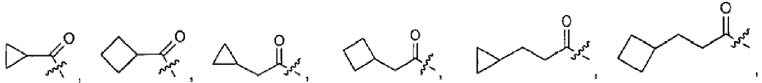

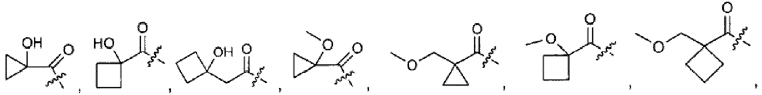

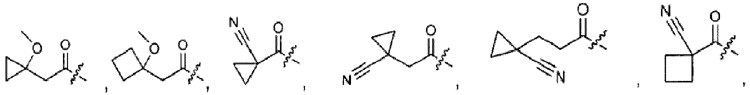

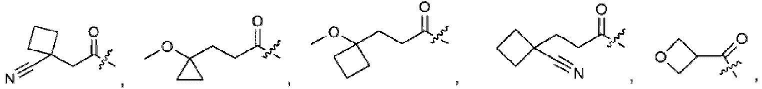

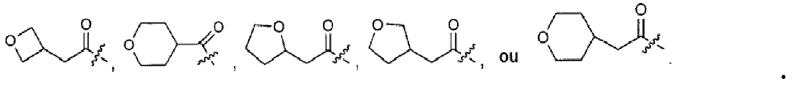

11. Composé selon l'une des revendications 1 à 9, du groupe :
| | |
|---|---|
| 1 ; 2 | 8-benzyl-8-(diméthyl-amino)-3-azaspiro[4.5]décan-4-one |
| 3 ; 4 | (8-benzyl-3-azaspiro[4.5]décan-8-yl)-diméthyl-amine |
| 5 ; 6 | 8-benzyl-3-méthyl-3-azaspiro[4.5]décan-8-yl)-diméthyl-amine |
| 7 ; 8 | 1-[8-benzyl-8-(diméthyl-amino)-3-azaspiro[4.5]décan-3-yl)-éthanone |
| 9 | (8-benzyl-3-butyl-3-azaspiro[4.5]décan-8-yl)-diméthyl-amine |
| 10 ; 11 | 1-[8-benzyl-8-(diméthyl-amino)-3-azaspiro[4.5]décan-3-yl)-butan-1-one |
| 12 | [8-benzyl-3-(cyclopentyl-méthyl)-3-azaspiro[4.5]décan-8-yl)-diméthyl-amine |
| 13 | 8-diméthylamino-8-phényl-3-azaspiro[4.5]décan-4-one |
| 14 ; 15 | 8-(diméthyl-amino)-8-phényl-3-azaspiro[4.5]décan-2-one |
| 16 | 8-butyl-8-diméthylamino-3-azaspiro[4.5]décan -4-one |
| 17 | 8-diméthylamino-8-thiophén-2-yl-3-azaspiro[4.5]décan-4-one |
| 18 ; 25 | Diméthyl-(8-thiophén-2-yl-3-azaspiro[4.5]décan-8-yl)-amine |
| 19 | Diméthyl-(3-méthyl-8-thiophén-2-yl-3-azaspiro[4.5]décan-8-yl)-amine |
| 20 | 1-(8-diméthylamino-8-thiophén-2-yl-3-azaspiro[4.5]décan-3-yl)butan-1-one |
| 21 | (3-butyl-8-thiophén-2-yl-3-azaspiro[4.5]décan-8-yl)-diméthylamine |
| 22 | [3-(cyclopentyl-méthyl)-8-thiophén-2-yl-3-azaspiro[4.5]décan-8-yl)-diméthylamine |
| 23 | 8-(diméthyl-amino)-8-thiophén-2-yl-2-azaspiro[4.5]décan-3-one |
| 24a/b | 8-(diméthyl-amino)-8-thiophén-2-yl-2-azaspiro[4.5]décan-3-one |
| 26 | 1-[8-(diméthyl-amino)-8-thiophén-2-yl-3-azaspiro[4.5]décan-3-yl]-butan-1-one |
| 27 | (8-benzyl-3-butyl-3-azaspiro[4.5]décan-8-yl)-diméthylamine |
| 28 ; 29 | 1-[8-(diméthyl-amino)-8-phényl-3-azaspiro[4.5]décan-3-yl)-butan-1-one |
| 30 | 8-(diméthyl-amino)-8-(5-méthyl-thiophén-2-yl)-3-azaspiro[4.5]décan-4-one |
| 31 | Diméthyl-[8-(5-méthyl-thiophén-2-yl)-3-azaspiro[4.5]décan-8-yl)-amine |
| 32 | 1-[8-(diméthyl-amino)-8-(5-méthyl-thiophén-2-yl)-3-azaspiro[4.5]décan-3-yl]-butan-1-one |
| 33 | 3-butyl-8-(diméthyl-amino)-8-thiophén-2-yl-3-azaspiro[4.5]décan-2-one |
| 34 | 8-(diméthyl-amino)-3-méthyl-8-thiophén-2-yl)-3-azaspiro[4.5]décan-2-one |
| 35 | [3-butyl-8-(5-méthyl-thiophén-2-yl)-3-azaspiro[4.5]décan-8-yl)-diméthyl-amine |
| 36 | Cyclobutyl-[8-diméthylamino-8-(5-méthyl-thiophén-2-yl)-3-azaspiro[4.5]décan-3-yl]-méthanone |
| 37 | Cyclopropyl-[8-diméthylamino-8-(5-méthyl-thiophén-2-yl)-3-azaspiro[4.5]décan-3-yl]-méthanone |
| 38 | 1-[8-diméthylamino-8-(5-méthyl-thiophén-2-yl)-3-azaspiro[4.5]décan-3-yl]-2-méthyl-propan-1-one |
| 39 | 1-[8-diméthylamino-8-(5-méthyl-thiophén-2-yl)-3-azaspiro[4.5]décan-3-yl]-3-méthyl-butan-1-one |
| 40 | 1-[8-diméthylamino-8-(5-méthyl-thiophén-2-yl)-3-azaspiro[4.5]décan-3-yl]-propan-1-one |
| 41 | [3-(2-méthoxy-éthyl)-8-(5-méthyl-thiophén-2-yl)-3-azaspiro[4.5]décan-8-yl)-diméthyl-amine |
| 42 | 1-[8-(diméthylamino)-8-thiophén-2-yl-3-azaspiro[4.5]décan-3-yl]-2-méthoxy-éthanone |
| 43 | Cyclobutyl-[8-(diméthyl-amino-8-thiophén-2-yl)-3-azaspiro[4.5]décan-3-yl]-méthanone |
| 44 | Cyclopropyl-[8-(diméthyl-amino)-8-thiophén-2-yl-3-azaspiro[4.5]décan-3-yl]-méthanone |
| 45 | 1-[8-(diméthyl-amino)-8-thiophén-2-yl-3-azaspiro[4.5]décan-3-yl]-2-méthyl-propan-1-one |
| 46 | 1-[8-(diméthyl-amino)-8-thiophén-2-yl-3-azaspiro[4.5]décan-3-yl]-3-méthyl-butan-1-one |
| 47 | [3-(2-méthoxy-éthyl)-8-thiophén-2-yl-3-azaspiro[4.5]décan-8-yl]-diméthyl-amine |
| 48 | 2-cyclopropyl-1-[8-(diméthyl-amino)-8-thiophén-2-yl-3-azaspiro[4.5]décan-3-yl]-éthanone |
| 49 | [3-(cyclopropyl-méthyl)-8-thiophén-2-yl-3-azaspiro[4.5]décan-8-yl]-diméthyl-amine |
| 50 | 2-cyclobutyl-1-[8-(diméthyl-amino)-8-thiophén-2-yl-3-azaspiro[4.5]décan-3-yl]-éthanone |
| 51 | [3-(2-cyclopropyl-éthyl)-8-thiophén-2-yl-3-azaspiro[4.5]décan-8-yl]-diméthyl-amine |
| 52 | [3-(2-cyclobutyl-éthyl)-8-thiophén-2-yl-3-azaspiro[4.5]décan-8-yl]-diméthyl-amine |
| 53 | [3-(cyclobutyl-méthyl)-8-thiophén-2-yl-3-azaspiro[4.5]décan-8-yl]-diméthyl-amine |
| 54 | 1-[8-(diméthyl-amino)-8-thiophén-2-yl-3-azaspiro[4.5]décan-3-yl]-4-méthyl-pentan-1-one |
| 55 | 3-[8-(diméthyl-amino)-8-thiophén-2-yl-3-azaspiro[4.5]décan-3-yl]-2,2-diméthyl-3-oxo-propionitrile |
| 56 | 1-[8-(diméthyl-amino)-8-thiophén-2-yl-3-azaspiro[4.5]décan-3-yl]-2-tétrahydrofuran-3-yl-éthanone |
| 57 | (8-diméthylamino-8-thiophén-2-yl-3-azaspiro[4.5]décan-3-yl)-(oxétan-3-yl)-méthanone |
| 58 | 1-(8-diméthylamino-8-thiophén-2-yl-3-azaspiro[4.5]décan-3-carbonyl)-cyclopropan-1-carbonitrile |
| 59 | 1-(8-diméthylamino-8-thiophén-2-yl-3-azaspiro[4.5]décan-3-yl)-4-méthoxy-4-méthyl-pentan-1-one |
| 60 | [8-(diméthyl-amino)-8-thiophén-2-yl-3-azaspiro[4.5]décan-3-yl]-tétrahydropyran-4-yl-méthanone |
| 61 | 1-[8-(diméthyl-amino)-8-thiophén-2-yl-3-azaspiro[4.5]décan-3-carbonyl]-cyclobutan-1-carbonitrile |
| 62 | 3-cyclopropyl-1-[8-(diméthyl-amino)-8-thiophén-2-yl-3-azaspiro[4.5]décan-3-yl]-propan-1-one |
| 63 | 1-[8-(diméthyl-amino)-8-thiophén-2-yl-3-azaspiro[4.5]décan-3-yl]-2-méthoxy-2-méthyl-propan-1-one |
| 64 | 1-[8-(diméthyl-amino)-8-thiophén-2-yl-3-azaspiro[4.5]décan-3-yl]-2-tétrahydrofuran-2-yl-éthanone |
| 65 | 1-[8-(diméthyl-amino)-8-thiophén-2-yl-3-azaspiro[4.5]décan-3-yl]-4-hydroxy-4-méthyl-pentan-1-one |
| 66 | [8-(diméthyl-amino)-8-thiophén-2-yl-3-azaspiro[4.5]décan-3-yl]-(1-hydroxy-cyclobutyl)-méthanone |
| 67 | 1-[2-[8-(diméthyl-amino)-8-thiophén-2-yl-3-azaspiro[4.5]décan-3-yl]-2-oxo-éthyl]-cyclopropan-1-carbonitrile |
| 68 | 1-[2-[8-(diméthyl-amino)-8-thiophén-2-yl-3-azaspiro[4.5]décan-3-yl]-2-oxo-éthyl]-cyclobutan-1-carbonitrile |
| 69 | 3-cyclobutyl-1-[8-(diméthyl-amino)-8-thiophén-2-yl-3-azaspiro[4.5]décan-3-yl]-propan-1-one |
| 70 | Diméthyl-[3-(oxétan-3-yl-méthyl)-8-thiophén-2-yl-3-azaspiro[4.5]décan-8-yl]-amine |
| 71 | Diméthyl-(8-phényl-3-azaspiro[4.5]décan-8-yl)-amine |
| 72 | 1-[8-(diméthyl-amino)-8-phényl-3-azaspiro[4.5]décan-3-yl]-butan-1-one |
| 73 | 1-(8-diméthylamino-8-phényl-3-azaspiro[4.5]décan-3-yl)-éthanone |
| 74 | (3-butyl-8-phényl-3-azaspiro[4.5]décan-8-yl)-diméthylamine |
| 75 | 1-[8-(diméthyl-amino)-8-phényl-3-azaspiro[4.5]décan-3-yl]-2-tétrahydro-furan-2-yl-éthanone |
| 76 | 1-[2-[8-(diméthyl-amino)-8-phényl-3-azaspiro[4.5]décan-3-yl]-2-oxo-éthyl]-cyclobutan-1-carbonitrile |
| 77 | 1-[2-[8-(diméthyl-amino)-8-phényl-3-azaspiro[4.5]décan-3-yl]-2-oxo-éthyl]-cyclopropan-1-carbonitrile |
| 78 | 1-[8-(diméthyl-amino)-8-phényl-3-azaspiro[4.5]décan-3-yl]-2-(oxétan-3-yl)-éthanone |
| 79 | 1-[8-(diméthyl-amino)-8-phényl-3-azaspiro[4.5]décan-3-yl]-4-méthyl-pentan-1-one |
| 80 | 3-[8-(diméthyl-amino)-8-phényl-3-azaspiro[4.5]décan-3-yl]-2,2-diméthyl-3-oxo-propionitrile |
| 81 | [8-(diméthyl-amino)-8-phényl-3-azaspiro[4.5]décan-3-yl]-tétrahydro-pyran-4-yl-méthanone |
| 82 | 1-[8-(diméthyl-amino)-8-phényl-3-azaspiro[4.5]décan-3-yl]-4-hydroxy-4-méthyl-pentan-1-one |
| 83 | 1-[8-(diméthyl-amino)-8-phényl-3-azaspiro[4.5]décan-3-yl]-4-méthoxy-4-méthyl-pentan-1-one |
| 84 | [8-(diméthyl-amino)-8-phényl-3-azaspiro[4.5]décan-3-yl]-(1-hydroxy-cyclobutyl)méthanone |
| 85 | 2-cyclopropyl-1-[8-(diméthyl-amino)-8-phényl-3-azaspiro[4.5]décan-3-yl]-éthanone |
| 86 | 2-cyclobutyl-1-[8-(diméthyl-amino)-8-phényl-3-azaspiro[4.5]décan-3-yl]-éthanone |
| 87 | [3-(4-méthoxy-4-méthyl-pentyl)-8-phényl-3-azaspiro[4.5]décan-8-yl]-diméthylamine |
| 88 | 1-[8-(5-chloro-thiophén-2-yl)-8-diméthylamino-3-azaspiro[4.5]décan-3-yl]-butan-1-one |
| 89 | [3-butyl-8-(5-chloro-tiophén-2-yl)-3-azaspiro[4.5]décan-8-yl]-diméthylamine |
| 90 | 1-[8-diméthylamino-8-(5-fluoro-tiophén-2-yl)-3-azaspiro[4.5]décan-3-yl]-butan-1-one |
| 91 | [3-butyl-8-(5-fluoro-tiophén-2-yl)-3-azaspiro[4.5]décan-8-yl]-diméthyl-amine |
| 92 | 8-(cyclohexyl-méthyl)-8-diméthylamino-3-azaspiro[4.5]décan-4-one |
| 93 | 1-[8-(cyclohexyl-méthyl)-8-diméthylamino-3-azaspiro[4.5]décan-3-yl]-butan-1-one |
| 94 | [3-butyl-8-(cyclohexyl-méthyl)-3-azaspiro[4.5]décan-8-yl]-diméthyl-amine |
| 95 | 1-[8-(cyclopentyl-méthyl)-8-diméthylamino-3-azaspiro[4.5]décan-3-yl]-butan-1-one |
| 96 | [3-butyl-8-(cyclopentyl-méthyl)-3-azaspiro[4.5]décan-8-yl]-diméthyl-amine |
| 97 | 1-(8-cyclopentyl-8-diméthylamino-3-azaspiro[4.5]décan-3-yl]-butan-1-one |
| 98 | (3-butyl-8-cyclopentyl-3-azaspiro[4.5]décan-8-yl)-diméthyl-amine |
| 99 | Cyclobutyl-(8-cyclopentyl-8-diméthylamino-3-azaspiro[4.5]décan-3-yl)-méthanone |
| 100 | (8-cyclopentyl-8-diméthylamino-3-azaspiro[4.5]décan-3-yl)-cyclopropyl-méthanone |
| 101 | [8-(cyclopentyl-3-(cyclopropyl-méthyl)-3-azaspiro[4.5]décan-8-yl]-diméthyl-amine |
| 102 | [3-(cyclobutyl-méthyl)-8-cyclopentyl-3-azaspiro[4.5]décan-8-yl]-diméthyl-amine |
| 103 | 1-(8-cyclopentyl-8-diméthylamino-3-azaspiro[4.5]décan-3-yl)-2-cyclopropyl-éthanone |
| 104 | [8-(cyclopentyl-3-(2-cyclopropyl-éthyl)-3-azaspiro[4.5]décan-8-yl]-diméthyl-amine |
| 105 | 2(cyclobutyl-1-(8-cyclopentyl-8-diméthylamino-3-azaspiro[4.5]décan-3-yl)-éthanone |
| 106 ; 107 | [3-butyl-8-(diméthyl-amino)-3-azaspiro[4.5]décan-8-yl]-phényl-méthanone |
| 108 ; 109 | 1-[8-(azétidin-1-yl)-8-thiophén-2-yl-3-azaspiro[4.5]décan-3-yl]-butan-1-one |
| 110 ; 111 | 1-[8-(azétidin-1-yl)-8-phényl-3-azaspiro[4.5]décan-3-yl]-butan-1-one |
| 112 ; 113 | 8-(azétidin-1-yl)-3-butyl-8-phényl-3-azaspiro[4.5]décane |
| 114 | 8-diméthylamino-3-méthyl-8-thiophén-2-yl-3-azaspiro[4.5]décan-4-one |
| 115 | 3-butyl-8-diméthylamino-8-thiophén-2-yl-3-azaspiro[4.5]décan-4-one |
| 116 | 3-(cyclopentyl-méthyl)-8-diméthylamino-8-thiophén-2-yl-3-azaspiro[4.5]décan-4-one |
| 117 | 8-(5-chloro-thiophén-2-yl)-8-diméthylamino-3-azaspiro[4.5]décan-4-one |
| 118 ; 119 | 8-benzyl-8-(diméthyl-amino)-3-méthyl-3-azaspiro[4.5]décan-4-one |
| 120 ; 121 | 8-benzyl-3-butyl-8-(diméthyl-amino)-3-azaspiro[4.5]décan-4-one |
| 122 ; 123 | 8-benzyl-3-(cyclopentyl-méthyl)-8-(diméthyl-amino)-3-azaspiro[4.5]décan-4-one |
| 124 | 8-(diméthyl-amino)-3-méthyl-8-thiophén-2-yl-3-azaspiro[4.5]décan-2-one |
| 125 | 3-butyl-8-(diméthyl-amino)-8-thiophén-2-yl-3-azaspiro[4.5]décan-2-one |
| 126 ; 127 | 3-(cyclopentyl-méthyl)-8-(diméthyl-amino)-8-thiophén-2-yl-3-azaspiro[4.5]décan-2-one |
| 128 ; 129 | 3-(2-cyclobutyl-éthyl)-8-(diméthyl-amino)-8-thiophén-2-yl-3-azaspiro[4.5]décan-2-one |
| 130 ; 131 | 3-(2-cyclopropyl-éthyl)-8-(diméthyl-amino)-8-thiophén-2-yl-3-azaspiro[4.5]décan-2-one |
| 132 ; 133 | 3-(cyclobutyl-méthyl)-8-(diméthyl-amino)-8-thiophén-2-yl-3-azaspiro[4.5]décan-2-one |
| 134 ; 135 | 3-(cyclopropyl-méthyl)-8-(diméthyl-amino)-8-thiophén-2-yl-3-azaspiro[4.5]décan-2-one |
| 136 ; 137 | 8-(diméthyl-amino)-3-(2-tétrahydro-furan-2-yl-éthyl)-8-thiophén-2-yl-3-azaspiro[4.5]décan-2-one |
| 138 ; 139 | 8-(diméthyl-amino)-3-(tétrahydro-pyran-4-yl-méthyl)-8-thiophén-2-yl-3-azaspiro[4.5]décan-2-one |
| 140 ; 141 | 8-(diméthyl-amino)-3-(2-tétrahydro-furan-3-yl-éthyl)-8-thiophén-2-yl-3-azaspiro[4.5]décan-2-one |
| 142 ; 143 | 8-(diméthyl-amino)-3-[2-(oxétan-3-yl)-éthyl]-8-thiophén-2-yl-3-azaspiro[4.5]décan-2-one |
| 144 ; 145 | 1-[2-[8-(diméthyl-amino)-2-oxo-8-thiophén-2-yl-3-azaspiro[4.5]décan-3-yl]-éthyl]-cyclopropan-1-carbonitrile |
| 146 ; 147 | 8-(diméthyl-amino)-3-(oxétan-2-yl-méthyl)-8-thiophén-2-yl-3-azaspiro[4.5]décan-2-one |
| 148 ; 149 | 8-(diméthyl-amino)-3-(oxétan-3-yl-méthyl)-8-thiophén-2-yl-3-azaspiro[4.5]décan-2-one |
| 150 ; 151 | 1-[2-[8-(diméthyl-amino)-2-oxo-8-thiophén-2-yl-3-azaspiro[4.5]décan-3-yl]-éthyl]-cyclobutan-1-carbonitrile |
| 152 ; 153 | 8-(diméthyl-amino)-8-(5-méthyl-thiophén-2-yl)-3-azaspiro[4.5]décan-2-one |
| 154 ; 155 | 8-(diméthyl-amino)-3-méthyl-8-(5-méthyl-thiophén-2-yl)-3-azaspiro[4.5]décan-2-one |
| 156 ; 157 | 3-butyl-8-(diméthyl-amino)-8-(5-méthyl-thiophén-2-yl)-3-azaspiro[4.5]décan-2-one |
| 158 ; 159 | 3-(cyclopentyl-méthyl)-8-(diméthyl-amino)-8-(5-méthyl-thiophén-2-yl)-3-azaspiro[4.5]décan-2-one |
| 160 | 3-(cyclopropyl-méthyl)-8-(diméthyl-amino)-8-(5-méthyl-thiophén-2-yl)-3-azaspiro[4.5]décan-2-one |
| 161 | 3-(cyclobutyl-méthyl)-8-(diméthyl-amino)-8-(5-méthyl-thiophén-2-yl)-3-azaspiro[4.5]décan-2-one |
| 162 | 8-(diméthyl-amino)-3-méthyl-8-phényl-3-azaspiro[4.5]décan-2-one |
| 163 ; 164 | 3-butyl-8-(diméthyl-amino)-8-phényl-3-azaspiro[4.5]décan-2-one |
| 165 ; 166 | 3-(cyclopentyl-méthyl)-8-(diméthyl-amino)-8-phényl-3-azaspiro[4.5]décan-2-one |
| 167 ; 168 | 3-(2-cyclopropyl-éthyl)-8-(diméthyl-amino)-8-phényl-3-azaspiro[4.5]décan-2-one |
| 169 ; 170 | 3-(cyclobutyl-méthyl)-8-(diméthyl-amino)-8-phényl-3-azaspiro[4.5]décan-2-one |
| 171 ; 172 | 3-(cyclopropyl-méthyl)-8-(diméthyl-amino)-8-phényl-3-azaspiro[4.5]décan-2-one |
| 173 ; 174 | 3-(2-cyclobutyl-éthyl)-8-(diméthyl-amino)-8-phényl-3-azaspiro[4.5]décan-2-one |
| 175 ; 176 | 1-[2-[8-(diméthyl-amino)-2-oxo-8-phényl-3-azaspiro[4.5]décan-3-yl]-éthyl]-cyclopropane-1-carbonitrile |
| 177 ; 178 | 1-[2-[8-(diméthyl-amino)-2-oxo-8-phényl-3-azaspiro[4.5]décan-3-yl]-éthyl]-cyclobutane-1-carbonitrile |
| 179 ; 180 | 8-(diméthyl-amino)-3-[(1-hydroxy-cyclobutyl)-méthyl]-8-phényl-3-azaspiro[4.5]décan-2-one |
| 181 | 8-(diméthyl-amino)-3-(2-hydroxy-2-méthyl-propyl)-8-phényl-3-azaspiro[4.5]décan-2-one |
| 182 ; 183 | 8-(diméthyl-amino)-3-(3-méthoxy-3-méthyl-butyl)-8-phényl-3-azaspiro[4.5]décan-2-one |
| 184 ; 185 | 8-(diméthyl-amino)-3-[2-(1-méthoxy-cyclobutyl)-éthyl]-8-phényl-3-azaspiro[4.5]décan-2-one |
| 186 ; 187 | 8-(diméthyl-amino)-3-(3-hydroxy-3-méthyl-butyl)-8-phényl-3-azaspiro[4.5]décan-2-one |
| 188 | 1-[8-(diméthyl-amino)-8-phényl-3-azaspiro[4.5]décan-3-yl]-2-tétrahydro-furan-3-yl-éthanone |
| 189 | [8-(diméthyl-amino)-8-phényl-3-azaspiro[4.5]décan-3-yl]-(oxétan-3-yl)-méthanone |
| 190 | 1-[8-(diméthyl-amino)-8-phényl-3-azaspiro[4.5]décan-3-yl]-2-méthoxy-2-méthyl-propan-1-one |
| 191 | [3-(2-cyclopropyl-éthyl)-8-phényl-3-azaspiro[4.5]décan-8-yl]-diméthyl-amine |
| 192 | [3-(2-cyclobutyl-éthyl)-8-phényl-3-azaspiro[4.5]décan-8-yl]-diméthyl-amine |
| 193 | 1-[[8-(diméthyl-amino)-8-phényl-3-azaspiro[4.5]décan-3-yl]-méthyl]-cyclobutan-1-ol |
| 194 | 1-[2-[8-(diméthyl-amino)-8-phényl-3-azaspiro[4.5]décan-3-yl]-éthyl]-cyclobutan-1-carbonitrile |
| 195 | 3-cyclobutyl-1-[8-(diméthyl-amino)-8-phényl-3-azaspiro[4.5]décan-3-yl]-propan-1-one |
| 196 | Diméthyl-[3-(oxétan-3-yl-méthyl)-8-phényl-3-azaspiro[4.5]décan-8-yl]-amine |
| 197 | 3-cyclopropyl-1-[8-(diméthyl-amino)-8-phényl-3-azaspiro[4.5]décan-3-yl]-propan-1-one |
| 198 | 1-[8-(diméthyl-amino)-8-phényl-3-azaspiro[4.5]décan-3-carbonyl]-cyclopropan-1-carbonitrile |
| 199 | 1-[8-(diméthyl-amino)-8-phényl-3-azaspiro[4.5]décan-3-carbonyl]-cyclobutan-1-carbonitrile |
| 200 | 1-[2-[8-(diméthyl-amino)-8-phényl-3-azaspiro[4.5]décan-3-yl]-éthyl]-cyclopropane-1-carbonitrile |
| 201 | Diméthyl-[8-phényl-3-(2-tétrahydro-furan-3-yl-éthyl)-3-azaspiro[4.5]décan-8-yl]amine |
| 202 | [3-(2-méthoxy-2-méthyl-propyl)-8-phényl-3-azaspiro[4.5]décan-8-yl]diméthylamine |
| 203 | [3-[3-cyclobutyl-propyl)-8-phényl-3-azaspiro[4.5]décan-8-yl]-diméthyl-amine |
| 204 | [3-(3-cyclopropyl-propyl)-8-phényl-3-azaspiro[4.5]décan-8-yl]diméthyl-amine |
| 205 | [8-(diméthyl-amino)-8-phényl-3-azaspiro[4.5]décan-3-yl]-(1-hydroxy-cyclopropyl)-méthanone |
| 206 | 1-[[8-(diméthyl-amino)-8-phényl-3-azaspiro[4.5]décan-3-yl]-méthyl]-cyclopropan-1-ol |
| 207 | 1-[[8-(diméthyl-amino)-8-thiophén-2-yl-3-azaspiro[4.5]décan-3-yl]-méthyl]-cyclobutan-1-ol |
| 208 | 1-[2-[8-(diméthyl-amino)-8-thiophén-2-yl-3-azaspiro[4.5]décan-3-yl]-éthyl]-cyclobutan-1-carbonitrile |
| 209 | [8-(diméthyl-amino)-8-thiophén-2-yl-3-azaspiro[4.5]décan-3-yl]-(1-hydroxy-cyclopropyl)-méthanone |
| 210 | 1-[[8-(diméthyl-amino)-8-thiophén-2-yl-3-azaspiro[4.5]décan-3-yl]-méthyl]-cyclopropan-1-ol |
| 211 | 1-[2-[8-(diméthyl-amino)-8-thiophén-2-yl-3-azaspiro[4.5]décan-3-yl]-éthyl]-cyclopropan-1-carbonitrile |
| 212 | 1-(8-(diméthylamino-8-thiophén-2-yl-3-azaspiro[4.5]décan-3-yl]-2-(oxétan-3-yl)éthanone |
| 213 | 1-[8-(diméthyl-amino)-8-thiophén-2-yl-3-azaspiro[4.5]décan-3-yl]-3-éthyl-pentan-3-ol |
| 214 | 1-[8-(diméthyl-amino)-8-(thiophén-2-yl-méthyl)-3-azaspiro[4.5]décan-3-yl]-butan-1-one |
| 215 ; 216 | 8-(diméthyl-amino)-3-[(1-hydroxy-cyclobutyl)-méthyl]-8-thiophén-2-yl-3-azaspiro[4.5]décan-2-one |
| 217 ; 218 | 8-(diméthyl-amino-3-[2-(1-méthoxy-cyclobutyl)-éthyl]-8-thiophén-2-yl-3-azaspiro[4.5]décan-2-one |
| 219 ; 220 | 8-(diméthyl-amino)-3-(3-méthoxy-3-méthyl-butyl)-8-thiophén-2-yl-3-azaspiro[4.5]décan-2-one |
| 221 ; 222 | 8-(diméthyl-amino)-3-(3-hydroxy-3-méthyl-butyl)-8-thiophén-2-yl-3-azaspiro[4.5]décan-2-one |
| 223 | 1-[8-(diméthyl-amino)-8-thiophén-2-yl-3-azaspiro[4.5]décan-3-yl]-2-hydroxy-2-méthyl-propan-1-one |
| 224 ; 225 | 8-(diméthyl-amino)-3-(2-hydroxy-2-méthyl-propyl)-8-thiophén-2-yl-3-azaspiro[4.5]décan-2-one |
| 226 | 4-[8-(diméthyl-amino)-8-thiophén-2-yl-3-azaspiro[4.5]décan-3-yl]-2-méthyl-butan-2-ol |
| 227 | 1-[8-(diméthyl-amino)-8-thiophén-2-yl-3-azaspiro[4.5]décan-3-yl]-2-méthyl-propan-2-ol |
| 228 | 1-[8-(diméthyl-amino)-8-phényl-3-azaspiro[4.5]décan-3-yl]-2-hydroxy-2-méthyl-propan-1-one |
| 229 | 1-[8-(diméthyl-amino)-8-phényl-3-azaspiro[4.5]décan-3-yl]-2-méthyl-propan-2-ol |
| 230 ; 231 | 4-[8-(diméthyl-amino)-2-oxo-8-phényl-3-azaspiro[4.5]décan-3-yl]-2,2-diméthyl-butyronitrile |
| 232 | 4-[8-(diméthyl-amino)-8-phényl-3-azaspiro[4.5]décan-3-yl]-2,2-diméthyl-4-oxo-butyronitrile |
| 233 | 4-[8-(diméthyl-amino)-8-thiophén-2-yl-3-azaspiro[4.5]décan-3-yl]-2,2-diméthyl-4-oxo-butyronitrile |
| 234 | 8-cyclopentyl-3-(2-cyclopropyl-éthyl)-8-(diméthyl-amino)-3-azaspiro[4.5]décan-2-one |
| 235 | 1-[8-(diméthyl-amino)-8-thiophén-2-yl-3-azaspiro[4.5]décan-3-yl]-2-(1-méthoxy-cyclobutyl)-éthanone |
| 236 | 3-(2-cyclobutyl-éthyl)-8-cyclopenthyl-8-(diméthyl-amino)-3-azaspiro[4.5]décan-2-one |
| 237 | 1-[8-(diméthyl-amino)-8-phényl-3-azaspiro[4.5]décan-3-yl]-2-(1-méthoxy-cyclobutyl)-éthanone |
| 238 | [3-[2-(1-méthoxy-cyclobutyl)-éthyl]-8-thiophén-2-yl-3-azaspiro[4.5]décan-8-yl]-diméthyl-amine |
| 239 | [3-[2-(1-méthoxy-cyclobutyl)-éthyl]-8-phényl-3-azaspiro[4.5]décan-8-yl]-diméthyl-amine |
| 240 | 4-[8-(diméthyl-amino)-2-oxo-8-thiophén-2-yl-3-azaspiro[4.5]décan-3-yl]-2,2-diméthyl-butyronitrile |
| 241 ; 242 | 8-(diméthyl-amino)-3-[2-(oxétan-3-yl)-éthyl]-8-thiophén-2-yl-3-azaspiro[4.5]décan-2-one |
| 243 | 8-cyclopentyl-3-(2-cyclopropyl-éthyl)-8-(diméthyl-amino)-3-azaspiro[4.5]décan-2-one |
| 244 | 8-cyclopentyl-8-(diméthyl-amino)-3-(oxétan-3-yl-méthyl)-3-azaspiro[4.5]décan-2-one |
| 245 | 1-[2-[8-cyclopentyl-8-(diméthyl-amino)-2-oxo-3-azaspiro[4.5]décan-3-yl]-éthyl]-cyclopropan-1-carbonitrile |
| 246 | 8-cyclopentyl-8-(diméthyl-amino)-3-[2-(oxétan-3-yl)-éthyl]-3-azaspiro[4.5]décan-2-one |
| 247 | 1-(8-cyclopentyl-8-diméthylamino-3-azaspiro[4.5]décan-3-yl)-2-(1-méthoxy-cyclobutyl)-éthanone |
| 248 | 4-[8-(diméthyl-amino)-8-thiophén-2-yl-3-azaspiro[4.5]décan-3-yl]-2,2-diméthyl-butyronitrile |
| 249 | 4-[8-(diméthyl-amino)-8-phényl-3-azaspiro[4.5]décan-3-yl]-2,2-diméthyl-butyronitrile |
| 250 | 4-[8-(diméthyl-amino)-2-oxo-8-thiophén-2-yl-3-azaspiro[4.5]décan-3-yl]-2,2-diméthyl-butyronitrile |
| 251 ; 252 | 8-(diméthyl-amino)-3-(oxétan-3-yl-méthyl)-8-phényl-3-azaspiro[4.5]décan-2-one |
| 253 | 1-[2-[8-(diméthyl-amino)-8-thiophén-2-yl-3-azaspiro[4.5]décan-3-yl]-éthyl]-cyclopropan-1-ol |
| 254 ; 255 | 8-(diméthyl-amino)-3-[2-(oxétan-3-yl)-éthyl]-8-phényl-3-azaspiro[4.5]décan-2-one |
| 256 | 1-[2-[8-cyclopentyl-8-(diméthyl-amino)-2-oxo-3-azaspiro[4.5]décan-3-yl]-éthyl]-cyclobutan-1-carbonitrile |
| 257 | [8-cyclopentyl-3-[2-(1-méthoxy-cyclobutyl)-éthyl]-3-azaspiro[4.5]décan-8-yl]-diméthyl-amine |
| 258 | 4-(8-cyclopentyl-8-diméthylamino-3-azaspiro[4.5]décan-3-yl)-2,2-diméthyl-butyronitrile |
| 259 | 4-(8-cyclopentyl-8-diméthylamino-3-azaspiro[4.5]décan-3-yl)-2,2-diméthyl-4-oxo-butyronitrile |
| 260 ; 261 | 8-(diméthyl-amino)-8-phényl-3-(tétrahydro-furan-3-yl-méthyl)-3-azaspiro[4.5]décan-2-one |
| 262 ; 263 | 8-(diméthyl-amino)-8-phényl-3-(2-tétrahydro-furan-2-yl-éthyl)-3-azaspiro[4.5]décan-2-one |
| 264 | 8-(diméthyl-amino)-8-phényl-3-(2-tétrahydro-furan-3-yl-éthyl)-3-azaspiro[4.5]décan-2-one |
| 265 | [8-cyclopentyl-3-(tétrahydro-pyran-4-yl-méthyl)-3-azaspiro[4.5]décan-8-yl]-diméthyl-amine |
| 266 | [8-cyclopentyl-3-(3-méthyl-butyl)-3-azaspiro[4.5]décan-8-yl]-diméthyl-amine |
| 267 | 1-[2-(8-cyclopentyl-8-diméthylamino-3-azaspiro[4.5]décan-3-yl)-éthyl]-cyclopropane-1-carbonitrile |
| 268 | 1-[2-(8-cyclopentyl-8-diméthylamino-3-azaspiro[4.5]décan-3-yl)-éthyl]-cyclobutane-1-carbonitrile |
| 269 ; 270 | 8-(diméthyl-amino)-8-phényl-3-(tétrahydro-pyran-4-yl-méthyl)-3-azaspiro[4.5]décan-2-one |
| 271 | 8-(diméthyl-amino)-8-phényl-3-(2-tétrahydro-furan-3-yl-éthyl)-3-azaspiro[4.5]décan-2-one |
| 272 | 4-[8-(diméthyl-amino)-8-phényl-3-azaspiro[4.5]décan-3-yl]-2-méthyl-butan-2-ol |
| 273 | [8-cyclopentyl-3-(2-méthyl-propyl)-3-azaspiro[4.5]décan-8-yl]-diméthyl-amine |
| 274 | 3-(cyclobutyl-méthyl)-8-cyclopentyl-8-(diméthyl-amino)-3-azaspiro[4.5]décan-2-one |
| 275 | 3-(2-cyclopropyl-éthyl)-8-méthylamino-8-phényl-3-azaspiro[4.5]décan-2-one |
| 276 | 8-cyclopentyl-3-(cyclopropyl-méthyl)-8-(diméthyl-amino)-3-azaspiro[4.5]décan-2-one |
| 277 | 3-(cyclohexyl-méthyl)-8-cyclopentyl-8-(diméthyl-amino)-3-azaspiro[4.5]décan-2-one |
| 278 | 8-cyclopentyl-3-(cyclopentyl-méthyl)-8-(diméthyl-amino)-3-azaspiro[4.5]décan-2-one |
| 279 ; 280 | 8-(diméthyl-amino)-3-[[(3S)-tétrahydro-furan-3-yl]-méthyl]-8-thiophén-2-yl-3-azaspiro[4.5]décan-2-one |
| 281 | 3-butyl-8-cyclopentyl-8-(diméthyl-amino)-3-azaspiro[4.5]décan-2-one |
| 282 | 8-cyclopentyl-8-(diméthyl-amino)-3-méthyl-3-azaspiro[4.5]décan-2-one |
| 283 | Diméthyl-[3-[2-(oxétan-3-yl)-éthyl]-8-phényl]-3-azaspiro[4.5]décan-8-yl]-amine |
| 284 | Diméthyl-[8-phényl-3-(tétrahydro-furane-3-yl-méthyl)-3-azaspiro[4.5]décan-8-yl]-amine |
| 285 | 1-[2-[8-(diméthyl-amino)-8-phényl-3-azaspiro[4.5]décan-3-yl]-éthyl]-cyclopropan-1-ol |
| 286 | 3,8-dicyclopenthyl-8-(diméthyl-amino)-3-azaspiro[4.5]décan-2-one |
| 287 | 8-cyclopentyl-8-(diméthyl-amino)-3-[2-(1-méthoxy-cyclobutyl)-éthyl]-3-azaspiro[4.5]décan-2-one |
| 288 ; 289 | 3-[8-(diméthyl-amino)-2-oxo-8-thiophén-2-yl-3-azaspiro[4.5]décan-3-yl]-2,2-diméthyl-propionitrile |
| 290 | 3-(2-cyclopropyl-éthyl)-8-méthylamino)-8-thiophén-2-yl-3-azaspiro[4.5]décan-2-one |
| 291 | (8-cyclopentyl-8-diméthylamino-3-azaspiro[4.5]décan-3-yl)-(oxétan-3-yl)-méthanone |
| 292 | [8-cyclopentyl-3-(3-méthoxy-3-méthyl-butyl)-3-azaspiro[4.5]décan-8-yl]-diméthyl-amine |
| 293 ; 294 | 8-cyclopentyl-8-(diméthyl-amino)-3-[[(3S)-tétrahydro-furan-3-yl]-méthyl]-3-azaspiro[4.5]décan-2-one |
| 295 | Diméthyl-[3-[2-(oxétan-3-yl)-éthyl]-8-thiophén-2-yl-3-azaspiro[4.5]décan-8-yl]-amine |
| 296 | Diméthyl-[3-(tétrahydro-furan-3-yl-méthyl)-8-thiophén-2-yl-3-azaspiro[4.5]décan-8-yl]-amine |
| 297 | 1-(8-cyclopentyl-8-diméthylamino-3-azaspiro[4.5]décan-3-yl)-2-méthoxy-2-méthyl-propan-1-one |
| 298 | [8-cyclopentyl-3-(2-méthoxy-2-méthyl-propyl)-3-azaspiro[4.5]décan-8-yl]-diméthyl-amine |
| 299 | [3-(3-méthoxy-3-méthyl-butyl)-8-thiophén-2-yl-3-azaspiro[4.5]décan-8-yl]-diméthyl-amine |
| 300 | [3-(3-méthoxy-3-méthyl-butyl)-8-phényl-3-azaspiro[4.5]décan-8-yl]-diméthyl-amine |
| 301 | 3-(8-cyclopentyl-8-diméthylamino-3-azaspiro[4.5]décan-3-yl)-2-méthyl-propan-1-ol |
| 302 | [8-cyclopentyl-3-(oxétan-3-yl-méthyl)-3-azaspiro[4.5]décan-8-yl]-diméthyl-amine |
| 303 | Diméthyl-[8-phényl-3-(tétrahydro-pyran-4-yl-méthyl)-3-azaspiro[4.5]décan-8-yl]-amine |
| 304 | Diméthyl-[3-(tétrahydro-pyran-4-yl-méthyl)-8-thiophén-2-yl-3-azaspiro[4.5]décan-8-yl]-amine |
| 305 | Diméthyl-[8-phényl-3-(2-tétrahydro-furan-2-yl-éthyl)-3-azaspiro[4.5]décan-8-yl]-amine |
| 306 | Diméthyl-[3-(2-tétrahydro-furan-2-yl-éthyl)-8-thiophén-2-yl-3-azaspiro[4.5]décan-8-yl]-amine |
| 307 ; 308 | 3-(2-cyclobutyl-éthyl)-8-méthylamino-8-tiophén-2-yl-3-azaspiro[4.5]décan-2-one |
| 309 | Diméthyl-[3-(2-tétrahydro-furan-3-yl-éthyl)-8-thiophén-2-yl-3-azaspiro[4.5]décan-8-yl]-amine |
| 310 | 3-(8-diméthylamino-2-oxo-8-phényl-3-azaspiro[4.5]décan-3-yl)-2,2-diméthyl-propionitrile |
| 311 | 8-diméthylamino-3-[(1-méthoxy-cyclobutyl)-méthyl]-8-tiophén-2-yl-3-azaspiro[4.5]décan-2-one |
| 312 | 8-diméthylamino-3-[(1-méthoxy-cyclobutyl)-méthyl]-8-phényl-3-azaspiro[4.5]décan-2-one |
| 313 | 8-diméthylamino-3-(2-éthoxy-éthyl)-8-thiophén-2-yl-3-azaspiro[4.5]décan-2-one |
| 314 | 1-(8-diméthylamino-8-thiophén-2-yl-3-azaspiro[4.5]décan-3-yl)-3-méthoxy-3-méthyl-butan-1-one |
| 315 | 1-(8-diméthylamino-8-thiophén-2-yl-3-azaspiro[4.5]décan-3-yl)-3-méthoxy-propan-1-one |
| 316 | 3-(2-cyclopropyl-éthyl)-8-méthylamino-8-thiophén-2-yl-3-azaspiro[4.5]décan-2-one |
| 317 | 8-(diméthyl-amino)-3-(3-méthoxy-butyl)-8-thiophén-2-yl-3-azaspiro[4.5]décan-2-one |
| 318 | 8-(diméthyl-amino)-3-(2-méthoxy-propyl)-8-thiophén-2-yl-3-azaspiro[4.5]décan-2-one |
| 319 | 3-[8-(diméthyl-amino)-2-oxo-8-phényl-3-azaspiro[4.5]décan-3-yl]-2,2-diméthyl-propionitrile |
| 320 | 1-[8-(diméthyl-amino)-8-phényl-3-azaspiro[4.5]décan-3-yl]-3-méthoxy-3-méthyl-butan-1-one |
| 321 | 8-(diméthyl-amino)-3-[(1-méthoxy-cyclobutyl)méthyl]-8-thiophén-2-yl-3-azaspiro[4.5]décan-2-one |
| 322 | 8-(diméthyl-amino)-3-[(1-méthoxy-cyclobutyl)méthyl]-8-phényl-3-azaspiro[4.5]décan-2-one |
| 323 | 8-(diméthyl-amino)-3-(2-méthoxy-propyl)-8-phényl-3-azaspiro[4.5]décan-2-one |
| 324 | 8-(diméthyl-amino)-3-(3-méthoxy-butyl)-8-phényl-3-azaspiro[4.5]décan-2-one |
| 325 | 8-(diméthyl-amino)-3-(2-méthoxy-éhyl)-8-phényl-3-azaspiro[4.5]décan-2-one |
| 326 | 8-(diméthyl-amino)-3-(2-éthoxy-éthyl)-8-phényl-3-azaspiro[4.5]décan-2-one |
| 327 | 1-[8-(diméthyl-amino)-8-thiophén-2-yl-3-azaspiro[4.5]décan-3-yl]-3-méthoxy-butan-1-one |
| 328 | 1-[8-(diméthyl-amino)-8-phényl-3-azaspiro[4.5]décan-3-yl]-3-méthoxy-butan-1-one |
| 329 | 1-[8-(diméthyl-amino)-8-phényl-3-azaspiro[4.5]décan-3-yl]-3-méthoxy-propan-1-one |
| 330 | 3-(2-cyclopropyl-éthyl)-8-méthylamino-8-phényl-3-azaspiro[4.5]décan-2-one |
| 331 | 3-(2-cyclobutyl-éthyl)-8-méthylamino-8-phényl-3-azaspiro[4.5]décan-2-one |
| 332 | 8-(diméthyl-amino)-3-(2-méthoxy-2-méthyl-propyl)-8-thiophén-2-yl-3-azaspiro[4.5]décan-2-one |
| 333 | 2-cyclobutyl-1-(8-méthylamino-8-thiophén-2-yl-3-azaspiro[4.5]décan-3-yl)-éthanone |
| 334 | 8-(diméthyl-amino)-3-(2-éthoxy-propyl)-8-thiophén-2-yl-3-azaspiro[4.5]décan-2-one |
| 335 | 8-(diméthyl-amino)-3-(2-éthoxy-propyl)-8-phényl-3-azaspiro[4.5]décan-2-one |
| 336 | 8-(diméthyl-amino)-3-(3-méthoxy-3-méthyl-butyl)-8-(5-méthyl-thiophén-2-yl)-3-azaspiro[4.5]décan-2-one |
| 337 | 8-(5-chloro-thiophén-2-yl)-8-(diméthyl-amino)-3-(3-méthoxy-3-méthyl-butyl)-3-azaspiro[4.5]décan-2-one |
| 338 | 1-[8-(diméthyl-amino)-8-phényl-3-azaspiro[4.5]décan-3-yl]-3-éthoxy-butan-1-one |
| 339 | 1-[8-(diméthyl-amino)-8-thiophén-2-yl-3-azaspiro[4.5]décan-3-yl]-3-éthoxy-butan-1-one |
| 340 | 8-(diméthyl-amino)-3-(2-méthoxy-2-méthyl-propyl)-8-phényl-3-azaspiro[4.5]décan-2-one |
| 341 | 3-[2-(1-méthoxy-cyclobutyl)-éthyl]-8-méthylamino-8-thiophén-2-yl-3-azaspiro[4.5]décan-2-one |
| 342 | Cyclobutyl-[8-(diméthyl-amino)-8-phényl-3-azaspiro[4.5]décan-3-yl]-méthanone |
| 343 | 1-[8-(diméthyl-amino)-8-phényl-3-azaspiro[4.5]décan-3-yl]-2-méthyl-propan-1-one |
| 344 | 1-[[8-(diméthyl-amino)-2-oxo-8-thiophén-2-yl-3-azaspiro[4.5]décan-3-yl]-méthyl]-cyclopropan-1-carbonitrile |
| 345 | [8-(diméthyl-amino)-8-phényl-3-azaspiro[4.5]décan-3-yl]-(1-méthoxy-cyclopropyl)-méthanone |
| 346 | [3-(2-cyclobutyl-éthyl)-8-thiophén-2-yl-3-azaspiro[4.5]décan-8-yl]-méthyl-amine |
| 347 | 8-(diméthyl-amino)-3-(2-tétrahydro-pyran-4-yl-éthyl)-8-thiophén-2-yl-3-azaspiro[4.5]décan-2-one |
| 348 | 8-(diméthyl-amino)-8-phényl-3-(2-tétrahydro-pyran-4-yl-éthyl)-3-azaspiro[4.5]décan-2-one |
| 349 | 1-[[8-(diméthyl-amino)-2-oxo-8-phényl-3-azaspiro[4.5]décan-3-yl]-méthyl]-cyclopropan-1-carbonitrile |
| 350 | 1-[8-(diméthyl-amino)-8-phényl-3-azaspiro[4.5]décan-3-yl]-2-tétrahydro-pyran-4-yl-éthanone |
| 351 | 1-[8-(diméthyl-amino)-8-thiophén-2-yl-3-azaspiro[4.5]décan-3-yl]-2-tétrahydropyran-4-yl-éthanone |
| 352 | 1-[[8-(diméthyl-amino)-8-phényl-3-azaspiro[4.5]décan-3-yl]-méthyl]-cyclopropan-1-carbonitrile |
| 353 | 1-[[8-(diméthyl-amino)-2-oxo-8-thiophén-2-yl-3-azaspiro[4.5]décan-3-yl]-méthyl]-cyclobutan-1-carbonitrile |
| 354 | 1-[8-(diméthyl-amino)-8-phényl-3-azaspiro[4.5]décan-3-yl]-3-(1-méthoxy-cyclobutyl)-propan-1-one |
| 355 | 1-[8-(diméthyl-amino)-8-thiophén-2-yl-3-azaspiro[4.5]décan-3-yl]-3-(1-méthoxy-cyclobutyl)-propan-1-one |
| 356 | [8-(diméthyl-amino)-8-thiophén-2-yl-3-azaspiro[4.5]décan-3-yl]-(1-méthoxy-cyclopropyl)-méthanone |
| 357 | 1-[[8-(diméthyl-amino)-2-oxo-8-phényl-3-azaspiro[4.5]décan-3-yl]-méthyl]-cyclobutan-1-carbonitrile |
| 358 | [3[(1-méthoxy-cyclopropyl)méthyl]-8-thiophén-2-yl-3-azaspiro[4.5]décan-8-yl]-diméthyl-amine |
| 359 | 1-[[8-(diméthyl-amino)-8-phényl-3-azaspiro[4.5]décan-3-yl]-méthyl]-cyclobutan-1-carbonitrile |
| 360 | 1-[[8-(diméthyl-amino)-8-thiophén-2-yl-3-azaspiro[4.5]décan-3-yl]-méthyl]-cyclopropan-1-carbonitrile |
| 361 | 1-[[8-(diméthyl-amino)-8-thiophén-2-yl-3-azaspiro[4.5]décan-3-yl]-méthyl]-cyclobutan-1-carbonitrile |
| 362 | 1-[3-[8-(diméthyl-amino)-8-phényl-3-azaspiro[4.5]décan-3-yl]-3-oxo-propyl]-cyclobutan-1-carbonitrile |
| 363 | 1-[3-[8-(diméthyl-amino)-8-thiophén-2-yl-3-azaspiro[4.5]décan-3-yl]-3-oxo-propyl]-cyclobutan-1-carbonitrile |
| 364 | 1-(8-butyl-8-diméthylamino-3-azaspiro[4.5]décan-3-yl]-2-cyclopropyl-éthanone |
| 365 | Cyclopropyl-(8-diméthylamino-8-phényl-3-azaspiro[4.5]décan-3-yl)-méthanone |
| 366 | 1-(8-diméthylamino-8-phényl-3-azaspiro[4.5]décan-3-yl)-3-méthyl-butan-1-one |
| 367 | 1-(8-butyl-8-diméthylamino-3-azaspiro[4.5]décan-3-yl)-3-cyclopropyl-propan-1-one |
| 368 | 1-(8-butyl-8-diméthylamino-3-azaspiro[4.5]décan-3-yl)-4-méthoxy-4-méthylpentan-1-one |
| 369 | [3-[(1-méthoxy-cyclopropyl)-méthyl]-8-phényl-3-azaspiro[4.5]décan-8-yl]-diméthyl-amine |
| 370 | 8-(diméthyl-amino)-3-(3-méthyl-butyl)-8-thiophén-2-yl-3-azaspiro[4.5]décan-2-one |
| 371 | 1-[3-[8-(diméthyl-amino)-8-phényl-3-azaspiro[4.5]décan-3-yl)-3-oxo-propyl]-cyclopropan-1-carbonitrile |
| 372 | 1-[3-[8-(diméthyl-amino)-8-thiophén-2-yl-3-azaspiro[4.5]décan-3-yl]-3-oxo-propyl]-cyclopropan-1-carbonitrile |
| 373 | 1-(8-diméthylamino)-8-thiophén-2-yl-3-azaspiro[4.5]décan-3-yl)-2-(1-méthoxy-cyclopropyl)éthanone |
| 374 | 1-(8-diméthylamino-8-phényl-3-azaspiro[4.5]décan-3-yl)-2-(1-méthoxy-cyclopropyl)-éthanone |
| 375 | 1-(8-butyl-8-diméthylamino-2-azaspiro[4.5]décan-2-yl)-2-cyclobutyl-éthanone |
| 376 | 8-diméthylamino-3-(3-méthyl-butyl)-8-phényl-3-azaspiro[4.5]décan-2-one |
| 377 | 8-diméthylamino-3-(2-méthyl-propyl)-8-thiophén-2-yl-3-azaspiro[4.5]décan-2-one |
| 378 | 8-diméthylamino-3-(2-méthyl-propyl)-8-phényl-3-azaspiro[4.5]décan-2-one |
| 379 | (8-diméthylamino-8-phényl-2-azaspiro[4.5]décan-2-yl)-[1-(méthoxyméthyl)-cyclopropyl]-méthanone |
| 380 | 1-(8-diméthylamino-8-phényl-2-azaspiro[4.5]décan-2-yl)-3-méthoxy-2,2-diméthyl-propan-1-one |
| 381 | (8-diméthylamino-8-phényl-2-azaspiro[4.5]décan-2-yl)-[1-(méthoxyméthyl)-cyclobutyl]-méthanone |
| 382 | (8-diméthylamino-8-thiophén-2-yl-3-azaspiro[4.5]décan-3-yl)-(1-(méthoxy-cyclobutyl)-méthanone |
| 383 | (8-diméthylamino-8-thiophén-2-yl-3-azaspiro[4.5]décan-3-yl)-[1-(méthoxyméthyl)-cyclopropyl]-méthanone |
| 384 | 1-(8-diméthylamino-8-thiophén-2-yl-3-azaspiro[4.5]décan-3-yl)-3-méthoxy-2,2-diméthyl-propan-1-one |
| 385 | (8-diméthylamino-8-thiophén-2-yl-3-azaspiro[4.5]décan-3-yl)-[1-(méthoxyméthyl)-cyclobutyl]-méthanone |
| 386 | 8-diméthylamino-3-[2-(1-méthoxy-cyclopropyl)-éthyl]-8-thiophén-2-yl-3-azaspiro[4.5]décan-2-one |
| 387 | 8-diméthylamino-3-[2-(1-méthoxy-cyclopropyl)-éthyl]-8-phényl-3-azaspiro[4.5]décan-2-one |
| 388 | 1-(8-butyl-8-diméthylamino-3-azaspiro[4.5]décan-3-yl)-2-(1-(méthoxy-cyclobutyl)-éthanone |
| 389 | 1-(8-butyl-8-diméthylamino-3-azaspiro[4.5]décan-3-yl)-2-tétrahydro-furan-3-yl-éthanone |
| 390 | (8-diméthylamino-8-phényl-3-azaspiro[4.5]décan-3-yl)-(1-méthoxy-cyclobutyl)-méthanone |
| 391 | [8-diméthylamino-8-(5-méthyl-thiophén-2-yl)-3-azaspiro[4.5]décan-3-yl]-[1-(méthoxyméthyl)-cyclopropyl]-méthanone |
| 392 | [8-diméthylamino-8-(5-méthyl-thiophén-2-yl)-3-azaspiro[4.5]décan-3-yl]-[1-(méthoxyméthyl)-cyclobutyl]-méthanone |
| 393 | 1-[8-diméthylamino-8-(5-méthyl-thiophén-2-yl)-3-azaspiro[4.5]décan-3-yl]-3-méthoxy-2,2-diméthyl-propan-1-one |
| 394 | 5-(8-diméthylamino-8-phényl-3-azaspiro[4.5]décan-3-yl)-2,2-diméthyl-5-oxo-pentanitrile |
| 395 | 5-(8-diméthylamino-8-thiophén-2-yl-3-azaspiro[4.5]décan-3-yl)-2,2-diméthyl-5-oxo-pentanitrile |
| 396 | [8-(5-chloro-thiophén-2-yl)-8-diméthylamino-3-azaspiro[4.5]décan-3-yl]-[1-(méthoxyméthyl)-cyclopropyl]-méthanone |
| 397 | [8-(5-chloro-thiophén-2-yl)-8-diméthylamino-3-azaspiro[4.5]décan-3-yl]-[1-(méthoxyméthyl)-cyclobutyl]-méthanone |
| 398 | 1-[8-(5-chloro-thiophén-2-yl)-8-diméthylamino-3-azaspiro[4.5]décan-3-yl]-3-méthoxy-2,2-diméthyl-propan-1-one |
| 399 ; 400 | 8-butyl-8-(diméthyl-amino)-3-(2-tétrahydro-furan-3-yl-éthyl)-3-azaspiro[4.5]décan-2-one |
| 401 ; 402 | 1-[2-[8-butyl-8-(diméthyl-amino)-2-oxo-3-azaspiro[4.5]décan-3-yl]-éthyl]-cyclopropan-1-carbonitrile |
| 403 ; 404 | 1-[2-[8-butyl-8-(diméthyl-amino)-2-oxo-3-azaspiro[4.5]décan-3-yl]-éthyl]-cyclobutan-1-carbonitrile |
| 405 | 1-[8-(diméthyl-amino)-8-phényl-3-azaspiro[4.5]décan-3-yl]-3-(1-méthoxy-cyclopropyl)-propan-1-one |
| 406 | 8-butyl-8-(diméthyl-amino)-3-[2-(1-méthoxy-cyclobutyl)-éthyl]-3-azaspiro[4.5]décan-2-one |
| 407 | [8-(diméthyl-amino)-8-(5-fluoro-thiophén-2-yl)-3-azaspiro[4.5]décan-3-yl]-[1-(méthoxy-méthyl)-cyclobutyl]-méthanone |
| 408 | 2-cyclopropyl-1-[8-(diméthyl-amino)-8-phényl-3-azaspiro[4.5]décan-3-yl]-éthanone |
| 409 ; 410 | 8-butyl-8-(diméthyl-amino)-3-[2-oxétan-3-yl)-éthyl]-3-azaspiro[4.5]décan-2-one |
| 411 | [8-(diméthyl-amino)-8-(5-fluoro-thiophén-2-yl)-3-azaspiro[4.5]décan-3-yl)-[1-(méthoxyméthyl)-cyclopropyl]-méthanone |
| 412 | 1-[8-(diméthyl-amino)-8-thiophén-2-yl-3-azaspiro[4.5]décan-3-yl]-3-(1-(méthoxy-cyclopropyl)-propan-1-one |
| 413 | 8-butyl-8-(diméthyl-amino)-3-[2-(1-(méthoxy-cyclobutyl)-éthyl]-3-azaspiro[4.5]décan-2-one |
| 414 | 2-cyclobutyl-1-[8-(diméthyl-amino)-8-phényl-3-azaspiro[4.5]décan-3-yl]-éthanone |
| 415 | 1-[8-(diméthyl-amino)-8-thiophén-2-yl-3-azaspiro[4.5]décan-3-yl]-3-méthoxy-propan-1-one |
| 416 | 1-[8-(diméthyl-amino)-8-thiophén-2-yl-3-azaspiro[4.5]décan-3-yl]-3-méthyl-butan-1-one |
| 417 | 8-(diméthyl-amino)-8-(5-fluoro-thiophén-2-yl)-3-(3-méthoxy-3-méthyl-butyl)-3-azaspiro[4.5]décan-2-one |
| 418 | 1-[8-diméthylamino-8-(5-fluoro-thiophén-2-yl-3-azaspiro[4.5]décan-3-yl]-3-méthoxy-3-méthyl-butan-1-one |
| 419 | 3-méthoxy-1-(8-méthylamino-8-thiophén-2-yl-3-azaspiro[4.5]décan-3-yl)-propan-1-one |
| 420 | 2-cyclobutyl-1-(8-méthylamino-8-phényl-3-azaspiro[4.5]décan-3-yl)-éthanone |
| 421 | 8-(diméthyl-amino)-8-(5-fluoro-thiophén-2-yl)-3-[2-(1-méthoxy-cyclopropyl)-éthyl]-3-azaspiro[4.5]décan-2-one |
| 422 | 2-cyclopropyl-1-(8-méthylamino-8-phényl-3-azaspiro[4.5]décan-3-yl)-éthanone |
| 423 | 3-méthyl-1-(8-méthylamino-8-thiophén-2-yl-3-azaspiro[4.5]décan-3-yl)-butan-1-one |
| 424 | 8-cyclopentyl-8-diméthylamino-2-azaspiro[4.5]décane |
| 425 | 8-(5-chloro-2-thiophén-2-yl)-2-aza-spiro[4.5]déc-8-yl-diméthylamine |
| 426 | [8-(5-fluoro-thiophén-2-yl)-2-aza-spiro[4.5]déc-8-yl]-diméthylamine |
| 427 ; 428 | 8-(azétidin-1-yl)-8-(2-thiényl)-3-aza-spiro[4.5]décane |
| 429, 430 | 8-azétidin-1-yl-8-phényl-2-azaspiro[4.5]décane |
| 431 | 8-diméthylamino-8-phényl-2-azaspiro[4.5]décan-3-one |
| 432 ; 433 | 8-butyl-8-diméthylamino-2-azaspiro[4.5]décan-3-one |
sous la forme d'un stéréo-isomère individuel ou leur mélange, des composés libres et/ou leurs sels et/ou solvates physiologiquement acceptables.

12. Médicament contenant au moins un composé selon l'une des revendications 1 à 11 sous la forme d'un stéréo-isomère individuel ou leur mélange, des composés libres et/ou leurs sels et/ou solvates physiologiquement acceptables, ainsi que, éventuellement des additifs et/ou adjuvants appropriés et/ou éventuellement d'autres principes actifs.

13. Composé selon l'une des revendications 1 à 11, sous la forme d'un stéréo-isomère individuel ou leur mélange, du composé libre et/ou ses sels et/ou solvates physiologiquement acceptables, pour son utilisation dans le traitement de la douleur

14. Composé selon l'une des revendications 1 à 1, sous la forme d'un stéréo-isomère individuel ou leur mélange, des composés libres et/ou leurs sels et/ou solvates physiologiquement acceptables pour son utilisation dans le traitement des états anxieux, du stress et des syndromes liés au stress, des dépressions, de l'épilepsie, de la maladie d'Alzheimer, de la démence sénile, des dysfonctionnements cognitifs généraux, des troubles de l'apprentissage et de la mémoire (en tant que nootrope), des manifestations de sevrage, de l'abus d'alcool et/ou de drogues et/ou de médicaments et/ou la dépendance à ceux-ci, des dysfonctionnements sexuels, des affections cardiovasculaires, de l'hypotension, de l'hypertension, des acouphènes, du prurit, de la migraine, de la surdité, de la motilité intestinale insuffisante, des troubles de l'absorption de nourriture, de l'anorexie, de l'obésité, des troubles locomoteurs, de la diarrhée, de la cachexie, de l'incontinence urinaire ou comme myorelaxant, anticonvulsivant ou anesthésique ou en co-administration dans un traitement avec un analgésique opiacé ou avec un anesthésique, pour la diurèse ou l'anti-natriurèse, l'anxiolyse, pour la modulation de l'activité motrice, pour la modulation de la sécrétion de neurotransmetteurs et le traitement des maladies neurodégénératives qui sont liées à ceux-ci, pour le traitement des symptômes de sevrage et/ou pour la réduction du potentiel de dépendance des opiacés.
